# EUROPEAN PATENT APPLICATION

(11) **EP 2 422 811 A2**
(43) Date of publication of application: **29.02.2012**
(21) Application number: 10173137.0
(22) Date of filing: 27.10.2005
(51) Int. Cl.: A61K 39/395, C07K 16/00, C07K 16/28

(54) **Modulation of antibody specificity by tailoring the affinity to cognate antigens**

(30) Priority: 27.10.2004 US 622711 P; 16.09.2005 US 717209 P
(62) Divisional of application: 05818923.4
(71) Applicant: MedImmune, LLC, Gaithersburg, MD 20878 (US)
(72) Inventor: Dall'Acqua, William, Gaithersburg, MD 20878 (US); Kinch, Michael, Laytonsville, MD 20882 (US); Carles-Kinch, Kelly, Laytonsville, MD MD 20882 (US)
(74) Representative: Winter, Christopher Spencer

(57) **Abstract**

The present invention relates to methods and compositions designed for the treatment, management, prevention and/or amelioration of various disorders associated with aberrant expression and/or activity of one or more Eph receptor tyrosine kinase family members and/or one or more Eph receptor ligands, particularly the ephrins. In particular, the invention provides methods for the treatment, management, prevention and/or amelioration of a disorder associated with aberrant expression and/or activity(ies) of one or more Eph receptors and/or one or more ephrins, the method comprising administering to a subject in need thereof an effective amount of one or more Eph/ephrin modulators. The present invention further relates to methods of modulating antibody specificity by tailoring the affinity to cognate antigens.

SEQUENCE LISTING

<110> MedImmune
      Kinch, Michael
      Carles-Kinch, Kelly
      Dall'Acqua, William

<120> Modulation of antibody specificity by tailoring the affinity to cognate
antigens

<130> MED0138.2EP

<150> 60/622,711
<151> 2004-10-27

<150> 60/717,209
<151> 2005-09-16

<160> 205

<170> PatentIn version 3.3

<210> 1
<211> 2931
<212> DNA
<213> Homo sapiens

<400> 1
atggagcggc gctggcccct ggggctaggg ctggtgctgc tgctctgcgc cccgctgccc 60
ccgggggcgc gcgccaagga agttactctg atggacacaa gcaaggcaca gggagagctg 120
ggctggctgc tggatccccc aaaagatggg tggagtgaac agcaacagat actgaatggg 180
acacccctgt acatgtacca ggactgccca atgcaaggac gcagagacac tgaccactgg 240
cttcgctcca attggatcta ccgcggggag gaggcttccc gcgtccacgt ggagctgcag 300
ttcaccgtgc gggactgcaa gagtttccct gggggagccg ggcctctggg ctgcaaggag 360
accttcaacc ttctgtacat ggagagtgac caggatgtgg gcattcagct ccgacggccc 420
ttgttccaga aggtaaccac ggtggctgca gaccagagct tcaccattcg agaccttgcg 480
tctggctccg tgaagctgaa tgtggagcgc tgctctctgg gccgcctgac ccgccgtggc 540
ctctacctcg ctttccacaa cccgggtgcc tgtgtggccc tggtgtctgt ccgggtcttc 600
taccagcgct gtcctgagac cctgaatggc ttggcccaat tcccagacac tctgcctggc 660
cccgctgggt tggtggaagt ggcggggacc tgcttgcccc acgcgcgggc cagccccagg 720
ccctcaggtg caccccgcat gcactgcagc cctgatggcg agtggctggt gcctgtagga 780
cggtgccact gtgagcctgg ctatgaggaa ggtggcagtg gcgaagcatg tgttgcctgc 840
cctagcggct cctaccggat ggacatggac acaccccatt gtctcacgtg cccccagcag 900
agcactgctg agtctgaggg ggccaccatc tgtacctgtg agagcggcca ttacagagct 960
cccggggagg gcccccaggt ggcatgcaca ggtcccccct cggccccccg aaacctgagc 1020
ttctctgcct cagggactca gctctccctg cgttgggaac ccccagcaga tacgggggga 1080
cgccaggatg tcagatacag tgtgaggtgt tcccagtgtc agggcacagc acaggacggg 1140
gggccctgcc agccctgtgg ggtgggcgtg cacttctcgc cgggggcccg ggcgctcacc 1200
acacctgcag tgcatgtcaa tggccttgaa ccttatgcca actacacctt taatgtggaa 1260
gcccaaaatg gagtgtcagg gctgggcagc tctggccatg ccagcacctc agtcagcatc 1320
agcatggggc atgcagagtc actgtcaggc ctgtctctga gactggtgaa gaaagaaccg 1380
aggcaactag agctgacctg ggcggggtcc cggccccgaa gccctggggc gaacctgacc 1440
tatgagctgc acgtgctgaa ccaggatgaa gaacggtacc agatggttct agaacccagg 1500
gtcttgctga cagagctgca gcctgacacc acatacatcg tcagagtccg aatgctgacc 1560
ccactgggtc ctggcccttt ctcccctgat catgagtttc ggaccagccc accagtgtcc 1620
aggggcctga ctggaggaga gattgtagcc gtcatctttg ggctgctgct tggtgcagcc 1680
ttgctgcttg ggattctcgt tttccggtcc aggagagccc agcggcagag gcagcagagg 1740
cagcgtgacc gcgccaccga tgtggatcga gaggacaagc tgtggctgaa gccttatgtg 1800
gacctccagg catacgagga ccctgcacag ggagccttgg actttacccg ggagcttgat 1860
ccagcgtggc tgatggtgga cactgtcata ggagaaggag agtttgggga agtgtatcga 1920
gggaccctga ggctccccag ccaggactgc aagactgtgg ccattaagac cttaaaagac 1980
acatccccag gtggccagtg gtggaacttc cttcgagagg caactatcat gggccagttt 2040
agccacccgc atattctgca tctggaaggc gtcgtcacaa agcgaaagcc gatcatgatc 2100
atcacagaat ttatggagaa tggagccctg gatgccttcc tgagggagcg ggaggaccag 2160
ctggtccctg ggcagctagt ggccatgctg cagggcatag catctggcat gaactacctc 2220
agtaatcaca attatgtcca ccgggacctg gctgccagaa acatcttggt gaatcaaaac 2280

ctgtgctgca aggtgtctga ctttggcctg actcgcctcc tggatgactt tgatggcaca 2340
tacgaaaccc agggaggaaa gatccctatc cgttggacag cccctgaagc cattgcccat 2400
cggatcttca ccacagccag cgatgtgtgg agctttggga ttgtgatgtg ggaggtgctg 2460
agctttgggg acaagcctta tggggagatg agcaatcagg aggttatgaa gagcattgag 2520
gatgggtacc ggttgccccc tcctgtggac tgccctgccc ctctgtatga gctcatgaag 2580
aactgctggg catatgaccg tgcccgccgg ccacacttcc agaagcttca ggcacatctg 2640
gagcaactgc ttgccaaccc ccactccctg cggaccattg ccaactttga ccccagggtg 2700
actcttcgcc tgcccagcct gagtggctca gatgggatcc cgtatcgaac cgtctctgag 2760
tggctcgagt ccatacgcat gaaacgctac atcctgcact tccactcggc tgggctggac 2820
accatggagt gtgtgctgga gctgaccgct gaggacctga cgcagatggg aatcacactg 2880
cccgggcacc agaagcgcat tctttgcagt attcagggat tcaaggactg a 2931

<210> 2
<211> 976
<212> PRT
<213> Homo sapiens

<220>
<221> BINDING
<222> (27)...(204)
<223> Ephrin receptor ligand binding domain

<220>
<221> CHAIN
<222> (333)...(440)
<223> Fibronectin type 3 domain

<220>
<221> CHAIN
<222> (453)...(535)
<223> Fibronectin type 3 domain

<220>
<221> CHAIN
<222> (618)...(883)
<223> Tyrosine kinase, catalytic domain

<220>
<221> CHAIN
<222> (911)...(975)
<223> SAM domain (Sterile alpha motif)

<400> 2
Met Glu Arg Arg Trp Pro Leu Gly Leu Gly Leu Val Leu Leu Leu Cys
1               5                   10                  15
Ala Pro Leu Pro Pro Gly Ala Arg Ala Lys Glu Val Thr Leu Met Asp
            20                  25                  30
Thr Ser Lys Ala Gln Gly Glu Leu Gly Trp Leu Leu Asp Pro Pro Lys
        35                  40                  45
Asp Gly Trp Ser Glu Gln Gln Gln Ile Leu Asn Gly Thr Pro Leu Tyr
    50                  55                  60
Met Tyr Gln Asp Cys Pro Met Gln Gly Arg Arg Asp Thr Asp His Trp
65                  70                  75                  80
Leu Arg Ser Asn Trp Ile Tyr Arg Gly Glu Glu Ala Ser Arg Val His
                85                  90                  95
Val Glu Leu Gln Phe Thr Val Arg Asp Cys Lys Ser Phe Pro Gly Gly
            100                 105                 110
Ala Gly Pro Leu Gly Cys Lys Glu Thr Phe Asn Leu Leu Tyr Met Glu
        115                 120                 125
Ser Asp Gln Asp Val Gly Ile Gln Leu Arg Arg Pro Leu Phe Gln Lys
    130                 135                 140
Val Thr Thr Val Ala Ala Asp Gln Ser Phe Thr Ile Arg Asp Leu Ala
145                 150                 155                 160
Ser Gly Ser Val Lys Leu Asn Val Glu Arg Cys Ser Leu Gly Arg Leu
                165                 170                 175
Thr Arg Arg Gly Leu Tyr Leu Ala Phe His Asn Pro Gly Ala Cys Val
            180                 185                 190
Ala Leu Val Ser Val Arg Val Phe Tyr Gln Arg Cys Pro Glu Thr Leu
        195                 200                 205

Asn Gly Leu Ala Gln Phe Pro Asp Thr Leu Pro Gly Pro Ala Gly Leu
    210                 215                 220
Val Glu Val Ala Gly Thr Cys Leu Pro His Ala Arg Ala Ser Pro Arg
225                 230                 235                 240
Pro Ser Gly Ala Pro Arg Met His Cys Ser Pro Asp Gly Glu Trp Leu
                245                 250                 255
Val Pro Val Gly Arg Cys His Cys Glu Pro Gly Tyr Glu Glu Gly Gly
            260                 265                 270
Ser Gly Glu Ala Cys Val Ala Cys Pro Ser Gly Ser Tyr Arg Met Asp
        275                 280                 285
Met Asp Thr Pro His Cys Leu Thr Cys Pro Gln Gln Ser Thr Ala Glu
    290                 295                 300
Ser Glu Gly Ala Thr Ile Cys Thr Cys Glu Ser Gly His Tyr Arg Ala
305                 310                 315                 320
Pro Gly Glu Gly Pro Gln Val Ala Cys Thr Gly Pro Pro Ser Ala Pro
                325                 330                 335
Arg Asn Leu Ser Phe Ser Ala Ser Gly Thr Gln Leu Ser Leu Arg Trp
            340                 345                 350
Glu Pro Pro Ala Asp Thr Gly Gly Arg Gln Asp Val Arg Tyr Ser Val
        355                 360                 365
Arg Cys Ser Gln Cys Gln Gly Thr Ala Gln Asp Gly Gly Pro Cys Gln
    370                 375                 380
Pro Cys Gly Val Gly Val His Phe Ser Pro Gly Ala Arg Ala Leu Thr
385                 390                 395                 400
Thr Pro Ala Val His Val Asn Gly Leu Glu Pro Tyr Ala Asn Tyr Thr
                405                 410                 415
Phe Asn Val Glu Ala Gln Asn Gly Val Ser Gly Leu Gly Ser Ser Gly
            420                 425                 430
His Ala Ser Thr Ser Val Ser Ile Ser Met Gly His Ala Glu Ser Leu
        435                 440                 445
Ser Gly Leu Ser Leu Arg Leu Val Lys Lys Glu Pro Arg Gln Leu Glu
    450                 455                 460
Leu Thr Trp Ala Gly Ser Arg Pro Arg Ser Pro Gly Ala Asn Leu Thr
465                 470                 475                 480
Tyr Glu Leu His Val Leu Asn Gln Asp Glu Glu Arg Tyr Gln Met Val
                485                 490                 495
Leu Glu Pro Arg Val Leu Leu Thr Glu Leu Gln Pro Asp Thr Thr Tyr
            500                 505                 510
Ile Val Arg Val Arg Met Leu Thr Pro Leu Gly Pro Gly Pro Phe Ser
        515                 520                 525
Pro Asp His Glu Phe Arg Thr Ser Pro Pro Val Ser Arg Gly Leu Thr
    530                 535                 540
Gly Gly Glu Ile Val Ala Val Ile Phe Gly Leu Leu Leu Gly Ala Ala
545                 550                 555                 560
Leu Leu Leu Gly Ile Leu Val Phe Arg Ser Arg Arg Ala Gln Arg Gln
                565                 570                 575
Arg Gln Gln Arg Gln Arg Asp Arg Ala Thr Asp Val Asp Arg Glu Asp
            580                 585                 590
Lys Leu Trp Leu Lys Pro Tyr Val Asp Leu Gln Ala Tyr Glu Asp Pro
        595                 600                 605
Ala Gln Gly Ala Leu Asp Phe Thr Arg Glu Leu Asp Pro Ala Trp Leu
    610                 615                 620
Met Val Asp Thr Val Ile Gly Glu Gly Glu Phe Gly Glu Val Tyr Arg
625                 630                 635                 640
Gly Thr Leu Arg Leu Pro Ser Gln Asp Cys Lys Thr Val Ala Ile Lys
                645                 650                 655
Thr Leu Lys Asp Thr Ser Pro Gly Gly Gln Trp Trp Asn Phe Leu Arg
            660                 665                 670
Glu Ala Thr Ile Met Gly Gln Phe Ser His Pro His Ile Leu His Leu
        675                 680                 685
Glu Gly Val Val Thr Lys Arg Lys Pro Ile Met Ile Ile Thr Glu Phe
    690                 695                 700
Met Glu Asn Gly Ala Leu Asp Ala Phe Leu Arg Glu Arg Glu Asp Gln
705                 710                 715                 720
Leu Val Pro Gly Gln Leu Val Ala Met Leu Gln Gly Ile Ala Ser Gly
                725                 730                 735
Met Asn Tyr Leu Ser Asn His Asn Tyr Val His Arg Asp Leu Ala Ala
            740                 745                 750
Arg Asn Ile Leu Val Asn Gln Asn Leu Cys Cys Lys Val Ser Asp Phe

755                 760                 765
Gly Leu Thr Arg Leu Leu Asp Asp Phe Asp Gly Thr Tyr Glu Thr Gln
    770                 775                 780
Gly Gly Lys Ile Pro Ile Arg Trp Thr Ala Pro Glu Ala Ile Ala His
785                 790                 795                 800
Arg Ile Phe Thr Thr Ala Ser Asp Val Trp Ser Phe Gly Ile Val Met
                805                 810                 815
Trp Glu Val Leu Ser Phe Gly Asp Lys Pro Tyr Gly Glu Met Ser Asn
            820                 825                 830
Gln Glu Val Met Lys Ser Ile Glu Asp Gly Tyr Arg Leu Pro Pro Pro
        835                 840                 845
Val Asp Cys Pro Ala Pro Leu Tyr Glu Leu Met Lys Asn Cys Trp Ala
    850                 855                 860
Tyr Asp Arg Ala Arg Arg Pro His Phe Gln Lys Leu Gln Ala His Leu
865                 870                 875                 880
Glu Gln Leu Leu Ala Asn Pro His Ser Leu Arg Thr Ile Ala Asn Phe
                885                 890                 895
Asp Pro Arg Val Thr Leu Arg Leu Pro Ser Leu Ser Gly Ser Asp Gly
            900                 905                 910
Ile Pro Tyr Arg Thr Val Ser Glu Trp Leu Glu Ser Ile Arg Met Lys
        915                 920                 925
Arg Tyr Ile Leu His Phe His Ser Ala Gly Leu Asp Thr Met Glu Cys
    930                 935                 940
Val Leu Glu Leu Thr Ala Glu Asp Leu Thr Gln Met Gly Ile Thr Leu
945                 950                 955                 960
Pro Gly His Gln Lys Arg Ile Leu Cys Ser Ile Gln Gly Phe Lys Asp
                965                 970                 975

<210> 3
<211> 2931
<212> DNA
<213> Homo sapiens

<400> 3
atggagctcc aggcagcccg cgcctgcttc gccctgctgt ggggctgtgc gctggccgcg 60
gccgcggcgg cgcagggcaa ggaagtggta ctgctggact ttgctgcagc tggaggggag 120
ctcggctggc tcacacaccc gtatggcaaa gggtgggacc tgatgcagaa catcatgaat 180
gacatgccga tctacatgta ctccgtgtgc aacgtgatgt ctggcgacca ggacaactgg 240
ctccgcacca actgggtgta ccgaggagag gctgagcgta tcttcattga gctcaagttt 300
actgtacgtg actgcaacag cttccctggt ggcgccagct cctgcaagga gactttcaac 360
ctctactatg ccgagtcgga cctggactac ggcaccaact tccagaagcg cctgttcacc 420
aagattgaca ccattgcgcc cgatgagatc accgtcagca gcgacttcga ggcacgccac 480
gtgaagctga acgtggagga gcgctccgtg gggccgctca cccgcaaagg cttctacctg 540
gccttccagg atatcggtgc ctgtgtggcg ctgctctccg tccgtgtcta ctacaagaag 600
tgccccgagc tgctgcaggg cctggcccac ttccctgaga ccatcgccgg ctctgatgca 660
ccttccctgg ccactgtggc cggcacctgt gtggaccatg ccgtggtgcc accggggggt 720
gaagagcccc gtatgcactg tgcagtggat ggcgagtggc tggtgcccat tgggcagtgc 780
ctgtgccagg caggctacga gaaggtggag gatgcctgcc aggcctgctc gcctggattt 840
tttaagtttg aggcatctga gagcccctgc ttggagtgcc ctgagcacac gctgccatcc 900
cctgagggtg ccacctcctg cgagtgtgag gaaggcttct tccgggcacc tcaggaccca 960
gcgtcgatgc cttgcacacg acccccctcc gccccacact acctcacagc cgtgggcatg 1020
ggtgccaagg tggagctgcg ctggacgccc cctcaggaca gcgggggccg cgaggacatt 1080
gtctacagcg tcacctgcga acagtgctgg cccgagtctg gggaatgcgg gccgtgtgag 1140
gccagtgtgc gctactcgga gcctcctcac ggactgaccc gcaccagtgt gacagtgagc 1200
gacctggagc cccacatgaa ctacaccttc accgtggagg cccgcaatgg cgtctcaggc 1260
ctggtaacca gccgcagctt ccgtactgcc agtgtcagca tcaaccagac agagcccccc 1320
aaggtgaggc tggagggccg cagcaccacc tcgcttagcg tctcctggag catccccccg 1380
ccgcagcaga gccgagtgtg gaagtacgag gtcacttacc gcaagaaggg agactccaac 1440
agctacaatg tgcgccgcac cgagggtttc tccgtgaccc tggacgacct ggccccagac 1500
accacctacc tggtccaggt gcaggcactg acgcaggagg gccagggggc cggcagcaag 1560
gtgcacgaat tccagacgct gtccccggag ggatctggca acttggcggt gattggcggc 1620
gtggctgtcg gtgtggtcct gcttctggtg ctggcaggag ttggcttctt tatccaccgc 1680
aggaggaaga accagcgtgc ccgccagtcc ccggaggacg tttacttctc caagtcagaa 1740
caactgaagc ccctgaagac atacgtggac ccccacacat atgaggaccc caaccaggct 1800
gtgttgaagt tcactaccga gatccatcca tcctgtgtca ctcggcagaa ggtgatcgga 1860
gcaggagagt ttggggaggt gtacaagggc atgctgaaga catcctcggg gaagaaggag 1920
gtgccggtgg ccatcaagac gctgaaagcc ggctacacag agaagcagcg agtggacttc 1980
ctcggcgagg ccggcatcat gggccagttc agccaccaca acatcatccg cctagagggc 2040

gtcatctcca aatacaagcc catgatgatc atcactgagt acatggagaa tggggccctg 2100
gacaagttcc ttcgggagaa ggatggcgag ttcagcgtgc tgcagctggt gggcatgctg 2160
cggggcatcg cagctggcat gaagtacctg gccaacatga actatgtgca ccgtgacctg 2220
gctgcccgca acatcctcgt caacagcaac ctggtctgca aggtgtctga ctttggcctg 2280
tcccgcgtgc tggaggacga ccccgaggcc acctacacca ccagtggcgg caagatcccc 2340
atccgctgga ccgccccgga ggccatttcc taccggaagt tcacctctgc cagcgacgtg 2400
tggagctttg gcattgtcat gtgggaggtg atgacctatg gcgagcggcc ctactgggag 2460
ttgtccaacc acgaggtgat gaaagccatc aatgatggct tccggctccc cacacccatg 2520
gactgcccct ccgccatcta ccagctcatg atgcagtgct ggcagcagga gcgtgcccgc 2580
cgccccaagt tcgctgacat cgtcagcatc ctggacaagc tcattcgtgc ccctgactcc 2640
ctcaagaccc tggctgactt tgacccccgc gtgtctatcc ggctccccag cacgagcggc 2700
tcggaggggg tgcccttccg cacggtgtcc gagtggctgg agtccatcaa gatgcagcag 2760
tatacggagc acttcatggc ggccggctac actgccatcg agaaggtggt gcagatgacc 2820
aacgacgaca tcaagaggat tggggtgcgg ctgcccggcc accagaagcg catcgcctac 2880
agcctgctgg gactcaagga ccaggtgaac actgtgggga tccccatctg a 2931

<210> 4
<211> 976
<212> PRT
<213> Homo sapiens

<220>
<221> BINDING
<222> (28)...(201)
<223> Ephrin receptor ligand binding domain

<220>
<221> CHAIN
<222> (332)...(424)
<223> Fibronectin type 3 domain

<220>
<221> CHAIN
<222> (439)...(519)
<223> Fibronectin type 3 domain

<220>
<221> CHAIN
<222> (607)...(874)
<223> Tyrosine kinase, catalytic domain

<220>
<221> CHAIN
<222> (902)...(968)
<223> SAM Domain (Sterile alpha motif)

<400> 4

<210> 5
<211> 2952
<212> DNA
<213> Homo sapiens

<400> 5
atggattgtc agctctccat cctcctcctt ctcagctgct ctgttctcga cagcttcggg 60
gaactgattc cgcagccttc caatgaagtc aatctactgg attcaaaaac aattcaaggg 120
gagctgggct ggatctctta tccatcacat gggtgggaag agatcagtgg tgtggatgaa 180
cattacacac ccatcaggac ttaccaggtg tgcaatgtca tggaccacag tcaaaacaat 240
tggctgagaa caaactgggt ccccaggaac tcagctcaga agatttatgt ggagctcaag 300
ttcactctac gagactgcaa tagcattcca ttggttttag gaacttgcaa ggagacattc 360
aacctgtact acatggagtc tgatgatgat catggggtga aatttcgaga gcatcagttt 420
acaaagattg acaccattgc agctgatgaa agtttcactc aaatggatct tggggaccgt 480
attctgaagc tcaacactga gattagagaa gtaggtcctg tcaacaagaa gggattttat 540
ttggcatttc aagatgttgg tgcttgtgtt gccttggtgt ctgtgagagt atacttcaaa 600
aagtgcccat ttacagtgaa gaatctggct atgtttccag acacggtacc catggactcc 660
cagtccctgg tggaggttag agggtcttgt gtcaacaatt ctaaggagga agatcctcca 720
aggatgtact gcagtacaga aggcgaatgg cttgtaccca ttggcaagtg ttcctgcaat 780
gctggctatg aagaaagagg ttttatgtgc caagcttgtc gaccaggttt ctacaaggca 840
ttggatggta atatgaagtg tgctaagtgc ccgcctcaca gttctactca ggaagatggt 900
tcaatgaact gcaggtgtga gaataattac ttccgggcag acaaagaccc tccatccatg 960
gcttgtaccc gacctccatc ttcaccaaga aatgttatct ctaatataaa cgagacctca 1020
gttatcctgg actggagttg gcccctggac acaggaggcc ggaaagatgt taccttcaac 1080
atcatatgta aaaaatgtgg gtggaatata aaacagtgtg agccatgcag cccaaatgtc 1140
cgcttcctcc ctcgacagtt tggactcacc aacaccacgg tgacagtgac agaccttctg 1200
gcacatacta actacacctt tgagattgat gccgttaatg gggtgtcaga gctgagctcc 1260
ccaccaagac agtttgctgc ggtcagcatc acaactaatc aggctgctcc atcacctgtc 1320
ctgacgatta agaaagatcg gacctccaga aatagcatct ctttgtcctg gcaagaacct 1380
gaacatccta atgggatcat attggactac gaggtcaaat actatgaaaa gcaggaacaa 1440
gaaacaagtt ataccattct gagggcaaga ggcacaaatg ttaccatcag tagcctcaag 1500
cctgacacta tatacgtatt ccaaatccga gcccgaacag ccgctggata tgggacgaac 1560
agccgcaagt ttgagtttga aactagtcca gactctttct ccatctctgg tgaaagtagc 1620
caagtggtca tgatcgccat ttcagcggca gtagcaatta ttctcctcac tgttgtcatc 1680
tatgttttga ttgggaggtt ctgtggctat aagtcaaaac atggggcaga tgaaaaaaga 1740
cttcattttg gcaatgggca tttaaaactt ccaggtctca ggacttatgt tgacccacat 1800

acatatgaag accctaccca agctgttcat gagtttgcca aggaattgga tgccaccaac 1860
atatccattg ataaagttgt tggagcaggt gaatttggag aggtgtgcag tggtcgctta 1920
aaacttcctt caaaaaaaga gatttcagtg gccattaaga ccctgaaagt tggctacaca 1980
gaaaagcaga ggagagactt cctgggagaa gcaagcatta tgggacagtt tgaccacccc 2040
aatatcattc gactggaagg agttgttacc aaaagtaagc cagttatgat tgtcacagaa 2100
tacatggaga atggttcctt ggatagtttc ctacgtaaac acgatgccca gtttactgtc 2160
attcagctag tggggatgct tcgagggata gcatctggca tgaagtacct gtcagacatg 2220
ggctatgttc accgagacct cgctgctcgg aacatcttga tcaacagtaa cttggtgtgt 2280
aaggtttctg atttcggact ttcgcgtgtc ctggaggatg acccagaagc tgcttataca 2340
acaagaggag ggaagatccc aatcaggtgg acatcaccag aagctatagc ctaccgcaag 2400
ttcacgtcag ccagcgatgt atggagttat gggattgttc tctgggaggt gatgtcttat 2460
ggagagagac catactggga gatgtccaat caggatgtaa ttaaagctgt agatgagggc 2520
tatcgactgc caccccccat ggactgccca gctgccttgt atcagctgat gctggactgc 2580
tggcagaaag acaggaacaa cagacccaag tttgagcaga ttgttagtat tctggacaag 2640
cttatccgga atcccggcag cctgaagatc atcaccagtg cagccgcaag gccatcaaac 2700
cttcttctgg accaaagcaa tgtggatatc actaccttcc gcacaacagg tgactggctt 2760
aatggtgtct ggacagcaca ctgcaaggaa atcttcacgg gtgtggagta cagttcttgt 2820
gacacaatag ccaagatttc cacagatgac atgaaaaagg ttggtgtcac cgtggttggg 2880
ccacagaaga agatcatcag tagcattaaa gctctagaaa cgcaatcaaa gaatggccca 2940
gttcccgtgt aa 2952

<210> 6
<211> 983
<212> PRT
<213> Homo sapiens

<220>
<221> BINDING
<222> (28)...(202)
<223> Ephrin receptor ligand binding domain

<220>
<221> CHAIN
<222> (326)...(421)
<223> Fibronectin type 3 domain

<220>
<221> CHAIN
<222> (437)...(528)
<223> Fibronectin type 3 domain

<220>
<221> CHAIN
<222> (615)...(881)
<223> Tyrosine kinase, catalytic domain

<220>
<221> CHAIN
<222> (912)...(973)
<223> SAM Domain (Sterile alpha motif)

<400> 6
Met Asp Cys Gln Leu Ser Ile Leu Leu Leu Leu Ser Cys Ser Val Leu
1               5                   10                  15
Asp Ser Phe Gly Glu Leu Ile Pro Gln Pro Ser Asn Glu Val Asn Leu
            20                  25                  30
Leu Asp Ser Lys Thr Ile Gln Gly Glu Leu Gly Trp Ile Ser Tyr Pro
        35                  40                  45
Ser His Gly Trp Glu Glu Ile Ser Gly Val Asp Glu His Tyr Thr Pro
    50                  55                  60
Ile Arg Thr Tyr Gln Val Cys Asn Val Met Asp His Ser Gln Asn Asn
65                  70                  75                  80
Trp Leu Arg Thr Asn Trp Val Pro Arg Asn Ser Ala Gln Lys Ile Tyr
                85                  90                  95
Val Glu Leu Lys Phe Thr Leu Arg Asp Cys Asn Ser Ile Pro Leu Val
            100                 105                 110
Leu Gly Thr Cys Lys Glu Thr Phe Asn Leu Tyr Tyr Met Glu Ser Asp
        115                 120                 125
Asp Asp His Gly Val Lys Phe Arg Glu His Gln Phe Thr Lys Ile Asp

130                 135                 140
Thr Ile Ala Ala Asp Glu Ser Phe Thr Gln Met Asp Leu Gly Asp Arg
145                 150                 155                 160
Ile Leu Lys Leu Asn Thr Glu Ile Arg Glu Val Gly Pro Val Asn Lys
                165                 170                 175
Lys Gly Phe Tyr Leu Ala Phe Gln Asp Val Gly Ala Cys Val Ala Leu
            180                 185                 190
Val Ser Val Arg Val Tyr Phe Lys Lys Cys Pro Phe Thr Val Lys Asn
        195                 200                 205
Leu Ala Met Phe Pro Asp Thr Val Pro Met Asp Ser Gln Ser Leu Val
    210                 215                 220
Glu Val Arg Gly Ser Cys Val Asn Asn Ser Lys Glu Glu Asp Pro Pro
225                 230                 235                 240
Arg Met Tyr Cys Ser Thr Glu Gly Glu Trp Leu Val Pro Ile Gly Lys
                245                 250                 255
Cys Ser Cys Asn Ala Gly Tyr Glu Glu Arg Gly Phe Met Cys Gln Ala
            260                 265                 270
Cys Arg Pro Gly Phe Tyr Lys Ala Leu Asp Gly Asn Met Lys Cys Ala
        275                 280                 285
Lys Cys Pro Pro His Ser Ser Thr Gln Glu Asp Gly Ser Met Asn Cys
    290                 295                 300
Arg Cys Glu Asn Asn Tyr Phe Arg Ala Asp Lys Asp Pro Pro Ser Met
305                 310                 315                 320
Ala Cys Thr Arg Pro Pro Ser Ser Pro Arg Asn Val Ile Ser Asn Ile
                325                 330                 335
Asn Glu Thr Ser Val Ile Leu Asp Trp Ser Trp Pro Leu Asp Thr Gly
            340                 345                 350
Gly Arg Lys Asp Val Thr Phe Asn Ile Ile Cys Lys Lys Cys Gly Trp
        355                 360                 365
Asn Ile Lys Gln Cys Glu Pro Cys Ser Pro Asn Val Arg Phe Leu Pro
    370                 375                 380
Arg Gln Phe Gly Leu Thr Asn Thr Thr Val Thr Val Thr Asp Leu Leu
385                 390                 395                 400
Ala His Thr Asn Tyr Thr Phe Glu Ile Asp Ala Val Asn Gly Val Ser
                405                 410                 415
Glu Leu Ser Ser Pro Pro Arg Gln Phe Ala Ala Val Ser Ile Thr Thr
            420                 425                 430
Asn Gln Ala Ala Pro Ser Pro Val Leu Thr Ile Lys Lys Asp Arg Thr
        435                 440                 445
Ser Arg Asn Ser Ile Ser Leu Ser Trp Gln Glu Pro Glu His Pro Asn
    450                 455                 460
Gly Ile Ile Leu Asp Tyr Glu Val Lys Tyr Tyr Glu Lys Gln Glu Gln
465                 470                 475                 480
Glu Thr Ser Tyr Thr Ile Leu Arg Ala Arg Gly Thr Asn Val Thr Ile
                485                 490                 495
Ser Ser Leu Lys Pro Asp Thr Ile Tyr Val Phe Gln Ile Arg Ala Arg
            500                 505                 510
Thr Ala Ala Gly Tyr Gly Thr Asn Ser Arg Lys Phe Glu Phe Glu Thr
        515                 520                 525
Ser Pro Asp Ser Phe Ser Ile Ser Gly Glu Ser Ser Gln Val Val Met
    530                 535                 540
Ile Ala Ile Ser Ala Ala Val Ala Ile Ile Leu Leu Thr Val Val Ile
545                 550                 555                 560
Tyr Val Leu Ile Gly Arg Phe Cys Gly Tyr Lys Ser Lys His Gly Ala
                565                 570                 575
Asp Glu Lys Arg Leu His Phe Gly Asn Gly His Leu Lys Leu Pro Gly
            580                 585                 590
Leu Arg Thr Tyr Val Asp Pro His Thr Tyr Glu Asp Pro Thr Gln Ala
        595                 600                 605
Val His Glu Phe Ala Lys Glu Leu Asp Ala Thr Asn Ile Ser Ile Asp
    610                 615                 620
Lys Val Val Gly Ala Gly Glu Phe Gly Glu Val Cys Ser Gly Arg Leu
625                 630                 635                 640
Lys Leu Pro Ser Lys Lys Glu Ile Ser Val Ala Ile Lys Thr Leu Lys
                645                 650                 655
Val Gly Tyr Thr Glu Lys Gln Arg Arg Asp Phe Leu Gly Glu Ala Ser
            660                 665                 670
Ile Met Gly Gln Phe Asp His Pro Asn Ile Ile Arg Leu Glu Gly Val
        675                 680                 685

Val Thr Lys Ser Lys Pro Val Met Ile Val Thr Glu Tyr Met Glu Asn
    690                 695                 700
Gly Ser Leu Asp Ser Phe Leu Arg Lys His Asp Ala Gln Phe Thr Val
705                 710                 715                 720
Ile Gln Leu Val Gly Met Leu Arg Gly Ile Ala Ser Gly Met Lys Tyr
                725                 730                 735
Leu Ser Asp Met Gly Tyr Val His Arg Asp Leu Ala Ala Arg Asn Ile
                740                 745                 750
Leu Ile Asn Ser Asn Leu Val Cys Lys Val Ser Asp Phe Gly Leu Ser
        755                 760                 765
Arg Val Leu Glu Asp Asp Pro Glu Ala Ala Tyr Thr Thr Arg Gly Gly
    770                 775                 780
Lys Ile Pro Ile Arg Trp Thr Ser Pro Glu Ala Ile Ala Tyr Arg Lys
785                 790                 795                 800
Phe Thr Ser Ala Ser Asp Val Trp Ser Tyr Gly Ile Val Leu Trp Glu
                805                 810                 815
Val Met Ser Tyr Gly Glu Arg Pro Tyr Trp Glu Met Ser Asn Gln Asp
                820                 825                 830
Val Ile Lys Ala Val Asp Glu Gly Tyr Arg Leu Pro Pro Pro Met Asp
        835                 840                 845
Cys Pro Ala Ala Leu Tyr Gln Leu Met Leu Asp Cys Trp Gln Lys Asp
    850                 855                 860
Arg Asn Asn Arg Pro Lys Phe Glu Gln Ile Val Ser Ile Leu Asp Lys
865                 870                 875                 880
Leu Ile Arg Asn Pro Gly Ser Leu Lys Ile Ile Thr Ser Ala Ala Ala
                885                 890                 895
Arg Pro Ser Asn Leu Leu Leu Asp Gln Ser Asn Val Asp Ile Thr Thr
                900                 905                 910
Phe Arg Thr Thr Gly Asp Trp Leu Asn Gly Val Trp Thr Ala His Cys
        915                 920                 925
Lys Glu Ile Phe Thr Gly Val Glu Tyr Ser Ser Cys Asp Thr Ile Ala
    930                 935                 940
Lys Ile Ser Thr Asp Asp Met Lys Lys Val Gly Val Thr Val Val Gly
945                 950                 955                 960
Pro Gln Lys Lys Ile Ile Ser Ser Ile Lys Ala Leu Glu Thr Gln Ser
                965                 970                 975
Lys Asn Gly Pro Val Pro Val
                980

<210> 7
<211> 1620
<212> DNA
<213> Homo sapiens

<400> 7
atggattgtc agctctccat cctcctcctt ctcagctgct ctgttctcga cagcttcggg 60
gaactgattc cgcagccttc caatgaagtc aatctactgg attcaaaaac aattcaaggg 120
gagctgggct ggatctctta tccatcacat gggtgggaag agatcagtgg tgtggatgaa 180
cattacacac ccatcaggac ttaccaggtg tgcaatgtca tggaccacag tcaaaacaat 240
tggctgagaa caaactgggt ccccaggaac tcagctcaga agatttatgt ggagctcaag 300
ttcactctac gagactgcaa tagcattcca ttggttttag gaacttgcaa ggagacattc 360
aacctgtact acatggagtc tgatgatgat catggggtga aatttcgaga gcatcagttt 420
acaaagattg acaccattgc agctgatgaa agtttcactc aaatggatct tggggaccgt 480
attctgaagc tcaacactga gattagagaa gtaggtcctg tcaacaagaa gggattttat 540
ttggcatttc aagatgttgg tgcttgtgtt gccttggtgt ctgtgagagt atacttcaaa 600
aagtgcccat ttacagtgaa gaatctggct atgtttccag acacggtacc catggactcc 660
cagtccctgg tggaggttag agggtcttgt gtcaacaatt ctaaggagga agatcctcca 720
aggatgtact gcagtacaga aggcgaatgg cttgtaccca ttggcaagtg ttcctgcaat 780
gctggctatg aagaaagagg ttttatgtgc caagcttgtc gaccaggttt ctacaaggca 840
ttggatggta atatgaagtg tgctaagtgc ccgcctcaca gttctactca ggaagatggt 900
tcaatgaact gcaggtgtga gaataattac ttccgggcag acaaagaccc tccatccatg 960
gcttgtaccc gacctccatc ttcaccaaga aatgttatct ctaatataaa cgagacctca 1020
gttatcctgg actggagttg gcccctggac acaggaggcc ggaaagatgt taccttcaac 1080
atcatatgta aaaaatgtgg gtggaatata aaacagtgtg agccatgcag cccaaatgtc 1140
cgcttcctcc ctcgacagtt tggactcacc aacaccacgg tgacagtgac agaccttctg 1200
gcacatacta actacacctt tgagattgat gccgttaatg gggtgtcaga gctgagctcc 1260
ccaccaagac agtttgctgc ggtcagcatc acaactaatc aggctgctcc atcacctgtc 1320
ctgacgatta agaaagatcg gacctccaga aatagcatct ctttgtcctg gcaagaacct 1380

gaacatccta atgggatcat attggactac gaggtcaaat actatgaaaa gcaggaacaa 1440
gaaacaagtt ataccattct gagggcaaga ggcacaaatg ttaccatcag tagcctcaag 1500
cctgacacta tatacgtatt ccaaatccga gcccgaacag ccgctggata tgggacgaac 1560
agccgcaagt ttgagtttga aactagtcca gactgtatgt attatttcaa tgcagtctag 1620

<210> 8
<211> 539
<212> PRT
<213> Homo sapiens

<220>
<221> BINDING
<222> (29)...(202)
<223> Ephrin receptor ligand binding domain

<220>
<221> CHAIN
<222> (326)...(421)
<223> Fibronectin type 3 domain

<220>
<221> CHAIN
<222> (437)...(528)
<223> Fibronectin type 3 domain

<400> 8
Met Asp Cys Gln Leu Ser Ile Leu Leu Leu Leu Ser Cys Ser Val Leu
1               5                   10                  15
Asp Ser Phe Gly Glu Leu Ile Pro Gln Pro Ser Asn Glu Val Asn Leu
            20                  25                  30
Leu Asp Ser Lys Thr Ile Gln Gly Glu Leu Gly Trp Ile Ser Tyr Pro
        35                  40                  45
Ser His Gly Trp Glu Glu Ile Ser Gly Val Asp Glu His Tyr Thr Pro
    50                  55                  60
Ile Arg Thr Tyr Gln Val Cys Asn Val Met Asp His Ser Gln Asn Asn
65                  70                  75                  80
Trp Leu Arg Thr Asn Trp Val Pro Arg Asn Ser Ala Gln Lys Ile Tyr
                85                  90                  95
Val Glu Leu Lys Phe Thr Leu Arg Asp Cys Asn Ser Ile Pro Leu Val
            100                 105                 110
Leu Gly Thr Cys Lys Glu Thr Phe Asn Leu Tyr Tyr Met Glu Ser Asp
        115                 120                 125
Asp Asp His Gly Val Lys Phe Arg Glu His Gln Phe Thr Lys Ile Asp
    130                 135                 140
Thr Ile Ala Ala Asp Glu Ser Phe Thr Gln Met Asp Leu Gly Asp Arg
145                 150                 155                 160
Ile Leu Lys Leu Asn Thr Glu Ile Arg Glu Val Gly Pro Val Asn Lys
                165                 170                 175
Lys Gly Phe Tyr Leu Ala Phe Gln Asp Val Gly Ala Cys Val Ala Leu
            180                 185                 190
Val Ser Val Arg Val Tyr Phe Lys Lys Cys Pro Phe Thr Val Lys Asn
        195                 200                 205
Leu Ala Met Phe Pro Asp Thr Val Pro Met Asp Ser Gln Ser Leu Val
    210                 215                 220
Glu Val Arg Gly Ser Cys Val Asn Asn Ser Lys Glu Glu Asp Pro Pro
225                 230                 235                 240
Arg Met Tyr Cys Ser Thr Glu Gly Glu Trp Leu Val Pro Ile Gly Lys
                245                 250                 255
Cys Ser Cys Asn Ala Gly Tyr Glu Glu Arg Gly Phe Met Cys Gln Ala
            260                 265                 270
Cys Arg Pro Gly Phe Tyr Lys Ala Leu Asp Gly Asn Met Lys Cys Ala
        275                 280                 285
Lys Cys Pro Pro His Ser Ser Thr Gln Glu Asp Gly Ser Met Asn Cys
    290                 295                 300
Arg Cys Glu Asn Asn Tyr Phe Arg Ala Asp Lys Asp Pro Pro Ser Met
305                 310                 315                 320
Ala Cys Thr Arg Pro Pro Ser Ser Pro Arg Asn Val Ile Ser Asn Ile
                325                 330                 335

Asn Glu Thr Ser Val Ile Leu Asp Trp Ser Trp Pro Leu Asp Thr Gly
            340                 345                 350
Gly Arg Lys Asp Val Thr Phe Asn Ile Ile Cys Lys Lys Cys Gly Trp
        355                 360                 365
Asn Ile Lys Gln Cys Glu Pro Cys Ser Pro Asn Val Arg Phe Leu Pro
    370                 375                 380
Arg Gln Phe Gly Leu Thr Asn Thr Thr Val Thr Val Thr Asp Leu Leu
385                 390                 395                 400
Ala His Thr Asn Tyr Thr Phe Glu Ile Asp Ala Val Asn Gly Val Ser
                405                 410                 415
Glu Leu Ser Ser Pro Pro Arg Gln Phe Ala Ala Val Ser Ile Thr Thr
            420                 425                 430
Asn Gln Ala Ala Pro Ser Pro Val Leu Thr Ile Lys Lys Asp Arg Thr
        435                 440                 445
Ser Arg Asn Ser Ile Ser Leu Ser Trp Gln Glu Pro Glu His Pro Asn
    450                 455                 460
Gly Ile Ile Leu Asp Tyr Glu Val Lys Tyr Tyr Glu Lys Gln Glu Gln
465                 470                 475                 480
Glu Thr Ser Tyr Thr Ile Leu Arg Ala Arg Gly Thr Asn Val Thr Ile
                485                 490                 495
Ser Ser Leu Lys Pro Asp Thr Ile Tyr Val Phe Gln Ile Arg Ala Arg
            500                 505                 510
Thr Ala Ala Gly Tyr Gly Thr Asn Ser Arg Lys Phe Glu Phe Glu Thr
        515                 520                 525
Ser Pro Asp Cys Met Tyr Tyr Phe Asn Ala Val
    530                 535

<210> 9
<211> 2961
<212> DNA
<213> Homo sapiens

<400> 9
atggctggga ttttctattt cgccctattt tcgtgtctct tcgggatttg cgacgctgtc 60
acaggttcca gggtataccc cgcgaatgaa gttaccttat tggattccag atctgttcag 120
ggagaacttg ggtggatagc aagccctctg gaaggagggt gggaggaagt gagtatcatg 180
gatgaaaaaa atacaccaat ccgaacctac caagtgtgca atgtgatgga acccagccag 240
aataactggc tacgaactga ttggatcacc cgagaagggg ctcagagggt gtatattgag 300
attaaattca ccttgaggga ctgcaatagt cttccgggcg tcatggggac ttgcaaggag 360
acgtttaacc tgtactacta tgaatcagac aacgacaaag agcgtttcat cagagagaac 420
cagtttgtca aaattgacac cattgctgct gatgagagct tcacccaagt ggacattggt 480
gacagaatca tgaagctgaa caccgagatc cgggatgtag ggccattaag caaaaagggg 540
ttttacctgg cttttcagga tgtgggggcc tgcatcgccc tggtatcagt ccgtgtgttc 600
tataaaaagt gtccactcac agtccgcaat ctggcccagt ttcctgacac catcacaggg 660
gctgatacgt cttccctggt ggaagttcga ggctcctgtg tcaacaactc agaagagaaa 720
gatgtgccaa aaatgtactg tggggcagat ggtgaatggc tggtacccat tggcaactgc 780
ctatgcaacg ctgggcatga ggagcggagc ggagaatgcc aagcttgcaa aattggatat 840
tacaaggctc tctccacgga tgccacctgt gccaagtgcc caccccacag ctactctgtc 900
tgggaaggag ccacctcgtg cacctgtgac cgaggctttt tcagagctga caacgatgct 960
gcctctatgc cctgcacccg tccaccatct gctcccctga acttgatttc aaatgtcaac 1020
gagacatctg tgaacttgga atggagtagc cctcagaata caggtggccg ccaggacatt 1080
tcctataatg tggtatgcaa gaaatgtgga gctggtgacc ccagcaagtg ccgaccctgt 1140
ggaagtgggg tccactacac cccacagcag aatggcttga agaccaccaa agtctccatc 1200
actgacctcc tagctcatac caattacacc tttgaaatct gggctgtgaa tggagtgtcc 1260
aaatataacc ctaacccaga ccaatcagtt tctgtcactg tgaccaccaa ccaagcagca 1320
ccatcatcca ttgctttggt ccaggctaaa gaagtcacaa gatacagtgt ggcactggct 1380
tggctggaac cagatcggcc caatggggta atcctggaat atgaagtcaa gtattatgag 1440
aaggatcaga atgagcgaag ctatcgtata gttcggacag ctgccaggaa cacagatatc 1500
aaaggcctga accctctcac ttcctatgtt ttccacgtgc gagccaggac agcagctggc 1560
tatggagact tcagtgagcc cttggaggtt acaaccaaca cagtgccttc ccggatcatt 1620
ggagatgggg ctaactccac agtccttctg gtctctgtct cgggcagtgt ggtgctggtg 1680
gtaattctca ttgcagcttt tgtcatcagc cggagacgga gtaaatacag taaagccaaa 1740
caagaagcgg atgaagagaa acatttgaat caaggtgtaa gaacatatgt ggaccccttt 1800
acgtacgaag atcccaacca agcagtgcga gagtttgcca aagaaattga cgcatcctgc 1860
attaagattg aaaaagttat aggagttggt gaatttggtg aggtatgcag tgggcgtctc 1920
aaagtgcctg gcaagagaga gatctgtgtg gctatcaaga ctctgaaagc tggttataca 1980
gacaaacaga ggagagactt cctgagtgag gccagcatca tgggacagtt tgaccatccg 2040
aacatcattc acttggaagg cgtggtcact aaatgtaaac cagtaatgat cataacagag 2100

tacatggaga atggctcctt ggatgcattc ctcaggaaaa atgatggcag atttacagtc 2160
attcagctgg tgggcatgct tcgtggcatt gggtctggga tgaagtattt atctgatatg 2220
agctatgtgc atcgtgatct ggccgcacgg aacatcctgg tgaacagcaa cttggtctgc 2280
aaagtgtctg attttggcat gtcccgagtg cttgaggatg atccggaagc agcttacacc 2340
accaggggtg gcaagattcc tatccggtgg actgcgccag aagcaattgc ctatcgtaaa 2400
ttcacatcag caagtgatgt atggagctat ggaatcgtta tgtgggaagt gatgtcgtac 2460
ggggagaggc cctattggga tatgtccaat caagatgtga ttaaagccat tgaggaaggc 2520
tatcggttac cccctccaat ggactgcccc attgcgctcc accagctgat gctagactgc 2580
tggcagaagg agaggagcga caggcctaaa tttgggcaga ttgtcaacat gttggacaaa 2640
ctcatccgca accccaacag cttgaagagg acagggacgg agagctccag acctaacact 2700
gccttgttgg atccaagctc ccctgaattc tctgctgtgg tatcagtggg cgattggctc 2760
caggccatta aaatggaccg gtataaggat aacttcacag ctgctggtta taccacacta 2820
gaggctgtgg tgcacgtgaa ccaggaggac ctggcaagaa ttggtatcac agccatcacg 2880
caccagaata agattttgag cagtgtccag gcaatgcgaa cccaaatgca gcagatgcac 2940
ggcagaatgg ttcccgtctg a 2961

<210> 10
<211> 986
<212> PRT
<213> Homo sapiens

<220>
<221> BINDING
<222> (30)...(204)
<223> Ephrin receptor ligand binding domain

<220>
<221> CHAIN
<222> (329)...(436)
<223> Fibronectin type 3 domain

<220>
<221> CHAIN
<222> (441)...(532)
<223> Fibronectin type 3 domain

<220>
<221> CHAIN
<222> (615)...(881)
<223> Tyrosine kinase, catalytic domain

<220>
<221> CHAIN
<222> (915)...(973)
<223> SAM Domain (Sterile alpha motif)

<400> 10


<210> 11
<211> 3114
<212> DNA
<213> Homo sapiens

<400> 11
atgcggggct cggggccccg gggtgcggga caccggcggc ccccaagcgg cggcggcgac 60
acccccatca ccccagcgtc cctggccggc tgctactctg cacctcgacg ggctcccctc 120
tggacgtgcc ttctcctgtg cgccgcactc cggaccctcc tggccagccc cagcaacgaa 180
gtgaatttat tggattcacg cactgtcatg ggggacctgg gatggattgc ttttccaaaa 240
aatgggtggg aagagattgg tgaagtggat gaaaattatg cccctatcca cacataccaa 300
gtatgcaaag tgatggaaca gaatcagaat aactggcttt tgaccagttg gatctccaat 360
gaaggtgctt ccagaatctt catagaactc aaatttaccc tgcgggactg caacagcctt 420
cctggaggac tggggacctg taaggaaacc tttaatatgt attactttga gtcagatgat 480
cagaatggga gaaacatcaa ggaaaaccaa tacatcaaaa ttgataccat tgctgccgat 540
gaaagcttta cagaacttga tcttggtgac cgtgttatga aactgaatac agaggtcaga 600
gatgtaggac ctctaagcaa aaagggattt tatcttgctt ttcaagatgt tggtgcttgc 660
attgctctgg tttctgtgcg tgtatactat aaagaatgcc cttctgtggt acgacacttg 720
gctgtcttcc ctgacaccat cactggagct gattcttccc aattgctcga agtgtcaggc 780
tcctgtgtca accattctgt gaccgatgaa cctcccaaaa tgcactgcag cgccgaaggg 840
gagtggctgg tgcccatcgg gaaatgcatg tgcaaggcag gatatgaaga gaaaaatggc 900
acctgtcaag tgtgcagacc tgggttcttc aaagcctcac ctcacatcca gagctgcggc 960
aaatgtccac ctcacagtta tacccatgag gaagcttcaa cctcttgtgt ctgtgaaaag 1020
gattatttca ggagagagtc tgatccaccc acaatggcat gcacaagacc cccctctgct 1080
cctcggaatg ccatctcaaa tgttaatgaa actagtgtct ttctggaatg gattccgcct 1140
gctgacactg gtggaaggaa agacgtgtca tattatattg catgcaagaa gtgcaactcc 1200
catgcaggtg tgtgtgagga gtgtggcggt catgtcaggt accttccccg gcaaagcggc 1260
ctgaaaaaca cctctgtcat gatggtggat ctactcgctc acacaaacta tacctttgag 1320
attgaggcag tgaatggagt gtccgacttg agcccaggag cccggcagta tgtgtctgta 1380
aatgtaacca caaatcaagc agctccatct ccagtcacca atgtgaaaaa agggaaaatt 1440
gcaaaaaaca gcatctcttt gtcttggcaa gaaccagatc gtcccaatgg aatcatccta 1500
gagtatgaaa tcaagtattt tgaaaaggac caagagacca gctacacgat tatcaaatct 1560
aaagagacaa ctattactgc agagggcttg aaaccagctt cagtttatgt cttccaaatt 1620
cgagcacgta cagcagcagg ctatggtgtc ttcagtcgaa gatttgagtt tgaaaccacc 1680

ccagtgtttg cagcatccag cgatcaaagc cagattcctg taattgctgt gtctgtgaca 1740
gtgggagtca ttttgttggc agtggttatc ggcgtcctcc tcagtggaag ttgctgcgaa 1800
tgtggctgtg ggagggcttc ttccctgtgc gctgttgccc atccaagcct aatatggcgg 1860
tgtggctaca gcaaagcaaa acaagatcca gaagaggaaa agatgcattt tcataatggg 1920
cacattaaac tgccaggagt aagaacttac attgatccac atacctatga ggatcccaat 1980
caagctgtcc acgaatttgc taaggagata gaagcatcat gtatcaccat tgagagagtt 2040
attggagcag gtgaatttgg tgaagtttgt agtggacgtt tgaaactacc aggaaaaaga 2100
gaattacctg tggctatcaa aacccttaaa gtaggctata ctgaaaagca acgcagagat 2160
ttcctaggtg aagcaagtat catgggacag tttgatcatc ctaacatcat ccatttagaa 2220
ggtgtggtga ccaaaagtaa accagtgatg atcgtgacag agtatatgga gaatggctct 2280
ttagatacat ttttgaagaa aaacgatggg cagttcactg tgattcagct tgttggcatg 2340
ctgagaggta tctctgcagg aatgaagtac ctttctgaca tgggctatgt gcatagagat 2400
cttgctgcca gaaacatctt aatcaacagt aaccttgtgt gcaaagtgtc tgactttgga 2460
ctttcccggg tactggaaga tgatcccgag gcagcctaca ccacaagggg aggaaaaatt 2520
ccaatcagat ggactgcccc agaagcaata gctttccgaa agtttacttc tgccagtgat 2580
gtctggagtt atggaatagt aatgtgggaa gttgtgtctt atggagagag accctactgg 2640
gagatgacca atcaagatgt gattaaagcg gtagaggaag gctatcgtct gccaagcccc 2700
atggattgtc ctgctgctct ctatcagtta atgctggatt gctggcagaa agagcgaaat 2760
agcaggccca agtttgatga aatagtcaac atgttggaca agctgatacg taacccaagt 2820
agtctgaaga cgctggttaa tgcatcctgc agagtatcta atttattggc agaacatagc 2880
ccactaggat ctggggccta cagatcagta ggtgaatggc tagaggcaat caagatgggc 2940
cggtatacag agattttcat ggaaaatgga tacagttcaa tggacgctgt ggctcaggtg 3000
accttggagg atttgagacg gcttggagtg actcttgtcg gtcaccagaa gaagatcatg 3060
aacagccttc aagaaatgaa ggtgcagctg gtaaacggaa tggtgccatt gtaa 3114

<210> 12
<211> 1037
<212> PRT
<213> Homo sapiens

<220>
<221> BINDING
<222> (60)...(223)
<223> Ephrin receptor ligand binding domain

<220>
<221> CHAIN
<222> (358)...(452)
<223> Fibronectin type 3 domain

<220>
<221> CHAIN
<222> (469)...(559)
<223> Fibronectin type 3 domain

<220>
<221> CHAIN
<222> (675)...(932)
<223> Tyrosine kinase, catalytic domain

<220>
<221> CHAIN
<222> (968)...(1027)
<223> SAM Domain (Sterile alpha motif)

<400> 12
Met Arg Gly Ser Gly Pro Arg Gly Ala Gly His Arg Arg Pro Pro Ser
1               5                   10                  15
Gly Gly Gly Asp Thr Pro Ile Thr Pro Ala Ser Leu Ala Gly Cys Tyr
            20                  25                  30
Ser Ala Pro Arg Arg Ala Pro Leu Trp Thr Cys Leu Leu Leu Cys Ala
        35                  40                  45
Ala Leu Arg Thr Leu Leu Ala Ser Pro Ser Asn Glu Val Asn Leu Leu
    50                  55                  60
Asp Ser Arg Thr Val Met Gly Asp Leu Gly Trp Ile Ala Phe Pro Lys
65                  70                  75                  80
Asn Gly Trp Glu Glu Ile Gly Glu Val Asp Glu Asn Tyr Ala Pro Ile
                85                  90                  95
His Thr Tyr Gln Val Cys Lys Val Met Glu Gln Asn Gln Asn Asn Trp

            100                 105                 110
Leu Leu Thr Ser Trp Ile Ser Asn Glu Gly Ala Ser Arg Ile Phe Ile
        115                 120                 125
Glu Leu Lys Phe Thr Leu Arg Asp Cys Asn Ser Leu Pro Gly Gly Leu
    130                 135                 140
Gly Thr Cys Lys Glu Thr Phe Asn Met Tyr Tyr Phe Glu Ser Asp Asp
145                 150                 155                 160
Gln Asn Gly Arg Asn Ile Lys Glu Asn Gln Tyr Ile Lys Ile Asp Thr
                165                 170                 175
Ile Ala Ala Asp Glu Ser Phe Thr Glu Leu Asp Leu Gly Asp Arg Val
            180                 185                 190
Met Lys Leu Asn Thr Glu Val Arg Asp Val Gly Pro Leu Ser Lys Lys
        195                 200                 205
Gly Phe Tyr Leu Ala Phe Gln Asp Val Gly Ala Cys Ile Ala Leu Val
    210                 215                 220
Ser Val Arg Val Tyr Tyr Lys Glu Cys Pro Ser Val Val Arg His Leu
225                 230                 235                 240
Ala Val Phe Pro Asp Thr Ile Thr Gly Ala Asp Ser Ser Gln Leu Leu
                245                 250                 255
Glu Val Ser Gly Ser Cys Val Asn His Ser Val Thr Asp Glu Pro Pro
            260                 265                 270
Lys Met His Cys Ser Ala Glu Gly Glu Trp Leu Val Pro Ile Gly Lys
        275                 280                 285
Cys Met Cys Lys Ala Gly Tyr Glu Glu Lys Asn Gly Thr Cys Gln Val
    290                 295                 300
Cys Arg Pro Gly Phe Phe Lys Ala Ser Pro His Ile Gln Ser Cys Gly
305                 310                 315                 320
Lys Cys Pro Pro His Ser Tyr Thr His Glu Glu Ala Ser Thr Ser Cys
                325                 330                 335
Val Cys Glu Lys Asp Tyr Phe Arg Arg Glu Ser Asp Pro Pro Thr Met
            340                 345                 350
Ala Cys Thr Arg Pro Pro Ser Ala Pro Arg Asn Ala Ile Ser Asn Val
        355                 360                 365
Asn Glu Thr Ser Val Phe Leu Glu Trp Ile Pro Pro Ala Asp Thr Gly
    370                 375                 380
Gly Arg Lys Asp Val Ser Tyr Tyr Ile Ala Cys Lys Lys Cys Asn Ser
385                 390                 395                 400
His Ala Gly Val Cys Glu Glu Cys Gly Gly His Val Arg Tyr Leu Pro
                405                 410                 415
Arg Gln Ser Gly Leu Lys Asn Thr Ser Val Met Met Val Asp Leu Leu
            420                 425                 430
Ala His Thr Asn Tyr Thr Phe Glu Ile Glu Ala Val Asn Gly Val Ser
        435                 440                 445
Asp Leu Ser Pro Gly Ala Arg Gln Tyr Val Ser Val Asn Val Thr Thr
    450                 455                 460
Asn Gln Ala Ala Pro Ser Pro Val Thr Asn Val Lys Lys Gly Lys Ile
465                 470                 475                 480
Ala Lys Asn Ser Ile Ser Leu Ser Trp Gln Glu Pro Asp Arg Pro Asn
                485                 490                 495
Gly Ile Ile Leu Glu Tyr Glu Ile Lys Tyr Phe Glu Lys Asp Gln Glu
            500                 505                 510
Thr Ser Tyr Thr Ile Ile Lys Ser Lys Glu Thr Thr Ile Thr Ala Glu
        515                 520                 525
Gly Leu Lys Pro Ala Ser Val Tyr Val Phe Gln Ile Arg Ala Arg Thr
    530                 535                 540
Ala Ala Gly Tyr Gly Val Phe Ser Arg Arg Phe Glu Phe Glu Thr Thr
545                 550                 555                 560
Pro Val Phe Ala Ala Ser Ser Asp Gln Ser Gln Ile Pro Val Ile Ala
                565                 570                 575
Val Ser Val Thr Val Gly Val Ile Leu Leu Ala Val Val Ile Gly Val
            580                 585                 590
Leu Leu Ser Gly Ser Cys Cys Glu Cys Gly Cys Gly Arg Ala Ser Ser
        595                 600                 605
Leu Cys Ala Val Ala His Pro Ser Leu Ile Trp Arg Cys Gly Tyr Ser
    610                 615                 620
Lys Ala Lys Gln Asp Pro Glu Glu Glu Lys Met His Phe His Asn Gly
625                 630                 635                 640
His Ile Lys Leu Pro Gly Val Arg Thr Tyr Ile Asp Pro His Thr Tyr
                645                 650                 655

Glu Asp Pro Asn Gln Ala Val His Glu Phe Ala Lys Glu Ile Glu Ala
            660                 665                 670
Ser Cys Ile Thr Ile Glu Arg Val Ile Gly Ala Gly Glu Phe Gly Glu
        675                 680                 685
Val Cys Ser Gly Arg Leu Lys Leu Pro Gly Lys Arg Glu Leu Pro Val
    690                 695                 700
Ala Ile Lys Thr Leu Lys Val Gly Tyr Thr Glu Lys Gln Arg Arg Asp
705                 710                 715                 720
Phe Leu Gly Glu Ala Ser Ile Met Gly Gln Phe Asp His Pro Asn Ile
                725                 730                 735
Ile His Leu Glu Gly Val Val Thr Lys Ser Lys Pro Val Met Ile Val
            740                 745                 750
Thr Glu Tyr Met Glu Asn Gly Ser Leu Asp Thr Phe Leu Lys Lys Asn
        755                 760                 765
Asp Gly Gln Phe Thr Val Ile Gln Leu Val Gly Met Leu Arg Gly Ile
    770                 775                 780
Ser Ala Gly Met Lys Tyr Leu Ser Asp Met Gly Tyr Val His Arg Asp
785                 790                 795                 800
Leu Ala Ala Arg Asn Ile Leu Ile Asn Ser Asn Leu Val Cys Lys Val
                805                 810                 815
Ser Asp Phe Gly Leu Ser Arg Val Leu Glu Asp Asp Pro Glu Ala Ala
            820                 825                 830
Tyr Thr Thr Arg Gly Gly Lys Ile Pro Ile Arg Trp Thr Ala Pro Glu
        835                 840                 845
Ala Ile Ala Phe Arg Lys Phe Thr Ser Ala Ser Asp Val Trp Ser Tyr
    850                 855                 860
Gly Ile Val Met Trp Glu Val Val Ser Tyr Gly Glu Arg Pro Tyr Trp
865                 870                 875                 880
Glu Met Thr Asn Gln Asp Val Ile Lys Ala Val Glu Glu Gly Tyr Arg
                885                 890                 895
Leu Pro Ser Pro Met Asp Cys Pro Ala Ala Leu Tyr Gln Leu Met Leu
            900                 905                 910
Asp Cys Trp Gln Lys Glu Arg Asn Ser Arg Pro Lys Phe Asp Glu Ile
        915                 920                 925
Val Asn Met Leu Asp Lys Leu Ile Arg Asn Pro Ser Ser Leu Lys Thr
    930                 935                 940
Leu Val Asn Ala Ser Cys Arg Val Ser Asn Leu Leu Ala Glu His Ser
945                 950                 955                 960
Pro Leu Gly Ser Gly Ala Tyr Arg Ser Val Gly Glu Trp Leu Glu Ala
                965                 970                 975
Ile Lys Met Gly Arg Tyr Thr Glu Ile Phe Met Glu Asn Gly Tyr Ser
            980                 985                 990
Ser Met Asp Ala Val Ala Gln Val Thr Leu Glu Asp Leu Arg Arg Leu
        995                 1000                1005
Gly Val Thr Leu Val Gly His Gln Lys Lys Ile Met Asn Ser Leu Gln
    1010                1015                1020
Glu Met Lys Val Gln Leu Val Asn Gly Met Val Pro Leu
1025                1030                1035

<210> 13
<211> 3048
<212> DNA
<213> Homo sapiens

<400> 13
atgcggggct cggggccccg gggtgcggga caccggcggc ccccaagcgg cggcggcgac 60
acccccatca ccccagcgtc cctggccggc tgctactctg cacctcgacg ggctcccctc 120
tggacgtgcc ttctcctgtg cgccgcactc cggaccctcc tggccagccc cagcaacgaa 180
gtgaatttat tggattcacg cactgtcatg ggggacctgg gatggattgc ttttccaaaa 240
aatgggtggg aagagattgg tgaagtggat gaaaattatg cccctatcca cacataccaa 300
gtatgcaaag tgatggaaca gaatcagaat aactggcttt tgaccagttg gatctccaat 360
gaaggtgctt ccagaatctt catagaactc aaatttaccc tgcgggactg caacagcctt 420
cctggaggac tggggacctg taaggaaacc tttaatatgt attactttga gtcagatgat 480
cagaatggga gaaacatcaa ggaaaaccaa tacatcaaaa ttgataccat tgctgccgat 540
gaaagcttta cagaacttga tcttggtgac cgtgttatga aactgaatac agaggtcaga 600
gatgtaggac ctctaagcaa aaagggattt tatcttgctt ttcaagatgt tggtgcttgc 660
attgctctgg tttctgtgcg tgtatactat aaagaatgcc cttctgtggt acgacacttg 720
gctgtcttcc ctgacaccat cactggagct gattcttccc aattgctcga agtgtcaggc 780

tcctgtgtca accattctgt gaccgatgaa cctcccaaaa tgcactgcag cgccgaaggg 840
gagtggctgg tgcccatcgg gaaatgcatg tgcaaggcag gatatgaaga gaaaaatggc 900
acctgtcaag tgtgcagacc tgggttcttc aaagcctcac ctcacatcca gagctgcggc 960
aaatgtccac ctcacagtta tacccatgag gaagcttcaa cctcttgtgt ctgtgaaaag 1020
gattatttca ggagagagtc tgatccaccc acaatggcat gcacaagacc cccctctgct 1080
cctcggaatg ccatctcaaa tgttaatgaa actagtgtct ttctggaatg gattccgcct 1140
gctgacactg gtggaaggaa agacgtgtca tattatattg catgcaagaa gtgcaactcc 1200
catgcaggtg tgtgtgagga gtgtggcggt catgtcaggt accttccccg gcaaagcggc 1260
ctgaaaaaca cctctgtcat gatggtggat ctactcgctc acacaaacta tacctttgag 1320
attgaggcag tgaatggagt gtccgacttg agcccaggag cccggcagta tgtgtctgta 1380
aatgtaacca caaatcaagc agctccatct ccagtcacca atgtgaaaaa agggaaaatt 1440
gcaaaaaaca gcatctcttt gtcttggcaa gaaccagatc gtcccaatgg aatcatccta 1500
gagtatgaaa tcaagtattt tgaaaaggac caagagacca gctacacgat tatcaaatct 1560
aaagagacaa ctattactgc agagggcttg aaaccagctt cagtttatgt cttccaaatt 1620
cgagcacgta cagcagcagg ctatggtgtc ttcagtcgaa gatttgagtt tgaaaccacc 1680
ccagtgtttg cagcatccag cgatcaaagc cagattcctg taattgctgt gtctgtgaca 1740
gtgggagtca ttttgttggc agtggttatc ggcgtcctcc tcagtggaag gcggtgtggc 1800
tacagcaaag caaaacaaga tccagaagag gaaaagatgc attttcataa tgggcacatt 1860
aaactgccag gagtaagaac ttacattgat ccacatacct atgaggatcc caatcaagct 1920
gtccacgaat ttgctaagga gatagaagca tcatgtatca ccattgagag agttattgga 1980
gcaggtgaat ttggtgaagt ttgtagtgga cgtttgaaac taccaggaaa aagagaatta 2040
cctgtggcta tcaaaaccct taaagtaggc tatactgaaa agcaacgcag agatttccta 2100
ggtgaagcaa gtatcatggg acagtttgat catcctaaca tcatccattt agaaggtgtg 2160
gtgaccaaaa gtaaaccagt gatgatcgtg acagagtata tggagaatgg ctctttagat 2220
acatttttga agaaaaacga tgggcagttc actgtgattc agcttgttgg catgctgaga 2280
ggtatctctg caggaatgaa gtacctttct gacatgggct atgtgcatag agatcttgct 2340
gccagaaaca tcttaatcaa cagtaacctt gtgtgcaaag tgtctgactt tggactttcc 2400
cgggtactgg aagatgatcc cgaggcagcc tacaccacaa ggggaggaaa aattccaatc 2460
agatggactg ccccagaagc aatagctttc cgaaagttta cttctgccag tgatgtctgg 2520
agttatggaa tagtaatgtg ggaagttgtg tcttatggag agagacccta ctgggagatg 2580
accaatcaag atgtgattaa agcggtagag gaaggctatc gtctgccaag ccccatggat 2640
tgtcctgctg ctctctatca gttaatgctg gattgctggc agaaagagcg aaatagcagg 2700
cccaagtttg atgaaatagt caacatgttg gacaagctga tacgtaaccc aagtagtctg 2760
aagacgctgg ttaatgcatc ctgcagagta tctaatttat tggcagaaca tagcccacta 2820
ggatctgggg cctacagatc agtaggtgaa tggctagagg caatcaagat gggccggtat 2880
acagagattt tcatggaaaa tggatacagt tcaatggacg ctgtggctca ggtgaccttg 2940
gaggatttga gacggcttgg agtgactctt gtcggtcacc agaagaagat catgaacagc 3000
cttcaagaaa tgaaggtgca gctggtaaac ggaatggtgc cattgtaa 3048

<210> 14
<211> 1015
<212> PRT
<213> Homo sapiens

<220>
<221> BINDING
<222> (60)...(233)
<223> Ephrin receptor ligand binding domain

<220>
<221> CHAIN
<222> (358)...(452)
<223> Fibronectin type 3 domain

<220>
<221> CHAIN
<222> (469)...(559)
<223> Fibronectin type 3 domain

<220>
<221> CHAIN
<222> (653)...(910)
<223> Tyrosine kinase, catalytic domain

<220>
<221> CHAIN
<222> (646)...(1005)
<223> SAM Domain (Sterile alpha motif)

<400> 14
Met Arg Gly Ser Gly Pro Arg Gly Ala Gly His Arg Arg Pro Pro Ser
1               5                   10                  15
Gly Gly Gly Asp Thr Pro Ile Thr Pro Ala Ser Leu Ala Gly Cys Tyr
            20                  25                  30
Ser Ala Pro Arg Arg Ala Pro Leu Trp Thr Cys Leu Leu Leu Cys Ala
        35                  40                  45
Ala Leu Arg Thr Leu Leu Ala Ser Pro Ser Asn Glu Val Asn Leu Leu
    50                  55                  60
Asp Ser Arg Thr Val Met Gly Asp Leu Gly Trp Ile Ala Phe Pro Lys
65                  70                  75                  80
Asn Gly Trp Glu Glu Ile Gly Glu Val Asp Glu Asn Tyr Ala Pro Ile
                85                  90                  95
His Thr Tyr Gln Val Cys Lys Val Met Glu Gln Asn Gln Asn Asn Trp
            100                 105                 110
Leu Leu Thr Ser Trp Ile Ser Asn Glu Gly Ala Ser Arg Ile Phe Ile
        115                 120                 125
Glu Leu Lys Phe Thr Leu Arg Asp Cys Asn Ser Leu Pro Gly Gly Leu
    130                 135                 140
Gly Thr Cys Lys Glu Thr Phe Asn Met Tyr Tyr Phe Glu Ser Asp Asp
145                 150                 155                 160
Gln Asn Gly Arg Asn Ile Lys Glu Asn Gln Tyr Ile Lys Ile Asp Thr
                165                 170                 175
Ile Ala Ala Asp Glu Ser Phe Thr Glu Leu Asp Leu Gly Asp Arg Val
            180                 185                 190
Met Lys Leu Asn Thr Glu Val Arg Asp Val Gly Pro Leu Ser Lys Lys
        195                 200                 205
Gly Phe Tyr Leu Ala Phe Gln Asp Val Gly Ala Cys Ile Ala Leu Val
    210                 215                 220
Ser Val Arg Val Tyr Tyr Lys Glu Cys Pro Ser Val Val Arg His Leu
225                 230                 235                 240
Ala Val Phe Pro Asp Thr Ile Thr Gly Ala Asp Ser Ser Gln Leu Leu
                245                 250                 255
Glu Val Ser Gly Ser Cys Val Asn His Ser Val Thr Asp Glu Pro Pro
            260                 265                 270
Lys Met His Cys Ser Ala Glu Gly Glu Trp Leu Val Pro Ile Gly Lys
        275                 280                 285
Cys Met Cys Lys Ala Gly Tyr Glu Glu Lys Asn Gly Thr Cys Gln Val
    290                 295                 300
Cys Arg Pro Gly Phe Phe Lys Ala Ser Pro His Ile Gln Ser Cys Gly
305                 310                 315                 320
Lys Cys Pro Pro His Ser Tyr Thr His Glu Glu Ala Ser Thr Ser Cys
                325                 330                 335
Val Cys Glu Lys Asp Tyr Phe Arg Arg Glu Ser Asp Pro Pro Thr Met
            340                 345                 350
Ala Cys Thr Arg Pro Pro Ser Ala Pro Arg Asn Ala Ile Ser Asn Val
        355                 360                 365
Asn Glu Thr Ser Val Phe Leu Glu Trp Ile Pro Pro Ala Asp Thr Gly
    370                 375                 380
Gly Arg Lys Asp Val Ser Tyr Tyr Ile Ala Cys Lys Lys Cys Asn Ser
385                 390                 395                 400
His Ala Gly Val Cys Glu Glu Cys Gly Gly His Val Arg Tyr Leu Pro
                405                 410                 415
Arg Gln Ser Gly Leu Lys Asn Thr Ser Val Met Met Val Asp Leu Leu
            420                 425                 430
Ala His Thr Asn Tyr Thr Phe Glu Ile Glu Ala Val Asn Gly Val Ser
        435                 440                 445
Asp Leu Ser Pro Gly Ala Arg Gln Tyr Val Ser Val Asn Val Thr Thr
    450                 455                 460
Asn Gln Ala Ala Pro Ser Pro Val Thr Asn Val Lys Lys Gly Lys Ile
465                 470                 475                 480
Ala Lys Asn Ser Ile Ser Leu Ser Trp Gln Glu Pro Asp Arg Pro Asn
                485                 490                 495
Gly Ile Ile Leu Glu Tyr Glu Ile Lys Tyr Phe Glu Lys Asp Gln Glu
            500                 505                 510
Thr Ser Tyr Thr Ile Ile Lys Ser Lys Glu Thr Thr Ile Thr Ala Glu
        515                 520                 525
Gly Leu Lys Pro Ala Ser Val Tyr Val Phe Gln Ile Arg Ala Arg Thr
    530                 535                 540

Ala Ala Gly Tyr Gly Val Phe Ser Arg Arg Phe Glu Phe Glu Thr Thr
545                 550                 555                 560
Pro Val Phe Ala Ala Ser Ser Asp Gln Ser Gln Ile Pro Val Ile Ala
                565                 570                 575
Val Ser Val Thr Val Gly Val Ile Leu Leu Ala Val Val Ile Gly Val
            580                 585                 590
Leu Leu Ser Gly Arg Arg Cys Gly Tyr Ser Lys Ala Lys Gln Asp Pro
        595                 600                 605
Glu Glu Glu Lys Met His Phe His Asn Gly His Ile Lys Leu Pro Gly
    610                 615                 620
Val Arg Thr Tyr Ile Asp Pro His Thr Tyr Glu Asp Pro Asn Gln Ala
625                 630                 635                 640
Val His Glu Phe Ala Lys Glu Ile Glu Ala Ser Cys Ile Thr Ile Glu
                645                 650                 655
Arg Val Ile Gly Ala Gly Glu Phe Gly Glu Val Cys Ser Gly Arg Leu
            660                 665                 670
Lys Leu Pro Gly Lys Arg Glu Leu Pro Val Ala Ile Lys Thr Leu Lys
        675                 680                 685
Val Gly Tyr Thr Glu Lys Gln Arg Arg Asp Phe Leu Gly Glu Ala Ser
    690                 695                 700
Ile Met Gly Gln Phe Asp His Pro Asn Ile Ile His Leu Glu Gly Val
705                 710                 715                 720
Val Thr Lys Ser Lys Pro Val Met Ile Val Thr Glu Tyr Met Glu Asn
                725                 730                 735
Gly Ser Leu Asp Thr Phe Leu Lys Lys Asn Asp Gly Gln Phe Thr Val
            740                 745                 750
Ile Gln Leu Val Gly Met Leu Arg Gly Ile Ser Ala Gly Met Lys Tyr
        755                 760                 765
Leu Ser Asp Met Gly Tyr Val His Arg Asp Leu Ala Ala Arg Asn Ile
    770                 775                 780
Leu Ile Asn Ser Asn Leu Val Cys Lys Val Ser Asp Phe Gly Leu Ser
785                 790                 795                 800
Arg Val Leu Glu Asp Asp Pro Glu Ala Ala Tyr Thr Thr Arg Gly Gly
                805                 810                 815
Lys Ile Pro Ile Arg Trp Thr Ala Pro Glu Ala Ile Ala Phe Arg Lys
            820                 825                 830
Phe Thr Ser Ala Ser Asp Val Trp Ser Tyr Gly Ile Val Met Trp Glu
        835                 840                 845
Val Val Ser Tyr Gly Glu Arg Pro Tyr Trp Glu Met Thr Asn Gln Asp
    850                 855                 860
Val Ile Lys Ala Val Glu Glu Gly Tyr Arg Leu Pro Ser Pro Met Asp
865                 870                 875                 880
Cys Pro Ala Ala Leu Tyr Gln Leu Met Leu Asp Cys Trp Gln Lys Glu
                885                 890                 895
Arg Asn Ser Arg Pro Lys Phe Asp Glu Ile Val Asn Met Leu Asp Lys
            900                 905                 910
Leu Ile Arg Asn Pro Ser Ser Leu Lys Thr Leu Val Asn Ala Ser Cys
        915                 920                 925
Arg Val Ser Asn Leu Leu Ala Glu His Ser Pro Leu Gly Ser Gly Ala
    930                 935                 940
Tyr Arg Ser Val Gly Glu Trp Leu Glu Ala Ile Lys Met Gly Arg Tyr
945                 950                 955                 960
Thr Glu Ile Phe Met Glu Asn Gly Tyr Ser Ser Met Asp Ala Val Ala
                965                 970                 975
Gln Val Thr Leu Glu Asp Leu Arg Arg Leu Gly Val Thr Leu Val Gly
            980                 985                 990
His Gln Lys Lys Ile Met Asn Ser Leu Gln Glu Met Lys Val Gln Leu
        995                 1000                1005
Val Asn Gly Met Val Pro Leu
    1010                1015

<210> 15
<211> 2139
<212> DNA
<213> Homo sapiens

<400> 15
atggccgcga cagaggagcg gagcctgcac aacttctttg ccaatcggga caagaagaag 60

aaggagcaga gcaaccgggc ggcgagttcc gcgggcgcag caggcagcgc ggcgggagca 120
gtggagctcc gcattctaga gggcggcggg gcgggcgcag ggacctggct gggcgaaggc 180
aggaccgcga gtgcggaggc tgcgggccca ggggccacca ccaaggctgt gaagaatgga 240
aaggcttgga gtaaaaagag ccgcgaaggc ggctactgcg ctgagccgct cgctctgctg 300
gtcaagtttg ggcgacccgc gcggaggagg gtcgggctga ctgccgccgc tgagctgtcc 360
ccggacggga gcgcctgtcc acggcactca ccccctccag cggtggaaat gtggagaacc 420
cgagctcgct cttgcgcgcg cgcgctctct ccggcccaag tgaatagtcc tcgcgcaagc 480
gggacactgt ggtggatgca attcccctcg cctccagccg cgaggagctc cccggcgccg 540
caggcagcgt cctcctccga agcagctgca cctgcaactg ggcagcctgg accctcgtgc 600
cctgttcccg ggacctcgcg cagggggcgc cccgggacac cccctgcggg ccgggtggag 660
gaggaagagg aggaggagga agaagacgtg gacaaggacc cccatcctac ccagaacacc 720
tgcctgcgct gccgccactt ctctttaagg gagaggaaaa gagagcctag gagaaccatg 780
gggggctgcg aagtccggga atttcttttg caatttggtt tcttcttgcc tctgctgaca 840
gcgtggccag gcgactgcag tcacgtctcc aacaaccaag ttgtgttgct tgatacaaca 900
actgtactgg gagagctagg atggaaaaca tatccattaa atgggtggga tgccatcact 960
gaaatggatg aacataatag gcccattcac acataccagg tatgtaatgt aatggaacca 1020
aaccaaaaca actggcttcg tacaaactgg atctcccgtg atgcagctca gaaaatttat 1080
gtggaaatga aattcacact aagggattgt aacagcatcc catgggtctt ggggacttgc 1140
aaagaaacat ttaatctgtt ttatatggaa tcagatgagt cccacggaat taaattcaag 1200
ccaaaccagt atacaaagat cgacacaatt gctgctgatg agagttttac ccagatggat 1260
ttgggtgatc gcatcctcaa actcaacact gaaattcgtg aggtggggcc tatagaaagg 1320
aaaggatttt atctggcttt tcaagacatt ggggcgtgca ttgccctggt ttcagtccgt 1380
gttttctaca agaaatgccc cttcactgtt cgtaacttgg ccatgtttcc tgataccatt 1440
ccaagggttg attcctcctc tttggttgaa gtacggggtt cttgtgtgaa gagtgctgaa 1500
gagcgtgaca ctcctaaact gtattgtgga gctgatggag attggctggt tcctcttgga 1560
aggtgcatct gcagtacagg atatgaagaa attgagggtt cttgccatgc ttgcagacca 1620
ggattctata aagcttttgc tgggaacaca aaatgttcta aatgtcctcc acacagttta 1680
acatacatgg aagcaacttc tgtctgtcag tgtgaaaagg gttatttccg agctgaaaaa 1740
gacccacctt ctatggcatg taccaggcca ccttcagctc ctaggaatgt ggtttttaac 1800
atcaatgaaa cagcccttat tttggaatgg agcccaccaa gtgacacagg agggagaaaa 1860
gatctcacat acagtgtaat ctgtaagaaa tgtggcttag acaccagcca gtgtgaggac 1920
tgtggtggag gactccgctt catcccaaga catacaggcc tgatcaacaa ttccgtgata 1980
gtacttgact ttgtgtctca cgtgaattac acctttgaaa tagaagcaat gaatggagtt 2040
tctgagttga gtttttctcc caagccattc acagctatta cagtgaccac ggatcaagat 2100
ggtaagttcc actgctgttc tctcaaaaca gacccataa 2139

<210> 16
<211> 712
<212> PRT
<213> Homo sapiens

<220>
<221> BINDING
<222> (293)...(466)
<223> Ephrin receptor ligand binding domain

<220>
<221> CHAIN
<222> (591)...(691)
<223> Fibronectin type 3 domain

<220>
<221> CHAIN
<222> (591)...(682)
<223> Fibronectin type 3 domain

<220>
<221> CHAIN
<222> (594)...(686)
<223> Tyrosine kinase, catalytic domain

<400> 16
Met Ala Ala Thr Glu Glu Arg Ser Leu His Asn Phe Phe Ala Asn Arg
1               5                   10                  15
Asp Lys Lys Lys Lys Glu Gln Ser Asn Arg Ala Ala Ser Ser Ala Gly
            20                  25                  30
Ala Ala Gly Ser Ala Ala Gly Ala Val Glu Leu Arg Ile Leu Glu Gly
        35                  40                  45
Gly Gly Ala Gly Ala Gly Thr Trp Leu Gly Glu Gly Arg Thr Ala Ser

50                  55                  60
Ala Glu Ala Ala Gly Pro Gly Ala Thr Thr Lys Ala Val Lys Asn Gly
65                  70                  75                  80
Lys Ala Trp Ser Lys Lys Ser Arg Glu Gly Gly Tyr Cys Ala Glu Pro
                85                  90                  95
Leu Ala Leu Leu Val Lys Phe Gly Arg Pro Ala Arg Arg Arg Val Gly
            100                 105                 110
Leu Thr Ala Ala Ala Glu Leu Ser Pro Asp Gly Ser Ala Cys Pro Arg
        115                 120                 125
His Ser Pro Pro Pro Ala Val Glu Met Trp Arg Thr Arg Ala Arg Ser
    130                 135                 140
Cys Ala Arg Ala Leu Ser Pro Ala Gln Val Asn Ser Pro Arg Ala Ser
145                 150                 155                 160
Gly Thr Leu Trp Trp Met Gln Phe Pro Ser Pro Pro Ala Ala Arg Ser
                165                 170                 175
Ser Pro Ala Pro Gln Ala Ala Ser Ser Ser Glu Ala Ala Ala Pro Ala
            180                 185                 190
Thr Gly Gln Pro Gly Pro Ser Cys Pro Val Pro Gly Thr Ser Arg Arg
        195                 200                 205
Gly Arg Pro Gly Thr Pro Pro Ala Gly Arg Val Glu Glu Glu Glu Glu
    210                 215                 220
Glu Glu Glu Glu Asp Val Asp Lys Asp Pro His Pro Thr Gln Asn Thr
225                 230                 235                 240
Cys Leu Arg Cys Arg His Phe Ser Leu Arg Glu Arg Lys Arg Glu Pro
                245                 250                 255
Arg Arg Thr Met Gly Gly Cys Glu Val Arg Glu Phe Leu Leu Gln Phe
            260                 265                 270
Gly Phe Phe Leu Pro Leu Leu Thr Ala Trp Pro Gly Asp Cys Ser His
        275                 280                 285
Val Ser Asn Asn Gln Val Val Leu Leu Asp Thr Thr Thr Val Leu Gly
    290                 295                 300
Glu Leu Gly Trp Lys Thr Tyr Pro Leu Asn Gly Trp Asp Ala Ile Thr
305                 310                 315                 320
Glu Met Asp Glu His Asn Arg Pro Ile His Thr Tyr Gln Val Cys Asn
                325                 330                 335
Val Met Glu Pro Asn Gln Asn Asn Trp Leu Arg Thr Asn Trp Ile Ser
            340                 345                 350
Arg Asp Ala Ala Gln Lys Ile Tyr Val Glu Met Lys Phe Thr Leu Arg
        355                 360                 365
Asp Cys Asn Ser Ile Pro Trp Val Leu Gly Thr Cys Lys Glu Thr Phe
    370                 375                 380
Asn Leu Phe Tyr Met Glu Ser Asp Glu Ser His Gly Ile Lys Phe Lys
385                 390                 395                 400
Pro Asn Gln Tyr Thr Lys Ile Asp Thr Ile Ala Ala Asp Glu Ser Phe
                405                 410                 415
Thr Gln Met Asp Leu Gly Asp Arg Ile Leu Lys Leu Asn Thr Glu Ile
            420                 425                 430
Arg Glu Val Gly Pro Ile Glu Arg Lys Gly Phe Tyr Leu Ala Phe Gln
        435                 440                 445
Asp Ile Gly Ala Cys Ile Ala Leu Val Ser Val Arg Val Phe Tyr Lys
    450                 455                 460
Lys Cys Pro Phe Thr Val Arg Asn Leu Ala Met Phe Pro Asp Thr Ile
465                 470                 475                 480
Pro Arg Val Asp Ser Ser Ser Leu Val Glu Val Arg Gly Ser Cys Val
                485                 490                 495
Lys Ser Ala Glu Glu Arg Asp Thr Pro Lys Leu Tyr Cys Gly Ala Asp
            500                 505                 510
Gly Asp Trp Leu Val Pro Leu Gly Arg Cys Ile Cys Ser Thr Gly Tyr
        515                 520                 525
Glu Glu Ile Glu Gly Ser Cys His Ala Cys Arg Pro Gly Phe Tyr Lys
    530                 535                 540
Ala Phe Ala Gly Asn Thr Lys Cys Ser Lys Cys Pro Pro His Ser Leu
545                 550                 555                 560
Thr Tyr Met Glu Ala Thr Ser Val Cys Gln Cys Glu Lys Gly Tyr Phe
                565                 570                 575
Arg Ala Glu Lys Asp Pro Pro Ser Met Ala Cys Thr Arg Pro Pro Ser
            580                 585                 590
Ala Pro Arg Asn Val Val Phe Asn Ile Asn Glu Thr Ala Leu Ile Leu
        595                 600                 605

Glu Trp Ser Pro Pro Ser Asp Thr Gly Gly Arg Lys Asp Leu Thr Tyr
    610                 615                 620
Ser Val Ile Cys Lys Lys Cys Gly Leu Asp Thr Ser Gln Cys Glu Asp
625                 630                 635                 640
Cys Gly Gly Gly Leu Arg Phe Ile Pro Arg His Thr Gly Leu Ile Asn
                645                 650                 655
Asn Ser Val Ile Val Leu Asp Phe Val Ser His Val Asn Tyr Thr Phe
            660                 665                 670
Glu Ile Glu Ala Met Asn Gly Val Ser Glu Leu Ser Phe Ser Pro Lys
        675                 680                 685
Pro Phe Thr Ala Ile Thr Val Thr Thr Asp Gln Asp Gly Lys Phe His
    690                 695                 700
Cys Cys Ser Leu Lys Thr Asp Pro
705                 710

<210> 17
<211> 2997
<212> DNA
<213> Homo sapiens

<400> 17
atggtttttc aaactcggta cccttcatgg attattttat gctacatctg gctgctccgc 60
tttgcacaca caggggaggc gcaggctgcg aaggaagtac tactgctgga ttctaaagca 120
caacaaacag agttggagtg gatttcctct ccacccaatg ggtgggaaga aattagtggt 180
ttggatgaga actatacccc gatacgaaca taccaggtgt gccaagtcat ggagcccaac 240
caaaacaact ggctgcggac taactggatt tccaaaggca atgcacaaag gatttttgta 300
gaattgaaat tcaccctgag ggattgtaac agtcttcctg gagtactggg aacttgcaag 360
gaaacattta atttgtacta ttatgaaaca gactatgaca ctggcaggaa tataagagaa 420
aacctctatg taaaaataga caccattgct gcagatgaaa gttttaccca aggtgacctt 480
ggtgaaagaa agatgaagct taacactgag gtgagagaga ttggaccttt gtccaaaaag 540
ggattctatc ttgcctttca ggatgtaggg gcttgcatag ctttggtttc tgtcaaagtg 600
tactacaaga agtgctggtc cattattgag aacttagcta tctttccaga tacagtgact 660
ggttcagaat tttcctcttt agtcgaggtt cgagggacat gtgtcagcag tgcagaggaa 720
gaagcggaaa acgcccccag gatgcactgc agtgcagaag gagaatggtt agtgcccatt 780
ggaaaatgta tctgcaaagc aggctaccag caaaaaggag acacttgtga accctgtggc 840
cgtgggttct acaagtcttc ctctcaagat cttcagtgct ctcgttgtcc aactcacagt 900
ttttctgata aagaaggctc ctccagatgt gaatgtgaag atgggtatta cagggctcca 960
tctgacccac catacgttgc atgcacaagg cctccatctg caccacagaa cctcattttc 1020
aacatcaacc aaaccacagt aagtttggaa tggagtcctc ctgcagacaa tgggggaaga 1080
aacgatgtga cctacagaat attgtgtaag cggtgcagtt gggagcaggg cgaatgtgtt 1140
ccctgtggga gtaacattgg atacatgccc cagcagactg gattagagga taactatgtc 1200
actgtcatgg acctgctagc ccacgctaat tatacttttg aagttgaagc tgtaaatgga 1260
gtttctgact taagccgatc ccagaggctc tttgctgctg tcagtatcac cactggtcaa 1320
gcagctccct cgcaagtgag cggagtaatg aaggagagag tactgcagcg gagtgtcgag 1380
ctttcctggc aggaaccaga gcatcccaat ggagtcatca cagaatatga aatcaagtat 1440
tacgagaaag atcaaaggga acggacctac tcaacagtaa aaaccaagtc tacttcagcc 1500
tccattaata atctgaaacc aggaacagtg tatgttttcc agattcgggc ttttactgct 1560
gctggttatg gaaattacag tcccagactt gatgttgcta cactagagga agctacaggt 1620
aaaatgtttg aagctacagc tgtctccagt gaacagaatc ctgttattat cattgctgtg 1680
gttgctgtag ctgggaccat cattttggtg ttcatggtct ttggcttcat cattgggaga 1740
aggcactgtg gttatagcaa agctgaccaa gaaggcgatg aagagcttta ctttcatttt 1800
aaatttccag gcaccaaaac ctacattgac cctgaaacct atgaggaccc aaatagagct 1860
gtccatcaat tcgccaagga gctagatgcc tcctgtatta aaattgagcg tgtgattggt 1920
gcaggagaat tcggtgaagt ctgcagtggc cgtttgaaac ttccagggaa aagagatgtt 1980
gcagtagcca taaaaaccct gaaagttggt tacacagaaa aacaaaggag agactttttg 2040
tgtgaagcaa gcatcatggg gcagtttgac cacccaaatg ttgtccattt ggaaggggtt 2100
gttacaagag ggaaaccagt catgatagta atagagttca tggaaaatgg agccctagat 2160
gcatttctca ggaaacatga tgggcaattt acagtcattc agttagtagg aatgctgaga 2220
ggaattgctg ctggaatgag atatttggct gatatgggat atgttcacag ggaccttgca 2280
gctcgcaata ttcttgtcaa cagcaatctc gtttgtaaag tgtcagattt tggcctgtcc 2340
cgagttatag aggatgatcc agaagctgtc tatacaacta ctggtggaaa aattccagta 2400
aggtggacag cacccgaagc catccagtac cggaaattca catcagccag tgatgtatgg 2460
agctatggaa tagtcatgtg ggaagttatg tcttatggag aaagacctta ttgggacatg 2520
tcaaatcaag atgttataaa agcaatagaa gaaggttatc gtttaccagc acccatggac 2580
tgcccagctg gccttcacca gctaatgttg gattgttggc aaaaggagcg tgctgaaagg 2640
ccaaaatttg aacagatagt tggaattcta gacaaaatga ttcgaaaccc aaatagtctg 2700
aaaactcccc tgggaacttg tagtaggcca ataagccctc ttctggatca aaacactcct 2760
gatttcacta ccttttgttc agttggagaa tggctacaag ctattaagat ggaaagatat 2820

aaagataatt tcacggcagc tggctacaat tcccttgaat cagtagccag gatgactatt 2880
gaggatgtga tgagtttagg gatcacactg gttggtcatc aaaagaaaat catgagcagc 2940
attcagacta tgagagcaca aatgctacat ttacatggaa ctggcattca agtgtga    2997

<210> 18
<211> 998
<212> PRT
<213> Homo sapiens

<220>
<221> BINDING
<222> (32)...(205)
<223> Ephrin receptor ligand binding domain

<220>
<221> CHAIN
<222> (275)...(324)
<223> Tumor necrosis factor receptor (TNFR) domain

<220>
<221> CHAIN
<222> (332)...(427)
<223> Fibronectin type 3 domain

<220>
<221> CHAIN
<222> (443)...(534)
<223> Fibronectin type 3 domain

<220>
<221> CHAIN
<222> (627)...(893)
<223> Tyrosine kinase, catalytic domain

<220>
<221> CHAIN
<222> (924)...(985)
<223> SAM Domain (Sterile alpha motif)

<400> 18
Met Val Phe Gln Thr Arg Tyr Pro Ser Trp Ile Ile Leu Cys Tyr Ile
 1               5                  10                  15
Trp Leu Leu Arg Phe Ala His Thr Gly Glu Ala Gln Ala Ala Lys Glu
            20                  25                  30
Val Leu Leu Leu Asp Ser Lys Ala Gln Gln Thr Glu Leu Glu Trp Ile
        35                  40                  45
Ser Ser Pro Pro Asn Gly Trp Glu Glu Ile Ser Gly Leu Asp Glu Asn
    50                  55                  60
Tyr Thr Pro Ile Arg Thr Tyr Gln Val Cys Gln Val Met Glu Pro Asn
65                  70                  75                  80
Gln Asn Asn Trp Leu Arg Thr Asn Trp Ile Ser Lys Gly Asn Ala Gln
                85                  90                  95
Arg Ile Phe Val Glu Leu Lys Phe Thr Leu Arg Asp Cys Asn Ser Leu
            100                 105                 110
Pro Gly Val Leu Gly Thr Cys Lys Glu Thr Phe Asn Leu Tyr Tyr Tyr
        115                 120                 125
Glu Thr Asp Tyr Asp Thr Gly Arg Asn Ile Arg Glu Asn Leu Tyr Val
    130                 135                 140
Lys Ile Asp Thr Ile Ala Ala Asp Glu Ser Phe Thr Gln Gly Asp Leu
145                 150                 155                 160
Gly Glu Arg Lys Met Lys Leu Asn Thr Glu Val Arg Glu Ile Gly Pro
                165                 170                 175
Leu Ser Lys Lys Gly Phe Tyr Leu Ala Phe Gln Asp Val Gly Ala Cys
            180                 185                 190
Ile Ala Leu Val Ser Val Lys Val Tyr Tyr Lys Lys Cys Trp Ser Ile
        195                 200                 205
Ile Glu Asn Leu Ala Ile Phe Pro Asp Thr Val Thr Gly Ser Glu Phe
    210                 215                 220
Ser Ser Leu Val Glu Val Arg Gly Thr Cys Val Ser Ser Ala Glu Glu

225                 230                 235                 240
Glu Ala Glu Asn Ala Pro Arg Met His Cys Ser Ala Glu Gly Glu Trp
                245                 250                 255
Leu Val Pro Ile Gly Lys Cys Ile Cys Lys Ala Gly Tyr Gln Gln Lys
            260                 265                 270
Gly Asp Thr Cys Glu Pro Cys Gly Arg Gly Phe Tyr Lys Ser Ser Ser
        275                 280                 285
Gln Asp Leu Gln Cys Ser Arg Cys Pro Thr His Ser Phe Ser Asp Lys
    290                 295                 300
Glu Gly Ser Ser Arg Cys Glu Cys Glu Asp Gly Tyr Tyr Arg Ala Pro
305                 310                 315                 320
Ser Asp Pro Pro Tyr Val Ala Cys Thr Arg Pro Pro Ser Ala Pro Gln
                325                 330                 335
Asn Leu Ile Phe Asn Ile Asn Gln Thr Thr Val Ser Leu Glu Trp Ser
            340                 345                 350
Pro Pro Ala Asp Asn Gly Gly Arg Asn Asp Val Thr Tyr Arg Ile Leu
        355                 360                 365
Cys Lys Arg Cys Ser Trp Glu Gln Gly Glu Cys Val Pro Cys Gly Ser
    370                 375                 380
Asn Ile Gly Tyr Met Pro Gln Gln Thr Gly Leu Glu Asp Asn Tyr Val
385                 390                 395                 400
Thr Val Met Asp Leu Leu Ala His Ala Asn Tyr Thr Phe Glu Val Glu
                405                 410                 415
Ala Val Asn Gly Val Ser Asp Leu Ser Arg Ser Gln Arg Leu Phe Ala
            420                 425                 430
Ala Val Ser Ile Thr Thr Gly Gln Ala Ala Pro Ser Gln Val Ser Gly
        435                 440                 445
Val Met Lys Glu Arg Val Leu Gln Arg Ser Val Glu Leu Ser Trp Gln
    450                 455                 460
Glu Pro Glu His Pro Asn Gly Val Ile Thr Glu Tyr Glu Ile Lys Tyr
465                 470                 475                 480
Tyr Glu Lys Asp Gln Arg Glu Arg Thr Tyr Ser Thr Val Lys Thr Lys
                485                 490                 495
Ser Thr Ser Ala Ser Ile Asn Asn Leu Lys Pro Gly Thr Val Tyr Val
            500                 505                 510
Phe Gln Ile Arg Ala Phe Thr Ala Ala Gly Tyr Gly Asn Tyr Ser Pro
        515                 520                 525
Arg Leu Asp Val Ala Thr Leu Glu Glu Ala Thr Gly Lys Met Phe Glu
    530                 535                 540
Ala Thr Ala Val Ser Ser Glu Gln Asn Pro Val Ile Ile Ile Ala Val
545                 550                 555                 560
Val Ala Val Ala Gly Thr Ile Ile Leu Val Phe Met Val Phe Gly Phe
                565                 570                 575
Ile Ile Gly Arg Arg His Cys Gly Tyr Ser Lys Ala Asp Gln Glu Gly
            580                 585                 590
Asp Glu Glu Leu Tyr Phe His Phe Lys Phe Pro Gly Thr Lys Thr Tyr
        595                 600                 605
Ile Asp Pro Glu Thr Tyr Glu Asp Pro Asn Arg Ala Val His Gln Phe
    610                 615                 620
Ala Lys Glu Leu Asp Ala Ser Cys Ile Lys Ile Glu Arg Val Ile Gly
625                 630                 635                 640
Ala Gly Glu Phe Gly Glu Val Cys Ser Gly Arg Leu Lys Leu Pro Gly
                645                 650                 655
Lys Arg Asp Val Ala Val Ala Ile Lys Thr Leu Lys Val Gly Tyr Thr
            660                 665                 670
Glu Lys Gln Arg Arg Asp Phe Leu Cys Glu Ala Ser Ile Met Gly Gln
        675                 680                 685
Phe Asp His Pro Asn Val Val His Leu Glu Gly Val Val Thr Arg Gly
    690                 695                 700
Lys Pro Val Met Ile Val Ile Glu Phe Met Glu Asn Gly Ala Leu Asp
705                 710                 715                 720
Ala Phe Leu Arg Lys His Asp Gly Gln Phe Thr Val Ile Gln Leu Val
                725                 730                 735
Gly Met Leu Arg Gly Ile Ala Ala Gly Met Arg Tyr Leu Ala Asp Met
            740                 745                 750
Gly Tyr Val His Arg Asp Leu Ala Ala Arg Asn Ile Leu Val Asn Ser
        755                 760                 765
Asn Leu Val Cys Lys Val Ser Asp Phe Gly Leu Ser Arg Val Ile Glu
    770                 775                 780

Asp Asp Pro Glu Ala Val Tyr Thr Thr Thr Gly Gly Lys Ile Pro Val
785                 790                 795                 800
Arg Trp Thr Ala Pro Glu Ala Ile Gln Tyr Arg Lys Phe Thr Ser Ala
                805                 810                 815
Ser Asp Val Trp Ser Tyr Gly Ile Val Met Trp Glu Val Met Ser Tyr
            820                 825                 830
Gly Glu Arg Pro Tyr Trp Asp Met Ser Asn Gln Asp Val Ile Lys Ala
        835                 840                 845
Ile Glu Glu Gly Tyr Arg Leu Pro Ala Pro Met Asp Cys Pro Ala Gly
    850                 855                 860
Leu His Gln Leu Met Leu Asp Cys Trp Gln Lys Glu Arg Ala Glu Arg
865                 870                 875                 880
Pro Lys Phe Glu Gln Ile Val Gly Ile Leu Asp Lys Met Ile Arg Asn
                885                 890                 895
Pro Asn Ser Leu Lys Thr Pro Leu Gly Thr Cys Ser Arg Pro Ile Ser
            900                 905                 910
Pro Leu Leu Asp Gln Asn Thr Pro Asp Phe Thr Thr Phe Cys Ser Val
        915                 920                 925
Gly Glu Trp Leu Gln Ala Ile Lys Met Glu Arg Tyr Lys Asp Asn Phe
    930                 935                 940
Thr Ala Ala Gly Tyr Asn Ser Leu Glu Ser Val Ala Arg Met Thr Ile
945                 950                 955                 960
Glu Asp Val Met Ser Leu Gly Ile Thr Leu Val Gly His Gln Lys Lys
                965                 970                 975
Ile Met Ser Ser Ile Gln Thr Met Arg Ala Gln Met Leu His Leu His
            980                 985                 990
Gly Thr Gly Ile Gln Val
        995

<210> 19
<211> 3018
<212> DNA
<213> Homo sapiens

<400> 19
atggcccccg cccggggccg cctgccccct gcgctctggg tcgtcacggc cgcggcggcg 60
gcggccacct gcgtgtccgc ggcgcgcggc gaagtgaatt tgctggacac gtcgaccatc 120
cacggggact ggggctggct cacgtatccg gctcatgggt gggactccat caacgaggtg 180
gacgagtcct tccagcccat ccacacgtac caggtttgca acgtcatgag ccccaaccag 240
aacaactggc tgcgcacgag ctgggtcccc cgagacggcg cccggcgcgt ctatgctgag 300
atcaagttta ccctgcgcga ctgcaacagc atgcctggtg tgctgggcac ctgcaaggag 360
accttcaacc tctactacct ggagtcggac cgcgacctgg gggccagcac acaagaaagc 420
cagttcctca aaatcgacac cattgcggcc gacgagagct tcacaggtgc cgaccttggt 480
gtgcggcgtc tcaagctcaa cacggaggtg cgcagtgtgg gtcccctcag caagcgcggc 540
ttctacctgg ccttccagga cataggtgcc tgcctggcca tcctctctct ccgcatctac 600
tataagaagt gccctgccat ggtgcgcaat ctggctgcct tctcggaggc agtgacgggg 660
gccgactcgt cctcactggt ggaggtgagg ggccagtgcg tgcggcactc agaggagcgg 720
gacacaccca agatgtactg cagcgcggag ggcgagtggc tcgtgcccat cggcaaatgc 780
gtgtgcagtg ccggctacga ggagcggcgg gatgcctgtg tggcctgtga gctgggcttc 840
tacaagtcag cccctgggga ccagctgtgt gcccgctgcc ctccccacag ccactccgca 900
gctccagccg cccaagcctg ccactgtgac ctcagctact accgtgcagc cctggacccg 960
ccgtcctcag cctgcacccg gccaccctcg gcaccagtga acctgatctc cagtgtgaat 1020
gggacatcag tgactctgga gtgggcccct cccctggacc caggtggccg cagtgacatc 1080
acctacaatg ccgtgtgccg ccgctgcccc tgggcactga gccgctgcga ggcatgtggg 1140
agcggcaccc gctttgtgcc ccagcagaca agcctggtgc aggccagcct gctggtggcc 1200
aacctgctgg cccacatgaa ctactccttc tggatcgagg ccgtcaatgg cgtgtccgac 1260
ctgagccccg agccccgccg ggccgctgtg gtcaacatca ccacgaacca ggcagccccg 1320
tcccaggtgg tggtgatccg tcaagagcgg gcggggcaga ccagcgtctc gctgctgtgg 1380
caggagcccg agcagccgaa cggcatcatc ctggagtatg agatcaagta ctacgagaag 1440
gacaaggaga tgcagagcta ctccaccctc aaggccgtca ccaccagagc caccgtctcc 1500
ggcctcaagc cgggcacccg ctacgtgttc caggtccgag cccgcacctc agcaggctgt 1560
ggccgcttca gccaggccat ggaggtggag accgggaaac cccggccccg ctatgacacc 1620
aggaccattg tctggatctg cctgacgctc atcacgggcc tggtggtgct tctgctcctg 1680
ctcatctgca agaagaggca ctgtggctac agcaaggcct tccaggactc ggacgaggag 1740
aagatgcact atcagaatgg acaggcaccc ccacctgtct tcctgcctct gcatcacccc 1800
ccgggaaagc tcccagagcc ccagttctat gcggaacccc acacctacga ggagccaggc 1860
cgggcgggcc gcagtttcac tcgggagatc gaggcctcta ggatccacat cgagaaaatc 1920
atcggctctg gagactccgg ggaagtctgc tacgggaggc tgcgggtgcc agggcagcgg 1980

gatgtgcccg tggccatcaa ggccctcaaa gccggctaca cggagagaca gaggcgggac 2040
ttcctgagcg aggcgtccat catggggcaa ttcgaccatc ccaacatcat ccgcctcgag 2100
ggtgtcgtca cccgtggccg cctggcaatg attgtgactg agtacatgga gaacggctct 2160
ctggacacct tcctgaggac ccacgacggg cagttcacca tcatgcagct ggtgggcatg 2220
ctgagaggag tgggtgccgg catgcgctac ctctcagacc tgggctatgt ccaccgagac 2280
ctggccgccc gcaacgtcct ggttgacagc aacctggtct gcaaggtgtc tgacttcggg 2340
ctctcacggg tgctggagga cgacccggat gctgcctaca ccaccacggg cgggaagatc 2400
cccatccgct ggacggcccc agaggccatc gccttccgca ccttctcctc ggccagcgac 2460
gtgtggagct tcggcgtggt catgtgggag gtgctggcct atggggagcg gccctactgg 2520
aacatgacca accgggatgt catcagctct gtggaggagg ggtaccgcct gcccgcaccc 2580
atgggctgcc cccacgccct gcaccagctc atgctcgact gttggcacaa ggaccgggcg 2640
cagcggcctc gcttctccca gattgtcagt gtcctcgatg cgctcatccg cagccctgag 2700
agtctcaggg ccaccgccac agtcagcagg tgcccacccc ctgccttcgt ccggagctgc 2760
tttgacctcc gagggggcag cggtggcggt gggggcctca ccgtggggga ctggctggac 2820
tccatccgca tgggccggta ccgagaccac ttcgctgcgg gcggatactc ctctctgggc 2880
atggtgctac gcatgaacgc ccaggacgtg cgcgccctgg gcatcaccct catgggccac 2940
cagaagaaga tcctgggcag cattcagacc atgcgggccc agctgaccag cacccagggg 3000
ccccgccggc acctctga 3018

<210> 20
<211> 1005
<212> PRT
<213> Homo sapiens

<220>
<221> BINDING
<222> (31)...(204)
<223> Ephrin receptor ligand binding domain

<220>
<221> CHAIN
<222> (191)...(325)
<223> cysteine-rich region

<220>
<221> CHAIN
<222> (329)...(424)
<223> Fibronectin type 3 domain

<220>
<221> CHAIN
<222> (440)...(531)
<223> Fibronectin type 3 domain

<220>
<221> CHAIN
<222> (635)...(892)
<223> Tyrosine kinase, catalytic domain

<220>
<221> CHAIN
<222> (934)...(992)
<223> SAM Domain (Sterile alpha motif)

<400> 20
Met Ala Pro Ala Arg Gly Arg Leu Pro Pro Ala Leu Trp Val Val Thr
1               5                   10                  15
Ala Ala Ala Ala Ala Ala Thr Cys Val Ser Ala Ala Arg Gly Glu Val
            20                  25                  30
Asn Leu Leu Asp Thr Ser Thr Ile His Gly Asp Trp Gly Trp Leu Thr
        35                  40                  45
Tyr Pro Ala His Gly Trp Asp Ser Ile Asn Glu Val Asp Glu Ser Phe
    50                  55                  60
Gln Pro Ile His Thr Tyr Gln Val Cys Asn Val Met Ser Pro Asn Gln
65                  70                  75                  80
Asn Asn Trp Leu Arg Thr Ser Trp Val Pro Arg Asp Gly Ala Arg Arg
                85                  90                  95
Val Tyr Ala Glu Ile Lys Phe Thr Leu Arg Asp Cys Asn Ser Met Pro
            100                 105                 110

Gly Val Leu Gly Thr Cys Lys Glu Thr Phe Asn Leu Tyr Tyr Leu Glu
        115                 120                 125
Ser Asp Arg Asp Leu Gly Ala Ser Thr Gln Glu Ser Gln Phe Leu Lys
    130                 135                 140
Ile Asp Thr Ile Ala Ala Asp Glu Ser Phe Thr Gly Ala Asp Leu Gly
145                 150                 155                 160
Val Arg Arg Leu Lys Leu Asn Thr Glu Val Arg Ser Val Gly Pro Leu
                165                 170                 175
Ser Lys Arg Gly Phe Tyr Leu Ala Phe Gln Asp Ile Gly Ala Cys Leu
            180                 185                 190
Ala Ile Leu Ser Leu Arg Ile Tyr Tyr Lys Lys Cys Pro Ala Met Val
        195                 200                 205
Arg Asn Leu Ala Ala Phe Ser Glu Ala Val Thr Gly Ala Asp Ser Ser
    210                 215                 220
Ser Leu Val Glu Val Arg Gly Gln Cys Val Arg His Ser Glu Glu Arg
225                 230                 235                 240
Asp Thr Pro Lys Met Tyr Cys Ser Ala Glu Gly Glu Trp Leu Val Pro
                245                 250                 255
Ile Gly Lys Cys Val Cys Ser Ala Gly Tyr Glu Glu Arg Arg Asp Ala
            260                 265                 270
Cys Val Ala Cys Glu Leu Gly Phe Tyr Lys Ser Ala Pro Gly Asp Gln
        275                 280                 285
Leu Cys Ala Arg Cys Pro Pro His Ser His Ser Ala Ala Pro Ala Ala
    290                 295                 300
Gln Ala Cys His Cys Asp Leu Ser Tyr Tyr Arg Ala Ala Leu Asp Pro
305                 310                 315                 320
Pro Ser Ser Ala Cys Thr Arg Pro Pro Ser Ala Pro Val Asn Leu Ile
                325                 330                 335
Ser Ser Val Asn Gly Thr Ser Val Thr Leu Glu Trp Ala Pro Pro Leu
            340                 345                 350
Asp Pro Gly Gly Arg Ser Asp Ile Thr Tyr Asn Ala Val Cys Arg Arg
        355                 360                 365
Cys Pro Trp Ala Leu Ser Arg Cys Glu Ala Cys Gly Ser Gly Thr Arg
    370                 375                 380
Phe Val Pro Gln Gln Thr Ser Leu Val Gln Ala Ser Leu Leu Val Ala
385                 390                 395                 400
Asn Leu Leu Ala His Met Asn Tyr Ser Phe Trp Ile Glu Ala Val Asn
                405                 410                 415
Gly Val Ser Asp Leu Ser Pro Glu Pro Arg Arg Ala Ala Val Val Asn
            420                 425                 430
Ile Thr Thr Asn Gln Ala Ala Pro Ser Gln Val Val Val Ile Arg Gln
        435                 440                 445
Glu Arg Ala Gly Gln Thr Ser Val Ser Leu Leu Trp Gln Glu Pro Glu
    450                 455                 460
Gln Pro Asn Gly Ile Ile Leu Glu Tyr Glu Ile Lys Tyr Tyr Glu Lys
465                 470                 475                 480
Asp Lys Glu Met Gln Ser Tyr Ser Thr Leu Lys Ala Val Thr Thr Arg
                485                 490                 495
Ala Thr Val Ser Gly Leu Lys Pro Gly Thr Arg Tyr Val Phe Gln Val
            500                 505                 510
Arg Ala Arg Thr Ser Ala Gly Cys Gly Arg Phe Ser Gln Ala Met Glu
        515                 520                 525
Val Glu Thr Gly Lys Pro Arg Pro Arg Tyr Asp Thr Arg Thr Ile Val
    530                 535                 540
Trp Ile Cys Leu Thr Leu Ile Thr Gly Leu Val Val Leu Leu Leu Leu
545                 550                 555                 560
Leu Ile Cys Lys Lys Arg His Cys Gly Tyr Ser Lys Ala Phe Gln Asp
                565                 570                 575
Ser Asp Glu Glu Lys Met His Tyr Gln Asn Gly Gln Ala Pro Pro Pro
            580                 585                 590
Val Phe Leu Pro Leu His His Pro Pro Gly Lys Leu Pro Glu Pro Gln
        595                 600                 605
Phe Tyr Ala Glu Pro His Thr Tyr Glu Glu Pro Gly Arg Ala Gly Arg
    610                 615                 620
Ser Phe Thr Arg Glu Ile Glu Ala Ser Arg Ile His Ile Glu Lys Ile
625                 630                 635                 640
Ile Gly Ser Gly Asp Ser Gly Glu Val Cys Tyr Gly Arg Leu Arg Val
                645                 650                 655
Pro Gly Gln Arg Asp Val Pro Val Ala Ile Lys Ala Leu Lys Ala Gly

660                 665                 670
Tyr Thr Glu Arg Gln Arg Arg Asp Phe Leu Ser Glu Ala Ser Ile Met
        675                 680                 685
Gly Gln Phe Asp His Pro Asn Ile Ile Arg Leu Glu Gly Val Val Thr
    690                 695                 700
Arg Gly Arg Leu Ala Met Ile Val Thr Glu Tyr Met Glu Asn Gly Ser
705                 710                 715                 720
Leu Asp Thr Phe Leu Arg Thr His Asp Gly Gln Phe Thr Ile Met Gln
                725                 730                 735
Leu Val Gly Met Leu Arg Gly Val Gly Ala Gly Met Arg Tyr Leu Ser
            740                 745                 750
Asp Leu Gly Tyr Val His Arg Asp Leu Ala Ala Arg Asn Val Leu Val
        755                 760                 765
Asp Ser Asn Leu Val Cys Lys Val Ser Asp Phe Gly Leu Ser Arg Val
    770                 775                 780
Leu Glu Asp Asp Pro Asp Ala Ala Tyr Thr Thr Thr Gly Gly Lys Ile
785                 790                 795                 800
Pro Ile Arg Trp Thr Ala Pro Glu Ala Ile Ala Phe Arg Thr Phe Ser
                805                 810                 815
Ser Ala Ser Asp Val Trp Ser Phe Gly Val Val Met Trp Glu Val Leu
            820                 825                 830
Ala Tyr Gly Glu Arg Pro Tyr Trp Asn Met Thr Asn Arg Asp Val Ile
        835                 840                 845
Ser Ser Val Glu Glu Gly Tyr Arg Leu Pro Ala Pro Met Gly Cys Pro
    850                 855                 860
His Ala Leu His Gln Leu Met Leu Asp Cys Trp His Lys Asp Arg Ala
865                 870                 875                 880
Gln Arg Pro Arg Phe Ser Gln Ile Val Ser Val Leu Asp Ala Leu Ile
                885                 890                 895
Arg Ser Pro Glu Ser Leu Arg Ala Thr Ala Thr Val Ser Arg Cys Pro
            900                 905                 910
Pro Pro Ala Phe Val Arg Ser Cys Phe Asp Leu Arg Gly Gly Ser Gly
        915                 920                 925
Gly Gly Gly Gly Leu Thr Val Gly Asp Trp Leu Asp Ser Ile Arg Met
    930                 935                 940
Gly Arg Tyr Arg Asp His Phe Ala Ala Gly Gly Tyr Ser Ser Leu Gly
945                 950                 955                 960
Met Val Leu Arg Met Asn Ala Gln Asp Val Arg Ala Leu Gly Ile Thr
                965                 970                 975
Leu Met Gly His Gln Lys Lys Ile Leu Gly Ser Ile Gln Thr Met Arg
            980                 985                 990
Ala Gln Leu Thr Ser Thr Gln Gly Pro Arg Arg His Leu
        995                 1000                1005


<210> 21
<211> 2955
<212> DNA
<213> Homo sapiens

<400> 21
atggccctgg attatctact actgctcctc ctggcatccg cagtggctgc gatggaagaa 60
acgttaatgg acaccagaac ggctactgca gagctgggct ggacggccaa tcctgcgtcc 120
gggtgggaag aagtcagtgg ctacgatgaa aacctgaaca ccatccgcac ctaccaggtg 180
tgcaatgtct tcgagcccaa ccagaacaat tggctgctca ccaccttcat caaccggcgg 240
ggggcccatc gcatctacac agagatgcgc ttcactgtga gagactgcag cagcctccct 300
aatgtcccag gatcctgcaa ggagaccttc aacttgtatt actatgagac tgactctgtc 360
attgccacca agaagtcagc cttctggtct gaggccccct acctcaaagt agacaccatt 420
gctgcagatg agagcttctc ccaggtggac tttgggggaa ggctgatgaa ggtaaacaca 480
gaagtcagga gctttgggcc tcttactcgg aatggttttt acctcgcttt tcaggattat 540
ggagcctgta tgtctcttct ttctgtccgt gtcttcttca aaaagtgtcc cagcattgtg 600
caaaattttg cagtgtttcc agagactatg acaggggcag agagcacatc tctggtgatt 660
gctcggggca catgcatccc caacgcagag gaagtggacg tgcccatcaa actctactgc 720
aacggggatg gggaatggat ggtgcctatt gggcgatgca cctgcaagcc tggctatgag 780
cctgagaaca gcgtggcatg caaggcttgc cctgcaggga cattcaaggc cagccaggaa 840
gctgaaggct gctcccactg cccctccaac agccgctccc ctgcagaggc gtctcccatc 900
tgcacctgtc ggaccggtta ttaccgagcg gactttgacc ctccagaagt ggcatgcact 960
agcgtcccat caggtccccg caatgttatc tccatcgtca atgagacgtc catcattctg 1020
gagtggcacc ctccaaggga gacaggtggg cgggatgatg tgacctacaa catcatctgc 1080

aaaaagtgcc gggcagaccg ccggagctgc tcccgctgtg acgacaatgt ggagtttgtg 1140
cccaggcagc tgggcctgac ggagtgccgc gtctccatca gcagcctgtg ggcccacacc 1200
ccctacacct ttgacatcca ggccatcaat ggagtctcca gcaagagtcc cttcccccca 1260
cagcacgtct ctgtcaacat caccacaaac caagccgccc cctccaccgt tcccatcatg 1320
caccaagtca gtgccactat gaggagcatc accttgtcat ggccacagcc ggagcagccc 1380
aatggcatca tcctggacta tgagatccgg tactatgaga aggaacacaa tgagttcaac 1440
tcctccatgg ccaggagtca gaccaacaca gcaaggattg atgggctgcg gcctggcatg 1500
gtatatgtgg tacaggtgcg tgcccgcact gttgctggct acggcaagtt cagtggcaag 1560
atgtgcttcc agactctgac tgacgatgat tacaagtcag agctgaggga gcagctgccc 1620
ctgattgctg gctcggcagc ggccggggtc gtgttcgttg tgtccttggt ggccatctct 1680
atcgtctgta gcaggaaacg ggcttatagc aaagaggctg tgtacagcga taagctccag 1740
cattacagca caggccgagg ctccccaggg atgaagatct acattgaccc cttcacttat 1800
gaggatccca acgaagctgt ccgggagttt gccaaggaga ttgatgtatc ttttgtgaaa 1860
attgaagagg tcatcggagc aggggagttt ggagaagtgt acaaggggcg tttgaaactg 1920
ccaggcaaga gggaaatcta cgtggccatc aagaccctga aggcagggta ctcggagaag 1980
cagcgtcggg actttctgag tgaggcgagc atcatgggcc agttcgacca tcctaacatc 2040
attcgcctgg agggtgtggt caccaagagt cggcctgtca tgatcatcac agagttcatg 2100
gagaatggtg cattggattc tttcctcagg caaaatgacg ggcagttcac cgtgatccag 2160
cttgtgggta tgctcagggg catcgctgct ggcatgaagt acctggctga gatgaattat 2220
gtgcatcggg acctggctgc taggaacatt ctggtcaaca gtaacctggt gtgcaaggtg 2280
tccgactttg gcctctcccg ctacctccag gatgacacct cagatcccac ctacaccagc 2340
tccttgggag ggaagatccc tgtgagatgg acagctccag aggccatcgc ctaccgcaag 2400
ttcacttcag ccagcgacgt ttggagctat gggatcgtca tgtgggaagt catgtcattt 2460
ggagagagac cctattggga tatgtccaac caagatgtca tcaatgccat cgagcaggac 2520
taccggctgc ccccacccat ggactgtcca gctgctctac accagctcat gctggactgt 2580
tggcagaagg accggaacag ccggccccgg tttgcggaga ttgtcaacac cctagataag 2640
atgatccgga acccggcaag tctcaagact gtggcaacca tcaccgccgt gccttcccag 2700
cccctgctcg accgctccat cccagacttc acggccttta ccaccgtgga tgactggctc 2760
agcgccatca aaatggtcca gtacagggac agcttcctca ctgctggctt cacctccctc 2820
cagctggtca cccagatgac atcagaagac ctcctgagaa taggcatcac cttggcaggc 2880
catcagaaga agatcctgaa cagcattcat tctatgaggg tccagataag tcagtcacca 2940
acggcaatgg catga 2955

<210> 22
<211> 984
<212> PRT
<213> Homo sapiens

<220>
<221> BINDING
<222> (19)...(196)
<223> Ephrin receptor ligand binding domain

<220>
<221> CHAIN
<222> (255)...(317)
<223> Tumor necrosis factor receptor (TNFR) domain

<220>
<221> CHAIN
<222> (323)...(423)
<223> Fibronectin type 3 domain

<220>
<221> CHAIN
<222> (437)...(518)
<223> Fibronectin type III domain

<220>
<221> CHAIN
<222> (613)...(881)
<223> Tyrosine kinase, catalytic domain

<220>
<221> CHAIN
<222> (908)...(973)
<223> SAM Domain (Sterile alpha motif)

<400> 22

Met Ala Leu Asp Tyr Leu Leu Leu Leu Leu Leu Ala Ser Ala Val Ala
1               5                   10                  15
Ala Met Glu Glu Thr Leu Met Asp Thr Arg Thr Ala Thr Ala Glu Leu
            20                  25                  30
Gly Trp Thr Ala Asn Pro Ala Ser Gly Trp Glu Glu Val Ser Gly Tyr
        35                  40                  45
Asp Glu Asn Leu Asn Thr Ile Arg Thr Tyr Gln Val Cys Asn Val Phe
    50                  55                  60
Glu Pro Asn Gln Asn Asn Trp Leu Leu Thr Thr Phe Ile Asn Arg Arg
65                  70                  75                  80
Gly Ala His Arg Ile Tyr Thr Glu Met Arg Phe Thr Val Arg Asp Cys
                85                  90                  95
Ser Ser Leu Pro Asn Val Pro Gly Ser Cys Lys Glu Thr Phe Asn Leu
            100                 105                 110
Tyr Tyr Tyr Glu Thr Asp Ser Val Ile Ala Thr Lys Lys Ser Ala Phe
        115                 120                 125
Trp Ser Glu Ala Pro Tyr Leu Lys Val Asp Thr Ile Ala Ala Asp Glu
    130                 135                 140
Ser Phe Ser Gln Val Asp Phe Gly Gly Arg Leu Met Lys Val Asn Thr
145                 150                 155                 160
Glu Val Arg Ser Phe Gly Pro Leu Thr Arg Asn Gly Phe Tyr Leu Ala
                165                 170                 175
Phe Gln Asp Tyr Gly Ala Cys Met Ser Leu Leu Ser Val Arg Val Phe
            180                 185                 190
Phe Lys Lys Cys Pro Ser Ile Val Gln Asn Phe Ala Val Phe Pro Glu
        195                 200                 205
Thr Met Thr Gly Ala Glu Ser Thr Ser Leu Val Ile Ala Arg Gly Thr
    210                 215                 220
Cys Ile Pro Asn Ala Glu Glu Val Asp Val Pro Ile Lys Leu Tyr Cys
225                 230                 235                 240
Asn Gly Asp Gly Glu Trp Met Val Pro Ile Gly Arg Cys Thr Cys Lys
                245                 250                 255
Pro Gly Tyr Glu Pro Glu Asn Ser Val Ala Cys Lys Ala Cys Pro Ala
            260                 265                 270
Gly Thr Phe Lys Ala Ser Gln Glu Ala Glu Gly Cys Ser His Cys Pro
        275                 280                 285
Ser Asn Ser Arg Ser Pro Ala Glu Ala Ser Pro Ile Cys Thr Cys Arg
    290                 295                 300
Thr Gly Tyr Tyr Arg Ala Asp Phe Asp Pro Pro Glu Val Ala Cys Thr
305                 310                 315                 320
Ser Val Pro Ser Gly Pro Arg Asn Val Ile Ser Ile Val Asn Glu Thr
                325                 330                 335
Ser Ile Ile Leu Glu Trp His Pro Pro Arg Glu Thr Gly Gly Arg Asp
            340                 345                 350
Asp Val Thr Tyr Asn Ile Ile Cys Lys Lys Cys Arg Ala Asp Arg Arg
        355                 360                 365
Ser Cys Ser Arg Cys Asp Asp Asn Val Glu Phe Val Pro Arg Gln Leu
    370                 375                 380
Gly Leu Thr Glu Cys Arg Val Ser Ile Ser Ser Leu Trp Ala His Thr
385                 390                 395                 400
Pro Tyr Thr Phe Asp Ile Gln Ala Ile Asn Gly Val Ser Ser Lys Ser
                405                 410                 415
Pro Phe Pro Pro Gln His Val Ser Val Asn Ile Thr Thr Asn Gln Ala
            420                 425                 430
Ala Pro Ser Thr Val Pro Ile Met His Gln Val Ser Ala Thr Met Arg
        435                 440                 445
Ser Ile Thr Leu Ser Trp Pro Gln Pro Glu Gln Pro Asn Gly Ile Ile
    450                 455                 460
Leu Asp Tyr Glu Ile Arg Tyr Tyr Glu Lys Glu His Asn Glu Phe Asn
465                 470                 475                 480
Ser Ser Met Ala Arg Ser Gln Thr Asn Thr Ala Arg Ile Asp Gly Leu
                485                 490                 495
Arg Pro Gly Met Val Tyr Val Val Gln Val Arg Ala Arg Thr Val Ala
            500                 505                 510
Gly Tyr Gly Lys Phe Ser Gly Lys Met Cys Phe Gln Thr Leu Thr Asp
        515                 520                 525
Asp Asp Tyr Lys Ser Glu Leu Arg Glu Gln Leu Pro Leu Ile Ala Gly
    530                 535                 540
Ser Ala Ala Ala Gly Val Val Phe Val Val Ser Leu Val Ala Ile Ser

545                 550                 555                 560
Ile Val Cys Ser Arg Lys Arg Ala Tyr Ser Lys Glu Ala Val Tyr Ser
                565                 570                 575
Asp Lys Leu Gln His Tyr Ser Thr Gly Arg Gly Ser Pro Gly Met Lys
            580                 585                 590
Ile Tyr Ile Asp Pro Phe Thr Tyr Glu Asp Pro Asn Glu Ala Val Arg
        595                 600                 605
Glu Phe Ala Lys Glu Ile Asp Val Ser Phe Val Lys Ile Glu Glu Val
    610                 615                 620
Ile Gly Ala Gly Glu Phe Gly Glu Val Tyr Lys Gly Arg Leu Lys Leu
625                 630                 635                 640
Pro Gly Lys Arg Glu Ile Tyr Val Ala Ile Lys Thr Leu Lys Ala Gly
                645                 650                 655
Tyr Ser Glu Lys Gln Arg Arg Asp Phe Leu Ser Glu Ala Ser Ile Met
            660                 665                 670
Gly Gln Phe Asp His Pro Asn Ile Ile Arg Leu Glu Gly Val Val Thr
        675                 680                 685
Lys Ser Arg Pro Val Met Ile Ile Thr Glu Phe Met Glu Asn Gly Ala
    690                 695                 700
Leu Asp Ser Phe Leu Arg Gln Asn Asp Gly Gln Phe Thr Val Ile Gln
705                 710                 715                 720
Leu Val Gly Met Leu Arg Gly Ile Ala Ala Gly Met Lys Tyr Leu Ala
                725                 730                 735
Glu Met Asn Tyr Val His Arg Asp Leu Ala Ala Arg Asn Ile Leu Val
            740                 745                 750
Asn Ser Asn Leu Val Cys Lys Val Ser Asp Phe Gly Leu Ser Arg Tyr
        755                 760                 765
Leu Gln Asp Asp Thr Ser Asp Pro Thr Tyr Thr Ser Ser Leu Gly Gly
    770                 775                 780
Lys Ile Pro Val Arg Trp Thr Ala Pro Glu Ala Ile Ala Tyr Arg Lys
785                 790                 795                 800
Phe Thr Ser Ala Ser Asp Val Trp Ser Tyr Gly Ile Val Met Trp Glu
                805                 810                 815
Val Met Ser Phe Gly Glu Arg Pro Tyr Trp Asp Met Ser Asn Gln Asp
            820                 825                 830
Val Ile Asn Ala Ile Glu Gln Asp Tyr Arg Leu Pro Pro Pro Met Asp
        835                 840                 845
Cys Pro Ala Ala Leu His Gln Leu Met Leu Asp Cys Trp Gln Lys Asp
    850                 855                 860
Arg Asn Ser Arg Pro Arg Phe Ala Glu Ile Val Asn Thr Leu Asp Lys
865                 870                 875                 880
Met Ile Arg Asn Pro Ala Ser Leu Lys Thr Val Ala Thr Ile Thr Ala
                885                 890                 895
Val Pro Ser Gln Pro Leu Leu Asp Arg Ser Ile Pro Asp Phe Thr Ala
            900                 905                 910
Phe Thr Thr Val Asp Asp Trp Leu Ser Ala Ile Lys Met Val Gln Tyr
        915                 920                 925
Arg Asp Ser Phe Leu Thr Ala Gly Phe Thr Ser Leu Gln Leu Val Thr
    930                 935                 940
Gln Met Thr Ser Glu Asp Leu Leu Arg Ile Gly Ile Thr Leu Ala Gly
945                 950                 955                 960
His Gln Lys Lys Ile Leu Asn Ser Ile His Ser Met Arg Val Gln Ile
                965                 970                 975
Ser Gln Ser Pro Thr Ala Met Ala
            980

<210> 23
<211> 3168
<212> DNA
<213> Homo sapiens

<400> 23
atggctctgc ggaggctggg ggccgcgctg ctgctgctgc cgctgctcgc cgccgtggaa 60
gaaacgctaa tggactccac tacagcgact gctgagctgg gctggatggt gcatcctcca 120
tcagggtggg aagaggtgag tggctacgat gagaacatga acacgatccg cacgtaccag 180
gtgtgcaacg tgtttgagtc aagccagaac aactggctac ggaccaagtt tatccggcgc 240
cgtggcgccc accgcatcca cgtggagatg aagttttcgg tgcgtgactg cagcagcatc 300
cccagcgtgc ctggctcctg caaggagacc ttcaacctct attactatga ggctgacttt 360

gactcggcca ccaagacctt ccccaactgg atggagaatc catgggtgaa ggtggatacc 420
attgcagccg acgagagctt ctcccaggtg gacctgggtg gccgcgtcat gaaaatcaac 480
accgaggtgc ggagcttcgg acctgtgtcc cgcagcggct tctacctggc cttccaggac 540
tatggcggct gcatgtccct catcgccgtg cgtgtcttct accgcaagtg cccccgcatc 600
atccagaatg gcgccatctt ccaggaaacc ctgtcggggg ctgagagcac atcgctggtg 660
gctgcccggg gcagctgcat cgccaatgcg gaagaggtgg atgtacccat caagctctac 720
tgtaacgggg acggcgagtg gctggtgccc atcgggcgct gcatgtgcaa agcaggcttc 780
gaggccgttg agaatggcac cgtctgccga ggttgtccat ctgggacttt caaggccaac 840
caaggggatg aggcctgtac ccactgtccc atcaacagcc ggaccacttc tgaaggggcc 900
accaactgtg tctgccgcaa tggctactac agagcagacc tggaccccct ggacatgccc 960
tgcacaacca tcccctccgc gccccaggct gtgatttcca gtgtcaatga gacctccctc 1020
atgctggagt ggacccctcc ccgcgactcc ggaggccgag aggacctcgt ctacaacatc 1080
atctgcaaga gctgtggctc gggccggggt gcctgcaccc gctgcgggga caatgtacag 1140
tacgcaccac gccagctagg cctgaccgag ccacgcattt acatcagtga cctgctggcc 1200
cacacccagt acaccttcga gatccaggct gtgaacggcg ttactgacca gagccccttc 1260
tcgcctcagt tcgcctctgt gaacatcacc accaaccagg cagctccatc ggcagtgtcc 1320
atcatgcatc aggtgagccg caccgtggac agcattaccc tgtcgtggtc ccagccagac 1380
cagcccaatg gcgtgatcct ggactatgag ctgcagtact atgagaagga gctcagtgag 1440
tacaacgcca cagccataaa aagccccacc aacacggtca ccgtgcaggg cctcaaagcc 1500
ggcgccatct atgtcttcca ggtgcgggca cgcaccgtgg caggctacgg gcgctacagc 1560
ggcaagatgt acttccagac catgacagaa gccgagtacc agacaagcat ccaggagaag 1620
ttgccactca tcatcggctc ctcggccgct ggcctggtct tcctcattgc tgtggttgtc 1680
atcgccatcg tgtgtaacag acgggggttt gagcgtgctg actcggagta cacggacaag 1740
ctgcaacact acaccagtgg ccacatgacc ccaggcatga agatctacat cgatcctttc 1800
acctacgagg accccaacga ggcagtgcgg gagtttgcca aggaaattga catctcctgt 1860
gtcaaaattg agcaggtgat cggagcaggg gagtttggcg aggtctgcag tggccacctg 1920
aagctgccag gcaagagaga gatctttgtg gccatcaaga cgctcaagtc gggctacacg 1980
gagaagcagc gccgggactt cctgagcgaa gcctccatca tgggccagtt cgaccatccc 2040
aacgtcatcc acctggaggg tgtcgtgacc aagagcacac ctgtgatgat catcaccgag 2100
ttcatggaga atggctccct ggactccttt ctccggcaaa acgatgggca gttcacagtc 2160
atccagctgg tgggcatgct tcggggcatc gcagctggca tgaagtacct ggcagacatg 2220
aactatgttc accgtgacct ggctgcccgc aacatcctcg tcaacagcaa cctggtctgc 2280
aaggtgtcgg actttggcct ctcacgcttt ctagaggacg atacctcaga ccccacctac 2340
accagtgccc tgggcggaaa gatccccatc cgctggacag ccccggaagc catccagtac 2400
cggaagttca cctcggccag tgatgtgtgg agctacggca ttgtcatgtg ggaggtgatg 2460
tcctatgggg agcggcccta ctgggacatg accaaccagg atgtaatcaa tgccattgag 2520
caggactatc ggctgccacc gcccatggac tgcccgagcg ccctgcacca actcatgctg 2580
gactgttggc agaaggaccg caaccaccgg cccaagttcg gccaaattgt caacacgcta 2640
gacaagatga tccgcaatcc caacagcctc aaagccatgg cgcccctctc ctctggcatc 2700
aacctgccgc tgctggaccg cacgatcccc gactacacca gctttaacac ggtggacgag 2760
tggctggagg ccatcaagat ggggcagtac aaggagagct tcgccaatgc cggcttcacc 2820
tcctttgacg tcgtgtctca gatgatgatg gaggacattc tccgggttgg ggtcactttg 2880
gctggccacc agaaaaaaat cctgaacagt atccaggtga tgcgggcgca gatgaaccag 2940
attcagtctg tggagggcca gccactcgcc aggaggccac gggccacggg aagaaccaag 3000
cggtgccagc cacgagacgt caccaagaaa acatgcaact caaacgacgg aaaaaaaaag 3060
ggaatgggaa aaaagaaaac agatcctggg agggggcggg aaatacaagg aatatttttt 3120
aaagaggatt ctcataagga aagcaatgac tgttcttgcg gggataa 3168

<210> 24
<211> 1055
<212> PRT
<213> Homo sapiens

<220>
<221> BINDING
<222> (20)...(197)
<223> Ephrin receptor ligand binding domain

<220>
<221> CHAIN
<222> (332)...(419)
<223> Fibronectin type III domain

<220>
<221> CHAIN
<222> (439)...(520)
<223> Fibronectin type III domain

<220>

<221> CHAIN
<222> (615)...(883)
<223> Tyrosine kinase, catalytic domain

<220>
<221> CHAIN
<222> (910)...(977)
<223> SAM Domain (Sterile alpha motif)

<400> 24
Met Ala Leu Arg Arg Leu Gly Ala Ala Leu Leu Leu Leu Pro Leu Leu
 1               5                  10                  15
Ala Ala Val Glu Glu Thr Leu Met Asp Ser Thr Thr Ala Thr Ala Glu
            20                  25                  30
Leu Gly Trp Met Val His Pro Pro Ser Gly Trp Glu Glu Val Ser Gly
        35                  40                  45
Tyr Asp Glu Asn Met Asn Thr Ile Arg Thr Tyr Gln Val Cys Asn Val
    50                  55                  60
Phe Glu Ser Ser Gln Asn Asn Trp Leu Arg Thr Lys Phe Ile Arg Arg
65                  70                  75                  80
Arg Gly Ala His Arg Ile His Val Glu Met Lys Phe Ser Val Arg Asp
                85                  90                  95
Cys Ser Ser Ile Pro Ser Val Pro Gly Ser Cys Lys Glu Thr Phe Asn
            100                 105                 110
Leu Tyr Tyr Tyr Glu Ala Asp Phe Asp Ser Ala Thr Lys Thr Phe Pro
        115                 120                 125
Asn Trp Met Glu Asn Pro Trp Val Lys Val Asp Thr Ile Ala Ala Asp
    130                 135                 140
Glu Ser Phe Ser Gln Val Asp Leu Gly Gly Arg Val Met Lys Ile Asn
145                 150                 155                 160
Thr Glu Val Arg Ser Phe Gly Pro Val Ser Arg Ser Gly Phe Tyr Leu
                165                 170                 175
Ala Phe Gln Asp Tyr Gly Gly Cys Met Ser Leu Ile Ala Val Arg Val
            180                 185                 190
Phe Tyr Arg Lys Cys Pro Arg Ile Ile Gln Asn Gly Ala Ile Phe Gln
        195                 200                 205
Glu Thr Leu Ser Gly Ala Glu Ser Thr Ser Leu Val Ala Ala Arg Gly
    210                 215                 220
Ser Cys Ile Ala Asn Ala Glu Glu Val Asp Val Pro Ile Lys Leu Tyr
225                 230                 235                 240
Cys Asn Gly Asp Gly Glu Trp Leu Val Pro Ile Gly Arg Cys Met Cys
                245                 250                 255
Lys Ala Gly Phe Glu Ala Val Glu Asn Gly Thr Val Cys Arg Gly Cys
            260                 265                 270
Pro Ser Gly Thr Phe Lys Ala Asn Gln Gly Asp Glu Ala Cys Thr His
        275                 280                 285
Cys Pro Ile Asn Ser Arg Thr Thr Ser Glu Gly Ala Thr Asn Cys Val
    290                 295                 300
Cys Arg Asn Gly Tyr Tyr Arg Ala Asp Leu Asp Pro Leu Asp Met Pro
305                 310                 315                 320
Cys Thr Thr Ile Pro Ser Ala Pro Gln Ala Val Ile Ser Ser Val Asn
                325                 330                 335
Glu Thr Ser Leu Met Leu Glu Trp Thr Pro Pro Arg Asp Ser Gly Gly
            340                 345                 350
Arg Glu Asp Leu Val Tyr Asn Ile Ile Cys Lys Ser Cys Gly Ser Gly
        355                 360                 365
Arg Gly Ala Cys Thr Arg Cys Gly Asp Asn Val Gln Tyr Ala Pro Arg
    370                 375                 380
Gln Leu Gly Leu Thr Glu Pro Arg Ile Tyr Ile Ser Asp Leu Leu Ala
385                 390                 395                 400
His Thr Gln Tyr Thr Phe Glu Ile Gln Ala Val Asn Gly Val Thr Asp
                405                 410                 415
Gln Ser Pro Phe Ser Pro Gln Phe Ala Ser Val Asn Ile Thr Thr Asn
            420                 425                 430
Gln Ala Ala Pro Ser Ala Val Ser Ile Met His Gln Val Ser Arg Thr
        435                 440                 445
Val Asp Ser Ile Thr Leu Ser Trp Ser Gln Pro Asp Gln Pro Asn Gly
    450                 455                 460
Val Ile Leu Asp Tyr Glu Leu Gln Tyr Tyr Glu Lys Glu Leu Ser Glu

465                 470                 475                 480
Tyr Asn Ala Thr Ala Ile Lys Ser Pro Thr Asn Thr Val Thr Val Gln
                485                 490                 495
Gly Leu Lys Ala Gly Ala Ile Tyr Val Phe Gln Val Arg Ala Arg Thr
            500                 505                 510
Val Ala Gly Tyr Gly Arg Tyr Ser Gly Lys Met Tyr Phe Gln Thr Met
        515                 520                 525
Thr Glu Ala Glu Tyr Gln Thr Ser Ile Gln Glu Lys Leu Pro Leu Ile
    530                 535                 540
Ile Gly Ser Ser Ala Ala Gly Leu Val Phe Leu Ile Ala Val Val Val
545                 550                 555                 560
Ile Ala Ile Val Cys Asn Arg Arg Gly Phe Glu Arg Ala Asp Ser Glu
                565                 570                 575
Tyr Thr Asp Lys Leu Gln His Tyr Thr Ser Gly His Met Thr Pro Gly
            580                 585                 590
Met Lys Ile Tyr Ile Asp Pro Phe Thr Tyr Glu Asp Pro Asn Glu Ala
        595                 600                 605
Val Arg Glu Phe Ala Lys Glu Ile Asp Ile Ser Cys Val Lys Ile Glu
    610                 615                 620
Gln Val Ile Gly Ala Gly Glu Phe Gly Glu Val Cys Ser Gly His Leu
625                 630                 635                 640
Lys Leu Pro Gly Lys Arg Glu Ile Phe Val Ala Ile Lys Thr Leu Lys
                645                 650                 655
Ser Gly Tyr Thr Glu Lys Gln Arg Arg Asp Phe Leu Ser Glu Ala Ser
            660                 665                 670
Ile Met Gly Gln Phe Asp His Pro Asn Val Ile His Leu Glu Gly Val
        675                 680                 685
Val Thr Lys Ser Thr Pro Val Met Ile Ile Thr Glu Phe Met Glu Asn
    690                 695                 700
Gly Ser Leu Asp Ser Phe Leu Arg Gln Asn Asp Gly Gln Phe Thr Val
705                 710                 715                 720
Ile Gln Leu Val Gly Met Leu Arg Gly Ile Ala Ala Gly Met Lys Tyr
                725                 730                 735
Leu Ala Asp Met Asn Tyr Val His Arg Asp Leu Ala Ala Arg Asn Ile
            740                 745                 750
Leu Val Asn Ser Asn Leu Val Cys Lys Val Ser Asp Phe Gly Leu Ser
        755                 760                 765
Arg Phe Leu Glu Asp Asp Thr Ser Asp Pro Thr Tyr Thr Ser Ala Leu
    770                 775                 780
Gly Gly Lys Ile Pro Ile Arg Trp Thr Ala Pro Glu Ala Ile Gln Tyr
785                 790                 795                 800
Arg Lys Phe Thr Ser Ala Ser Asp Val Trp Ser Tyr Gly Ile Val Met
                805                 810                 815
Trp Glu Val Met Ser Tyr Gly Glu Arg Pro Tyr Trp Asp Met Thr Asn
            820                 825                 830
Gln Asp Val Ile Asn Ala Ile Glu Gln Asp Tyr Arg Leu Pro Pro Pro
        835                 840                 845
Met Asp Cys Pro Ser Ala Leu His Gln Leu Met Leu Asp Cys Trp Gln
    850                 855                 860
Lys Asp Arg Asn His Arg Pro Lys Phe Gly Gln Ile Val Asn Thr Leu
865                 870                 875                 880
Asp Lys Met Ile Arg Asn Pro Asn Ser Leu Lys Ala Met Ala Pro Leu
                885                 890                 895
Ser Ser Gly Ile Asn Leu Pro Leu Leu Asp Arg Thr Ile Pro Asp Tyr
            900                 905                 910
Thr Ser Phe Asn Thr Val Asp Glu Trp Leu Glu Ala Ile Lys Met Gly
        915                 920                 925
Gln Tyr Lys Glu Ser Phe Ala Asn Ala Gly Phe Thr Ser Phe Asp Val
    930                 935                 940
Val Ser Gln Met Met Met Glu Asp Ile Leu Arg Val Gly Val Thr Leu
945                 950                 955                 960
Ala Gly His Gln Lys Lys Ile Leu Asn Ser Ile Gln Val Met Arg Ala
                965                 970                 975
Gln Met Asn Gln Ile Gln Ser Val Glu Gly Gln Pro Leu Ala Arg Arg
            980                 985                 990
Pro Arg Ala Thr Gly Arg Thr Lys Arg Cys Gln Pro Arg Asp Val Thr
        995                 1000                1005
Lys Lys Thr Cys Asn Ser Asn Asp Gly Lys Lys Lys Gly Met Gly Lys
    1010                1015                1020

Lys Lys Thr Asp Pro Gly Arg Gly Arg Glu Ile Gln Gly Ile Phe Phe
1025                1030                1035                1040
Lys Glu Asp Ser His Lys Glu Ser Asn Asp Cys Ser Cys Gly Gly
                1045                1050                1055

<210> 25
<211> 2964
<212> DNA
<213> Homo sapiens

<400> 25
atggctctgc ggaggctggg ggccgcgctg ctgctgctgc cgctgctcgc cgccgtggaa 60
gaaacgctaa tggactccac tacagcgact gctgagctgg gctggatggt gcatcctcca 120
tcagggtggg aagaggtgag tggctacgat gagaacatga acacgatccg cacgtaccag 180
gtgtgcaacg tgtttgagtc aagccagaac aactggctac ggaccaagtt tatccggcgc 240
cgtggcgccc accgcatcca cgtggagatg aagttttcgg tgcgtgactg cagcagcatc 300
cccagcgtgc ctggctcctg caaggagacc ttcaacctct attactatga ggctgacttt 360
gactcggcca ccaagacctt ccccaactgg atggagaatc catgggtgaa ggtggatacc 420
attgcagccg acgagagctt ctcccaggtg gacctgggtg gccgcgtcat gaaaatcaac 480
accgaggtgc ggagcttcgg acctgtgtcc cgcagcggct tctacctggc cttccaggac 540
tatggcggct gcatgtccct catcgccgtg cgtgtcttct accgcaagtg cccccgcatc 600
atccagaatg gcgccatctt ccaggaaacc ctgtcggggg ctgagagcac atcgctggtg 660
gctgcccggg gcagctgcat cgccaatgcg gaagaggtgg atgtacccat caagctctac 720
tgtaacgggg acggcgagtg gctggtgccc atcgggcgct gcatgtgcaa agcaggcttc 780
gaggccgttg agaatggcac cgtctgccga ggttgtccat ctgggacttt caaggccaac 840
caaggggatg aggcctgtac ccactgtccc atcaacagcc ggaccacttc tgaaggggcc 900
accaactgtg tctgccgcaa tggctactac agagcagacc tggaccccct ggacatgccc 960
tgcacaacca tcccctccgc gccccaggct gtgatttcca gtgtcaatga gacctccctc 1020
atgctggagt ggacccctcc ccgcgactcc ggaggccgag aggacctcgt ctacaacatc 1080
atctgcaaga gctgtggctc gggccggggt gcctgcaccc gctgcgggga caatgtacag 1140
tacgcaccac gccagctagg cctgaccgag ccacgcattt acatcagtga cctgctggcc 1200
cacacccagt acaccttcga gatccaggct gtgaacggcg ttactgacca gagccccttc 1260
tcgcctcagt tcgcctctgt gaacatcacc accaaccagg cagctccatc ggcagtgtcc 1320
atcatgcatc aggtgagccg caccgtggac agcattaccc tgtcgtggtc ccagccggac 1380
cagcccaatg gcgtgatcct ggactatgag ctgcagtact atgagaagga gctcagtgag 1440
tacaacgcca cagccataaa aagccccacc aacacggtca ccgtgcaggg cctcaaagcc 1500
ggcgccatct atgtcttcca ggtgcgggca cgcaccgtgg caggctacgg gcgctacagc 1560
ggcaagatgt acttccagac catgacagaa gccgagtacc agacaagcat ccaggagaag 1620
ttgccactca tcatcggctc ctcggccgct ggcctggtct tcctcattgc tgtggttgtc 1680
atcgccatcg tgtgtaacag aagacggggg tttgagcgtg ctgactcgga gtacacggac 1740
aagctgcaac actacaccag tggccacatg accccaggca tgaagatcta catcgatcct 1800
ttcacctacg aggaccccaa cgaggcagtg cgggagtttg ccaaggaaat tgacatctcc 1860
tgtgtcaaaa ttgagcaggt gatcggagca ggggagtttg gcgaggtctg cagtggccac 1920
ctgaagctgc caggcaagag agagatcttt gtggccatca agacgctcaa gtcgggctac 1980
acggagaagc agcgccggga cttcctgagc gaagcctcca tcatgggcca gttcgaccat 2040
cccaacgtca tccacctgga gggtgtcgtg accaagagca cacctgtgat gatcatcacc 2100
gagttcatgg agaatggctc cctggactcc tttctccggc aaaacgatgg gcagttcaca 2160
gtcatccagc tggtgggcat gcttcggggc atcgcagctg gcatgaagta cctggcagac 2220
atgaactatg ttcaccgtga cctggctgcc cgcaacatcc tcgtcaacag caacctggtc 2280
tgcaaggtgt cggactttgg gctctcacgc tttctagagg acgatacctc agaccccacc 2340
tacaccagtg ccctgggcgg aaagatcccc atccgctgga cagccccgga agccatccag 2400
taccggaagt tcacctcggc cagtgatgtg tggagctacg gcattgtcat gtgggaggtg 2460
atgtcctatg gggagcggcc ctactgggac atgaccaacc aggatgtaat caatgccatt 2520
gagcaggact atcggctgcc accgcccatg gactgcccga gcgccctgca ccaactcatg 2580
ctggactgtt ggcagaagga ccgcaaccac cggcccaagt tcggccaaat tgtcaacacg 2640
ctagacaaga tgatccgcaa tcccaacagc ctcaaagcca tggcgcccct ctcctctggc 2700
atcaacctgc cgctgctgga ccgcacgatc cccgactaca ccagctttaa cacggtggac 2760
gagtggctgg aggccatcaa gatggggcag tacaaggaga gcttcgccaa tgccggcttc 2820
acctcctttg acgtcgtgtc tcagatgatg atggaggaca ttctccgggt tggggtcact 2880
ttggctggcc accagaaaaa aatcctgaac agtatccagg tgatgcgggc gcagatgaac 2940
cagattcagt ctgtggaggt ttga                                         2964

<210> 26
<211> 987
<212> PRT
<213> Homo sapiens

<220>

<221> BINDING
<222> (20)...(197)
<223> Ephrin receptor ligand binding domain

<220>
<221> CHAIN
<222> (332)...(419)
<223> Fibronectin type III domain

<220>
<221> CHAIN
<222> (439)...(520)
<223> Fibronectin type III domain

<220>
<221> CHAIN
<222> (616)...(884)
<223> Tyrosine kinase, catalytic domain

<220>
<221> CHAIN
<222> (911)...(978)
<223> SAM Domain (Sterile alpha motif)

<400> 26
Met Ala Leu Arg Arg Leu Gly Ala Ala Leu Leu Leu Leu Pro Leu Leu
 1               5                  10                  15
Ala Ala Val Glu Glu Thr Leu Met Asp Ser Thr Thr Ala Thr Ala Glu
            20                  25                  30
Leu Gly Trp Met Val His Pro Pro Ser Gly Trp Glu Glu Val Ser Gly
        35                  40                  45
Tyr Asp Glu Asn Met Asn Thr Ile Arg Thr Tyr Gln Val Cys Asn Val
    50                  55                  60
Phe Glu Ser Ser Gln Asn Asn Trp Leu Arg Thr Lys Phe Ile Arg Arg
65                  70                  75                  80
Arg Gly Ala His Arg Ile His Val Glu Met Lys Phe Ser Val Arg Asp
                85                  90                  95
Cys Ser Ser Ile Pro Ser Val Pro Gly Ser Cys Lys Glu Thr Phe Asn
            100                 105                 110
Leu Tyr Tyr Tyr Glu Ala Asp Phe Asp Ser Ala Thr Lys Thr Phe Pro
        115                 120                 125
Asn Trp Met Glu Asn Pro Trp Val Lys Val Asp Thr Ile Ala Ala Asp
    130                 135                 140
Glu Ser Phe Ser Gln Val Asp Leu Gly Gly Arg Val Met Lys Ile Asn
145                 150                 155                 160
Thr Glu Val Arg Ser Phe Gly Pro Val Ser Arg Ser Gly Phe Tyr Leu
                165                 170                 175
Ala Phe Gln Asp Tyr Gly Gly Cys Met Ser Leu Ile Ala Val Arg Val
            180                 185                 190
Phe Tyr Arg Lys Cys Pro Arg Ile Ile Gln Asn Gly Ala Ile Phe Gln
        195                 200                 205
Glu Thr Leu Ser Gly Ala Glu Ser Thr Ser Leu Val Ala Ala Arg Gly
    210                 215                 220
Ser Cys Ile Ala Asn Ala Glu Glu Val Asp Val Pro Ile Lys Leu Tyr
225                 230                 235                 240
Cys Asn Gly Asp Gly Glu Trp Leu Val Pro Ile Gly Arg Cys Met Cys
                245                 250                 255
Lys Ala Gly Phe Glu Ala Val Glu Asn Gly Thr Val Cys Arg Gly Cys
            260                 265                 270
Pro Ser Gly Thr Phe Lys Ala Asn Gln Gly Asp Glu Ala Cys Thr His
        275                 280                 285
Cys Pro Ile Asn Ser Arg Thr Thr Ser Glu Gly Ala Thr Asn Cys Val
    290                 295                 300
Cys Arg Asn Gly Tyr Tyr Arg Ala Asp Leu Asp Pro Leu Asp Met Pro
305                 310                 315                 320
Cys Thr Thr Ile Pro Ser Ala Pro Gln Ala Val Ile Ser Ser Val Asn
                325                 330                 335
Glu Thr Ser Leu Met Leu Glu Trp Thr Pro Pro Arg Asp Ser Gly Gly
            340                 345                 350

Arg Glu Asp Leu Val Tyr Asn Ile Ile Cys Lys Ser Cys Gly Ser Gly
            355                 360                 365
Arg Gly Ala Cys Thr Arg Cys Gly Asp Asn Val Gln Tyr Ala Pro Arg
        370                 375                 380
Gln Leu Gly Leu Thr Glu Pro Arg Ile Tyr Ile Ser Asp Leu Leu Ala
385                 390                 395                 400
His Thr Gln Tyr Thr Phe Glu Ile Gln Ala Val Asn Gly Val Thr Asp
                405                 410                 415
Gln Ser Pro Phe Ser Pro Gln Phe Ala Ser Val Asn Ile Thr Thr Asn
            420                 425                 430
Gln Ala Ala Pro Ser Ala Val Ser Ile Met His Gln Val Ser Arg Thr
        435                 440                 445
Val Asp Ser Ile Thr Leu Ser Trp Ser Gln Pro Asp Gln Pro Asn Gly
    450                 455                 460
Val Ile Leu Asp Tyr Glu Leu Gln Tyr Tyr Glu Lys Glu Leu Ser Glu
465                 470                 475                 480
Tyr Asn Ala Thr Ala Ile Lys Ser Pro Thr Asn Thr Val Thr Val Gln
                485                 490                 495
Gly Leu Lys Ala Gly Ala Ile Tyr Val Phe Gln Val Arg Ala Arg Thr
            500                 505                 510
Val Ala Gly Tyr Gly Arg Tyr Ser Gly Lys Met Tyr Phe Gln Thr Met
        515                 520                 525
Thr Glu Ala Glu Tyr Gln Thr Ser Ile Gln Glu Lys Leu Pro Leu Ile
    530                 535                 540
Ile Gly Ser Ser Ala Ala Gly Leu Val Phe Leu Ile Ala Val Val Val
545                 550                 555                 560
Ile Ala Ile Val Cys Asn Arg Arg Arg Gly Phe Glu Arg Ala Asp Ser
                565                 570                 575
Glu Tyr Thr Asp Lys Leu Gln His Tyr Thr Ser Gly His Met Thr Pro
            580                 585                 590
Gly Met Lys Ile Tyr Ile Asp Pro Phe Thr Tyr Glu Asp Pro Asn Glu
        595                 600                 605
Ala Val Arg Glu Phe Ala Lys Glu Ile Asp Ile Ser Cys Val Lys Ile
    610                 615                 620
Glu Gln Val Ile Gly Ala Gly Glu Phe Gly Glu Val Cys Ser Gly His
625                 630                 635                 640
Leu Lys Leu Pro Gly Lys Arg Glu Ile Phe Val Ala Ile Lys Thr Leu
                645                 650                 655
Lys Ser Gly Tyr Thr Glu Lys Gln Arg Arg Asp Phe Leu Ser Glu Ala
            660                 665                 670
Ser Ile Met Gly Gln Phe Asp His Pro Asn Val Ile His Leu Glu Gly
        675                 680                 685
Val Val Thr Lys Ser Thr Pro Val Met Ile Ile Thr Glu Phe Met Glu
    690                 695                 700
Asn Gly Ser Leu Asp Ser Phe Leu Arg Gln Asn Asp Gly Gln Phe Thr
705                 710                 715                 720
Val Ile Gln Leu Val Gly Met Leu Arg Gly Ile Ala Ala Gly Met Lys
                725                 730                 735
Tyr Leu Ala Asp Met Asn Tyr Val His Arg Asp Leu Ala Ala Arg Asn
            740                 745                 750
Ile Leu Val Asn Ser Asn Leu Val Cys Lys Val Ser Asp Phe Gly Leu
        755                 760                 765
Ser Arg Phe Leu Glu Asp Asp Thr Ser Asp Pro Thr Tyr Thr Ser Ala
    770                 775                 780
Leu Gly Gly Lys Ile Pro Ile Arg Trp Thr Ala Pro Glu Ala Ile Gln
785                 790                 795                 800
Tyr Arg Lys Phe Thr Ser Ala Ser Asp Val Trp Ser Tyr Gly Ile Val
                805                 810                 815
Met Trp Glu Val Met Ser Tyr Gly Glu Arg Pro Tyr Trp Asp Met Thr
            820                 825                 830
Asn Gln Asp Val Ile Asn Ala Ile Glu Gln Asp Tyr Arg Leu Pro Pro
        835                 840                 845
Pro Met Asp Cys Pro Ser Ala Leu His Gln Leu Met Leu Asp Cys Trp
    850                 855                 860
Gln Lys Asp Arg Asn His Arg Pro Lys Phe Gly Gln Ile Val Asn Thr
865                 870                 875                 880
Leu Asp Lys Met Ile Arg Asn Pro Asn Ser Leu Lys Ala Met Ala Pro
                885                 890                 895
Leu Ser Ser Gly Ile Asn Leu Pro Leu Leu Asp Arg Thr Ile Pro Asp

900                 905                 910
Tyr Thr Ser Phe Asn Thr Val Asp Glu Trp Leu Glu Ala Ile Lys Met
        915                 920                 925
Gly Gln Tyr Lys Glu Ser Phe Ala Asn Ala Gly Phe Thr Ser Phe Asp
    930                 935                 940
Val Val Ser Gln Met Met Met Glu Asp Ile Leu Arg Val Gly Val Thr
945                 950                 955                 960
Leu Ala Gly His Gln Lys Lys Ile Leu Asn Ser Ile Gln Val Met Arg
                965                 970                 975
Ala Gln Met Asn Gln Ile Gln Ser Val Glu Val
            980                 985

<210> 27
<211> 2997
<212> DNA
<213> Homo sapiens

<400> 27
atggccagag cccgcccgcc gccgccgccg tcgccgccgc cggggcttct gccgctgctc 60
cctccgctgc tgctgctgcc gctgctgctg ctgcccgccg gctgccgggc gctggaagag 120
accctcatgg acacaaaatg ggtaacatct gagttggcgt ggacatctca tccagaaagt 180
gggtgggaag aggtgagtgg ctacgatgag gccatgaatc ccatccgcac ataccaggtg 240
tgtaatgtgc gcgagtcaag ccagaacaac tggcttcgca cggggttcat ctggcggcgg 300
gatgtgcagc gggtctacgt ggagctcaag ttcactgtgc gtgactgcaa cagcatcccc 360
aacatccccg gctcctgcaa ggagaccttc aacctcttct actacgaggc tgacagcgat 420
gtggcctcag cctcctcccc cttctggatg gagaacccct acgtgaaagt ggacaccatt 480
gcacccgatg agagcttctc gcggctggat gccggccgtg tcaacaccaa ggtgcgcagc 540
tttgggccac tttccaaggc tggcttctac ctggccttcc aggaccaggg cgcctgcatg 600
tcgctcatct ccgtgcgcgc cttctacaag aagtgtgcat ccaccaccgc aggcttcgca 660
ctcttccccg agaccctcac tggggcggag cccacctcgc tggtcattgc tcctggcacc 720
tgcatcccta acgccgtgga ggtgtcggtg ccactcaagc tctactgcaa cggcgatggg 780
gagtggatgg tgcctgtggg tgcctgcacc tgtgccaccg gccatgagcc agctgccaag 840
gagtcccagt gccgcccctg tccccctggg agctacaagg cgaagcaggg agaggggccc 900
tgcctcccat gtccccccaa cagccgtacc acctccccag ccgccagcat ctgcacctgc 960
cacaataact tctaccgtgc agactcggac tctgcggaca gtgcctgtac caccgtgcca 1020
tctccacccc gaggtgtgat ctccaatgtg aatgaaacct cactgatcct cgagtggagt 1080
gagccccggg acctgggtgg ccgggatgac ctcctgtaca atgtcatctg caagaagtgc 1140
catggggctg gaggggcctc agcctgctca cgctgtgatg acaacgtgga gtttgtgcct 1200
cggcagctgg gcctgacgga gcgccgggtc cacatcagcc atctgctggc ccacacgcgc 1260
tacacctttg aggtgcaggc ggtcaacggt gtctcgggca agagccctct gccgcctcgt 1320
tatgcggccg tgaatatcac cacaaaccag gctgccccgt ctgaagtgcc cacactacgc 1380
ctgcacagca gctcaggcag cagcctcacc ctatcctggg cacccccaga gcggcccaac 1440
ggagtcatcc tggactacga gatgaagtac tttgagaaga gcgagggcat cgcctccaca 1500
gtgaccagcc agatgaactc cgtgcagctg gacgggcttc ggcctgacgc ccgctatgtg 1560
gtccaggtcc gtgcccgcac agtagctggc tatgggcagt acagccgccc tgccgagttt 1620
gagaccacaa gtgagagagg ctctggggcc cagcagctcc aggagcagct tcccctcatc 1680
gtgggctccg ctacagctgg gcttgtcttc gtggtggctg tcgtggtcat cgctatcgtc 1740
tgcctcagga agcagcgaca cggctctgat tcggagtaca cggagaagct gcagcagtac 1800
attgctcctg gaatgaaggt ttatattgac ccttttacct acgaggaccc taatgaggct 1860
gttcgggagt ttgccaagga gatcgacgtg tcctgcgtca agatcgagga ggtgatcgga 1920
gctggggaat ttggggaagt gtgccgtggt cgactgaaac agcctggccg ccgagaggtg 1980
tttgtggcca tcaagacgct gaaggtgggc tacaccgaga ggcagcggcg ggacttccta 2040
agcgaggcct ccatcatggg tcagtttgat caccccaata taatccggct cgagggcgtg 2100
gtcaccaaaa gtcggccagt tatgatcctc actgagttca tggaaaactg cgccctggac 2160
tccttcctcc ggctcaacga tgggcagttc acggtcatcc agctggtggg catgttgcgg 2220
ggcattgctg ccggcatgaa gtacctgtcc gagatgaact atgtgcaccg cgacctggct 2280
gctcgcaaca tccttgtcaa cagcaacctg gtctgcaaag tctcagactt tggcctctcc 2340
cgcttcctgg aggatgaccc ctccgatcct acctacacca gttccctggg cgggaagatc 2400
cccatccgct ggactgcccc agaggccata gcctatcgga agttcacttc tgctagtgat 2460
gtctggagct acggaattgt catgtgggag gtcatgagct atggagagcg accctactgg 2520
gacatgagca accaggatgt catcaatgcc gtggagcagg attaccggct gccaccaccc 2580
atggactgtc ccacagcact gcaccagctc atgctggact gctgggtgcg ggaccggaac 2640
ctcaggccca aattctccca gattgtcaat accctggaca agctcatccg caatgctgcc 2700
agcctcaagg tcattgccag cgctcagtct ggcatgtcac agcccctcct ggaccgcacg 2760
gtcccagatt acacaacctt cacgacagtt ggtgattggc tggatgccat caagatgggg 2820
cggtacaagg agagcttcgt cagtgcgggg tttgcatctt ttgacctggt ggcccagatg 2880
acggcagaag acctgctccg tattggggtc accctggccg gccaccagaa gaagatcctg 2940
agcagtatcc aggacatgcg gctgcagatg aaccagacgc tgcctgtgca ggtctga 2997

<210> 28
<211> 998
<212> PRT
<213> Homo sapiens

<220>
<221> BINDING
<222> (39)...(212)
<223> Ephrin receptor ligand binding domain

<220>
<221> CHAIN
<222> (256)...(331)
<223> Tumor necrosis factor receptor (TNFR) domain

<220>
<221> CHAIN
<222> (340)...(436)
<223> Fibronectin type 3 domain

<220>
<221> CHAIN
<222> (473)...(542)
<223> Fibronectin type III domain

<220>
<221> TRANSMEM
<222> (556)...(582)
<223> transmembrane-region

<220>
<221> CHAIN
<222> (627)...(895)
<223> Tyrosine kinase, catalytic domain

<220>
<221> CHAIN
<222> (922)...(989)
<223> SAM Domain (Sterile alpha motif)

<400> 28
Met Ala Arg Ala Arg Pro Pro Pro Pro Pro Ser Pro Pro Pro Gly Leu
 1               5                  10                  15
Leu Pro Leu Leu Pro Pro Leu Leu Leu Leu Pro Leu Leu Leu Leu Pro
            20                  25                  30
Ala Gly Cys Arg Ala Leu Glu Glu Thr Leu Met Asp Thr Lys Trp Val
        35                  40                  45
Thr Ser Glu Leu Ala Trp Thr Ser His Pro Glu Ser Gly Trp Glu Glu
    50                  55                  60
Val Ser Gly Tyr Asp Glu Ala Met Asn Pro Ile Arg Thr Tyr Gln Val
65                  70                  75                  80
Cys Asn Val Arg Glu Ser Ser Gln Asn Asn Trp Leu Arg Thr Gly Phe
                85                  90                  95
Ile Trp Arg Arg Asp Val Gln Arg Val Tyr Val Glu Leu Lys Phe Thr
            100                 105                 110
Val Arg Asp Cys Asn Ser Ile Pro Asn Ile Pro Gly Ser Cys Lys Glu
        115                 120                 125
Thr Phe Asn Leu Phe Tyr Tyr Glu Ala Asp Ser Asp Val Ala Ser Ala
    130                 135                 140
Ser Ser Pro Phe Trp Met Glu Asn Pro Tyr Val Lys Val Asp Thr Ile
145                 150                 155                 160
Ala Pro Asp Glu Ser Phe Ser Arg Leu Asp Ala Gly Arg Val Asn Thr
                165                 170                 175
Lys Val Arg Ser Phe Gly Pro Leu Ser Lys Ala Gly Phe Tyr Leu Ala
            180                 185                 190
Phe Gln Asp Gln Gly Ala Cys Met Ser Leu Ile Ser Val Arg Ala Phe
        195                 200                 205
Tyr Lys Lys Cys Ala Ser Thr Thr Ala Gly Phe Ala Leu Phe Pro Glu

210                 215                 220
Thr Leu Thr Gly Ala Glu Pro Thr Ser Leu Val Ile Ala Pro Gly Thr
225                 230                 235                 240
Cys Ile Pro Asn Ala Val Glu Val Ser Val Pro Leu Lys Leu Tyr Cys
                245                 250                 255
Asn Gly Asp Gly Glu Trp Met Val Pro Val Gly Ala Cys Thr Cys Ala
            260                 265                 270
Thr Gly His Glu Pro Ala Ala Lys Glu Ser Gln Cys Arg Pro Cys Pro
        275                 280                 285
Pro Gly Ser Tyr Lys Ala Lys Gln Gly Glu Gly Pro Cys Leu Pro Cys
    290                 295                 300
Pro Pro Asn Ser Arg Thr Thr Ser Pro Ala Ala Ser Ile Cys Thr Cys
305                 310                 315                 320
His Asn Asn Phe Tyr Arg Ala Asp Ser Asp Ser Ala Asp Ser Ala Cys
                325                 330                 335
Thr Thr Val Pro Ser Pro Pro Arg Gly Val Ile Ser Asn Val Asn Glu
            340                 345                 350
Thr Ser Leu Ile Leu Glu Trp Ser Glu Pro Arg Asp Leu Gly Gly Arg
        355                 360                 365
Asp Asp Leu Leu Tyr Asn Val Ile Cys Lys Lys Cys His Gly Ala Gly
    370                 375                 380
Gly Ala Ser Ala Cys Ser Arg Cys Asp Asp Asn Val Glu Phe Val Pro
385                 390                 395                 400
Arg Gln Leu Gly Leu Thr Glu Arg Arg Val His Ile Ser His Leu Leu
                405                 410                 415
Ala His Thr Arg Tyr Thr Phe Glu Val Gln Ala Val Asn Gly Val Ser
            420                 425                 430
Gly Lys Ser Pro Leu Pro Pro Arg Tyr Ala Ala Val Asn Ile Thr Thr
        435                 440                 445
Asn Gln Ala Ala Pro Ser Glu Val Pro Thr Leu Arg Leu His Ser Ser
    450                 455                 460
Ser Gly Ser Ser Leu Thr Leu Ser Trp Ala Pro Pro Glu Arg Pro Asn
465                 470                 475                 480
Gly Val Ile Leu Asp Tyr Glu Met Lys Tyr Phe Glu Lys Ser Glu Gly
                485                 490                 495
Ile Ala Ser Thr Val Thr Ser Gln Met Asn Ser Val Gln Leu Asp Gly
            500                 505                 510
Leu Arg Pro Asp Ala Arg Tyr Val Val Gln Val Arg Ala Arg Thr Val
        515                 520                 525
Ala Gly Tyr Gly Gln Tyr Ser Arg Pro Ala Glu Phe Glu Thr Thr Ser
    530                 535                 540
Glu Arg Gly Ser Gly Ala Gln Gln Leu Gln Glu Gln Leu Pro Leu Ile
545                 550                 555                 560
Val Gly Ser Ala Thr Ala Gly Leu Val Phe Val Val Ala Val Val Val
                565                 570                 575
Ile Ala Ile Val Cys Leu Arg Lys Gln Arg His Gly Ser Asp Ser Glu
            580                 585                 590
Tyr Thr Glu Lys Leu Gln Gln Tyr Ile Ala Pro Gly Met Lys Val Tyr
        595                 600                 605
Ile Asp Pro Phe Thr Tyr Glu Asp Pro Asn Glu Ala Val Arg Glu Phe
    610                 615                 620
Ala Lys Glu Ile Asp Val Ser Cys Val Lys Ile Glu Glu Val Ile Gly
625                 630                 635                 640
Ala Gly Glu Phe Gly Glu Val Cys Arg Gly Arg Leu Lys Gln Pro Gly
                645                 650                 655
Arg Arg Glu Val Phe Val Ala Ile Lys Thr Leu Lys Val Gly Tyr Thr
            660                 665                 670
Glu Arg Gln Arg Arg Asp Phe Leu Ser Glu Ala Ser Ile Met Gly Gln
        675                 680                 685
Phe Asp His Pro Asn Ile Ile Arg Leu Glu Gly Val Val Thr Lys Ser
    690                 695                 700
Arg Pro Val Met Ile Leu Thr Glu Phe Met Glu Asn Cys Ala Leu Asp
705                 710                 715                 720
Ser Phe Leu Arg Leu Asn Asp Gly Gln Phe Thr Val Ile Gln Leu Val
                725                 730                 735
Gly Met Leu Arg Gly Ile Ala Ala Gly Met Lys Tyr Leu Ser Glu Met
            740                 745                 750
Asn Tyr Val His Arg Asp Leu Ala Ala Arg Asn Ile Leu Val Asn Ser
        755                 760                 765

Asn Leu Val Cys Lys Val Ser Asp Phe Gly Leu Ser Arg Phe Leu Glu
    770                 775                 780
Asp Asp Pro Ser Asp Pro Thr Tyr Thr Ser Ser Leu Gly Gly Lys Ile
785                 790                 795                 800
Pro Ile Arg Trp Thr Ala Pro Glu Ala Ile Ala Tyr Arg Lys Phe Thr
                805                 810                 815
Ser Ala Ser Asp Val Trp Ser Tyr Gly Ile Val Met Trp Glu Val Met
            820                 825                 830
Ser Tyr Gly Glu Arg Pro Tyr Trp Asp Met Ser Asn Gln Asp Val Ile
        835                 840                 845
Asn Ala Val Glu Gln Asp Tyr Arg Leu Pro Pro Pro Met Asp Cys Pro
    850                 855                 860
Thr Ala Leu His Gln Leu Met Leu Asp Cys Trp Val Arg Asp Arg Asn
865                 870                 875                 880
Leu Arg Pro Lys Phe Ser Gln Ile Val Asn Thr Leu Asp Lys Leu Ile
                885                 890                 895
Arg Asn Ala Ala Ser Leu Lys Val Ile Ala Ser Ala Gln Ser Gly Met
            900                 905                 910
Ser Gln Pro Leu Leu Asp Arg Thr Val Pro Asp Tyr Thr Thr Phe Thr
        915                 920                 925
Thr Val Gly Asp Trp Leu Asp Ala Ile Lys Met Gly Arg Tyr Lys Glu
    930                 935                 940
Ser Phe Val Ser Ala Gly Phe Ala Ser Phe Asp Leu Val Ala Gln Met
945                 950                 955                 960
Thr Ala Glu Asp Leu Leu Arg Ile Gly Val Thr Leu Ala Gly His Gln
                965                 970                 975
Lys Lys Ile Leu Ser Ser Ile Gln Asp Met Arg Leu Gln Met Asn Gln
            980                 985                 990
Thr Leu Pro Val Gln Val
        995


<210> 29
<211> 2964
<212> DNA
<213> Homo sapiens

<400> 29
atggagctcc gggtgctgct ctgctgggct tcgttggccg cagctttgga agagaccctg 60
ctgaacacaa aattggaaac tgctgatctg aagtgggtga cattccctca ggtggacggg 120
cagtgggagg aactgagcgg cctggatgag gaacagcaca gcgtgcgcac ctacgaagtg 180
tgtgacgtgc agcgtgcccc gggccaggcc cactggcttc gcacaggttg ggtcccacgg 240
cggggcgccg tccacgtgta cgccacgctg cgcttcacca tgctcgagtg cctgtccctg 300
cctcgggctg ggcgctcctg caaggagacc ttcaccgtct tctactatga gagcgatgcg 360
gacacggcca cggccctcac gccagcctgg atggagaacc cctacatcaa ggtggacacg 420
gtggccgcgg agcatctcac ccggaagcgc cctggggccg aggccaccgg gaaggtgaat 480
gtcaagacgc tgcgtctggg accgctcagc aaggctggct tctacctggc cttccaggac 540
cagggtgcct gcatggccct gctatccctg cacctcttct acaaaaagtg cgcccagctg 600
actgtgaacc tgactcgatt cccggagact gtgcctcggg agctggttgt gcccgtggcc 660
ggtagctgcg tggtggatgc cgtccccgcc cctggcccca gccccagcct ctactgccgt 720
gaggatggcc agtgggccga acagccggtc acgggctgca gctgtgctcc ggggttcgag 780
gcagctgagg ggaacaccaa gtgccgagcc tgtgcccagg gcaccttcaa gcccctgtca 840
ggagaagggt cctgccagcc atgcccagcc aatagccact ctaacaccat tggatcagcc 900
gtctgccagt gccgcgtcgg gtacttccgg gcacgcacag acccccgggg tgcaccctgc 960
accacccctc cttcggctcc gcggagcgtg gtttcccgcc tgaacggctc ctccctgcac 1020
ctggaatgga gtgcccccct ggagtctggt ggccgagagg acctcaccta cgccctccgc 1080
tgccgggagt gccgacccgg aggctcctgt gcgccctgcg ggggagacct gacttttgac 1140
cccggccccc gggacctggt ggagccctgg gtggtggttc gagggctacg tcctgacttc 1200
acctatacct ttgaggtcac tgcattgaac ggggtatcct ccttagccac ggggcccgtc 1260
ccatttgagc ctgtcaatgt caccactgac cgagaggtac ctcctgcagt gtctgacatc 1320
cgggtgacgc ggtcctcacc cagcagcttg agcctggcct gggctgttcc ccgggcaccc 1380
agtggggctg tgctggacta cgaggtcaaa taccatgaga agggcgccga gggtcccagc 1440
agcgtgcggt tcctgaagac gtcagaaaac cgggcagagc tgcgggggct gaagcgggga 1500
gccagctacc tggtgcaggt acgggcgcgc tctgaggccg gctacgggcc cttcggccag 1560
gaacatcaca gccagaccca actggatgag agcgagggct ggcgggagca gctggccctg 1620
attgcgggca cggcagtcgt gggtgtggtc ctggtcctgg tggtcattgt ggtcgcagtt 1680
ctctgcctca ggaagcagag caatgggaga gaagcagaat attcggacaa acacggacag 1740
tatctcatcg gacatggtac taaggtctac atcgacccct tcacttatga agaccctaat 1800
gaggctgtga gggaatttgc aaaagagatc gatgtctcct acgtcaagat tgaagaggtg 1860

attggtgcag gtgagtttgg cgaggtgtgc cgggggcggc tcaaggcccc agggaagaag 1920
gagagctgtg tggcaatcaa gaccctgaag ggtggctaca cggagcggca gcggcgtgag 1980
tttctgagcg aggcctccat catgggccag ttcgagcacc ccaatatcat ccgcctggag 2040
ggcgtggtca ccaacagcat gcccgtcatg attctcacag agttcatgga gaacggcgcc 2100
ctggactcct tcctgcggct aaacgacgga cagttcacag tcatccagct cgtgggcatg 2160
ctgcggggca tcgcctcggg catgcggtac cttgccgaga tgagctacgt ccaccgagac 2220
ctggctgctc gcaacatcct agtcaacagc aacctcgtct gcaaagtgtc tgactttggc 2280
ctttcccgat tcctggagga gaactcttcc gatcccacct acacgagctc cctgggagga 2340
aagattccca tccgatggac tgccccggag gccattgcct tccggaagtt cacttccgcc 2400
agtgatgcct ggagttacgg gattgtgatg tgggaggtga tgtcatttgg ggagaggccg 2460
tactgggaca tgagcaatca ggacgtgatc aatgccattg aacaggacta ccggctgccc 2520
ccgcccccag actgtcccac ctccctccac cagctcatgc tggactgttg gcagaaagac 2580
cggaatgccc ggccccgctt cccccaggtg gtcagcgccc tggacaagat gatccggaac 2640
cccgccagcc tcaaaatcgt ggcccgggag aatggcgggg cctcacaccc tctcctggac 2700
cagcggcagc ctcactactc agcttttggc tctgtgggcg agtggcttcg ggccatcaaa 2760
atgggaagat acgaagaaag tttcgcagcc gctggctttg gctccttcga gctggtcagc 2820
cagatctctg ctgaggacct gctccgaatc ggagtcactc tggcgggaca ccagaagaaa 2880
atcttggcca gtgtccagca catgaagtcc caggccaagc cgggaacccc gggtgggaca 2940
ggaggaccgg ccccgcagta ctga 2964

<210> 30
<211> 987
<212> PRT
<213> Homo sapiens

<220>
<221> BINDING
<222> (29)...(197)
<223> Ephrin receptor ligand binding domain

<220>
<221> CHAIN
<222> (239)...(321)
<223> Tumor necrosis factor receptor (TNFR) domain

<220>
<221> CHAIN
<222> (324)...(429)
<223> Fibronectin type 3 domain

<220>
<221> CHAIN
<222> (434)...(526)
<223> Fibronectin type 3 domain

<220>
<221> CHAIN
<222> (609)...(877)
<223> Tyrosine kinase, catalytic domain

<220>
<221> CHAIN
<222> (904)...(971)
<223> SAM Domain (Sterile alpha motif)

<400> 30

<210> 31
<211> 3021
<212> DNA
<213> Homo Sapiens

<400> 31
atggtgtgta gcctatgggt gctgctcctg gtgtcttcag ttctggctct ggaagaggta 60
ttgctggaca ccaccggaga gacatctgag attggctggc tcacctaccc accagggggg 120
tgggacgagg tgagtgttct ggacgaccag cgacgcctga ctcggacctt tgaggcatgt 180
catgtggcag gggcccctcc aggcaccggg caggacaatt ggttgcagac acactttgtg 240
gagcggcgcg gggcccagag ggcgcacatt cgactccact tctctgtgcg ggcatgctcc 300
agcctgggtg tgagcggcgg cacctgccgg gagaccttca ccctttacta ccgtcaggct 360
gaggagcccg acagccctga cagcgtttcc tcctggcacc tcaaacgctg gaccaaggtg 420
gacacaattg cagcagacga gagctttccc tcctcctcct cctcctcctc ctcttcttcc 480
tctgcagcgt gggctgtggg accccacggg gctgggcagc gggctggact gcaactgaac 540
gtcaaagagc ggagctttgg gcctctcacc caacgcggct tctacgtggc cttccaggac 600
acgggggcct gcctggccct ggtcgctgtc aggctcttct cctacacctg ccctgccgtg 660
ctccgatcct ttgcttcctt tccagagacg caggccagtg gggctggggg ggcctccctg 720
gtggcagctg tgggcacctg tgtggctcat gcagagccag aggaggatgg agtagggggc 780
caggcaggag gcagcccccc caggctgcac tgcaacgggg agggcaagtg gatggtagct 840
gtcgggggct gccgctgcca gcctggatac caaccagcac gaggagacaa ggcctgccaa 900
gcctgcccac gggggctcta taagtcttct gctgggaatg ctccctgctc accatgccct 960
gcccgcagtc acgctcccaa cccagcagcc cccgtttgcc cctgcctgga gggcttctac 1020
cgggccagtt ccgacccacc agaggccccc tgcactggtc ctccatcggc tccccaggag 1080
ctttggtttg aggtgcaagg ctcagcactc atgctacact ggcgcctgcc tcgggagctg 1140

gggggtcgag gggacctgct cttcaatgtc gtgtgcaagg agtgtgaagg ccgccaggaa 1200
cctgccagcg gtggtggggg cacttgtcac cgctgcaggg atgaggtcca cttcgaccct 1260
cgccagagag gcctgactga gagccgagtg ttagtggggg gactccgggc acacgtaccc 1320
tacatcttag aggtgcaggc tgttaatggg gtgtctgagc tcagccctga ccctcctcag 1380
gctgcagcca tcaatgtcag caccagccat gaagtgccct ctgctgtccc tgtggtgcac 1440
caggtgagcc gggcatccaa cagcatcacg gtgtcctggc cgcagcccga ccagaccaat 1500
gggaacatcc tggactatca gctccgctac tatgaccagg cagaagacga atcccactcc 1560
ttcaccctga ccagcgagac caacactgcc accgtgacac agctgagccc tggccacatc 1620
tatggtttcc aggtgcgggc ccggactgct gccggccacg gcccctacgg gggcaaagtc 1680
tatttccaga cacttcctca aggggagctg tcttcccagc ttccggaaag actctccttg 1740
gtgatcggct ccatcctggg ggctttggcc ttcctcctgc tggcagccat caccgtgctg 1800
gcggtcgtct tccagcggaa gcggcgtggg actggctaca cggagcagct gcagcaatac 1860
agcagcccag gactcggggt gaagtattac atcgacccct ccacctacga ggacccctgt 1920
caggccatcc gagaacttgc ccgggaagtc gatcctgctt atatcaagat tgaggaggtc 1980
attgggacag gctcttttgg agaagtgcgc cagggccgcc tgcagccacg gggacggagg 2040
gagcagactg tggccatcca ggccctgtgg gccgggggcg ccgaaagcct gcagatgacc 2100
ttcctgggcc gggccgcagt gctgggtcag ttccagcacc ccaacatcct gcggctggag 2160
ggcgtggtca ccaagagccg acccctcatg gtgctgacgg agttcatgga gcttggcccc 2220
ctggacagct tcctcaggca gcgggagggc cagttcagca gcctgcagct ggtggccatg 2280
cagcggggag tggctgctgc catgcagtac ctgtccagct ttgccttcgt ccatcgctcg 2340
ctgtctgccc acagcgtgct ggtgaatagc cacttggtgt gcaaggtggc ccgtcttggc 2400
cacagtcctc agggcccaag ttgtttgctt cgctgggcag ccccagaggt cattgcacat 2460
ggaaagcata caacatccag tgatgtctgg agctttggga tactcatgtg ggaagtgatg 2520
agttatggag aacggcctta ctgggacatg agtgagcagg aggtactaaa tgcaatagag 2580
caggagttcc ggctgccccc gcctccaggc tgtcctcctg gattacatct acttatgttg 2640
gacacttggc agaaggaccg tgcccggcgg cctcattttg accagctggt ggctgcattt 2700
gacaagatga tccgcaagcc agataccctg caggctggcg gggacccagg ggaaaggcct 2760
tcccaggccc ttctgacccc tgtggccctg gactttcctt gtctggactc accccaggcc 2820
tggctttcag ccattggact ggagtgctac caggacaact tctccaagtt tggcctctgt 2880
accttcagtg atgtggctca gctcagccta gaagacctgc ctgccctggg catcaccctg 2940
gctggccacc agaagaagct gctgcaccac atccagctcc ttcagcaaca cctgaggcag 3000
cagggctcag tggaggtctg a 3021

<210> 32
<211> 1006
<212> PRT
<213> Homo sapiens

<220>
<221> BINDING
<222> (23)...(217)
<223> Ephrin receptor ligand binding domain

<220>
<221> CHAIN
<222> (473)...(564)
<223> Fibronectin type 3 domain

<220>
<221> CHAIN
<222> (655)...(900)
<223> Tyrosine kinase, catalytic domain

<220>
<221> CHAIN
<222> (931)...(982)
<223>
SAM Domain (Sterile alpha motif)

<400> 32
Met Val Cys Ser Leu Trp Val Leu Leu Leu Val Ser Ser Val Leu Ala
1               5                   10                  15
Leu Glu Glu Val Leu Leu Asp Thr Thr Gly Glu Thr Ser Glu Ile Gly
            20                  25                  30
Trp Leu Thr Tyr Pro Pro Gly Gly Trp Asp Glu Val Ser Val Leu Asp
        35                  40                  45
Asp Gln Arg Arg Leu Thr Arg Thr Phe Glu Ala Cys His Val Ala Gly
    50                  55                  60
Ala Pro Pro Gly Thr Gly Gln Asp Asn Trp Leu Gln Thr His Phe Val

65                  70                  75                  80
Glu Arg Arg Gly Ala Gln Arg Ala His Ile Arg Leu His Phe Ser Val
                85                  90                  95
Arg Ala Cys Ser Ser Leu Gly Val Ser Gly Gly Thr Cys Arg Glu Thr
            100                 105                 110
Phe Thr Leu Tyr Tyr Arg Gln Ala Glu Glu Pro Asp Ser Pro Asp Ser
        115                 120                 125
Val Ser Ser Trp His Leu Lys Arg Trp Thr Lys Val Asp Thr Ile Ala
    130                 135                 140
Ala Asp Glu Ser Phe Pro Ser Ser Ser Ser Ser Ser Ser Ser Ser Ser
145                 150                 155                 160
Ser Ala Ala Trp Ala Val Gly Pro His Gly Ala Gly Gln Arg Ala Gly
                165                 170                 175
Leu Gln Leu Asn Val Lys Glu Arg Ser Phe Gly Pro Leu Thr Gln Arg
            180                 185                 190
Gly Phe Tyr Val Ala Phe Gln Asp Thr Gly Ala Cys Leu Ala Leu Val
        195                 200                 205
Ala Val Arg Leu Phe Ser Tyr Thr Cys Pro Ala Val Leu Arg Ser Phe
    210                 215                 220
Ala Ser Phe Pro Glu Thr Gln Ala Ser Gly Ala Gly Gly Ala Ser Leu
225                 230                 235                 240
Val Ala Ala Val Gly Thr Cys Val Ala His Ala Glu Pro Glu Glu Asp
                245                 250                 255
Gly Val Gly Gly Gln Ala Gly Gly Ser Pro Pro Arg Leu His Cys Asn
            260                 265                 270
Gly Glu Gly Lys Trp Met Val Ala Val Gly Gly Cys Arg Cys Gln Pro
        275                 280                 285
Gly Tyr Gln Pro Ala Arg Gly Asp Lys Ala Cys Gln Ala Cys Pro Arg
    290                 295                 300
Gly Leu Tyr Lys Ser Ser Ala Gly Asn Ala Pro Cys Ser Pro Cys Pro
305                 310                 315                 320
Ala Arg Ser His Ala Pro Asn Pro Ala Ala Pro Val Cys Pro Cys Leu
                325                 330                 335
Glu Gly Phe Tyr Arg Ala Ser Ser Asp Pro Pro Glu Ala Pro Cys Thr
            340                 345                 350
Gly Pro Pro Ser Ala Pro Gln Glu Leu Trp Phe Glu Val Gln Gly Ser
        355                 360                 365
Ala Leu Met Leu His Trp Arg Leu Pro Arg Glu Leu Gly Gly Arg Gly
    370                 375                 380
Asp Leu Leu Phe Asn Val Val Cys Lys Glu Cys Glu Gly Arg Gln Glu
385                 390                 395                 400
Pro Ala Ser Gly Gly Gly Gly Thr Cys His Arg Cys Arg Asp Glu Val
                405                 410                 415
His Phe Asp Pro Arg Gln Arg Gly Leu Thr Glu Ser Arg Val Leu Val
            420                 425                 430
Gly Gly Leu Arg Ala His Val Pro Tyr Ile Leu Glu Val Gln Ala Val
        435                 440                 445
Asn Gly Val Ser Glu Leu Ser Pro Asp Pro Pro Gln Ala Ala Ala Ile
    450                 455                 460
Asn Val Ser Thr Ser His Glu Val Pro Ser Ala Val Pro Val Val His
465                 470                 475                 480
Gln Val Ser Arg Ala Ser Asn Ser Ile Thr Val Ser Trp Pro Gln Pro
                485                 490                 495
Asp Gln Thr Asn Gly Asn Ile Leu Asp Tyr Gln Leu Arg Tyr Tyr Asp
            500                 505                 510
Gln Ala Glu Asp Glu Ser His Ser Phe Thr Leu Thr Ser Glu Thr Asn
        515                 520                 525
Thr Ala Thr Val Thr Gln Leu Ser Pro Gly His Ile Tyr Gly Phe Gln
    530                 535                 540
Val Arg Ala Arg Thr Ala Ala Gly His Gly Pro Tyr Gly Gly Lys Val
545                 550                 555                 560
Tyr Phe Gln Thr Leu Pro Gln Gly Glu Leu Ser Ser Gln Leu Pro Glu
                565                 570                 575
Arg Leu Ser Leu Val Ile Gly Ser Ile Leu Gly Ala Leu Ala Phe Leu
            580                 585                 590
Leu Leu Ala Ala Ile Thr Val Leu Ala Val Val Phe Gln Arg Lys Arg
        595                 600                 605
Arg Gly Thr Gly Tyr Thr Glu Gln Leu Gln Gln Tyr Ser Ser Pro Gly
    610                 615                 620

Leu Gly Val Lys Tyr Tyr Ile Asp Pro Ser Thr Tyr Glu Asp Pro Cys
625                 630                 635                 640
Gln Ala Ile Arg Glu Leu Ala Arg Glu Val Asp Pro Ala Tyr Ile Lys
                645                 650                 655
Ile Glu Glu Val Ile Gly Thr Gly Ser Phe Gly Glu Val Arg Gln Gly
            660                 665                 670
Arg Leu Gln Pro Arg Gly Arg Arg Glu Gln Thr Val Ala Ile Gln Ala
        675                 680                 685
Leu Trp Ala Gly Gly Ala Glu Ser Leu Gln Met Thr Phe Leu Gly Arg
    690                 695                 700
Ala Ala Val Leu Gly Gln Phe Gln His Pro Asn Ile Leu Arg Leu Glu
705                 710                 715                 720
Gly Val Val Thr Lys Ser Arg Pro Leu Met Val Leu Thr Glu Phe Met
                725                 730                 735
Glu Leu Gly Pro Leu Asp Ser Phe Leu Arg Gln Arg Glu Gly Gln Phe
            740                 745                 750
Ser Ser Leu Gln Leu Val Ala Met Gln Arg Gly Val Ala Ala Met
        755                 760                 765
Gln Tyr Leu Ser Ser Phe Ala Phe Val His Arg Ser Leu Ser Ala His
    770                 775                 780
Ser Val Leu Val Asn Ser His Leu Val Cys Lys Val Ala Arg Leu Gly
785                 790                 795                 800
His Ser Pro Gln Gly Pro Ser Cys Leu Leu Arg Trp Ala Ala Pro Glu
                805                 810                 815
Val Ile Ala His Gly Lys His Thr Thr Ser Ser Asp Val Trp Ser Phe
            820                 825                 830
Gly Ile Leu Met Trp Glu Val Met Ser Tyr Gly Glu Arg Pro Tyr Trp
        835                 840                 845
Asp Met Ser Glu Gln Glu Val Leu Asn Ala Ile Glu Gln Glu Phe Arg
    850                 855                 860
Leu Pro Pro Pro Pro Gly Cys Pro Pro Gly Leu His Leu Leu Met Leu
865                 870                 875                 880
Asp Thr Trp Gln Lys Asp Arg Ala Arg Arg Pro His Phe Asp Gln Leu
                885                 890                 895
Val Ala Ala Phe Asp Lys Met Ile Arg Lys Pro Asp Thr Leu Gln Ala
            900                 905                 910
Gly Gly Asp Pro Gly Glu Arg Pro Ser Gln Ala Leu Leu Thr Pro Val
        915                 920                 925
Ala Leu Asp Phe Pro Cys Leu Asp Ser Pro Gln Ala Trp Leu Ser Ala
    930                 935                 940
Ile Gly Leu Glu Cys Tyr Gln Asp Asn Phe Ser Lys Phe Gly Leu Cys
945                 950                 955                 960
Thr Phe Ser Asp Val Ala Gln Leu Ser Leu Glu Asp Leu Pro Ala Leu
                965                 970                 975
Gly Ile Thr Leu Ala Gly His Gln Lys Lys Leu Leu His His Ile Gln
            980                 985                 990
Leu Leu Gln Gln His Leu Arg Gln Gln Gly Ser Val Glu Val
        995                 1000                1005

<210> 33
<211> 618
<212> DNA
<213> Homo sapiens

<400> 33
atggagttcc tctgggcccc tctcttgggt ctgtgctgca gtctggccgc tgctgatcgc 60
cacaccgtct tctggaacag ttcaaatccc aagttccgga atgaggacta caccatacat 120
gtgcagctga atgactacgt ggacatcatc tgtccgcact atgaagatca ctctgtggca 180
gacgctgcca tggagcagta catactgtac ctggtggagc atgaggagta ccagctgtgc 240
cagccccagt ccaaggacca agtccgctgg cagtgcaacc ggcccagtgc caagcatggc 300
ccggagaagc tgtctgagaa gttccagcgc ttcacacctt tcaccctggg caaggagttc 360
aaagaaggac acagctacta ctacatctcc aaacccatcc accagcatga agaccgctgc 420
ttgaggttga aggtgactgt cagtggcaaa atcactcaca gtcctcaggc ccatgacaat 480
ccacaggaga agagacttgc agcagatgac ccagaggtgc gggttctaca tagcatcggt 540
cacagtgctg ccccacgcct cttcccactt gcctggactg tgctgctcct tccacttctg 600
ctgctgcaaa ccccgtga                                                618

<210> 34

<211> 205
<212> PRT
<213> Homo sapiens

<400> 34
Met Glu Phe Leu Trp Ala Pro Leu Leu Gly Leu Cys Cys Ser Leu Ala
1               5                   10                  15
Ala Ala Asp Arg His Thr Val Phe Trp Asn Ser Ser Asn Pro Lys Phe
            20                  25                  30
Arg Asn Glu Asp Tyr Thr Ile His Val Gln Leu Asn Asp Tyr Val Asp
        35                  40                  45
Ile Ile Cys Pro His Tyr Glu Asp His Ser Val Ala Asp Ala Ala Met
    50                  55                  60
Glu Gln Tyr Ile Leu Tyr Leu Val Glu His Glu Glu Tyr Gln Leu Cys
65                  70                  75                  80
Gln Pro Gln Ser Lys Asp Gln Val Arg Trp Gln Cys Asn Arg Pro Ser
                85                  90                  95
Ala Lys His Gly Pro Glu Lys Leu Ser Glu Lys Phe Gln Arg Phe Thr
            100                 105                 110
Pro Phe Thr Leu Gly Lys Glu Phe Lys Glu Gly His Ser Tyr Tyr Tyr
        115                 120                 125
Ile Ser Lys Pro Ile His Gln His Glu Asp Arg Cys Leu Arg Leu Lys
    130                 135                 140
Val Thr Val Ser Gly Lys Ile Thr His Ser Pro Gln Ala His Asp Asn
145                 150                 155                 160
Pro Gln Glu Lys Arg Leu Ala Ala Asp Asp Pro Glu Val Arg Val Leu
                165                 170                 175
His Ser Ile Gly His Ser Ala Ala Pro Arg Leu Phe Pro Leu Ala Trp
            180                 185                 190
Thr Val Leu Leu Leu Pro Leu Leu Leu Leu Gln Thr Pro
        195                 200                 205


<210> 35
<211> 552
<212> DNA
<213> Homo sapiens

<400> 35
atggagttcc tctgggcccc tctcttgggt ctgtgctgca gtctggccgc tgctgatcgc 60
cacaccgtct tctggaacag ttcaaatccc aagttccgga atgaggacta caccatacat 120
gtgcagctga atgactacgt ggacatcatc tgtccgcact atgaagatca ctctgtggca 180
gacgctgcca tggagcagta catactgtac ctggtggagc atgaggagta ccagctgtgc 240
cagccccagt ccaaggacca agtccgctgg cagtgcaacc ggcccagtgc caagcatggc 300
ccggagaagc tgtctgagaa gttccagcgc ttcacacctt tcaccctggg caaggagttc 360
aaagaaggac acagctacta ctacatctct cacagtcctc aggcccatga caatccacag 420
gagaagagac ttgcagcaga tgacccagag gtgcgggttc tacatagcat cggtcacagt 480
gctgccccac gcctcttccc acttgcctgg actgtgctgc tccttccact tctgctgctg 540
caaaccccgt ga                                                     552

<210> 36
<211> 183
<212> PRT
<213> Homo sapiens

<400> 36
Met Glu Phe Leu Trp Ala Pro Leu Leu Gly Leu Cys Cys Ser Leu Ala
1               5                   10                  15
Ala Ala Asp Arg His Thr Val Phe Trp Asn Ser Ser Asn Pro Lys Phe
            20                  25                  30
Arg Asn Glu Asp Tyr Thr Ile His Val Gln Leu Asn Asp Tyr Val Asp
        35                  40                  45
Ile Ile Cys Pro His Tyr Glu Asp His Ser Val Ala Asp Ala Ala Met
    50                  55                  60
Glu Gln Tyr Ile Leu Tyr Leu Val Glu His Glu Glu Tyr Gln Leu Cys
65                  70                  75                  80
Gln Pro Gln Ser Lys Asp Gln Val Arg Trp Gln Cys Asn Arg Pro Ser
                85                  90                  95
Ala Lys His Gly Pro Glu Lys Leu Ser Glu Lys Phe Gln Arg Phe Thr

100                 105                 110
Pro Phe Thr Leu Gly Lys Glu Phe Lys Glu Gly His Ser Tyr Tyr Tyr
        115                 120                 125
Ile Ser His Ser Pro Gln Ala His Asp Asn Pro Gln Glu Lys Arg Leu
    130                 135                 140
Ala Ala Asp Asp Pro Glu Val Arg Val Leu His Ser Ile Gly His Ser
145                 150                 155                 160
Ala Ala Pro Arg Leu Phe Pro Leu Ala Trp Thr Val Leu Leu Leu Pro
                165                 170                 175
Leu Leu Leu Leu Gln Thr Pro
            180

<210> 37
<211> 642
<212> DNA
<213> Homo sapiens

<400> 37
atggcgcccg cgcagcgccc gctgctcccg ctgctgctcc tgctgttacc gctgccgccg 60
ccgcccttcg cgcgcgccga ggacgccgcc cgcgccaact cggaccgcta cgccgtctac 120
tggaaccgca gcaaccccag gttccacgca ggcgcggggg acgacggcgg gggctacacg 180
gtggaggtga gcatcaatga ctacctggac atctactgcc cgcactatgg ggcgccgctg 240
ccgccggccg agcgcatgga gcactacgtg ctgtacatgg tcaacggcga gggccacgcc 300
tcctgcgacc accgccagcg cggcttcaag cgctgggagt gcaaccggcc cgcggcgccc 360
ggggggccgc tcaagttctc ggagaagttc cagctcttca cgcccttctc cctgggcttc 420
gagttccggc ccggccacga gtattactac atctctgcca cgcctcccaa tgctgtggac 480
cggccctgcc tgcgactgaa ggtgtacgtg cggccgacca acgagaccct gtacgaggct 540
cctgagccca tcttcaccag caataactcg tgtagcagcc cgggcggctg ccgcctcttc 600
ctcagcacca tccccgtgct ctggaccctc ctgggttcct ag                    642

<210> 38
<211> 213
<212> PRT
<213> Homo sapiens

<400> 38
Met Ala Pro Ala Gln Arg Pro Leu Leu Pro Leu Leu Leu Leu Leu Leu
1               5                   10                  15
Pro Leu Pro Pro Pro Pro Phe Ala Arg Ala Glu Asp Ala Ala Arg Ala
            20                  25                  30
Asn Ser Asp Arg Tyr Ala Val Tyr Trp Asn Arg Ser Asn Pro Arg Phe
        35                  40                  45
His Ala Gly Ala Gly Asp Asp Gly Gly Gly Tyr Thr Val Glu Val Ser
    50                  55                  60
Ile Asn Asp Tyr Leu Asp Ile Tyr Cys Pro His Tyr Gly Ala Pro Leu
65                  70                  75                  80
Pro Pro Ala Glu Arg Met Glu His Tyr Val Leu Tyr Met Val Asn Gly
                85                  90                  95
Glu Gly His Ala Ser Cys Asp His Arg Gln Arg Gly Phe Lys Arg Trp
            100                 105                 110
Glu Cys Asn Arg Pro Ala Ala Pro Gly Gly Pro Leu Lys Phe Ser Glu
        115                 120                 125
Lys Phe Gln Leu Phe Thr Pro Phe Ser Leu Gly Phe Glu Phe Arg Pro
    130                 135                 140
Gly His Glu Tyr Tyr Tyr Ile Ser Ala Thr Pro Pro Asn Ala Val Asp
145                 150                 155                 160
Arg Pro Cys Leu Arg Leu Lys Val Tyr Val Arg Pro Thr Asn Glu Thr
                165                 170                 175
Leu Tyr Glu Ala Pro Glu Pro Ile Phe Thr Ser Asn Asn Ser Cys Ser
            180                 185                 190
Ser Pro Gly Gly Cys Arg Leu Phe Leu Ser Thr Ile Pro Val Leu Trp
        195                 200                 205
Thr Leu Leu Gly Ser
    210

<210> 39
<211> 717

<212> DNA
<213> Homo sapiens

<400> 39
atggcggcgg ctccgctgct gctgctgctg ctgctcgtgc ccgtgccgct gctgccgctg 60
ctggcccaag ggcccggagg ggcgctggga aaccggcatg cggtgtactg gaacagctcc 120
aaccagcacc tgcggcgaga gggctacacc gtgcaggtga acgtgaacga ctatctggat 180
atttactgcc cgcactacaa cagctcgggg gtgggccccg gggcgggacc ggggcccgga 240
ggcggggcag agcagtacgt gctgtacatg gtgagccgca acggctaccg cacctgcaac 300
gccagccagg gcttcaagcg ctgggagtgc aaccggccgc acgccccgca cagccccatc 360
aagttctcgg agaagttcca gcgctacagc gccttctctc tgggctacga gttccacgcc 420
ggccacgagt actactacat ctccacgccc actcacaacc tgcactggaa gtgtctgagg 480
atgaaggtgt tcgtctgctg cgcctccaca tcgcactccg gggagaagcc ggtccccact 540
ctcccccagt tcaccatggg ccccaatgtg aagatcaacg tgctggaaga ctttgaggga 600
gagaaccctc aggtgcccaa gcttgagaag agcatcagcg ggaccagccc caaacgggaa 660
cacctgcccc tggccgtggg catcgccttc ttcctcatga cgttcttggc ctcctag 717

<210> 40
<211> 238
<212> PRT
<213> Homo sapiens

<400> 40
Met Ala Ala Ala Pro Leu Leu Leu Leu Leu Leu Leu Val Pro Val Pro
1               5                   10                  15
Leu Leu Pro Leu Leu Ala Gln Gly Pro Gly Gly Ala Leu Gly Asn Arg
            20                  25                  30
His Ala Val Tyr Trp Asn Ser Ser Asn Gln His Leu Arg Arg Glu Gly
        35                  40                  45
Tyr Thr Val Gln Val Asn Val Asn Asp Tyr Leu Asp Ile Tyr Cys Pro
    50                  55                  60
His Tyr Asn Ser Ser Gly Val Gly Pro Gly Ala Gly Pro Gly Pro Gly
65                  70                  75                  80
Gly Gly Ala Glu Gln Tyr Val Leu Tyr Met Val Ser Arg Asn Gly Tyr
                85                  90                  95
Arg Thr Cys Asn Ala Ser Gln Gly Phe Lys Arg Trp Glu Cys Asn Arg
            100                 105                 110
Pro His Ala Pro His Ser Pro Ile Lys Phe Ser Glu Lys Phe Gln Arg
        115                 120                 125
Tyr Ser Ala Phe Ser Leu Gly Tyr Glu Phe His Ala Gly His Glu Tyr
    130                 135                 140
Tyr Tyr Ile Ser Thr Pro Thr His Asn Leu His Trp Lys Cys Leu Arg
145                 150                 155                 160
Met Lys Val Phe Val Cys Cys Ala Ser Thr Ser His Ser Gly Glu Lys
                165                 170                 175
Pro Val Pro Thr Leu Pro Gln Phe Thr Met Gly Pro Asn Val Lys Ile
            180                 185                 190
Asn Val Leu Glu Asp Phe Glu Gly Glu Asn Pro Gln Val Pro Lys Leu
        195                 200                 205
Glu Lys Ser Ile Ser Gly Thr Ser Pro Lys Arg Glu His Leu Pro Leu
    210                 215                 220
Ala Val Gly Ile Ala Phe Phe Leu Met Thr Phe Leu Ala Ser
225                 230                 235

<210> 41
<211> 606
<212> DNA
<213> Homo sapiens

<400> 41
atgcggctgc tgcccctgct gcggactgtc ctctgggccg cgttcctcgg ctcccctctg 60
cgcgggggct ccagcctccg ccacgtagtc tactggaact ccagtaaccc caggttgctt 120
cgaggagacg ccgtggtgga gctgggcctc aacgattacc tagacattgt ctgcccccac 180
tacgaaggcc cagggccccc tgagggcccc gagacgtttg ctttgtacat ggtggactgg 240
ccaggctatg agtcctgcca ggcagagggc ccccgggcct acaagcgctg ggtgtgctcc 300
ctgccctttg gccatgttca attctcagag aagattcagc gcttcacacc cttctccctc 360
ggctttgagt tcttacctgg agagacttac tactacatct cggtgcccac tccagagagt 420
tctggccagt gcttgaggct ccaggtgtct gtctgctgca aggagaggaa gtctgagtca 480

gcccatcctg ttgggagccc tggagagagt ggcacatcag ggtggcgagg gggggacact 540
cccagccccc tctgtctctt gctattactg ctgcttctga ttcttcgtct tctgcgaatt 600
ctgtga 606

<210> 42
<211> 201
<212> PRT
<213> Homo sapiens

<400> 42
Met Arg Leu Leu Pro Leu Leu Arg Thr Val Leu Trp Ala Ala Phe Leu
1               5                   10                  15
Gly Ser Pro Leu Arg Gly Gly Ser Ser Leu Arg His Val Val Tyr Trp
            20                  25                  30
Asn Ser Ser Asn Pro Arg Leu Leu Arg Gly Asp Ala Val Val Glu Leu
        35                  40                  45
Gly Leu Asn Asp Tyr Leu Asp Ile Val Cys Pro His Tyr Glu Gly Pro
    50                  55                  60
Gly Pro Pro Glu Gly Pro Glu Thr Phe Ala Leu Tyr Met Val Asp Trp
65                  70                  75                  80
Pro Gly Tyr Glu Ser Cys Gln Ala Glu Gly Pro Arg Ala Tyr Lys Arg
                85                  90                  95
Trp Val Cys Ser Leu Pro Phe Gly His Val Gln Phe Ser Glu Lys Ile
            100                 105                 110
Gln Arg Phe Thr Pro Phe Ser Leu Gly Phe Glu Phe Leu Pro Gly Glu
        115                 120                 125
Thr Tyr Tyr Tyr Ile Ser Val Pro Thr Pro Glu Ser Ser Gly Gln Cys
    130                 135                 140
Leu Arg Leu Gln Val Ser Val Cys Cys Lys Glu Arg Lys Ser Glu Ser
145                 150                 155                 160
Ala His Pro Val Gly Ser Pro Gly Glu Ser Gly Thr Ser Gly Trp Arg
                165                 170                 175
Gly Gly Asp Thr Pro Ser Pro Leu Cys Leu Leu Leu Leu Leu Leu Leu
            180                 185                 190
Leu Ile Leu Arg Leu Leu Arg Ile Leu
        195                 200

<210> 43
<211> 624
<212> DNA
<213> Homo sapiens

<400> 43
atgcggctgc tgcccctgct gcggactgtc ctctgggccg cgttcctcgg ctcccctctg 60
cgcgggggct ccagcctccg ccacgtagtc tactggaact ccagtaaccc caggttgctt 120
cgaggagacg ccgtggtgga gctgggcctc aacgattacc tagacattgt ctgcccccac 180
tacgaaggcc cagggccccc tgagggcccc gagacgtttg ctttgtacat ggtggactgg 240
ccaggctatg agtcctgcca ggcagagggc ccccgggcct acaagcgctg ggtgtgctcc 300
ctgccctttg gccatgttca attctcagag aagattcagc gcttcacacc cttctccctc 360
ggctttgagt tcttacctgg agagacttac tactacatct cggtgcccac tccagagagt 420
tctggccagt gcttgaggct ccaggtgtct gtctgctgca aggagaggag agccagagtc 480
ctcccaagat cccctggagg aggagggatc cctgctgcct gcactggggg tgccaattca 540
gaccgacaag atggagcatt gatgggggag atcagagggt ctgaggtgac tcttgcagga 600
gcctgtcccc tcatcacagg ctaa 624

<210> 44
<211> 207
<212> PRT
<213> Homo sapiens

<400> 44
Met Arg Leu Leu Pro Leu Leu Arg Thr Val Leu Trp Ala Ala Phe Leu
1               5                   10                  15
Gly Ser Pro Leu Arg Gly Gly Ser Ser Leu Arg His Val Val Tyr Trp
            20                  25                  30
Asn Ser Ser Asn Pro Arg Leu Leu Arg Gly Asp Ala Val Val Glu Leu
        35                  40                  45
Gly Leu Asn Asp Tyr Leu Asp Ile Val Cys Pro His Tyr Glu Gly Pro

50                  55                  60
Gly Pro Pro Glu Gly Pro Glu Thr Phe Ala Leu Tyr Met Val Asp Trp
65                  70                  75                  80
Pro Gly Tyr Glu Ser Cys Gln Ala Glu Gly Pro Arg Ala Tyr Lys Arg
                85                  90                  95
Trp Val Cys Ser Leu Pro Phe Gly His Val Gln Phe Ser Glu Lys Ile
            100                 105                 110
Gln Arg Phe Thr Pro Phe Ser Leu Gly Phe Glu Phe Leu Pro Gly Glu
        115                 120                 125
Thr Tyr Tyr Tyr Ile Ser Val Pro Thr Pro Glu Ser Ser Gly Gln Cys
    130                 135                 140
Leu Arg Leu Gln Val Ser Val Cys Cys Lys Glu Arg Arg Ala Arg Val
145                 150                 155                 160
Leu Pro Arg Ser Pro Gly Gly Gly Gly Ile Pro Ala Ala Cys Thr Gly
                165                 170                 175
Gly Ala Asn Ser Asp Arg Gln Asp Gly Ala Leu Met Gly Glu Ile Arg
            180                 185                 190
Gly Ser Glu Val Thr Leu Ala Gly Ala Cys Pro Leu Ile Thr Gly
        195                 200                 205


<210> 45
<211> 645
<212> DNA
<213> Homo sapiens

<400> 45
atgcggctgc tgcccctgct gcggactgtc ctctgggccg cgttcctcgg ctcccctctg 60
cgcgggggct ccagcctccg ccacgtagtc tactggaact ccagtaaccc caggttgctt 120
cgaggagacg ccgtggtgga gctgggcctc aacgattacc tagacattgt ctgcccccac 180
tacgaaggcc cagggccccc tgagggcccc gagacgtttg ctttgtacat ggtggactgg 240
ccaggctatg agtcctgcca ggcagagggc ccccgggcct acaagcgctg ggtgtgctcc 300
ctgccctttg gccatgttca attctcagag aagattcagc gcttcacacc cttctccctc 360
ggctttgagt tcttacctgg agagacttac tactacatct cggtgcccac tccagagagt 420
tctggccagt gcttgaggct ccaggtgtct gtctgctgca aggagaggag accttccctc 480
tcatcccaag gagccagagt cctcccaaga tcccctggag gaggagggat ccctgctgcc 540
tgcactgggg gtgccaattc agaccgacaa gatggagcat tgatggggga gatcagaggg 600
tctgaggtga ctcttgcagg agcctgtccc ctcatcacag gctaa                 645

<210> 46
<211> 214
<212> PRT
<213> Homo sapiens

<400> 46
Met Arg Leu Leu Pro Leu Leu Arg Thr Val Leu Trp Ala Ala Phe Leu
1               5                   10                  15
Gly Ser Pro Leu Arg Gly Gly Ser Ser Leu Arg His Val Val Tyr Trp
            20                  25                  30
Asn Ser Ser Asn Pro Arg Leu Leu Arg Gly Asp Ala Val Val Glu Leu
        35                  40                  45
Gly Leu Asn Asp Tyr Leu Asp Ile Val Cys Pro His Tyr Glu Gly Pro
    50                  55                  60
Gly Pro Pro Glu Gly Pro Glu Thr Phe Ala Leu Tyr Met Val Asp Trp
65                  70                  75                  80
Pro Gly Tyr Glu Ser Cys Gln Ala Glu Gly Pro Arg Ala Tyr Lys Arg
                85                  90                  95
Trp Val Cys Ser Leu Pro Phe Gly His Val Gln Phe Ser Glu Lys Ile
            100                 105                 110
Gln Arg Phe Thr Pro Phe Ser Leu Gly Phe Glu Phe Leu Pro Gly Glu
        115                 120                 125
Thr Tyr Tyr Tyr Ile Ser Val Pro Thr Pro Glu Ser Ser Gly Gln Cys
    130                 135                 140
Leu Arg Leu Gln Val Ser Val Cys Cys Lys Glu Arg Arg Pro Ser Leu
145                 150                 155                 160
Ser Ser Gln Gly Ala Arg Val Leu Pro Arg Ser Pro Gly Gly Gly Gly
                165                 170                 175
Ile Pro Ala Ala Cys Thr Gly Gly Ala Asn Ser Asp Arg Gln Asp Gly
            180                 185                 190

Ala Leu Met Gly Glu Ile Arg Gly Ser Glu Val Thr Leu Ala Gly Ala
        195                 200                 205
Cys Pro Leu Ile Thr Gly
    210

<210> 47
<211> 687
<212> DNA
<213> Homo sapiens

<400> 47
atgttgcacg tggagatgtt gacgctggtg tttctggtgc tctggatgtg tgtgttcagc 60
caggacccgg gctccaaggc cgtcgccgac cgctacgctg tctactggaa cagcagcaac 120
cccagattcc agaggggtga ctaccatatt gatgtctgta tcaatgacta cctggatgtt 180
ttctgccctc actatgagga ctccgtccca gaagataaga ctgagcgcta tgtcctctac 240
atggtgaact ttgatggcta cagtgcctgc gaccacactt ccaaagggtt caagagatgg 300
gaatgtaacc ggcctcactc tccaaatgga ccgctgaagt tctctgaaaa attccagctc 360
ttcactccct tttctctagg atttgaattc aggccaggcc gagaatattt ctacatctcc 420
tctgcaatcc cagataatgg aagaaggtcc tgtctaaagc tcaaagtctt tgtgagacca 480
acaaatagct gtatgaaaac tataggtgtt catgatcgtg ttttcgatgt taacgacaaa 540
gtagaaaatt cattagaacc agcagatgac accgtacatg agtcagccga gccatcccgc 600
ggcgagaacg cggcacaaac accaaggata cccagccgcc ttttggcaat cctactgttc 660
ctcctggcga tgcttttgac attatag                                     687

<210> 48
<211> 228
<212> PRT
<213> Homo sapiens

<400> 48
Met Leu His Val Glu Met Leu Thr Leu Val Phe Leu Val Leu Trp Met
 1               5                  10                  15
Cys Val Phe Ser Gln Asp Pro Gly Ser Lys Ala Val Ala Asp Arg Tyr
            20                  25                  30
Ala Val Tyr Trp Asn Ser Ser Asn Pro Arg Phe Gln Arg Gly Asp Tyr
        35                  40                  45
His Ile Asp Val Cys Ile Asn Asp Tyr Leu Asp Val Phe Cys Pro His
    50                  55                  60
Tyr Glu Asp Ser Val Pro Glu Asp Lys Thr Glu Arg Tyr Val Leu Tyr
65                  70                  75                  80
Met Val Asn Phe Asp Gly Tyr Ser Ala Cys Asp His Thr Ser Lys Gly
                85                  90                  95
Phe Lys Arg Trp Glu Cys Asn Arg Pro His Ser Pro Asn Gly Pro Leu
            100                 105                 110
Lys Phe Ser Glu Lys Phe Gln Leu Phe Thr Pro Phe Ser Leu Gly Phe
        115                 120                 125
Glu Phe Arg Pro Gly Arg Glu Tyr Phe Tyr Ile Ser Ser Ala Ile Pro
    130                 135                 140
Asp Asn Gly Arg Arg Ser Cys Leu Lys Leu Lys Val Phe Val Arg Pro
145                 150                 155                 160
Thr Asn Ser Cys Met Lys Thr Ile Gly Val His Asp Arg Val Phe Asp
                165                 170                 175
Val Asn Asp Lys Val Glu Asn Ser Leu Glu Pro Ala Asp Asp Thr Val
            180                 185                 190
His Glu Ser Ala Glu Pro Ser Arg Gly Glu Asn Ala Ala Gln Thr Pro
        195                 200                 205
Arg Ile Pro Ser Arg Leu Leu Ala Ile Leu Leu Phe Leu Leu Ala Met
    210                 215                 220
Leu Leu Thr Leu
225

<210> 49
<211> 1041
<212> DNA
<213> Homo sapiens

<400> 49

atggctcggc ctgggcagcg ttggctcggc aagtggcttg tggcgatggt cgtgtgggcg 60
ctgtgccggc tcgccacacc gctggccaag aacctggagc ccgtatcctg gagctccctc 120
aaccccaagt tcctgagtgg gaagggcttg gtgatctatc cgaaaattgg agacaagctg 180
gacatcatct gcccccgagc agaagcaggg cggccctatg agtactacaa gctgtacctg 240
gtgcggcctg agcaggcagc tgcctgtagc acagttctcg accccaacgt gttggtcacc 300
tgcaataggc cagagcagga aatacgcttt accatcaagt tccaggagtt cagccccaac 360
tacatgggcc tggagttcaa gaagcaccat gattactaca ttacctcaac atccaatgga 420
agcctggagg ggctggaaaa ccgggagggc ggtgtgtgcc gcacacgcac catgaagatc 480
atcatgaagg ttgggcaaga tcccaatgct gtgacgcctg agcagctgac taccagcagg 540
cccagcaagg aggcagacaa cactgtcaag atggccacac aggcccctgg tagtcggggc 600
tccctgggtg actctgatgg caagcatgag actgtgaacc aggaagagaa gagtggccca 660
ggtgcaagtg gggcagcag cggggaccct gatggcttct tcaactccaa ggtggcattg 720
ttcgcggctg tcggtgccgg ttgcgtcatc ttcctgctca tcatcatctt cctgacggtc 780
ctactactga agctacgcaa gcggcaccgc aagcacacac agcagcgggc ggctgccctc 840
tcgctcagta ccctggccag tcccaagggg ggcagtggca cagcgggcac cgagcccagc 900
gacatcatca ttcccttacg gactacagag aacaactact gcccccacta tgagaaggtg 960
agtggggact acgggcaccc tgtctacatc gtccaagaga tgccgcccca gagcccggcg 1020
aacatctact acaaggtctg a 1041

<210> 50
<211> 346
<212> PRT
<213> Homo sapiens

<400> 50

<210> 51
<211> 1002
<212> DNA
<213> Homo sapiens

<400> 51
atggctgtga gaagggactc cgtgtggaag tactgctggg gtgttttgat ggttttatgc 60
agaactgcga tttccaaatc gatagtttta gagcctatct attggaattc ctcgaactcc 120
aaatttctac ctggacaagg actggtacta tacccacaga taggagacaa attggatatt 180
atttgcccca aagtggactc taaaactgtt ggccagtatg aatattataa agtttatatg 240
gttgataaag accaagcaga cagatgcact attaagaagg aaaatacccc tctcctcaac 300
tgtgccaaac cagaccaaga tatcaaattc accatcaagt ttcaagaatt cagccctaac 360
ctctggggtc tagaatttca gaagaacaaa gattattaca ttatatctac atcaaatggg 420
tctttggagg gcctggataa ccaggaggga ggggtgtgcc agacaagagc catgaagatc 480
ctcatgaaag ttggacaaga tgcaagttct gctggatcaa ccaggaataa agatccaaca 540
agacgtccag aactagaagc tggtacaaat ggaagaagtt cgacaacaag tccctttgta 600
aaaccaaatc caggttctag cacagacggc aacagcgccg gacattcggg gaacaacatc 660
ctcggttccg aagtggcctt atttgcaggg attgcttcag gatgcatcat cttcatcgtc 720
atcatcatca cgctggtggt cctcttgctg aagtaccgga ggagacacag gaagcactcg 780
ccgcagcaca cgaccacgct gtcgctcagc acactggcca cacccaagcg cagcggcaac 840
aacaacggct cagagcccag tgacattatc atcccgctaa ggactgcgga cagcgtcttc 900
tgccctcact acgagaaggt cagcggggac tacgggcacc cggtgtacat cgtccaggag 960
atgcccccgc agagcccggc gaacatttac tacaaggtct ga 1002

<210> 52
<211> 333
<212> PRT
<213> Homo sapiens

<400> 52
Met Ala Val Arg Arg Asp Ser Val Trp Lys Tyr Cys Trp Gly Val Leu
1               5                   10                  15
Met Val Leu Cys Arg Thr Ala Ile Ser Lys Ser Ile Val Leu Glu Pro
            20                  25                  30
Ile Tyr Trp Asn Ser Ser Asn Ser Lys Phe Leu Pro Gly Gln Gly Leu
        35                  40                  45
Val Leu Tyr Pro Gln Ile Gly Asp Lys Leu Asp Ile Ile Cys Pro Lys
    50                  55                  60
Val Asp Ser Lys Thr Val Gly Gln Tyr Glu Tyr Tyr Lys Val Tyr Met
65                  70                  75                  80
Val Asp Lys Asp Gln Ala Asp Arg Cys Thr Ile Lys Lys Glu Asn Thr
                85                  90                  95
Pro Leu Leu Asn Cys Ala Lys Pro Asp Gln Asp Ile Lys Phe Thr Ile
            100                 105                 110
Lys Phe Gln Glu Phe Ser Pro Asn Leu Trp Gly Leu Glu Phe Gln Lys
        115                 120                 125
Asn Lys Asp Tyr Tyr Ile Ile Ser Thr Ser Asn Gly Ser Leu Glu Gly
    130                 135                 140
Leu Asp Asn Gln Glu Gly Gly Val Cys Gln Thr Arg Ala Met Lys Ile
145                 150                 155                 160
Leu Met Lys Val Gly Gln Asp Ala Ser Ser Ala Gly Ser Thr Arg Asn
                165                 170                 175
Lys Asp Pro Thr Arg Arg Pro Glu Leu Glu Ala Gly Thr Asn Gly Arg
            180                 185                 190
Ser Ser Thr Thr Ser Pro Phe Val Lys Pro Asn Pro Gly Ser Ser Thr
        195                 200                 205
Asp Gly Asn Ser Ala Gly His Ser Gly Asn Asn Ile Leu Gly Ser Glu
    210                 215                 220
Val Ala Leu Phe Ala Gly Ile Ala Ser Gly Cys Ile Ile Phe Ile Val
225                 230                 235                 240
Ile Ile Ile Thr Leu Val Val Leu Leu Leu Lys Tyr Arg Arg Arg His
                245                 250                 255
Arg Lys His Ser Pro Gln His Thr Thr Thr Leu Ser Leu Ser Thr Leu
            260                 265                 270
Ala Thr Pro Lys Arg Ser Gly Asn Asn Asn Gly Ser Glu Pro Ser Asp
        275                 280                 285
Ile Ile Ile Pro Leu Arg Thr Ala Asp Ser Val Phe Cys Pro His Tyr

290                 295                 300
Glu Lys Val Ser Gly Asp Tyr Gly His Pro Val Tyr Ile Val Gln Glu
305                 310                 315                 320
Met Pro Pro Gln Ser Pro Ala Asn Ile Tyr Tyr Lys Val
                325                 330

<210> 53
<211> 1023
<212> DNA
<213> Homo sapiens

<400> 53
atggggcccc cccattctgg gccggggggc gtgcgagtcg gggccctgct gctgctgggg 60
gttttggggc tggtgtctgg gctcagcctg gagcctgtct actggaactc ggcgaataag 120
aggttccagg cagagggtgg ttatgtgctg taccctcaga tcggggaccg gctagacctg 180
ctctgccccc gggcccggcc tcctggccct cactcctctc ctaattatga gttctacaag 240
ctgtacctgg tagggggtgc tcagggccgg cgctgtgagg caccccctgc cccaaacctc 300
cttctcactt gtgatcgccc agacctggat ctccgcttca ccatcaagtt ccaggagtat 360
agccctaatc tctggggcca cgagttccgc tcgcaccacg attactacat cattgccaca 420
tcggatggga cccgggaggg cctggagagc ctgcagggag gtgtgtgcct aaccagaggc 480
atgaaggtgc ttctccgagt gggacaaagt ccccgaggag gggctgtccc ccgaaaacct 540
gtgtctgaaa tgcccatgga aagagaccga ggggcagccc acagcctgga gcctgggaag 600
gagaacctgc caggtgaccc caccagcaat gcaacctccc ggggtgctga aggccccctg 660
ccccctccca gcatgcctgc agtggctggg gcagcagggg ggctggcgct gctcttgctg 720
ggcgtggcag gggctggggg tgccatgtgt tggcggagac ggcgggccaa gccttcggag 780
agtcgccacc ctggtcctgg ctccttcggg aggggagggt ctctgggcct ggggggtgga 840
ggtgggatgg gacctcggga ggctgagcct ggggagctag ggatagctct gcggggtggc 900
ggggctgcag atcccccctt ctgcccccac tatgagaagg tgagtggtga ctatgggcat 960
cctgtgtata tcgtgcagga tgggcccccc cagagccctc caaacatcta ctacaaggta 1020
tga                                                               1023

<210> 54
<211> 340
<212> PRT
<213> Homos sapiens

<400> 54
Met Gly Pro Pro His Ser Gly Pro Gly Gly Val Arg Val Gly Ala Leu
1               5                   10                  15
Leu Leu Leu Gly Val Leu Gly Leu Val Ser Gly Leu Ser Leu Glu Pro
            20                  25                  30
Val Tyr Trp Asn Ser Ala Asn Lys Arg Phe Gln Ala Glu Gly Gly Tyr
        35                  40                  45
Val Leu Tyr Pro Gln Ile Gly Asp Arg Leu Asp Leu Leu Cys Pro Arg
    50                  55                  60
Ala Arg Pro Pro Gly Pro His Ser Ser Pro Asn Tyr Glu Phe Tyr Lys
65                  70                  75                  80
Leu Tyr Leu Val Gly Gly Ala Gln Gly Arg Arg Cys Glu Ala Pro Pro
                85                  90                  95
Ala Pro Asn Leu Leu Leu Thr Cys Asp Arg Pro Asp Leu Asp Leu Arg
            100                 105                 110
Phe Thr Ile Lys Phe Gln Glu Tyr Ser Pro Asn Leu Trp Gly His Glu
        115                 120                 125
Phe Arg Ser His His Asp Tyr Tyr Ile Ile Ala Thr Ser Asp Gly Thr
    130                 135                 140
Arg Glu Gly Leu Glu Ser Leu Gln Gly Gly Val Cys Leu Thr Arg Gly
145                 150                 155                 160
Met Lys Val Leu Leu Arg Val Gly Gln Ser Pro Arg Gly Gly Ala Val
                165                 170                 175
Pro Arg Lys Pro Val Ser Glu Met Pro Met Glu Arg Asp Arg Gly Ala
            180                 185                 190
Ala His Ser Leu Glu Pro Gly Lys Glu Asn Leu Pro Gly Asp Pro Thr
        195                 200                 205
Ser Asn Ala Thr Ser Arg Gly Ala Glu Gly Pro Leu Pro Pro Pro Ser
    210                 215                 220
Met Pro Ala Val Ala Gly Ala Ala Gly Gly Leu Ala Leu Leu Leu Leu
225                 230                 235                 240
Gly Val Ala Gly Ala Gly Gly Ala Met Cys Trp Arg Arg Arg Arg Ala

245                         250                         255
Lys Pro Ser Glu Ser Arg His Pro Gly Pro Gly Ser Phe Gly Arg Gly
            260                         265                         270
Gly Ser Leu Gly Leu Gly Gly Gly Gly Gly Met Gly Pro Arg Glu Ala
        275                         280                         285
Glu Pro Gly Glu Leu Gly Ile Ala Leu Arg Gly Gly Gly Ala Ala Asp
    290                         295                         300
Pro Pro Phe Cys Pro His Tyr Glu Lys Val Ser Gly Asp Tyr Gly His
305                         310                         315                         320
Pro Val Tyr Ile Val Gln Asp Gly Pro Pro Gln Ser Pro Pro Asn Ile
                        325                         330                         335
Tyr Tyr Lys Val
            340


<210> 55
<211> 9
<212> PRT
<213> Homo sapiens

<400> 55
Ile Met Asn Asp Met Pro Ile Tyr Met
 1               5


<210> 56
<211> 9
<212> PRT
<213> Homo sapiens

<400> 56
Val Leu Ala Gly Val Gly Phe Phe Ile
 1               5


<210> 57
<211> 9
<212> PRT
<213> Homo sapiens

<400> 57
Thr Leu Ala Asp Phe Asp Pro Arg Val
 1               5


<210> 58
<211> 9
<212> PRT
<213> Homo sapiens

<400> 58
Val Leu Leu Leu Val Leu Ala Gly Val
 1               5


<210> 59
<211> 9
<212> PRT
<213> Homo sapiens

<400> 59
Val Leu Ala Gly Val Gly Phe Phe Ile
 1               5


<210> 60
<211> 9
<212> PRT
<213> Homo sapiens

<400> 60
Ile Met Asn Asp Met Pro Ile Tyr Met
1               5

<210> 61
<211> 9
<212> PRT
<213> Homo sapiens

<400> 61
Ser Leu Leu Gly Leu Lys Asp Gln Val
1               5

<210> 62
<211> 9
<212> PRT
<213> Homo sapiens

<400> 62
Trp Leu Val Pro Ile Gly Gln Cys Leu
1               5

<210> 63
<211> 9
<212> PRT
<213> Homo sapiens

<400> 63
Leu Leu Trp Gly Cys Ala Leu Ala Ala
1               5

<210> 64
<211> 9
<212> PRT
<213> Homo sapiens

<400> 64
Gly Leu Thr Arg Thr Ser Val Thr Val
1               5

<210> 65
<211> 9
<212> PRT
<213> Homo sapiens

<400> 65
Asn Leu Tyr Tyr Ala Glu Ser Asp Leu
1               5

<210> 66
<211> 9
<212> PRT
<213> Homo sapiens

<400> 66
Lys Leu Asn Val Glu Glu Arg Ser Val
1               5

<210> 67
<211> 9
<212> PRT

<213> Homo sapiens

<400> 67
Ile Met Gly Gln Phe Ser His His Asn
1               5


<210> 68
<211> 9
<212> PRT
<213> Homo sapiens

<400> 68
Tyr Ser Val Cys Asn Val Met Ser Gly
1               5


<210> 69
<211> 9
<212> PRT
<213> Homo sapiens

<400> 69
Met Gln Asn Ile Met Asn Asp Met Pro
1               5


<210> 70
<211> 15
<212> PRT
<213> Homo sapiens

<400> 70
Glu Ala Gly Ile Met Gly Gln Phe Ser His His Asn Ile Ile Arg
1               5                   10                  15


<210> 71
<211> 13
<212> PRT
<213> Homo sapiens

<400> 71
Pro Ile Tyr Met Tyr Ser Val Cys Asn Val Met Ser Gly
1               5                   10


<210> 72
<211> 16
<212> PRT
<213> Homo sapiens

<400> 72
Asp Leu Met Gln Asn Ile Met Asn Asp Met Pro Ile Tyr Met Tyr Ser
1               5                   10                  15


<210> 73
<211> 15
<212> PRT
<213> Homo sapiens

<400> 73
Gly Gly Gly Gly Ser Gly Gly Gly Gly Ser Gly Gly Gly Gly Ser
1               5                   10                  15


<210> 74
<211> 15

<212> PRT
<213> Homo sapiens

<400> 74
Glu Ser Gly Arg Ser Gly Gly Gly Gly Ser Gly Gly Gly Gly Ser
1               5                   10                  15


<210> 75
<211> 14
<212> PRT
<213> Homo sapiens

<400> 75
Glu Gly Lys Ser Ser Gly Ser Gly Ser Glu Ser Lys Ser Thr
1               5                   10


<210> 76
<211> 15
<212> PRT
<213> Homo sapiens

<400> 76
Glu Gly Lys Ser Ser Gly Ser Gly Ser Glu Ser Lys Ser Thr Gln
1               5                   10                  15


<210> 77
<211> 14
<212> PRT
<213> Homo sapiens

<400> 77
Glu Gly Lys Ser Ser Gly Ser Gly Ser Glu Ser Lys Val Asp
1               5                   10


<210> 78
<211> 14
<212> PRT
<213> Homo sapiens

<400> 78
Gly Ser Thr Ser Gly Ser Gly Lys Ser Ser Glu Gly Lys Gly
1               5                   10


<210> 79
<211> 18
<212> PRT
<213> Homo sapiens

<400> 79
Lys Glu Ser Gly Ser Val Ser Ser Glu Gln Leu Ala Gln Phe Arg Ser
1               5                   10                  15
Leu Asp


<210> 80
<211> 16
<212> PRT
<213> Homo sapiens

<400> 80
Glu Ser Gly Ser Val Ser Ser Glu Glu Leu Ala Phe Arg Ser Leu Asp
1               5                   10                  15

<210> 81
<211> 4
<212> PRT
<213> Homo sapiens

<400> 81
Lys Asp Glu Leu
1


<210> 82
<211> 4
<212> PRT
<213> Homo sapiens

<400> 82
Asp Asp Glu Leu
1


<210> 83
<211> 4
<212> PRT
<213> Homo sapiens

<400> 83
Asp Glu Glu Leu
1


<210> 84
<211> 4
<212> PRT
<213> Homo sapiens

<400> 84
Gln Glu Asp Leu
1


<210> 85
<211> 4
<212> PRT
<213> Homo sapiens

<400> 85
Arg Asp Glu Leu
1


<210> 86
<211> 7
<212> PRT
<213> Homo sapiens

<400> 86
Pro Lys Lys Lys Arg Lys Val
 1               5


<210> 87
<211> 7
<212> PRT
<213> Homo sapiens

<400> 87
Pro Gln Lys Lys Ile Lys Ser
 1               5

<210> 88
<211> 5
<212> PRT
<213> Homo sapiens

<400> 88
Gln Pro Lys Lys Pro
1               5


<210> 89
<211> 4
<212> PRT
<213> Homo sapiens

<400> 89
Arg Lys Lys Arg
1


<210> 90
<211> 5
<212> PRT
<213> Homo sapiens

<400> 90
Lys Lys Lys Arg Lys
1               5


<210> 91
<211> 12
<212> PRT
<213> Homo sapiens

<400> 91
Arg Lys Lys Arg Arg Gln Arg Arg Arg Ala His Gln
1               5                   10


<210> 92
<211> 16
<212> PRT
<213> Homo sapiens

<400> 92
Arg Gln Ala Arg Arg Asn Arg Arg Arg Arg Trp Arg Glu Arg Gln Arg
1               5                   10                  15


<210> 93
<211> 19
<212> PRT
<213> Homo sapiens

<400> 93
Met Pro Leu Thr Arg Arg Arg Pro Ala Ala Ser Gln Ala Leu Ala Pro
1               5                   10                  15
Pro Thr Pro


<210> 94
<211> 15
<212> PRT
<213> Homo sapiens

<400> 94
Met Asp Asp Gln Arg Asp Leu Ile Ser Asn Asn Glu Gln Leu Pro
 1               5                   10                  15


<210> 95
<211> 32
<212> PRT
<213> Homo sapiens

<220>
<221> VARIANT
<222> (7)...(7)
<223> Xaa can be any naturally ocurring amino acid

<220>
<221> VARIANT
<222> (8)...(8)
<223> Xaa can be any naturally ocurring amino acid

<220>
<221> VARIANT
<222> (32)...(32)
<223> Xaa can be any naturally ocurring amino acid

<400> 95
Met Leu Phe Asn Leu Arg Xaa Xaa Leu Asn Asn Ala Ala Phe Arg His
 1               5                   10                  15
Gly His Asn Phe Met Val Arg Asn Phe Arg Cys Gly Gln Pro Leu Xaa
            20                  25                  30


<210> 96
<211> 3
<212> PRT
<213> Homo sapiens

<400> 96
Ala Lys Leu
1


<210> 97
<211> 6
<212> PRT
<213> Homo sapiens

<400> 97
Ser Asp Tyr Gln Arg Leu
 1               5


<210> 98
<211> 8
<212> PRT
<213> Homo sapiens

<400> 98
Gly Cys Val Cys Ser Ser Asn Pro
 1               5


<210> 99
<211> 8
<212> PRT
<213> Homo sapiens

<400> 99
Gly Gln Thr Val Thr Thr Pro Leu

1               5


<210> 100
<211> 8
<212> PRT
<213> Homo sapiens

<400> 100
Gly Gln Glu Leu Ser Gln His Glu
1               5


<210> 101
<211> 8
<212> PRT
<213> Homo sapiens

<400> 101
Gly Asn Ser Pro Ser Tyr Asn Pro
1               5


<210> 102
<211> 8
<212> PRT
<213> Homo sapiens

<400> 102
Gly Val Ser Gly Ser Lys Gly Gln
1               5


<210> 103
<211> 8
<212> PRT
<213> Homo sapiens

<400> 103
Gly Gln Thr Ile Thr Thr Pro Leu
1               5


<210> 104
<211> 8
<212> PRT
<213> Homo sapiens

<400> 104
Gly Gln Thr Leu Thr Thr Pro Leu
1               5


<210> 105
<211> 8
<212> PRT
<213> Homo sapiens

<400> 105
Gly Gln Ile Phe Ser Arg Ser Ala
1               5


<210> 106
<211> 8
<212> PRT
<213> Homo sapiens

<400> 106

Gly Gln Ile His Gly Leu Ser Pro
1               5

<210> 107
<211> 8
<212> PRT
<213> Homo sapiens

<400> 107
Gly Ala Arg Ala Ser Val Leu Ser
1               5

<210> 108
<211> 8
<212> PRT
<213> Homo sapiens

<400> 108
Gly Cys Thr Leu Ser Ala Glu Glu
1               5

<210> 109
<211> 16
<212> PRT
<213> Homo sapiens

<400> 109
Ala Ala Val Ala Leu Leu Pro Ala Val Leu Leu Ala Leu Leu Ala Pro
1               5                   10                  15

<210> 110
<211> 12
<212> PRT
<213> Homo sapiens

<400> 110
Ala Ala Val Leu Leu Pro Val Leu Leu Ala Ala Pro
1               5                   10

<210> 111
<211> 15
<212> PRT
<213> Homo sapiens

<400> 111
Val Thr Val Leu Ala Leu Gly Ala Leu Ala Gly Val Gly Val Gly
1               5                   10                  15

<210> 112
<211> 3712
<212> DNA
<213> Gallus gallus

<400> 112
cggctctgac tttgtgttaa cggtttatgg actggttcca aagagctcaa aggtaccaaa 60
acactccaag caacctctga accattcaag caagtagtgt gtgtttattg gatatggtgg 120
agtctacaga gaatcttcat ggattctaat gctgacatca gtgcaagaag agtgtcagga 180
atggattggc tctggctggt ttgcttcttt catctagtca cttcactaga agaaatactt 240
ctggatacaa ctggagaaac ctcagagatt ggctggacct ctcaccctcc tgatgggtgg 300
gaagaagtaa gtgtccggga tgataaggag cgccagatcc gaacctttca agtttgtaac 360
atggatgaac caggtcagaa taactggttg cgtactcact tcatagagcg acgtggagcc 420
caccgagtcc atgtccgcct tcatttctca gtgagggact gtgccagcat gcgtactgtg 480
gcctctactt gcaaagagac tttcacactc tactaccacc agtcagatgt cgacatagcc 540

tctcaggaac tgccagagtg gcatgaaggc ccctggacca aggtggatac tattgcagct 600
gatgaaagct tttcccaggt ggacagaact gggaaggtgg taaggatgaa tgttaaagta 660
cgcagctttg ggccactcac aaagcatggc ttctacctgg ccttccagga ctcaggagcc 720
tgtatgtccc tggtggcagt ccaagtcttt ttctacaagt gtccagctgt ggtgaaagga 780
tttgcctcct tccctgaaac ttttgctgga ggagagagga cctcactggt ggagtcacta 840
gggacgtgtg tagcaaatgc tgaagaggca agcacaactg ggtcatcagg tgttcggttg 900
cactgcaatg gagaaggaga gtggatggtg gccactggac gatgctcttg caaggctggt 960
taccaatctg ttgacaatga gcaagcttgt caagcttgtc ccattggttc ctttaaagca 1020
tctgtgggag atgacccttg ccttctctgc cctgcccaca gccatgctcc actgccactg 1080
ccaggttcca ttgaatgtgt gtgtcagagt cactactacc gatctgcttc tgacaattct 1140
gatgctccct gcactggcat cccctctgct ccccgtgacc tcagttatga aattgttggc 1200
tccaacgtgc tcctgacctg gcgcctcccc aaggacttgg gtggccgcaa ggatgtcttc 1260
ttcaatgtca tctgcaagga atgcccaaca aggtcagcag ggacatgtgt gcgctgtggg 1320
gacaatgtac agtttgaacc acgccaagtg ggcctgacag aaagtcgtgt tcaagtctcc 1380
aacctattgg cccgtgtgca gtacactttt gagatccagg ctgtcaattt ggtgactgag 1440
ttgagttcag aagcacccca gtatgctacc atcaacgtta gcaccagcca gtcagtgccc 1500
tccgcaatcc ctatgatgca tcaggtgagt cgtgctacca gtagcatcac actgtcttgg 1560
cctcagccag accagcccaa tggggttatc ctggattacc agctacggta ctttgacaag 1620
gcagaagatg aggataattc atttactttg actagtgaaa ctaacatggc cactatatta 1680
aatctgagtc caggcaagat ctatgtcttc caagtacgag ctagaacagc agtgggttat 1740
ggcccataca gtggaaagat gtatttccag actttaatgg caggagagca ctcggagatg 1800
gcacaggacc gactgccact tattgtgggc tcagcacttg gtggtctggc attcttggta 1860
attgctgcca ttgccattct tgccatcatc ttcaagagta aaaggcgaga gactccatac 1920
acagaccgcc tgcagcagta tatcagtaca cgaggacttg gagtgaagta ttacattgat 1980
ccttccacgt atgaagatcc caatgaagct attcgagagt ttgccaaaga gatagatgtg 2040
tccttcatca aaattgagga ggtcattgga tcaggagaat ttggagaggt gtgctttggg 2100
cgcctaaaac acccagggaa acgtgaatac acagtagcta ttaaaaccct gaagtcaggt 2160
tatactgatg aacagcgtcg agagttcctg agcgaggcca gcatcatggg gcaatttgag 2220
catcccaatg tcatccacct ggagggcgtg gtcaccaaaa gccgaccagt catgattgtc 2280
acagaattca tggagaatgg atcactggat tccttcctca ggcagaagga gggacagttc 2340
agtgtgttac agctggtggg aatgctacga gggattgcag caggcatgcg ctacctttca 2400
gacatgaact atgtgcatcg tgatctcgca gcacgtaaca tcttagtcaa cagtaacctt 2460
gtatgcaagg tgtcagactt tggtttgtct cgctttctgg aagatgatgc ttcaaatccc 2520
acttatactg gagctctggg ttgcaaaatc cccatccgtt ggactgcccc tgaagctgtc 2580
cagtatcgca agttcacctc ctccagtgat gtctggagct atggcattgt catgtgggag 2640
gtgatgtcct atggtgagag accttactgg gacatgtcca accaggatgt aattaatgcc 2700
attgaccagg actatcgcct gccaccaccc ccagactgcc caactgtttt gcatctgctg 2760
atgcttgact gctggcagaa ggatcgagtc cagagaccaa aatttgaaca aatagtcagt 2820
gccctagata aaatgatccg caagccatct gctctcaaag ccactggcac tgggagcagc 2880
agaccatctc agcctctcct gagcaactcc cctccagatt ttccttcact cagcaatgcc 2940
cacgagtggt tggatgccat caagatgggt cgttacaagg agaattttga ccaggctggt 3000
ctgattacat ttgatgtcat atcacgcatg actctggaag atctccagcg tattggaatc 3060
accctggttg gtcaccagaa aaagattcta aacagcatcc agctcatgaa agttcatttg 3120
aaccagcttg aaccagttga agtgtgatgc tttaagtctc tatttcacca gactcaaatt 3180
ctgaaagagt cctgagggga ttcagaggga ttgtcactgt atgaaaagga aatggcaaga 3240
tgctccttga agacttactg cacctagaga gtagacatta cacattccat tccaccagca 3300
aaaagagaat cttgccatca tttaaaagca gagttaaata gctggtggtt aaatatgact 3360
ggcatcatac actaggagta ggtcagggag ggaaagttat agtaatgcag agtggagctg 3420
gtataatagt ttggacagac cacaagcacc tgctagctct tctccactaa ataaaaaatc 3480
agacaattct ccagtgccat cagcaggctt tatctgtgac tgggaacaaa gaaatcacaa 3540
tttttccaag agagtatcag cacattgtga gagttatcac tcagttggaa atggacatca 3600
cttgctatgc cagatttgtg agaaactgga gttccactga gtgcaccata tgtggtaaac 3660
aataaggtac atcacctcgt aatttttaca gaggttgaga gtaaagggcc ca 3712

<210> 113
<211> 1002
<212> PRT
<213> Gallus gallus

<400> 113
Met Asp Ser Asn Ala Asp Ile Ser Ala Arg Arg Val Ser Gly Met Asp
1               5                   10                  15
Trp Leu Trp Leu Val Cys Phe Phe His Leu Val Thr Ser Leu Glu Glu
            20                  25                  30
Ile Leu Leu Asp Thr Thr Gly Glu Thr Ser Glu Ile Gly Trp Thr Ser
        35                  40                  45
His Pro Pro Asp Gly Trp Glu Glu Val Ser Val Arg Asp Asp Lys Glu
    50                  55                  60
Arg Gln Ile Arg Thr Phe Gln Val Cys Asn Met Asp Glu Pro Gly Gln

65                  70                  75                  80
Asn Asn Trp Leu Arg Thr His Phe Ile Glu Arg Arg Gly Ala His Arg
                85                  90                  95
Val His Val Arg Leu His Phe Ser Val Arg Asp Cys Ala Ser Met Arg
            100                 105                 110
Thr Val Ala Ser Thr Cys Lys Glu Thr Phe Thr Leu Tyr Tyr His Gln
        115                 120                 125
Ser Asp Val Asp Ile Ala Ser Gln Glu Leu Pro Glu Trp His Glu Gly
    130                 135                 140
Pro Trp Thr Lys Val Asp Thr Ile Ala Ala Asp Glu Ser Phe Ser Gln
145                 150                 155                 160
Val Asp Arg Thr Gly Lys Val Val Arg Met Asn Val Lys Val Arg Ser
                165                 170                 175
Phe Gly Pro Leu Thr Lys His Gly Phe Tyr Leu Ala Phe Gln Asp Ser
            180                 185                 190
Gly Ala Cys Met Ser Leu Val Ala Val Gln Val Phe Phe Tyr Lys Cys
        195                 200                 205
Pro Ala Val Val Lys Gly Phe Ala Ser Phe Pro Glu Thr Phe Ala Gly
    210                 215                 220
Gly Glu Arg Thr Ser Leu Val Glu Ser Leu Gly Thr Cys Val Ala Asn
225                 230                 235                 240
Ala Glu Glu Ala Ser Thr Thr Gly Ser Ser Gly Val Arg Leu His Cys
                245                 250                 255
Asn Gly Glu Gly Glu Trp Met Val Ala Thr Gly Arg Cys Ser Cys Lys
            260                 265                 270
Ala Gly Tyr Gln Ser Val Asp Asn Glu Gln Ala Cys Gln Ala Cys Pro
        275                 280                 285
Ile Gly Ser Phe Lys Ala Ser Val Gly Asp Asp Pro Cys Leu Leu Cys
    290                 295                 300
Pro Ala His Ser His Ala Pro Leu Pro Leu Pro Gly Ser Ile Glu Cys
305                 310                 315                 320
Val Cys Gln Ser His Tyr Tyr Arg Ser Ala Ser Asp Asn Ser Asp Ala
                325                 330                 335
Pro Cys Thr Gly Ile Pro Ser Ala Pro Arg Asp Leu Ser Tyr Glu Ile
            340                 345                 350
Val Gly Ser Asn Val Leu Leu Thr Trp Arg Leu Pro Lys Asp Leu Gly
        355                 360                 365
Gly Arg Lys Asp Val Phe Phe Asn Val Ile Cys Lys Glu Cys Pro Thr
    370                 375                 380
Arg Ser Ala Gly Thr Cys Val Arg Cys Gly Asp Asn Val Gln Phe Glu
385                 390                 395                 400
Pro Arg Gln Val Gly Leu Thr Glu Ser Arg Val Gln Val Ser Asn Leu
                405                 410                 415
Leu Ala Arg Val Gln Tyr Thr Phe Glu Ile Gln Ala Val Asn Leu Val
            420                 425                 430
Thr Glu Leu Ser Ser Glu Ala Pro Gln Tyr Ala Thr Ile Asn Val Ser
        435                 440                 445
Thr Ser Gln Ser Val Pro Ser Ala Ile Pro Met Met His Gln Val Ser
    450                 455                 460
Arg Ala Thr Ser Ser Ile Thr Leu Ser Trp Pro Gln Pro Asp Gln Pro
465                 470                 475                 480
Asn Gly Val Ile Leu Asp Tyr Gln Leu Arg Tyr Phe Asp Lys Ala Glu
                485                 490                 495
Asp Glu Asp Asn Ser Phe Thr Leu Thr Ser Glu Thr Asn Met Ala Thr
            500                 505                 510
Ile Leu Asn Leu Ser Pro Gly Lys Ile Tyr Val Phe Gln Val Arg Ala
        515                 520                 525
Arg Thr Ala Val Gly Tyr Gly Pro Tyr Ser Gly Lys Met Tyr Phe Gln
    530                 535                 540
Thr Leu Met Ala Gly Glu His Ser Glu Met Ala Gln Asp Arg Leu Pro
545                 550                 555                 560
Leu Ile Val Gly Ser Ala Leu Gly Gly Leu Ala Phe Leu Val Ile Ala
                565                 570                 575
Ala Ile Ala Ile Leu Ala Ile Ile Phe Lys Ser Lys Arg Arg Glu Thr
            580                 585                 590
Pro Tyr Thr Asp Arg Leu Gln Gln Tyr Ile Ser Thr Arg Gly Leu Gly
        595                 600                 605
Val Lys Tyr Tyr Ile Asp Pro Ser Thr Tyr Glu Asp Pro Asn Glu Ala
    610                 615                 620

Ile Arg Glu Phe Ala Lys Glu Ile Asp Val Ser Phe Ile Lys Ile Glu
625                 630                 635                 640
Glu Val Ile Gly Ser Gly Glu Phe Gly Glu Val Cys Phe Gly Arg Leu
                645                 650                 655
Lys His Pro Gly Lys Arg Glu Tyr Thr Val Ala Ile Lys Thr Leu Lys
            660                 665                 670
Ser Gly Tyr Thr Asp Glu Gln Arg Arg Glu Phe Leu Ser Glu Ala Ser
        675                 680                 685
Ile Met Gly Gln Phe Glu His Pro Asn Val Ile His Leu Glu Gly Val
    690                 695                 700
Val Thr Lys Ser Arg Pro Val Met Ile Val Thr Glu Phe Met Glu Asn
705                 710                 715                 720
Gly Ser Leu Asp Ser Phe Leu Arg Gln Lys Glu Gly Gln Phe Ser Val
                725                 730                 735
Leu Gln Leu Val Gly Met Leu Arg Gly Ile Ala Ala Gly Met Arg Tyr
            740                 745                 750
Leu Ser Asp Met Asn Tyr Val His Arg Asp Leu Ala Ala Arg Asn Ile
        755                 760                 765
Leu Val Asn Ser Asn Leu Val Cys Lys Val Ser Asp Phe Gly Leu Ser
    770                 775                 780
Arg Phe Leu Glu Asp Asp Ala Ser Asn Pro Thr Tyr Thr Gly Ala Leu
785                 790                 795                 800
Gly Cys Lys Ile Pro Ile Arg Trp Thr Ala Pro Glu Ala Val Gln Tyr
                805                 810                 815
Arg Lys Phe Thr Ser Ser Ser Asp Val Trp Ser Tyr Gly Ile Val Met
            820                 825                 830
Trp Glu Val Met Ser Tyr Gly Glu Arg Pro Tyr Trp Asp Met Ser Asn
        835                 840                 845
Gln Asp Val Ile Asn Ala Ile Asp Gln Asp Tyr Arg Leu Pro Pro Pro
    850                 855                 860
Pro Asp Cys Pro Thr Val Leu His Leu Leu Met Leu Asp Cys Trp Gln
865                 870                 875                 880
Lys Asp Arg Val Gln Arg Pro Lys Phe Glu Gln Ile Val Ser Ala Leu
                885                 890                 895
Asp Lys Met Ile Arg Lys Pro Ser Ala Leu Lys Ala Thr Gly Thr Gly
            900                 905                 910
Ser Ser Arg Pro Ser Gln Pro Leu Leu Ser Asn Ser Pro Pro Asp Phe
        915                 920                 925
Pro Ser Leu Ser Asn Ala His Glu Trp Leu Asp Ala Ile Lys Met Gly
    930                 935                 940
Arg Tyr Lys Glu Asn Phe Asp Gln Ala Gly Leu Ile Thr Phe Asp Val
945                 950                 955                 960
Ile Ser Arg Met Thr Leu Glu Asp Leu Gln Arg Ile Gly Ile Thr Leu
                965                 970                 975
Val Gly His Gln Lys Lys Ile Leu Asn Ser Ile Gln Leu Met Lys Val
            980                 985                 990
His Leu Asn Gln Leu Glu Pro Val Glu Val
        995                 1000

<210>  114
<211>  121
<212>  PRT
<213>  Artificial

<220>
<223>  recombinant antibody variable region

<400>  114

Gln Val Gln Leu Leu Gln Ser Gly Ala Glu Val Lys Lys Pro Gly Ala
1               5                   10                  15
Ser Val Lys Val Pro Cys Lys Ala Ser Gly Tyr Thr Phe Thr Ser Tyr
            20                  25                  30
Ala Met Ser Trp Val Arg Gln Ala Pro Gly Gln Gly Leu Glu Trp Met
        35                  40                  45
Gly Trp Ile Asn Thr Asn Thr Gly Asn Pro Thr Tyr Ala Gln Gly Phe
    50                  55                  60
Thr Gly Arg Phe Val Phe Ser Leu Asp Thr Ser Val Ser Thr Ala Tyr
65                  70                  75                  80

Leu Gln Ile Ser Ser Leu Lys Ala Glu Asp Thr Ala Val Tyr Tyr Cys
                85                  90                  95
Ala Arg Val Arg Thr Thr Val Tyr Gly Asp Gly Met Asp Val Trp Gly
            100                 105                 110
Gln Gly Thr Leu Val Thr Val Ser Ser
        115                 120


<210>  115
<211>  106
<212>  PRT
<213>  Artificial

<220>
<223>  recombinant antibody variable region

<400>  115

Glu Ile Val Leu Thr Gln Ser Pro Ala Thr Leu Ser Val Ser Pro Gly
1               5                   10                  15
Glu Arg Ala Thr Leu Ser Cys Arg Ala Ser Gln Ser Val Ser Ser Asn
            20                  25                  30
Leu Ala Trp Tyr Gln Gln Lys Pro Gly Gln Ala Pro Arg Leu Leu Ile
        35                  40                  45
Tyr Gly Ala Ser Thr Arg Ala Thr Gly Ile Pro Asp Arg Phe Ser Ala
    50                  55                  60
Ser Gly Ser Gly Thr Asp Phe Thr Leu Thr Ile Ser Arg Val Glu Pro
65                  70                  75                  80
Glu Asp Phe Ala Val Tyr Tyr Cys Gln Gln Tyr Gly Ser Ser Trp Thr
                85                  90                  95
Phe Gly Gln Gly Thr Lys Val Glu Ile Lys
            100                 105


<210>  116
<211>  121
<212>  PRT
<213>  Artificial

<220>
<223>  recombinant antibody variable region

<400>  116

Gln Val Gln Leu Leu Gln Ser Gly Ala Glu Val Lys Lys Pro Gly Ala
1               5                   10                  15
Ser Val Lys Val Pro Cys Lys Ala Ser Gly Tyr Thr Phe Thr Ser Arg
            20                  25                  30
Ala Met Ser Trp Val Arg Gln Ala Pro Gly Gln Gly Leu Glu Trp Met
        35                  40                  45
Gly Trp Ile Asn Thr Asn Thr Gly Asn Pro Thr Tyr Ala Gln Gly Ser
    50                  55                  60
Thr Gly Arg Phe Val Phe Ser Leu Asp Thr Ser Val Ser Thr Ala Tyr
65                  70                  75                  80
Leu Gln Ile Ser Ser Leu Lys Ala Glu Asp Thr Ala Val Tyr Tyr Cys
                85                  90                  95
Ala Arg Val Arg Phe Thr Val Tyr Gly Asp Gly Met Asp Val Trp Gly
            100                 105                 110
Gln Gly Thr Leu Val Thr Val Ser Ser
        115                 120


<210>  117
<211>  106
<212>  PRT
<213>  Artificial

<220>
<223>  recombinant antibody variable region

<400>  117

Glu Ile Val Leu Thr Gln Ser Pro Ala Thr Leu Ser Val Ser Pro Gly
1               5                   10                  15
Glu Arg Ala Thr Leu Ser Cys Arg Ala Ser Gln Ser Val Ser Ser Asn
            20                  25                  30
Pro Ala Trp Tyr Gln Gln Lys Pro Gly Gln Ala Pro Arg Leu Leu Ile
        35                  40                  45
Tyr Gly Ala Ser Thr Arg Ala Thr Gly Ile Pro Asp Arg Phe Ser Ala
    50                  55                  60
Ser Gly Ser Gly Thr Asp Phe Thr Leu Thr Ile Ser Arg Val Glu Pro
65                  70                  75                  80
Glu Asp Phe Ala Val Tyr Tyr Cys Gln Gln Tyr Gly Pro Ser Trp Thr
                85                  90                  95
Phe Gly Gln Gly Thr Lys Val Glu Ile Lys
            100                 105


<210>  118
<211>  121
<212>  PRT
<213>  Artificial

<220>
<223>  recombinant antibody variable region

<400>  118

Gln Val Gln Leu Leu Gln Ser Gly Ala Glu Val Lys Lys Pro Gly Ala
1               5                   10                  15
Ser Val Lys Val Pro Cys Lys Ala Ser Gly Tyr Thr Phe Thr Ser Arg
            20                  25                  30
Ala Met Ser Trp Val Arg Gln Ala Pro Gly Gln Gly Leu Glu Trp Met
        35                  40                  45
Gly Trp Ile Asn Thr Asn Thr Gly Asn Pro Thr Tyr Ala Gln Gly Phe
    50                  55                  60
Thr Gly Arg Phe Val Phe Ser Leu Asp Thr Ser Val Ser Thr Ala Tyr
65                  70                  75                  80
Leu Gln Ile Ser Ser Leu Lys Ala Glu Asp Thr Ala Val Tyr Tyr Cys
                85                  90                  95
Ala Arg Val Arg Phe Thr Val Tyr Gly Asp Gly Met Asp Val Trp Gly
            100                 105                 110
Gln Gly Thr Leu Val Thr Val Ser Ser
        115                 120


<210>  119
<211>  106
<212>  PRT
<213>  Artificial

<220>
<223>  recombinant antibody variable region

<400>  119

Glu Ile Val Leu Thr Gln Ser Pro Ala Thr Leu Ser Val Ser Pro Gly
1               5                   10                  15
Glu Arg Ala Thr Leu Ser Cys Arg Ala Ser Gln Ser Val Ser Ser Asn
            20                  25                  30
Pro Ala Trp Tyr Gln Gln Lys Pro Gly Gln Ala Pro Arg Leu Leu Ile
        35                  40                  45
Tyr Gly Ala Ser Thr Trp Ala Thr Gly Ile Pro Asp Arg Phe Ser Ala
    50                  55                  60
Ser Gly Ser Gly Thr Asp Phe Thr Leu Thr Ile Ser Arg Val Glu Pro
65                  70                  75                  80
Glu Asp Phe Ala Val Tyr Tyr Cys Gln Gln Tyr Gly Pro Ser Trp Thr
                85                  90                  95

Phe Gly Gln Gly Thr Lys Val Glu Ile Lys
            100                 105

<210>  120
<211>  121
<212>  PRT
<213>  Artificial

<220>
<223>  recombinant antibody variable region

<400>  120

Gln Val Gln Leu Leu Gln Ser Gly Ala Glu Val Lys Lys Pro Gly Ala
1               5                   10                  15
Ser Val Lys Val Pro Cys Lys Ala Ser Gly Tyr Thr Phe Thr Ser Arg
            20                  25                  30
Ala Met Ser Trp Val Arg Gln Ala Pro Gly Gln Gly Leu Glu Trp Met
        35                  40                  45
Gly Trp Ile Asn Thr Asn Thr Gly Asn Pro Thr Tyr Ala Gln Gly Phe
    50                  55                  60
Thr Gly Arg Phe Val Phe Ser Leu Asp Thr Ser Val Ser Thr Ala Tyr
65                  70                  75                  80
Leu Gln Ile Ser Ser Leu Lys Ala Glu Asp Thr Ala Val Tyr Tyr Cys
                85                  90                  95
Ala Arg Val Arg Thr Thr Val Tyr Gly Asp Asn Met Asp Val Trp Gly
            100                 105                 110
Gln Gly Thr Leu Val Thr Val Ser Ser
        115                 120

<210>  121
<211>  106
<212>  PRT
<213>  Artificial

<220>
<223>  recombinant antibody variable region

<400>  121

Glu Ile Val Leu Thr Gln Ser Pro Ala Thr Leu Ser Val Ser Pro Gly
1               5                   10                  15
Glu Arg Ala Thr Leu Ser Cys Arg Ala Ser Gln Ser Val Ser Ser Asn
            20                  25                  30
Leu Pro Trp Tyr Gln Gln Lys Pro Gly Gln Ala Pro Arg Leu Leu Ile
        35                  40                  45
Tyr Gly Ala Ser Thr Arg Ala Thr Gly Ile Pro Asp Arg Phe Ser Ala
    50                  55                  60
Ser Gly Ser Gly Thr Asp Phe Thr Leu Thr Ile Ser Arg Val Glu Pro
65                  70                  75                  80
Glu Asp Phe Ala Val Tyr Tyr Cys Gln Gln Tyr Gly Pro Ser Trp Thr
                85                  90                  95
Phe Gly Gln Gly Thr Lys Val Glu Ile Lys
            100                 105

<210>  122
<211>  121
<212>  PRT
<213>  Artificial

<220>
<223>  recombinant antibody variable region

<400>  122

Gln Val Gln Leu Leu Gln Ser Gly Ala Glu Val Lys Lys Pro Gly Ala

1               5                   10                  15
Ser Val Lys Val Pro Cys Lys Ala Ser Gly Tyr Thr Phe Thr Ser Arg
            20                  25                  30
Ala Met Ser Trp Val Arg Gln Ala Pro Gly Gln Gly Leu Glu Trp Met
        35                  40                  45
Gly Trp Ile Asn Thr Asn Thr Gly Asn Pro Thr Tyr Ala Gln Gly Phe
    50                  55                  60
Thr Gly Arg Phe Val Phe Ser Leu Asp Thr Ser Val Ser Thr Ala Tyr
65                  70                  75                  80
Leu Gln Ile Ser Ser Leu Lys Ala Glu Asp Thr Ala Val Tyr Tyr Cys
                85                  90                  95
Ala Arg Val Arg Phe Thr Val Tyr Gly Asp Gly Met Asp Val Trp Gly
            100                 105                 110
Gln Gly Thr Leu Val Thr Val Ser Ser
        115                 120


<210>  123
<211>  106
<212>  PRT
<213>  Artificial

<220>
<223>  recombinant antibody variable region

<400>  123

Glu Ile Val Leu Thr Gln Ser Pro Ala Thr Leu Ser Val Ser Pro Gly
1               5                   10                  15
Glu Arg Ala Thr Leu Ser Cys Arg Ala Ser Gln Ser Val Ser Ser Asn
            20                  25                  30
Leu Pro Trp Tyr Gln Gln Lys Pro Gly Gln Ala Pro Arg Leu Leu Ile
        35                  40                  45
Tyr Gly Ala Ser Thr Arg Pro Thr Gly Ile Pro Asp Arg Phe Ser Ala
    50                  55                  60
Ser Gly Ser Gly Thr Asp Phe Thr Leu Thr Ile Ser Arg Val Glu Pro
65                  70                  75                  80
Glu Asp Phe Ala Val Tyr Tyr Cys Gln Gln Tyr Gly Pro Ser Trp Thr
                85                  90                  95
Phe Gly Gln Gly Thr Lys Val Glu Ile Lys
            100                 105


<210>  124
<211>  121
<212>  PRT
<213>  Artificial

<220>
<223>  recombinant antibody variable region

<400>  124

Gln Val Gln Leu Leu Gln Ser Gly Ala Glu Val Lys Lys Pro Gly Ala
1               5                   10                  15
Ser Val Lys Val Pro Cys Lys Ala Ser Gly Tyr Thr Phe Thr Ser Arg
            20                  25                  30
Ala Met Ser Trp Val Arg Gln Ala Pro Gly Gln Gly Leu Glu Trp Met
        35                  40                  45
Gly Trp Ile Asn Thr Asn Thr Gly Asn Pro Thr Tyr Ala Gln Gly Phe
    50                  55                  60
Thr Gly Arg Phe Val Phe Ser Leu Asp Thr Ser Val Ser Thr Ala Tyr
65                  70                  75                  80
Leu Gln Ile Ser Ser Leu Lys Ala Glu Asp Thr Ala Val Tyr Tyr Cys
                85                  90                  95
Ala Arg Val Arg Phe Thr Val Tyr Gly Asp Gly Met Asp Val Trp Gly
            100                 105                 110
Gln Gly Thr Leu Val Thr Val Ser Ser
        115                 120

<210>  125
<211>  106
<212>  PRT
<213>  Artificial

<220>
<223>  recombinant antibody variable region

<400>  125

Glu Ile Val Leu Thr Gln Ser Pro Ala Thr Leu Ser Val Ser Pro Gly
1               5                   10                  15
Glu Arg Ala Thr Leu Ser Cys Arg Ala Ser Gln Ser Val Ser Ser Asn
            20                  25                  30
Leu Ala Trp Tyr Gln Gln Lys Pro Gly Gln Ala Pro Arg Leu Leu Ile
        35                  40                  45
Tyr Gly Ala Ser Thr Arg Pro Thr Gly Ile Pro Asp Arg Phe Ser Ala
    50                  55                  60
Ser Gly Ser Gly Thr Asp Phe Thr Leu Thr Ile Ser Arg Val Glu Pro
65                  70                  75                  80
Glu Asp Phe Ala Val Tyr Tyr Cys Gln Gln Tyr Gly Pro Ser Trp Thr
                85                  90                  95
Phe Gly Gln Gly Thr Lys Val Glu Ile Lys
            100                 105


<210>  126
<211>  121
<212>  PRT
<213>  Artificial

<220>
<223>  recombinant antibody variable region

<400>  126

Gln Val Gln Leu Leu Gln Ser Gly Ala Glu Val Lys Lys Pro Gly Ala
1               5                   10                  15
Ser Val Lys Val Pro Cys Lys Ala Ser Gly Tyr Thr Phe Thr Ser Arg
            20                  25                  30
Ala Met Ser Trp Val Arg Gln Ala Pro Gly Gln Gly Leu Glu Trp Met
        35                  40                  45
Gly Trp Ile Asn Thr Asn Thr Gly Asn Pro Thr Tyr Ala Gln Gly Phe
    50                  55                  60
Thr Gly Arg Phe Val Phe Ser Leu Asp Thr Ser Val Ser Thr Ala Tyr
65                  70                  75                  80
Leu Gln Ile Ser Ser Leu Lys Ala Glu Asp Thr Ala Val Tyr Tyr Cys
                85                  90                  95
Ala Arg Val Arg Phe Thr Val Tyr Gly Asp Gly Met Asp Val Trp Gly
            100                 105                 110
Gln Gly Thr Leu Val Thr Val Ser Ser
        115                 120


<210>  127
<211>  106
<212>  PRT
<213>  Artificial

<220>
<223>  recombinant antibody variable region

<400>  127

Glu Ile Val Leu Thr Gln Ser Pro Ala Thr Leu Ser Val Ser Pro Gly
1               5                   10                  15
Glu Arg Ala Thr Leu Ser Cys Arg Ala Ser Gln Ser Val Ser Ser Asn

            20                  25                  30
Leu Ala Trp Tyr Gln Gln Lys Pro Gly Gln Ala Pro Arg Leu Leu Ile
        35                  40                  45
Tyr Gly Ala Ser Thr Trp Ala Thr Gly Ile Pro Asp Arg Phe Ser Ala
    50                  55                  60
Ser Gly Ser Gly Thr Asp Phe Thr Leu Thr Ile Ser Arg Val Glu Pro
65                  70                  75                  80
Glu Asp Phe Ala Val Tyr Tyr Cys Gln Gln Tyr Gly Pro Ser Trp Thr
                85                  90                  95
Phe Gly Gln Gly Thr Lys Val Glu Ile Lys
            100                 105

<210> 128
<211> 121
<212> PRT
<213> Artificial

<220>
<223> recombinant antibody variable region

<400> 128

Gln Val Gln Leu Leu Gln Ser Gly Ala Glu Val Lys Lys Pro Gly Ala
1               5                   10                  15
Ser Val Lys Val Pro Cys Lys Ala Ser Gly Tyr Thr Phe Thr Ser Arg
            20                  25                  30
Ala Met Ser Trp Val Arg Gln Ala Pro Gly Gln Gly Leu Glu Trp Met
        35                  40                  45
Gly Trp Ile Asn Thr Asn Thr Gly Asn Pro Thr Tyr Ala Gln Gly Phe
    50                  55                  60
Thr Gly Arg Phe Val Phe Ser Leu Asp Thr Ser Val Ser Thr Ala Tyr
65                  70                  75                  80
Leu Gln Ile Ser Ser Leu Lys Ala Glu Asp Thr Ala Val Tyr Tyr Cys
                85                  90                  95
Ala Arg Val Arg Leu Thr Val Tyr Gly Asp Gly Met Asp Val Trp Gly
            100                 105                 110
Gln Gly Thr Leu Val Thr Val Ser Ser
        115                 120

<210> 129
<211> 106
<212> PRT
<213> Artificial

<220>
<223> recombinant antibody variable region

<400> 129

Glu Ile Val Leu Thr Gln Ser Pro Ala Thr Leu Ser Val Ser Pro Gly
1               5                   10                  15
Glu Arg Ala Thr Leu Ser Cys Arg Ala Ser Gln Ser Val Ser Ser Asn
            20                  25                  30
Pro Pro Trp Tyr Gln Gln Lys Pro Gly Gln Ala Pro Arg Leu Leu Ile
        35                  40                  45
Tyr Gly Ala Ser Thr Arg Ala Thr Gly Ile Pro Asp Arg Phe Ser Ala
    50                  55                  60
Ser Gly Ser Gly Thr Asp Phe Thr Leu Thr Ile Ser Arg Val Glu Pro
65                  70                  75                  80
Glu Asp Phe Ala Val Tyr Tyr Cys Gln Gln Tyr Gly Ser Ser Trp Thr
                85                  90                  95
Phe Gly Gln Gly Thr Lys Val Glu Ile Lys
            100                 105

<210> 130
<211> 121

<212>  PRT
<213>  Artificial

<220>
<223>  recombinant antibody variable region

<400>  130

Gln Val Gln Leu Leu Gln Ser Gly Ala Glu Val Lys Lys Pro Gly Ala
1               5                   10                  15
Ser Val Lys Val Pro Cys Lys Ala Ser Gly Tyr Thr Phe Thr Ser Arg
            20                  25                  30
Ala Met Ser Trp Val Arg Gln Ala Pro Gly Gln Gly Leu Glu Trp Met
        35                  40                  45
Gly Trp Ile Asn Thr Asn Thr Gly Asn Pro Thr Tyr Ala Gln Gly Phe
    50                  55                  60
Thr Gly Arg Phe Val Phe Ser Leu Asp Thr Ser Val Ser Thr Ala Tyr
65                  70                  75                  80
Leu Gln Ile Ser Ser Leu Lys Ala Glu Asp Thr Ala Val Tyr Tyr Cys
                85                  90                  95
Ala Arg Val Arg Phe Thr Val Tyr Gly Asp Gly Met Asp Val Trp Gly
            100                 105                 110
Gln Gly Thr Leu Val Thr Val Ser Ser
        115                 120


<210>  131
<211>  106
<212>  PRT
<213>  Artificial

<220>
<223>  recombinant antibody variable region

<400>  131

Glu Ile Val Leu Thr Gln Ser Pro Ala Thr Leu Ser Val Ser Pro Gly
1               5                   10                  15
Glu Arg Ala Thr Leu Ser Cys Arg Ala Ser Gln Ser Val Ser Ser Asn
            20                  25                  30
Pro Ala Trp Tyr Gln Gln Lys Pro Gly Gln Ala Pro Arg Leu Leu Ile
        35                  40                  45
Tyr Gly Ala Ser Thr Arg Ala Thr Gly Ile Pro Asp Arg Phe Ser Ala
    50                  55                  60
Ser Gly Ser Gly Thr Asp Phe Thr Leu Thr Ile Ser Arg Val Glu Pro
65                  70                  75                  80
Glu Asp Phe Ala Val Tyr Tyr Cys Gln Gln Tyr Gly Pro Ser Trp Thr
                85                  90                  95
Phe Gly Gln Gly Thr Lys Val Glu Ile Lys
            100                 105


<210>  132
<211>  121
<212>  PRT
<213>  Artificial

<220>
<223>  recombinant antibody variable region

<400>  132

Gln Val Gln Leu Leu Gln Ser Gly Ala Glu Val Lys Lys Pro Gly Ala
1               5                   10                  15
Ser Val Lys Val Pro Cys Lys Ala Ser Gly Tyr Thr Phe Thr Ser Arg
            20                  25                  30
Ala Met Ser Trp Val Arg Gln Ala Pro Gly Gln Gly Leu Glu Trp Met
        35                  40                  45
Gly Trp Ile Asn Thr Asn Thr Gly Asn Pro Thr Tyr Ala Gln Gly Phe

50                  55                  60
Thr Gly Arg Phe Val Phe Ser Leu Asp Thr Ser Val Ser Thr Ala Tyr
65                  70                  75                  80
Leu Gln Ile Ser Ser Leu Lys Ala Glu Asp Thr Ala Val Tyr Tyr Cys
                85                  90                  95
Ala Arg Val Arg Leu Thr Val Tyr Gly Asp Gly Met Asp Val Trp Gly
            100                 105                 110
Gln Gly Thr Leu Val Thr Val Ser Ser
        115                 120


<210>  133
<211>  106
<212>  PRT
<213>  Artificial

<220>
<223>  recombinant antibody variable region

<400>  133

Glu Ile Val Leu Thr Gln Ser Pro Ala Thr Leu Ser Val Ser Pro Gly
1               5                   10                  15
Glu Arg Ala Thr Leu Ser Cys Arg Ala Ser Gln Ser Val Ser Ser Asn
            20                  25                  30
Pro Ala Trp Tyr Gln Gln Lys Pro Gly Gln Ala Pro Arg Leu Leu Ile
        35                  40                  45
Tyr Gly Leu Ser Thr Arg Ala Thr Gly Ile Pro Asp Arg Phe Ser Ala
    50                  55                  60
Ser Gly Ser Gly Thr Asp Phe Thr Leu Thr Ile Ser Arg Val Glu Pro
65                  70                  75                  80
Glu Asp Phe Ala Val Tyr Tyr Cys Gln Gln Tyr Gly Pro Ser Trp Thr
                85                  90                  95
Phe Gly Gln Gly Thr Lys Val Glu Ile Lys
            100                 105


<210>  134
<211>  121
<212>  PRT
<213>  Artificial

<220>
<223>  recombinant antibody variable region

<400>  134

Gln Val Gln Leu Leu Gln Ser Gly Ala Glu Val Lys Lys Pro Gly Ala
1               5                   10                  15
Ser Val Lys Val Pro Cys Lys Ala Ser Gly Tyr Thr Phe Thr Ser Tyr
            20                  25                  30
Ala Met Ser Trp Val Arg Gln Ala Pro Gly Gln Gly Leu Glu Trp Met
        35                  40                  45
Gly Trp Ile Asn Thr Asn Thr Gly Asn Pro Thr Tyr Ala Gln Gly Phe
    50                  55                  60
Thr Gly Arg Phe Val Phe Ser Leu Asp Thr Ser Val Ser Thr Ala Tyr
65                  70                  75                  80
Leu Gln Ile Ser Ser Leu Lys Ala Glu Asp Thr Ala Val Tyr Tyr Cys
                85                  90                  95
Ala Arg Val Arg Leu Thr Val Tyr Gly Asp Gly Met Asp Val Trp Gly
            100                 105                 110
Gln Gly Thr Leu Val Thr Val Ser Ser
        115                 120


<210>  135
<211>  106
<212>  PRT
<213>  Artificial

<220>
<223>  recombinant antibody variable region

<400>  135

Glu Ile Val Leu Thr Gln Ser Pro Ala Thr Leu Ser Val Ser Pro Gly
1               5                   10                  15
Glu Arg Ala Thr Leu Ser Cys Arg Ala Ser Gln Ser Val Ser Ser Asn
            20                  25                  30
Leu Ala Trp Tyr Gln Gln Lys Pro Gly Gln Ala Pro Arg Leu Leu Ile
        35                  40                  45
Tyr Gly Ala Ser Thr Arg Ala Thr Gly Ile Pro Asp Arg Phe Ser Ala
    50                  55                  60
Ser Gly Ser Gly Thr Asp Phe Thr Leu Thr Ile Ser Arg Val Glu Pro
65                  70                  75                  80
Glu Asp Phe Ala Val Tyr Tyr Cys Gln Gln Tyr Gly Ser Ser Trp Thr
                85                  90                  95
Phe Gly Gln Gly Thr Lys Val Glu Ile Lys
            100                 105


<210>  136
<211>  5
<212>  PRT
<213>  Artificial

<220>
<223>  recombinant CDR

<400>  136

Ser Tyr Ala Met Ser
1               5


<210>  137
<211>  17
<212>  PRT
<213>  Artificial

<220>
<223>  recombinant CDR

<400>  137

Trp Ile Asn Thr Asn Thr Gly Asn Pro Thr Tyr Ala Gln Gly Phe Thr
1               5                   10                  15
Gly



<210>  138
<211>  12
<212>  PRT
<213>  Artificial

<220>
<223>  recombinant CDR

<400>  138

Val Arg Thr Thr Val Tyr Gly Asp Gly Met Asp Val
1               5                   10


<210>  139
<211>  11
<212>  PRT

<213>  Artificial

<220>
<223>  recombinant CDR

<400>  139

Arg Ala Ser Gln Ser Val Ser Ser Asn Leu Ala
1               5                   10


<210>  140
<211>  7
<212>  PRT
<213>  Artificial

<220>
<223>  recombinant CDR

<400>  140

Gly Ala Ser Thr Arg Ala Thr
1               5


<210>  141
<211>  8
<212>  PRT
<213>  Artificial

<220>
<223>  recombinant CDR

<400>  141

Gln Gln Tyr Gly Ser Ser Trp Thr
1               5


<210>  142
<211>  5
<212>  PRT
<213>  Artificial

<220>
<223>  recombinant CDR

<400>  142

Ser Arg Ala Met Ser
1               5


<210>  143
<211>  17
<212>  PRT
<213>  Artificial

<220>
<223>  recombinant CDR

<400>  143

Trp Ile Asn Thr Asn Thr Gly Asn Pro Thr Tyr Ala Gln Gly Ser Thr
1               5                   10                  15
Gly

<210>  144
<211>  12
<212>  PRT
<213>  Artificial

<220>
<223>  recombinant CDR

<400>  144

Val Arg Phe Thr Val Tyr Gly Asp Gly Met Asp Val
1               5                   10


<210>  145
<211>  11
<212>  PRT
<213>  Artificial

<220>
<223>  recombinant CDR

<400>  145

Arg Ala Ser Gln Ser Val Ser Ser Asn Pro Ala
1               5                   10


<210>  146
<211>  7
<212>  PRT
<213>  Artificial

<220>
<223>  recombinant CDR

<400>  146

Gly Ala Ser Thr Arg Ala Thr
1               5


<210>  147
<211>  8
<212>  PRT
<213>  Artificial

<220>
<223>  recombinant CDR

<400>  147

Gln Gln Tyr Gly Pro Ser Trp Thr
1               5


<210>  148
<211>  5
<212>  PRT
<213>  Artificial

<220>
<223>  recombinant CDR

<400>  148

Ser Arg Ala Met Ser
1               5

<210>  149
<211>  17
<212>  PRT
<213>  Artificial

<220>
<223>  recombinant CDR

<400>  149

Trp Ile Asn Thr Asn Thr Gly Asn Pro Thr Tyr Ala Gln Gly Phe Thr
1               5                   10                  15
Gly



<210>  150
<211>  12
<212>  PRT
<213>  Artificial

<220>
<223>  recombinant CDR

<400>  150

Val Arg Phe Thr Val Tyr Gly Asp Gly Met Asp Val
1               5                   10


<210>  151
<211>  11
<212>  PRT
<213>  Artificial

<220>
<223>  recombinant CDR

<400>  151

Arg Ala Ser Gln Ser Val Ser Ser Asn Pro Ala
1               5                   10


<210>  152
<211>  7
<212>  PRT
<213>  Artificial

<220>
<223>  recombinant CDR

<400>  152

Gly Ala Ser Thr Trp Ala Thr
1               5


<210>  153
<211>  8
<212>  PRT
<213>  Artificial

<220>
<223>  recombinant CDR

<400>  153

Gln Gln Tyr Gly Pro Ser Trp Thr
1               5


<210>  154
<211>  5
<212>  PRT
<213>  Artificial

<220>
<223>  recombinant CDR

<400>  154

Ser Arg Ala Met Ser
1               5


<210>  155
<211>  17
<212>  PRT
<213>  Artificial

<220>
<223>  recombinant CDR

<400>  155

Trp Ile Asn Thr Asn Thr Gly Asn Pro Thr Tyr Ala Gln Gly Phe Thr
1               5                   10                  15
Gly



<210>  156
<211>  12
<212>  PRT
<213>  Artificial

<220>
<223>  recombinant CDR

<400>  156

Val Arg Thr Thr Val Tyr Gly Asp Asn Met Asp Val
1               5                   10


<210>  157
<211>  11
<212>  PRT
<213>  Artificial

<220>
<223>  recombinant CDR

<400>  157

Arg Ala Ser Gln Ser Val Ser Ser Asn Leu Pro
1               5                   10


<210>  158
<211>  7
<212>  PRT
<213>  Artificial

<220>
<223>  recombinant CDR

<400>  158

Gly Ala Ser Thr Arg Ala Thr
1               5


<210>  159
<211>  8
<212>  PRT
<213>  Artificial

<220>
<223>  recombinant CDR

<400>  159

Gln Gln Tyr Gly Pro Ser Trp Thr
1               5


<210>  160
<211>  5
<212>  PRT
<213>  Artificial

<220>
<223>  recombinant CDR

<400>  160

Ser Arg Ala Met Ser
1               5


<210>  161
<211>  17
<212>  PRT
<213>  Artificial

<220>
<223>  recombinant CDR

<400>  161

Trp Ile Asn Thr Asn Thr Gly Asn Pro Thr Tyr Ala Gln Gly Phe Thr
1               5                   10                  15
Gly


<210>  162
<211>  12
<212>  PRT
<213>  Artificial

<220>
<223>  recombinant CDR

<400>  162

Val Arg Phe Thr Val Tyr Gly Asp Gly Met Asp Val
1               5                   10


<210>  163
<211>  11
<212>  PRT
<213>  Artificial

<220>
<223>  recombinant CDR

<400>  163

Arg Ala Ser Gln Ser Val Ser Ser Asn Leu Pro
1               5                   10


<210>  164
<211>  7
<212>  PRT
<213>  Artificial

<220>
<223>  recombinant CDR

<400>  164

Gly Ala Ser Thr Arg Pro Thr
1               5


<210>  165
<211>  8
<212>  PRT
<213>  Artificial

<220>
<223>  recombinant CDR

<400>  165

Gln Gln Tyr Gly Pro Ser Trp Thr
1               5


<210>  166
<211>  5
<212>  PRT
<213>  Artificial

<220>
<223>  recombinant CDR

<400>  166

Ser Arg Ala Met Ser
1               5


<210>  167
<211>  17
<212>  PRT
<213>  Artificial

<220>
<223>  recombinant CDR

<400>  167

Trp Ile Asn Thr Asn Thr Gly Asn Pro Thr Tyr Ala Gln Gly Phe Thr
1               5                   10                  15
Gly



<210>  168

<211>  12
<212>  PRT
<213>  Artificial

<220>
<223>  recombinant CDR

<400>  168

Val Arg Phe Thr Val Tyr Gly Asp Gly Met Asp Val
1               5                   10


<210>  169
<211>  11
<212>  PRT
<213>  Artificial

<220>
<223>  recombinant CDR

<400>  169

Arg Ala Ser Gln Ser Val Ser Ser Asn Leu Ala
1               5                   10


<210>  170
<211>  7
<212>  PRT
<213>  Artificial

<220>
<223>  recombinant CDR

<400>  170

Gly Ala Ser Thr Arg Pro Thr
1               5


<210>  171
<211>  8
<212>  PRT
<213>  Artificial

<220>
<223>  recombinant CDR

<400>  171

Gln Gln Tyr Gly Pro Ser Trp Thr
1               5


<210>  172
<211>  5
<212>  PRT
<213>  Artificial

<220>
<223>  recombinant CDR

<400>  172

Ser Arg Ala Met Ser
1               5

<210>  173
<211>  17
<212>  PRT
<213>  Artificial

<220>
<223>  recombinant CDR

<400>  173

Trp Ile Asn Thr Asn Thr Gly Asn Pro Thr Tyr Ala Gln Gly Phe Thr
1               5                   10                  15

Gly

<210>  174
<211>  12
<212>  PRT
<213>  Artificial

<220>
<223>  recombinant CDR

<400>  174

Val Arg Phe Thr Val Tyr Gly Asp Gly Met Asp Val
1               5                   10

<210>  175
<211>  11
<212>  PRT
<213>  Artificial

<220>
<223>  recombinant CDR

<400>  175

Arg Ala Ser Gln Ser Val Ser Ser Asn Leu Ala
1               5                   10

<210>  176
<211>  7
<212>  PRT
<213>  Artificial

<220>
<223>  recombinant CDR

<400>  176

Gly Ala Ser Thr Trp Ala Thr
1               5

<210>  177
<211>  8
<212>  PRT
<213>  Artificial

<220>
<223>  recombinant CDR

<400>  177

Gln Gln Tyr Gly Pro Ser Trp Thr
1               5


<210>  178
<211>  5
<212>  PRT
<213>  Artificial

<220>
<223>  recombinant CDR

<400>  178

Ser Arg Ala Met Ser
1               5


<210>  179
<211>  17
<212>  PRT
<213>  Artificial

<220>
<223>  recombinant CDR

<400>  179

Trp Ile Asn Thr Asn Thr Gly Asn Pro Thr Tyr Ala Gln Gly Phe Thr
1               5                   10                  15
Gly



<210>  180
<211>  12
<212>  PRT
<213>  Artificial

<220>
<223>  recombinant CDR

<400>  180

Val Arg Leu Thr Val Tyr Gly Asp Gly Met Asp Val
1               5                   10


<210>  181
<211>  11
<212>  PRT
<213>  Artificial

<220>
<223>  recombinant CDR

<400>  181

Arg Ala Ser Gln Ser Val Ser Ser Asn Pro Pro
1               5                   10


<210>  182
<211>  7
<212>  PRT
<213>  Artificial

<220>

<223>  recombinant CDR

<400>  182

Gly Ala Ser Thr Arg Ala Thr
1               5


<210>  183
<211>  8
<212>  PRT
<213>  Artificial

<220>
<223>  recombinant CDR

<400>  183

Gln Gln Tyr Gly Ser Ser Trp Thr
1               5


<210>  184
<211>  5
<212>  PRT
<213>  Artificial

<220>
<223>  recombinant CDR

<400>  184

Ser Arg Ala Met Ser
1               5


<210>  185
<211>  17
<212>  PRT
<213>  Artificial

<220>
<223>  recombinant CDR

<400>  185

Trp Ile Asn Thr Asn Thr Gly Asn Pro Thr Tyr Ala Gln Gly Phe Thr
1               5                   10                  15
Gly



<210>  186
<211>  12
<212>  PRT
<213>  Artificial

<220>
<223>  recombinant CDR

<400>  186

Val Arg Phe Thr Val Tyr Gly Asp Gly Met Asp Val
1               5                   10


<210>  187
<211>  11
<212>  PRT

<213>  Artificial

<220>
<223>  recombinant CDR

<400>  187

Arg Ala Ser Gln Ser Val Ser Ser Asn Pro Ala
1               5                   10


<210>  188
<211>  7
<212>  PRT
<213>  Artificial

<220>
<223>  recombinant CDR

<400>  188

Gly Ala Ser Thr Arg Ala Thr
1               5


<210>  189
<211>  8
<212>  PRT
<213>  Artificial

<220>
<223>  recombinant CDR

<400>  189

Gln Gln Tyr Gly Pro Ser Trp Thr
1               5


<210>  190
<211>  5
<212>  PRT
<213>  Artificial

<220>
<223>  recombinant CDR

<400>  190

Ser Arg Ala Met Ser
1               5


<210>  191
<211>  17
<212>  PRT
<213>  Artificial

<220>
<223>  recombinant CDR

<400>  191

Trp Ile Asn Thr Asn Thr Gly Asn Pro Thr Tyr Ala Gln Gly Phe Thr
1               5                   10                  15
Gly

<210>  192
<211>  12
<212>  PRT
<213>  Artificial

<220>
<223>  recombinant CDR

<400>  192

Val Arg Leu Thr Val Tyr Gly Asp Gly Met Asp Val
1               5                   10


<210>  193
<211>  11
<212>  PRT
<213>  Artificial

<220>
<223>  recombinant CDR

<400>  193

Arg Ala Ser Gln Ser Val Ser Ser Asn Pro Ala
1               5                   10


<210>  194
<211>  7
<212>  PRT
<213>  Artificial

<220>
<223>  recombinant CDR

<400>  194

Gly Leu Ser Thr Arg Ala Thr
1               5


<210>  195
<211>  8
<212>  PRT
<213>  Artificial

<220>
<223>  recombinant CDR

<400>  195

Gln Gln Tyr Gly Pro Ser Trp Thr
1               5


<210>  196
<211>  5
<212>  PRT
<213>  Artificial

<220>
<223>  recombinant CDR

<400>  196

Ser Tyr Ala Met Ser
1               5

<210>  197
<211>  17
<212>  PRT
<213>  Artificial

<220>
<223>  recombinant CDR

<400>  197

Trp Ile Asn Thr Asn Thr Gly Asn Pro Thr Tyr Ala Gln Gly Phe Thr
1               5                   10                  15
Gly



<210>  198
<211>  12
<212>  PRT
<213>  Artificial

<220>
<223>  recombinant CDR

<400>  198

Val Arg Leu Thr Val Tyr Gly Asp Gly Met Asp Val
1               5                   10


<210>  199
<211>  11
<212>  PRT
<213>  Artificial

<220>
<223>  recombinant CDR

<400>  199

Arg Ala Ser Gln Ser Val Ser Ser Asn Leu Ala
1               5                   10


<210>  200
<211>  7
<212>  PRT
<213>  Artificial

<220>
<223>  recombinant CDR

<400>  200

Gly Ala Ser Thr Arg Ala Thr
1               5


<210>  201
<211>  8
<212>  PRT
<213>  Artificial

<220>
<223>  recombinant CDR

<400>  201

Gln Gln Tyr Gly Ser Ser Trp Thr
1               5

<210>  202
<211>  127
<212>  PRT
<213>  Artificial

<220>
<223>  recombinant antibody variable region

<400>  202

Gln Val Gln Leu Gln Glu Ser Gly Pro Gly Leu Val Lys Pro Ser Gly
1               5                   10                  15
Thr Leu Ser Leu Thr Cys Ala Val Ser Gly Gly Ser Ile Ser Ser Ser
            20                  25                  30
Asn Trp Trp Ser Trp Val Arg Gln Pro Pro Gly Lys Gly Leu Glu Trp
        35                  40                  45
Ile Gly Glu Ile Tyr His Ser Gly Ser Thr Asn Tyr Asn Pro Ser Leu
    50                  55                  60
Lys Ser Arg Val Thr Ile Ser Val Asp Lys Ser Lys Asn Gln Phe Ser
65                  70                  75                  80
Leu Lys Leu Ser Ser Val Thr Ala Ala Asp Thr Ala Val Tyr Tyr Cys
                85                  90                  95
Ala Arg Gly Gly Ile Ala Ala Ala Gly Tyr Trp Gly Leu Gly Tyr Asn
            100                 105                 110
Trp Phe Asp Pro Trp Gly Gln Gly Thr Leu Val Thr Val Ser Ser
        115                 120                 125

<210>  203
<211>  381
<212>  DNA
<213>  Artificial

<220>
<223>  recombinant antibody variable region

<400>  203
caggtgcagc tgcaggagtc gggcccagga ctggtgaagc cttcggggac cctgtccctc      60
acctgcgctg tctctggtgg ctccatcagc agtagtaact ggtggagttg ggtccgccag     120
cccccaggga aggggctgga gtggattggg gaaatctatc atagtgggag caccaactac     180
aacccgtccc tcaagagtcg agtcaccata tcagtagaca agtccaagaa ccagttctcc     240
ctgaagctga gctctgtgac cgccgcggac acggccgtgt attactgtgc gagggggggt     300
atagcagcag ctggttactg gggcttgggg tacaactggt tcgacccctg gggccaggga     360
accctggtca ccgtctcctc a                                               381

<210>  204
<211>  112
<212>  PRT
<213>  Artificial

<220>
<223>  recombinant antibody variable region

<400>  204

Gln Ser Val Leu Thr Gln Pro Pro Ser Val Ser Gly Ala Pro Gly Gln
1               5                   10                  15
Arg Val Thr Ile Ser Cys Thr Gly Ser Ser Ser Asn Ile Gly Ala Gly
            20                  25                  30
Tyr Asp Val His Trp Tyr Gln Gln Leu Pro Gly Thr Ala Pro Lys Leu
        35                  40                  45
Leu Ile Tyr Gly Asn Ser Asn Arg Pro Ser Gly Val Pro Asp Arg Phe
    50                  55                  60

Ser Gly Ser Lys Ser Gly Thr Ser Ala Ser Leu Ala Ile Thr Gly Leu
65                  70                  75                  80
Gln Ala Glu Asp Glu Ala Asp Tyr Tyr Cys Gln Ser Tyr Asp Asn Ser
                85                  90                  95
Leu Ser Gly Ser Val Val Phe Gly Gly Gly Thr Lys Leu Thr Val Leu
            100                 105                 110

<210>  205
<211>  336
<212>  DNA
<213>  Artificial

<220>
<223>  recombinant antibody variable region

<400>  205
cagtctgtgt tgacgcagcc gccctcagtg tctggggccc cagggcagag ggtcaccatc      60
tcctgcactg ggagcagctc caacatcggg gcaggttatg atgtacactg gtaccagcag     120
cttccaggaa cagcccccaa actcctcatc tatggtaaca gcaatcggcc ctcaggggtc     180
cctgaccgat tctctggctc caagtctggc acctcagcct ccctggccat cactgggctc     240
caggctgagg atgaggctga ttattactgc cagtcctatg acaacagcct gagtggttcg     300
gtggttttcg gcggagggac caagctgacc gtccta                               336

## Description

This application claims priority to U.S. Provisional Patent Application No. 60/622,711, filed October 27, 2004 and U.S. Provisional Patent Application No. 60/717,209, filed September 16, 2005, each of which is incorporated by reference herein in its entirety.

### 1. FIELD OF THE INVENTION

The present invention relates to methods for modulating antibody specificity by tailoring the affinity of said antibody to a cognate antigen. The present invention further relates to methods and compositions designed for the treatment, management, prevention and/or amelioration of various disorders associated with aberrant expression and/or activity of one or more Eph receptor tyrosine kinase family members and/or one or more Eph receptor ligands, particularly the Ephrins. In particular, the invention provides methods for the treatment, management, prevention and/or amelioration of a disorder associated with aberrant expression and/or activity(ies) of one or more Eph receptors and/or one or more Ephrins, the method comprising administering to a subject in need thereof an effective amount of one or more Eph/Ephrin Modulators. The invention further provides antibodies with tailored affinities to a cognate antigen.

### 2. BACKGROUND OF THE INVENTION

Receptor tyrosine kinases (RTKs) are transmembrane proteins which consist of an extracellular ligand binding domain and an intracellular domain with tyrosin kinase activity (Surawska et al., 2004, Cytokine Growth Factor Rev. 15:419-433). This family of proteins contains over fifty different members that are organized into at least nineteen different classes based on structural organization, and includes receptors for growth factors (*e.g*. EGF, PDGF, FGF) and insulin (Grassot et al, 2003, Nucl Acids Res., 31(1):353-358; Surawska et al., 2004, Cytokine Growth Factor Rev. 15:419-433). Class I RTK's comprise, for example, EGFR, ERBB2, ERBB3 and ERBB4; Class II RTK's comprise, for example, INSR, IRR and IG1R; Class III RTK's comprise, for example, PDGFα, PDGFβ, Fms, Kit and Flt3; Class IV RTK's comprise, for example, FGFR1, FGFR2, FGFR3, FGFR4 and BFR2; Class V RTK's comprise Flt1, Flt2 and Flt4; Class VI RTK's comprise EphA1-EphA8 and EphB1-EphB6; Class VII RTK's comprise TrkA, TrkB and TrkC (Grassot et al., 2003, Nucl Acids Res., 31(1):353-358; Grassot et al., Grassot et al., www.irisa.fr/jobim/papiers/O-p199-012.pdf). Autophosphorylation of the tyrosine residues in the intracellular (cytosolic) domain is induced by ligand binding to the extracellular binding domain, which in turn leads to the formation of signaling complexes and activation of downstream signal transduction cascades (Surawska et al., 2004, Cytokine Growth Factor Rev. 15:419-433).

Eph receptors, the largest subfamily of receptor tyrosine kinases (RTKs), and their ligands, the Ephrins, play critical roles in a diverse array of biological processes during development as well as in the mature animal (for reviews, see, Zhou et al., 1998, Pharmacol. Ther. 77:151-181; Himanen and Nikolov, 2003, Trends in Neurosci. 26:46-51; Murai and Pasquale, 2003, J Cell Sci. 116: 2823-2832; and Kullander and Klein, 2002, Nature Rev. 3:475-486). Eph/Ephrin-mediated signaling plays a role in many important biological functions, including morphogenesis, vascular development, cell migration, axon guidance and synaptic plasticity (Kullander and Klein, 2002, Nature Rev. 3:475-486).
To date, fifteen Eph receptors (EphA1-A8 and EphA10, and EphB1-B6 and 8 Ephrin ligands (EphrinAl-A5 and EphrinB1-B3) have been identified in mammals (see, *e.g.*, "Unified Nomenclature For Eph Family Receptors And Their Ligands, The Ephrins," by the Eph Nomenclature Committee, reproduced in Cell 90:403-404, 1997; Surawska et al., 2004, Cytokine Growth Factor Rev. 15:419-433); Siddiqui and Cramer, 2005, J Comp Neurol. 482(4):309-319; Aasheim et al., 2005, Biochim Biophys Acta 1723(1-3):1-7; and Zhou et al.,1998, Pharmacol. Ther. 77:151-181). Both Eph receptors and Ephrins are divided into two subclasses, A and B, based on sequence conservation and their binding affinities (Eph Nomenclature Committee, 1997, Cell 90:403-404). With the exception of EphA4, which can bind to both A-type and B-type ligands, generally, eight of the identified A-type Eph receptors (EphA1-A8) interact promiscuously (although with varying affinity) with five A-type Ephrins (EFNA1-A5), that are bound to the cell membrane by a glycosylphosphatidylinositol (GPI) anchor (Kullander and Klein, 2002, Nature Rev. 3:475-486). The B-type Eph receptors (EphB1-B6) have been shown to interact with three B-type Ephrins (EFNB1-B3), which are attached to the cell membrane by a hydrophobic transmembrane region and a short cytoplasmic domain (Kullander and Klein, 2002, Nature Rev. 3:475-486).

Eph/Ephrin-mediated signaling is dynamic due to the fact that it is bi-directional (see, *e.g*., Gauthier and Robbins, 2003, Life Sciences 74:207-216; Murai and Pasquale, 2003, J. Cell Sci. 116:2823-2832; Kullander and Klein, 2002, Nature Rev. 3:475-486; and Holder and Klein, 1999, Development 126:2033-2044). Engagement of an Eph receptor by its ligand results in conformational changes in the receptor, and a concomitant activation of the highly conserved Eph tyrosine kinase domain and transduction of the typical receptor forward signal within the receptor-expressing cell. Simultaneously, there is transduction of a reverse signal into the Ephrin-expressing cell. Eph/Ephrin-mediated signaling converges on a number of cell signaling pathways through Eph and/or Ephrin interactions with other signaling adaptor molecules near the cell membrane, including the Src family of kinases involved in mitogen-activated protein kinase (MAPK) pathway signaling; Grb2, which is involved in platelet-derived growth factor (PDGF) and epidermal growth factor (EGF) signaling; phosphatidylinositol 3-kinase (PI3K); Crk, which is involved in Rho-mediated signaling (see, e.g., Kullander and Klein, 2002, Nature Rev. 3:475-486); and low molecular weight phosphotyrosine phosphatase (LMW-PTP), the recruitment of which has been shown to correlate with functional responses such as endothelial capillary-like assembly and cell attachment (Stein et al., 1998, Genes Dev. 12:667-678).

However, it is their role in diseases, particularly cancer, that have become increasingly scrutinized as mounting evidence supports a role for Eph/Ephrin-mediated signaling in disease processes such as angiogenesis, tumorigenesis and metastasis (*see, e.g.*, Sullivan and Bicknell, 2003, British J. Cancer 89:228-231; Cheng et al., 2002, Cytokine & Growth Factor Rev. 13:75-85; Nakamoto and Bergemann, 2002, Microscopy Res. & Technique 59:58-67). Eph receptor expression has been studied in various types of cancers, including but not limited to, breast cancer (Wu et al., 2004, Pathol. Oncol. Res. 10:26-33), colon cancer (Stephenson et al., 2001, BMC Mol. Biol. 2:15-23), osteosarcomas (Varelias et al., 2002, Cancer 95:862-869) and esophageal cancer (Nemoto et al., 1997, Pathobiology 65:195-203). Indeed, the first Eph receptor to be identified, EphA1 was isolated from a human erythropoietin-producing hepatocellular (*eph*) carcinoma cell line (Hirai et al., 1987, Science 238:1717-1720).

### 3. SUMMARY OF THE INVENTION

The invention is based, in part, on the discovery that specific Eph receptor tyrosine kinases and/or their ligands, the Ephrins, are aberrantly expressed (*i.e*., inappropriately expressed, overexpressed or underexpressed) in certain tissues and cell types associated with various disorders, including but not limited to, cancer and inflammation. The present invention takes advantage of the multiple interactions between the Eph receptors and the Ephrins, as well as their interactions with multiple signaling molecules downstream. Exploiting the high conservation in the domains of Eph receptors and Ephrins, and the high degree of functional overlap, the present invention provides therapeutic methods and compositions designed for the treatment, management, prevention and/or amelioration of various disorders associated with aberrant expression and/or activity of one or more Eph receptor tyrosine kinase family members and/or one or more Eph kinase ligands, particularly the Ephrins. The prophylactic and therapeutic methods of the invention comprise the administration of an effective amount of one or more Eph/Ephrin Modulators that preferentially target multiple Eph kinases and/or Ephrins either alone or in combination with other prophylactic or therapeutic agents. In certain embodiments, depending on the disease to be treated, an Eph/Ephrin Modulator of the invention modulates the expression and/or activity of one or more Eph kinases and/or Ephrins to agonize and/or antagonize Eph receptor and/or Ephrin activity and/or stability.

In specific embodiments of the invention, the Eph/Ephrin Modulators of the invention may be designed to bind and/or interact with a unique region of a particular Eph receptor(s) and/or Ephrin(s) to modulate the activity and/or expression of such Eph receptor and/or Ephrin. In another specific embodiment, the Eph/Ephrin Modulators of the invention may be designed to bind and/or interact with regions that are common or homologous between particular Eph receptors and/or Ephrins to simultaneously modulate the activity and/or expression multiple Eph receptors and/or Ephrins in accordance with the methods of the present invention.

The invention provides modulators of Eph receptors and/or Ephrins ("Eph/Ephrin Modulators"). Non-limiting examples of Eph/Ephrin Modulators are agents that confer a biological effect by modulating (directly or indirectly): (i) the expression of an Eph receptor (*e.g*., EphA1, EphA2, EphA3, EphA4, EphA2, EphA6, EphA7, EphA8, EphA10, EphB1, EphB2, EphB3, EphB4, EphB5 or EphB6) and/or an endogenous ligand(s) of an Eph receptor (*e.g*., EphrinA1, EphrinA2, EphrinA3, EphrinA4, EphrinA5, EphrinB1, EphrinB2 or EphrinB3), at, *e.g*., the transcriptional, post-transcriptional, translational or post-translation level; and/or (ii) an activity(ies) of an Eph receptor and/or an Ephrin.

Examples of Eph/Ephrin Modulators include, but are not limited to, agents that inhibit or reduce the interaction between an Eph receptor and an endogenous ligand(s) of the Eph receptor, for example, an Ephrin (hereinafter "Eph/Ephrin Interaction Inhibitors"). Non-limiting examples of Eph/Ephrin Interaction Inhibitors include: (i) agents that bind to an Eph receptor, prevent or reduce the interaction between the Eph receptor and an Ephrin, and induce Eph receptor signal transduction (*e.g*., soluble forms of an Ephrin (*e.g*., in monomeric or multimeric form), and antibodies that bind to an Eph receptor, induce signaling and phosphorylation of the Eph receptor (*i.e*., an Eph receptor agonistic antibody)); (ii) agents that bind to an Eph receptor, prevent or reduce the interaction between the Eph receptor and an Ephrin, and prevent or induce very low to negligible levels of Eph receptor signal transduction (*e.g*., Eph receptor antagonistic antibodies and dominant negative forms of an Ephrin); (iii) agents that bind to an Ephrin, prevent or reduce the interaction between an Eph receptor and the Ephrin, and induce Ephrin signal transduction (*e.g*., soluble forms of an Eph receptor and antibodies that bind to an Ephrin and induce Ephrin signal transduction); and (iv) agents that bind to an Ephrin, prevent or reduce the interaction between an Eph receptor and the Ephrin, and prevent or induce very low to negligible levels of Ephrin signal transduction (*e.g*., dominant negative forms of an Eph receptor and anti-Ephrin antibodies).

In further embodiments, Eph/Ephrin Modulators include, but are not limited to, agents that modulate the expression of an Eph receptor. Such agents can decrease/downregulate Eph receptor expression (*e.g*., Eph receptor antisense molecules, RNAi and ribozymes) or increase/upregulate Eph receptor expression such that the amount of an Eph receptor on the cell surface exceeds the amount of endogenous ligand (*e.g.*, an Ephrin) available for binding, and thus, increases the amount of unbound Eph receptor (*e.g*., nucleic acids encoding an Eph receptor)).

In other embodiments, Eph/Ephrin Modulators are agents that modulate the expression of an Ephrin. Such agents can decrease/downregulate Ephrin expression (*e.g*., Ephrin antisense molecules, RNAi and ribozymes) or increase/upregulate Ephrin expression (*e.g*., nucleic acids encoding an Ephrin)).

In yet other embodiments, Eph/Ephrin Modulators of the invention include, but are not limited to, agents that modulate the protein stability or protein accumulation of an Eph receptor or an Ephrin.

In further embodiments, Eph/Ephrin Modulators of the invention are agents that promote kinase activity (*e.g*., of an Eph receptor, an Ephrin or of a heterologous protein known to associate with the Eph receptor or Ephrin at the cell membrane).

In yet further embodiments, Eph/Ephrin Modulators include, but are not limited to, agents that bind to an Eph receptor and prevent or reduce Eph receptor signal transduction but do not inhibit or reduce the interaction between the Eph receptor and an Ephrin (*e.g.*, an Eph receptor intrabody); and agents that bind to an Ephrin and prevent or reduce Ephrin signal transduction but do not inhibit or reduce the interaction between an Ephrin and an Eph receptor (*e.g.*, a B-type Ephrin intrabody).

In a specific embodiment, an Eph/Ephrin Modulator is not an agent that inhibits or reduces the interaction between an Eph receptor and an endogenous ligand, for example, an Ephrin. In another specific embodiment, an Eph/Ephrin Modulator is not an agent that modulates (increases or decreases) the expression of an Eph receptor and/or of an Ephrin. In a further embodiment, an Eph/Ephrin Modulator is not an agent that modulates the protein stability or protein accumulation of an Eph receptor or an Ephrin. In yet a further embodiment of the invention, an Eph/Ephrin Modulator is not an agent that promotes kinase activity (*e.g*., of an Eph receptor, an Ephrin or of a heterologous protein known to associate with an Eph receptor or Ephrin at the cell membrane). In another specific embodiment, an Eph/Ephrin Modulator is not an agent that binds to an Eph receptor and prevents or reduces Eph receptor signal transduction but does not prevent the interaction between the Eph receptor and an Ephrin; or an agent that binds to an Ephrin and prevents or reduces Ephrin signal transduction but does not prevent the interaction between the Ephrin and an Eph receptor. In a specific embodiment, an Eph/Ephrin Modulator is not an EphA2 agonistic antibody or an EphA2 antagonistic antibody. In a further embodiment, an Eph/Ephrin Modulator is not an Eph receptor antisense molecule or an Ephrin antisense molecule. In yet a further embodiment, an Eph/Ephrin Modulator is not a soluble form of an Eph receptor (*e.g*., Eph-Fc) or is not a soluble form of an Ephrin (*e.g*., Ephrin-Fc).

The invention is further based, in part, on the discovery that, given a pan-specific antibody to a member or class of the receptor tyrosine kinase (RTK) family of receptors (*e.g*., the Eph family of receptors), the specificity of that antibody can be tailored to alter the specificity to those cognate antigens within the same RTK family or class. In one embodiment, the invention provides a method of tailoring the specificity of a pan-specific antibody that specifically binds at least two members within a receptor tyrosine kinase family, said method comprising randomizing each amino acid of the CDRs of said pan-specific antibody; generating a library comprising said randomized CDRs; screening said library for clones with increased or decreased affinity for a desired receptor(s).

In another embodiment, the invention further provides a monoclonal antibody that specifically binds at least one member of the Eph family of receptors, said monoclonal antibody being tailored to have a desired affinity(ies) towards said at least one member of the Eph family.

### 3.1 DEFINITIONS

As used herein, the term "aberrant" refers to a deviation from the norm, *e.g*., the average healthy subject or cell and/or a population of average healthy subjects or cells. The term "aberrant expression," as used herein, refers to abnormal expression of a gene product (*e.g*., RNA, protein, polypeptide, or peptide) by a cell or subject relative to a normal, healthy cell or subject and/or a population of normal, healthy cells or subjects. Such aberrant expression may be the result of the amplification of a gene or the inhibition of the expression of a gene. In a specific embodiment, "aberrant expression" with respect to an Eph receptor or Ephrin refers to an increase, decrease, or inappropriate expression of one or more Eph receptors and/or Ephrins. In specific embodiments, the term "aberrant activity" refers to an Eph receptor or Ephrin activity that deviates from that normally found in a healthy cell or subject and/or a population of normal, healthy cells or subjects.

As used herein, the term "agent" refers to a molecule that has a desired biological effect. An agent can be prophylactic or therapeutic. Agents include, but are not limited to, proteinaceous molecules, including, but not limited to, peptides, polypeptides, proteins, including post-translationally modified proteins, fusion proteins, antibodies, etc.; small molecules (less than 1000 daltons), including inorganic or organic compounds; nucleic acid molecules including, but not limited to, double-stranded or single-stranded DNA, or double-stranded or single-stranded RNA (*e.g*., antisense, RNAi, etc.), intron sequences, triple helix nucleic acid molecules and aptamers; or vaccines (*e.g., Listeria*-based and non-*Listeria*-based vaccines). Agents can be derived from any known organism (including, but not limited to, animals, plants, bacteria, fungi, and protista, or viruses) or from a library of synthetic molecules. Agents that are Eph/Ephrin Modulators modulate (directly or indirectly): (i) the expression (*e.g*., at the transcriptional, post-transcriptional, translational or post-translation level) of an Eph receptor, for example, EphA1, EphA2, EphA3, EphA4, EphA5, EphA6, EphA7, EphA8, EphA10, EphB1, EphB2, EphB3, EphB4, EphB5 or EphB6 and/or an endogenous ligand(s) of an Eph receptor, for example, EphrinA1 EphrinA2, EphrinA3, EphrinA4, EphrinA5, EphrinB1, EphrinB2 or EphrinB3; and/or (ii) an activity(ies) of an Eph receptor and/or an endogenous ligand(s) of an Eph receptor, for example, EphrinA1, EphrinA2, EphrinA3, EphrinA4, EphrinA5, EphrinB1, EphrinB2 or EphrinB3.

As used herein, the term "analog" in the context of a proteinaceous agent (*e.g*., a peptide, polypeptide, protein or antibody) refers to a proteinaceous agent that possesses a similar or identical function as a second proteinaceous agent (*e.g*., an Eph receptor polypeptide or an Ephrin polypeptide) but does not necessarily comprise a similar or identical amino acid sequence or structure of the second proteinaceous agent. A proteinaceous agent that has a similar amino acid sequence refers to a proteinaceous agent that satisfies at least one of the following: (a) a proteinaceous agent having an amino acid sequence that is at least 30%, at least 35%, at least 40%, at least 45%, at least 50%, at least 55%, at least 60%, at least 65%, at least 70%, at least 75%, at least 80%, at least 85%, at least 90%, at least 95% or at least 99% identical to the amino acid sequence of a second proteinaceous agent; (b) a proteinaceous agent encoded by a nucleotide sequence that hybridizes under stringent conditions to a nucleotide sequence encoding a second proteinaceous agent of at least 20 amino acid residues, at least 30 amino acid residues, at least 40 amino acid residues, at least 50 amino acid residues, at least 60 amino residues, at least 70 amino acid residues, at least 80 amino acid residues, at least 90 amino acid residues, at least 100 amino acid residues, at least 125 amino acid residues, or at least 150 amino acid residues; and (c) a proteinaceous agent encoded by a nucleotide sequence that is at least 30%, at least 35%, at least 40%, at least 45%, at least 50%, at least 55%, at least 60%, at least 65%, at least 70%, at least 75%, at least 80%, at least 85%, at least 90%, at least 95% or at least 99% identical to the nucleotide sequence encoding a second proteinaceous agent. A proteinaceous agent with similar structure to a second proteinaceous agent refers to a proteinaceous agent that has a similar secondary, tertiary or quaternary structure of the second proteinaceous agent. The structure of a proteinaceous agent can be determined by methods known to those skilled in the art, including but not limited to, X-ray crystallography, nuclear magnetic resonance, and crystallographic electron microscopy. Preferably, the proteinaceous agent has Eph receptor or Ephrin activity.

To determine the percent identity of two amino acid sequences or of two nucleic acid sequences, the sequences are aligned for optimal comparison purposes (*e.g*., gaps can be introduced in the sequence of a first amino acid or nucleic acid sequence for optimal alignment with a second amino acid or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences (*i.e*., % identity = number of identical overlapping positions/total number of positions x 100%). In one embodiment, the two sequences are the same length.

The determination of percent identity between two sequences can also be accomplished using a mathematical algorithm. A preferred, non-limiting example of a mathematical algorithm utilized for the comparison of two sequences is the algorithm of Karlin and Altschul, 1990, Proc. Natl. Acad. Sci. U.S.A. 87: 2264-2268, modified as in Karlin and Altschul, 1993, Proc. Natl. Acad. Sci. U.S.A. 90: 5873-5877. Such an algorithm is incorporated into the NBLAST and XBLAST programs of Altschul et al., 1990, J. Mol. Biol. 215: 403. BLAST nucleotide searches can be performed with the NBLAST nucleotide program parameters set, *e.g*., for score=100, wordlength=12 to obtain nucleotide sequences homologous to a nucleic acid molecules of the present invention. BLAST protein searches can be performed with the XBLAST program parameters set, *e.g*., to score-50, wordlength=3 to obtain amino acid sequences homologous to a protein molecule of the present invention. To obtain gapped alignments for comparison purposes, Gapped BLAST can be utilized as described in Altschul et al., 1997, Nucleic Acids Res. 25: 3389-3402. Alternatively, PSI-BLAST can be used to perform an iterated search which detects distant relationships between molecules (*Id*.). When utilizing BLAST, Gapped BLAST; and PSI-Blast programs, the default parameters of the respective programs (*e.g*., of XBLAST and NBLAST) can be used (see, *e.g*., the NCBI website). Another preferred, non-limiting example of a mathematical algorithm utilized for the comparison of sequences is the algorithm of Myers and Miller, 1988, CABIOS 4: 11-17. Such an algorithm is incorporated in the ALIGN program (version 2.0) which is part of the GCG sequence alignment software package. When utilizing the ALIGN program for comparing amino acid sequences, a PAM120 weight residue table, a gap length penalty of 12, and a gap penalty of 4 can be used.

The percent identity between two sequences can be determined using techniques similar to those described above, with or without allowing gaps. In calculating percent identity, typically only exact matches are counted.

As used herein, the term "analog" in the context of a non-proteinaceous analog refers to a second organic or inorganic molecule which possesses a similar or identical function as a first organic or inorganic molecule and is structurally similar to the first organic or inorganic molecule.

As used herein, the term "antibodies that specifically bind to an Eph receptor" and analogous terms refer to antibodies that specifically bind to an Eph receptor (*e.g*., EphA1, EphA2, EphA3, EphA4, EphA2, EphA6, EphA7, EphA8, EphA10, EphB1, EphB2, EphB3, EphB4, EphB5 and EphB6) polypeptide or a fragment of an Eph receptor polypeptide, and do not specifically bind to non-Eph receptor polypeptides (or in certain specific embodiments, do not specifically bind to other Eph receptors). Antibodies that specifically bind to an Eph receptor polypeptide or a fragment thereof do not cross-react with other antigens outside of the Eph receptor family. Antibodies that specifically bind to an Eph receptor polypeptide or a fragment thereof can be identified, for example, by immunoassays or other techniques known to those of skill in the art. In one embodiment, antibodies of the invention that specifically bind to an Eph receptor polypeptide or a fragment thereof only modulate an activity(ies) of the Eph receptor and do not significantly affect other activities. In a specific embodiment, antibodies of the invention specifically bind one or more members within a receptor tyrosine kinase family or class. In a further specific embodiment, antibodies of the invention specifically bind all members of the Eph family of receptors. In another specific embodiment, antibodies of the invention specifically bind all members of the EphA family of receptors. In another specific embodiment, antibodies of the invention specifically bind at least one, at least two, at least three, at least four, at least five, at least six, at least seven, at least eight, at least nine, at least ten, at least eleven, at least twelve, at least thirteen, at least fourteen, or at least fifteen members of the Eph receptor family.

The specific binding of the antibodies of the invention to one or more members of the receptor tyrosine kinase family or class of receptors (*e.g*. the Eph receptor family) is not to be confused with the term "cross react" with regard to binding specificity. The term "cross-react" can be understood to mean nonspecific binding that is undesirable. In certain embodiments, the antibodies of the invention bind to several different distinct members within the Eph family of receptors. In certain embodiments this property is a desired characteristic of the antibodies of the present invention, and their binding (to one or more members within the Eph family) is considered to be specific in the context of the present invention. In a further specific embodiment, an Eph receptor antibody is not an anti-EphA2 antibody or is not an anti-EphA4 antibody. In yet a further specific embodiment, an Eph receptor antibody is not D7, EA2, EA5, B233, B207 or B208.

As used herein, the terms "antibodies that specifically bind to an Ephrin" and analogous terms refer to antibodies that specifically bind to an Ephrin (*e.g*., EphrinA1, EphrinA2, EphrinA3, EphrinA4, EphrinA5, EphrinB1, EphrinB2 and EphrinB3) polypeptide or a fragment of an Ephrin polypeptide, and do not specifically bind to non-Ephrin polypeptides (or preferably, do not bind to other Ephrins). In one embodiment, antibodies that specifically bind to an Ephrin polypeptide or a fragment thereof do not cross-react with other antigens. Antibodies that specifically bind to an Ephrin polypeptide or a fragment thereof can be identified, for example, by immunoassays or other techniques known to those of skill in the art. In one embodiment, antibodies that specifically bind to an Ephrin polypeptide or a fragment thereof only modulate an Ephrin activity(ies) and do not significantly affect other activities.

Antibodies of the invention include, but are not limited to, synthetic antibodies, monoclonal antibodies, recombinantly produced antibodies, multispecific antibodies (including bi-specific antibodies), human antibodies, humanized antibodies, chimeric antibodies, intrabodies, single-chain Fvs (scFv) (*e.g*., including monospecific and bi-specific, etc.), Fab fragments, F(ab') fragments, disulfide-linked Fvs (sdFv), anti-idiotypic (anti-Id) antibodies, and epitope-binding fragments of any of the above. In particular, antibodies of the present invention include immunoglobulin molecules and immunologically active portions of immunoglobulin molecules, *i.e*., molecules that contain an antigen-binding site that specifically binds to an Eph receptor antigen or an Ephrin antigen (*e.g*., one or more complementarity determining regions (CDRs) of an anti-Eph receptor antibody (e.g., an anti-EphA1 -EphA2, -EphA3, -EphA4, -EphA5, -EphA6, -EphA7, -EphA8, -EphA10, -EphB1, - EphB2, -EphB3, -EphB4, -EphB5 or -EphB6 antibody) or of an anti-Ephrin antibody (*e.g*., an anti-EphrinA1 -EphrinA2, -EphrinA3, -EphrinA4, -EphrinA5, -EphrinB1 -EphrinB2 or - EphrinB3 antibody). The antibodies of the invention can be of any type (*e.g*., IgG, IgE, IgM, IgD, IgA and IgY), class (*e.g*., IgG₁, IgG₂, IgG₃, IgG₄, IgA₁ and IgA₂) or subclass of immunoglobulin molecule.

As used herein, the term "cancer" refers to a disease involving cells that have the potential to metastasize to distal sites and exhibit phenotypic traits that differ from those of non-cancer cells, for example, formation of colonies in a three-dimensional substrate such as soft agar or the formation of tubular networks or weblike matrices in a three-dimensional basement membrane or extracellular matrix preparation, such as MATRIGEL™. Non-cancer cells do not form colonies in soft agar and form distinct sphere-like structures in three-dimensional basement membrane or extracellular matrix preparations. Cancer cells acquire a characteristic set of functional capabilities during their development, albeit through various mechanisms. Such capabilities include evading apoptosis, self-sufficiency in growth signals, insensitivity to anti-growth signals, tissue invasion/metastasis, limitless replicative potential, and sustained angiogenesis. The term "cancer cell" is meant to encompass both premalignant and malignant cancer cells.

As used herein, the term "cell proliferation stimulative" refers to the ability of proteinaceous molecules (including, but not limited to, peptides, polypeptides, proteins, post-translationally modified proteins, antibodies, etc.), small molecules (less than 1000 daltons), inorganic or organic compounds, and nucleic acid molecules (including, but not limited to, double-stranded or single-stranded DNA, or double-stranded or single-stranded RNA (*e.g.,* antisense, RNAi, etc.), and triple helix nucleic acid molecules) to maintain, amplify, accelerate, or prolong cell proliferation, growth and/or survival *in vivo* or *in vitro.* Any method that detects cell proliferation, growth and/or survival, *e.g*., cell proliferation assays or epithelial barrier integrity assays, can be used to determine whether an agent is a cell proliferation stimulative agent. Cell proliferation stimulative agents may also cause maintenance, regeneration, or reconstitution of epithelium when added to established colonies of hyperproliferative or damaged cells.

As used herein, the term "derivative" in the context of a proteinaceous agent (*e.g*., proteins, polypeptides, peptides, and antibodies) refers to a proteinaceous agent that comprises the amino acid sequence which has been altered by the introduction of amino acid residue substitutions, deletions, and/or additions. The term "derivative" as used herein also refers to a proteinaceous agent which has been modified, *i.e.,* by the covalent attachment of a type of molecule to the proteinaceous agent. For example, but not by way of limitation, a derivative of a proteinaceous agent may be produced, *e.g*., by glycosylation, acetylation, pegylation, phosphorylation, amidation, derivatization by known protecting/blocking groups, proteolytic cleavage, linkage to a cellular ligand or other protein, etc. A derivative of a proteinaceous agent may also be produced by chemical modifications using techniques known to those of skill in the art, including, but not limited to specific chemical cleavage, acetylation, formylation, metabolic synthesis of tunicamycin, etc. Further, a derivative of a proteinaceous agent may contain one or more non-classical amino acids. A derivative of a proteinaceous agent possesses an identical function(s) as the proteinaceous agent from which it was derived. In a specific embodiment, a derivative of a proteinaceous agent is a derivative of an Eph receptor polypeptide (e.g., an EphA1 EphA2, EphA3, EphA4, EphA5, EphA6, EphA7, EphA8, EphA10, EphB1 EphB2, EphB3, EphB4, EphB5 or EphB6 polypeptide), an Ephrin polypeptide (e.g., an EphrinA1 EphrinA2, EphrinA3, EphrinA4, EphrinA5, EphrinB1 EphrinB2 or EphrinB3 polypeptide), a fragment of an Eph receptor polypeptide or Ephrin polypeptide, an antibody that specifically binds to an Eph receptor polypeptide or fragment thereof, or an antibody that specifically binds to an Ephrin polypeptide or fragment thereof. In one embodiment, a derivative of an Eph receptor polypeptide or an Ephrin polypeptide, a fragment of an Eph receptor polypeptide or Ephrin polypeptide, an antibody that specifically binds to an Eph receptor polypeptide or fragment thereof, or an antibody that specifically binds to an Ephrin polypeptide or fragment thereof possesses a similar or identical function as an Eph receptor polypeptide, an Ephrin polypeptide, a fragment of an Eph receptor polypeptide or Ephrin polypeptide, an antibody that specifically binds to an Eph receptor polypeptide or fragment thereof, or an antibody that specifically binds to an Ephrin polypeptide or fragment thereof. In another embodiment, a derivative of an Eph receptor polypeptide, an Ephrin polypeptide, a fragment of an Eph receptor polypeptide or Ephrin polypeptide, an antibody that specifically binds to an Eph receptor polypeptide or fragment thereof, or an antibody that specifically binds to an Ephrin polypeptide or fragment thereof has an altered activity when compared to an unaltered polypeptide. For example, a derivative antibody or fragment thereof can bind to its epitope more tightly or be more resistant to proteolysis.

As used herein, the term "effective amount" refers to the amount of a therapy (*e.g*., a prophylactic or therapeutic agent) which is sufficient to reduce and/or ameliorate the severity and/or duration of a disorder, or a symptom thereof, prevent the advancement of said disorder, cause regression of said disorder, prevent the recurrence, development, or onset of one or more symptoms associated with said disorder, or enhance or improve the prophylactic or therapeutic effect(s) of another therapy (*e.g*., prophylactic or therapeutic agent). Non-limiting examples of effective amounts of Eph/Ephrin Modulators are provided in Section X, *infra.*

As used herein, the term "endogenous ligand" or "natural ligand" refers to a molecule that normally binds a particular receptor *in vivo.* For example, and not by way of limitation, any of the A-type Ephrin ligands (*e.g*., EphrinA1, EphrinA2, EphrinA3, EphrinA4 and Ephrin5) may bind to any of the A-type Eph receptors (*e.g.*, EphA1, EphA2, EphA3, EphA4, EphA5, EphA6, EphA7, EphA8, and EphA10) and any of the B-type Ephrin ligands (*e.g*., EphrinB1, EphrinB2 and EphrinB3) may bind to any of the B-type Eph receptors (*e.g*., EphB1, EphB2, EphB3, EphB4, EphB5 and EphB6). Also, by way of example and not by way of limitation, EphA4 may bind to both A-type and B-type Ephrin ligands as disclosed herein.

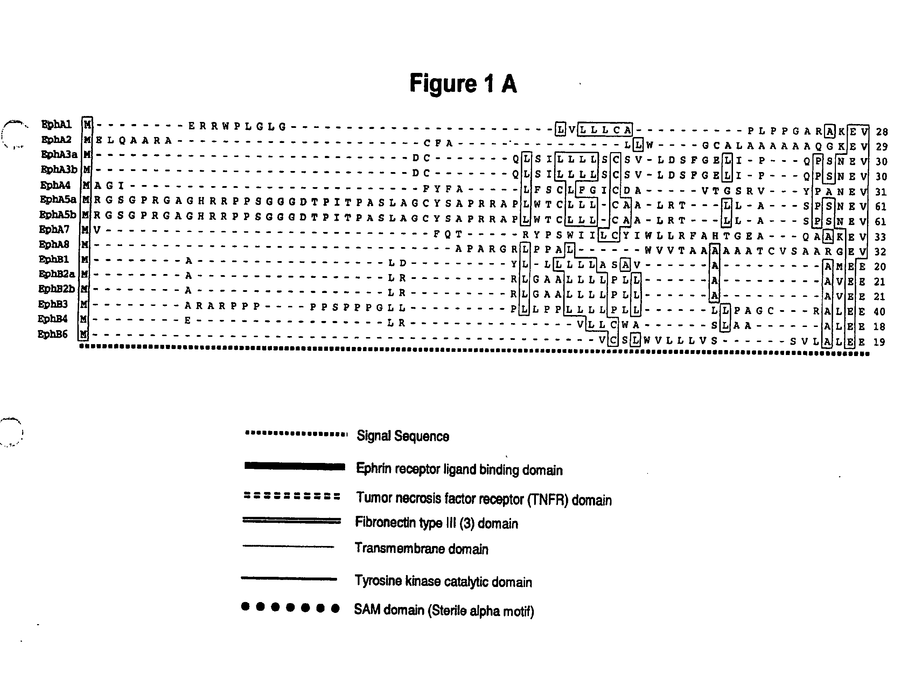

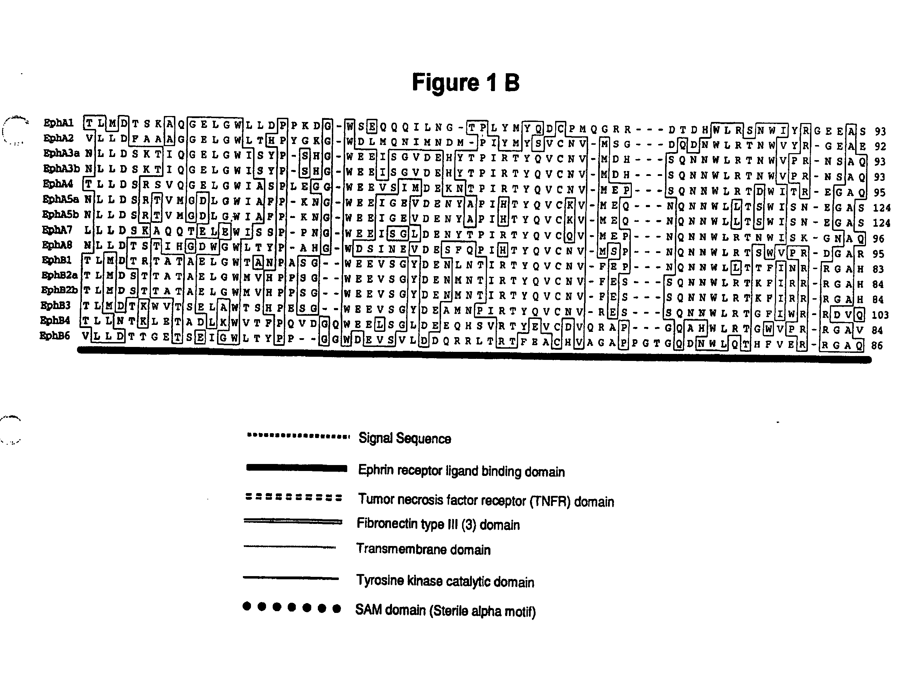

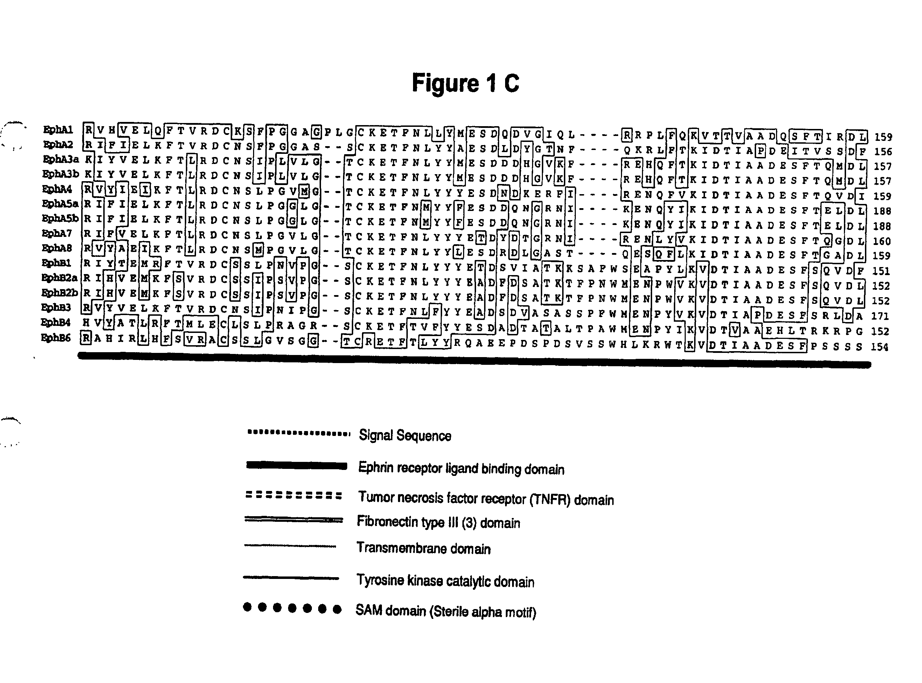

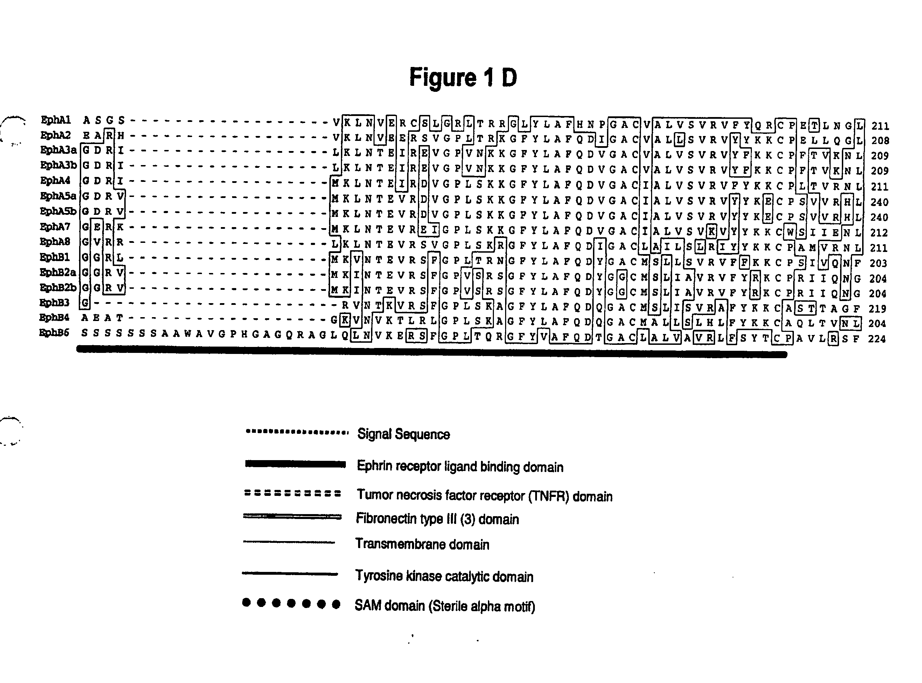

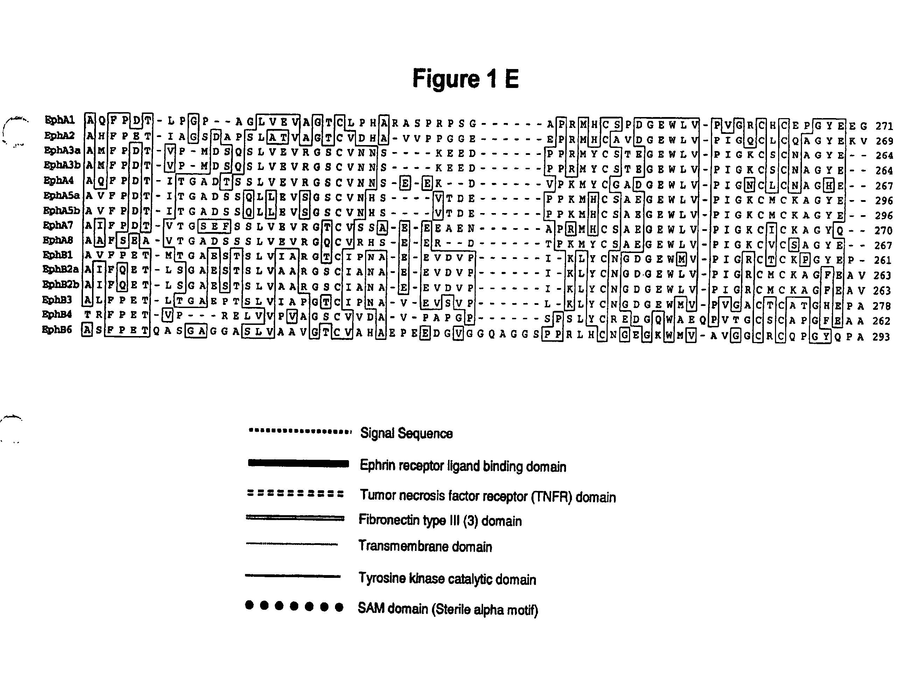

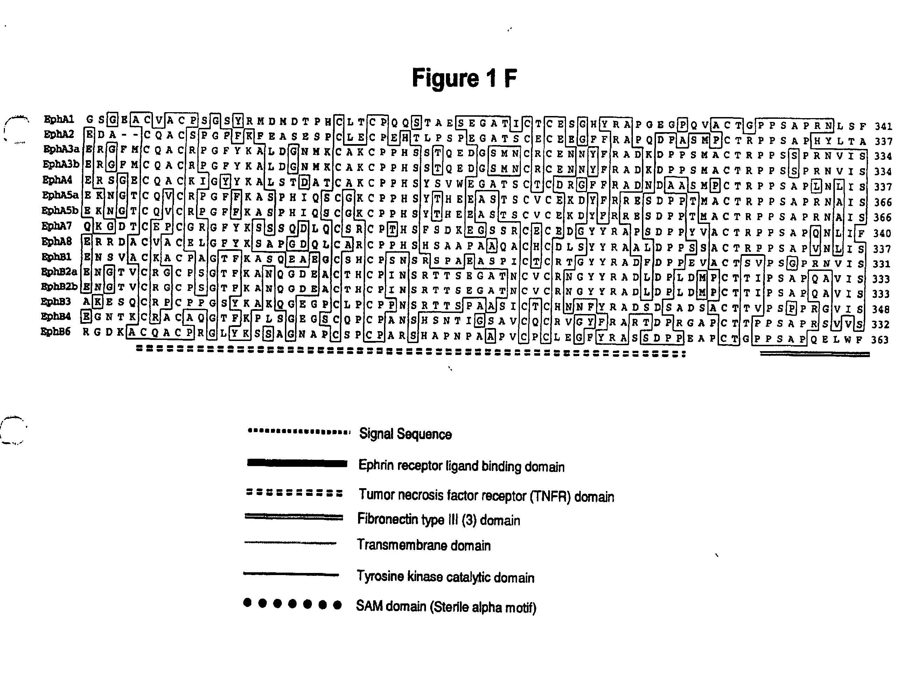

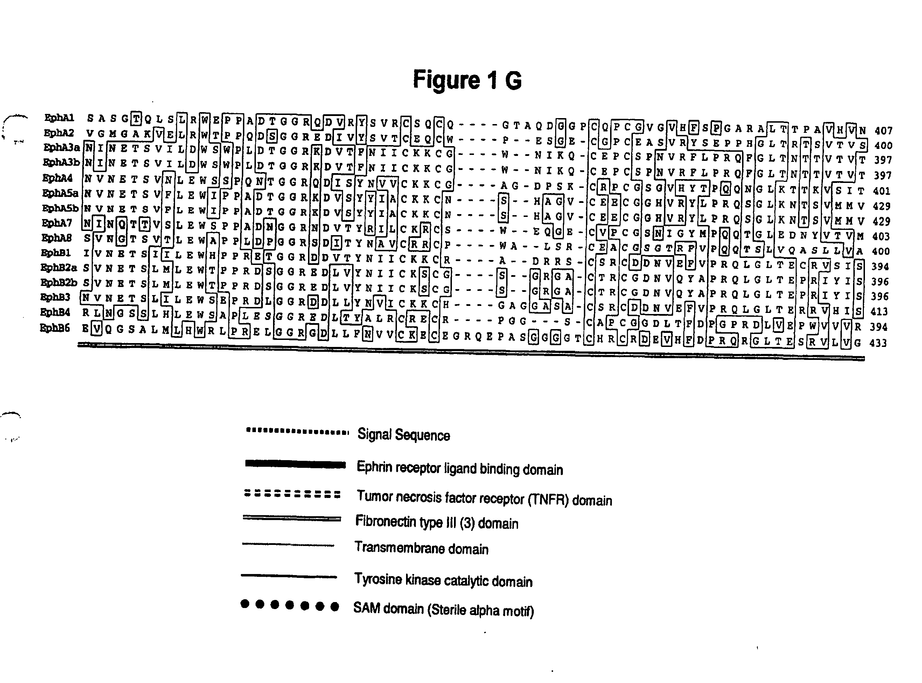

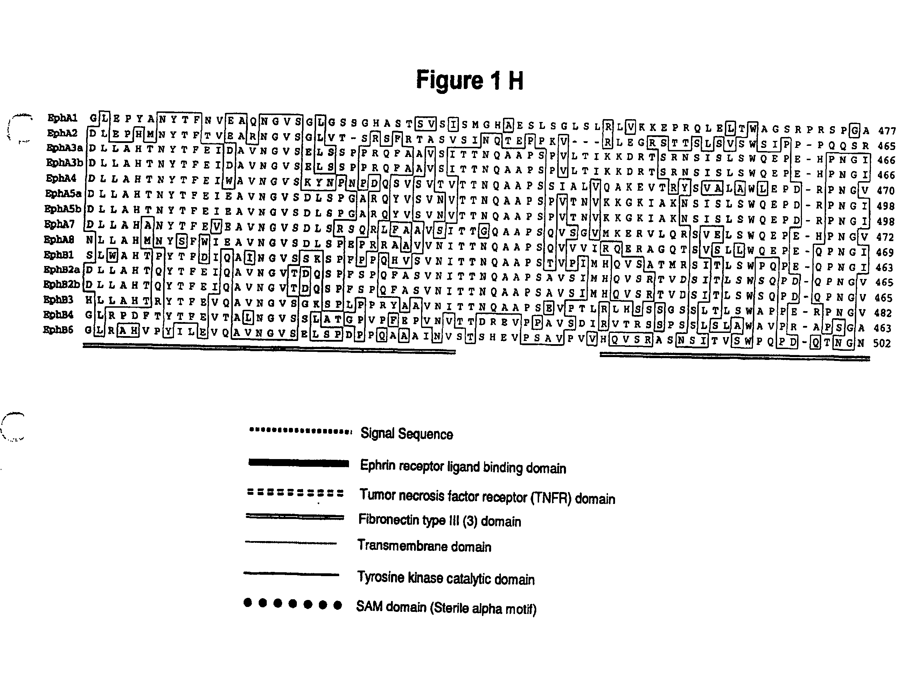

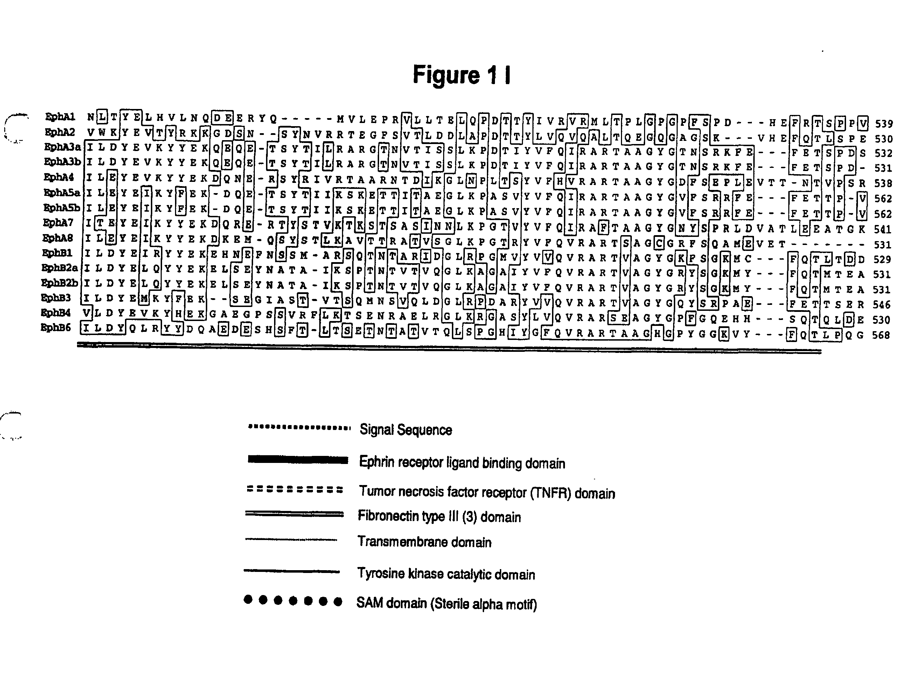

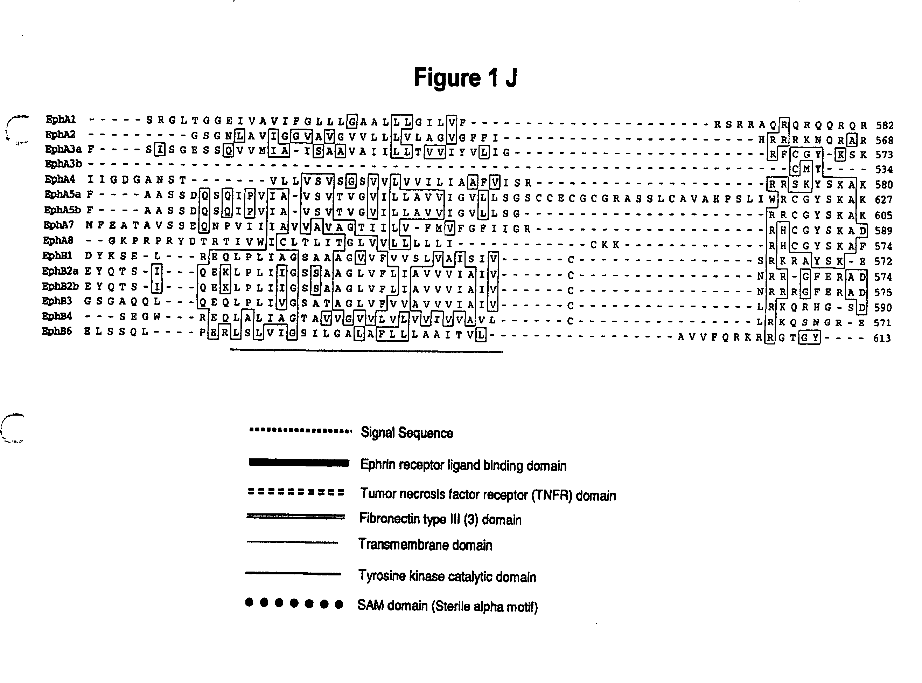

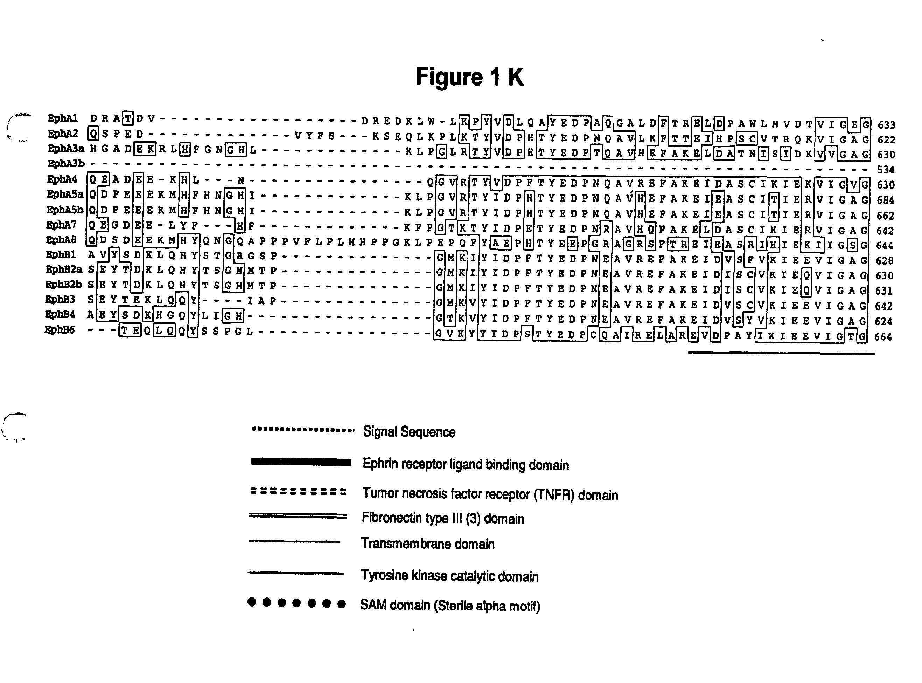

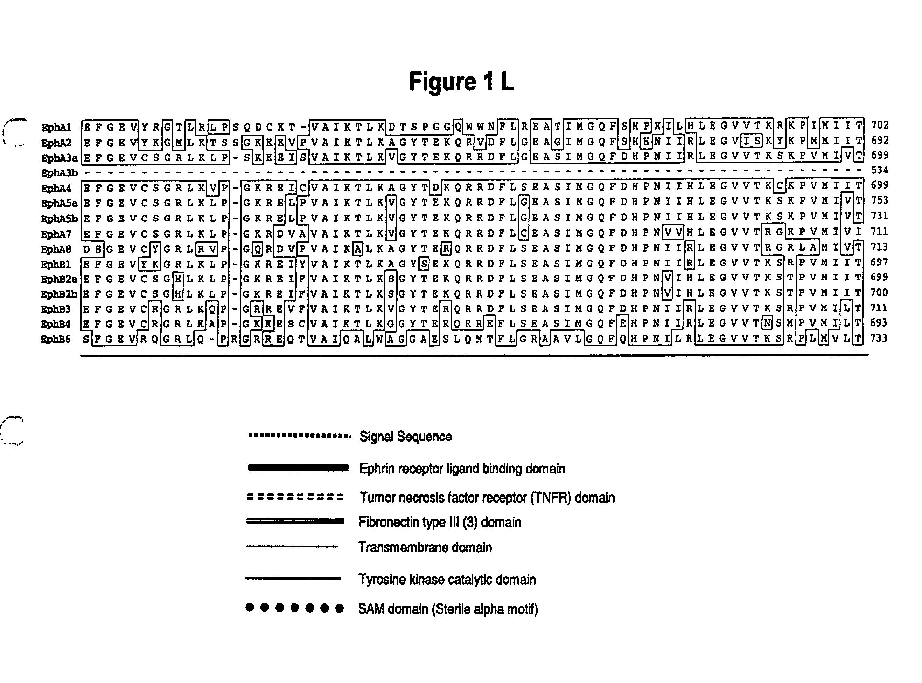

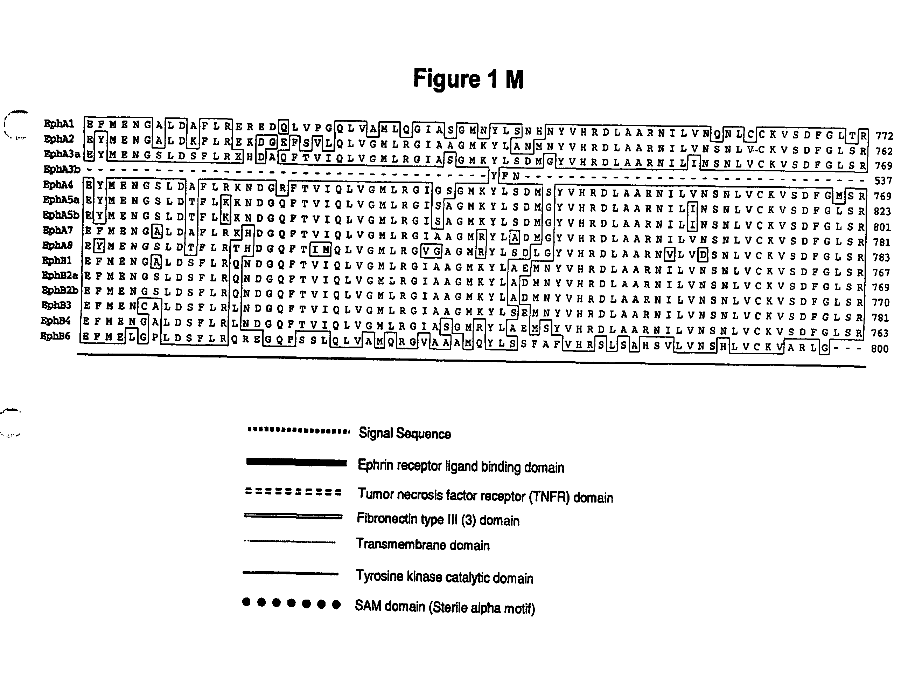

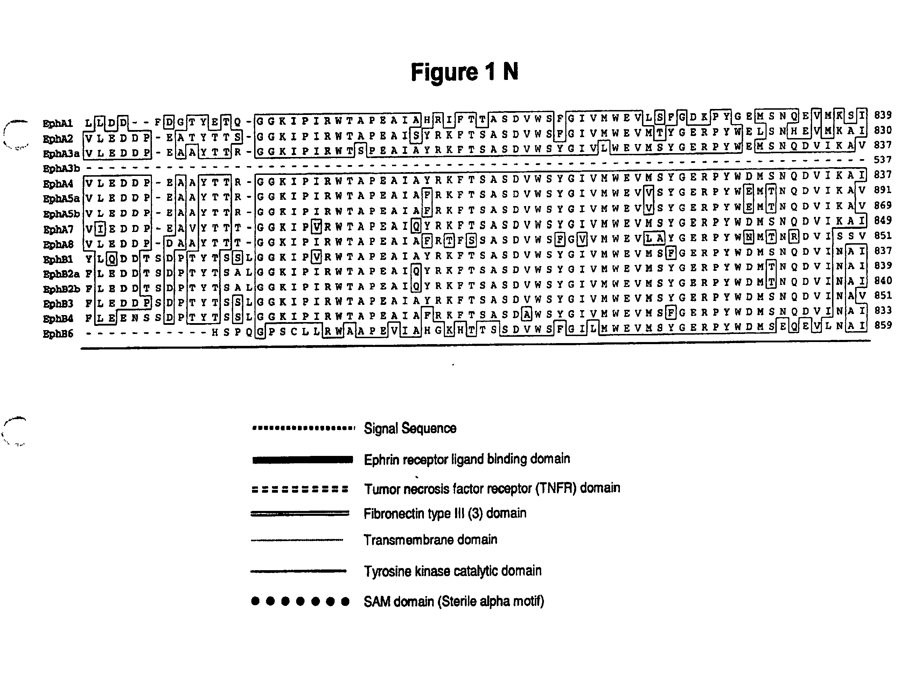

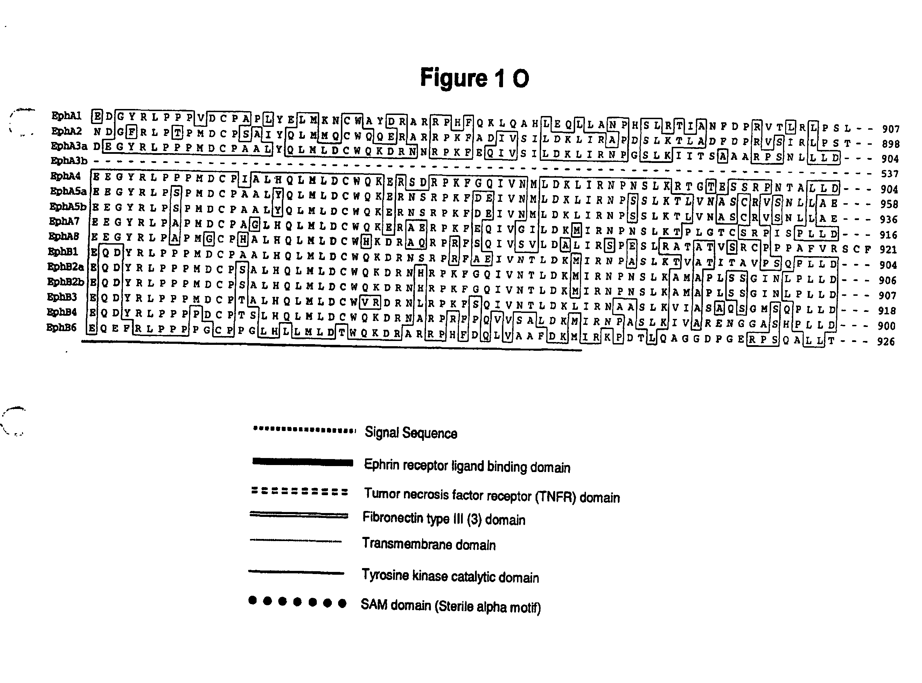

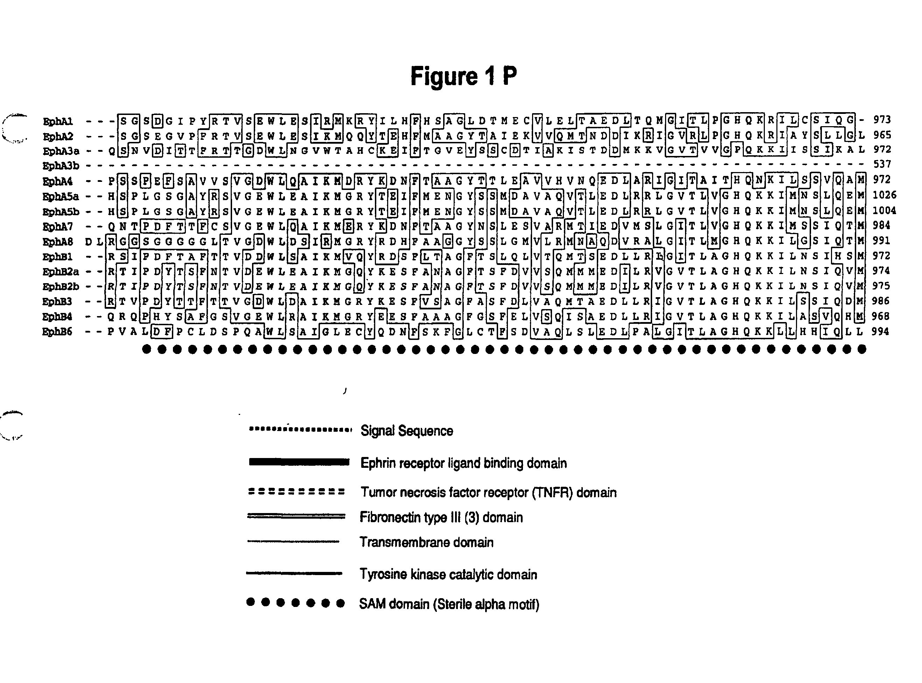

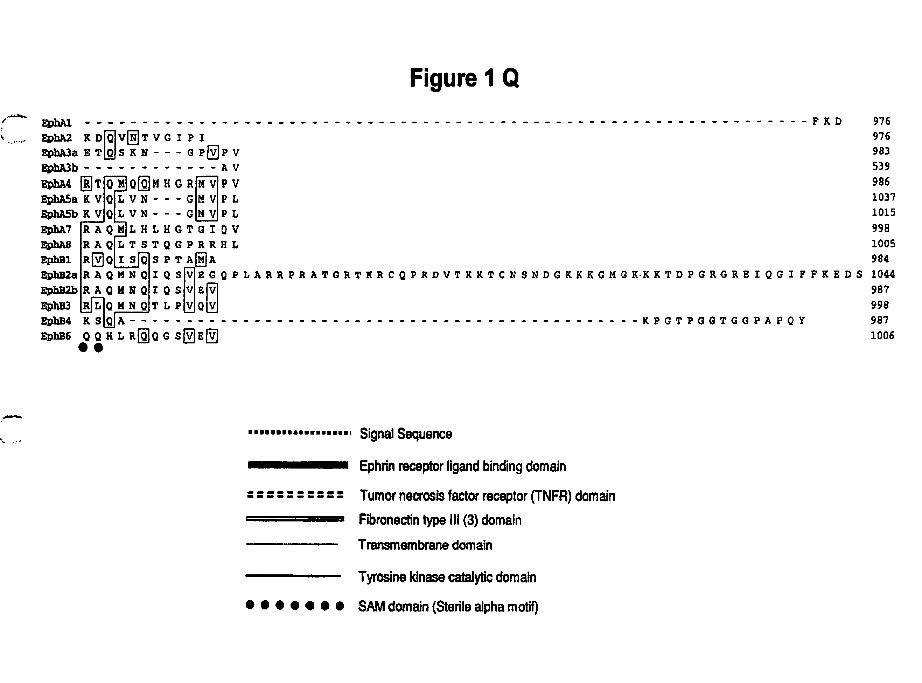

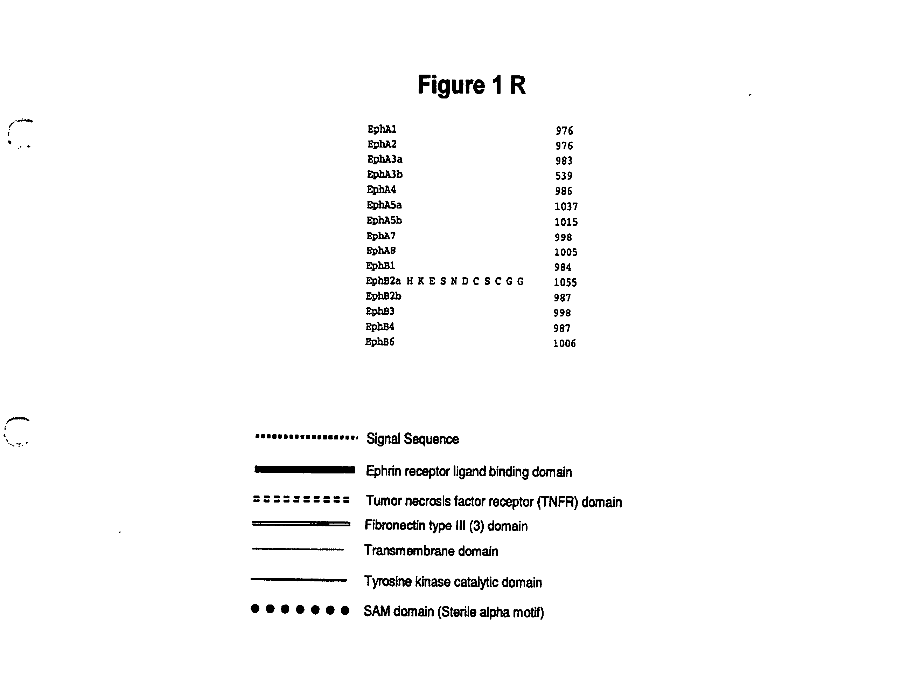

As used herein, the term "Eph receptor" or "Eph receptor tyrosine kinase" refers to any Eph receptor that has or will be identified and recognized by the Eph Nomenclature Committee (Eph Nomenclature Committee, 1997, Cell 90:403-404). Eph receptors of the present invention include, but are not limited to EphA1, EphA2, EphA3, EphA4, EphA2, EphA6, EphA7, EphA8, EphA10 EphB1, EphB2, EphB3, EphB4, EphB5 and EphB6. In a specific embodiment, an Eph receptor polypeptide is from any species. In another specific embodiment, an Eph receptor polypeptide is human. The nucleotide and/or amino acid sequences of Eph receptor polypeptides can be found in the literature or public databases (*e.g.*, GenBank), or the nucleotide and/or amino acid sequences can be determined using cloning and sequencing techniques known to one of skill in the art. The GenBank Accession Nos. for the nucleotide and amino acid sequences of the human Eph receptors are summarized in Table 1 below. See also Figure 1 for the amino acid sequences of the Eph receptors in mammals that have been identified to date.

**Table 1**

| **Eph Receptor** | **Nucleotide Sequence** | **Amino Acid Sequence** |
|---|---|---|
| EphA1 | NM_005232.2 (SEQ ID NO:1) | NP_005223.2 (SEQ ID NO:2) |
| EphA2 | NM_004431.2 (SEQ ID NO:3) | NP_004422.2 (SEQ ID NO:4) |
| EphA3, variant 1 | NM_005233.3 (SEQ ID NO:5) | NP_005224.2 (SEQ ID NO:6) |
| EphA3, variant 2 | NM_182644.1 (SEQ ID NO:7) | NP_872585.1 (SEQ ID NO:8) |
| EphA4 | NM_004438.3 (SEQ ID NO:9) | NP_004429.1 (SEQ ID NO:10) |
| EphA5, variant 1 | NM_004439.3 (SEQ ID NO:11) | NP_004430.2 (SEQ ID NO:12) |
| EphA5, variant 2 | NM_182472.1 (SEQ ID NO:13) | NP_872272.1 (SEQ ID NO:14) |
| EphA6 (predicted) | XM_114973.4 (SEQ ID NO:15) | XP_114973.4 ((SEQ ID NO: 16) |
| EphA7 | NM_004440.2 (SEQ ID NO:17) | NP_004431.1 (SEQ ID NO:18) |
| EphA8 | NM_020526.2 (SEQ ID NO:19) | NP_065387.1 (SEQ ID NO:20) |
| EphA10 | AJ872185.1 (SEQ ID NO:206) | CAI43321.1 (SEQ ID NO:207) |
| EphB1 | NM_004441.2 (SEQ ID NO:21) | NP_004432.1 (SEQ ID NO:22) |
| EphB2, variant 1 | NM_017449.1 (SEQ ID NO:23) | NP_059145.1 (SEQ ID NO:24) |
| EphB2, variant 2 | NM_004442.4 (SEQ ID NO:25) | NP_004433.2 (SEQ ID NO:26) |
| EphB3 | NM_004443.3 (SEQ ID NO:27) | NP_004434.2 (SEQ ID NO:28) |
| EphB4 | NM_004444.3 (SEQ ID NO:29) | NP_004435.3 (SEQ ID NO:30) |
| EphB5 (chicken; human sequence not reported) | NM_001004387.1 (SEQ ID NO:112) | NP_001004387.1 (SEQ ID NO:113) |
| EphB6 | NM_004445.1 (SEQ ID NO:31) | NP_004436.1 (SEQ ID NO:32) |

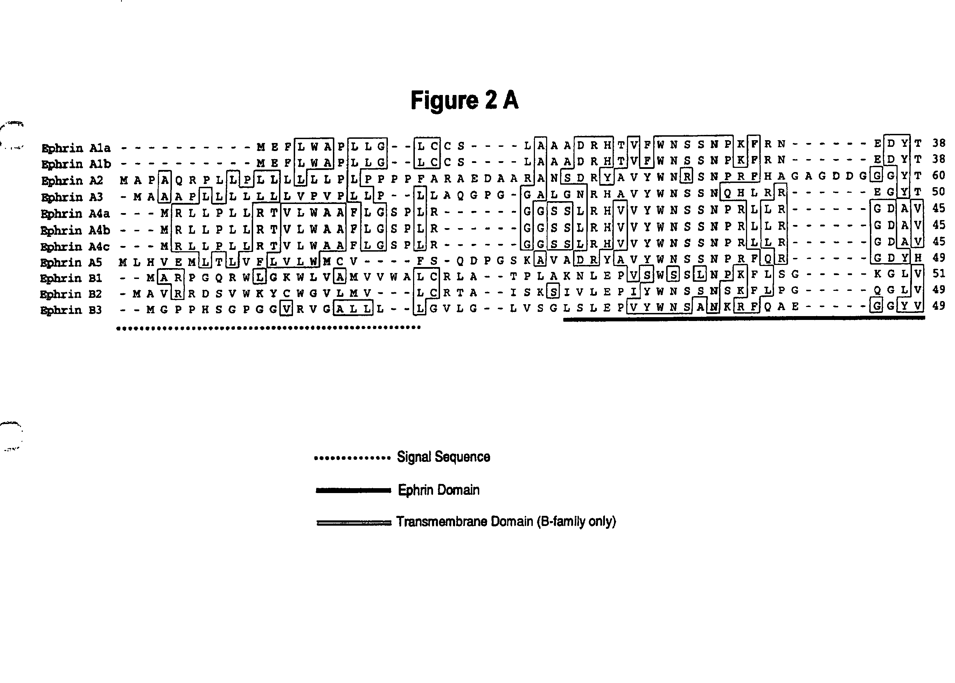

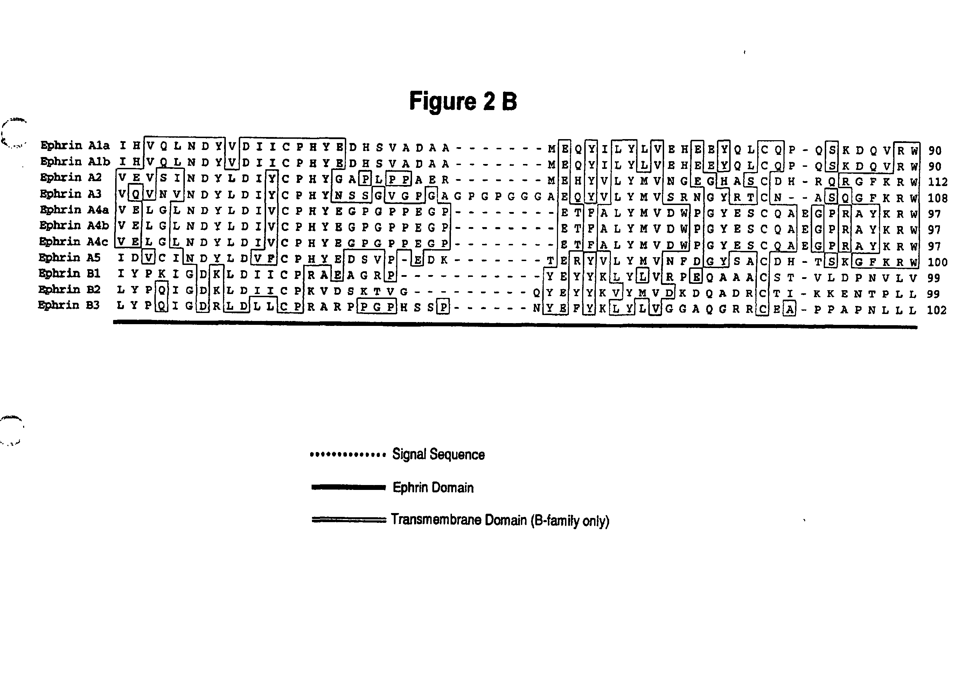

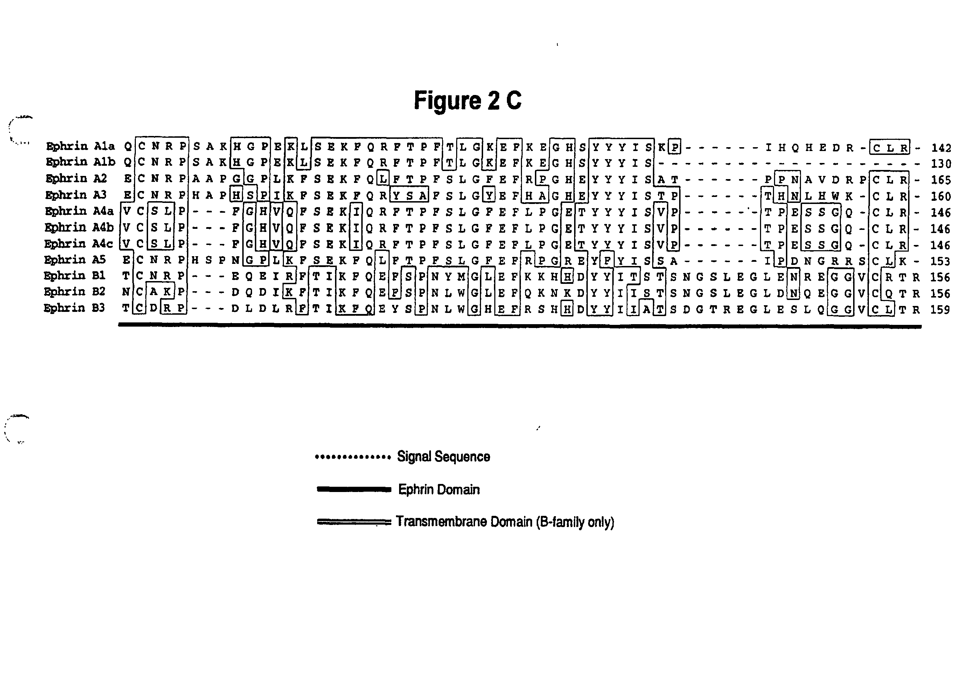

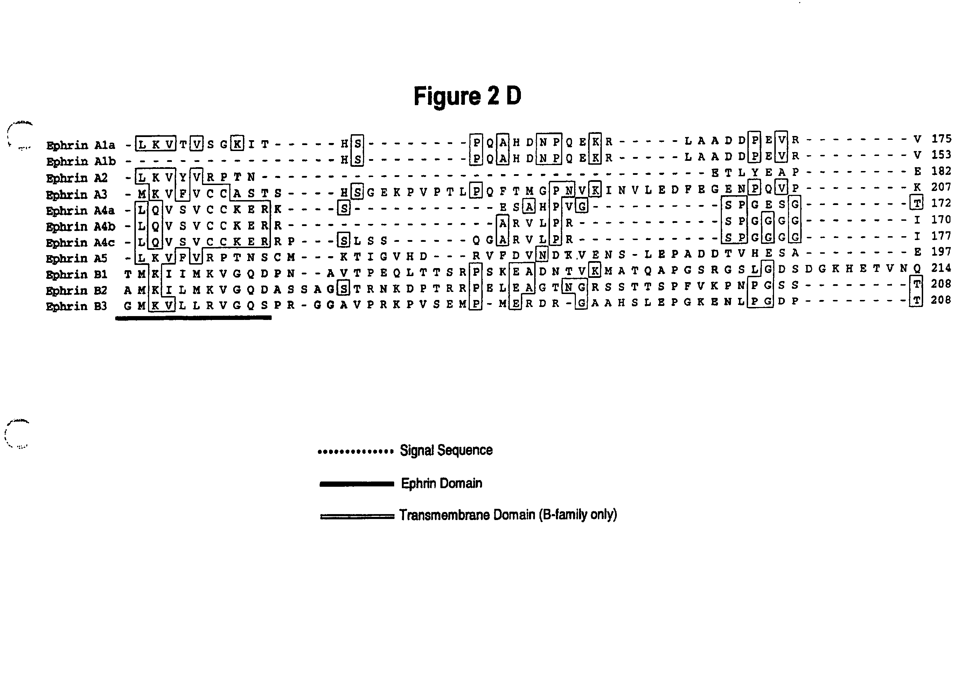

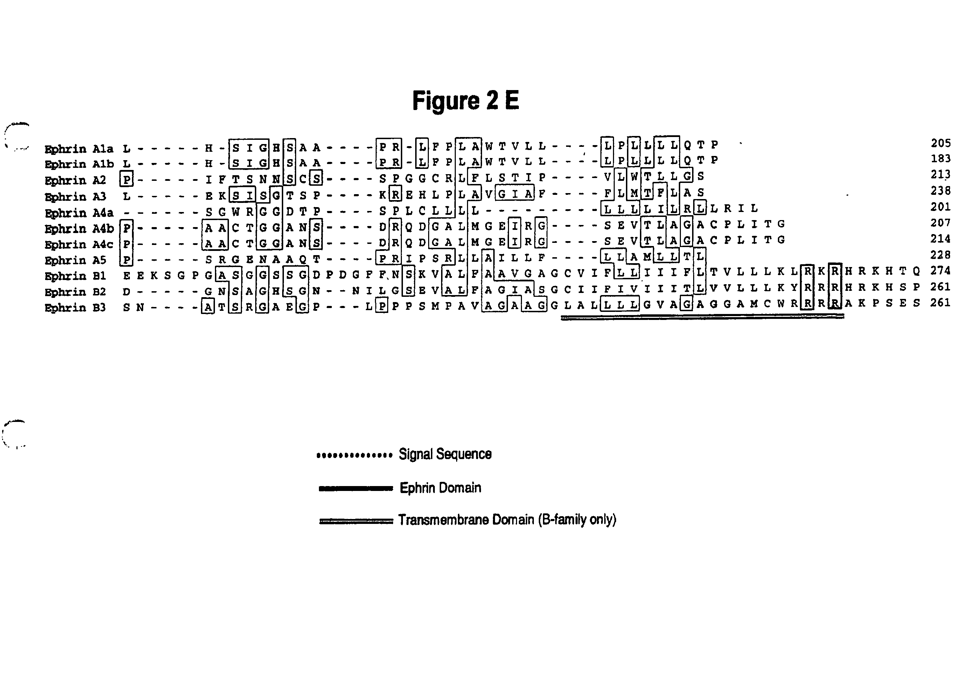

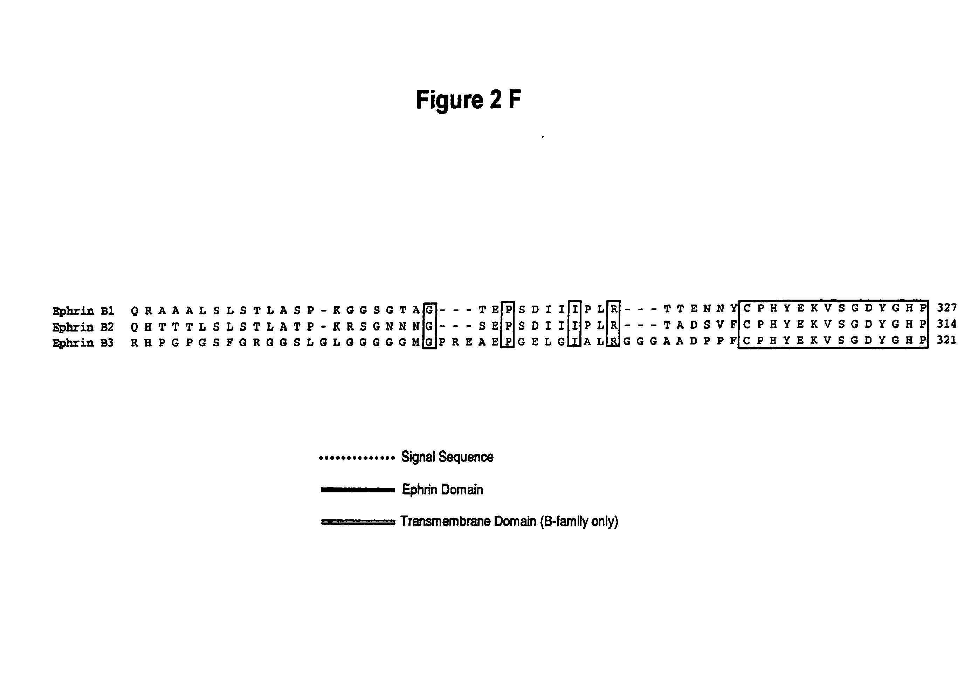

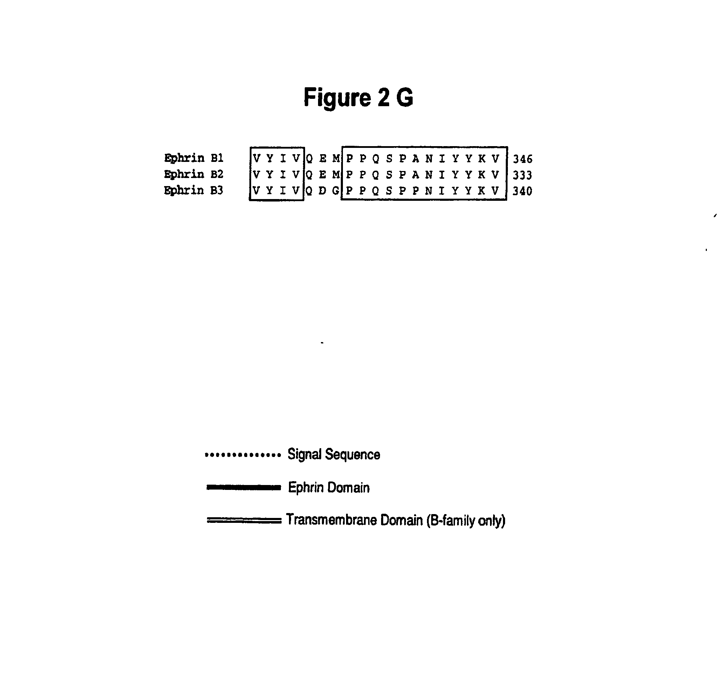

As used herein, the term "Ephrin" or "Ephrin ligand" refers to any Ephrin ligand that has or will be identified and recognized by the Eph Nomenclature Committee (Eph Nomenclature Committee, 1997, *Cell* 90:403-404). Ephrins of the present invention include, but are not limited to, EphrinA1, EphrinA2, EphrinA3, EphrinA4, EphrinA5, EphrinB1, EphrinB2 and EphrinB3. In a specific embodiment, an Ephrin polypeptide is from any species. In a another specific embodiment, an Ephrin polypeptide is human. The nucleotide and/or amino acid sequences of Ephrin polypeptides can be found in the literature or public databases (e.g., GenBank), or the nucleotide and/or amino acid sequences can be determined using cloning and sequencing techniques known to one of skill in the art. The GenBank Accession Nos. for the nucleotide and amino acid sequences of the human Ephrins are summarized in Table 2 below. See also Figure 2 for the amino acid sequences of the Ephrins in mammals that have been identified to date.

**Table 2**

| **Ephrin** | **Nucleotide Sequence** | **Amino Acid Sequence** |
|---|---|---|
| EprinA1, variant 1 | NM_004428.2 (SEQ ID NO:33) | NP_004419.2 (SEQ ID NO:34) |
| EphrinA1, variant 2 | NM_182685.1 (SEQ ID NO: 35) | NP_872626.1 (SEQ ID NO:36) |
| EphrinA2 | NM_001405.2 (SEQ ID NO:37) | NP_001396.2 (SEQ ID NO:38) |
| EphrinA3 | NM_004952.3 (SEQ ID NO:39) | NM_004952.3 (SEQ ID NO:40) |
| EphrinA4, variant 1 | NM_005227.2 (SEQ ID NO:41) | NP_005218.1 (SEQ ID NO:42) |
| EphrinA4, variant 2 | NM_182689.1 (SEQ ID NO:43) | NP_872631.1 (SEQ ID NO:44) |
| EphrinA4, variant 3 | NM_182690.1 (SEQ ID NO:45) | NP_872632.1 (SEQ ID NO:46) |
| EphrinA5 | NM_001962.1 (SEQ ID NO:47) | NP_001953.1 (SEQ ID NO:48) |
| EphrinB1 | NM_004429.3 (SEQ ID NO:49) | NP_004420.1 (SEQ ID NO:50) |
| EphrinB2 | NM_004093.2 (SEQ ID NO:51) | NP_004084.1 (SEQ ID NO:52) |
| EphrinB3 | NM_001406.3 (SEQ ID NO:53) | NP_001397.1 (SEQ ID NO:54) |

As used herein, the term "Eph/Ephrin Modulator" refers to an agent(s) that confers a biological effect by modulating (directly or indirectly): (i) the expression of an Eph receptor (*e.g*., EphA1 EphA2, EphA3, EphA4, EphA2, EphA6, EphA7, EphA8, EphA10, EphB1, EphB2, EphB3, EphB4, EphB5 or EphB6) and/or an endogenous ligand(s) of an Eph receptor (*e.g*., EphrinA1 EphrinA2, EphrinA3, EphrinA4, EphrinA5, EphrinB1 EphrinB2 or EphrinB3), at, *e.g*., the transcriptional, post-transcriptional, translational or post-translation level; and/or (ii) an activity(ies) of an Eph receptor and/or an Ephrin.

Examples of Eph/Ephrin Modulators include, but are not limited to, agents that inhibit or reduce the interaction between an Eph receptor and an endogenous ligand(s) of the Eph receptor, for example, an Ephrin (hereinafter "Eph/Ephrin Interaction Inhibitors"). Non-limiting examples of Eph/Ephrin Interaction Inhibitors include: (i) agents that bind to an Eph receptor, prevent or reduce the interaction between the Eph receptor and an Ephrin, and induce Eph receptor signal transduction (*e.g*., soluble forms of an Ephrin (*e.g*., in monomeric or multimeric form), and antibodies that bind to an Eph receptor, induce signaling and phosphorylation of the Eph receptor (*i.e*., an Eph receptor agonistic antibody)); (ii) agents that bind to an Eph receptor, prevent or reduce the interaction between the Eph receptor and an Ephrin, and prevent or induce very low to negligible levels of Eph receptor signal transduction (*e.g*., Eph receptor antagonistic antibodies and dominant negative forms of an Ephrin); (iii) agents that bind to an Ephrin, prevent or reduce the interaction between an Eph receptor and the Ephrin, and induce Ephrin signal transduction (*e.g*., soluble forms of an Eph receptor and antibodies that bind to an Ephrin and induce Ephrin signal transduction); and (iv) agents that bind to an Ephrin, prevent or reduce the interaction between an Eph receptor and the Ephrin, and prevent or induce very low to negligible levels of Ephrin signal transduction (*e.g*., dominant negative forms of an Eph receptor and anti-Ephrin antibodies).

In further embodiments, Eph/Ephrin Modulators include, but are not limited to, agents that modulate the expression of an Eph receptor. Such agents can decrease/downregulate Eph receptor expression (*e.g*., Eph receptor antisense molecules, RNAi and ribozymes) or increase/upregulate Eph receptor expression such that the amount of an Eph receptor on the cell surface exceeds the amount of endogenous ligand (*e.g*., an Ephrin) available for binding, and thus, increases the amount of unbound Eph receptor (*e.g*., nucleic acids encoding an Eph receptor)).

In other embodiments, Eph/Ephrin Modulators are agents that modulate the expression of an Ephrin. Such agents can decrease/downregulate Ephrin expression (*e.g*., Ephrin antisense molecules, RNAi and ribozymes) or increase/upregulate Ephrin expression (*e.g*., nucleic acids encoding an Ephrin)).

In yet other embodiments, Eph/Ephrin Modulators of the invention include, but are not limited to, agents that modulate the protein stability or protein accumulation of an Eph receptor or an Ephrin.

In further embodiments, Eph/Ephrin Modulators of the invention are agents that promote kinase activity (*e.g*., of an Eph receptor, an Ephrin or of a heterologous protein known to associate with the Eph receptor or Ephrin at the cell membrane).

In yet further embodiments, Eph/Ephrin Modulators include, but are not limited to, agents that bind to an Eph receptor and prevent or reduce Eph receptor signal transduction but do not inhibit or reduce the interaction between the Eph receptor and an Ephrin (*e.g*., an Eph receptor intrabody); and agents that bind to an Ephrin and prevent or reduce Ephrin signal transduction but do not inhibit or reduce the interaction between an Ephrin and an Eph receptor (*e.g*., a B-type Ephrin intrabody).

In a specific embodiment, an Eph/Ephrin Modulator is not an agent that inhibits or reduces the interaction between an Eph receptor and an endogenous ligand, for example, an Ephrin. In another specific embodiment, an Eph/Ephrin Modulator is not an agent that modulates (increases or decreases) the expression of an Eph receptor and/or of an Ephrin. In a further embodiment, an Eph/Ephrin Modulator is not an agent that modulates the protein stability or protein accumulation of an Eph receptor or an Ephrin. In yet a further embodiment of the invention, an Eph/Ephrin Modulator is not an agent that promotes kinase activity (*e.g*., of an Eph receptor, an Ephrin or of a heterologous protein known to associate with an Eph receptor or Ephrin at the cell membrane). In another specific embodiment, an Eph/Ephrin Modulator is not an agent that binds to an Eph receptor and prevents or reduces Eph receptor signal transduction but does not prevent the interaction between the Eph receptor and an Ephrin; or an agent that binds to an Ephrin and prevents or reduces Ephrin signal transduction but does not prevent the interaction between the Ephrin and an Eph receptor. In a specific embodiment, an Eph/Ephrin Modulator is not an EphA2 agonistic antibody or an EphA2 antagonistic antibody. In another specific embodiment, an Eph/Ephrin Modulator is not an anti-EphA4 antibody. In a further embodiment, an Eph/Ephrin Modulator is not an Eph receptor antisense molecule or an Ephrin antisense molecule. In yet a further embodiment, an Eph/Ephrin Modulator is not a soluble form of an Eph receptor (*e.g*., Eph-Fc) or is not a soluble form of an Ephrin (*e.g*., Ephrin-Fc).

As used herein, the term "epitope" refers to a portion of an Eph receptor or Ephrin polypeptide having antigenic or immunogenic activity in an animal, preferably in a mammal, and most preferably in a human. An epitope having immunogenic activity is a portion of an Eph receptor or Ephrin polypeptide that elicits an antibody response in an animal. An epitope having antigenic activity is a portion of an Eph receptor or Ephrin polypeptide to which an antibody specifically binds as determined by any method well known in the art, for example, by immunoassays. Antigenic epitopes need not necessarily be immunogenic. In a specific embodiment, an epitope is not EphA2₅₈ (IMNDMPIYM; SEQ ID NO:55) or is not EphA2₅₅₀ (VLAGVGFFI; SEQ ID NO:56). In another specific embodiment, an epitope is not (TLADFDPRV; SEQ ID NO:57), (VLLLVLAGV; SEQ ID NO:58), (VLAGVGFFI; SEQ ID NO:59), (IMNDMPIYM; SEQ ID NO:60), (SLLGLKDQV; SEQ ID NO:61), (WLVPIGQCL; SEQ ID NO:62), (LLWGCALAA; SEQ ID NO:63), GLTRTSVTV; SEQ ID NO:64), (NLYYAESDL; SEQ ID NO:65), or (KLNVEERSV; SEQ ID NO:66). In yet another sepcific embodiment, an epitope is not (IMGQFSHHN; SEQ ID NO:67), (YSVCNVMSG; SEQ ID NO:68), (MQNIMNDMP; SEQ ID NO:69), (EAGIMGQFSHHNIIR; SEQ ID NO:70), (PIYMYSVCNVMSG; SEQ ID NO:71) or (DLMQNIMNDMPIYMYS; SEQ ID NO:72).

As used herein, the term "fragment" in the context of a proteinaceous agent refers to a peptide or polypeptide comprising an amino acid sequence of at least 5 contiguous amino acid residues, at least 10 contiguous amino acid residues, at least 15 contiguous amino acid residues, at least 20 contiguous amino acid residues, at least 30 contiguous amino acid residues, at least 40 contiguous amino acid residues, at least 50 contiguous amino acid residues, at least 60 contiguous amino residues, at least 70 contiguous amino acid residues, at least contiguous 80 amino acid residues, at least 90 contiguous amino acid residues, at least 100 contiguous amino acid residues, at least 125 contiguous amino acid residues, at least 150 contiguous amino acid residues, at least 175 contiguous amino acid residues, at least 200 contiguous amino acid residues, or at least 250 contiguous amino acid residues of the amino acid sequence of an Eph receptor or Ephrin polypeptide, a fragment of an Eph receptor or Ephrin polypeptide, an antibody that specifically binds to an Eph receptor or Ephrin polypeptide, or an antibody fragment that specifically binds to an Eph receptor or Ephrin polypeptide which has been altered by the introduction of amino acid residue substitutions, deletions or additions. For example, antibody fragments are epitope-binding fragments.

As used herein, the term "fusion protein" refers to a polypeptide or protein that comprises the amino acid sequence of a first polypeptide or protein or fragment, analog or derivative thereof, and the amino acid sequence of a heterologous polypeptide or protein (*i.e*., a second polypeptide or protein or fragment, analog or derivative thereof different than the first polypeptide or protein or fragment, analog or derivative thereof, or not normally part of the first polypeptide or protein or fragment, analog or derivative thereof). In one embodiment, a fusion protein comprises a prophylactic or therapeutic agent fused to a heterologous protein, polypeptide or peptide. In accordance with this embodiment, the heterologous protein, polypeptide or peptide may or may not be a different type of prophylactic or therapeutic agent. For example, two different proteins, polypeptides, or peptides with immunomodulatory activity may be fused together to form a fusion protein. In one embodiment, fusion proteins retain or have improved activity relative to the activity of the original polypeptide or protein prior to being fused to a heterologous protein, polypeptide, or peptide.

As used herein, the term "humanized antibody" refers to forms of non-human (*e.g.*, murine) antibodies, such as chimeric antibodies, which contain minimal sequence derived from non-human immunoglobulin. For the most part, humanized antibodies are human immunoglobulins (recipient antibody) in which hypervariable region or complementarity determining (CDR) residues of the recipient are replaced by hypervariable region residues or CDR residues from an antibody from a non-human species (donor antibody) such as mouse, rat, rabbit or non-human primate having the desired specificity, affinity, and capacity. In some instances, one or more Framework Region (FR) residues of the human immunoglobulin are replaced by corresponding non-human residues or other residues based upon structural modeling, *e.g*., to improve affinity of the humanized antibody. Furthermore, humanized antibodies may comprise residues which are not found in the recipient antibody or in the donor antibody. These modifications are made to further refine antibody performance. In general, the humanized antibody will comprise substantially all of at least one, and typically two, variable domains, in which all or substantially all of the hypervariable regions correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. For further details, see Jones et al., 1986, Nature 321:522-525; Reichmann et al., 1988, Nature 332:323-329; Presta, 1992, Curr. Op. Struct. Biol. 2:593-596; and Queen et al., U.S. Patent No. 5,585,089.

As used herein, the term "hypervariable region" refers to the amino acid residues of an antibody which are responsible for antigen binding. The hypervariable region comprises amino acid residues from a "Complementarity Determining Region" or "CDR" (*i.e*. residues 24-34 (L1), 50-56 (L2) and 89-97 (L3) in the light chain variable domain and 31-35 (H1), 50-65 (H2) and 95-102 (H3) in the heavy chain variable domain; Kabat et al., *Sequences of Proteins of Immunological Interest,* 5th Ed. Public Health Service, National Institutes of Health, Bethesda, MD (1991)) and/or those residues from a "hypervariable loop" (*i.e*. residues 26-32 (L1), 50-52 (L2) and 91-96 (L3) in the light chain variable domain and 26-32 (H1), 53-55 (H2) and 96-101 (H3) in the heavy chain variable domain; Chothia and Lesk, 1987, J. Mol. Biol. 196:901-917). "Framework Region" or "FR" residues are those variable domain residues other than the hypervariable region residues as herein defined.

As used herein, the term "hybridizes under stringent conditions" describes conditions for hybridization and washing under which nucleotide sequences at least 30% (*e.g.,* 35%, 40%, 45%, 50%, 55%, 60%, 65%, 70%, 75%, 80%, 85%, 90%, 95% or 98%) identical to each other typically remain hybridized to each other. Such stringent conditions are known to those skilled in the art and can be found in Current Protocols in Molecular Biology, John Wiley & Sons, N.Y. (1989), 6.3.1-6.3.6.

Generally, stringent conditions are selected to be about 5 to 10°C lower than the thermal melting point (Tm) for the specific sequence at a defined ionic strength pH. The Tm is the temperature (under defined ionic strength, pH, and nucleic concentration) at which 50% of the probes complementary to the target hybridize to the target sequence at equilibrium (as the target sequences are present in excess, at Tm, 50% of the probes are occupied at equilibrium). Stringent conditions will be those in which the salt concentration is less than about 1.0 M sodium ion, typically about 0.01 to 1.0 M sodium ion concentration (or other salts) at pH 7.0 to 8.3 and the temperature is at least about 30°C for short probes (for example, 10 to 50 nucleotides) and at least about 60°C for long probes (for example, greater than 50 nucleotides). Stringent conditions may also be achieved with the addition of destabilizing agents, for example, formamide. For selective or specific hybridization, a positive signal is at least two times background, preferably 10 times background hybridization.

In one, non-limiting example stringent hybridization conditions are hybridization at 6X sodium chloride/sodium citrate (SSC) at about 45°C, followed by one or more washes in 0.1X SSC, 0.2% SDS at about 68°C. In a non-limiting example, stringent hybridization conditions are hybridization in 6X SSC at about 45°C, followed by one or more washes in 0.2 X SSC, 0.1% SDS at 50-65°C *(i.e.,* one or more washes at 50°C, 55°C, 60°C or 65°C). It is understood that the nucleic acids of the invention do not include nucleic acid molecules that hybridize under these conditions solely to a nucleotide sequence consisting of only A or T nucleotides.

As used herein, the term "hyperproliferative cell disorder" or "excessive cell accumulation disorder" refers to a disorder that is not neoplastic, in which cellular hyperproliferation or any form of excessive cell accumulation causes or contributes to the pathological state or symptoms of the disorder. In some embodiments, the hyperproliferative cell or excessive cell accumulation disorder is characterized by hyperproliferating epithelial cells. Hyperproliferative epithelial cell disorders include, but are not limited to, asthma, COPD, lung fibrosis, bronchial hyper responsiveness, psoriasis, seborrheic dermatitis, and cystic fibrosis. In other embodiments, the hyperproliferative cell or excessive cell accumulation disorder is characterized by hyperproliferating endothelial cells. Hyperproliferative endothelial cell disorders include, but are not limited to restenosis, hyperproliferative vascular disease, Behcet's Syndrome, atherosclerosis, and macular degeneration. In other embodiments, the hyperproliferative cell or excessive cell accumulation disorder is characterized by hyperproliferating fibroblasts.

As used herein, the term "immunomodulatory agent" refers to an agent that modulates a subject's immune system. In particular, an immunomodulatory agent is an agent that alters the ability of a subject's immune system to respond to one or more foreign antigens. In a specific embodiment, an immunomodulatory agent is an agent that shifts one aspect of a subject's immune response. In another specific embodiment of the invention, an immunomodulatory agent is an agent that inhibits or reduces a subject's immune response (*i.e.,* an immunosuppressant agent). In one embodiment, an immunomodulatory agent that inhibits or reduces a subject's immune response inhibits or reduces the ability of a subject's immune system to respond to one or more foreign antigens.

As used herein, the term "in combination" refers to the use of more than one prophylactic and/or therapeutic agents. The use of the term "in combination" does not restrict the order in which prophylactic and/or therapeutic agents are administered to a subject in need of treatment. A first prophylactic or therapeutic agent can be administered prior to (*e.g*., 1 minute, 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (*e.g*., 1 minute, 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second prophylactic or therapeutic agent to a subject in need of treatment. Any additional prophylactic or therapeutic agent can be administered in any order with the other additional prophylactic or therapeutic agents. In certain embodiments, Eph/Ephrin Modulators of the invention can be administered in combination with one or more prophylactic or therapeutic agents (*e.g*., non-Eph/Ephrin Modulators currently administered to treat a disorder or disorder, analgesic agents, anesthetic agents, antibiotics, immunomodulatory agents).

As used herein, the term "isolated" in the context of an organic or inorganic molecule (whether it be a small or large molecule), other than a proteinaceous agent or a nucleic acid, refers to an organic or inorganic molecule substantially free of a different organic or inorganic molecule. In one embodiment, an organic or inorganic molecule is 60%, 65%, 70%, 75%, 80%, 85%, 90%, 95%, or 99% free of a second, different organic or inorganic molecule. In another embodiment, an organic and/or inorganic molecule is isolated.

As used herein, the term "isolated" in the context of a proteinaceous agent (*e.g*., a peptide, polypeptide, fusion protein, or antibody) refers to a proteinaceous agent which is substantially free of cellular material or contaminating proteins from the cell or tissue source from which it is derived, or substantially free of chemical precursors or other chemicals when chemically synthesized. The language "substantially free of cellular material" includes preparations of a proteinaceous agent in which the proteinaceous agent is separated from cellular components of the cells from which it is isolated or recombinantly produced. Thus, a proteinaceous agent that is substantially free of cellular material includes preparations of a proteinaceous agent having less than about 30%, 20%, 10%, or 5% (by dry weight) of heterologous protein, polypeptide, peptide, or antibody (also referred to as a "contaminating protein"). When the proteinaceous agent is recombinantly produced, it is also preferably substantially free of culture medium, *i.e*., culture medium represents less than about 20%, 10%, or 5% of the volume of the proteinaceous agent preparation. When the proteinaceous agent is produced by chemical synthesis, it is preferably substantially free of chemical precursors or other chemicals, *i.e*., it is separated from chemical precursors or other chemicals which are involved in the synthesis of the proteinaceous agent. Accordingly, such preparations of a proteinaceous agent have less than about 30%, 20%, 10%, 5% (by dry weight) of chemical precursors or compounds other than the proteinaceous agent of interest. In a specific embodiment, proteinaceous agents disclosed herein are isolated. In another specific embodiment, an antibody of the invention is isolated.

As used herein, the term "isolated" in the context of nucleic acid molecules refers to a nucleic acid molecule which is separated from other nucleic acid molecules which are present in the natural source of the nucleic acid molecule. Moreover, an "isolated" nucleic acid molecule, such as a cDNA molecule, is preferably substantially free of other cellular material, or culture medium when produced by recombinant techniques, or substantially free of chemical precursors or other chemicals when chemically synthesized. In a specific embodiment, nucleic acid molecules are isolated. In another specific embodiment, a nucleic acid molecule encoding an antibody of the invention is isolated.

As used herein, the term "low tolerance" refers to a state in which the patient suffers from side effects from treatment so that the patient does not benefit from and/or will not continue therapy because of the adverse effects and/or the harm from side effects outweighs the benefit of the treatment.

As used herein, the terms "manage", "managing" and "management" refer to the beneficial effects that a subject derives from a prophylactic or therapeutic agent, which does not result in a cure of the disorder. In certain embodiments, a subject is administered one or more prophylactic or therapeutic agents to "manage" a disorder so as to prevent the progression or worsening of the disorder (*i.e*., hold disease progress).

As used herein, the term "neoplastic" refers to a disease involving cells that have the potential to metastasize to distal sites and exhibit phenotypic traits that differ from those of non-neoplastic cells, for example, formation of colonies in a three-dimensional substrate such as soft agar or the formation of tubular networks or web-like matrices in a three-dimensional basement membrane or extracellular matrix preparation, such as MATRIGEL™. Non-neoplastic cells do not form colonies in soft agar and form distinct sphere-like structures in three-dimensional basement membrane or extracellular matrix preparations. Neoplastic cells acquire a characteristic set of functional capabilities during their development, albeit through various mechanisms. Such capabilities include evading apoptosis, self-sufficiency in growth signals, insensitivity to anti-growth signals, tissue invasion/metastasis, limitless replicative potential, and sustained angiogenesis. Thus, "non-neoplastic" means that the condition, disease, or disorder does not involve cancer cells.

As used herein, the phrase "non-responsive/ refractory" is used to describe patients treated with one or more currently available therapies (*e.g*., cancer therapies) such as chemotherapy, radiation therapy, surgery, hormonal therapy and/or biological therapy/immunotherapy, particularly a standard therapeutic regimen for the particular cancer, wherein the therapy is not clinically adequate to treat the patients such that these patients need additional effective therapy, *e.g*., remain unsusceptible to therapy. The phrase can also describe patients who respond to therapy yet suffer from side effects, relapse, develop resistance, etc. In various embodiments, "non-responsive/refractory" means that at least some significant portion of the cancer cells are not killed or their cell division arrested. The determination of whether particular cells are "non-responsive/refractory" can be made either *in vivo* or *in vitro* by any method known in the art for assaying the effectiveness of treatment such cells, using the art-accepted meanings of "refractory" in such a context. In various embodiments, a cell is "non-responsive/refractory" where the number of cells has not been significantly reduced, or has increased during the treatment.

As used herein, the term "pan-specific" refers to antibodies that specifically bind multiple members within a desired family or class of proteins (*e.g.* receptor tyrosine kinases). For example, a "pan-specific antibody" describes an antibody that binds more than one member within the Eph family of receptors (*e.g*. EphA1-A8, EphA10 EphB1-B6

As used herein, the phrase "pharmaceutically acceptable" means approved by a regulatory agency of the federal or a state government, or listed in the U.S. Pharmacopeia, European Pharmacopeia, or other generally recognized pharmacopeia for use in animals, and more particularly, in humans.

As used herein, the term "potentiate" refers to an improvement in the efficacy of a prophylactic or therapeutic agent at its common or approved dose.

As used herein, the terms "prevent," "preventing," and "prevention" refer to the inhibition of the development or onset of a disorder to be prevented, treated, managed or ameliorated by the methods of the present invention, or the prevention of the recurrence, onset, or development of one or more symptoms of such disorder resulting from the administration of a therapy (*e.g*., a prophylactic or therapeutic agent), or the administration of a combination of therapies (*e.g*., a combination of prophylactic or therapeutic agents).

As used herein, the terms "prophylactic agent" and "prophylactic agents" refer to any agent(s) that can be used in the prevention of the onset, recurrence or spread of a diesease or disorder associated with aberrant (*i.e*., increased, decreased or inappropriate) expression of one or more Eph receptors and/or Ephrins. In certain embodiments, the term "prophylactic agent" refers to an Eph/Ephrin Modulator of the invention. In certain other embodiments, the terms "prophylactic agent" and "prophylactic agents" refer to cancer chemotherapeutics, radiation therapy, hormonal therapy, and/or biological therapy (*e.g*., immunotherapy). In other embodiments, more than one prophylactic agent may be administered in combination with other agents prophylactic and/or therapeutic agents.

As used herein, a "prophylactically effective amount" refers to that amount of the prophylactic agent sufficient to result in the prevention of the recurrence, spread or onset of a disorder associated with aberrant (*i.e*., increased, decreased or inappropriate) Eph receptor and/or Ephrin expression. A prophylactically effective amount may refer to the amount of prophylactic agent sufficient to prevent the occurrence, spread or recurrence of a disorder in a patient associated with aberrant (*i.e*., increased, decreased or inappropriate) Eph receptor and/or Ephrin expression in a patient or subject, including but not limited to those patients predisposed to a such a disorder, for example those genetically predisposed or those having previously suffered from such a disorder. A prophylactically effective amount may also refer to the amount of the prophylactic agent that provides a prophylactic benefit in the prevention of a disorder associated with aberrant (*i.e*., increased, decreased or inappropriate) Eph receptor and/or Ephrin expression. Further, a prophylactically effective amount with respect to a prophylactic agent of the invention means that amount of prophylactic agent alone, or in combination with one or more other agents (*e.g*., non-Eph/Ephrin Modulators currently administered to treat the disorder, analgesic agents, anesthetic agents, antibiotics, immunomodulatory agents) that provides a prophylactic benefit in the prevention of a disorder associated with aberrant (*i.e*., increased, decreased or inappropriate) Eph receptor and/or Ephrin expression. Used in connection with an amount of an Eph/Ephrin Modulator of the invention, the term can encompass an amount that improves overall prophylaxis or enhances the prophylactic efficacy of or synergies with another prophylactic agent.

As used herein, a "protocol" includes dosing schedules and dosing regimens.

As used herein, the term "refractory" refers to a disorder associated with aberrant (*i.e*., increased, decreased or inappropriate) Eph receptor and/or Ephrin expression that is not responsive to a particular treatment. In a certain embodiment, that a disorder associated with aberrant (*i.e*., increased, decreased or inappropriate) Eph receptor and/or Ephrin expression is refractory to a therapy means that at least some significant portion of the symptoms associated with said disorder is not eliminated or lessened by that therapy. The determination of whether a disorder associated with aberrant (*i.e*., increased, decreased or inappropriate) Eph receptor and/or Ephrin expression is refractory can be made either *in vivo* or *in vitro* by any method known in the art for assaying the effectiveness of treatment of a disorder associated with aberrant (*i.e*., increased, decreased or inappropriate) Eph receptor and/or Ephrin expression.

As used herein, the phrase "side effects" encompasses unwanted and adverse effects of a prophylactic or therapeutic agent. Adverse effects are always unwanted, but unwanted effects are not necessarily adverse. An adverse effect from a prophylactic or therapeutic agent might be harmful or uncomfortable or risky. Side effects from chemotherapy include, but are not limited to, gastrointestinal toxicity such as, but not limited to, early and late-forming diarrhea and flatulence, nausea, vomiting, anorexia, leukopenia, anemia, neutropenia, asthenia, abdominal cramping, fever, pain, loss of body weight, dehydration, alopecia, dyspnea, insomnia, dizziness, mucositis, xerostomia, and kidney failure, as well as constipation, nerve and muscle effects, temporary or permanent damage to kidneys and bladder, flu-like symptoms, fluid retention, and temporary or permanent infertility. Side effects from radiation therapy include but are not limited to fatigue, dry mouth, and loss of appetite. Side effects from biological therapies/immunotherapies include but are not limited to rashes or swellings at the site of administration, flu-like symptoms such as fever, chills and fatigue, digestive tract problems and allergic reactions. Side effects from hormonal therapies include but are not limited to nausea, fertility problems, depression, loss of appetite, eye problems, headache, and weight fluctuation. Additional undesired effects typically experienced by patients are numerous and known in the art. Many are described in the Physicians' Desk Reference (56th ed., 2002).

As used herein, the term "single-chain Fv" or "scFv" refers to antibody fragments comprising the V_{H} and V_{L} domains of antibody, wherein these domains are present in a single polypeptide chain. Generally, the Fv polypeptide further comprises a polypeptide linker between the V_{H} and V_{L} domains which enables the scFv to form the desired structure for antigen binding. For a review of scFvs, see Pluckthun in The Pharmacology of Monoclonal Antibodies, vol. 113, Rosenburg and Moore eds. Springer-Verlag, New York, pp. 269-315 (1994).As used herein, the terms "subject" and "patient" are used interchangeably. As used herein, a subject is preferably a mammal such as a non-primate (*e.g*., cows, pigs, horses, cats, dogs, rats etc.) and a primate (*e.g*., monkey and human), most preferably a human. In one embodiment, the subject is a mammal, preferably a human, with a disorder associated with aberrant (*i.e*., increased, decreased or inappropriate) Eph receptor and/or Ephrin expression. In another embodiment, the subject is a farm animal (*e.g*., a horse, pig, or cow), a pet (*e.g.,* a guinea pig, dog or cat), or a laboratory animal (*e.g*., an animal model) with a disorder associated with aberrant (*i.e*., increased, decreased or inappropriate) Eph receptor and/or Ephrin expression. In another embodiment, the subject is a mammal, preferably a human, at risk of developing a disorder associated with aberrant (*i.e*., increased, decreased or inappropriate) Eph receptor and/or Ephrin expression (*e.g*., an immunocompromised or immunosuppressed mammal, or a genetically predisposed mammal). In another embodiment, the subject is not an immunocompromised or immunosuppressed mammal, preferably a human. In another embodiment, the subject is a mammal, preferably a human, with a lymphocyte count that is not under approximately 500 cells/mm³.

As used herein, the term "synergistic" refers to a combination of therapies (*e.g*., prophylactic or therapeutic agents) which is more effective than the additive effects of any two or more single therapies (*e.g*., one or more prophylactic or therapeutic agents). A synergistic effect of a combination of therapies (*e.g.*, a combination of prophylactic or therapeutic agents) permits the use of lower dosages of one or more of therapies (*e.g*., one or more prophylactic or therapeutic agents) and/or less frequent administration of said therapies to a subject with a disorder associated with aberrant *(i.e.,* increased, decreased or inappropriate) Eph receptor and/or Ephrin expression. The ability to utilize lower dosages of therapies (*e.g*., prophylactic or therapeutic agents) and/or to administer said therapies less frequently reduces the toxicity associated with the administration of said therapies to a subject without reducing the efficacy of said therapies in the prevention or treatment of a disorder associated with aberrant (*i.e*., increased, decreased or inappropriate) Eph receptor and/or Ephrin expression. In addition, a synergistic effect can result in improved efficacy of therapies (*e.g*., prophylactic or therapeutic agents) in the prevention or treatment of a disorder associated with aberrant (*i.e*., increased, decreased or inappropriate) Eph receptor and/or Ephrin expression. Finally, synergistic effect of a combination of therapies (*e.g*., prophylactic or therapeutic agents) may avoid or reduce adverse or unwanted side effects associated with the use of any single therapy.

As used herein, the term "therapeutic agent" refers to any agent that can be used in the treatment, management, prevention, amelioration or symptom reduction of a disorder associated with aberrant (*i.e*., increased, decreased or inappropriate) Eph receptor and/or Ephrin expression. As used herein, the term "therapeutic agent" refers to any agent that can be used in the treatment, management, prevention, amelioration or symptom reduction of a disorder associated with aberrant (*i.e*., increased, decreased or inappropriate) Eph receptor and/or Ephrin expression. In certain embodiments, the term "therapeutic agent" refers to an EphA/Ephrin Modulator of the invention. In certain other embodiments, the term "therapeutic agent" refers an agent other than an Eph/Ephrin Modulator of the invention. Preferably, a therapeutic agent is an agent which is known to be useful for, or has been or is currently being used for the prevention, treatment, management, or amelioration of disorder associated with aberrant (*i.e*., increased, decreased or inappropriate) Eph receptor and/or Ephrin expression, or one or more symptoms thereof.

As used herein, a "therapeutically effective amount" refers to that amount of the therapeutic agent sufficient to treat, manage, or ameliorate symptoms of a disorder associated with aberrant (*i.e.,* increased, decreased or inappropriate) Eph receptor and/or Ephrin expression, and, preferably, the amount sufficient to eliminate, modify, or control symptoms associated with such a disorder. A therapeutically effective amount may refer to the amount of therapeutic agent sufficient to delay or minimize the onset or severity of the disorder associated with aberrant (*i.e*., increased, decreased or inappropriate) Eph receptor and/or Ephrin expression. A therapeutically effective amount may also refer to the amount of the therapeutic agent that provides a therapeutic benefit in the treatment or management of a disorder associated with aberrant (*i.e*., increased, decreased or inappropriate) Eph receptor and/or Ephrin expression. Further, a therapeutically effective amount with respect to a therapeutic agent of the invention means that amount of therapeutic agent alone, or in combination with other therapies, that provides a therapeutic benefit in the treatment or management of a disorder associated with aberrant (*i.e*., increased, decreased or inappropriate) Eph receptor and/or Ephrin expression. Used in connection with an amount of an Eph/Ephrin Modulator of the invention, the term can encompass an amount that improves overall therapy, reduces or avoids unwanted effects, or enhances the therapeutic efficacy of or synergies with another therapeutic agent.

As used herein, the term "therapy" refers to any protocol, method and/or agent that can be used in the prevention, treatment, management or amelioration of a disorder associated with aberrant (*i.e*., increased, decreased or inappropriate) Eph receptor and/or Ephrin expression. In certain embodiments, the terms "therapies" and "therapy" refer to a biological therapy, supportive therapy, and/or other therapies useful in treatment, management, prevention, or amelioration of a disorder associated with aberrant (*i.e*., increased, decreased or inappropriate) Eph receptor and/or Ephrin expression or one or more symptoms thereof known to one of skill in the art such as medical personnel.

As used herein, the terms "treat", "treating" and "treatment" refer to the eradication, reduction or amelioration of symptoms of a disorder, particularly, the eradication, removal, modification, or control of a disorder associated with aberrant (*i.e*., increased, decreased or inappropriate) Eph receptor and/or Ephrin expression that results from the administration of one or more therapies (*e.g*., prophylactic or therapeutic agents). In certain embodiments, such terms refer to the minimizing or delay of the spread of the a disorder associated with aberrant (*i.e.*, increased, decreased or inappropriate) Eph receptor and/or Ephrin expression resulting from the administration of one or more therapies (*e.g*., prophylactic or therapeutic agents) to a subject with such a disorder.

### 4. DESCRIPTION OF THE FIGURES

**FIG. 1A-1R**. Structural features of the human Eph family receptors. The consensus sequences are delineated by the boxed sequences. The signal sequence is represented by the dashed line; the Ephrin ligand binding domain is represented by bold line line; the tumor necrosis factor receptor (TNFR) domain is represented by the double-dashed lines; the fibronectin type III domains are represented by the double lines; the transmembrane is represented by fine dotted line; the tyrosine kinase catalytic domain is depicted by a single plain line; and the sterile alpha motif (SAM) domain is represented by large dotted line. The GenBank accession numbers for each of the human Eph receptor nucleotide and amino acid sequences are listed in Table 1, *supra.*

**FIG. 2A-2G**. Structural features of the human Ephrin family ligands. The consensus sequences are delineated by the boxed sequences. The signal sequence is represented by the dotted line; the Ephrin domain is represented by the single bold line; and the transmembrane domain (for B-type Ephrins only) is represented by the double lines. The GenBank accession numbers for each of the human Ephrin nucleotide and amino acid sequences are listed in Table 2, *supra*

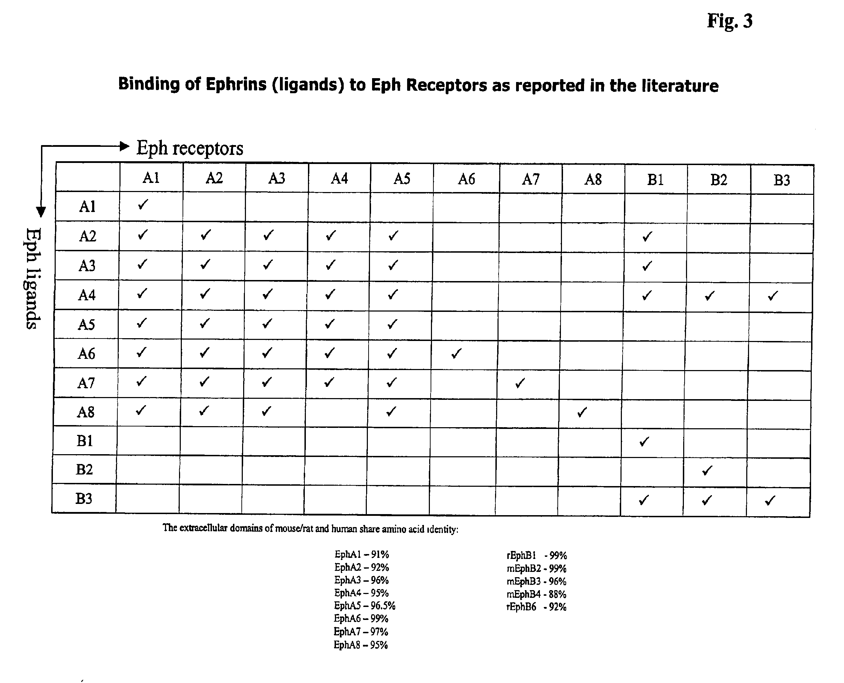

**FIG. 3****.** Binding of Ephrins (ligands) to Eph receptors as reported in the literature. The Eph receptors are listed across the top row of the chart; the Eph ligands (Ephrins) are listed down the first column of the chart. A checked box indicates binding of the Ephrin to the corresponding Eph receptor. Percent identity between human Eph receptors and the corresponding mouse/rat Eph receptors is listed below the table.

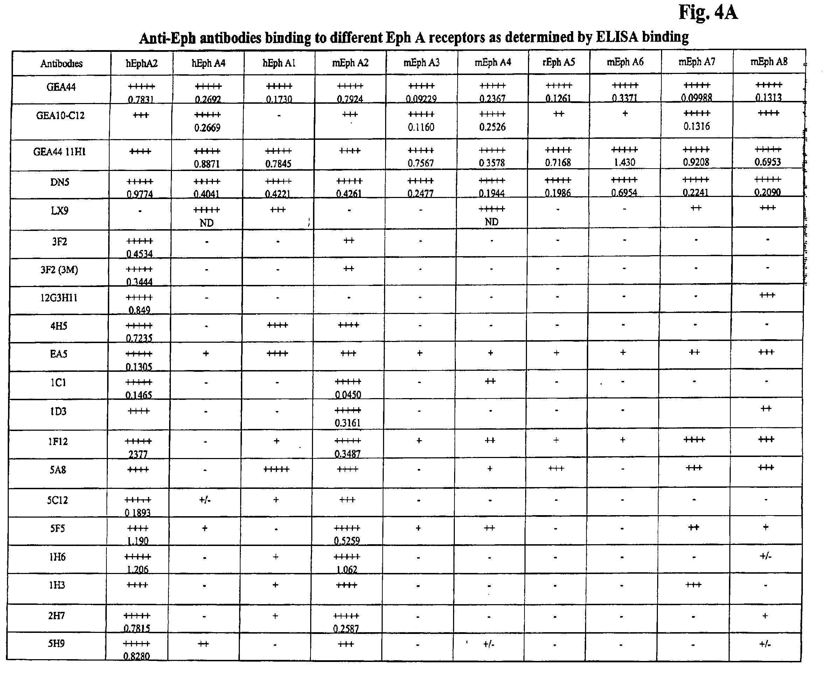

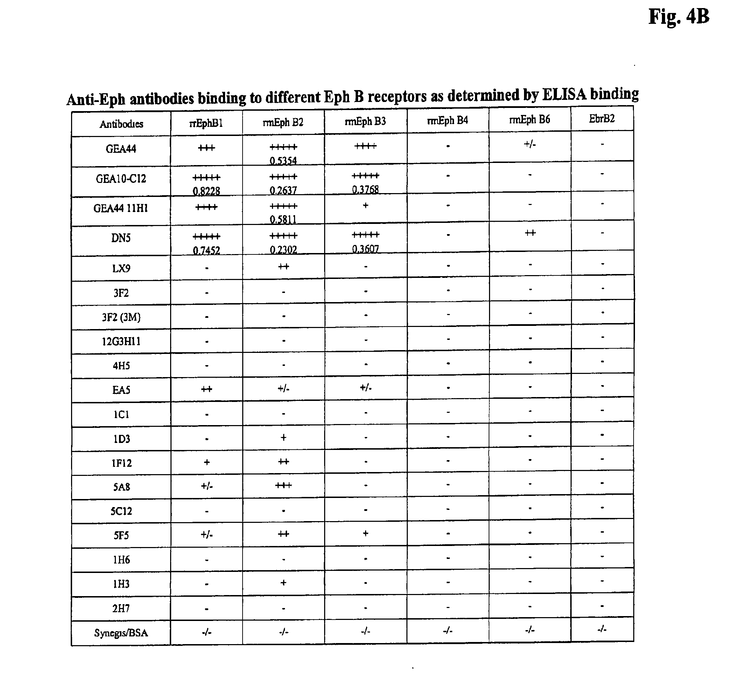

**FIG. 4A-4B****.** Anti-Eph antibodies binding to different EphA and EphB receptors as determined by ELISA binding. This table summarizes the binding affinities of the different antibodies to various Eph receptors. The Eph receptors are listed across the top row of the chart; the antibodies are listed down the first column of the chart. Binding is indicated using (-) or (+) signs, with increased binding indicated by multiple (+) signs. Additionally, ELISA O.D. values are listed for several antibodies. The data from these two figures demonstrates the generation of pan-specific antibodies to a member of the receptor tyrosine kinase family of receptors (the Eph family of receptors).

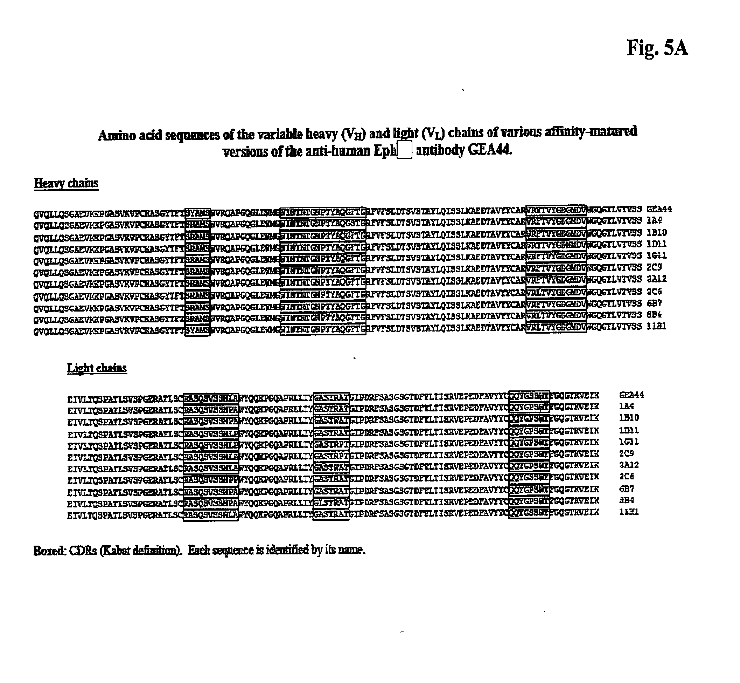

**FIG. 5A****.** Amino acid sequences of the variable heavy (V_{H}) and light (V_{L}) chains of various affinity-matured versions of the anti-Eph antibody GEA44. Amino acid sequences for the heavy chains of the following antibodies are listed in the upper half of the Figure: GEA44, 1A4, 1B10, 1D11, 1G11, 2C9, 3A12, 3C6, 6B7, 6B4, and 11H1 (SEQ ID Nos. 114, 116, 118, 120, 122, 124, 126, 128, 130, 132, and 134, respectively). Amino acid sequences for the light chains of the following antibodies are listed in the lower half of the Figure: GEA44, 1A4, 1B10, 1D11, 1G11, 2C9, 3A12, 3C6, 6B7, 6B4, and 11H1 (SEQ ID Nos. 115, 117, 119, 121, 123, 125, 127, 129, 131, 133, and 135, respectively). The boxed portion of the sequences indicates the CDRs (Kabat definition), with their corresponding SEQ ID Nos. listed in Table 3.

**Table 3.**

| **Antibody** | **V_{H} CDRs SEQ ID Nos.** | **V_{L} CDRs SEQ ID Nos.** |
|---|---|---|
| GEA44 | 136-138 | 139-141 |
| 1A4 | 142-144 | 145-147 |
| 1B10 | 148-150 | 151-153 |
| 1D11 | 154-156 | 157-159 |
| 1G11 | 160-162 | 163-165 |
| 2C9 | 166-168 | 169-171 |
| 3A12 | 172-174 | 175-177 |
| 3C6 | 178-180 | 181-183 |
| 6B7 | 184-186 | 187-189 |
| 8B4 | 190-192 | 193-195 |
| 11H1 | 196-198 | 199-201 |

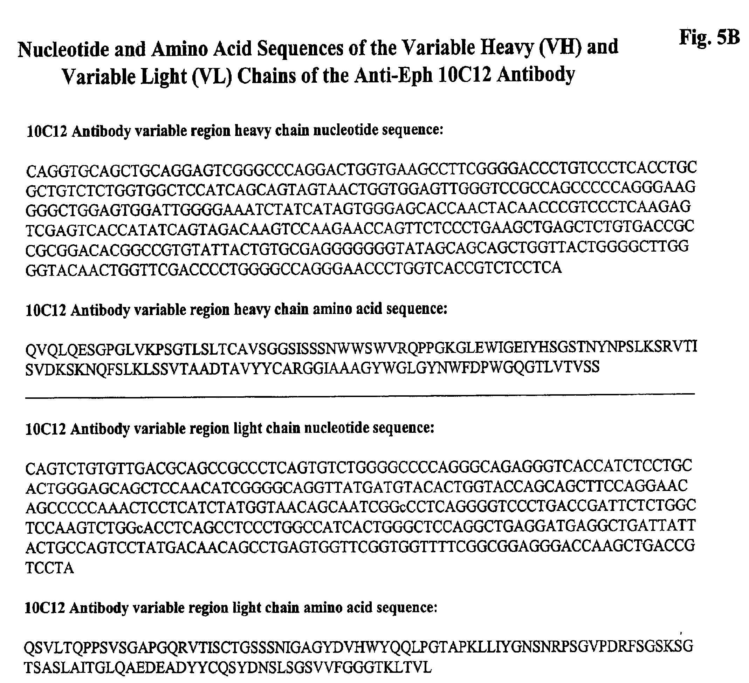

**FIG. 5B**. Nucleotide and amino acid sequences of the variable heavy (V_{H}) and variable light (V_{L}) chains of the pan-Eph antibody 10C12 (GEA10C12). The variable region heavy chain nucleotide and amino acid sequences are listed in the upper half of the Figure (SEQ ID's Nos. 203 and 202, respectively); the variable region light chain nucleotide and amino acid sequences are listed in the lower half of the Figure (SEQ ID's Nos 205 and 204, respectively).

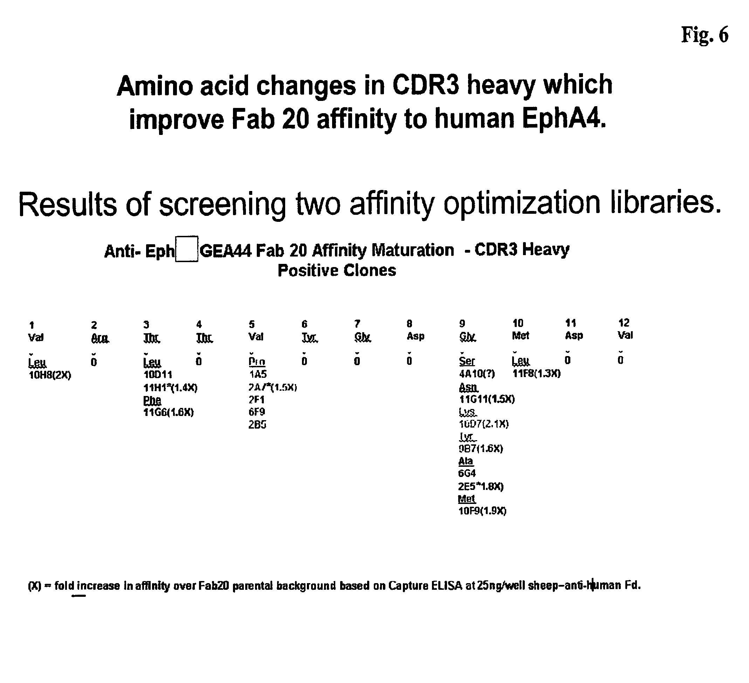

**FIG. 6****.** Amino acid changes in CDR3 heavy, which improve Fab20 affinity to human EphA4. The amino acid sequence of the Fab20 (Fab from the mAb GEA44) CDR3 heavy is listed across the Figure, with a corresponding number above each amino acid (1-12). The substituted Fab20 amino acid is listed below the numbered position, along with the antibody name. (x) represents the fold increase in affinity over the Fab20 parental background based on a capture ELISA.

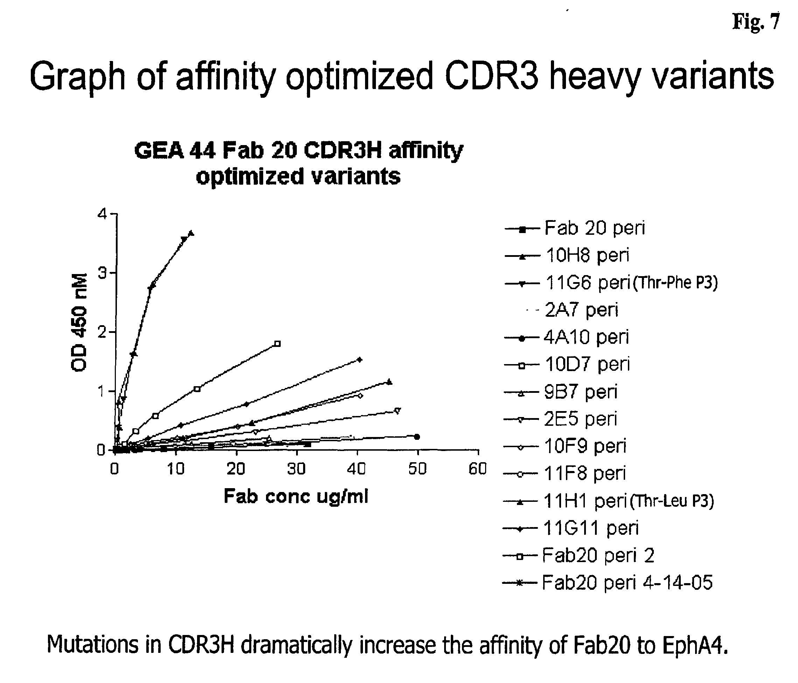

**FIG. 7****.** Graph of affinity optimized CDR3 heavy variants of GEA44. The graph summarizes ELISA results comparing EphA4 binding of the GEA44 Fab20 to affinity optimized variants. Comparisons are between Fab20, 10H8, 11G6, 2A7, 4A10, 10D7, 9B7, 2E5, 10F9, 11F8, 11H1, and 11G11. Fab concentration in µg/ml is on the x-axis and O.D. 450nm is on the y-axis. The results demonstrate that mutations in CDR3 heavy dramatically increase the affinity of Fab20 to EphA4.

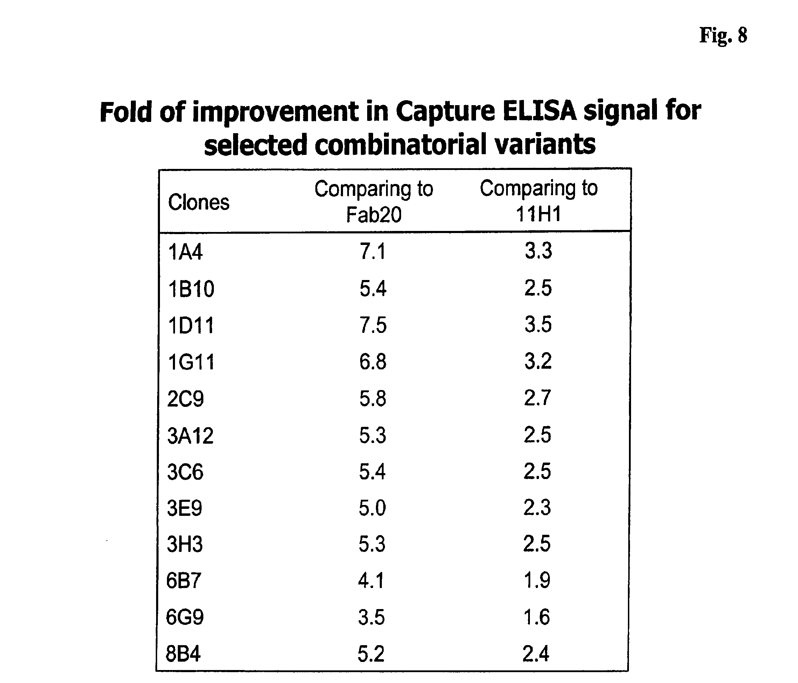

**FIG. 8****.** Fold of improvement in capture ELISA signal for selected combinatorial variants. The table summarizes the increase in affinity to EphA4 of affinity optimized variants of GEA44 Fab20. The first column lists the antibody clones; the second column lists the fold increase in binding affinity of each clone as compared to the parental Fab20; the third column lists the fold increase in binding affinity of each clone as compared to the GEA44 derivative 11H1.

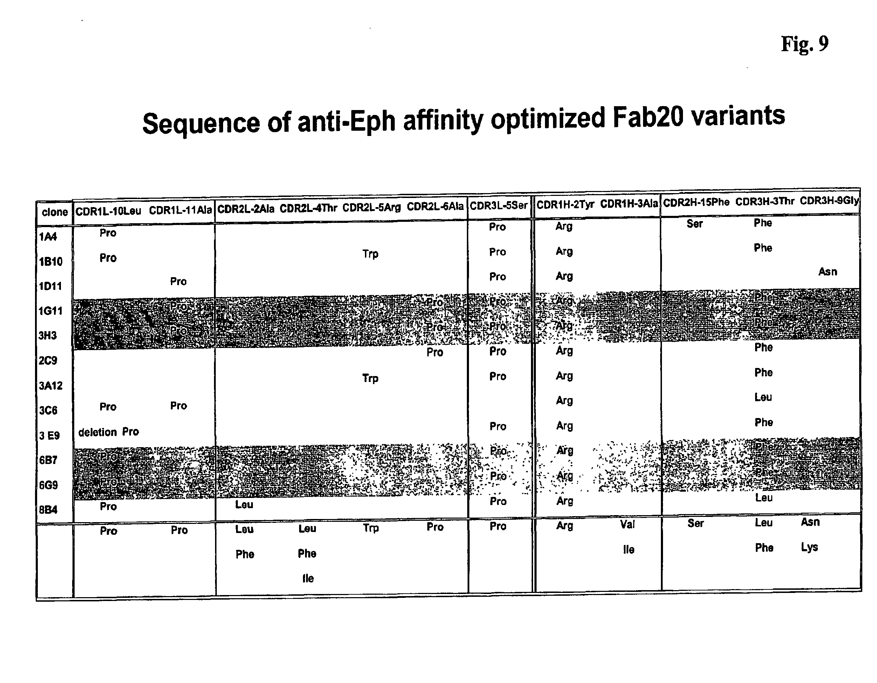

**FIG. 9****.** Sequence of anti-Eph affinity optimized Fab20 variants. The amino acid substitutions within the CDRs of each Fab20 variant is summarized in this table. The Fab20 variants (clones) are listed down the first column; the CDRs (CDR_{L}1-3 and CDR_{H}1-3) are listed across the top row. The substituted Fab20 amino acid is listed below the corresponding CDR.

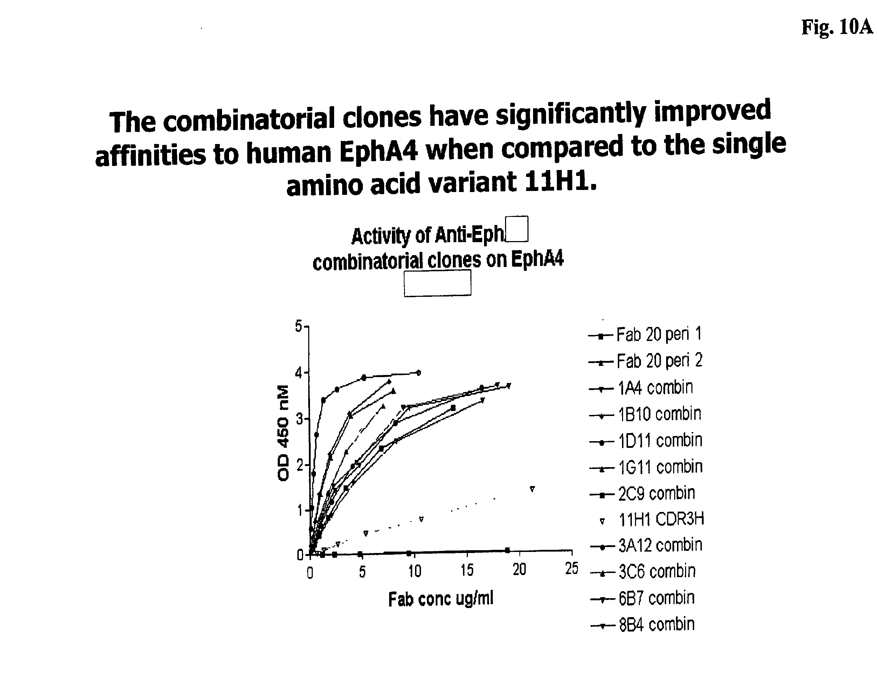

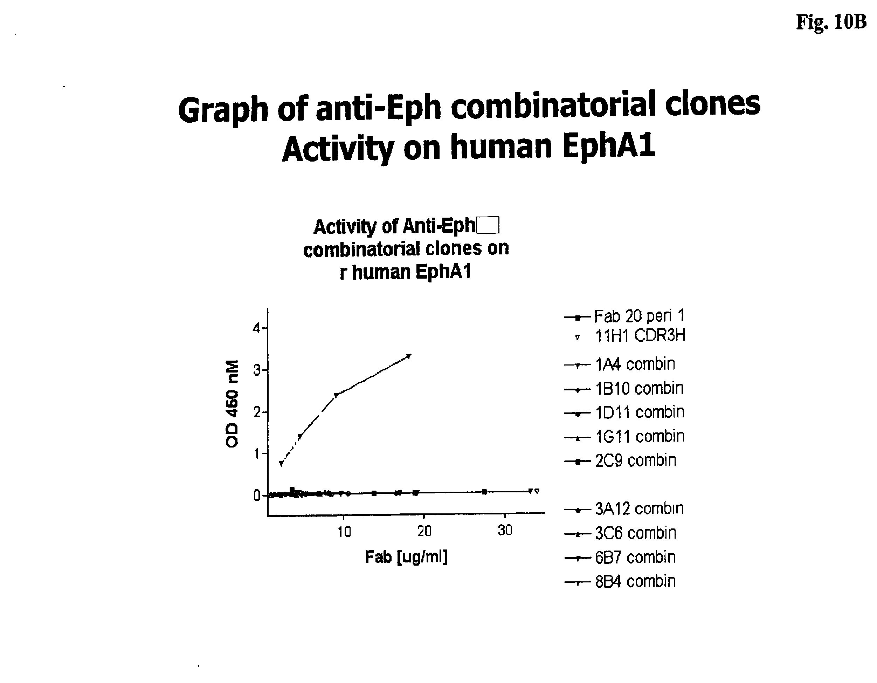

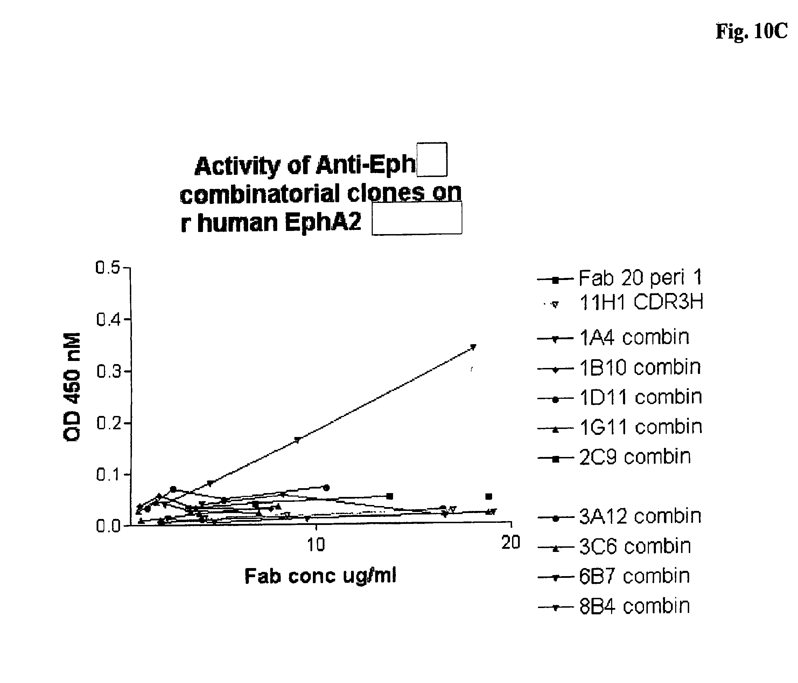

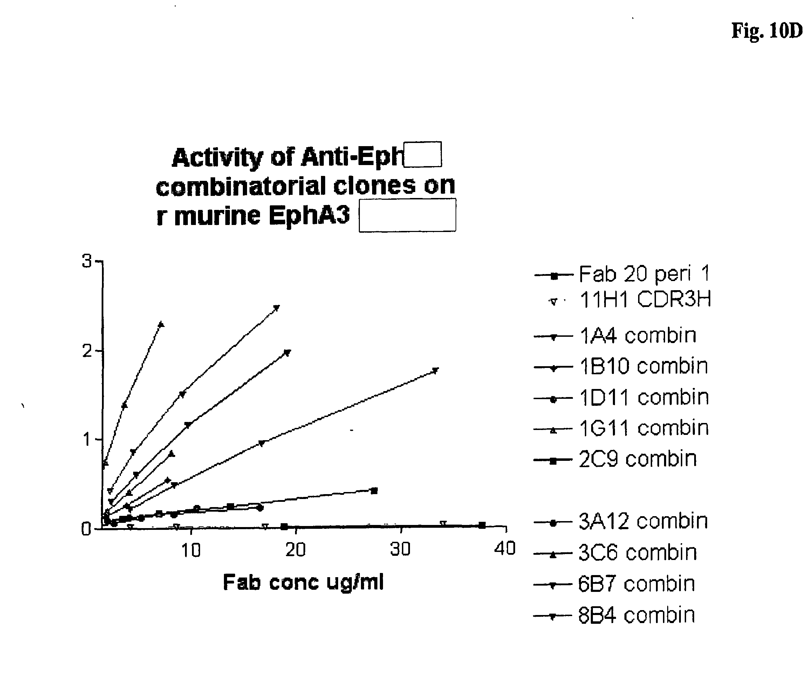

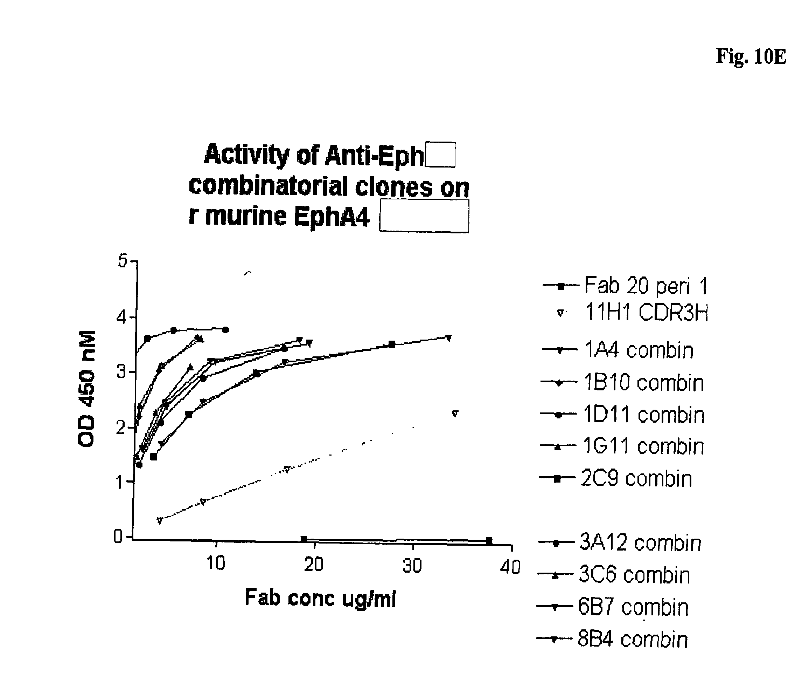

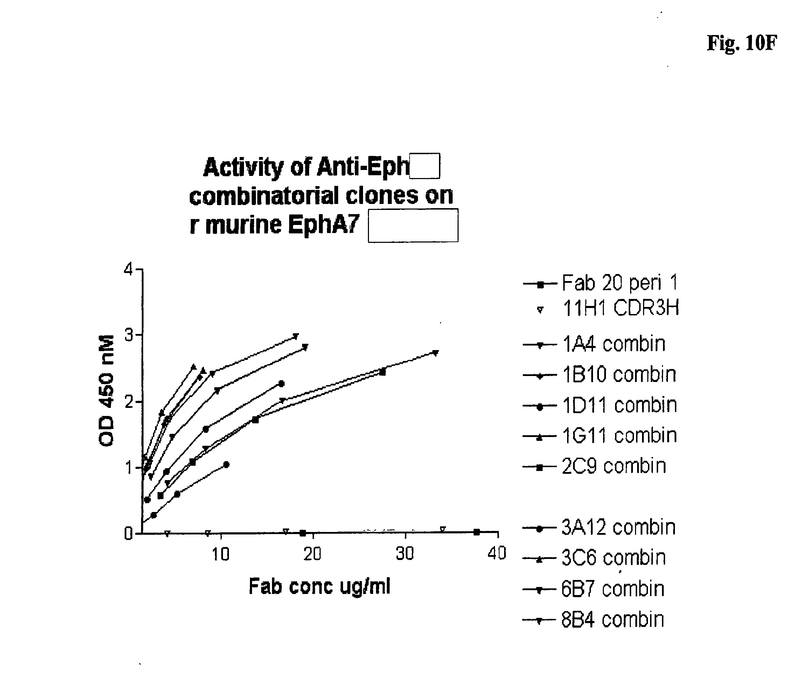

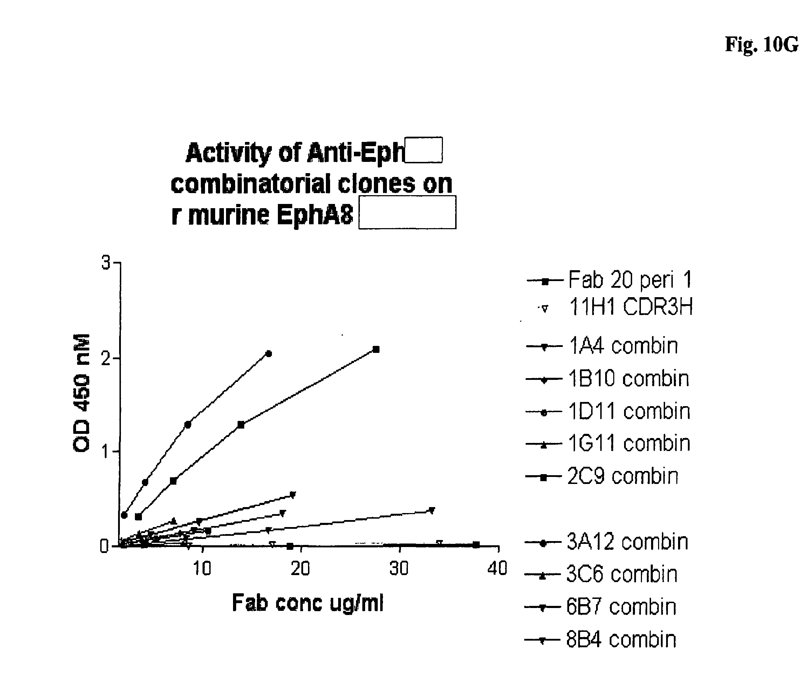

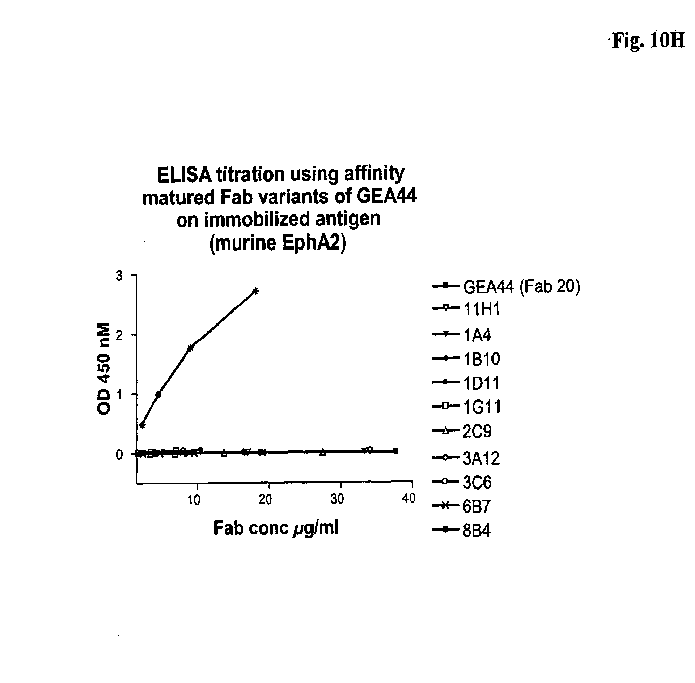

**FIG. 10A-10H****.** Graphs of affinity optimized variants of GEA44. These graphs summarize ELISA results comparing binding of the GEA44 Fab20 to the different affinity optimized variants. Fig. 10A compares binding to r-human EphA4; Fig. 10B compares binding to r-human EphA1; Fig. 10C compares binding to r-human EphA2; Fig. 10D compares binding to r-murine EphA3; Fig. 10E compares binding to r-murine EphA4; Fig. 10F compares binding to r-murine EphA7; Fig. 10G compares binding to r-murine EphA8; and Fig. 10H compares binding to r-murine EphA2. Fab concentration in µg/ml is on the x-axis and O.D. 450nm is on the y-axis of each graph. The results demonstrate the markedly altered affinities of the different variants towards the various Eph receptors.

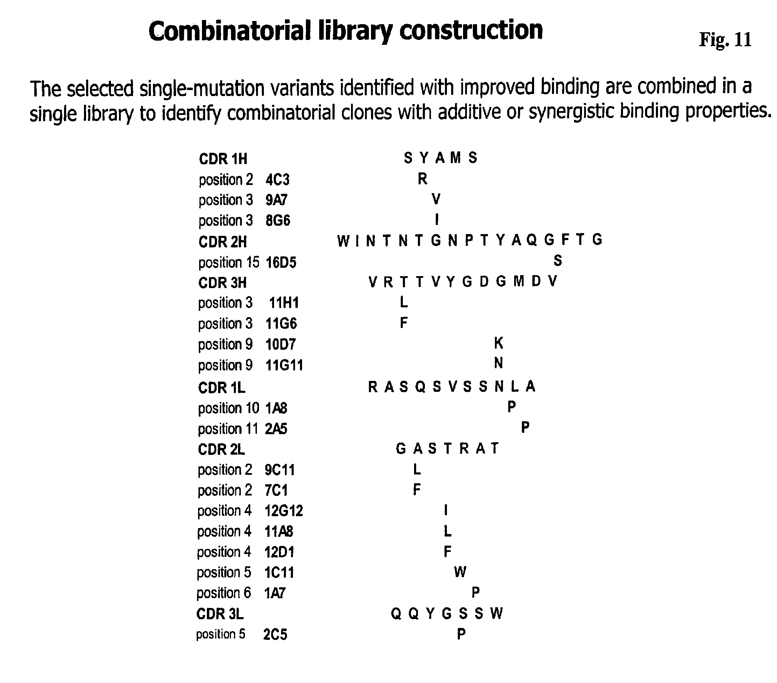

**FIG. 11****.** Combinatorial library construction. This figure is another representation of the various amino acid substitutions made within the CDRs of GEA44. The CDRs and corresponding substituted amino acid position are listed in the first column; the second column lists the clones that correspond to that substitution; the last column lists the amino acid sequence for each corresponding CDR, with the substituted amino acid listed below.

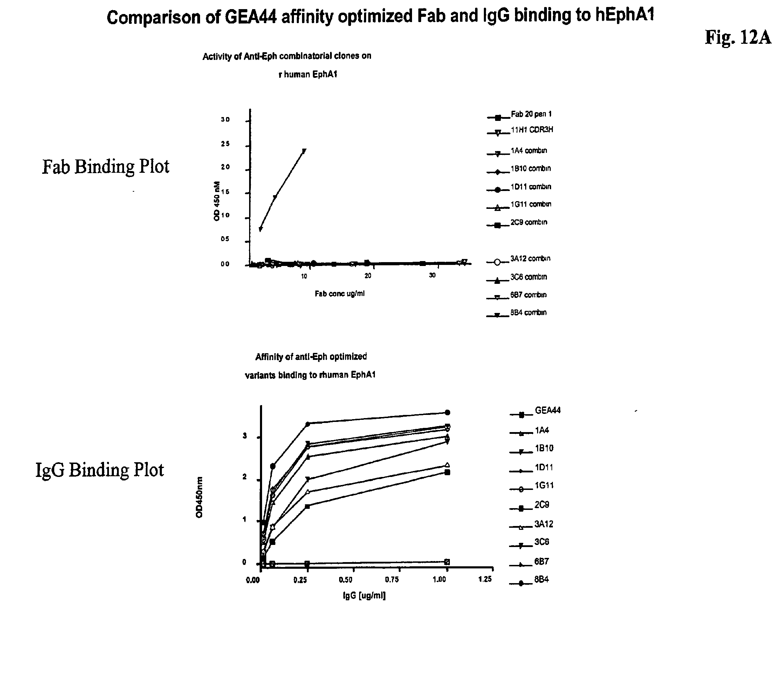

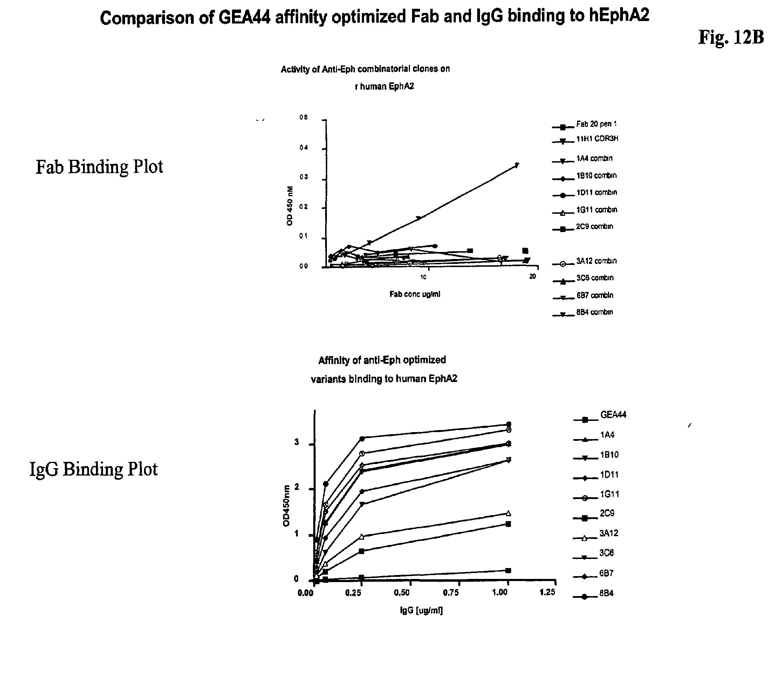

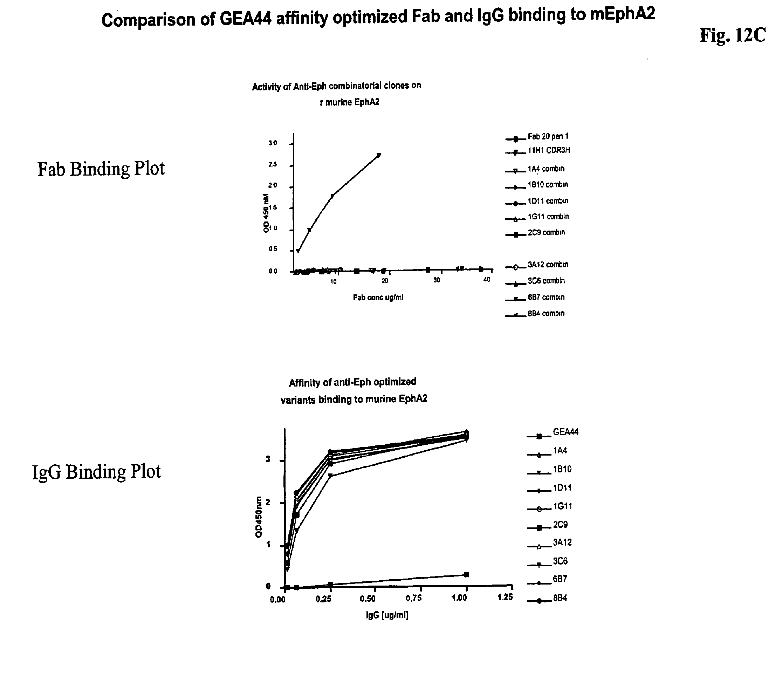

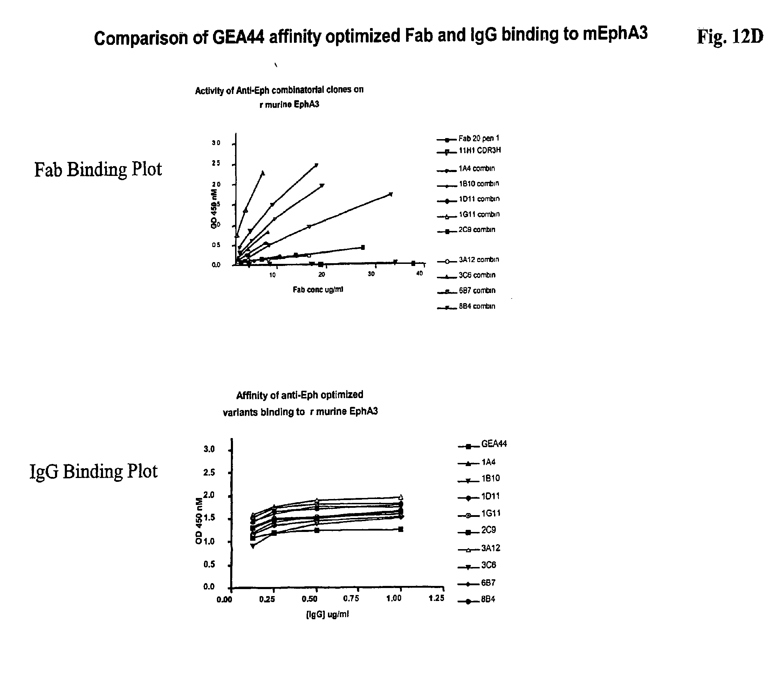

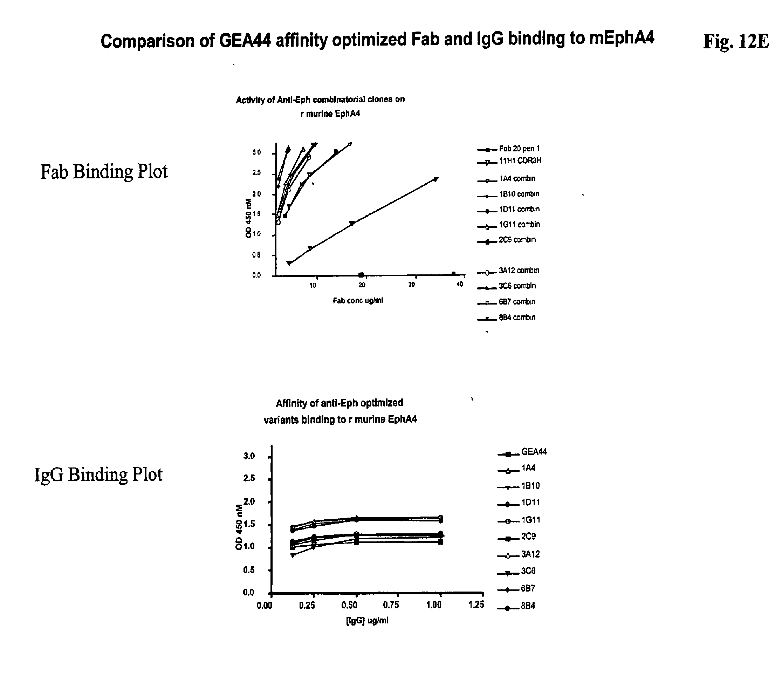

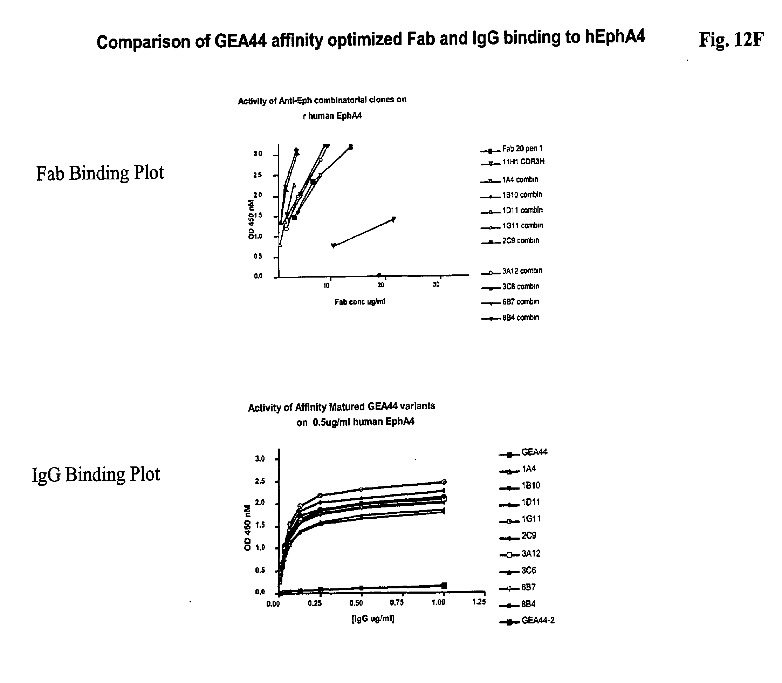

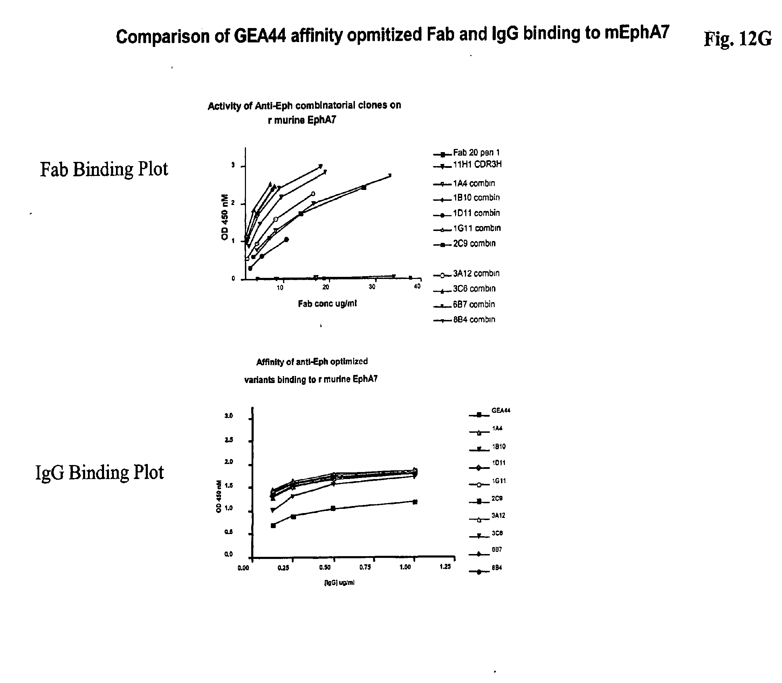

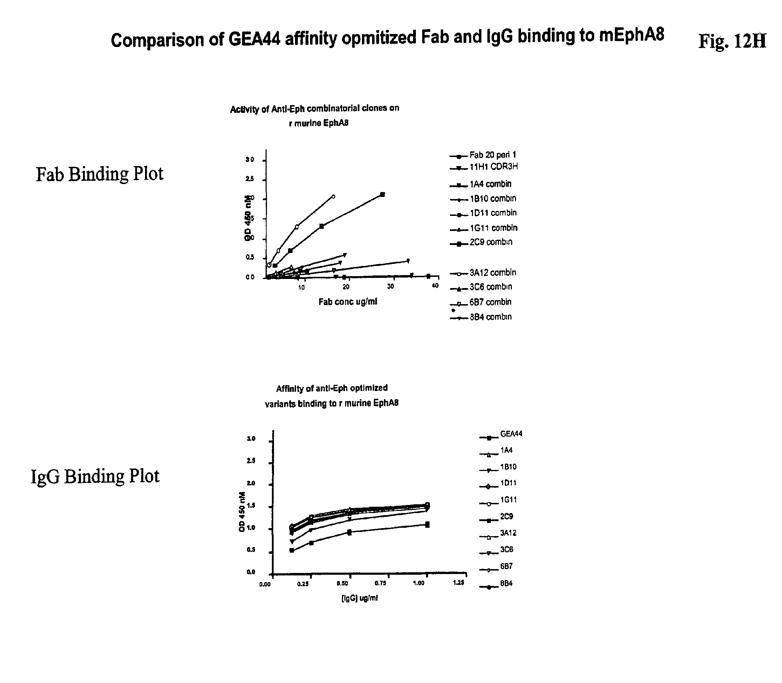

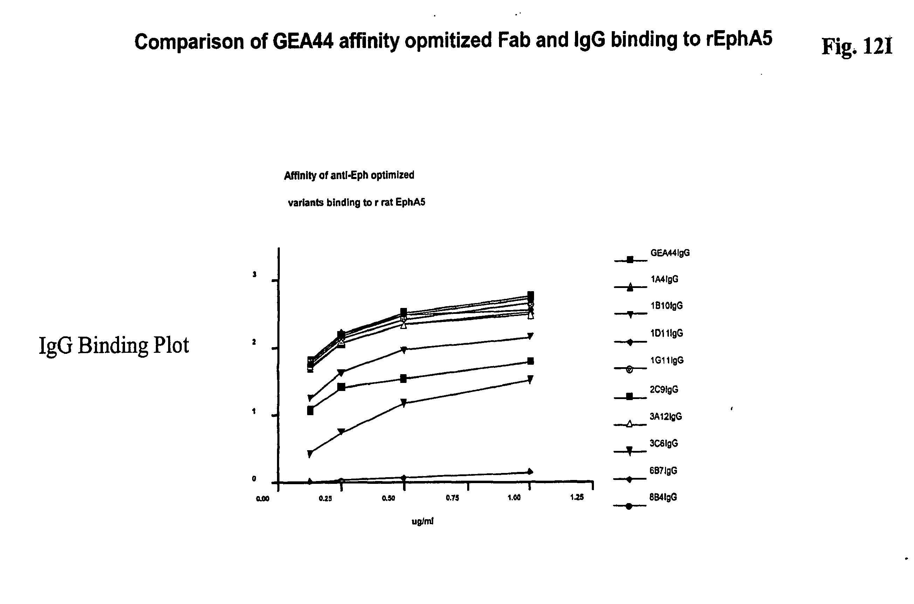

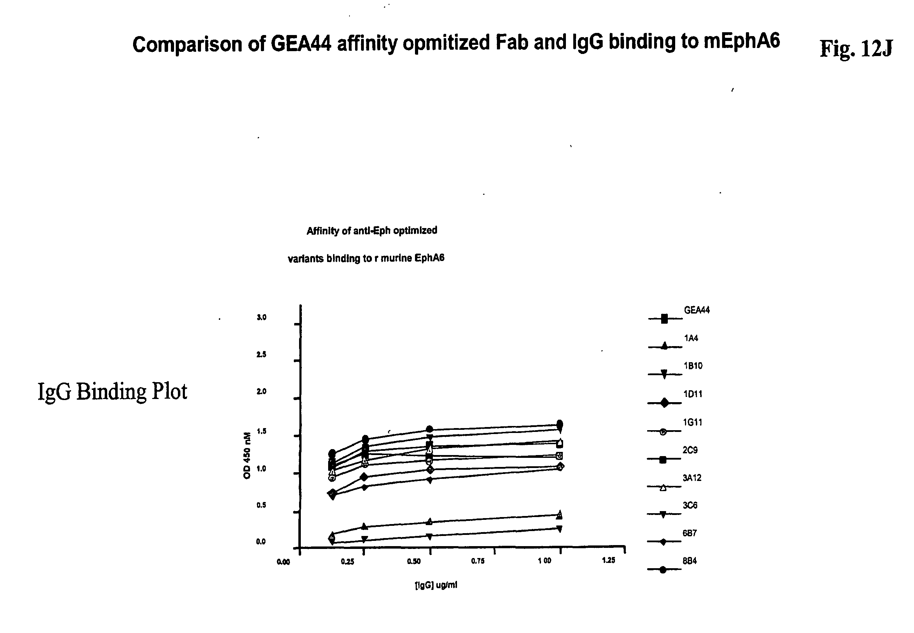

**FIG. 12A-12J****.** Graphs of comparisons of GEA44 affinity optimized Fab and IgG binding to various Eph receptors. The graphs summarize ELISA results comparing binding of the GEA44 Fab20 and GEA44 IgG to the different affinity optimized variants (Fab and corresponding IgG). Fig. 12A compares binding to r-human EphA1; Fig. 12B compares binding to r-human EphA2; Fig. 12C compares binding to r-murine EphA2; Fig. 12D compares binding to r-murine EphA3; Fig. 12E compares binding to r-murine EphA4; Fig. 12F compares binding to r-human EphA4; Fig. 12G compares binding to r-murine EphA7; Fig. 12H compares binding to r-murine EphA8; Fig. 12I compares binding to r-rat EphA2; and Fig. 12J compares binding to r-murine EphA6. Fab and IgG concentration in µg/ml is on the x-axis and O.D. 450nm is on the y-axis of each graph. The results demonstrate the markedly altered affinities of the different variants towards the various Eph receptors.

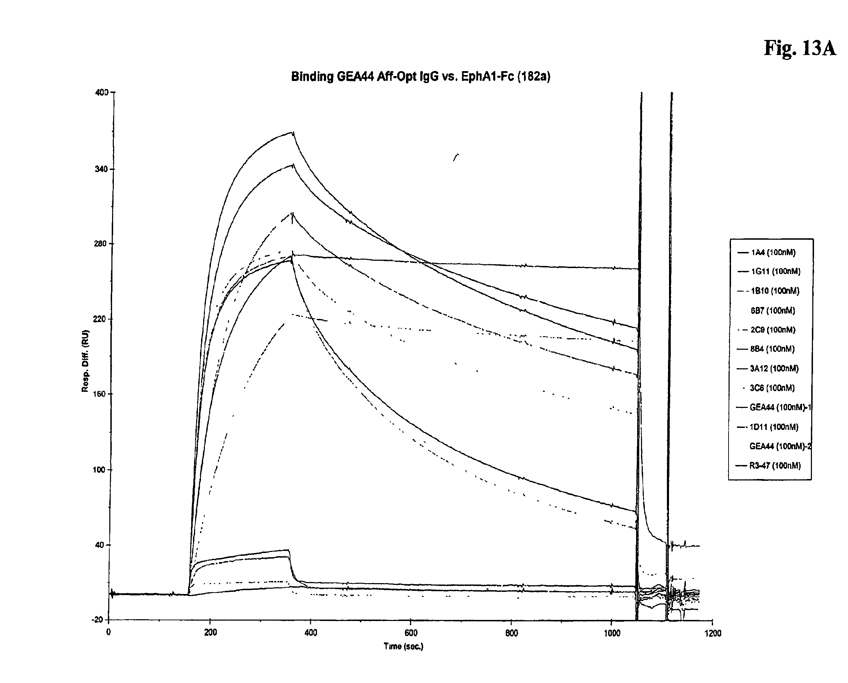

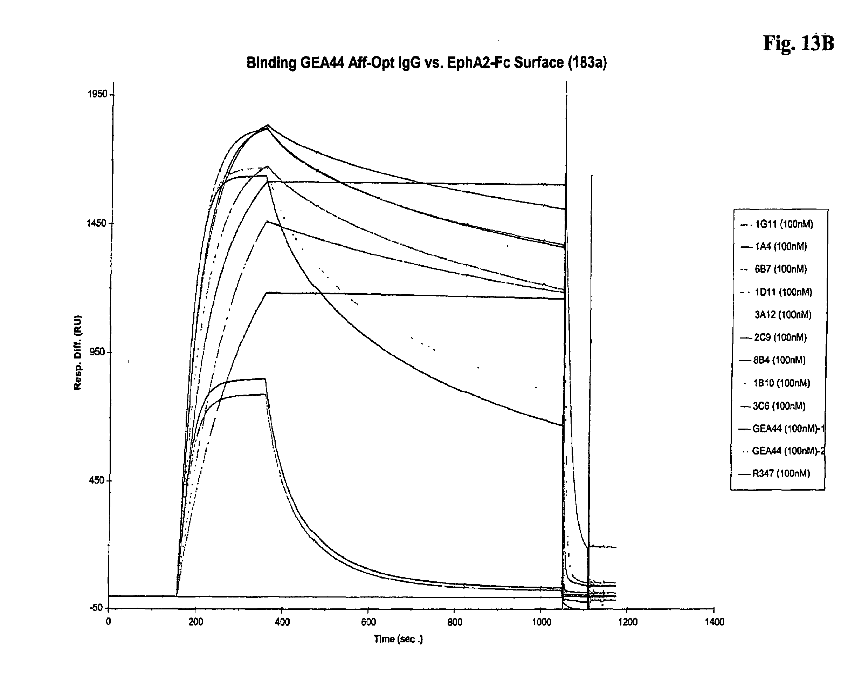

**FIG. 13A-13B****.** Binding affinities of GEA44 affinity optimized variants against EphA1-Fc. Binding affinities were measured using the BIAcore assay. Generally, the EphA-Fc fusion was immobilized on the sensor chip surfaces and the IgG's that were measured were flowed over the immobilized EphA receptor. BIAcore data was generated using immobilized antigens human EphA1 and human EphA2 surfaces, using each IgG variant mAb at 100nM concentration as analyte. Fig. 13A summarizes the affinity results against EphA1-Fc; Fig. 13B summarizes the affinity results against EphA2-Fc. Time in seconds is on the x-axis and Resp. Diff. is on the y-axis. The results further demonstrate the markedly altered affinities of the different variants towards a single Eph receptor.

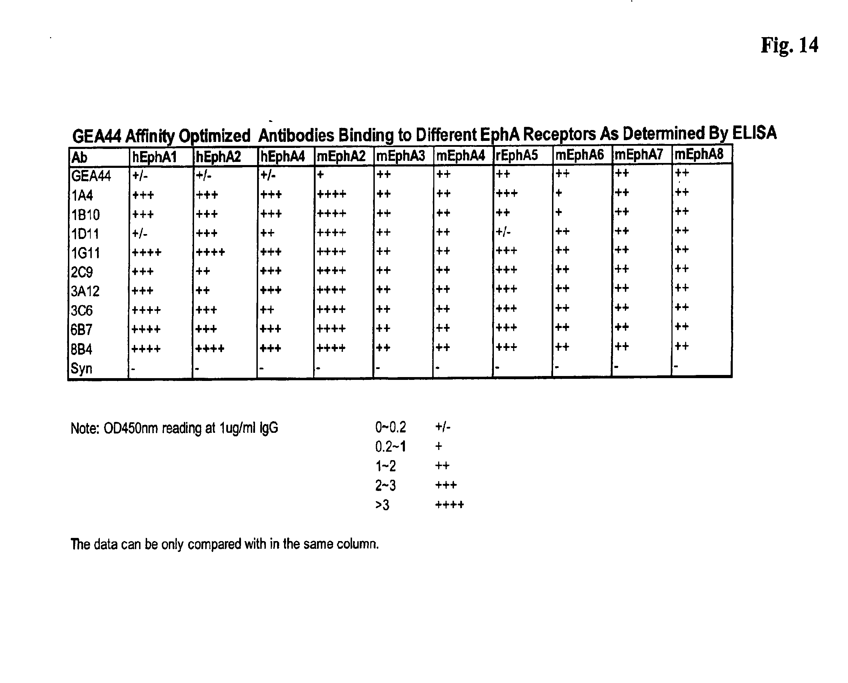

**FIG. 14****.** GEA44 affinity optimized antibodies binding to different EphA receptors as determined by ELISA. This figure summarizes the binding affinities of the different GEA44 affinity optimized variants to the various EphA receptors. The Eph receptors are listed across the top row of the chart; the antibodies are listed down the first column of the chart. Binding is indicated using (-) or (+) signs, with increased binding indicated by multiple (+) signs.

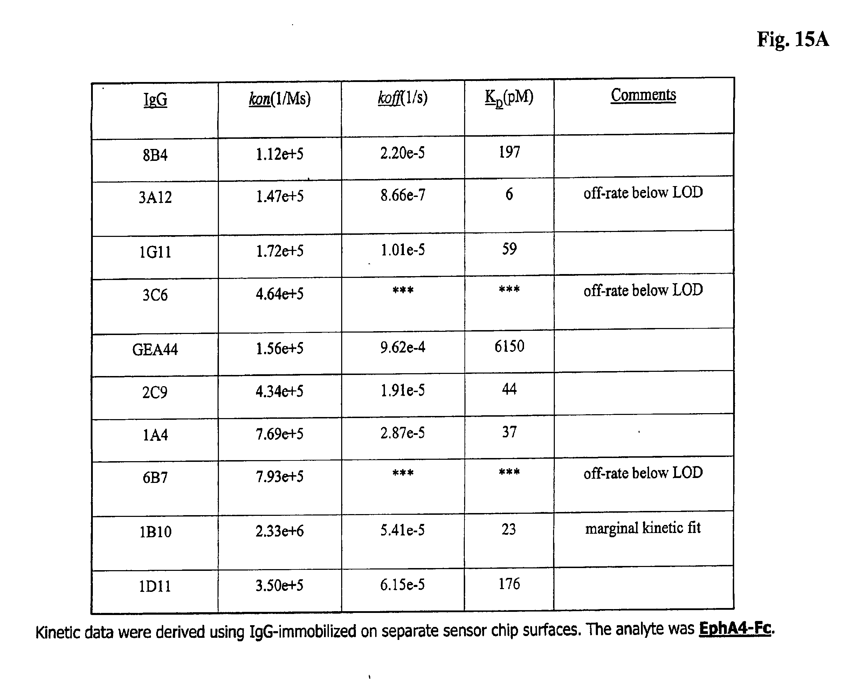

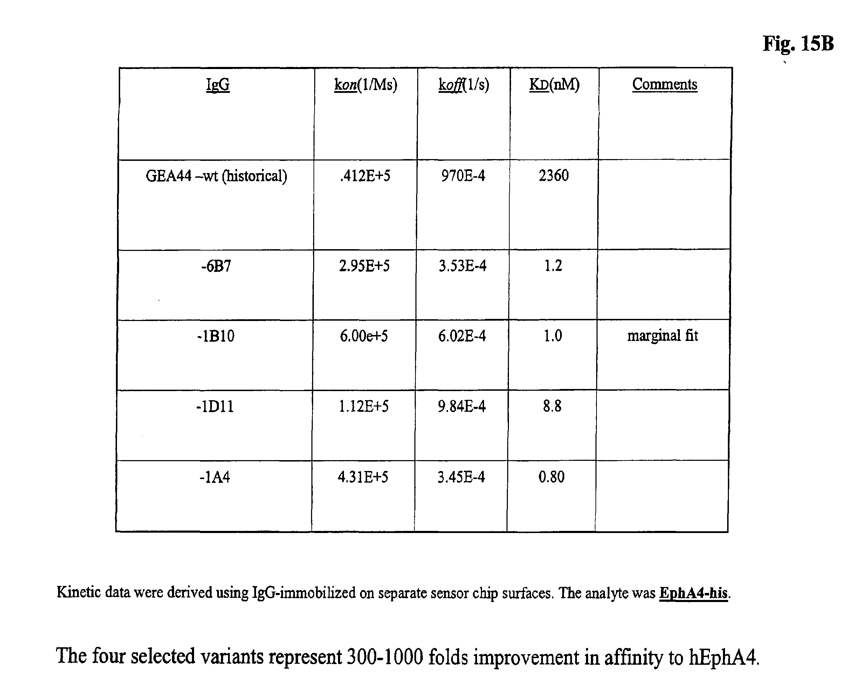

**FIG. 15A-15B****.** Affinities of anti-Eph IgG antibodies to EphA4-Fc and EphA4-his. Binding affinities were measured using the BIAcore assay. Generally, the IgG antibodies were immobilized on the sensor chip surfaces and the EphA4 analyte was flowed over the immobilized antibodies. Variant kinetic measurements for human EphA4 and were obtained using BIAcore with immobilized variant antibodies and human EphA4-Fc and EphA4-His as analytes. These tables summarize the binding affinities of the various affinity matured variants of GEA44 to EphA4. The first column lists the different antibodies; the second column gives kon(1/Ms) values; the third column gives *koff*(1/s) values; the fourth column gives K_{D}(nM) values; and the last column provides comments where appropriate. The data clearly demonstrates the marked increase in affinity to EphA4 of the affinity matured variants as compared to the parental GEA44.

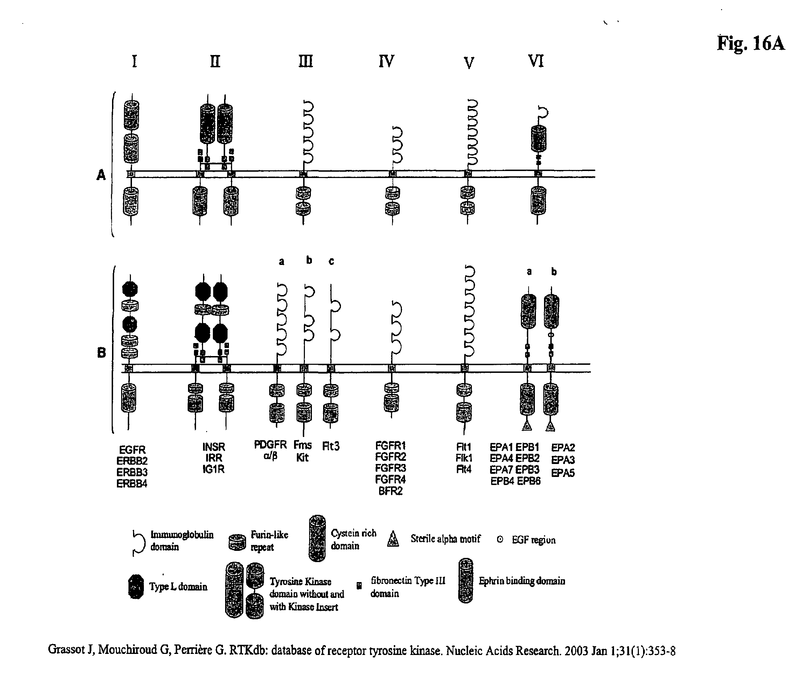

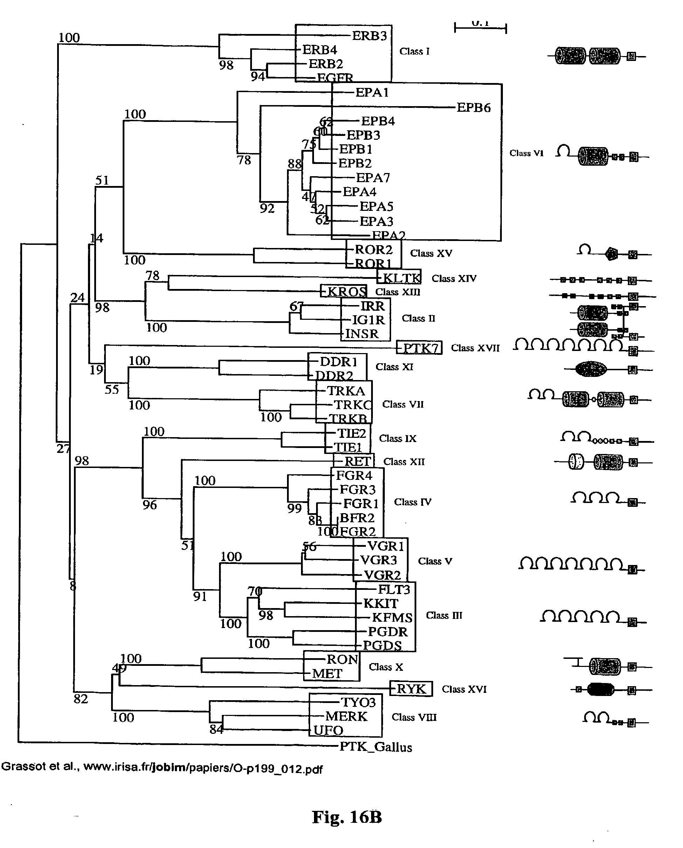

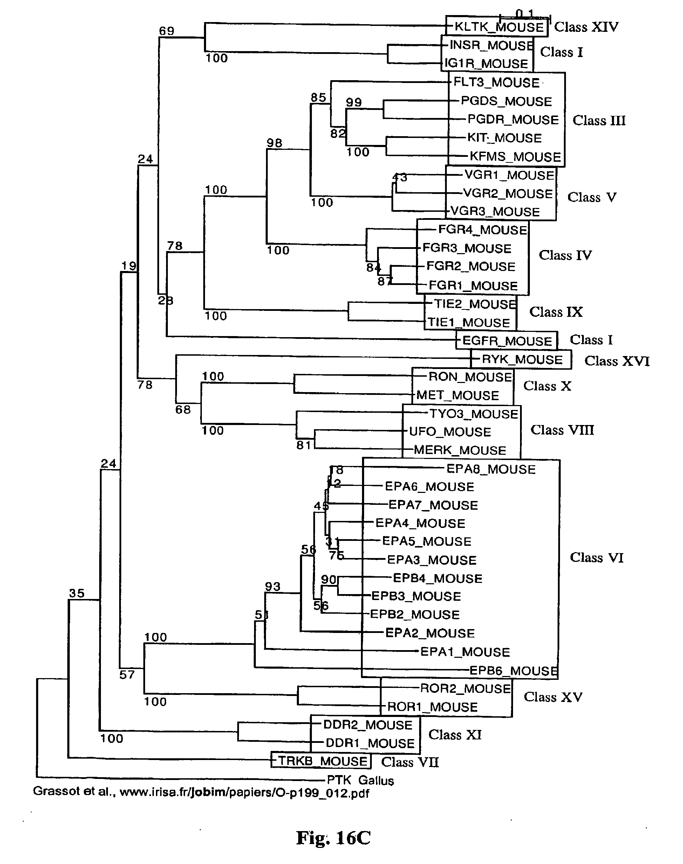

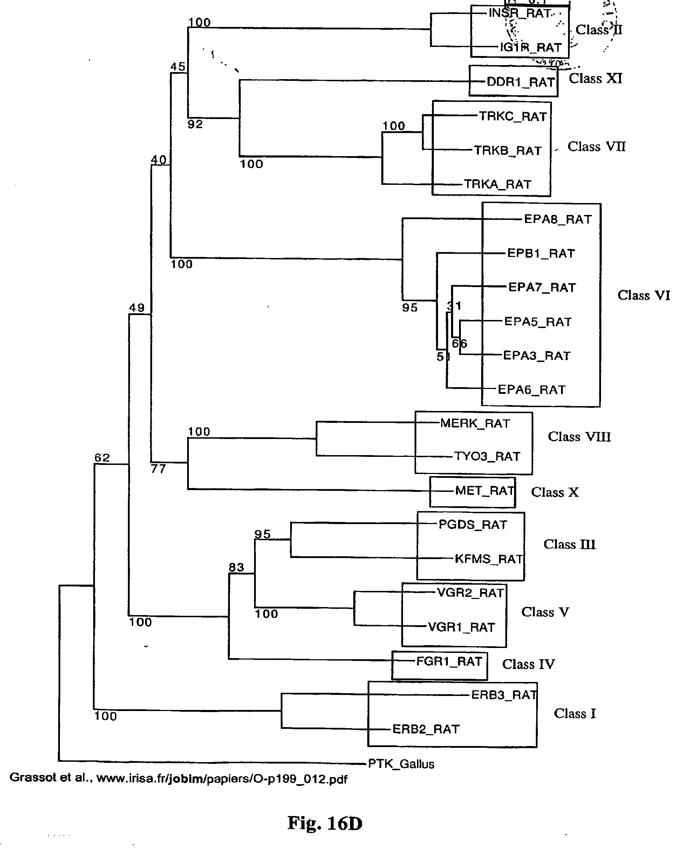

**FIG. 16A-16D****.** Members of the Receptor Tyrosine Kinase (RTK) family of receptors. These figures give a representation of the number of different members within the RTK family, the relationship between the representative different members, and the breakdown of the different classes within the RTK family.

### 5. DETAILED DESCRIPTION OF THE INVENTION

The methods of the invention comprise the administration of an effective amount of one or more Eph/Ephrin Modulators that preferentially target multiple Eph receptors and/or Ephrins either alone or in combination with other prophylactic or therapeutic agents. The present invention also provides methods for the prevention, management, treatment and/or amelioration of a disorder associated with aberrant (*i.e*., increased, decreased or inappropriate) Eph receptor and/or Ephrin expression or a symptom thereof, the methods comprising administering to a subject in need thereof an effective amount of an Eph/Ephrin Modulator and/or an effective amount of a therapy other than an Eph/Ephrin Modulator (*e.g*., an immunomodulatory therapy, an anti-angiogenic therapy, an anti-cancer therapy, an anti-inflammatory therapy, etc.).

Non-limiting examples of Eph/Ephrin Modulators of the invention are agents that confer a biological effect by modulating (directly or indirectly): (i) the expression of an Eph receptor (*e.g.*, EphA1 EphA2, EphA3, EphA4, EphA5, EphA6, EphA7, EphA8, EphA10 EphB1 EphB2, EphB3, EphB4, EphB5 or EphB6) and/or an endogenous ligand(s) of an Eph receptor (*e.g*., EphrinA1 EphrinA2, EphrinA3, EphrinA4, EphrinA5, EphrinB1 EphrinB2 or EphrinB3), at, *e.g*., the transcriptional, post-transcriptional, translational or post-translation level; and/or (ii) an activity(ies) of an Eph receptor and/or an Ephrin.

In specific embodiments of the invention, the Eph/Ephrin Modulators of the invention may be designed to bind and/or interact with a unique region of a particular Eph receptor(s) and/or Ephrin(s) to modulate the activity and/or expression of such Eph receptor and/or Ephrin. In another specific embodiment, the Eph/Ephrin Modulators of the invention may be designed to bind and/or interact with regions that are common or homologous between particular Eph receptors and/or Ephrins to simultaneously modulate the activity and/or expression multiple Eph receptors and/or Ephrins in accordance with the methods of the present invention.

Examples of Eph/Ephrin Modulators include, but are not limited to, agents that inhibit or reduce the interaction between an Eph receptor and an endogenous ligand(s) of the Eph receptor, for example, an Ephrin (hereinafter "Eph/Ephrin Interaction Inhibitors"). Non-limiting examples of Eph/Ephrin Interaction Inhibitors include: (i) agents that bind to an Eph receptor, prevent or reduce the interaction between the Eph receptor and an Ephrin, and induce Eph receptor signal transduction (*e.g.*, soluble forms of an Ephrin (*e.g.*, in monomeric or multimeric form), and antibodies that bind to an Eph receptor, induce signaling and phosphorylation of the Eph receptor (*i.e*., an Eph receptor agonistic antibody)); (ii) agents that bind to an Eph receptor, prevent or reduce the interaction between the Eph receptor and an Ephrin, and prevent or induce very low to negligible levels of Eph receptor signal transduction (*e.g*., Eph receptor antagonistic antibodies and dominant negative forms of an Ephrin); (iii) agents that bind to an Ephrin, prevent or reduce the interaction between an Eph receptor and the Ephrin, and induce Ephrin signal transduction (*e.g*., soluble forms of an Eph receptor and antibodies that bind to an Ephrin and induce Ephrin signal transduction); and (iv) agents that bind to an Ephrin, prevent or reduce the interaction between an Eph receptor and the Ephrin, and prevent or induce very low to negligible levels of Ephrin signal transduction (*e.g*., dominant negative forms of an Eph receptor and anti-Ephrin antibodies).

In further embodiments, Eph/Ephrin Modulators include, but are not limited to, agents that modulate the expression of an Eph receptor. Such agents can decrease/downregulate Eph receptor expression (*e.g*., Eph receptor antisense molecules, RNAi and ribozymes) or increase/upregulate Eph receptor expression such that the amount of an Eph receptor on the cell surface exceeds the amount of endogenous ligand (*e.g*., an Ephrin) available for binding, and thus, increases the amount of unbound Eph receptor (*e.g*., nucleic acids encoding an Eph receptor)).

In other embodiments, Eph/Ephrin Modulators are agents that modulate the expression of an Ephrin. Such agents can decrease/downregulate Ephrin expression (*e.g*., Ephrin antisense molecules, RNAi and ribozymes) or increase/upregulate Ephrin expression (*e.g*., nucleic acids encoding an Ephrin)).

In yet other embodiments, Eph/Ephrin Modulators of the invention include, but are not limited to, agents that modulate the protein stability or protein accumulation of an Eph receptor or an Ephrin.

In further embodiments, Eph/Ephrin Modulators of the invention are agents that promote kinase activity (*e.g*., of an Eph receptor, an Ephrin or of a heterologous protein known to associate with the Eph receptor or Ephrin at the cell membrane).

In yet further embodiments, Eph/Ephrin Modulators include, but are not limited to, agents that bind to an Eph receptor and prevent or reduce Eph receptor signal transduction but do not inhibit or reduce the interaction between the Eph receptor and an Ephrin (*e.g*., an Eph receptor intrabody); and agents that bind to an Ephrin and prevent or reduce Ephrin signal transduction but do not inhibit or reduce the interaction between an Ephrin and an Eph receptor (*e.g*., a B-type Ephrin intrabody).

In a specific embodiment, an Eph/Ephrin Modulator is not an agent that inhibits or reduces the interaction between an Eph receptor and an endogenous ligand, *e.g*., an Ephrin. In another specific embodiment, an Eph/Ephrin Modulator is not an agent that modulates (increases or decreases) the expression of an Eph receptor and/or of an Ephrin. In a further embodiment, an Eph/Ephrin Modulator is not an agent that modulates the protein stability or protein accumulation of an Eph receptor or an Ephrin. In yet a further embodiment of the invention, an Eph/Ephrin Modulator is not an agent that promotes kinase activity (*e.g*., of an Eph receptor, an Ephrin or of a heterologous protein known to associate with an Eph receptor or Ephrin at the cell membrane). In another specific embodiment, an Eph/Ephrin Modulator is not an agent that binds to an Eph receptor and prevents or reduces Eph receptor signal transduction but does not prevent the interaction between the Eph receptor and an Ephrin; or an agent that binds to an Ephrin and prevents or reduces Ephrin signal transduction but does not prevent the interaction between the Ephrin and an Eph receptor. In a specific embodiment, an Eph/Ephrin Modulator is not an EphA2 agonistic antibody or an EphA2 antagonistic antibody. In a further embodiment, an Eph/Ephrin Modulator is not an Eph receptor antisense molecule or an Ephrin antisense molecule. In yet a further embodiment, an Eph/Ephrin Modulator is not a soluble form of an Eph receptor (*e.g.*, Eph-Fc) or is not a soluble form of an Ephrin (*e.g*., Ephrin-Fc).

The present invention provides methods for the screening and identification of Eph/Ephrin Modulators that modulate the expression and/or activity of an Eph receptor (*e.g*., EphA1, EphA2, EphA3, EphA4, EphA5, EphA6, EphA7, EphA8, EphA10, EphB1, EphB2, EphB3, EphB4, EphB5 or EphB6) or an Ephrin ligand (e.g., EphrinA1, EphrinA2, EphrinA3, EphrinA4, EphrinA5, EphrinB1, EphrinB2 or EphrinB3).

The present invention further provides pharmaceutical compositions and prophylactic and therapeutic regimens designed to treat, manage, prevent and/or ameliorate a disorder associated with aberrant (*i.e*., increased, decreased or inappropriate) Eph receptor and/or Ephrin expression or a symptom thereof).

The invention yet further provides diagnostic methods using the Eph/Ephrin Modulators of the invention to evaluate the efficacy of a therapy for a disorder associated with aberrant (*i.e*., increased, decreased or inappropriate) Eph receptor and/or Ephrin expression or a symptom thereof).

The invention also provides kits comprising the pharmaceutical compositions or diagnostic reagents of the invention

Table 4, *infra,* summarizes the various Eph receptors and Ephrins, and non-limiting examples of tissues where they are expressed, disorders that are encompassed by the methods of the present invention, as well as relevant references, which are all incorporated by reference herein in their entireties.

**Table 4**

| **TARGET** | **TISSUES WHERE EXPRESSED** | **INDICATIONS** | **REFERENCES** |
|---|---|---|---|
| **EphA1** (Eph) | Liver, kidney, lung, skin | Liver cancer and metastases; lung cancer; kidney cancer; ovarian cancer; colon cancer and metastases | Zhou et al.,1998, Pharmacol. Ther. 77(3):151-181; U.S. Pat. Appn. Pub. No. US2003/0157082 (Aug. 21, 2003); WO 0194629 |
| **EphA2** (Eck) | Brain, kidney, lung, skin, ovary, Langerhans cells of the dendritic cell lineage, endothelial cells | Cancers of epithelial cell origin; ovarian cancer; breast cancer; Pulmonary Langerhans Cell Histiocytosis (PLCH); Graft Versus Host Disease (GVHD) | Zhou et al., 1998, Pharmacol. Ther. 77(3):151-181; U.S. Pat. Appn. Pub. Nos. US2004/0106132 (Jun. 3, 2004) and US2003/0224374 (Jun. 13, 2002); Cheng et al., 2002, Mol. Cancer Res. 1:2-11; Vassallo et al., 2000, New Engl. J. Med. 342:1969-1978; Sundar et al., 2003, Chest 123:1673-1683; Tazi et al.,1999; J. Immunol. 163:3511-3515; Zeid et al., 1995, Pathology 27:247-254; de Saint-Vis et al., 2003, Blood 102:4431-4440; Munthe et al., 2004, BMC Immunol. 5:9; Merad et al., 2004, Nat. Med. 10:510-517; Emerson SG, 2004, Nat. Med. 10:451-452 |
| **EphA3** (Hek/Mek4) | Brain, spleen, kidney, lung, skin, ovary | Primary congestive dilated cardiomyopathy; granulomatous myocarditis; prostate cancer, chronic pancreatitis; benign nodular hyperplasia; pancreatic cancer, liver cirrhosis (all causes); testicular cancer; kidney cancer | Zhou et al., 1998, Pharmacol. Ther. 77(3):151-181; WO 03009814; U.S. Pat. Appn. Pub. No. US2003/0108963 (Jun. 12, 2003) |
| **EphA4** (Sek/Hek8) | Brain, kidney, lung, muscle, heart, testes, ovary | Breast cancer; lung cancer (downregulated in lung carcinoma); prostate cancer | Zhou et al., 1998, Pharmacol. Ther. 77(3):151-181; U.S. Pat. Appn. Pub. No. US2003/0224374 (Jun. 13, 2002); WO 0194629; Ashida et al., 2004, Cancer Res. 64:5963-5972 |
| **EphA5** (Bsk/Hek7) | Brain, testes; nervous system | Brain tumors; neural cancer | Zhou et al., 1998, Pharmacol. Ther. 77(3):151-181; U.S. Pat. Nos. 5,798,448; 5,843,749; 6,280,732 |
| **EphA6** (Ehk2) | Brain, neuronal tissue, trachea, kidney, liver, testes, prostate, thyroid, stomach, intestine, colon, uterus, bladder, cervix, pericardium | Cancers of the brain, neuronal tissue, trachea, kidney, liver, testes, prostate, thyroid, stomach, intestine, colon, uterus, bladder, cervix, pericardium | Zhou et al., 1998, Pharmacol. Ther. 77(3):151-181; Park and Sanchez, 1997, Oncogene 14:533-542; U.S. Pat. No. 6,586,230 |
| **EphA7** (Hek11/MCK1) | Brain, kidney, lung, testes, neuronal tissue | Colorectal cancer; stomach adenocarcinoma; endometrial cancer; testicular cancer; prostate benign nodular hyperplasia; prostate cancer; ovarian cancer; kidney cancer (renal cell carcinoma) | Zhou et al., 1998, Pharmacol. Ther. 77(3):151-181; Park and Sanchez, 1997, Oncogene 14:533-542 |
| **EphA8** (Eek) | Brain, testes, breast, central nervous system, dermal fibroblast cells | Chronic pancreatitis; pancreatic cancer; breast cancer | Zhou et al., 1998, Pharmacol. Ther. 77(3):151-181; WO 02/30979 |
| **EphA10** (EphX) | Testis | Testicular cancer | Aasheim et al., 2005, Biochim Biophys Acta 1723(1-3):1-7 |
| **EphB1** (Elk/NET) | Brain, testes, neuronal tissue, breast | Breast cancer | Zhou et al., 1998, Pharmacol. Ther. 77(3):151-181; WO 03/016475; Chen et al., 2004, J. Cell Biochem. Electronic publication ahead of print (Sep. 24, 2004) |
| **EphB2** (Erk/Nuk) | Brain, neural tissue, thymus, kidney, lung, intestine, testes, ovary, breast | Breast cancer; gastric cancer; lung cancer (small cell and large cell carcinoma; primary); colon cancer (adenocarcinoma, excluding mucinous type; primary); soft tissue cancer (any body site, Schwannoma, malignant fibrous histiocytoma; primary); bone cancer (osteosarcoma; primary); testicular cancer (mixed germ cell tumor; primary); stomach cancer (adenocarcinoma, including and excluding Signet Ring cell type; primary); endometrial cancer (Mullerian mixed tumor; primary), kidney cancer (Wilm's tumor; primary);uterine/cervical cancer (squamous cell carcinoma; primary); esophageal cancer (adenocarcimona; primary) | Zhou et al., 1998, Pharmacol. Ther. 77(3):151-181; U.S. Pat. Nos. 6,218,356 and 6,440,663 |
| **EphB3** (Hek2/MDK5) | Brain, spleen, liver, kidney, lung, muscle, heart, intestine, testes, placenta, pancreas | Ovarian cancer; colon cancer (adenoma) and metastases; endometrial cancer; rectal cancer; breast cancer; synovial sarcoma; cancer of the parotid gland (pleomorphic adenoma, mixed tumor); soft tissue cancer (any body site, synovial sarcoma; primary); kidney cancer (Wilm's tumor; primary); liver metastases; lung cancer; liver cancer and metastases; prostate cancer; diseases associated with defective glucose transport | Zhou et al., 1998, Pharmacol. Ther. 77(3):151-181; U.S. Pat. Appn. Pub. Nos. US2003/0157082 (Aug. 21, 2003) and US2003/0224374 (Jun. 13, 2002); U.S. Pat. Nos. 5,506,205 and 6,506,607 |
| **EphB4** (Htk/MDK2) | Brain, liver, kidney, lung, muscle, heart, intestine, testes | Breast cancer (carcinoma); liposarcoma; rectal cancer; bone cancer (osteosarcoma); esophageal cancer; colon cancer (adenoma); testicular cancer; cancer of the parotid gland; colon cancer; lung cancer (squamous cell carcinoma); renal cancer; liver cancer | Zhou et al., 1998, Pharmacol. Ther. 77(3):151-181; U.S. Pat. Appn. Pub. No. US2004/0115625 (Jun. 17, 2004); U.S. Pat. No. 6,673,343; WO 0194629 |
| **EphB5** (Cek9) | Brain, thymus, kidney, neuronal tissue | Cancers of the brain, thymus, kidney, neural tissues | Zhou et al., 1998, Pharmacol. Ther. 77(3):151-181; Brambilla et. 1995, EMBO J. 14:3116-3126 |
| **EphB6** (Hep/Mep) | Brain, thymus, spleen, liver, kidney, lung, muscle, heart, endothelial cells, cardiovascular system | Neuroblastoma | Zhou et al., 1998, Pharmacol. Ther. 77(3):151-181; U.S. Pat. Appn. Pub. No. US2003/10100497 (May 29, 2003) |
| **EphrinA1** (B61/LERK-1) | Epithelial cells, liver, kidney, lung, muscle, heart, skin, ovary, intestine | Cancers of epithelial cell origin; lung cancer(small cell carcinoma; primary); bone disorders (degenerative joint disease; osteoarthritis); prostate cancer (adenocarcinoma); colon cancer; thyroid disorders (Hashimoto's thyroiditis); thyroid cancer; kidney cancer (renal cell carcinoma); lung cancer; myometrium cancer (leiomyoma); ovarian cancer, | Zhou et al., 1998. Pharmacal. Ther. 77(3):151-181; U.S. Pat. Nos. 6,087,167 and 5,716,934 |
| **EphrinA2** (ELF-1/LERK-6) | Brain, neuronal tissue, digestive tract, liver, lung | Leukemias, breast cancer | Zhou et al., 1998, Pharmacol. Ther. 77(3):151.181; Galang et al., 2004, J. Biol. Chem. 279:11281-11292; U.S. Pat. Nos. 5,795,734 and 6,472,174 |
| **EphrinA3** (LERK-3) | Brain, skin, ovary, breast | Breast cancer, leukemias | Zhou et al.,1998, Pharmacol. Ther. 77(3):151-181; Galang et al, 2004, J. Biol. Chem. 279:11281-11292; U.S. Pat. No. 6,274,117 |
| **EphrinA4** (LERK-4) | Thymus, spleen, liver, kidney, lung, muscle, ovary | Skin cancer (squamous cell and basal cell carcinoma); pancreatic disorders (acute pancreatitis); pancreatic cancer (adenocarcinoma; islet cell tumor); testicular cancer (mixed germ cell tumor); colon cancer, rectal cancer (adenocarcinoma); breast cancer; ovarian cancer; Alzheimer's Disease; leukemias (pre-B or T-cell) | Zhou et al.,1998, Pharmacol. Ther. 77(3):151-181; U.S. Pat. Nos. 6,274,117; 5,516,658; 5,738,844 |
| **EphrinA5** (AL-1/LERK-7) | Brain, kidney, heart, Nervous system | Neuroendocrine carcinoma of the lung (downregulated) | Zhou et al., 1998, Pharmacol. Ther. 77(3):151-181; U.S. Pat. Nos. 5,798,448; 6,610,296; WO 0194629 |
| **EphrinB1** (Elk-L/**LERK**-**2**) | Brain, thymus, spleen, liver, kidney, lung, muscle, heart, skin, ovary, neuronal tissue | Gastric cancer, ovarian cancer (theocoma-fibroma); soft tissue cancer (any body site; malignant fibrous histiocytoma; primary); myocardial infarction, gallblader disorders (acute cholecystitis); neurodegencrative diseases; colon carcinoma (downreguated) | Zhou et al., 1998, Pharmacol. Ther. 77(3):151-181; U.S. Pat. Nos. 5,512,457; 6,540,992; WO 0194629 |
| **EphrinB2** (Htk-L/LERK-5/NLERK-1) | Brain, thymus, spleen, liver, kidney, lung, muscle, heart, ovary, prostate | Neuroblastoma; gastric cancer; kidney cancer (renal cell carcinoma); Crohn's disease, lung cancer; rectal cancer; breast cancer; colon cancer; skin cancer (malignant melanoma; basal cell carcinoma); kidney cancer; diverticulitis; ovarian cancer (thecoma-fibroma); Non-Hodgkin's lymphoma (all types); Hodgkin's disease (all subtypes); ulcerative colitis (active, chronic inflammation); psoriasis; Alzheimer's disease; endometrial cancer (adenocarcinoma); thyroid cancer (follicular carcinoma); leukemias, lymphoma | Zhou et al., 1998, Pharmacol. Ther. 77(3):151-181; Cho et al., 2004, J.Biol. Chem. 279:19512-19522; Steube et al., 1999, Leuk. Lymphoma 33:371-376; WO 0160860 |
| **EphrinB3** (Elk-L3/NLERK-2) | Brain, heart, neuronal tissue | Neuroblastoma | Zhou et al., 1998, Pharmaco/. Ther. 77(3):151-181; U.S. Pat. Nos. 6,413,730 and 6.602,683 |

### 5.1 EPH/EPHRIN MODULATORS

The invention encompasses compositions and methods comprising Eph/Ephrin Modulators. An Eph/Ephrin Modulator includes, but is not limited to, an agent(s) that confers a biological effect by modulating (directly or indirectly): (i) the expression of an Eph receptor (*e.g*., EphA1, EphA2, EphA3, EphA4, EphA2, EphA6, EphA7, EphA8, EphA10, EphB1, EphB2, EphB3, EphB4, EphB5 or EphB6) and/or an endogenous ligand(s) of an Eph receptor (e.g., EphrinA1, EphrinA2, EphrinA3, EphrinA4, EphrinA5, EphrinB1, EphrinB2 or EphrinB3), at, e.g., the transcriptional, post-transcriptional, translational or post-translation level; and/or (ii) an activity(ies) of an Eph receptor and/or an Ephrin.

In specific embodiments of the invention, the Eph/Ephrin Modulators of the invention may be designed to bind and/or interact with a unique region of a particular Eph receptor(s) and/or Ephrin(s) to modulate the activity and/or expression of such Eph receptor and/or Ephrin. In another specific embodiment, the Eph/Ephrin Modulators of the invention may be designed to bind and/or interact with regions that are common or homologous between particular Eph receptors and/or Ephrins to simultaneously modulate the activity and/or expression multiple Eph receptors and/or Ephrins in accordance with the methods of the present invention.

Examples of Eph/Ephrin Modulators include, but are not limited to, agents that inhibit or reduce the interaction between an Eph receptor and an endogenous ligand(s) of the Eph receptor, for example, an Ephrin (hereinafter "Eph/Ephrin Interaction Inhibitors"). Non-limiting examples of Eph/Ephrin Interaction Inhibitors include: (i) agents that bind to an Eph receptor, prevent or reduce the interaction between the Eph receptor and an Ephrin, and induce Eph receptor signal transduction (*e.g*., soluble forms of an Ephrin (*e.g.*, in monomeric or multimeric form), and antibodies that bind to an Eph receptor, induce signaling and phosphorylation of the Eph receptor (*i.e*., an Eph receptor agonistic antibody)); (ii) agents that bind to an Eph receptor, prevent or reduce the interaction between the Eph receptor and an Ephrin, and prevent or induce very low to negligible levels of Eph receptor signal transduction (*e.g*., Eph receptor antagonistic antibodies and dominant negative forms of an Ephrin); (iii) agents that bind to an Ephrin, prevent or reduce the interaction between an Eph receptor and the Ephrin, and induce Ephrin signal transduction (*e.g*., soluble forms of an Eph receptor and antibodies that bind to an Ephrin and induce Ephrin signal transduction); and (iv) agents that bind to an Ephrin, prevent or reduce the interaction between an Eph receptor and the Ephrin, and prevent or induce very low to negligible levels of Ephrin signal transduction (*e.g*., dominant negative forms of an Eph receptor and anti-Ephrin antibodies).

In further embodiments, Eph/Ephrin Modulators include, but are not limited to, agents that modulate the expression of an Eph receptor. Such agents can decrease/downregulate Eph receptor expression (*e.g*., Eph receptor antisense molecules, RNAi and ribozymes) or increase/upregulate Eph receptor expression such that the amount of an Eph receptor on the cell surface exceeds the amount of endogenous ligand (*e.g*., an Ephrin) available for binding, and thus, increases the amount of unbound Eph receptor (*e.g*., nucleic acids encoding an Eph receptor)).

In other embodiments, Eph/Ephrin Modulators are agents that modulate the expression of an Ephrin. Such agents can decrease/downregulate Ephrin expression (*e.g*., Ephrin antisense molecules, RNAi and ribozymes) or increase/upregulate Ephrin expression (*e.g*., nucleic acids encoding an Ephrin)).

In yet other embodiments, Eph/Ephrin Modulators of the invention include, but are not limited to, agents that modulate the protein stability or protein accumulation of an Eph receptor or an Ephrin.

In further embodiments, Eph/Ephrin Modulators of the invention are agents that promote kinase activity (*e.g*., of an Eph receptor, an Ephrin or of a heterologous protein known to associate with the Eph receptor or Ephrin at the cell membrane).

In yet further embodiments, Eph/Ephrin Modulators include, but are not limited to, agents that bind to an Eph receptor and prevent or reduce Eph receptor signal transduction but do not inhibit or reduce the interaction between the Eph receptor and an Ephrin (*e.g*., an Eph receptor intrabody); and agents that bind to an Ephrin and prevent or reduce Ephrin signal transduction but do not inhibit or reduce the interaction between an Ephrin and an Eph receptor (*e.g*., a B-type Ephrin intrabody).

In a specific embodiment, an Eph/Ephrin Modulator is not an agent that inhibits or reduces the interaction between an Eph receptor and an endogenous ligand, *e.g*., an Ephrin. In another specific embodiment, an Eph/Ephrin Modulator is not an agent that modulates (increases or decreases) the expression of an Eph receptor and/or of an Ephrin. In a further embodiment, an Eph/Ephrin Modulator is not an agent that modulates the protein stability or protein accumulation of an Eph receptor or an Ephrin. In yet a further embodiment of the invention, an Eph/Ephrin Modulator is not an agent that promotes kinase activity (*e.g*., of an Eph receptor, an Ephrin or of a heterologous protein known to associate with an Eph receptor or Ephrin at the cell membrane). In another specific embodiment, an Eph/Ephrin Modulator is not an agent that binds to an Eph receptor and prevents or reduces Eph receptor signal transduction but does not prevent the interaction between the Eph receptor and an Ephrin; or an agent that binds to an Ephrin and prevents or reduces Ephrin signal transduction but does not prevent the interaction between the Ephrin and an Eph receptor. In a specific embodiment, an Eph/Ephrin Modulator is not an EphA2 agonistic antibody or an EphA2 antagonistic antibody. In a further embodiment, an Eph/Ephrin Modulator is not an Eph receptor antisense molecule or an Ephrin antisense molecule. In yet a further embodiment, an Eph/Ephrin Modulator is not a soluble form of an Eph receptor (*e.g*., Eph-Fc) or is not a soluble form of an Ephrin (*e.g*., Ephrin-Fc).

In a specific embodiment, an EphA/Ephrin Modulator is an agent that decreases or downregulates expression of an Eph receptor, for example, EphA1, EphA2, EphA3, EphA4, EphA2, EphA6, EphA7, EphA8, EphA10, EphB1, EphB2, EphB3, EphB4, EphB5 or EphB6, (*e.g*., using Eph receptor antisense molecules, RNAi and ribozymes). In a particular embodiment, the Eph/Ephrin Modulator of the invention decreases or downregulates expression of an Eph receptor by at least 25%, at least 30%, at least 35%, at least 40%, at least 45%, at least 50%, at least 55%, at least 60%, at least 65%, at least 70%, at least 75%, at least 80%, at least 85%, at least 90% or at least 95%, or at least 1.5 fold, at least 2 fold, at least 2.5 fold, at least 3 fold, at least 3.5 fold, at least 4 fold, at least 4.5, at least 5 fold, at least 7 fold or at least 10 fold relative to a control (*e.g*., phosphate buffered saline) in an assay described herein or known in the art (*e.g*., RT-PCR, a Northern blot or an immunoassay such as an ELISA). In alternative embodiment, an Eph/Ephrin Modulator of the invention is an agent that increases or upregulates the expression of an Eph receptor such that the amount of the Eph receptor on the cell surface exceeds the amount of endogenous ligand (*e.g*., EphrinA1, EphrinA2, EphrinA3, EphrinA4, EphrinA5, EphrinB1 EphrinB2 or EphrinB3) available for binding, and thus, increases the amount of unbound Eph receptor (*e.g*., nucleic acids encoding an Eph receptor)).

In a specific embodiment, an Eph/Ephrin Modulator is an agent that reduces the protein stability and/or protein accumulation of an Eph receptor (*e.g*., EphA1, EphA2, EphA3, EphA4, EphA5, EphA6, EphA7, EphA8, EphA10, EphB1, EphB2, EphB3, EphB4, EphB5 or EphB6). In a particular embodiment, the Eph/Ephrin Modulator reduces the protein stability and/or protein accumulation of an Eph receptor by at least 25%, at least 30%, at least 35%, at least 40%, at least 45%, at least 50%, at least 55%, at least 60%, at least 65%, at least 70%, at least 75%, at least 80%, at least 85%, at least 90% or at least 95%, or at least 1.5 fold, at least 2 fold, at least 2.5 fold, at least 3 fold, at least 3.5 fold, at least 4 fold, at least 4.5, at least 5 fold, at least 7 fold or at least 10 fold relative to a control (*e.g*., phosphate buffered saline) in an assay described herein or known in the art (*e.g*., an immunoassay). In an alternative embodiment, an Eph/Ephrin Modulator is an agent that increases the protein stability and/or protein accumulation of an Eph receptor.

In a specific embodiment, an Eph/Ephrin Modulator is an agent that promotes Eph receptor autophosphorylation and/or ligand-mediated Eph receptor signaling. In another embodiment, an Eph/Ephrin Modulator is an agent that inhibits or decreases the expression of an Ephrin (at the transcriptional, post-transcriptional or translation level), for example, EphrinA1, EphrinA2, EphrinA3, EphrinA4, EphrinA5, EphrinB1, EphrinB2 or EphrinB3 (*e.g.*, using Ephrin antisense molecules, RNAi and ribozymes). In a particular embodiment, the Eph/Ephrin Modulator of the invention decreases the expression of an Ephrin by at least 25%, at least 30%, at least 35%, at least 40%, at least 45%, at least 50%, at least 55%, at least 60%, at least 65%, at least 70%, at least 75%, at least 80%, at least 85%, at least 90% or at least 95%, or at least 1.5 fold, at least 2 fold, at least 2.5 fold, at least 3 fold, at least 3.5 fold, at least 4 fold, at least 4.5, at least 5 fold, at least 7 fold or at least 10 fold relative to a control (*e.g*., phosphate buffered saline) in an assay described herein or known in the art (*e.g*., RT-PCR, a Northern blot or an immunoassay such as an ELISA).

In another embodiment, an Eph/Ephrin Modulator is an agent that binds to an Eph receptor and prevents or reduces Eph receptor signal transduction but does not inhibit or reduce the interaction between the Eph receptor and an endogenous ligand(s) of the Eph receptor, (*e.g*., an Eph receptor intrabody or an Eph receptor antibody that does not compete with ligand binding). In a particular embodiment, the Eph/Ephrin Modulator reduces Eph receptor signal transduction by at least 25%, at least 30%, at least 35%, at least 40%, at least 45%, at least 50%, at least 55%, at least 60%, at least 65%, at least 70%, at least 75%, at least 80%, at least 85%, at least 90% or at least 95%, or at least 1.5 fold, at least 2 fold, at least 2.5 fold, at least 3 fold, at least 3.5 fold, at least 4 fold, at least 4.5, at least 5 fold, at least 7 fold or at least 10 fold relative to a control (*e.g*., phosphate buffered saline) in an assay described herein or known in the art (*e.g*., an immunoassay). In accordance with this embodiment, the Eph/Ephrin Modulator does not reduce or only reduces the interaction between the Eph receptor and an endogenous ligand(s) of the Eph receptor by 5% or less, 10% or less, 15% or less, 20% or less, 25% or less, 30% or less, 35% or less, 40% or less relative to a control (*e.g*., phosphate buffered saline) in an assay described herein or known in the art.

In another embodiment, an Eph/Ephrin Modulator is an agent that binds to an Ephrin and prevents or reduces Ephrin signal transduction but does not inhibit or reduce the interaction between the Ephrin and an Eph receptor. In a particular embodiment, the Eph/Ephrin Modulator reduces Ephrin signal transduction by at least 25%, at least 30%, at least 35%, at least 40%, at least 45%, at least 50%, at least 55%, at least 60%, at least 65%, at least 70%, at least 75%, at least 80%, at least 85%, at least 90% or at least 95%, or at least 1.5 fold, at least 2 fold, at least 2.5 fold, at least 3 fold, at least 3.5 fold, at least 4 fold, at least 4.5, at least 5 fold, at least 7 fold or at least 10 fold relative to a control (*e.g*., phosphate buffered saline) in an assay described herein or known in the art (*e.g*., an assay that detects Ephrin phosphorylation). In accordance with this embodiment, the Eph/Ephrin Modulator does not reduce or only reduces the interaction between an Eph receptor (*e.g*., EphA1, EphA2, EphA3, EphA4, EphA5, EphA6, EphA7, EphA8, EphA10, EphB1, EphB2, EphB3, EphB4, EphB5 or EphB6) and an endogenous ligand (*e.g.*, EphrinA1, EphrinA2, EphrinA3, EphrinA4, EphrinA5, EphrinB1, EphrinB2 or EphrinB3) by 5% or less, 10% or less, 15% or less, 20% or less, 25% or less, 30% or less, 35% or less, 40% or less, or 2 fold or less, 1.5 fold or less or 1 fold or less relative to a control (*e.g*., phosphate buffered saline) in an assay described herein or known in the art.

In a specific embodiment, an Eph/Ephrin Modulator is an Eph/Ephrin Interaction Inhibitor. In one embodiment, an Eph/Ephrin Interaction Inhibitor is an agent that binds to an Eph receptor (*e.g*., EphA1, EphA2, EphA3, EphA4, EphA5, EphA6, EphA7, EphA8, EphA10, EphB1, EphB2, EphB3, EphB4, EphB5 or EphB6), prevents or reduces the interaction between the Eph receptor and an endogenous ligand (*e.g*., EphrinA1, EphrinA2, EphrinA3, EphrinA4, EphrinA5, EphrinB1, EphrinB2 or EphrinB3) and induces Eph receptor signal transduction (*e.g*., soluble forms of an Ephrin and antibodies that bind to an Eph receptor), induce signaling and phosphorylation of an Eph receptor (*i.e.*, an agonistic antibody)). In a particular embodiment, such an Eph/Ephrin Interaction Inhibitor reduces the interaction between an Eph receptor and an endogenous ligand (*e.g*., an Ephrin) by at least 25%, at least 30%, at least 35%, at least 40%, at least 45%, at least 50%, at least 55%, at least 60%, at least 65%, at least 70%, at least 75%, at least 80%, at least 85%, at least 90% or at least 95%, or at least 1.5 fold, at least 2 fold, at least 2.5 fold, at least 3 fold, at least 3.5 fold, at least 4 fold, at least 4.5, at least 5 fold, at least 7 fold or at least 10 fold relative to a control (*e.g*., phosphate buffered saline) in an assay described herein or known in the art. In accordance with this embodiment, the Eph/Ephrin Interaction Inhibitor induces Eph receptor signal transduction by at least 25%, at least 30%, at least 35%, at least 40%, at least 45%, at least 50%, at least 55%, at least 60%, at least 65%, at least 70%, at least 75%, at least 80%, at least 85%, at least 90% or at least 95%, or at least 1.5 fold, at least 2 fold, at least 2.5 fold, at least 3 fold, at least 3.5 fold, at least 4 fold, at least 4.5, at least 5 fold, at least 7 fold or at least 10 fold relative to a control (*e.g*., phosphate buffered saline) in an assay described herein or known in the art (*e.g*., an immunoassay).

In another embodiment, an Eph/Ephrin Interaction Inhibitor is an agent that binds to an Eph receptor (*e.g.*, EphA1, EphA2, EphA3, EphA4, EphA5, EphA6, EphA7, EphA8, EphA10, EphB1, EphB2, EphB3, EphB4, EphB5 or EphB6), prevents or reduces the interaction between the Eph receptor and an endogenous ligand (*e.g*., EphrinA1, EphrinA2, EphrinA3, EphrinA4, EphrinA5, EphrinB1, EphrinB2 or EphrinB3), and prevents or induces very low to negligible levels of Eph receptor signal transduction (*e.g*., antibodies). In a particular embodiment, such an Eph/Ephrin Interaction Inhibitor reduces the interaction between an Eph receptor and an endogenous ligand of the Eph receptor (*e.g*., an Ephrin) by at least 25%, at least 30%, at least 35%, at least 40%, at least 45%, at least 50%, at least 55%, at least 60%, at least 65%, at least 70%, at least 75%, at least 80%, at least 85%, at least 90% or at least 95%, or at least 1.5 fold, at least 2 fold, at least 2.5 fold, at least 3 fold, at least 3.5 fold, at least 4 fold, at least 4.5, at least 5 fold, at least 7 fold or at least 10 fold relative to a control (*e.g*., phosphate buffered saline) in an assay described herein or known in the art. In accordance with this embodiment, the Eph/Ephrin Interaction Inhibitor induces Eph receptor signal transduction by 5% or less, 10% or less, 15% or less, 20% or less, 25% or less, 30% or less, 35% or less, 40% or less, or 2 fold or less, 1.5 fold or less or 1 fold or less relative to a control (*e.g*., phosphate buffered saline) in an assay described herein or known in the art (*e.g.,* an immunoassay).

In another embodiment, an Eph/Ephrin Interaction Inhibitor is an agent that binds to an Ephrin, prevents or reduces the interaction between an Eph receptor and an Ephrin and induces Ephrin signal transduction (*e.g*., soluble forms of an Eph receptor, dominant negative forms of the Eph receptor, and antibodies that bind to an Ephrin and induce Ephrin signal transduction). In a particular embodiment, such an Eph/Ephrin Interaction Inhibitor reduces the interaction between an Eph receptor and an Ephrin by at least 25%, at least 30%, at least 35%, at least 40%, at least 45%, at least 50%, at least 55%, at least 60%, at least 65%, at least 70%, at least 75%, at least 80%, at least 85%, at least 90% or at least 95%, or at least 1.5 fold, at least 2 fold, at least 2.5 fold, at least 3 fold, at least 3.5 fold, at least 4 fold, at least 4.5, at least 5 fold, at least 7 fold or at least 10 fold relative to a control (*e.g*., phosphate buffered saline) in an assay described herein or known in the art. In accordance with this embodiment, the Eph/Ephrin Interaction Inhibitor induces Ephrin signal transduction by at least 25%, at least 30%, at least 35%, at least 40%, at least 45%, at least 50%, at least 55%, at least 60%, at least 65%, at least 70%, at least 75%, at least 80%, at least 85%, at least 90% or at least 95%, or at least 1.5 fold, at least 2 fold, at least 2.5 fold, at least 3 fold, at least 3.5 fold, at least 4 fold, at least 4.5, at least 5 fold, at least 7 fold or at least 10 fold relative to a control (*e.g*., phosphate buffered saline) in an assay described herein or known in the art (*e.g*., an immunoassay).

In another embodiment, an Eph/Ephrin Interaction Inhibitor is an agent that binds to an Ephrin, prevents or reduces the interaction between an Eph receptor and an Ephrin, induces Ephrin signal transduction (*e.g*., soluble forms of an Eph receptor, dominant negative forms of the Eph receptor, and antibodies that bind to an Ephrin and induce Ephrin signal transduction). In a particular embodiment, such an Eph/Ephrin Interaction Inhibitor reduces the interaction between an Eph receptor and Ephrin by at least 25%, at least 30%, at least 35%, at least 40%, at least 45%, at least 50%, at least 55%, at least 60%, at least 65%, at least 70%, at least 75%, at least 80%, at least 85%, at least 90% or at least 95%, or at least 1.5 fold, at least 2 fold, at least 2.5 fold, at least 3 fold, at least 3.5 fold, at least 4 fold, at least 4.5, at least 5 fold, at least 7 fold or at least 10 fold relative to a control (*e.g*., phosphate buffered saline) in an assay described herein or known in the art. In accordance with this embodiment, the Eph/Ephrin Interaction Inhibitor induces Ephrin signal transduction by at least 25%, at least 30%, at least 35%, at least 40%, at least 45%, at least 50%, at least 55%, at least 60%, at least 65%, at least 70%, at least 75%, at least 80%, at least 85%, at least 90% or at least 95%, or at least 1.5 fold, at least 2 fold, at least 2.5 fold, at least 3 fold, at least 3.5 fold, at least 4 fold, at least 4.5, at least 5 fold, at least 7 fold or at least 10 fold relative to a control (*e.g*., phosphate buffered saline) in an assay described herein or known in the art (*e.g*., an immunoassay).

In a specific embodiment, an Eph/Ephrin Modulator is not an agent that inhibits or reduces Eph receptor gene expression (*e.g*., Eph receptor antisense, RNAi or ribozyme). In another embodiment, an Eph/Ephrin Modulator is not an Eph/Ephrin Inhibitor that is an agent that binds to an Eph receptor, prevents or reduces the interaction between an Eph receptor and an endogenous ligand of the Eph receptor, *e.g*., an Ephrin, and induces Eph receptor signal transduction. In another embodiment, an Eph/Ephrin Modulator is not an antibody that specifically binds to Eph receptor and induces signaling and phosphorylation of the Eph receptor (*i.e*., an agonistic antibody).

In a specific embodiment, an Eph/Ephrin Modulator has one, two or all of the following cellular effects: (i) increases Eph receptor signal transduction; (ii) increases Eph receptor cytoplasmic tail phosphorylation; (iii) increases Eph receptor autophosphorylation and/or degradation; (iv) decreases Eph receptor gene expression (*e.g*., at the transcriptional, post-transcriptional, translational or post-translation level), protein stability and/or accumulation; and (v) decreases or inhibits cell proliferation, metastasis and/or angiogenesis.

In yet another specific embodiment, an Eph/Ephrin Modulator has one, two or all of the following cellular effects: (i) decreases Eph receptor signal transduction; (ii) decreases Eph receptor cytoplasmic phosphorylation; (iii) decreases Eph receptor autophosphorylation and/or degradation; (iv) increases Eph receptor gene expression (*e.g*., at the transcriptional, post-transcriptional, translational or post-translation level), protein stability and/or accumulation; and (v) increases cell proliferation and/or cell survival.

In a further embodiment, an Eph/Ephrin Modulator of the invention has one, two or all of the following cellular effects: (i) increases Ephrin signal transduction in an Ephrin-expressing cell; and (ii) increases Ephrin gene expression (*e.g*., at the transcriptional, post-transcriptional, translational or post-translation level).

In yet a further embodiment, an Eph/Ephrin Modulator of the invention has one, two or all of the following cellular effects: (i) decreases Ephrin signal transduction in an Ephrin-expressing cell; and (ii) decreases Ephrin gene expression (*e.g*., at the transcriptional, post-transcriptional, translational or post-translation level).

Eph/Ephrin Modulators of the invention include, but are not limited to, proteinaceous molecules (including, but not limited to, peptides, polypeptides, proteins, post-translationally modified proteins, antibodies etc.), small molecules (less than 1000 daltons), inorganic or organic compounds, nucleic acid molecules (including, but not limited to, double-stranded, single-stranded DNA, double-stranded or single-stranded RNA (*e.g*., antisense, mediates RNAi, etc.), and triple helix nucleic acid molecules, aptamers and vaccines (*e.g., Listeria-*based and non-*Listeria*-based vaccines), and derivatives of any of the above.

### 5.1.1 Polypeptides That Are Eph/Ephrin Modulators

Methods of the present invention encompass Eph/Ephrin Modulators that are polypeptides. In one embodiment, a polypeptide Eph/Ephrin Modulator is an antibody (*e.g*., a monoclonal antibody) or a fragment thereof. In another embodiment, a polypeptide Eph/Ephrin Modulator is a soluble form of an Eph receptor (*e.g*., EphA1, EphA2, EphA3, EphA4, EphA5, EphA6, EphA7, EphA8, EphA10, EphB1, EphB2, EphB3, EphB4, EphB5 or EphB6), or of an Ephrin (*e.g*., EphrinA1, EphrinA2, EphrinA3, EphrinA4, Ephrin5, EphrinB1, EphrinB2 or EphrinB3). In one embodiment, a polypeptide Eph/Ephrin Modulator is a soluble form of an Eph receptor that retains its ability to bind an Ephrin and that is fused to the Fc portion of human immunoglobulin IgG1 (*e.g.,* Eph receptor-Fc). See, *e.g*., Cheng et al., 2002, Mol. Cancer Res. 1:2-11, which is incorporated by reference herein in its entirety. In another embodiment, a polypeptide Eph/Ephrin Modulator is a soluble form of an Ephrin that retains its ability to bind an Eph receptor and that is fused to an the Fc domain of human immunoglobulin IgG1 (*e.g*., Ephrin-Fc). See, *e.g*., Duxbury et al., 2004, Biochem. & Biophys. Res. Comm. 320:1096-1102, which is incorporated by reference herein in its entirety.

In another embodiment, a polypeptide Eph/Ephrin Modulator is a dominant negative form of an Eph receptor comprising, for example, the Ephrin ligand binding domain.

In one embodiment, a polypeptide Eph/Ephrin Modulator is an Eph/Ephrin Interaction Inhibitor. In a specific embodiment, an Eph/Ephrin Modulator is an Eph receptor antibody that specifically binds an Eph receptor, prevents or reduces the interaction between the Eph receptor and an endogenous ligand, *e.g*., an Ephrin, and induces Eph receptor signal transduction (including, but not limited to, Eph receptor autophosphorylation). In another embodiment, an Eph/EphrinModulator is an Eph receptor antibody that specifically binds to an Eph receptor, prevents or reduces the interaction between the Eph receptor and an endogenous ligand, *e.g*., an Ephrin, and prevents or induces very low to negligible levels of Eph receptor signal transduction (including, but not limited to, Eph receptor autophosphorylation). In certain embodiments, a polypeptide Eph/Ephrin Modulator is not an EphA2 antibody that specifically binds to EphA2, prevents or reduces the interaction between EphA2 and EphrinA1, and induces EphA2 signal transduction.

In a specific embodiment, a polypeptide Eph/Ephrin Modulator is an Ephrin antibody that specifically binds to an Ephrin, prevents or reduces the interaction between an Eph receptor and an Ephrin, and induces Ephrin signal transduction. In another embodiment, an Eph/Ephrin Modulator is an Ephrin antibody that specifically binds to an Ephrin, prevents or reduces the interaction between an Eph receptor and an Ephrin, and prevents or induces very low to negligible levels of Ephrin signal transduction in an Ephrin-expressing cell.

In a specific embodiment, an Eph/Ephrin Modulator is a soluble form of an Ephrin or a fragment of an Ephrin that binds an Eph receptor, prevents or reduces the interaction between the Eph receptor and an Ephrin, and induces Eph receptor signal transduction (including, but not limited to, autophosphorylation). In another embodiment, an Eph/Ephrin Modulator is a soluble form of an Ephrin or a fragment of an Ephrin that binds to an Eph receptor, prevents or reduces the interaction between the Eph receptor and an Ephrin, and prevents or induces very low to negligible levels of Eph receptor signal transduction (including, but not limited to, Eph receptor autophosphorylation).

In a specific embodiment, an Eph/Ephrin Modulator is a soluble form of an Eph receptor or a fragment of an Eph receptor that binds to an endogenous ligand (*e.g*., an Ephrin), prevents or reduces the interaction between an Eph receptor and an endogenous ligand (*e.g*., an Ephrin), and induces Ephrin signal transduction. In another embodiment, an Eph/Ephrin Modulator is a soluble form of an Eph receptor or a fragment of an Eph receptor that binds to an endogenous ligand (*e.g*., an Ephrin), prevents or reduces the interaction between the Eph receptor and an endogenous ligand (*e.g*., an Ephrin), and prevents or induces very low to negligible levels of Ephrin signal transduction in an Ephrin-expressing cell.

In a specific embodiment, an Eph/Ephrin Modulator is a dominant negative form of an Eph receptor that binds to an endogenous ligand (*e.g*., an Ephrin), prevents or reduces the interaction between an Eph receptor and an endogenous ligand (*e.g*., an Ephrin), and induces Ephrin signal transduction in the Ephrin-expressing cell. In another embodiment, an Eph/Ephrin Modulator is a dominant negative form of an Eph receptor that binds to an endogenous ligand (*e.g*., an Ephrin), prevents or reduces the interaction between the Eph receptor and an endogenous ligand (*e.g*., an Ephrin), and prevents or induces very low to negligible levels of Ephrin signal transduction in the Ephrin-expressing cell

In yet a further embodiment, an Eph/Ephrin Modulator is an isolated peptide which selectively binds to a member of the Eph receptor family. Nonlimiting examples of these peptides are disclosed in U.S. Patent Application Publication 2004/0180823 A1, which is incorporated by reference herein in its entirety.

### 5.1.1.1 Antibodies That Are Eph/Ephrin Modulators

Antibodies play a vital role in our immune responses. They can inactivate viruses and bacterial toxins, and are essential in recruiting the complement system and various types of white blood cells to kill invading microorganisms and large parasites. Antibodies are synthesized exclusively by B lymphocytes, and are produced in millions of forms, each with a different amino acid sequence and a different binding site for an antigen. Antibodies, collectively called immunoglobulins (Ig), are among the most abundant protein components in the blood. Alberts et al., Molecular Biology of the Cell, 2nd ed., 1989, Garland Publishing, Inc.

A typical antibody is a Y-shaped molecule with two identical heavy (H) chains (each containing about 440 amino acids) and two identical light (L) chains (each containing about 220 amino acids). The four chains are held together by a combination of noncovalent and covalent (disulfide) bonds. The proteolytic enzymes, such as papain and pepsin, can split an antibody molecule into different characteristic fragments. Papain produces two separate and identical Fab fragments, each with one antigen-binding site, and one Fc fragment. Pepsin produces one F(ab')₂ fragment. Alberts et al., Molecular Biology of the Cell, 2nd ed., 1989, Garland Publishing, Inc.

Both L and H chains have a variable sequence at their amino-terminal ends but a constant sequence at their carboxyl-terminal ends. The L chains have a constant region about 110 amino acids long and a variable region of the same size. The H chains also have a variable region about 110 amino acids long, but the constant region of the H chains is about 330 or 440 amino acid long, depending on the class of the H chain. Alberts et al., Molecular Biology of the Cell, 2nd ed., 1989, Garland Publishing, Inc. at pp1019.

Only part of the variable region participates directly in the binding of antigen. Studies have shown that the variability in the variable regions of both L and H chains is for the most part restricted to three small hypervariable regions (also called complementarity-determining regions, or CDRs) in each chain. The remaining parts of the variable region, known as framework regions (FR), are relatively constant. Alberts et al., Molecular Biology of the Cell, 2nd ed., 1989, Garland Publishing, Inc. at pp 1019 - 1020.

It will be understood that the complementarity determining regions (CDRs) residue numbers referred to herein are those of Kabat et al. (1991, NIH Publication 91-3242, National Technical Information Service, Springfield, VA). Specifically, residues 24-34 (CDR1), 50-56 (CDR2) and 89-97 (CDR3) in the light chain variable domain and 31-35 (CDR1), 50-65 (CDR2) and 95-102 (CDR3) in the heavy chain variable domain. Note that CDRs vary considerably from antibody to antibody (and by definition will not exhibit homology with the Kabat consensus sequences). Maximal alignment of framework residues frequently requires the insertion of "spacer" residues in the numbering system, to be used for the Fv region. It will be understood that the CDRs referred to herein are those of Kabat et al. supra. In addition, the identity of certain individual residues at any given Kabat site number may vary from antibody chain to antibody chain due to interspecies or allelic divergence.

It can be envisioned that antibody targeting of multiple tyrosine kinase receptors (*e.g*. EphAl-8, EphA10, EphB 1-6) would be desirable as a way of preventing the development of resistance to therapy. It can also be envisioned that it may also be desirable to target only specific tyrosine kinase receptors (*e.g*. EphA2 only) or combinations of receptors (*e.g.* EphA2 and EphA4 only) by a single antibody, or to reduce affinity to specific tyrosine kinase receptors, in order to enhance efficacy, minimize side effects, target specific cells, or other reasons related to expression of the tyrosine kinase receptors.

In one embodiment, an Eph/Ephrin Modulator is an antibody, for example, a monoclonal antibody, or a fragment thereof. Antibody Eph/Ephrin Modulators of the invention specifically bind an Eph receptor (*e.g*., EphA1, EphA2, EphA3, EphA4, EphA2, EphA6, EphA7, EphA8, EphA10, EphB1, EphB2, EphB3, EphB4, EphB5 or EphB6) or an Ephrin (*e.g*., EphrinA1, EphrinA2, EphrinA3, EphrinA4, EphrinA5, EphrinB1, EphrinB2 or EphrinB3) and modulates the activity and/or expression of one or more Eph receptors and/or Ephrin ligands.

In a specific embodiment, an antibody of the invention specifically binds to the extracellular domain of an Eph receptor (*e.g*., at an epitope either within or outside of the Eph receptor ligand binding site) and decreases receptor cytoplasmic tail phosphorylation without causing Eph receptor degradation. In another specific embodiment, the antibody binds to the extracellular domain of an Eph receptor (*e.g*., at an epitope either within or outside of the Eph receptor ligand binding site) and inhibits or reduces the extent of Eph receptor-ligand interaction. In another specific embodiment, an antibody of the invention specifically binds to the extracellular domain of an Eph receptor (*e.g*., at an epitope either within or outside of the Eph receptor ligand binding site) and decreases Eph receptor signal transduction (including, but not limited to, Eph receptor autophosphorylation). In yet another embodiment, an antibody of the invention specifically binds to the extracellular domain of an Eph receptor (*e.g*., at an epitope either within or outside of the Eph receptor ligand binding site), decreases Eph receptor signal transduction (including, but not limited to, Eph receptor autophosphorylation) and inhibits or reduces the extent of Eph receptor-ligand interaction.

In one embodiment, an antibody of the invention specifically binds to an Ephrin ligand (*e.g*., at an epitope either within or outside of the Eph receptor binding site) and prevents or reduces the binding to its cognate Eph receptor. In another embodiment, the Ephrin antibody of the invention specifically binds to an Ephrin (*e.g*., at an epitope either within or outside of the Eph receptor binding site) and modulates (induces or inhibits) Ephrin signaling in an Ephrin-expressing cell. In another specific embodiment, an antibody of the invention specifically binds to an Ephrin (*e.g*., at an epitope either within or outside of the Eph receptor binding site), decreases Ephrin signal transduction and inhibits or reduces the extent of Eph receptor-Ephrin interaction. In another specific embodiment, an antibody of the invention specifically binds to an Ephrin (*e.g.*, at an epitope either within or outside of the Eph receptor binding site), induces Ephrin signal transduction and inhibits or reduces the extent of Eph-Ephrin interaction. In a further embodiment, an antibody of the invention specifically binds to an Ephrin (*e.g*., at an epitope involved in Ephrin clustering), inhibits or reduces Ephrin interaction with other molecules such as the Src family kinases (*e.g*., Fyn), and inhibits or reduces Ephrin signal transduction.

In particular embodiments, differences in the subcellular localization, ligand binding properties or protein organization (*e.g*., structure, orientation in the cell membrane) can further distinguish a particular Eph receptor and/or Ephrin that is present on cells affected by a disorder associated with aberrant (*i.e*., increased, decreased or inappropriate) Eph receptor and/or Ephrin expression (*e.g.*, cancer cells) from an Eph receptor and/or Ephrin expressed on normal cells. For example, but not by limitation, on non-cancer cells, EphA2 is expressed at low levels and is localized to sites of cell-cell contact, where it can engage its membrane-anchored ligands. However, cancer cells generally display decreased cell-cell contacts and this can decrease EphA2-ligand binding. Furthermore, the overexpression of EphA2 can cause an excess of EphA2 relative to ligand that increases the amount of non-ligand bound EphA2. Consequently, changes in the subcellular distribution or membrane orientation of EphA2 can cause EphA2 to localize to sites in a cancer cell where it is inaccessible to ligand. Additionally, EphA2 may have altered ligand binding properties (*e.g*., due to an altered conformation) in cancer cells such that it is incapable of stable interactions with its ligand whether or not it is localized to the cell-cell junction. In each case, these changes can expose certain epitopes on the EphA2 in cancer cells that are not exposed in non-cancer cells (i.e. an occluded epitope). Accordingly, the invention also provides antibodies that specifically bind to an Eph receptor and/or Ephrin but preferably bind an an Eph receptor and/or Ephrin epitope exposed on cells affected by a disorder associated with aberrant (*i.e*., increased, decreased or inappropriate) Eph receptor and/or Ephrin expression (*e.g*., cancer cells) but not normal cells ("exposed Eph receptor epitope antibodies" or "exposed Ephrin epitope antibodies"). Exposing cells affected by a disorder associated with aberrant (*i.e*., increased, decreased or inappropriate) Eph receptor and/or Ephrin expression (*e.g*., cancer cells) to such exposed Eph receptor epitope antibodies or exposed Ephrin epitope antibodies that preferentially bind epitopes on an Eph receptor or Ephrin that are selectively exposed or increased on said cells but not non-normal cells targets the therapeutic/prophylactic antibody to affected cells and prevents or decreases the cells' ability to proliferate while sparing non-affected cells.

Antibodies of the invention include, but are not limited to, synthetic antibodies, monoclonal antibodies, recombinantly produced antibodies, multispecific antibodies (including bi-specific antibodies), human antibodies, humanized antibodies, chimeric antibodies, intrabodies, single-chain Fvs (scFv) (*e.g*., including monospecific and bi-specific, etc.), Fab fragments, F(ab') fragments, disulfide-linked Fvs (sdFv), anti-idiotypic (anti-Id) antibodies, and epitope-binding fragments of any of the above. In particular, antibodies of the present invention include immunoglobulin molecules and immunologically active portions of immunoglobulin molecules, *i.e*., molecules that contain an antigen-binding site that specifically binds to an EphA2 antigen or an EphrinA1 antigen (*e.g*., one or more complementarity determining regions (CDRs) of an anti-EphA2 antibody or of an anti-EphrinA1 antibody). The antibodies of the invention can be of any type (*e.g*., IgG, IgE, IgM, IgD, IgA and IgY), class (*e.g*., IgG₁, IgG₂, IgG₃, IgG₄, IgA₁ and IgA₂) or subclass of immunoglobulin molecule.

The present invention encompasses agonistic antibodies that specifically bind to an Eph receptor and agonize the Eph receptor, *i.e*., elicit Eph receptor signaling and decrease Eph receptor expression. Agonistic Eph receptor antibodies may induce Eph receptor autophosphorylation, thereby causing subsequent Eph receptor degradation to down-regulate Eph receptor expression and inhibit Eph receptor interaction with an endogenous ligand (*e.g.*, an Ephrin). Such antibodies (*e.g*., agonistic EphA2 antibodies (*e.g.*, EA2, EA3, EA4 and EA5)) are disclosed in U.S. Patent Pub. Nos. US 2004/0091486 A1 (May 13, 2004), and US 2004/0028685 A1 (Feb. 12, 2004), and U.S. Pat. Appn. Ser. No. 10/823,254 (April 12, 2004), entitled "EphA2 and Hyperproliferative Cell Disorders" (Attorney Docket No. 10271-060-999), which are all incorporated by reference herein in their entireties. In a particular embodiment, the antibodies used in the methods of the invention are Eph099B-102.147, Eph099B-208.261, Eph099B-210.248, B233, EA2 or EA5. In another embodiment, the antibodies used in the methods of the invention are human or humanized Eph099B-102.147, Eph099B-208.261, Eph099B-210.248, B233, EA2 or EA5. In a specific embodiment, an EphA2/EphrinA1 Modulator is not Eph099B-102.147, Eph099B-208.261, Eph099B-210.248, B233, EA2 or EA5.

In a specific embodiment, an antibody of the invention comprises Fab (comprising VH, VL, or CDRs) amino acid sequences from antibodies GEA-44, 1A4, 1B10, 1D11, 1G11, 2C9, 11H1, 3A12, 3C6, 6B7, or 8B4 (SEQ ID Nos 114-135, 136-201; Figures 5A-B).

In a further specific embodiment, an antibody of the invention is selected from the group consisting of GEA-44, 1A4, 1B10, 1D11, 1G11, 2C9, 11H1, 3A12, 3C6, 6B7, or 8B4.

In another specific embodiment, an antibody of the invention is not D7, EA2, EA5, B210, B233, or B208.

The present invention also encompasses the use of antibodies that specifically bind to a member of the Eph family of receptors and agonize and/or preferentially bind an Eph epitope exposed in cancer cells, said antibodies comprising one or more VH CDRs and one or more VL CDRs of GEA-44, 1A4, 1B10, 1D11, 1G11, 2C9, 11H1, 3A12, 3C6, 6B7, or 8B4. In particular, the invention encompasses the use of antibodies that specifically bind to a member of the Eph family of receptors and agonize and/or preferentially bind an Eph epitope exposed in cancer cells, said antibodies comprising a VH CDR1 and a VL CDR1; a VH CDR1 and a VL CDR2; a VH CDR1 and a VL CDR3; a VH CDR2 and a VL CDR1; VH CDR2 and VL CDR2; a VH CDR2 and a VL CDR3; a VH CDR3 and a VL CDR1; a VH CDR3 and a VL CDR2; a VH CDR3 and a VL CDR3; a VH1 CDR1, a VH CDR2 and a VL CDR1; a VH CDR1, a VH CDR2 and a VL CDR2; a VH CDR1, a VH CDR2 and a VL CDR3; a VH CDR2, a VH CDR3 and a VL CDR1, a VH CDR2, a VH CDR3 and a VL CDR2; a VH CDR2, a VH CDR3 and a VL CDR3; a VH1 CDR1, a VH CDR3 and a VL CDR1; a VH CDR1, a VH CDR3 and a VL CDR2; a VH CDR1, a VH CDR3 and a VL CDR3; a VH CDR1, a VL CDR1 and a VL CDR2; a VH CDR1, a VL CDR1 and a VL CDR3; a VH CDR1, a VL CDR2 and a VL CDR3; a VH CDR2, a VL CDR1 and a VL CDR2; a VH CDR2, a VL CDR1 and a VL CDR3; a VH CDR2, a VL CDR2 and a VL CDR3; a VH CDR3, a VL CDR1 and a VL CDR2; a VH CDR3, a VL CDR1 and a VL CDR3; a VH CDR3, a VL CDR2 and a VL CDR3; a VH CDR1, a VH CDR2, a VH CDR3 and a VL CDR1; a VH CDR1, a VH CDR2, a VH CDR3 and a VL CDR2; a VH CDR1, a VH CDR2, a VH CDR3 and a VL CDR3; a VH CDR1, a VL CDR1, a VL CDR2 and a VL CDR3; a VH CDR2, a VL CDR1, a VL CDR2 and a VL CDR3; a VH CDR3, a VL CDR1, a VL CDR2 and a VL CDR3; a VH CDR1, a VH CDR2, a VL CDR1 and a VL CDR2; a VH CDR1, a VH CDR2, a VL CDR1 and a VL CDR3; a VH CDR1, a VH CDR2, a VL CDR2 and a VL CDR3; a VH CDR1, a VH CDR3, a VL CDR1 and a VL CDR2; a VH CDR1, a VH CDR3, a VL CDR1 and a VL CDR3; a VH CDR1, a VH CDR3, a VL CDR2 and a VL CDR3; a VH CDR2, a VH CDR3, a VL CDR1 and a VL CDR2; a VH CDR2, a VH CDR3, a VL CDR1 and a VL CDR3; a VH CDR2, a VH CDR3, a VL CDR2 and a VL CDR3; a VH CDR1, a VH CDR2, a VH CDR3, a VL CDR1 and a VL CDR2; a VH CDR1, a VH CDR2, a VH CDR3, a VL CDR1 and a VL CDR3; a VH CDR1, a VH CDR2, a VH CDR3, a VL CDR2 and a VL CDR3; a VH CDR1, a VH CDR2, a VL CDR1, a VL CDR2, and a VL CDR3; a VH CDR1, a VH CDR3, a VL CDR1, a VL CDR2, and a VL CDR3; a VH CDR2, a VH CDR3, a VL CDR1, a VL CDR2, and a VL CDR3; a VH CDR1, a VH CDR2, a VH CDR3, a VL CDR1, a VL CDR2, and a VL CDR3 or any combination thereof of the VH CDRs and VL CDRs of GEA-44, 1A4, 1B10, 1D11, 1G11, 2C9, 11H1, 3A12, 3C6, 6B7, or 8B4.

The present invention also encompasses antibodies that compete with GEA44, 1A4, 1B10, 1D11,1G11, 2C9, 11H1, 3A12, 3C6, 6B7, or 8B4, or an antigen-binding fragment thereof for binding to an Eph receptor. Competition assays, which can be used to identify such antibodies, are well known to one skilled in the art. In a particular embodiment, 1 µg/ml of an antibody of the invention would prevent 75%, 80%, 85% or 90% of ORIGEN TAG labeled GEA44, 1A4, 1B10, 1D11, 1G11, 2C9, 11H1, 3A12, 3C6, 6B7, or 8B4 from binding to biotin-labeled Eph receptor as measured by well-known ORIGEN analysis.

The present invention also provides antibodies that comprise a framework region known to those of skill in the art. In one embodiment, the fragment region of an antibody of the invention or fragment thereof is human or humanized.

The present invention encompasses antibodies comprising the amino acid sequence of GEA44, 1A4, 1B10, 1D11, 1G11, 2C9, 11H1, 3A12, 3C6, 6B7, or 8B4 with mutations (*e.g*., one or more amino acid substitutions) in the framework or variable regions in addition to any other substitutions or changes (*e.g*., Fc substitution(s)). In one embodiment, mutations in these antibodies maintain, decrease, or enhance the avidity and/or affinity of the antibodies for the Eph receptor to which they specifically bind. Techniques for tailoring the affinity of the antibodies are described herein below. Standard techniques known to those skilled in the art (*e.g*., immunoassays) can be used to assay the affinity of an antibody for a particular antigen.

The present invention encompasses the use of a nucleic acid molecule(s), generally isolated, encoding an antibody that specifically binds to an Eph receptor. In a specific embodiment, an isolated nucleic acid molecule encodes an antibody that specifically binds to an Eph receptor, said antibody having the amino acid sequence of GEA44, 1A4, 1B10, 1D11, 1G11, 2C9, 11H1, 3A12, 3C6, 6B7, or 8B4. In another embodiment, an isolated nucleic acid molecule encodes an antibody that specifically binds to and Eph receptor, said antibody comprising a VH domain having the amino acid sequence of the VH domain of GEA44, 1A4, 1B10, 1D11, 1G11, 2C9, 11H1, 3A12, 3C6, 6B7, or 8B4. In another embodiment, an isolated nucleic acid molecule encodes an antibody that specifically binds to an Eph receptor, said antibody comprising a VL domain having the amino acid sequence of the VL domain of GEA44, 1A4, 1B10, 1D11, 1G11, 2C9, 11H1, 3A12, 3C6, 6B7, or 8B4.

The invention encompasses the use of an isolated nucleic acid molecule encoding an antibody that specifically binds to an Eph receptor, said antibody comprising a VH CDR having the amino acid sequence of any of the VH CDRs listed in Figures 5A-B and/or derived from the heavy chain of any of the antibodies listed in Figures 5A-B. In particular, the invention encompasses the use of an isolated nucleic acid molecule encoding an antibody that specifically binds to an Eph receptor, said antibody comprising one, two, or more VH CDRs having the amino acid sequence of any of the VH CDRs listed in Figures 5A-B and/or derived from the heavy chain of any of the antibodies listed in Figures 5A-B.

The present invention encompasses the use of an isolated nucleic acid molecule encoding an antibody that specifically binds to an Eph receptor, said antibody comprising a VL CDR having an amino acid sequence of any of the VL CDRs listed in Figures 5A-B, and/or derived from the light chain of any of the antibodies listed in Figures 5A-B. In particular, the invention encompasses the use of an isolated nucleic acid molecule encoding an antibody that specifically binds to an Eph receptor, said antibody comprising one, two or more VL CDRs having the amino acid sequence of any of the VL CDRs listed in Figures 5A-B and/or derived from the light chain of any of the antibodies listed in Figures 5A-B.

The present invention encompasses the use of antibodies that specifically bind to an Eph receptor, antibodies comprising derivatives of the VH domains, VH CDRs, VL domains, or VL CDRs described herein that specifically bind to an Eph receptor. Standard techniques known to those of skill in the art can be used to introduce mutations (*e.g*., additions, deletions, and/or substitutions) in the nucleotide sequence encoding an antibody of the invention, including, for example, site-directed mutagenesis and PCR-mediated mutagenesis are routinely used to generate amino acid substitutions. Techniques for guided selection and tailoring of antibodies are further described in more detail herein below.

In one embodiment, the VH and/or VL CDRs derivatives include less than 25 amino acid substitutions, less than 20 amino acid substitutions, less than 15 amino acid substitutions, less than 10 amino acid substitutions, less than 5 amino acid substitutions, less than 4 amino acid substitutions, less than 3 amino acid substitutions, or less than 2 amino acid substitutions in the relative to the original VH and/or VL CDRs. In another embodiment, the VH and/or VL CDRs derivatives have conservative amino acid substitutions (*e.g*. supra) are made at one or more predicted non-essential amino acid residues (i.e., amino acid residues which are not critical for the antibody to specifically bind to an Eph receptor). Alternatively, mutations can be introduced randomly along all or part of the VH and/or VL CDR coding sequence, such as by saturation mutagenesis, and the resultant mutants can be screened for biological activity to identify mutants that retain activity. Following mutagenesis, the encoded antibody can be expressed and the activity of the antibody can be determined.

The present invention encompasses antibodies of GEA44, 1A4, 1B10, 1D11, 1G11, 2C9, 11H1, 3A12, 3C6, 6B7, or 8B4 with one or more additional amino acid residue substitutions in the variable light (VL) domain and/or variable heavy (VH) domain. The present invention also encompasses antibodies of GEA44, 1A4, 1B10, 1D11, 1G11, 2C9, 11H1, 3A12, 3C6, 6B7, or 8B4 with one or more additional amino acid residue substitutions in one or more VL CDRs and/or one or more VH CDRs. The antibody generated by introducing substitutions in the VH domain, VH CDRs, VL domain and/or VL CDRs of an antibody of GEA44, 1A4, 1B10, 1D11, 1G11, 2C9, 11H1, 3A12, 3C6, 6B7, or 8B4 can be tested *in vitro* and *in vivo,* for example, for its ability to bind to an Eph receptor (by, *e.g.,* immunoassays including, but not limited to ELISAs and BIAcore), or for its ability to mediate ADCC, prevent, treat, manage or ameliorate cancer or one or more symptoms thereof.

The present invention also encompasses the use of antibodies that specifically bind to at least one Eph receptor or a fragment thereof, said antibodies comprising an amino acid sequence, or fragments thereof (*e.g*. one or more CDRs) of a variable heavy chain and/or variable light chain that is at least 45%, at least 50%, at least 55%, at least 60%, at least 65%, at least 70%, at least 75%, at least 80%, at least 85%, at least 90%, at least 95%, or at least 99% identical to the amino acid sequence of the variable heavy chain and/or light chain of GEA44, 1A4, 1B10, 1D11, 1G11, 2C9, 11H1, 3A12, 3C6, 6B7, or 8B4 (SEQ IDs 114-135, 136-201; Figures 5A-B). The present invention also encompasses the use of antibodies that specifically bind to at least one Eph receptor or a fragment thereof, said antibodies comprising an amino acid sequence of a variable heavy chain and/or variable light chain that is at least about 45%, at least about 50%, at least about 55%, at least about 60%, at least about 65%, at least about 70%, at least about 75%, at least about 80%, at least about 85%, at least about 90%, at least about 95%, or at least about 99% identical to the amino acid sequence of the variable heavy chain and/or light chain of GEA44, 1A4, 1B10, 1D11, 1G11, 2C9, 11H1, 3A12, 3C6, 6B7, or 8B4. The present invention further encompasses the use of antibodies that specifically bind to at least one Eph receptor or a fragment thereof, said antibodies or antibody fragments comprising an amino acid sequence of one or more CDRs that is at least 45%, at least 50%, at least 55%, at least 60%, at least 65%, at least 70%, at least 75%, at least 80%, at least 85%, at least 90%, at least 95%, or at least 99% identical to the amino acid sequence of one or more CDRs of GEA44, 1 A4, 1B10, 1D11, 1 G 11, 2C9, 11H1, 3A12, 3C6, 6B7, or 8B4.

The present invention further encompasses the use of antibodies that specifically bind to at least one Eph receptor or a fragment thereof, said antibodies or antibody fragments comprising an amino acid sequence of one or more CDRs that is at least about 45%, at least about 50%, at least about 55%, at least about 60%, at least about 65%, at least about 70%, at least about 75%, at least about 80%, at least about 85%, at least about 90%, at least about 95%, or at least about 99% identical to the amino acid sequence of one or more CDRs of GEA44, 1A4, 1B10, 1D11, 1G11, 2C9, 11H1, 3A12, 3C6, 6B7, or 8B4. The determination of percent identity of two amino acid sequences can be determined by any method known to one skilled in the art, including Basic Local Alignment Search Tool (BLAST) protein searches (Altschul, S.F. et al., 1990, J. Mol. Biol. 215:403-410).

The present invention also encompasses the use of antibodies that specifically bind to at least one Eph receptor or fragments thereof, where said antibodies are encoded by a nucleotide sequence that hybridizes to the nucleotide sequence of GEA44, 1A4, 1B10, 1D11, 1G11, 2C9, 11H1, 3A12, 3C6, 6B7, or 8B4 under stringent conditions. In another embodiment, the invention encompasses antibodies that specifically bind to an Eph receptor or a fragment thereof, said antibodies comprising one or more CDRs encoded by a nucleotide sequence that hybridizes under stringent conditions to the nucleotide sequence of one or more CDRs of GEA44, 1A4, 1B10, 1D11, 1G11, 2C9, 11H1, 3A12, 3C6, 6B7, or 8B4. Stringent hybridization conditions include, but are not limited to, hybridization to filter-bound DNA in 6X sodium chloride/sodium citrate (SSC) at about 45°C followed by one or more washes in 0.2X SSC/0.1% SDS at about 50-65°C, highly stringent conditions such as hybridization to filter-bound DNA in 6X SSC at about 45°C followed by one or more washes in 0.1X SSC/0.2% SDS at about 60°C, or any other stringent hybridization conditions known to those skilled in the art (see, for example, Ausubel, F.M. et al., eds. 1989 Current Protocols in Molecular Biology, vol. 1, Green Publishing Associates, Inc. and John Wiley and Sons, Inc., NY at pages 6.3.1 to 6.3.6 and 2.10.3).

In a specific embodiment, the antibodies of the invention comprise the variable heavy chain and/or variable light chain amino acid sequence of the antibody sequences as disclosed in Fig. 5A and 5B, wherein said antibodies possess at least 90% amino acid sequence identity with the variable heavy chain or light chain amino acid sequences of the antibodies as disclosed in Figures 5A and 5B. In a further specific embodiment, the antibodies of the invention comprise at least one CDR of said heavy chain and/or light chain sequence of the antibodies as disclosed in Figures 5A and 5B (SEQ ID Nos 114-201), wherein said CDR is identical to the corresponding CDR as disclosed in Figures 5A and 5B. In another specific embodiment, the antibodies of the invention comprise at least two CDRs of said heavy chain and/or light chain sequence of the antibodies as disclosed in Figures 5A and 5B, wherein said CDRs are identical to the corresponding CDRs as disclosed in Figures 5A and 5B. In yet another specific embodiment, the antibodies of the invention comprise at least three CDRs of said heavy chain and/or light chain sequence of the antibodies as disclosed in Figures 5A and 5B, wherein said CDRs are identical to the corresponding CDRs as disclosed in Figures 5A and 5B. In a further specific embodiment, the antibodies of the invention comprise at least four CDRs of said heavy chain and/or light chain sequence of the antibodies as disclosed in Figures 5A and 5B, wherein said CDRs are identical to the corresponding CDRs as disclosed in Figures 5A and 5B. In another specific embodiment, the antibodies of the invention comprise at least five CDRs of said heavy chain and/or light chain sequence of the antibodies as disclosed in Figures 5A and 5B, wherein said CDRs are identical to the corresponding CDRs as disclosed in Figures 5A and 5B. In further specific embodiment, the antibodies of the invention comprise all six of the CDRs of said heavy chain and/or light chain sequence of the antibodies as disclosed in Figures 5A and 5B, wherein said CDRs are identical to the corresponding CDRs as disclosed in Figures 5A and 5B.

In another specific embodiment, the antibodies bind to an Eph receptor or fragment thereof, said antibodies comprising at least two CDRs of said heavy chain and/or light chain sequence are identical to the corresponding CDRs of the antibodies as disclosed in Figures 5A and 5B. In a further specific embodiment, the antibodies bind to an Eph receptor or fragment thereof, said antibodies comprising at least three CDRs of said heavy chain and/or light chain sequence are identical to the corresponding CDRs of the antibodies as disclosed in Figures 5A and 5B. In another specific embodiment, the antibodies bind to an Eph receptor or fragment thereof, said antibodies comprising at least four CDRs of said heavy chain and/or light chain sequence are identical to the corresponding CDRs of the antibodies as disclosed in Figures 5A and 5B. In a further specific embodiment, the antibodies bind to an Eph receptor or fragment thereof, said antibodies comprising at least five CDRs of said heavy chain and/or light chain sequence are identical to the corresponding CDRs of the antibodies as disclosed in Figures 5A and 5B. In yet a further specific embodiment, the antibodies bind to an Eph receptor or fragment thereof, said antibodies comprising all six CDRs of said heavy chain and/or light chain sequence are identical to the corresponding CDRs of the antibodies as disclosed in Figures 5A and 5B.

It is envisioned that the antibodies of the present invention will be tailored and selected due to their varying binding affinities for a desired antigen (e.g. a member of the receptor tyrosine kinase family or class). Binding affinity can be measured using various techniques known in the art. For example, enzyme linked immunosorbent assay (ELISA) is used to screen for an antibody with desired binding activity. A nonlimiting example of using this technique is provided in Example 1 herein below. In general, ELISAs comprise preparing antigen, coating the wells of a microtiter plate with the antigen, washing away antigen that did not bind the wells, adding the antibody of interest conjugated to a detectable compound such as an enzymatic substrate (*e.g*., horseradish peroxidase or alkaline phosphatase) to the wells and incubating for a period of time, washing away unbound antibodies or non-specifically bound antibodies, and detecting the presence of the antibodies specifically bound to the antigen coating the well. In ELISAs, the antibody of interest does not have to be conjugated to a detectable compound; instead, a second antibody (which recognizes the antibody of interest) conjugated to a detectable compound may be added to the well. Further, instead of coating the well with the antigen, the antibody may be coated to the well. In this case, the detectable molecule could be the antigen conjugated to a detectable compound such as an enzymatic substrate (*e.g*., horseradish peroxidase or alkaline phosphatase). One of skill in the art would be knowledgeable as to the parameters that can be modified to increase the signal detected as well as other variations of ELISAs known in the art. For further discussion regarding ELISAs see, *e.g.*, Ausubel et al., eds, 1994, Current Protocols in Molecular Biology, Vol. I, John Wiley & Sons, Inc., New York at 11.2.1.

In another example of measuring affinity, BIAcore kinetic analysis can be used to determine the binding on and off rates (Kd) of antibodies of the invention to a specific antigen. BIAcore kinetic analysis comprises analyzing the binding and dissociation of an antigen from chips with immobilized antibodies of the invention on their surface. *See* Wu et al., 1999, J. Mol. Biol., 294:151-162, which is incorporated herein by reference in its entirety. Briefly, antigen-Ig fusion protein is immobilized to a (1-ethyl-3-(3-dimethylaminopropyl)-carbodiimide hydrochloride) and N-hydroxy-succinimide-activated sensor chip CM5 by injecting antigen-Ig in sodium acetate. Antigen-Ig is immobilized at a low density to prevent rebinding of Fabs during the dissociation phase. To obtain association rate constant (Kon), the binding rate at six different Fab concentrations is determined at certain flow rate. Dissociation rate constant (Koff) are the average of six measurements obtained by analyzing the dissociation phase. Sensorgrams are analyzed with the BIAevaluation 3.0 program. Kd is calculated from Kd = Koff/Kon. Residual Fab is removed after each measurement by prolonged dissociation.

The binding affinity of an antibody (including a scFv or other molecule comprising, or alternatively consisting of, antibody fragments or variants thereof) to an antigen and the off-rate of an antibody-antigen interaction can also be determined by competitive binding assays. One example of a competitive binding assay is a radioimmunoassay comprising the incubation of labeled antigen (*e.g*., ³H or ¹²¹I) with the antibody of interest in the presence of increasing amounts of unlabeled antigen, and the detection of the antibody bound to the labeled antigen. The affinity of the antibody of the present invention and the binding off-rates can be determined from the data by Scatchard plot analysis. Competition with a second antibody can also be determined using radioimmunoassays. In this case, an antigen is incubated with an antibody of the present invention conjugated to a labeled compound (*e.g*., ³H or ¹²¹I) in the presence of increasing amounts of an unlabeled second antibody.

Other assays, such as immunoassays, including but not limited to, competitive and non-competitive assay systems using techniques such as westem blots, radioimmunoassays, ELISA (enzyme linked immunosorbent assay), sandwich immunoassays, immunoprecipitation assays, precipitin reactions, gel diffusion precipitin reactions, immunodiffusion assays, agglutination assays, complement-fixation assays, fluorescent immunoassays, and protein A immunoassays, can also be used to screen or further characterization of the binding specificity of a humanized antibody. Such assays are routine and well known in the art (see, *e.g*., Ausubel et al., eds, 1994, Current Protocols in Molecular Biology, Vol. 1, John Wiley & Sons, Inc., New York, which is incorporated by reference herein in its entirety).

In a specific embodiment, the binding affinity of the antibodies of the invention is tailored such that the binding affinity to one member of the Eph family of receptors is increased as compared to the binding affinity to at least one other member of the Eph family of receptors by at least 1.1 fold, at least 1.2 fold, at least 1.3 fold, at least 1.4 fold, at least 1.5 fold, at least 1.6 fold, at least 1.7 fold, at least 1.8 fold, at least 1.9 fold, at least 2 fold, at least 2.5 fold, at least 3 fold, at least 3.5 fold, at least 4 fold, at least 4.5 fold, at least 5 fold, at least 6 fold, at least 7 fold, at least 8 fold, at least 9 fold, at least 10 fold, at least 15 fold, at least 20 fold, at least 30 fold, at least 40 fold, at least 50 fold, at least 100 fold, at least 200 fold, at least 300 fold at least 400 fold, at least 500 fold, or at least 1000 fold.

In a further specific embodiment, the binding affinity of the antibodies of the invention is tailored such that the binding affinity to one member of the Eph family of receptors is decreased as compared to the binding affinity to at least one other member of the Eph family of receptors by at least 1.1 fold, at least 1.2 fold, at least 1.3 fold, at least 1.4 fold, at least 1.5 fold, at least 1.6 fold, at least 1.7 fold, at least 1.8 fold, at least 1.9 fold, at least 2 fold, at least 2.5 fold, at least 3 fold, at least 3.5 fold, at least 4 fold, at least 4.5 fold, at least 5 fold, at least 6 fold, at least 7 fold, at least 8 fold, at least 9 fold, at least 10 fold, at least 15 fold, at least 20 fold, at least 30 fold, at least 40 fold, at least 50 fold, at least 100 fold, at least 200 fold, at least 300 fold at least 400 fold, at least 500 fold, or at least 1000 fold.

In another specific embodiment, the binding affinity of the antibodies of the invention is tailored such that the antibodies of the invention have a binding affinity with a K_{D} of at least 1×10⁶ M⁻¹, at least 1×10⁷ M⁻¹, at least 1×10⁸ M⁻¹, at least 1×10⁹ M⁻¹, at least 1×10¹⁰ M⁻¹ at least 1×10¹¹ M⁻¹¹, at least 1x10¹² M⁻¹, at least 1×10¹³ M⁻¹, at least 1x10¹⁴ M⁻¹, or at least 1x10¹⁵ M⁻¹ for a member or for members of the Eph receptor family. In yet another specific embodiment, the binding affinity of the antibodies of the invention is tailored such that the antibodies of the invention have a binding affinity with a K_{D} of between 1×10⁶ M⁻¹ and 1×10⁷ M⁻¹, between 1×10⁷ M⁻¹ and 1×10⁸ M⁻¹, between 1×10⁸ M⁻¹ and 1×10⁹ M⁻¹, between 1×10⁹ M⁻¹ and 1×10¹⁰ M⁻¹, between 1×10¹⁰ M⁻¹ and 1×10¹¹ M⁻¹, between 1×10¹¹ M⁻¹ and 1x10¹² M⁻¹, between 1×10¹² M⁻¹ and 1×10¹³ M⁻¹, between 1×10¹³ M⁻¹ and 1×10¹⁴ M⁻¹, or between 1×10¹⁴ m⁻¹ and 1×10¹⁵ M⁻¹ for a member or for members of the Eph receptor family.

In one embodiment, the antibody of the invention preferentially binds EphA2 over other Eph receptors. In another embodiment, the antibody of the invention preferentially binds EphA4 over other Eph receptors. In still another embodiment, the antibody of the invention specifically binds to one or more Eph receptor complex (*e.g*., an Eph receptor-Ephrin ligand complex). In still another embodiment, an antibody of the invention specifically binds more than one Eph receptor. Combinations of Eph receptors bound by an antibody that specifically binds more than one Eph receptor are represented by the following formulas: EphA(x) + [EphB(y)](n₂); EphA(x) + [EPhA(x)](n₁); EPhB(y)+ [EphB(y)](n₂); EphB(y) + [EphA(x)](n₁); or [EphA(x)](n₁) + [EphB(y)](n₂), wherein (x) is 1, 2, 3, 3a, 3b, 4, 5, 5a, 5b, 6, 7, 8, or 10 and (y) is 1, 2, 2a, 2b, 3, 4, 5 or 6, (n₁) is a multiple of 0-8 and (n₂) is a multiple of 0-5. In a specific embodiment, an antibody that specifically reacts with more than one Eph receptor binds to, for example, EphA2 + EphA4, or EphA2 + EphA3, or EphA2 + EphB4, or EphA4 + EphA3, or EphA4 + EphB4, or EphA2 + EphA3 + EphA4 + EphA5 + EphA6 + EphA7 + EphA8, or EphaA1 + EphA5, or EphA2 +EphA3 + EphA4 +EphA5 + EphA6 + EphA7 +EphA8 +EphB 1+ EphB2 + EphB3. It can be envisioned that the binding affinity of an antibody that binds more than one Eph receptor as represented in the formulas *supra* can be tailored to be the same affinity for each target receptor, increased affinity for one, some, or all target receptors, decreased affinity for one, some or all target receptors, or any combination of the same, increased, or decreased binding affinities.

In one specific embodiment, the antibody that specifically binds more than one Eph receptor is a bispecific antibody. In another specific embodiment, the antibody that specifically binds more than one Eph receptor is a trispecific antibody.

It will be recognized by one skilled in the art that the members of the Eph family of receptors, while being distinct proteins, have regions of homology (*see,* Figures 1A-1R and 2A-2G). Accordingly, it is contemplated that high affinity antibodies or fragments thereof may specifically bind to a first Eph receptor (*e.g*. EphA2) and antigenic fragments thereof and not bind to a second Eph receptor or antigenic fragments thereof. It is further contemplated that the high affinity antibodies of the invention may specifically bind an epitope that is common to one, several, or all of the Eph receptors, is common to one, several, or all of the EphA receptors, or is common to one, several, or all of the EphB receptors.

Regions of a molecule that an antigen specifically binds to are known as "antigenic determinants" or "epitopes" ( Janeway and Travers, Immunobiology, Chapter 3, Garland Publishing, Inc., 3rd Edition, 1997). These sites on proteins are typically composed of amino acids from different parts of the protein's amino acid sequence that are brought together as a three-dimensional epitope when the protein is folded ( Janeway and Travers, Immunobiology, Chapter 3, Garland Publishing, Inc., 3rd Edition, 1997). These types of epitopes are known as "conformational eptitopes" or "discontinuous epitopes," as they are formed from discontinuous amino acid sequences with regard to the primary amino acid sequence, yet are formed from contiguous amino acid sequences with regard to the three-dimensional structure ( Janeway and Travers, Immunobiology, Chapter 3, Garland Publishing, Inc., 3rd Edition, 1997). In contrast, epitopes that consist of a contiguous length of the primary amino acid sequence of a protein are called "continuous epitopes" or "linear epitopes" ( Janeway and Travers, Immunobiology, Chapter 3, Garland Publishing, Inc., 3rd Edition, 1997).

A common epitope may be identical in all of the Eph receptors, or in select Eph receptors. In such a case the amino acid sequence comprising the epitope is identical in the target Eph receptors. Accordingly, the antibodies of the invention may specifically bind to one, several, or all members of the Eph family (*e.g*., an antibody that specifically recognized a identical epitope that is present in one, several, or all Eph receptors). Alternatively, a common epitope may be similar in one, several, or all members of the Eph receptor family. For example, a similar epitope may share significant homology (*e.g.*, 60%-99% identity) and/or adopt a similar three-dimensional conformation between the different Eph receptors such that an antibody of the present invention will specifically bind to the shared epitope. Accordingly, the antibodies of the invention may specifically bind a common epitope of one, several, or all members of the Eph receptor family. An antibody of the present invention may have differing affinities for a common epitope or similar epitope present on one, several, or all members of the Eph family. Accordingly, it is further contemplated that the antibodies of the invention bind one, several, or all members of the Eph receptor family with either the same or altered binding affinities. In a specific embodiment, antibodies or fragments thereof specifically bind one, several, or all members of the Eph receptor family over other antigens. In a further specific embodiment, provided is a monoclonal antibody that specifically binds the Eph receptor epitope to which monoclonal antibody GEA44 binds.

The present invention also encompasses antagonistic antibodies that specifically bind to Eph receptor and antagonize the Eph receptor, *i.e*., decrease Eph receptor cytoplasmic tail phosphorylation, and decreases/disrupts Eph receptor-ligand interaction. Antagonistic Eph receptor antibodies may reduce or inhibit Eph receptor autophosphorylation, thereby causing an increase in Eph receptor protein stability or protein accumulation. Such antibodies (*e.g*., antagonistic EphA2 antibodies) are disclosed in U.S. Pat. Appn. Ser. No. 10/823,259 filed April 12, 2004, entitled "EphA2, Hypoproliferative Cell Disorders and Epithelial and Endothelial Cell Reconstitution", which is incorporated by reference herein in its entirety.

Antibodies, like all polypeptides, have an Isoelectric Point (pI), which is generally defined as the pH at which a polypeptide carries no net charge. It is known in the art that protein solubility is typically lowest when the pH of the solution is equal to the isoelectric point (pI) of the protein. It is possible to optimize solubility by altering the number and location of ionizable residues in the antibody to adjust the pI. For example the pI of a polypeptide can be manipulated by making the appropriate amino acid substitutions (*e.g*., by substituting a charged amino acid such as a lysine, for an uncharged residue such as alanine). Without wishing to be bound by any particular theory, amino acid substitutions of an antibody that result in changes of the pI of said antibody may improve solubility and/or the stability of the antibody. One skilled in the art would understand which amino acid substitutions would be most appropriate for a particular antibody to achieve a desired pI. The pI of a protein may be determined by a variety of methods including but not limited to, isoelectric focusing and various computer algorithms (see for example Bjellqvist et al., 1993, Electrophoresis 14:1023).

In one embodiment, substitutions resulting in alterations in the pI of the antibodies of the invention will not significantly diminish its binding affinity for a member of the receptor tyrosine kinase family or class of receptors. As used herein the pI value is defined as the pI of the predominant charge form. The pI of a protein may be determined by a variety of methods including but not limited to, isoelectric focusing and various computer algorithms (see, *e.g*., Bjellqvist et al., 1993, Electrophoresis 14:1023). In a specific embodiment, the antibodies of the invention have a specific pI of at least 6.0, at least 6.1, at least 6.2, at least 6.3, at least 6.4, at least 6.5, at least 6.6, at least 6.7, at least 6.8, at least 6.9, at least 7.0, at least 7.1, at least 7.2, at least 7.3, at least 7.4, at least 7.5, at least 7.6, at least 7.7, at least 7.8, at least 7.9, at least 8.0, at least 8.1, at least 8.2, at least 8.3, at least 8.4, at least 8.5, at least 8.6, at least 8.7, at least 8.8, at least 8.9, or at least 9.0.

The Tm of the Fab domain of an antibody, can be a good indicator of the thermal stability of an antibody and may further provide an indication of the shelf-life. A lower Tm indicates more aggregation/less stability, whereas a higher Tm indicates less aggregation/ more stability. Accordingly, antibodies having higher Tm are preferable. In one embodiment, the Fab domain of an antibody has a Tm value higher than at least 50°C, 55°C, 60°C, 65°C, 70°C, 75°C, 80°C, 85°C, 90°C, 95°C, 100°C, 105°C, 110°C, 115°C or 120°C. In another embodiment, the Fab domain of an antibody has a Tm value higher than at least about 50°C, about 55°C, about 60°C, about 65°C, about 70°C, about 75°C, about 80°C, about 85°C, about 90°C, about 95°C, about 100°C, about 105°C, about 110°C, about 115°C or about 120°C. Thermal melting temperatures (Tm) of a protein domain (e.g., a Fab domain) can be measured using any standard method known in the art, for example, by differential scanning calorimetry (see, e.g., Vermeer et al., 2000, Biophys. J. 78:394-404; Vermeer et al., 2000, Biophys. J. 79: 2150-2154).

The present invention also encompasses antibodies that are Fc variants with enhanced antibody dependent cell-mediated cytotoxicity activity. Nonlimiting examples of such Fc variant antibodies are disclosed in U.S. Patent Applications 11/203,253 (filed August 15, 2005) and 11/203,251 (filed August 15, 2005), and U.S. Provisional Patent Applications 60/674,674 (filed April 26, 2005) and 60/713,711 (filed September 6, 2005), each of which is incorporated by reference herein in its entirety.

The present invention also encompasses single domain antibodies, including camelized single domain antibodies (see, *e.g*., Muyldermans et al., 2001, Trends Biochem. Sci. 26:230; Nuttall et al., 2000, Cur. Pharm. Biotech. 1:253; Reichmann and Muyldermans, 1999, J. Immunol. Meth. 231:25; International Patent Publication Nos. WO 94/04678 and WO 94/25591; U.S. Patent No. 6,005,079; which are incorporated herein by reference in their entireties). In one embodiment, the present invention provides single domain antibodies comprising two V_{H} domains having the amino acid sequence of a V_{H} domain(s) of any Eph receptor or Ephrin antibody(ies) with modifications such that single domain antibodies are formed. In another embodiment, the present invention also provides single domain antibodies comprising two V_{H} domains comprising one or more of the V_{H} CDRs of any Eph receptor or Ephrin antibody(ies).

Antibodies of the invention include Eph receptor or Ephrin intrabodies (*see* Section 6.1.1.1.1). Antibody Eph/Ephrin Modulators of the invention that are intrabodies specifically bind an Eph receptor or an Ephrin and modulate (increase or decrease) the expression and/or activity of the Eph receptor or the Ephrin. In a specific embodiment, an intrabody of the invention specifically binds to the intracellular domain of an Eph receptor and decreases Eph receptor cytoplasmic tail phosphorylation without causing Eph receptor degradation. In another embodiment, an intrabody of the invention specifically binds to an Eph receptor and prevents or reduces Eph receptor signal transduction (including, but not limited to Eph receptor autophosphorylation) but does not inhibit or reduce the interaction between the Eph receptor and an endogenous ligand(s) of the Eph receptor, *e.g*., an Ephrin.

The antibodies used in the methods of the invention may be from any animal origin including birds and mammals (*e.g.*, human, murine, donkey, sheep, rabbit, goat, guinea pig, camel, horse, or chicken). In a specific embodiment, the antibody is human or has been humanized. As used herein, "human" antibodies include antibodies having the amino acid sequence of a human immunoglobulin and include antibodies isolated from human immunoglobulin libraries or from mice that express antibodies from human genes.

The antibodies used in the methods of the present invention may be monospecific, bispecific, trispecific or of greater multispecificity. Multispecific antibodies may specifically bind to different epitopes of an Eph receptor polypeptide or an Ephrin polypeptide or may specifically bind to multiple Eph receptor polypeptides or and/or multiple Ephrin polypeptide as well a heterologous epitope, such as a heterologous polypeptide or solid support material. See, *e.g*., International Patent Publication Nos. WO 93/17715, WO 92/08802, WO 91/00360, and WO 92/05793; Tutt et al., 1991, J. Immunol. 147:60-69; U.S. Patent Nos. 4,474,893, 4,714,681, 4,925,648, 5,573,920, and 5,601,819; and Kostelny et al., 1992, J. Immunol. 148:1547-1553.

In a specific embodiments, the present invention encompasses methods that could be used to minimize the potential toxicities to normal cells that could arise following the use of any of the antibodies of the invention, including, but not limited to, Eph receptor antibodies (*e.g*., EphA2) and Ephrin antibodies. In one embodiment, a method encompassed by the invention encompasses the use of pan phage or antibody libraries against normal tissues. Further to this embodiment, such method involves the pre-adsorption of antibodies that react against normal or healthy cells or tissues. The source of the normal or healthy cells or tissues could be isolated from, *e.g*., blood, skin, and/or specific organs of potential toxicity (*e.g.,* heart, lung, pancreas, ovary, spleen, testis, adrenal gland, colon, uterus, prostate, skin, kidney, urinary bladder or liver). In another embodiment, primary cell lines derived from normal tissues could be used for the adsorption of antibodies to prevent or minimize toxicities. In a specific embodiment, the Eph/Ephrin Modulator antibodies of the invention do not bind to normal cells of the human heart or are not potentially toxic to normal cells of the human heart.

### 5.1.1.1.1 Intrabodies

In certain embodiments, the antibody to be used with the invention binds to an intracellular epitope, *i.e*., is an intrabody. In a specific embodiment, an intrabody of the invention binds to the cytoplasmic domain of an Eph receptor and prevents Eph receptor signaling (*e.g*., autophosphorylation). In another specific embodiment, an intrabody of the invention binds to the cytoplasmic domain of a B-type Ephrin (*e.g*., EphrinB1, EphrinB2 or EphrinB3). An intrabody comprises at least a portion of an antibody that is capable of specifically binding an antigen and preferably does not contain sequences coding for its secretion. Such antibodies will bind antigen intracellularly. In one embodiment, the intrabody comprises a single-chain Fv ("scFv"). scFvs are antibody fragments comprising the V_{H} and V_{L} domains of antibody, wherein these domains are present in a single polypeptide chain. Generally, the scFv polypeptide further comprises a polypeptide linker between the V_{H} and V_{L} domains which enables the scFv to form the desired structure for antigen binding. For a review of scFvs see Pluckthun in The Pharmacology of Monoclonal Antibodies, vol. 113, Rosenburg and Moore eds. Springer-Verlag, New York, pp. 269-315 1994). In a further embodiment, the intrabody preferably does not encode an operable secretory sequence and thus remains within the cell (see generally Marasco, WA, 1998, "Intrabodies: Basic Research and Clinical Gene Therapy Applications" Springer:New York).

Generation of intrabodies is well-known to the skilled artisan and is described, for example, in U.S. Patent Nos. 6,004,940; 6,072,036; 5,965,371, which are incorporated by reference in their entireties herein. Further, the construction of intrabodies is discussed in Ohage and Steipe, 1999, J. Mol. Biol. 291:1119-1128; Ohage et al., 1999, J. Mol. Biol. 291:1129-1134; and Wirtz and Steipe, 1999, Protein Science 8:2245-2250, which references are incorporated herein by reference in their entireties. Recombinant molecular biological techniques such as those described for recombinant production of antibodies may also be used in the generation of intrabodies.

In one embodiment, intrabodies of the invention retain at least about 75% of the binding effectiveness of the complete antibody (*i.e*., having the entire constant domain as well as the variable regions) to the antigen. In another embodiment, the intrabody retains at least 85% of the binding effectiveness of the complete antibody. In a further embodiment, the intrabody retains at least 90% of the binding effectiveness of the complete antibody. In yet a further embodiment, the intrabody retains at least 95% of the binding effectiveness of the complete antibody.

In producing intrabodies, polynucleotides encoding variable region for both the V_{H} and V_{L} chains of interest can be cloned by using, for example, hybridoma mRNA or splenic mRNA as a template for PCR amplification of such domains (Huse et al., 1989, Science 246:1276). In one embodiment, the polynucleotides encoding the V_{H} and V_{L} domains are joined by a polynucleotide sequence encoding a linker to make a single chain antibody (scFv). The scFv typically comprises a single peptide with the sequence V_{H} -linker-V_{L} or V_{L}-linker-V_{H}. The linker is chosen to permit the heavy chain and light chain to bind together in their proper conformational orientation (see, for example, Huston et al., 1991, Methods in Enzym. 203:46-121, which is incorporated herein by reference). In a further embodiment, the linker can span the distance between its points of fusion to each of the variable domains (*e.g.*, 3.5 nm) to minimize distortion of the native Fv conformation. In such an embodiment, the linker is a polypeptide of at least 5 amino acid residues, at least 10 amino acid residues, at least 15 amino acid residues, or greater. In a further embodiment, the linker should not cause a steric interference with the V_{H} and V_{L} domains of the combining site. In such an embodiment, the linker is 35 amino acids or less, 30 amino acids or less, or 25 amino acids or less. Thus, in yet a further embodiment, the linker is between 15-25 amino acid residues in length. In a further embodiment, the linker is hydrophilic and sufficiently flexible such that the V_{H} and V_{L} domains can adopt the conformation necessary to detect antigen. Intrabodies can be generated with different linker sequences inserted between identical V_{H} and V_{L} domains. A linker with the appropriate properties for a particular pair of V_{H} and V_{L} domains can be determined empirically by assessing the degree of antigen binding for each. Examples of linkers include, but are not limited to, those sequences disclosed in Table 5.

**Table 5**

| **Sequence** | **SEQ ID NO.** |
|---|---|
| (Gly Gly Gly Gly Ser)₃ | SEQ ID NO:73 |
| Glu Ser Gly Arg Ser Gly Gly Gly Gly Ser Gly Gly Gly Gly Ser | SEQ ID NO:74 |
| Glu Gly Lys Ser Ser Gly Ser Gly Ser Glu Ser Lys Ser Thr | SEQ ID NO:75 |
| Glu Gly Lys Ser Ser Gly Ser Gly Ser Glu Ser Lys Ser Thr Gln | SEQ ID NO:76 |
| Glu Gly Lys Ser Ser Gly Ser Gly Ser Glu Ser Lys Val Asp | SEQ ID NO:77 |
| Gly Ser Thr Ser Gly Ser Gly Lys Ser Ser Glu Gly Lys Gly | SEQ ID NO:78 |
| Lys Glu Ser Gly Ser Val Ser Ser Glu Gln Leu Ala Gln Phe Arg Ser Leu Asp | SEQ ID NO:79 |
| Glu Ser Gly Ser Val Ser Ser Glu Glu Leu Ala Phe Arg Ser Leu Asp | SEQ ID NO:80 |

In one embodiment, intrabodies are expressed in the cytoplasm. In other embodiments, the intrabodies are localized to various intracellular locations. In such embodiments, specific localization sequences can be attached to the intrabody polypeptide to direct the intrabody to a specific location. Intrabodies can be localized, for example, to the following intracellular locations: endoplasmic reticulum (Munro et al., 1987, Cell 48:899-907; Hangejorden et al., 1991, J. Biol. Chem. 266:6015); nucleus (Lanford et al., 1986, Cell 46:575; Stanton et al.,1986, PNAS 83:1772; Harlow et al., 1985, Mol. Cell Biol. 5:1605; Pap et al., 2002, Exp. Cell Res. 265:288-93); nucleolar region (Seomi et al., 1990, J. Virology 64:1803; Kubota et al., 1989, Biochem. Biophys. Res. Comm. 162:963; Siomi et al., 1998, Cell 55:197); endosomal compartment (Bakke et al.., 1990, Cell 63:707-716); mitochondrial matrix (Pugsley, A. P., 1989, "Protein Targeting", Academic Press, Inc.); Golgi apparatus (Tang et al., 1992, J. Bio. Chem. 267:10122-6); liposomes (Letourneur et al., 1992, Cell 69:1183); peroxisome (Pap et al., 2002, Exp. Cell Res. 265:288-93); trans Golgi network (Pap et al., 2002, Exp. Cell Res. 265:288-93); and plasma membrane (Marchildon et al., 1984, PNAS 81:7679-82; Henderson et al., 1987, PNAS 89:339-43; Rhee et al., 1987, J. Virol. 61:1045-53; Schultz et al., 1984, J. Virol. 133:431-7; Ootsuyama et al., 1985, Jpn. J. Can. Res. 76:1132-5; Ratner et al., 1985, Nature 313:277-84). Examples of localization signals include, but are not limited to, those sequences disclosed in Table 6.

**Table 6**

| **Localization** | **Sequence** | **SEQ ID NO.** |
|---|---|---|
| endoplasmic reticulum | Lys Asp Glu Leu | SEQ ID NO:81 |
| endoplasmic reticulum | Asp Asp Glu Leu | SEQ ID NO:82 |
| endoplasmic reticulum | Asp Glu Glu Leu | SEQ ID NO:83 |
| endoplasmic reticulum | Gln Glu Asp Leu | SEQ ID NO:84 |
| endoplasmic reticulum | Arg Asp Glu Leu | SEQ ID NO:85 |
| Nucleus | Pro Lys Lys Lys Arg Lys Val | SEQ ID NO:86 |
| Nucleus | Pro Gln Lys Lys Ile Lys Ser | SEQ ID NO:87 |
| Nucleus | Gln Pro Lys Lys Pro | SEQ ID NO:88 |
| Nucleus | Arg Lys Lys Arg | SEQ ID NO:89 |
| Nucleus | Lys Lys Lys Arg Lys | SEQ ID NO:90 |
| nucleolar region | Arg Lys Lys Arg Arg Gln Arg Arg Arg Ala His Gln | SEQ ID NO:91 |
| nucleolar region | Arg Gln Ala Arg Arg Asn Arg Arg Arg Arg Trp Arg Glu Arg Gln Arg | SEQ ID NO:92 |
| nucleolar region | Met Pro Leu Thr Arg Arg Arg Pro Ala Ala Ser Gln Ala Leu Ala Pro Pro Thr Pro | SEQ ID NO:93 |
| endosomal compartment | Met Asp Asp Gln Arg Asp Leu Ile Ser Asn Asn Glu Gln Leu Pro | SEQ ID NO:94 |
| mitochondrial matrix | Met Leu Phe Asn Leu Arg Xaa Xaa Leu Asn Asn Ala Ala Phe Arg His Gly His Asn Phe Met Val Arg Asn Phe Arg Cys Gly Gln Pro Leu Xaa | SEQ ID NO:95 |
| Peroxisome | Ala Lys Leu | SEQ ID NO:96 |
| trans Golgi network | Ser Asp Tyr Gln Arg Leu | SEQ ID NO:97 |
| plasma membrane | Gly Cys Val Cys Ser Ser Asn Pro | SEQ ID NO:98 |
| plasma membrane | Gly Gln Thr Val Thr Thr Pro Leu | SEQ ID NO:99 |
| plasma membrane | Gly Gln Glu Leu Ser Gln His Glu | SEQ ID NO:100 |
| plasma membrane | Gly Asn Ser Pro Ser Tyr Asn Pro | SEQ ID NO:101 |
| plasma membrane | Gly Val Ser Gly Ser Lys Gly Gln | SEQ ID NO:102 |
| plasma membrane | Gly Gln Thr Ile Thr Thr Pro Leu | SEQ ID NO:103 |
| plasma membrane | Gly Gln Thr Leu Thr Thr Pro Leu | SEQ ID NO:104 |
| plasma membrane | Gly Gln Ile Phe Ser Arg Ser Ala | SEQ ID NO:105 |
| plasma membrane | Gly Gln Ile His Gly Leu Ser Pro | SEQ ID NO:106 |
| plasma membrane | Gly Ala Arg Ala Ser Val Leu Ser | SEQ ID NO:107 |
| plasma membrane | Gly Cys Thr Leu Ser Ala Glu Glu | SEQ ID NO:108 |

V_{H} and V_{L} domains are made up of the immunoglobulin domains that generally have a conserved structural disulfide bond. In embodiments where the intrabodies are expressed in a reducing environment (*e.g*., the cytoplasm), such a structural feature cannot exist. Mutations can be made to the intrabody polypeptide sequence to compensate for the decreased stability of the immunoglobulin structure resulting from the absence of disulfide bond formation. In one embodiment, the V_{H} and/or V_{L} domains of the intrabodies contain one or more point mutations such that their expression is stabilized in reducing environments (see Steipe et al., 1994, J. Mol. Biol. 240:188-92; Wirtz and Steipe, 1999, Protein Science 8:2245-50; Ohage and Steipe, 1999, J. Mol. Biol. 291:1119-28; Ohage et al., 1999, J. Mol Biol. 291:1129-34).

### Intrabody Proteins as Therapeutics

In one embodiment, the recombinantly expressed intrabody protein is administered to a patient. Such an intrabody polypeptide must be intracellular to mediate a prophylactic or therapeutic effect. In this embodiment of the invention, the intrabody polypeptide is associated with a "membrane permeable sequence". Membrane permeable sequences are polypeptides capable of penetrating through the cell membrane from outside of the cell to the interior of the cell. When linked to another polypeptide, membrane permeable sequences can also direct the translocation of that polypeptide across the cell membrane as well.

In one embodiment, the membrane permeable sequence is the hydrophobic region of a signal peptide (see, *e.g*., Hawiger, 1999, Curr. Opin. Chem. Biol. 3:89-94; Hawiger, 1997, Curr. Opin. Immunol. 9:189-94; U.S. Patent Nos. 5,807,746 and 6,043,339, which are incorporated herein by reference in their entireties). The sequence of a membrane permeable sequence can be based on the hydrophobic region of any signal peptide. The signal peptides can be selected, *e.g*., from the SIGPEP database (see *e.g*., von Heijne, 1987, Prot. Seq. Data Anal. 1:41-2; von Heijne and Abrahmsen, 1989, FEBS Lett. 224:439-46). When a specific cell type is to be targeted for insertion of an intrabody polypeptide, the membrane permeable sequence is preferably based on a signal peptide endogenous to that cell type. In another embodiment, the membrane permeable sequence is a viral protein (*e.g*., Herpes Virus Protein VP22) or fragment thereof (see *e.g.,* Phelan et al., 1998, Nat. Biotechnol. 16:440-3). A membrane permeable sequence with the appropriate properties for a particular intrabody and/or a particular target cell type can be determined empirically by assessing the ability of each membrane permeable sequence to direct the translocation of the intrabody across the cell membrane. Examples of membrane permeable sequences include, but are not limited to, those sequences disclosed in Table 7.

**Table 7**

| **Sequence** | **SEQ ID NO.** |
|---|---|
| Ala Ala Val Ala Leu Leu Pro Ala Val Leu Leu Ala Leu Leu Ala Pro | SEQ ID NO: 109 |
| Ala Ala Val Leu Leu Pro Val Leu Leu Ala Ala Pro | SEQ ID NO:110 |
| Val Thr Val Leu Ala Leu Gly Ala Leu Ala Gly Val Gly Val Gly | SEQ ID NO:111 |

In another embodiment, the membrane permeable sequence can be a derivative. In this embodiment, the amino acid sequence of a membrane permeable sequence has been altered by the introduction of amino acid residue substitutions, deletions, additions, and/or modifications. For example, but not by way of limitation, a polypeptide may be modified, *e.g*., by glycosylation, acetylation, pegylation, phosphorylation, amidation, derivatization by known protecting/blocking groups, proteolytic cleavage, linkage to a cellular ligand or other protein, etc. A derivative of a membrane permeable sequence polypeptide may be modified by chemical modifications using techniques known to those of skill in the art, including, but not limited to specific chemical cleavage, acetylation, formylation, metabolic synthesis of tunicamycin, etc. Further, a derivative of a membrane permeable sequence polypeptide may contain one or more non-classical amino acids. In one embodiment, a polypeptide derivative possesses a similar or identical function as an unaltered polypeptide. In another embodiment, a derivative of a membrane permeable sequence polypeptide has an altered activity when compared to an unaltered polypeptide. For example, a derivative membrane permeable sequence polypeptide can translocate through the cell membrane more efficiently or be more resistant to proteolysis.

The membrane permeable sequence can be attached to the intrabody in a number of ways. In one embodiment, the membrane permeable sequence and the intrabody are expressed as a fusion protein. In this embodiment, the nucleic acid encoding the membrane permeable sequence is attached to the nucleic acid encoding the intrabody using standard recombinant DNA techniques (see *e.g*., Rojas et al., 1998, Nat. Biotechnol. 16:370-5). In a further embodiment, there is a nucleic acid sequence encoding a spacer peptide placed in between the nucleic acids encoding the membrane permeable sequence and the intrabody. In another embodiment, the membrane permeable sequence polypeptide is attached to the intrabody polypeptide after each is separately expressed recombinantly (see *e.g*., Zhang et al., 1998, PNAS 95:9184-9). In this embodiment, the polypeptides can be linked by a peptide bond or a non-peptide bond (*e.g*. with a crosslinking reagent such as glutaraldehyde or a thiazolidino linkage see *e.g*., Hawiger, 1999, Curr. Opin. Chem. Biol. 3:89-94) by methods standard in the art.

The administration of the membrane permeable sequence-intrabody polypeptide can be by parenteral administration, *e.g*., by intravenous injection including regional perfusion through a blood vessel supplying the tissues(s) or organ(s) having the target cell(s), or by inhalation of an aerosol, subcutaneous or intramuscular injection, topical administration such as to skin wounds and lesions, direct transfection into, *e.g*., bone marrow cells prepared for transplantation and subsequent transplantation into the subject, and direct transfection into an organ that is subsequently transplanted into the subject. Further administration methods include oral administration, particularly when the complex is encapsulated, or rectal administration, particularly when the complex is in suppository form. A pharmaceutically acceptable carrier includes any material that is not biologically or otherwise undesirable, *i.e*., the material may be administered to an individual along with the selected complex without causing any undesirable biological effects or interacting in a deleterious manner with any of the other components of the pharmaceutical composition in which it is contained.

Conditions for the administration of the membrane permeable sequence-intrabody polypeptide can be readily be determined, given the teachings in the art (see *e.g*., Remington's Pharmaceutical Sciences, 18th Ed., E. W. Martin (ed.), Mack Publishing Co., Easton, Pa. (1990)). If a particular cell type *in vivo* is to be targeted, for example, by regional perfusion of an organ or tumor, cells from the target tissue can be biopsied and optimal dosages for import of the complex into that tissue can be determined *in vitro* to optimize the in vivo dosage, including concentration and time length. Alternatively, culture cells of the same cell type can also be used to optimize the dosage for the target cells *in vivo.*

### Intrabody Gene Therapy as Therapeutic

In another embodiment, a polynucleotide encoding an intrabody is administered to a patient (*e.g*., as in gene therapy). In this embodiment, methods as described in Section 6.4.1 can be used to administer the polynucleotide of the invention.

### 5.1.1.1.2 Antibody Conjugates

The present invention encompasses the use of Eph/Ephrin Modulators (*e.g*., Eph receptor (*e.g*., EphA1 EphA2, EphA3, EphA4, EphA5, EphA6, EphA7, EphA8, EphA10, EphB1 EphB2, EphB3, EphB4, EphB5 or EphB6) and/or Ephrin (*e.g*., EphrinA1 EphrinA2, EphrinA3, EphrinA4, EphrinA5, EphrinB1 EphrinB2 or EphrinB3) antibodies or fragments thereof that specifically bind to an Eph receptor and/or an Ephrin) that are recombinantly fused or chemically conjugated (including both covalent and non-covalent conjugations) to a heterologous protein or polypeptide (or fragment thereof, *e.g*., to a polypeptide of at least 10, at least 20, at least 30, at least 40, at least 50, at least 60, at least 70, at least 80, at least 90 or at least 100 amino acids) to generate fusion proteins. For example, antibodies may be used to target heterologous polypeptides to particular cell types, either *in vitro* or *in vivo,* by fusing or conjugating the antibodies to antibodies specific for particular cell surface receptors. Antibodies fused or conjugated to heterologous polypeptides may also be used in *in vitro* immunoassays and purification methods using methods known in the art. See *e.g.*, International Publication WO 93/21232; EP 439,095; Naramura et al., 1994, Immunol. Lett. 39:91-99; U.S. Patent 5,474,981; Gillies et al., 1992, PNAS 89:1428-1432; and Fell et al., 1991, J. Immunol. 146:2446-2452, which are incorporated by reference in their entireties. In specific embodiments, the disorder to be prevented, treated, managed or ameliorated by the methods of the present invention is a disorder associated with with aberrant (*i.e.*, increased, decreased or inappropriate) Eph receptor and/or Ephrin expression.

The present invention further includes compositions comprising heterologous polypeptides fused or conjugated to antibody fragments. For example, the heterologous polypeptides may be fused or conjugated to a Fab fragment, Fd fragment, Fv fragment, F(ab)₂ fragment, or portion thereof. Methods for fusing or conjugating proteins, polypeptides, or peptides to an antibody or an antibody fragment are known in the art. See, *e.g*., U.S. Patent Nos. 5,336,603, 5,622,929, 5,359,046, 5,349,053, 5,447,851, and 5,112,946; European Patent Nos. EP 307,434 and EP 367,166; International Publication Nos. WO 96/04388 and WO 91/06570; Ashkenazi et al., 1991, Proc. Natl. Acad. Sci. USA 88: 10535-10539; Zheng et al., 1995, J. Immunol. 154:5590-5600; and Vil et al., 1992, Proc. Natl. Acad. Sci. USA 89:11337- 11341 (said references are incorporated herein by reference in their entireties).

Additional fusion proteins, *e.g*., of any of the Eph/Ephrin Modulators of the invention, may be generated through the techniques of gene-shuffling, motif-shuffling, exon-shuffling, and/or codon-shuffling (collectively referred to as "DNA shuffling"). DNA shuffling may be employed to alter the activities of antibodies of the invention or fragments thereof (*e.g*., antibodies or fragments thereof with higher affinities and lower dissociation rates). See, generally, U.S. Patent Nos. 5,605,793; 5,811,238; 5,830,721; 5,834,252; and 5,837,458, and Patten et al., 1997, Curr. Opinion Biotechnol. 8:724-33; Harayama, 1998, Trends Biotechnol. 16:76; Hansson, et al., 1999, J. Mol. Biol. 287:265; and Lorenzo and Blasco, 1998, BioTechniques 24:308 (each of these patents and publications are hereby incorporated by reference in its entirety). Antibodies or fragments thereof, or the encoded antibodies or fragments thereof, may be altered by being subjected to random mutagenesis by error-prone PCR, random nucleotide insertion or other methods prior to recombination. One or more portions of a polynucleotide encoding an antibody or antibody fragment, which portions specifically bind to an Eph receptor (*e.g*., EphA1 EphA2, EphA3, EphA4, EphA5, EphA6, EphA7, EphA8, EphA10, EphB1, EphB2, EphB3, EphB4, EphB5 or EphB6) and/or Ephrin (*e.g*., EphrinA1 EphrinA2, EphrinA3, EphrinA4, EphrinA5, EphrinB1 EphrinB2 or EphrinB3) may be recombined with one or more components, motifs, sections, parts, domains, fragments, etc. of one or more heterologous molecules.

Moreover, the antibodies or fragments thereof can be fused to marker sequences, such as a peptide to facilitate purification. In certain embodiments, the marker amino acid sequence is a hexa-histidine peptide, such as the tag provided in a pQE vector (QIAGEN, Inc., Chatsworth, CA), among others, many of which are commercially available. As described in Gentz et al., 1989, PNAS 86:821, for instance, hexa-histidine provides for convenient purification of the fusion protein. Other peptide tags useful for purification include, but are not limited to, the hemagglutinin "HA" tag, which corresponds to an epitope derived from the influenza hemagglutinin protein (Wilson et al., 1984, Cell 37:767) and the "flag" tag.

In other embodiments, antibodies of the present invention or fragments or variants thereof are conjugated to a diagnostic or detectable agent. Such antibodies can be useful for monitoring or prognosing the development or progression of a cancer as part of a clinical testing procedure, such as determining the efficacy of a particular therapy. Additionally, such antibodies can be useful for monitoring or prognosing the development or progression of a disorder associated with aberrant (*i.e.*, increased, decreased or inappropriate) Eph receptor and/or Ephrin expression. In a specific embodiment, an Eph receptor (*e.g*., EphA1 EphA2, EphA3, EphA4, EphA5, EphA6, EphA7, EpbA8, EphA10, EphB1 EphB2, EphB3, EphB4, EphB5 or EphB6) antibody or an Ephrin (*e.g*., EphrinA1 EphrinA2, EphrinA3, EphrinA4, EphrinA5, EphrinB1, EphrinB2 or EphrinB3) antibody of the invention is conjugated to a diagnostic or detectable agent.

Such diagnosis and detection can accomplished by coupling the antibody to detectable substances including, but not limited to various enzymes, such as but not limited to horseradish peroxidase, alkaline phosphatase, beta-galactosidase, or acetylcholinesterase; prosthetic groups, such as but not limited to streptavidin/biotin and avidin/biotin; fluorescent materials, such as but not limited to, umbelliferone, fluorescein, fluorescein isothiocynate, rhodamine, dichlorotriazinylamine fluorescein, dansyl chloride or phycoerythrin; luminescent materials, such as but not limited to, luminol; bioluminescent materials, such as but not limited to, luciferase, luciferin, and aequorin; radioactive materials, such as but not limited to, bismuth (²¹³Bi), carbon (¹⁴C), chromium (⁵¹Cr), cobalt (⁵⁷Co), fluorine (¹⁸F), gadolinium (¹⁵³Gd, ¹⁵⁹Gd), gallium (⁶⁸Ga, ⁶⁷Ga), germanium (⁶⁸Ge), holmium (¹⁶⁶Ho), indium (¹¹⁵In, ¹¹³In, ¹¹²In, ¹¹¹In), iodine (¹³I, ¹²⁵I, ¹²³I, ¹²¹I), lanthanium (¹⁴⁰La), lutetium (¹⁷⁷Lu), manganese (⁵⁴Mn), molybdenum (⁹⁹Mo), palladium (¹⁰³Pd), phosphorous (³²P), praseodymium (¹⁴²Pr), promethium (¹⁴⁹Pm), rhenium (¹⁸⁶Re, ¹⁸⁸Re), rhodium (¹⁰⁵Rh), ruthemium (⁹⁷Ru), samarium (¹⁵³Sm), scandium (⁴⁷Sc), selenium (⁷⁵Se), strontium (⁸⁵Sr), sulfur (³⁵S), technetium (⁹⁹Tc), thallium (²⁰¹Ti), tin (¹¹³Sn, ¹¹⁷Sn), tritium (³H), xenon (¹³³Xe), ytterbium (¹⁶⁹Yb, ¹⁷⁵Yb), yttrium (⁹⁰Y), zinc (⁶⁵Zn); positron emitting metals using various positron emission tomographies, and nonradioactive paramagnetic metal ions.

The present invention further encompasses uses of antibodies or fragments thereof conjugated to a prophylactic or therapeutic agent. Nonlimiting examples of these conjugates are disclosed in U.S. Provisional Application 60/714,362, filed September 7, 2005, U.S. Patent Application Publication No. US2005/0180972 A1, and U.S. Patent Application Publication No. US2005/0123536 A1, each of which is hereby incorporated by reference in its entirety herein. An antibody or fragment thereof may be conjugated to a therapeutic moiety such as a cytotoxin, *e.g*., a cytostatic or cytocidal agent, a therapeutic agent or a radioactive metal ion, *e.g*., alpha-emitters. A cytotoxin or cytotoxic agent includes any agent that is detrimental to cells. Therapeutic moieties include, but are not limited to, antimetabolites (*e.g*., methotrexate, 6-mercaptopurine, 6-thioguanine, cytarabine, 5-fluorouracil decarbazine); alkylating agents (*e.g*., mechlorethamine, thioepa chlorambucil, melphalan, carmustine (BCNU) and lomustine (CCNU), cyclothosphamide, busulfan, dibromomannitol, streptozotocin, mitomycin C, and cisdichlorodiamine platinum (II) (DDP), and cisplatin); anthracyclines (*e.g*., daunorubicin (formerly daunomycin) and doxorubicin); antibiotics (*e.g*., dactinomycin (formerly actinomycin), bleomycin, mithramycin, and anthramycin (AMC)); Auristatin molecules (*e.g*., auristatin E, auristatin F, auristatin PHE, MMAE, MMAF, bryostatin 1, and solastatin 10; see Woyke et al., Antimicrob. Agents Chemother. 46:3802-8 (2002), Woyke et al., Antimicrob. Agents Chemother. 45:3580-4 (2001), Mohammad et al., Anticancer Drugs 12:735-40 (2001), Wall et al., Biochem. Biophys. Res. Commun. 266:76-80 (1999), Mohammad et al., Int. J. Oncol. 15:367-72 (1999), all of which are incorporated herein by reference); hormones (*e.g*., glucocorticoids, progestins, androgens, and estrogens), DNA-repair enzyme inhibitors (*e.g*., etoposide or topotecan), kinase inhibitors (*e.g*., compound ST1571, imatinib mesylate (Kantarjian et al., Clin Cancer Res. 8(7):2167-76 (2002)); cytotoxic agents (*e.g*., paclitaxel, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucorticoids, procaine, tetracaine, lidocaine, propranolol, and puromycin and analogs or homologs thereof and those compounds disclosed in U.S. Pat. Nos. 6,245,759, 6,399,633, 6,383,790, 6,335,156, 6,271,242, 6,242,196, 6,218,410, 6,218,372, 6,057,300, 6,034,053, 5,985,877, 5,958,769, 5,925,376, 5,922,844, 5,911,995, 5,872,223, 5,863,904, 5,840,745, 5,728,868, 5,648,239, 5,587,459); farnesyl transferase inhibitors (*e.g*., R115777, BMS-214662, and those disclosed by, for example, U.S. Patent Nos: 6,458,935, 6,451,812, 6,440,974, 6,436,960, 6,432,959, 6,420,387, 6,414,145, 6,410,541, 6,410,539, 6,403,581, 6,399,615, 6,387,905, 6,372,747, 6,369,034, 6,362,188, 6,342,765, 6,342,487, 6,300,501, 6,268,363, 6,265,422, 6,248,756, 6,239,140, 6,232,338, 6,228,865, 6,228,856, 6,225,322, 6,218,406, 6,211,193, 6,187,786, 6,169,096, 6,159,984, 6,143,766, 6,133,303, 6,127,366, 6,124,465, 6,124,295, 6,103,723, 6,093,737, 6,090,948, 6,080,870, 6,077,853, 6,071,935, 6,066,738, 6,063,930, 6,054,466, 6,051,582, 6,051,574, and 6,040,305); topoisomerase inhibitors (*e.g*., camptothecin; irinotecan; SN-38; topotecan; 9-aminocamptothecin; GG-211 (GI 147211); DX-8951f; IST-622; rubitecan; pyrazoloacridine; XR-5000; saintopin; UCE6; UCE1022; TAN-1518A; TAN-1518B; KT6006; KT6528; ED-110; NB-506; ED-110; NB-506; and rebeccamycin); bulgarein; DNA minor groove binders such as Hoescht dye 33342 and Hoechst dye 33258; nitidine; fagaronine; epiberberine; coralyne; beta-lapachone; BC-4-1; bisphosphonates (*e.g*., alendronate, cimadronte, clodronate, tiludronate, etidronate, ibandronate, neridronate, olpandronate, risedronate, piridronate, pamidronate, zolendronate) HMG-CoA reductase inhibitors, (*e.g*., lovastatin, simvastatin, atorvastatin, pravastatin, fluvastatin, statin, cerivastatin, lescol, lupitor, rosuvastatin and atorvastatin); antisense oligonucleotides (*e.g*., those disclosed in the U.S. Pat. Nos. 6,277,832, 5,998,596, 5,885,834, 5,734,033, and 5,618,709); adenosine deaminase inhibitors (*e.g*., Fludarabine phosphate and 2-Chlorodeoxyadenosine); ibritumomab tiuxetan (Zevalin®); tositumomab (Bexxar®)) and pharmaceutically acceptable salts, solvates, clathrates, and prodrugs thereof. In a specific embodiment, the prophylactic or therapeutic agent to be conjugated to an Eph/Ephrin Modulator of the invention is not cytotoxic to a target cell (*e.g*., an Eph receptor- or Ephrin-expressing cell).

Moreover, an antibody can be conjugated to therapeutic moieties such as a radioactive materials or macrocyclic chelators useful for conjugating radiometal ions (see above for examples of radioactive materials). In certain embodiments, the macrocyclic chelator is 1,4,7,10-tetraazacyclododecane-N, N', N", N"-tetraacetic acid (DOTA) which can be attached to the antibody via a linker molecule. Such linker molecules are commonly known in the art and described in Denardo et al., 1998, Clin Cancer Res. 4:2483-90; Peterson et al., 1999, Bioconjug. Chem. 10:553; and Zimmerman et al., 1999, Nucl. Med. Biol. 26:943-50 each incorporated by reference in their entireties.

Further, an antibody or fragment thereof may be conjugated to a prophylactic or therapeutic moiety or drug moiety that modifies a given biological response. Therapeutic moieties or drug moieties are not to be construed as limited to classical chemical therapeutic agents. For example, the drug moiety may be a protein, peptide, or polypeptide possessing a desired biological activity. Such proteins may include, for example, a toxin such as abrin, ricin A, pseudomonas exotoxin, cholera toxin, or diphtheria toxin; a protein such as tumor necrosis factor, α-interferon, β-interferon, nerve growth factor, platelet derived growth factor, tissue plasminogen activator, an apoptotic agent, *e.g*., TNF-α, TNF-β, AIM I (see, International Publication No. WO 97/33899), AIM II (see, International Publication No. WO 97/34911), Fas Ligand (Takahashi et al., 1994, J. Immunol., 6:1567-1574), and VEGF (see, International Publication No. WO 99/23105), an anti-angiogenic agent, *e.g*., angiostatin, endostatin or a component of the coagulation pathway (*e.g*., tissue factor); or, a biological response modifier such as, for example, a lymphokine (*e.g*., interferon gamma ("IFN-γ"), interleukin-1 ("IL-1"), interleukin-2 ("IL-2"), interleukin-5 ("IL-5"), interleukin-6 ("IL-6"), interleuking-7 ("IL-7"), interleukin-10 ("IL-10"), interleukin-12 ("IL-12"), interleukin-15 ("IL-15"), interleukin-23 ("IL-23"), granulocyte macrophage colony stimulating factor ("GM-CSF"), and granulocyte colony stimulating factor ("G-CSF")), or a growth factor (*e.g*., growth hormone ("GH")), or a coagulation agent (*e.g.*, calcium, vitamin K, tissue factors, such as but not limited to, Hageman factor (factor XII), high-molecular-weight kininogen (HMWK), prekallikrein (PK), coagulation proteins-factors II (prothrombin), factor V, XIIa, VIII, XIIIa, XI, XIa" IX, IXa, X, phospholipid fibrinopeptides A and B from the α and β chains of fibrinogen, fibrin monomer). In a specific embodiment, an antibody that specifically binds to an IL-9 polypeptide is conjugated with a leukotriene antagonist (*e.g*., montelukast, zafirlukast, pranlukast, and zyleuton).

Moreover, an antibody can be conjugated to prophylactic or therapeutic moieties such as a radioactive metal ion, such as alpha-emitters such as ²¹³Bi or macrocyclic chelators useful for conjugating radiometal ions, including but not limited to, ¹³¹In, ¹³¹L, ¹³¹Y, ¹³¹Ho, ¹³¹Sm, to polypeptides or any of those listed *supra.* In certain embodiments, the macrocyclic chelator is 1,4,7,10-tetraazacyclododecane-N, N', N", N"'-tetraacetic acid (DOTA) which can be attached to the antibody via a linker molecule. Such linker molecules are commonly known in the art and described in Denardo et al., 1998, Clin Cancer Res. 4(10):2483-90; Peterson et al., 1999, Bioconjug. Chem. 10(4):553-7; and Zimmerman et al., 1999, Nucl. Med. Biol. 26(8):943-50, each incorporated by reference in their entireties.

In another embodiment, antibodies can be fused or conjugated to liposomes, wherein the liposomes are used to encapsulate prophylactic or therapeutic agents (see *e.g.,* Park et al., 1997, Can. Lett. 118:153-160; Lopes de Menezes et al., 1998, Can. Res. 58:3320-30; Tseng et al., 1999, Int. J. Can. 80:723-30; Crosasso et al., 1997, J. Pharm. Sci. 86:832-9). In a further embodiment, the pharmokinetics and clearance of liposomes are improved by incorporating lipid derivatives of PEG into liposome formulations (see, *e.g*., Allen et al., 1991, Biochem Biophys Acta 1068:133-41; Huwyler et al., 1997, J. Pharmacol. Exp. Ther. 282:1541-6).

Techniques for conjugating prophylactic or therapeutic moieties to antibodies are well known. Moieties can be conjugated to antibodies by any method known in the art, including, but not limited to aldehyde/Schiff linkage, sulphydryl linkage, acid-labile linkage, cisaconityl linkage, hydrazone linkage, enzymatically degradable linkage (see generally Garnett, 2002, Adv. Drug Deliv. Rev. 53:171-216). Additional techniques for conjugating prophylactic or therapeutic moieties to antibodies are well known, see, *e.g*., Arnon et al., "Monoclonal Antibodies For Immunotargeting Of Drugs In Cancer Therapy," in Monoclonal Antibodies And Cancer Therapy, Reisfeld et al. (eds.), pp. 243-56 (Alan R. Liss, Inc. 1985); Hellstrom et al., "Antibodies For Drug Delivery," in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review," in Monoclonal Antibodies '84: Biological And Clinical Applications, Pinchera et al. (eds.), pp. 475-506 (1985*);* "Analysis, Results, And Future Prospective Of The Therapeutic Use Of Radiolabeled Antibody In Cancer Therapy," in Monoclonal Antibodies For Cancer Detection And Therapy, Baldwin et al. (eds.), pp. 303-16 (Academic Press 1985), and Thorpe et al., 1982, Immunol. Rev. 62:119-58. Methods for fusing or conjugating antibodies to polypeptide moieties are known in the art. See, e.g., U.S. Patent Nos. 5,336,603, 5,622,929, 5,359,046, 5,349,053, 5,447,851, and 5,112,946; EP 307,434; EP 367,166; International Publication Nos. WO 96/04388 and WO 91/06570; Ashkenazi et al., 1991, PNAS 88: 10535-10539; Zheng et al., 1995, J. Immunol. 154:5590-5600; and Vil et al., 1992, PNAS 89:11337- 11341. The fusion of an antibody to a moiety does not necessarily need to be direct, but may occur through linker sequences. Such linker molecules are commonly known in the art and described in Denardo et al., 1998, Clin Cancer Res. 4:2483-90; Peterson et al., 1999, Bioconjug. Chem. 10:553; Zimmerman et al., 1999, Nucl. Med. Biol. 26:943-50; Garnett, 2002, Adv. Drug Deliv. Rev. 53:171-216, each of which is incorporated herein by reference in its entirety.

A conjugated agent's relative efficacy in comparison to the free agent can depend on a number of factors. For example, rate of uptake of the antibody-agent into the cell (*e.g*., by endocytosis), rate/efficiency of release of the agent from the antibody, rate of export of the agent from the cell, etc. can all effect the action of the agent. Antibodies used for targeted delivery of agents can be assayed for the ability to be endocytosed by the relevant cell type (*i.e.,* the cell type associated with the disorder to be treated) by any method known in the art. Additionally, the type of linkage used to conjugate an agent to an antibody should be assayed by any method known in the art such that the agent action within the target cell is not impeded.

Alternatively, an antibody can be conjugated to a second antibody to form an antibody heteroconjugate as described by Segal in U.S. Patent No. 4,676,980, which is incorporated herein by reference in its entirety.

The prophylactic or therapeutic moiety or drug conjugated to an Eph/Ephrin Modulator of the invention (*e.g*., an Eph receptor or Ephrin antibody that specifically binds to an Eph receptor or an Ephrin polypeptide or fragment thereof, respectively) should be chosen to achieve the desired prophylactic or therapeutic effect(s) for the treatment, management or prevention of a disorder associated with aberrant (*i.e*., increased, decreased or inappropriate) Eph receptor and/or Ephrin expression. A clinician or other medical personnel should consider the following when deciding on which therapeutic moiety or drug to conjugate to an antibody that specifically binds to an Eph receptor or an Ephrin polypeptide or fragment thereof the nature of the disease, the severity of the disease, and the condition of the subject.

Antibodies may also be attached to solid supports, which are particularly useful for immunoassays or purification of the target antigen. Such solid supports include, but are not limited to, glass, cellulose, polyacrylamide, nylon, polystyrene, polyvinyl chloride or polypropylene.

Alternatively, any of the methods described above may be used to generate Eph/Ephrin Modulators that are Eph receptor and/or Ephrin fusion proteins (see Section 6.1.2, *infra*).

### 5.1.1.1.3 Multispecific Antibodies

In specific embodiments of the invention, provided are multispecific antibodies that can simultaneously bind one, or more preferably, two, three, four or five Eph receptors (*e.g*., EphA1, EphA2, EphA3, EphA4, EphA5, EphA6, EphA7, EphA2, EphA10 EphB1, EphB2, EphB3, EphB4, EphB5 or EphB6), and/or Ephrins (*e.g*., EphrinA1 EphrinA2, EphrinA3, EphrinA4, EphrinA5, EphrinB1, EphrinB2 or EphrinB3) to agonize or antagonize Eph receptor and/or Ephrin expression (*e.g*., at the transcriptional, post-transcriptional, translational or post-translation level) and/or activity. In specific embodiments, proteins of the invention can be conjugated to one or more heterologous proteins or peptides (or fragment thereof, *e.g*., to a polypeptide of at least 10, at least 20, at least 30, at least 40, at least 50, at least 60, at least 70, at least 90 or at least 100 amino acids) to generate conjugated proteins. In particular, the invention provides protein conjugates comprising an antigen-binding fragment of an antibody described herein (*e.g*., a Fab fragment, Fd fragment, Fv fragment, scFv, F(ab)₂ fragment, a V_{H} domain, a V_{H} CDR, a V_{L} domain or a V_{L} CDR) and one ore more heterologous proteins, polypeptide or peptides. Methods for conjugating proteins, polypeptides, or peptides to an antibody or an antibody fragment are known in the art. See, *e.g*., U.S. Patent Nos. 5,336,603, 5,622,929, 5,359,046, 5,349,053, 5,447,851, and 5,112,946; European Patent Nos. EP 307,434 and EP 367,166; International Publication Nos. WO 96/04388 and WO 91/06570; Ashkenazi et al., 1991, Proc. Natl. Acad. Sci. USA 88: 10535-10539; Zheng et al., 1995, J. Immunol. 154:5590-5600; and Vil et al., 1992, Proc. Natl. Acad. Sci. USA 89:11337-11341 (said references are incorporated by reference herein in their entireties).

### 5.1.1.1.4 BiTE Molecules

In a specific embodiment, antibodies for use in the methods of the invention are bispecific T cell engagers (BiTEs). Bispecific T cell engagers (BiTE) are bispecific antibodies that can redirect T cells for antigen-specific elimination of targets. A BiTE molecule has an antigen-binding domain that binds to a T cell antigen (*e.g*. CD3) at one end of the molecule and an antigen binding domain that will bind to an antigen on the target cell. A BiTE molecule was recently described in WO 99/54440, which is herein incorporated by reference. This publication describes a novel single-chain multifunctional polypeptide that comprises binding sites for the CD19 and CD3 antigens (CD19xCD3). This molecule was derived from two antibodies, one that binds to CD19 on the B cell and an antibody that binds to CD3 on the T cells. The variable regions of these different antibodies are linked by a polypeptide sequence, thus creating a single molecule. Also described, is the linking of the heavy chain (V_{H}) and light chain (V_{L}) variable domains with a flexible linker to create a single chain, bispecific antibody.

In an embodiment of this invention, an antibody or ligand that specifically binds a polypeptide of interest (*e.g*., an Eph receptor and/or an Ephrin) will comprise a portion of the BiTE molecule. For example, the V_{H} and/or V_{L} (*e.g*. a scFV) of an antibody that binds a polypeptide of interest (*e.g*., an Eph receptor and/or an Ephrin) can be fused to an anti-CD3 binding portion such as that of the molecule described above, thus creating a BiTE molecule that targets the polypeptide of interest (*e.g*., an Eph receptor and/or an Ephrin). In addition to the heavy and/or light chain variable domains of antibody against a polypeptide of interest (*e.g*., an Eph receptor and/or an Ephrin), other molecules that bind the polypeptide of interest (*e.g*., an Eph receptor and/or an Ephrin) can comprise the BiTE molecule, for example receptors (*e.g*., an Eph receptor and/or an Ephrin). In another embodiment, the BiTE molecule can comprise a molecule that binds to other T cell antigens (other than CD3). For example, ligands and/or antibodies that specifically bind to T-cell antigens like CD2, CD4, CD8, CD11a, TCR, and CD28 are contemplated to be part of this invention. This list is not meant to be exhaustive but only to illustrate that other molecules that can specifically bind to a T cell antigen can be used as part of a BiTE molecule. These molecules can include the VH and/or VL portions of the antibody or natural ligands (for example LFA3 whose natural ligand is CD3).

### 5.1.1.1.5 Methods of Producing Antibodies

The antibodies that specifically bind to an antigen can be produced by any method known in the art for the synthesis of antibodies, in particular, by chemical synthesis or preferably, by recombinant expression techniques.

Polyclonal antibodies specific for an antigen can be produced by various procedures well-known in the art. For example, a human antigen can be administered to various host animals including, but not limited to, rabbits, mice, rats, etc. to induce the production of sera containing polyclonal antibodies specific for the human antigen. Various adjuvants may be used to increase the immunological response, depending on the host species, and include but are not limited to, Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (bacille Calmette-Guerin) and corynebacterium parvum. Such adjuvants are also well known in the art.

Monoclonal antibodies can be prepared using a wide variety of techniques known in the art including the use of hybridoma, recombinant, and phage display technologies, or a combination thereof For example, monoclonal antibodies can be produced using hybridoma techniques including those known in the art and taught, for example, in Harlow et al., Antibodies: A Laboratory Manual, (Cold Spring Harbor Laboratory Press, 2nd ed. 1988); Hammerling, et al., in: Monoclonal Antibodies and T Cell Hybridomas 563 681 (Elsevier, N.Y., 1981) (said references incorporated by reference in their entireties). The term "monoclonal antibody" as used herein is not limited to antibodies produced through hybridoma technology. The term "monoclonal antibody" refers to an antibody that is derived from a single clone, including any eukaryotic, prokaryotic, or phage clone, and not the method by which it is produced.

Methods for producing and screening for specific antibodies using hybridoma technology are routine and well known in the art. Briefly, mice can be immunized with a non-murine antigen and once an immune response is detected, *e.g*., antibodies specific for the antigen are detected in the mouse serum, the mouse spleen is harvested and splenocytes isolated. The splenocytes are then fused by well known techniques to any suitable myeloma cells, for example cells from cell line SP20 available from the ATCC. Hybridomas are selected and cloned by limited dilution. The hybridoma clones are then assayed by methods known in the art for cells that secrete antibodies capable of binding a polypeptide of the invention. Ascites fluid, which generally contains high levels of antibodies, can be generated by immunizing mice with positive hybridoma clones.

The present invention provides methods of generating monoclonal antibodies as well as antibodies produced by the method comprising culturing a hybridoma cell secreting an antibody of the invention wherein, the hybridoma is generated by fusing splenocytes isolated from a mouse immunized with a non-murine antigen with myeloma cells and then screening the hybridomas resulting from the fusion for hybridoma clones that secrete an antibody able to bind to the antigen.

Antibody fragments which recognize specific particular epitopes may be generated by any technique known to those of skill in the art. For example, Fab and F(ab')2 fragments of the invention may be produced by proteolytic cleavage of immunoglobulin molecules, using enzymes such as papain (to produce Fab fragments) or pepsin (to produce F(ab')2 fragments). F(ab')2 fragments contain the variable region, the light chain constant region and the CH1 domain of the heavy chain. Further, the antibodies of the present invention can also be generated using various phage display methods known in the art.

Antibody engineering to generate monoclonal antibodies with altered characteristics (*e.g*. affinity) is well known in the art (*see,* for example, McCarthy et al., 2001, J Imm Meth. 251:137-149, Beiboer et al., 2000, J Mol Biol., 296:833-849, Mohan et al., 2003, Biophys J., 85:3221-3236, Diamond et al., 1984, PNAS, 81:5841-5844, Sinha et al., 2002, Biophys J., 83:2946-2968, Davies and Cohen, 1996, PNAS, 93:7-12, Cauerhff et al, 2004, PNAS, 101(10):3539-3544, Vasudevan et al., 2004, Blood Cells Mol Dis. 32:176-181, and Zebedee et al., 1992, PNAS, 89:3175-3179). The present invention describes methods that allow one skilled in the art to tailor the specificity of a given antibody (*e.g*. a pan-specific antibody) towards different members within a receptor tyrosine kinase family using a library based affinity maturation approach. Examples of the families or classes, and members of the families or classes of the receptor tyrosine kinase family are disclosed in Figures 19A-D of the present invention. It can be envisioned that the specificity of a given antibody is tailored to meet a desired therapeutic or diagnostic need. In one embodiment of the present invention, provided is a method of tailoring the specificity of a pan-specific antibody that specifically binds at least two members within a receptor tyrosine kinase family, said method comprising randomizing each amino acid of the CDRs of said pan-specific antibody; generating a library comprising said randomized CDRs; screening said library for clones with increased or decreased affinity for a desired receptor(s).

In one embodiment, the pan-specificity is to a member of the receptor tyrosine kinase family of receptors. In a specific embodiment, the receptor tyrosine kinase family is selected from the group consisting of class I, class II, class III, class IV, class V, class VI, class VII, class VIII, class IX, class X, class XI, class XII, class XIII, class XIV, class XV, class XVI, class XVII, class XVIII, and class XIX members of the receptor tyrosine kinase classes. In a further specific embodiment, the pan-specificity is to a member of the receptor tyrosine kinase family, wherein the member is the Eph family of receptors. In another specific embodiment, the library is a eukaryotic library. In a further specific embodiment, the library is a prokaryotic library. In a specific embodiment, the library is an expression library. In another specific embodiment, the library is a display library. In yet another specific embodiment, the library is a phage display library. In a further specific embodiment, the pan-specificity is to a member of the EphA family of receptors. In yet a further specific embodiment, the pan-specificity is to a member of the EphB family of receptors.

In one embodiment, the pan-specific antibody specifically binds at least two members of the Eph receptor family. In another embodiment, the pan-specific antibody specifically binds at least three members of the Eph receptor family. In a further embodiment, the pan-specific antibody specifically binds at least four members of the Eph receptor family. In yet a further embodiment, the pan-specific antibody specifically binds at least five members of the Eph receptor family. In another embodiment, the pan-specific antibody specifically binds at least six members of the Eph receptor family. In a further embodiment, the pan-specific antibody specifically binds at least seven members of the Eph receptor family. In another embodiment, the pan-specific antibody specifically binds at least eight members of the Eph receptor family. In yet another embodiment, the pan-specific antibody specifically binds at least nine members of the Eph receptor family. In a further embodiment, the pan-specific antibody specifically binds at least ten members of the Eph receptor family. In another embodiment, the pan-specific antibody specifically binds at least eleven members of the Eph receptor family. In a further embodiment, the pan-specific antibody specifically binds at least twelve members of the Eph receptor family. In yet a further embodiment, the pan-specific antibody specifically binds at least thirteen members of the Eph receptor family. In another embodiment, the pan-specific antibody specifically binds at least fourteen members of the Eph receptor family. In yet another embodiment, the pan-specific antibody specifically binds at least fifteen members of the Eph receptor family. In another embodiment, the pan-specific antibody specifically binds at least sixteen members of the Eph receptor family. In a further embodiment, the pan-specific antibody specifically binds all members of the Eph receptor family.

In another embodiment, the pan-specific antibody specifically binds at least one member of the EphA family of receptors. In a further embodiment, the pan-specific antibody specifically binds at least two members of the EphA family of receptors. In yet a further embodiment, the pan-specific antibody specifically binds at least three members of the EphA family of receptors. In another embodiment, the pan-specific antibody specifically binds at least four members of the EphA family of receptors. In a further embodiment, the pan-specific antibody specifically binds at least five members of the EphA family of receptors. In another embodiment, the pan-specific antibody specifically binds at least six members of the EphA family of receptors. In a further embodiment, the pan-specific antibody specifically binds at least seven members of the EphA family of receptors. In yet a further embodiment, the pan-specific antibody specifically binds at least eight members of the EphA family of receptors. In another embodiment, the pan-specific antibody specifically binds at least nine members of the EphA family of receptors. In yet another embodiment, the pan-specific antibody specifically binds all members of the EphA family of receptors.

In a further embodiment, the pan-specific antibody specifically binds at least one member of the EphB family of receptors. In another embodiment, the pan-specific antibody specifically binds at least two members of the EphB family of receptors. In a further embodiment, the pan-specific antibody specifically binds at least three members of the EphB family of receptors. In yet a further embodiment, the pan-specific antibody specifically binds at least four members of the EphB family of receptors. In another embodiment, the pan-specific antibody specifically binds at least five members of the EphB family of receptors. In yet another embodiment, the pan-specific antibody specifically binds at least six members of the EphB family of receptors. In a further embodiment, the pan-specific antibody specifically binds all members of the EphB family of receptors.

As described herein above, several different methods can be used to screen for desired antibody affinity characteristics. In phage display methods, functional antibody domains are displayed on the surface of phage particles which carry the polynucleotide sequences encoding them. In particular, DNA sequences encoding VH and VL domains are amplified from animal cDNA libraries (*e.g*., human or murine cDNA libraries of affected tissues). The DNA encoding the VH and VL domains are recombined together with an scFv linker by PCR and cloned into a phagemid vector. The vector is electroporated in *E*. *coli* and the *E*. *coli* is infected with helper phage. Phage used in these methods are typically filamentous phage including fd and M13 and the VH and VL domains are usually recombinantly fused to either the phage gene III or gene VIII. Phage expressing an antigen binding domain that binds to a particular antigen can be selected or identified with antigen, *e.g*., using labeled antigen or antigen bound or captured to a solid surface or bead. Examples of phage display methods that can be used to make the antibodies of the present invention include those disclosed in Brinkman et al., 1995, J. Immunol. Methods 182:41-50; Ames et al., 1995, J. Immunol. Methods 184:177-186; Kettleborough et al., 1994, Eur. J. Immunol. 24:952-958; Persic et al., 1997, Gene 187:9-18; Burton et al., 1994, Advances in Immunology 57:191-280; International application No. PCT/GB91/O1 134; International publication Nos. WO 90/02809, WO 91/10737, WO 92/01047, WO 92/18619, WO 93/11236, WO 95/15982, WO 95/20401, and WO97/13844; and U.S. Patent Nos. 5,698,426, 5,223,409, 5,403,484, 5,580,717, 5,427,908, 5,750,753, 5,821,047, 5,571,698, 5,427,908, 5,516,637, 5,780,225, 5,658,727, 5,733,743 and 5,969,108; each of which is incorporated herein by reference in its entirety.

As described in the above references, after phage selection, the antibody coding regions from the phage can be isolated and used to generate whole antibodies, including human antibodies, or any other desired antigen binding fragment, and expressed in any desired host, including mammalian cells, insect cells, plant cells, yeast, and bacteria, *e.g*., as described below. Techniques to recombinantly produce Fab, Fab' and F(ab')2 fragments can also be employed using methods known in the art such as those disclosed in PCT publication No. WO 92/22324; Mullinax et al., 1992, BioTechniques 12(6):864-869; Sawai et al., 1995, AJRI 34:26-34; and Better et al., 1988, Science 240:1041-1043 (said references incorporated by reference in their entireties).

To generate whole antibodies, PCR primers including VH or VL nucleotide sequences, a restriction site, and a flanking sequence to protect the restriction site can be used to amplify the VH or VL sequences in scFv clones. Utilizing cloning techniques known to those of skill in the art, the PCR amplified VH domains can be cloned into vectors expressing a VH constant region, *e.g*., the human gamma 4 constant region, and the PCR amplified VL domains can be cloned into vectors expressing a VL constant region, *e.g*., human kappa or lamba constant regions. In one embodiment, the vectors for expressing the VH or VL domains comprise an EF-1α promoter, a secretion signal, a cloning site for the variable domain, constant domains, and a selection marker such as neomycin. The VH and VL domains may also cloned into one vector expressing the necessary constant regions. The heavy chain conversion vectors and light chain conversion vectors are then co-transfected into cell lines to generate stable or transient cell lines that express full-length antibodies, *e.g*., IgG, using techniques known to those of skill in the art.

For some uses, including *in vivo* use of antibodies in humans and *in vitro* detection assays, it may be preferable to use humanized antibodies or chimeric antibodies. Completely human antibodies and humanized antibodies are particularly desirable for therapeutic treatment of human subjects. Human antibodies can be made by a variety of methods known in the art including phage display methods described above using antibody libraries derived from human immunoglobulin sequences. See also U.S. Patent Nos. 4,444,887 and 4,716,111; and International publication Nos. WO 98/46645, WO 98/50433, WO 98/24893, WO98/16654, WO 96/34096, WO 96/33735, and WO 91/10741; each of which is incorporated herein by reference in its entirety.

Human antibodies can also be produced using transgenic mice which are incapable of expressing functional endogenous immunoglobulins, but which can express human immunoglobulin genes. For example, the human heavy and light chain immunoglobulin gene complexes may be introduced randomly or by homologous recombination into mouse embryonic stem cells. Alternatively, the human variable region, constant region, and diversity region may be introduced into mouse embryonic stem cells in addition to the human heavy and light chain genes. The mouse heavy and light chain immunoglobulin genes may be rendered non functional separately or simultaneously with the introduction of human immunoglobulin loci by homologous recombination. In particular, homozygous deletion of the JH region prevents endogenous antibody production. The modified embryonic stem cells are expanded and microinjected into blastocysts to produce chimeric mice. The chimeric mice are then be bred to produce homozygous offspring which express human antibodies. The transgenic mice are immunized in the normal fashion with a selected antigen, *e.g*., all or a portion of a polypeptide of the invention. Monoclonal antibodies directed against the antigen can be obtained from the immunized, transgenic mice using conventional hybridoma technology. The human immunoglobulin transgenes harbored by the transgenic mice rearrange during B cell differentiation, and subsequently undergo class switching and somatic mutation. Thus, using such a technique, it is possible to produce therapeutically useful IgG, IgA, IgM and IgE antibodies. For an overview of this technology for producing human antibodies, see Lonberg and Huszar, 1995, Int. Rev. Immunol. 13:65 93. For a detailed discussion of this technology for producing human antibodies and human monoclonal antibodies and protocols for producing such antibodies, see, *e.g*., International publication Nos. WO 98/24893, WO 96/34096, and WO 96/33735; and U.S. Patent Nos. 5,413,923, 5,625,126, 5,633,425, 5,569,825, 5,661,016, 5,545,806, 5,814,318, and 5,939,598, which are incorporated by reference herein in their entirety. In addition, companies such as Abgenix, Inc. (Freemont, CA) and Genpharm (San Jose, CA) can be engaged to provide human antibodies directed against a selected antigen using technology similar to that described above.

A chimeric antibody is a molecule in which different portions of the antibody are derived from different immunoglobulin molecules. Methods for producing chimeric antibodies are known in the art. See, *e.g*., Morrison, 1985, Science 229:1202; Oi et al., 1986, BioTechniques 4:214; Gillies et al., 1989, J. Immunol. Methods 125:191-202; and U.S. Patent Nos. 5,807,715, 4,816,567, 4,816,397, and 6,311,415, which are incorporated herein by reference in their entireties.

Often, framework residues in the framework regions will be substituted with the corresponding residue from the CDR donor antibody to alter, improve, or decrease antigen binding. These framework substitutions are identified by methods well known in the art, *e.g*., by modeling of the interactions of the CDR and framework residues to identify framework residues important for antigen binding and sequence comparison to identify unusual framework residues at particular positions (see, *e.g*., U.S. Patent No. 5,585,089; and Riechmann et al., 1988, Nature 332:323, which are incorporated herein by reference in their entireties).

A humanized antibody is an antibody or its variant or fragment thereof which is capable of binding to a predetermined antigen and which comprises a framework region having substantially the amino acid sequence of a human immunoglobulin and a CDR having substantially the amino acid sequence of a non-human immuoglobulin. A humanized antibody comprises substantially all of at least one, and typically two, variable domains (Fab, Fab', F(ab')₂, Fabc, Fv) in which all or substantially all of the CDR regions correspond to those of a non human immunoglobulin (*i.e*., donor antibody) and all or substantially all of the framework regions are those of a human immunoglobulin consensus sequence. In one embodiment, a humanized antibody also comprises at least a portion of an immunoglobulin constant region (Fc), typically that of a human immunoglobulin. Ordinarily, the antibody will contain both the light chain as well as at least the variable domain of a heavy chain. The antibody also may include the CH1, hinge, CH2, CH3, and CH4 regions of the heavy chain. The humanized antibody can be selected from any class of immunoglobulins, including IgM, IgG, IgD, IgA and IgE, and any isotype, including IgG1, IgG2, IgG3 and IgG4. Usually the constant domain is a complement fixing constant domain where it is desired that the humanized antibody exhibit cytotoxic activity, and the class is typically IgG 1. Where such cytotoxic activity is not desirable, the constant domain may be of the IgG2 class. The humanized antibody may comprise sequences from more than one class or isotype, and selecting particular constant domains to optimize desired effector functions is within the ordinary skill in the art. The framework and CDR regions of a humanized antibody need not correspond precisely to the parental sequences, *e.g*., the donor CDR or the consensus framework may be mutagenized by substitution, insertion or deletion of at least one residue so that the CDR or framework residue at that site does not correspond to either the consensus or the import antibody. Such mutations, however, will not be extensive. Usually, at least 75% of the humanized antibody residues will correspond to those of the parental framework and CDR sequences, more often 90%, and most preferably greater than 95%. A humanized antibody can be produced using variety of techniques known in the art, including but not limited to, CDR grafting (see *e.g*., European Patent No. EP 239,400; International Publication No. WO 91/09967; and U.S. Patent Nos. 5,225,539, 5,530,101, and 5,585,089, each of which is incorporated herein in its entirety by reference), veneering or resurfacing (see *e.g*., European Patent Nos. EP 592,106 and EP 519,596; Padlan, 1991, Molecular Immunology 28(4/5):489-498; Studnicka et al., 1994, Protein Engineering 7(6):805-814; and Roguska et al., 1994, PNAS 91:969-973, each of which is incorporated herein by its entirety by reference), chain shuffling (see *e.g*., U.S. Patent No. 5,565,332, which is incorporated herein in its entirety by reference), and techniques disclosed in, *e.g*., U.S. Pat. No. 6,407,213, U.S. Pat. No. 5,766,886, International Publication No. WO 9317105, Tan et al., J. Immunol. 169:1119 25 (2002), Caldas et al., Protein Eng. 13(5):353 60 (2000), Morea et al., Methods 20(3):267 79 (2000), Baca et al., J. Biol. Chem. 272(16):10678 84 (1997), Roguska et al., Protein Eng. 9(10):895 904 (1996), Couto et al., Cancer Res. 55 (23 Supp):5973s-5977s (1995), Couto et al., Cancer Res. 55(8):1717 22 (1995), Sandhu JS, Gene 150(2):409 10 (1994), and Pedersen et al., J. Mol. Biol. 235(3):959 73 (1994), each of which is incorporated herein in its entirety by reference. Often, framework residues in the framework regions will be substituted with the corresponding residue from the CDR donor antibody to alter, improve, or decrease antigen binding. These framework substitutions are identified by methods well known in the art, *e.g*., by modeling of the interactions of the CDR and framework residues to identify framework residues important for antigen binding and sequence comparison to identify unusual framework residues at particular positions. (See, *e.g*., Queen *et al.,* U.S. Patent No. 5,585,089; and Riechmann et al., 1988, Nature 332:323, which are incorporated herein by reference in their entireties.)

Further, the antibodies that specifically bind to an Eph receptor (*e.g*., EphA1, EphA2, EphA3, EphA4, EphA5, EphA6, EphA7, EphA8, EphA10, EphB1, EphB2, EphB3, EphB4, EphB5 or EphB6) or an Ephrin (*e.g*., EphrinA1 EphrinA2, EphrinA3, EphrinA4, EphrinA5, EphrinB1, EphrinB2 or EphrinB3) or fragments thereof can, in turn, be utilized to generate anti-idiotype antibodies that "mimic" an antigen using techniques well known to those skilled in the art. (See, *e.g*., Greenspan & Bona, 1989, FASEB J. 7(5):437-444; and Nissinoff, 1991, J. Immunol. 147(8):2429-2438).

### 5.1.1.1.6 Recombinant Expression of An Antibody

Recombinant expression of an antibody of the invention, derivative, analog or fragment thereof, (*e.g*., a heavy or light chain of an antibody of the invention or a portion thereof or a single chain antibody of the invention), requires construction of an expression vector containing a polynucleotide that encodes the antibody. Once a polynucleotide encoding an antibody molecule or a heavy or light chain of an antibody, or portion thereof (for example, but not necessarily, containing the heavy or light chain variable domain), of the invention has been obtained, the vector for the production of the antibody molecule may be produced by recombinant DNA technology using techniques well known in the art. Thus, methods for preparing a protein by expressing a polynucleotide containing an antibody encoding nucleotide sequence are described herein. Methods which are well known to those skilled in the art can be used to construct expression vectors containing antibody coding sequences and appropriate transcriptional and translational control signals. These methods include, for example, *in vitro* recombinant DNA techniques, synthetic techniques, and *in vivo* genetic recombination. The invention, thus, provides replicable vectors comprising a nucleotide sequence encoding an antibody molecule of the invention, a heavy or light chain of an antibody, a heavy or light chain variable domain of an antibody or a portion thereof, or a heavy or light chain CDR, operably linked to a promoter. Such vectors may include the nucleotide sequence encoding the constant region of the antibody molecule (see, *e.g*., International Publication Nos. WO 86/05807 and WO 89/01036; and U.S. Patent No. 5,122,464) and the variable domain of the antibody may be cloned into such a vector for expression of the entire heavy, the entire light chain, or both the entire heavy and light chains.

The expression vector is transferred to a host cell by conventional techniques and the transfected cells are then cultured by conventional techniques to produce an antibody of the invention. Thus, the invention includes host cells containing a polynucleotide encoding an antibody of the invention or fragments thereof, or a heavy or light chain thereof, or portion thereof, or a single chain antibody of the invention, operably linked to a heterologous promoter. In certain embodiments for the expression of double-chained antibodies, vectors encoding both the heavy and light chains may be co-expressed in the host cell for expression of the entire immunoglobulin molecule, as detailed below.

A variety of host-expression vector systems may be utilized to express the antibody molecules of the invention (see, *e.g*., U.S. Patent No. 5,807,715). Such host-expression systems represent vehicles by which the coding sequences of interest may be produced and subsequently purified, but also represent cells which may, when transformed or transfected with the appropriate nucleotide coding sequences, express an antibody molecule of the invention *in situ.* These include but are not limited to microorganisms such as bacteria (*e.g., E. coli* and *B. subtilis*) transformed with recombinant bacteriophage DNA, plasmid DNA or cosmid DNA expression vectors containing antibody coding sequences; yeast (*e.g*., *Saccharomyces Pichia*) transformed with recombinant yeast expression vectors containing antibody coding sequences; insect cell systems infected with recombinant virus expression vectors (*e.g*., baculovirus) containing antibody coding sequences; plant cell systems infected with recombinant virus expression vectors (*e.g*., cauliflower mosaic virus, CaMV; tobacco mosaic virus, TMV) or transformed with recombinant plasmid expression vectors (*e.g*., Ti plasmid) containing antibody coding sequences; or mammalian cell systems (*e.g*., COS, CHO, BHK, 293, NS0, and 3T3 cells) harboring recombinant expression constructs containing promoters derived from the genome of mammalian cells (*e.g*., metallothionein promoter) or from mammalian viruses (*e.g*., the adenovirus late promoter; the vaccinia virus 7.5K promoter). For example, bacterial cells such as *Escherichia coli,* or eukaryotic cells, especially for the expression of whole recombinant antibody molecule, are used for the expression of a recombinant antibody molecule. For example, mammalian cells such as Chinese hamster ovary cells (CHO), in conjunction with a vector such as the major intermediate early gene promoter element from human cytomegalovirus is an effective expression system for antibodies (Foecking et al., 1986, Gene 45:101; and Cockett et al., 1990, BioTechnology 8:2). In a specific embodiment, the expression of nucleotide sequences encoding antibodies or fragments thereof which specifically bind to and agonize is regulated by a constitutive promoter, inducible promoter or tissue specific promoter.

In bacterial systems, a number of expression vectors may be advantageously selected depending upon the use intended for the antibody molecule being expressed. For example, when a large quantity of such a protein is to be produced, for the generation of pharmaceutical compositions of an antibody molecule, vectors which direct the expression of high levels of fusion protein products that are readily purified may be desirable. Such vectors include, but are not limited to, the *E. coli* expression vector pUR278 (Ruther et al., 1983, EMBO 12:1791), in which the antibody coding sequence may be ligated individually into the vector in frame with the lac Z coding region so that a fusion protein is produced; pIN vectors (Inouye & Inouye, 1985, Nucleic Acids Res. 13:3101-3109; Van Heeke & Schuster, 1989, J. Biol. Chem. 24:5503-5509); and the like. pGEX vectors may also be used to express foreign polypeptides as fusion proteins with glutathione 5-transferase (GST). In general, such fusion proteins are soluble and can easily be purified from lysed cells by adsorption and binding to matrix glutathione-agarose beads followed by elution in the presence of free glutathione. The pGEX vectors are designed to include thrombin or factor Xa protease cleavage sites so that the cloned target gene product can be released from the GST moiety.

In an insect system, *Autographa californica* nuclear polyhedrosis virus (AcNPV) is used as a vector to express foreign genes. The virus grows in *Spodoptera frugiperda* cells. The antibody coding sequence may be cloned individually into non-essential regions (for example the polyhedrin gene) of the virus and placed under control of an AcNPV promoter (for example the polyhedrin promoter).

In mammalian host cells, a number of viral-based expression systems may be utilized. In cases where an adenovirus is used as an expression vector, the antibody coding sequence of interest may be ligated to an adenovirus transcription/translation control complex, *e.g*., the late promoter and tripartite leader sequence. This chimeric gene may then be inserted in the adenovirus genome by *in vitro* or *in vivo* recombination. Insertion in a non-essential region of the viral genome (*e.g*., region El or E3) will result in a recombinant virus that is viable and capable of expressing the antibody molecule in infected hosts (*e.g*., see Logan & Shenk, 1984, PNAS 81:3655-3659). Specific initiation signals may also be required for efficient translation of inserted antibody coding sequences. These signals include the ATG initiation codon and adjacent sequences. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (see, *e.g.*, Bittner et al., 1987, Methods in Enzymol. 153:516-544).

In addition, a host cell strain may be chosen which modulates the expression of the inserted sequences, or modifies and processes the gene product in the specific fashion desired. Such modifications (*e.g.,* glycosylation) and processing (*e.g*., cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the gene product may be used. Such mammalian host cells include but are not limited to CHO, VERO, BHK, HeLa, COS, MDCK, 293, 3T3, W138, BT483, Hs578T, HTB2, BT2O, NS1, and T47D, NS0 (a murine myeloma cell line that does not endogenously produce any immunoglobulin chains), CRL7O3O and HsS78Bst cells.

For long-term, high-yield production of recombinant proteins, stable expression is preferred. For example, cell lines which stably express the antibody molecule may be engineered. Rather than using expression vectors which contain viral origins of replication, host cells can be transformed with DNA controlled by appropriate expression control elements (*e.g*., promoter, enhancer, sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express the antibody molecule. Such engineered cell lines may be particularly useful in screening and evaluation of compositions that interact directly or indirectly with the antibody molecule.

A number of selection systems may be used, including but not limited to, the herpes simplex virus thymidine kinase (Wigler et al., 1977, Cell 11:223), glutamine synthase, hypoxanthine guanine phosphoribosyltransferase (Szybalska & Szybalski, 1992, Proc. Natl. Acad. Sci. USA 48:202), and adenine phosphoribosyltransferase (Lowy et al., 1980, Cell 22:8-17) genes can be employed in tk-, gs-, hgprt- or aprt- cells, respectively. Also, antimetabolite resistance can be used as the basis of selection for the following genes: *dhfr,* which confers resistance to methotrexate (Wigler et al., 1980, PNAS 77:357; O'Hare et al., 1981, PNAS 78:1527); *gpt,* which confers resistance to mycophenolic acid (Mulligan & Berg, 1981, PNAS 78:2072); neo, which confers resistance to the aminoglycoside G-418 (Wu and Wu, 1991, Biotherapy 3:87; Tolstoshev, 1993, Ann. Rev. Pharmacol. Toxicol. 32:573; Mulligan, 1993, Science 260:926; and Morgan and Anderson, 1993, Ann. Rev. Biochem. 62: 191; May, 1993, TIB TECH 11:155-); and *hygro,* which confers resistance to hygromycin (Santerre et al., 1984, Gene 30:147). Methods commonly known in the art of recombinant DNA technology may be routinely applied to select the desired recombinant clone, and such methods are described, for example, in Ausubel et al. (eds.), Current Protocols in Molecular Biology, John Wiley & Sons, NY (1993); Kriegler, Gene Transfer and Expression, A Laboratory Manual, Stockton Press, NY (1990); and in Chapters 12 and 13, Dracopoli et al. (eds), Current Protocols in Human Genetics, John Wiley & Sons, NY (1994); Colberre-Garapin et al., 1981, J Mol. Biol. 150:1, which are incorporated by reference herein in their entireties.

The expression levels of an antibody molecule can be increased by vector amplification (for a review, see Bebbington and Hentschel, The use of vectors based on gene amplification for the expression of cloned genes in mammalian cells in DNA cloning, Vol.3. (Academic Press, New York, 1987)). When a marker in the vector system expressing antibody is amplifiable, increase in the level of inhibitor present in culture of host cell will increase the number of copies of the marker gene. Since the amplified region is associated with the antibody gene, production of the antibody will also increase (Crouse et al., 1983, Mol. Cell. Biol. 3:257).

The host cell may be co-transfected with two expression vectors of the invention, the first vector encoding a heavy chain derived polypeptide and the second vector encoding a light chain derived polypeptide. The two vectors may contain identical selectable markers which enable equal expression of heavy and light chain polypeptides. Alternatively, a single vector may be used which encodes, and is capable of expressing, both heavy and light chain polypeptides. In such situations, the light chain should be placed before the heavy chain to avoid an excess of toxic free heavy chain (Proudfoot, 1986, Nature 322:52; and Kohler, 1980, PNAS 77:2197). The coding sequences for the heavy and light chains may comprise cDNA or genomic DNA.

Once an antibody molecule of the invention has been produced by recombinant expression, it may be purified by any method known in the art for purification of an immunoglobulin molecule, for example, by chromatography (*e.g*., ion exchange, affinity, particularly by affinity for the specific antigen after Protein A, and sizing column chromatography), centrifugation, differential solubility, or by any other standard technique for the purification of proteins. Further, the antibodies of the present invention or fragments thereof may be fused to heterologous polypeptide sequences described herein or otherwise known in the art to facilitate purification.

### 5.1.2 Avimers As Eph/Ephrin Modulators

In one embodiment, an Eph/Epbrin Modulator of the invention is an Avimer (Avidia, Inc.). Avimers are a new class of therapeutic proteins that are from human origan, are unrelated to antibodies and antibody fragments, are composed of several modular and reusable binding domains, have individual binding domains with a molecular weight of 4.5kD each, have a molecular weight that typically ranges between 9kD and 18kD, can be designed to exert either antagonistic or agonistic activity, can bind with high affinity to multiple epitopes simultaneously, have sub-nM binding affinity (Kd) and blocking function (IC50), are non-glycosylated and can be produced by microbial expression, exhibit class behavior during production, can be produced at high yields, can be delivered by subcutaneous injection, have excellent tissue penetration, have a customized pharmacokinetic profile, and are non-immunogenic in animals (see, for example, U.S. Patent Application Publ. Nos. 2005/0221384, 2005/0164301, 2005/0053973 and 2005/0089932, 2005/0048512, and 2004/0175756, each of which is hereby incorporated by reference herein in its entirety).

### 5.1.3 Eph Receptor Fragments and Ephrin Fragments As Eph/Ephrin Modulators

In one embodiment, an Eph/Ephrin Modulator of the invention is an Eph receptor (e.g., EphA1, EphA2, EphA3, EphA4, EphA5, EphA6, EphA7, EphA8, EphA10 EphB1, EphB2, EphB3, EphB4, EphB5 or EphB6) polypeptide. In accordance with this embodiment, the Eph Receptor Fragment, in certain embodiments, retains the ability to bind to an endogenous ligand (*e.g*., EphrinA1, EphrinA2, EphrinA3, EphrinA4, EphrinA5, EphrinB1, EphrinB2 or EphrinB3). In one embodiment, the Eph Receptor Fragment retains the ability to bind to an Ephrin and inhibits binding of endogenous Eph receptors from binding to an endogenous ligand, *e.g*., an Ephrin.

Non-limiting examples of Eph Fragments include, but are not limited to, Eph Receptor Fragments comprising the ligand binding domain of human EphA1, EphA2, EphA3, EphA4, EphA5, EphA6, EphA7, EphA8, EphA10, EphB1, EphB2, EphB3, EphB4, EphB5 or EphB6 (for specific amino acid residues, see Table 1, *supra*) and any one or more of the following domains: the first fibronectin Type III domain; the second fibronectin Type III domain; the tyrosine kinase catalytic domain; and/or the sterile alpha motif "SAM" domain, the sequences of which may be found in the GenBank database (see Table 1, *infra).* In a specific embodiment, an Eph Receptor Fragment is soluble (*i.e*., not membrane-bound). In a specific embodiment, an Eph Receptor Fragment of the invention lacks the transmembrane domain of an Eph receptor (for specific amino acid residues, see Table 1, *supra*) and is not membrane-bound. In further embodiments, an Eph Receptor Fragment of the invention comprises the extracellular domain or a portion thereof and lacks the transmembrane domain or a portion thereof such that the Eph receptor is not membrane-bound. In yet further embodiments, an Eph Receptor Fragment of the invention comprises the cytoplasmic domain or a portion of the cytoplasmic domain of the Eph receptor and lacks the transmembrane domain or a portion thereof such that the Eph receptor is not membrane-bound. In specific embodiments, an Eph Receptor Fragment of the invention comprises specific fragments of the extracellular domain of human of human EphA1 EphA2, EphA3, EphA4, EphA5, EphA6, EphA7, EphA8, EphA10 EphB1, EphB2, EphB3, EphB4, EphB5 or EphB6 (for specific amino acid residues, see Table 1, *infra*)*.* In another specific embodiment, an Eph Receptor Fragment of the invention lacks the transmembrane domain of the Eph receptor such that the Eph receptor is not membrane-bound.

The Eph Receptor Fragments include polypeptides that are 100%, 98%, 95%, 90%, 85%, 80%, 75%, 70%, 65%, 60%, 55%, 50%, 45%, 40% identical to endogenous Eph receptor sequences (see, *e.g*., Table 1, *infra*)*.* The determination of percent identity of two amino acid sequences can be determined by any method known to one skilled in the art, including BLAST protein searches. In specific embodiments, Eph Receptor Fragments of the invention can be analogs or derivatives of EphA1 EphA2, EphA3, EphA4, EphA5, EphA6, EphA7, EphA8, EphA10, EphB1, EphB2, EphB3, EphB4, EphB5 or EphB6. For example, Eph Receptor Fragments of the invention include derivatives that are modified, *i.e*., by covalent attachment of any type of molecule to the polypeptide. For example, but not by way of limitation, the polypeptide derivatives (*e.g*., EphA1 EphA2, EphA3, EphA4, EphA5, EphA6, EphA7, EphA8, EphA10 EphB1 EphB2, EphB3, EphB4, EphB5 or EphB6 polypeptide derivatives) include polypeptides that have been modified, *e.g*., by glycosylation, acetylation, pegylation, phosphorylation, amidation, derivatization by known protecting/blocking groups, proteolytic cleavage, linkage to a cellular ligand, etc. Any of numerous chemical modifications may be carried out by known techniques, including, but not limited to, specific chemical cleavage, acetylation, formylation, metabolic synthesis of tunicamycin, etc. Additionally, the derivative may contain one or more non-classical amino acids.

In a specific embodiment, an Eph/Ephrin Modulator of the invention is a dominant negative form of an Eph receptor which lacks the cytoplasmic domain or a portion thereof required for signaling. In a specific embodiment, the dominant negative form of the Eph receptor retains the ability to bind an endogenous ligand (*e.g*., an Ephrin) but is incapable of signaling, induces low to negligible signaling or does not induce all the signal transduction pathways activated upon ligand-receptor interaction. In specific embodiments, low to negligible signaling in the context of Eph receptors refers to a decrease in any aspect of Eph receptor signaling upon ligand binding by at least 25%, at least 30%, at least 35%, at least 40%, at least 50%, at least 55%, at least 60%, at least 65%, at least 70%, at least 75%, at least 80%, at least 85%, at least 90%, at least 95%, or at least 98% relative to a control in an *in vivo* and/or an *in vitro* assay described herein or well known to one of skill in the art. In certain aspects of the invention, Eph receptor signaling encompasses any one or more of the signaling pathways that are activated upon Eph receptor binding to its endogenous ligand (*e.g*., EphrinA1, EphrinA2, EphrinA3, EphrinA4, EphrinA5, EphrinB1 EphrinB2 or EphrinB3). Non-limiting examples of such signaling pathways include but are not limited to, the mitogen-activated protein kinase (MAPK)/ERK pathway, the Ras pathway, and pathways involving the Src family of kinases (for other Eph receptor pathways, see, Cheng et al., 2002, Cytokine & Growth Factor Rev. 13:75-85; Kullander and Klein, 2002, Nature Rev. 3:475-486; Holder and Klein, 1999, Development 126:2033-2044; Zhou, 1998, Pharmacol. Ther. 77:151 -181; and Nakamoto and Bergemann, 2002, Microscopy Res. & Technique 59:58-67, which are all incorporated by reference herein in their entireties). In a specific embodiments, various assays known to one of skill in the art may be performed to measure Eph receptor signaling. For example, but not by way of limitation, Eph receptor (*e.g*., EphA1 EphA2, EphA3, EphA4, EphA5, EphA6, EphA7, EphA8, EphA10 EphB1, EphB2, EphB3, EphB4, EphB5 or EphB6) phosphorylation may be measured to determine whether Eph receptor signaling is activated upon ligand binding by measuring the amount of phosphorylated Eph receptor present in Ephrin-treated cells relative to control cells that are not treated with Ephrin. Eph receptor proteins may be isolated using any protein immunoprecipitation method known to one of skill in the art and an Eph receptor antibody of the invention. Phosphorylated Eph receptor may then be measured using anti-phosphotyrosine antibodies (Upstate Tiotechnology, Inc., Lake Placid, New York) using any standard immunoblotting method known to one of skill in the art. See, *e.g*., Cheng et al., 2002, Cytokine & Growth Factor Rev. 13:75-85. In another embodiment, MAPK phosphorylation may be measured to determine whether Eph receptor (*e.g*., EphA2) signaling is activated upon ligand binding by measuring the amount of phosphorylated MAPK present in Ephrin-treated cells relative to control cells that are not treated with Ephrin using standard immunoprecipitation and immunoblotting assays known to one of skill in the art (see, *e.g.,* Miao et al., 2003, J. Cell Biol. 7:1281-1292, which is incorporated by reference herein in its entirety).

In a specific embodiment, an Eph/Ephrin Modulator of the invention is a fragment of an Ephrin ("Ephrin Fragment"). In accordance with this embodiment, the Ephrin Fragment preferably retains the ability to bind to an Eph receptor. In a further specific embodiment, the Ephrin Fragment retains the ability to bind to an Eph receptor and inhibits binding of endogenous Ephrins to the Eph receptor.

Non-limiting examples of Ephrin Fragments include, but are not limited to, any fragment of human EphrinA1 EphrinA2, EphrinA3, EphrinA4, EphrinA5, EphrinB1, EphrinB2 or EphrinB3 as disclosed in the GenBank database (*e.g*., see Table 2, *infra).* In a specific embodiment, an Ephrin Fragment is soluble (*i.e*., not membrane-bound). In a specific embodiment, an Ephrin Fragment of the invention comprises the extracellular domain of human EphrinA1, EphrinA2, EphrinA3, EphrinA4, EphrinA5, EphrinB1, EphrinB2 or EphrinB3 or a portion thereof. In further embodiments, an Ephrin Fragment of the invention comprises the extracellular domain of human EphrinA1, EphrinA2, EphrinA3, EphrinA4, EphrinA5, EphrinB 1, EphrinB2 or EphrinB3 or a portion thereof and is not membrane-bound. In specific embodiments, an Ephrin Fragment of the invention comprises specific fragments of the extracellular domain of human EphrinA1, EphrinA2, EphrinA3, EphrinA4, EphrinA5, EphrinB1 EphrinB2 or EphrinB3 or a portion thereof and is not membrane bound.

The Ephrin Fragments include polypeptides that are 100%, 98%, 95%, 90%, 85%, 80%, 75%, 70%, 65%, 60%, 55%, 50%, 45%, 40% identical to endogenous EphrinA1, EphrinA2, EphrinA3, EphrinA4, EphrinA5, EphrinB1, EphrinB2 or EphrinB3 sequences (for specific sequences, see Table 2, *infra*). The determination of percent identity of two amino acid sequences can be determined by any method known to one skilled in the art, including BLAST protein searches. In specific embodiments, Ephrin Fragments of the invention can be analogs or derivatives of EphrinA1, EphrinA2, EphrinA3, EphrinA4, EphrinA5, EphrinB1, EphrinB2 or EphrinB3. For example, Ephrin Fragments of the invention include derivatives that are modified, *i.e*., by covalent attachment of any type of molecule to the polypeptide. For example, but not by way of limitation, the polypeptide derivatives (*e.g*., EphrinA1, EphrinA2, EphrinA3, EphrinA4, EphrinA5, EphrinB1 EphrinB2 or EphrinB3 polypeptide derivatives) include polypeptides that have been modified, *e.g*., by glycosylation, acetylation, pegylation, phosphorylation, amidation, derivatization by known protecting/blocking groups, proteolytic cleavage, linkage to a cellular ligand, etc. Any of numerous chemical modifications may be carried out by known techniques, including, but not limited to, specific chemical cleavage, acetylation, formylation, metabolic synthesis of tunicamycin, etc. Additionally, the derivative may contain one or more non-classical amino acids.

In a specific embodiment, an Eph/Ephrin Modulator is an Eph receptor (*e.g*., EphA1, EphA2, EphA3, EphA4, EphA5, EphA6, EphA7, EphA8, EphA10, EphB1, EphB2, EphB3, EphB4, EphB5 or EphB6) or Ephrin (*e.g*., EphrinA1, EphrinA2, EphrinA3, EphrinA4, EphrinA5, EphrinB1, EphrinB2 or EphrinB3) fusion protein. In one embodiment, an Eph receptor or Ephrin fusion protein is soluble. Non-limiting examples of Eph receptor fusion proteins include soluble forms of an Eph receptor (*e.g*., EphA2) such as EphA2-Fc (see, *e.g.,* Cheng et al., 2002, Mol. Cancer Res. 1:2-11, which is incorporated by reference herein in its entirety). In a specific embodiment, an Eph receptor fusion protein comprises an Eph receptor (*e.g*., EphA1, EphA2, EphA3, EphA4, EphA5, EphA6, EphA7, EphA8, EphA10, EphB1 EphB2, EphB3, EphB4, EphB5 or EphB6) fused to the Fc portion of human immunoglobulin IgG1. In another embodiment, an EphA2 fusion protein comprises an EphA2 Fragment which retains its ability to bind EphrinA1 (*e.g*., the extracellular domain of EphA2) fused to the Fc portion of human immunoglobulin IgG1 (see, *e.g*., Carles-Kinch et al., 2002, Cancer Res. 62:2840-2847; and Cheng et al., 2002, Mol. Cancer Res. 1:2-11, which are incorporated by reference herein in their entireties).

Non-limiting examples of Ephrin fusion proteins include soluble forms of an Ephrin (*e.g*., Ephrin-Fc). In a specific embodiment, an Ephrin fusion protein comprises an Ephrin fused to an the Fc domain of human immunoglobulin IgG. See, *e.g*., Duxbury et al., 2004, Biochem. & Biophys. Res. Comm. 320:1096-1102, which is incorporated by reference herein in its entirety. In another embodiment, an Ephrin fusion protein comprises an Ephrin Fragment which retains its ability to bind an Eph receptor fused to the Fc domain of human immunoglobulin IgG.

Fragments of Eph receptors or Ephrins can be made and assayed for the ability to bind any Ephrin or Eph receptor, respectively, using biochemical, biophysical, genetic, and/or computational techniques for studying protein-protein interactions that are described herein or by any method known in the art. Non-limiting examples of methods for detecting protein binding (*e.g*., for detecting Eph receptor binding to an Ephrin), qualitatively or quantitatively, *in vitro* or *in vivo,* include GST-affinity binding assays, far-Western Blot analysis, surface plasmon resonance (SRP), fluorescence resonance energy transfer (FRET), fluorescence polarization (FP), isothermal titration calorimetry (ITC), circular dichroism (CD), protein fragment complementation assays (PCA), various two-hybrid systems, and proteomics and bioinformatics-based approaches, such as the Scansite program for computational analysis (see, *e.g*., Fu, H., 2004, Protein-Protein Interactions: Methods and Applications (Humana Press, Totowa, NJ); and Protein-Protein Interactions: A Molecular Cloning Manual, 2002, Golemis, ed. (Cold Spring Harbor Laboratory Press, Cold Spring Harbor, New York) which are incorporated by reference herein in their entireties).

### 5.1.4 Modified Polypeptides As Eph/Ephrin Modulators

The polypeptide Eph/Ephrin Modulators used in the methods of the invention (*e.g*., antibodies, Eph Receptor Fragments and Ephrin Fragments) include derivatives that are modified, *i.e*, by the covalent attachment of any type of molecule to the polypeptide. For example, but not by way of limitation, the polypeptide derivatives include polypeptides that have been modified, *e.g*., by glycosylation, acetylation, pegylation, phosphorylation, amidation, derivatization by known protecting/blocking groups, proteolytic cleavage, linkage to a cellular ligand or other protein, etc. Any of numerous chemical modifications may be carried out by known techniques, including, but not limited to, specific chemical cleavage, acetylation, formylation, metabolic synthesis of tunicamycin, etc. Additionally, the derivative may contain one or more non-classical amino acids.

The methods of the present invention also encompass the use of polypeptide Eph/Ephrin Modulators that have half-lives (*e.g*., serum half-lives) in a mammal, preferably a human, of greater than 15 days, preferably greater than 20 days, greater than 25 days, greater than 30 days, greater than 35 days, greater than 40 days, greater than 45 days, greater than 2 months, greater than 3 months, greater than 4 months, or greater than 5 months. The increased half-lives of the polypeptide Eph/Ephrin Modulators in mammals, preferably humans, results in a higher concentration of said polypeptide Eph/Ephrin Modulators in the mammals, and thus, reduces the frequency of the administration of said polypeptide Eph/Ephrin Modulators and/or reduces the amount of said polypeptide Eph/Ephrin Modulators to be administered. Polypeptide Eph/Ephrin Modulators having increased *in vivo* half-lives can be generated by techniques known to those of skill in the art. For example, polypeptide Eph/Ephrin Modulators with increased *in vivo* half-lives can be generated by modifying (*e.g*., substituting, deleting or adding) amino acid residues. In one embodiment, when the polypeptide Eph/Ephrin Modulator is an antibody, such amino acid residues to be modified can be those residues involved in the interaction between the Fc domain and the FcRn receptor (see, *e.g*., International Patent Publication No. WO 97/34631, U.S. Patent Application Publication No. 2003/0190311 A1 and U.S. Patent Application Publication No. 2004/0191265 A1, which are incorporated herein by reference in their entireties). Polypeptide Eph/Ephrin Modulators with increased *in vivo* half-lives can also be generated by attaching to said polypeptides polymer molecules such as high molecular weight polyethylene glycol (PEG). PEG can be attached to said polypeptide Eph/Ephrin Modulators with or without a multifunctional linker either through site-specific conjugation of the PEG to the N- or C- terminus of said polypeptide or via epsilon-amino groups present on lysine residues. Linear or branched polymer derivatization that results in minimal loss of biological activity will be used. The degree of conjugation will be closely monitored by SDS-PAGE and mass spectrometry to ensure proper conjugation of PEG molecules to the polypeptide Eph/Ephrin Modulators. Unreacted PEG can be separated from polypeptide Eph/Ephrin Modulator-PEG conjugates by, *e.g*., size exclusion or ion-exchange chromatography.

### 5.1.5 Polynucleotides Encoding Polypeptide Eph/Ephrin Modulators

The Eph/Ephrin Modulators of the invention include polypeptides produced from polynucleotides that hybridize to polynucleotides which encode polypeptides disclosed in sections 6.1.1 to 6.1.3 above. In one embodiment, antibodies of the invention include Eph receptor and Ephrin monoclonal antibodies produced from polynucleotides that hybridize to polynucleotides encoding monoclonal antibodies that modulate the expression and/or activity an Eph receptor and/or Ephrin in one or more of the assays described herein or known to those skilled in the art. In another embodiment, Eph Fragments or Ephrin Fragments used in the methods of the invention include polypeptides produced from polynucleotides that hybridize to polynucleotides encoding a fragments of an Eph receptor or Ephrin. Conditions for hybridization include, but are not limited to, stringent hybridization conditions such as hybridization to filter-bound DNA in 6X sodium chloride/sodium citrate (SSC) at about 45°C followed by one or more washes in 0.2X SSC/0.1% SDS at about 50-65°C, highly stringent conditions such as hybridization to filter-bound DNA in 6X SSC at about 45°C followed by one or more washes in 0.1X SSC/0.2% SDS at about 60°C, or any other stringent hybridization conditions known to those skilled in the art (see, for example, Ausubel, F.M. et al., eds. 1989 Current Protocols in Molecular Biology, vol. 1, Green Publishing Associates, Inc. and John Wiley and Sons, Inc., NY at pages 6.3.1 to 6.3.6 and 2.10.3).

The Eph/Ephrin Modulators of the invention include polynucleotides encoding polypeptides described herein. The polynucleotides encoding the polypeptides described herein (*e.g*., the antibodies of the invention or the EphFragments and Ephrin Fragments) may be obtained and sequenced by any method known in the art. For example, a polynucleotide encoding a polypeptide Eph/Ephrin Modulator used in the methods of the invention may be assembled from chemically synthesized oligonucleotides (*e.g*., as described in Kutmeier et al., 1994, BioTechniques 17:242), which, briefly, involves the synthesis of overlapping oligonucleotides containing portions of the sequence encoding the polypeptide, annealing and ligating of those oligonucleotides, and then amplification of the ligated oligonucleotides by PCR.

Alternatively, a polynucleotide encoding polypeptide Eph/Bphrin Modulator used in the methods of the invention may be generated from nucleic acid from a suitable source. If a clone containing a nucleic acid encoding a particular polypeptide is not available, but the sequence of the polypeptide is known, a nucleic acid encoding the polypeptide may be chemically synthesized or obtained from a suitable source (*e.g*., an antibody cDNA library, or a cDNA library generated from, or nucleic acid, preferably poly A+ RNA, isolated from, any tissue or cells expressing the desired polypeptide, such as hybridoma cells selected to express an antibody of the invention or epithelial and/or endothelial cells that express EphA2 or EphrinA1) by PCR amplification using synthetic primers hybridizable to the 3' and 5' ends of the sequence or by cloning using an oligonucleotide probe specific for the particular gene sequence to identify, *e.g*., a cDNA clone from a cDNA library that encodes the polypeptide Eph/Ephrin Modulator. Amplified nucleic acids generated by PCR may then be cloned into replicable cloning vectors using any method well known in the art.

Once the nucleotide sequence of the polypeptide Eph/Ephrin Modulator used in the methods of the invention is determined, the nucleotide sequence may be manipulated using methods well known in the art for the manipulation of nucleotide sequences, *e.g*., recombinant DNA techniques, site directed mutagenesis, PCR, etc. (see, for example, the techniques described in Sambrook et al., 1990, Molecular Cloning, A Laboratory Manual, 2d Ed., Cold Spring Harbor Laboratory, Cold Spring Harbor, NY and Ausubel et al., eds., 1998, Current Protocols in Molecular Biology, John Wiley & Sons, NY, which are both incorporated by reference herein in their entireties), to generate polypeptides having a different amino acid sequence, for example to create amino acid substitutions, deletions, and/or insertions.

Standard techniques known to those skilled in the art can be used to introduce mutations in the nucleotide sequence encoding a polypeptide Eph/Ephrin Modulator including, *e.g*., site-directed mutagenesis and PCR-mediated mutagenesis, which results in amino acid substitutions. In one embodiment, the derivatives include less than 15 amino acid substitutions, less than 10 amino acid substitutions, less than 5 amino acid substitutions, less than 4 amino acid substitutions, less than 3 amino acid substitutions, or less than 2 amino acid substitutions relative to the original Eph/Ephrin Modulator. In a specific embodiment, the derivatives have conservative amino acid substitutions made at one or more predicted non-essential amino acid residues.

The present invention also encompasses the use of antibodies or antibody fragments comprising the amino acid sequence of any Eph receptor or Ephrin antibodies with mutations (*e.g*., one or more amino acid substitutions) in the framework or variable regions. In one embodiment, mutations in these antibodies maintain or enhance the avidity and/or affinity of the antibodies for the particular antigen(s) to which they specifically bind. In another embodiment, mutations in these antibodies lessen or decrease the avidity and/or affinity of the antibodies for the particular antigen(s) to which they specifically bind. Standard techniques known to those skilled in the art (*e.g*., immunoassays or ELISA assays) can be used to assay the degree of binding between a polypeptide Eph/Ephrin Modulator and its binding partner. In a specific embodiment, when a polypeptide Eph/Ephrin Modulator is an antibody, an Eph Receptor Fragment, an Ephrin Fragment, an Eph receptor fusion protein, an Ephrin fusion protein, a dominant negative form of an Eph receptor, or a dominant negative form of an Ephrin, binding to an Eph receptor or Ephrin, as appropriate, can be assessed.

### 5.1.6 Polynucleotide Eph/Ephrin Modulators

In addition to the polypeptide Eph/Ephrin Modulators of the invention, nucleic acid molecules can be used in methods of the invention. In one embodiment, a nucleic acid molecule Eph/Ephrin Modulator reduces the amount of endogenous ligand (*e.g*., an Ephrin) available for binding to an Eph receptor. In another embodiment, a nucleic acid molecule Eph/Ephrin Modulator reduces the amount of Eph receptor available for binding to an Ephrin. Any method known in the art to decrease expression of an Eph receptor and/or an Ephrin can be used in the methods of the invention including, but not limited to, antisense, RNA interference and aptamer technology. Thus, Eph/Ephrin Modulators encompasses those agents that serve to decrease Ephrin expression or availability for Eph receptor-binding, and those agents that serve to decrease Eph receptor expression or availability for binding to an endogenous Eph receptor ligand (*e.g*., an Ephrin).

### 5.1.6.1 Antisense

The present invention encompasses Eph receptor (*e.g*., EphA1, EphA2, EphA3, EphA4, EphA5, EphA6, EphA7, EphA8, EphA10, EphB1, EphB2, EphB3, EphB4, EphB5 or EphB6) or Ephrin (*e.g*., EphrinA1, EphrinA2, EphrinA3, EphrinA4, EphrinA5, EphrinB1, EphrinB2 or EphrinB3) antisense nucleic acid molecules, *i.e*., molecules which are complementary to all or part of a sense nucleic acid encoding an Eph receptor or an Ephrin, molecules which are complementary to the coding strand of a double-stranded Eph receptor or Ephrin cDNA molecule or molecules complementary to an Eph receptor or Ephrin mRNA sequence (*e.g.,* see Tables 1 and 2, *infra,* for GenBank nucleotide sequences disclosed therein). In certain embodiments, the Eph/Ephrin Modulator is not an EphA2 antisense nucleic acid molecule.

An antisense nucleic acid can hydrogen bond to a sense nucleic acid. The antisense nucleic acid can be complementary to an entire coding strand, or to only a portion thereof, *e.g*., all or part of the protein coding region (or open reading frame). An antisense nucleic acid molecule can be antisense to all or part of a non-coding region of the coding strand of a nucleotide sequence encoding a polypeptide of the invention. The non-coding regions ("5' and 3' untranslated regions") are the 5' and 3' sequences which flank the coding region and are not translated into amino acids.

An antisense oligonucleotide can be, for example, about 5, 10, 15, 20, 25, 30, 35, 40, 45 or 50 nucleotides in length. An antisense nucleic acid of the invention can be constructed using chemical synthesis and enzymatic ligation reactions using procedures known in the art. For example, an antisense nucleic acid (*e.g*., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, *e.g*., phosphorothioate derivatives and acridine substituted nucleotides can be used. Alternatively, the antisense nucleic acid can be produced biologically using an expression vector into which a nucleic acid has been subcloned in an antisense orientation (*i.e*., RNA transcribed from the inserted nucleic acid will be of an antisense orientation to a target nucleic acid of interest, *i.e*., EphrinA1).

The antisense nucleic acid molecules of the invention are typically administered to a subject or generated *in situ* such that they hybridize with or bind to cellular mRNA and/or genomic DNA encoding a selected polypeptide of the invention to thereby inhibit expression, *e.g*., by inhibiting transcription and/or translation. The hybridization can be by conventional nucleotide complementarity to form a stable duplex, or, for example, in the case of an antisense nucleic acid molecule which binds to DNA duplexes, through specific interactions in the major groove of the double helix. An example of a route of administration of antisense nucleic acid molecules of the invention includes direct injection at a tissue site. Alternatively, antisense nucleic acid molecules can be modified to target selected cells and then administered systemically. For example, for systemic administration, antisense molecules can be modified such that they specifically bind to receptors or antigens expressed on a selected cell surface, *e.g*., by linking the antisense nucleic acid molecules to peptides or antibodies which bind to cell surface receptors or antigens. The antisense nucleic acid molecules can also be delivered to cells using the vectors described herein. To achieve sufficient intracellular concentrations of the antisense molecules, vector constructs in which the antisense nucleic acid molecule is placed under the control of a strong pol II or pol III promoter are preferred.

An antisense nucleic acid molecule of the invention can be an α-anomeric nucleic acid molecule. An α-anomeric nucleic acid molecule forms specific double-stranded hybrids with complementary RNA in which, contrary to the usual β-units, the strands run parallel to each other (Gaultier et al., 1987, Nucleic Acids Res. 15:6625). The antisense nucleic acid molecule can also comprise a 2'-o-methylribonucleotide (Inoue et al., 1987, Nucleic Acids Res. 15:6131) or a chimeric RNA-DNA analogue (Inoue et al., 1987, FEBS Lett. 215:327).

### 5.1.6.2 RNA Interference

In certain embodiments, an RNA interference (RNAi) molecule is used to decrease expression of an Eph receptor and/or an Ephrin. RNAi is defined as the ability of double-stranded RNA (dsRNA) to suppress the expression of a gene corresponding to its own sequence. RNAi is also called post-transcriptional gene silencing or PTGS. Since the only RNA molecules normally found in the cytoplasm of a cell are molecules of single-stranded mRNA, the cell has enzymes that recognize and cut dsRNA into fragments containing 21-25 base pairs (approximately two turns of a double helix). The antisense strand of the fragment separates enough from the sense strand so that it hybridizes with the complementary sense sequence on a molecule of endogenous cellular mRNA (*e.g*., a human Ephrin receptor or an Ephrin). This hybridization triggers cutting of the mRNA in the double-stranded region, thus destroying its ability to be translated into a polypeptide. Introducing dsRNA corresponding to a particular gene thus knocks out the cell's own expression of that gene in particular tissues and/or at a chosen time.

Double-stranded (ds) RNA can be used to interfere with gene expression in mammals (Wianny & Zernicka-Goetz, 2000, Nature Cell Biology 2:70-75; incorporated herein by reference in its entirety). dsRNA is used as inhibitory RNA or RNAi of the function of Ephrin A1 to produce a phenotype that is the same as that of a null mutant of Ephrin A1 (Wianny & Zernicka-Goetz, 2000, Nature Cell Biology 2: 70-75). In certain embodiments, dsDNA encoding dsRNA (*e.g*., as hairpin structures) is used to express RNAi-mediating dsDNA in the cell.

### 5.1.6.3 Aptamers as Eph/Ephrin Modulators

In specific embodiments, the invention provides aptamers of an Eph receptor (*e.g*., EphA1 EphA2, EphA3, EphA4, EphA5, EphA6, EphA7, EphA8, EphA10, EphB1, EphB2, EphB3, EphB4, EphB5 or EphB6) and an Ephrin (*e.g*., EphrinA1, EphrinA2, EphrinA3, EphrinA4, EphrinA5, EphrinB1 EphrinB2 or EphrinB3). As is known in the art, aptamers are macromolecules composed of nucleic acid (*e.g*., RNA, DNA) that bind tightly to a specific molecular target (*e.g*., Eph receptor or Ephrin proteins, Eph receptor or Ephrin polypeptides and/or Eph receptor or Ephrin epitopes as described herein). A particular aptamer may be described by a linear nucleotide sequence and is typically about 15-60 nucleotides in length. The chain of nucleotides in an aptamer form intramolecular interactions that fold the molecule into a complex three-dimensional shape, and this three-dimensional shape allows the aptamer to bind tightly to the surface of its target molecule. Given the extraordinary diversity of molecular shapes that exist within the universe of all possible nucleotide sequences, aptamers may be obtained for a wide array of molecular targets, including proteins and small molecules. In addition to high specificity, aptamers have very high affinities for their targets (*e.g*., affinities in the picomolar to low nanomolar range for proteins). Aptamers are chemically stable and can be boiled or frozen without loss of activity. Because they are synthetic molecules, they are amenable to a variety of modifications, which can optimize their function for particular applications. For *in vivo* applications, aptamers can be modified to dramatically reduce their sensitivity to degradation by enzymes in the blood. In addition, modification of aptamers can also be used to alter their biodistribution or plasma residence time.

Selection of aptamers that can bind to an Eph receptor or an Ephrin or a fragment there of can be achieved through methods known in the art. For example, aptamers can be selected using the SELEX (Systematic Evolution of Ligands by Exponential Enrichment) method (Tuerk and Gold, 1990, Science 249:505-510, which is incorporated by reference herein in its entirety). In the SELEX method, a large library of nucleic acid molecules (*e.g*., 10¹⁵ different molecules) is produced and/or screened with the target molecule (*e.g*., Eph receptor or Ephrin proteins, Eph receptor or Ephrin polypeptides and/or Eph receptor or Ephrin epitopes or fragments thereof as described herein). The target molecule is allowed to incubate with the library of nucleotide sequences for a period of time. Several methods can then be used to physically isolate the aptamer target molecules from the unbound molecules in the mixture and the unbound molecules can be discarded. The aptamers with the highest affinity for the target molecule can then be purified away from the target molecule and amplified enzymatically to produce a new library of molecules that is substantially enriched for aptamers that can bind the target molecule. The enriched library can then be used to initiate a new cycle of selection, partitioning, and amplification. After 5-15 cycles of this selection, partitioning and amplification process, the library is reduced to a small number of aptamers that bind tightly to the target molecule. Individual molecules in the mixture can then be isolated, their nucleotide sequences determined, and their properties with respect to binding affinity and specificity measured and compared. Isolated aptamers can then be further refined to eliminate any nucleotides that do not contribute to target binding and/or aptamer structure (*i.e*., aptamers truncated to their core binding domain). See, *e.g*., Jayasena, 1999, Clin. Chem. 45:1628-1650 for review of aptamer technology, the entire teachings of which are incorporated herein by reference).

In particular embodiments, the aptamers of the invention have the binding specificity and/or functional activity described herein for the antibodies of the invention. Thus, for example, in certain embodiments, the present invention is drawn to aptamers that have the same or similar binding specificity as described herein for the antibodies of the invention (*e.g*., binding specificity for Eph receptor or Ephrin polypeptides, fragments of Eph receptor or Ephrin polypeptides, epitopic regions of vertebrate Eph receptor or Ephrin polypeptides (*e.g*., epitopic regions of an Eph receptor or an Ephrin that are bound by the antibodies of the invention)). In particular embodiments, the aptamers of the invention can bind to an Eph receptor or Ephrin polypeptide and inhibit one or more activities of the Eph receptor or Ephrin polypeptide.

### 5.1.7 Vaccines That Are Eph/Ephrin Modulators

In a specific embodiment, an Eph/EphrinModulator is an Eph receptor and/or an Ephrin vaccine. As used herein, the term "Eph receptor vaccine" refers to any reagent that elicits or mediates an immune response against cells that overexpress an Eph receptor. In certain embodiments, an Eph receptor vaccine is an Eph receptor antigenic peptide of the invention, an expression vehicle (*e.g*., a naked nucleic acid or a viral or bacterial vector or a cell) for an Eph receptor antigenic peptide (*e.g*., which delivers the Eph receptor antigenic peptide), or T cells or antigen presenting cells (*e.g*., dendritic cells or macrophages) that have been primed with the Eph receptor antigenic peptide of the invention. As used herein, the terms "Eph receptor antigenic peptide" and "Eph receptor antigenic polypeptide" refer to an Eph receptor polypeptide, or a fragment, analog, or derivative thereof comprising one or more B cell epitopes or T cell epitopes of an Eph receptor. The Eph receptor polypeptide may be from any species. In certain embodiments, an Eph receptor polypeptide refers to the mature, processed form of an Eph receptor. In other embodiments, an Eph receptor polypeptide refers to an immature form of an Eph receptor. For a description of Eph receptor vaccines, see, *e.g*., U.S. Provisional Application Ser. No. 60/556,601, entitled "EphA2 Vaccines," filed Mar. 26, 2004; U.S. Provisional Application Serial No. 60/602,588, filed August 18, 2004, entitled "EphA2 Vaccines"; U.S. Provisional Application Serial No. 60/615,548, filed October 1, 2004, entitled "EphA2 Vaccines"; U.S. Provisional Application Serial No. 60/617,564, filed October 7, 2004, entitled "EphA2 Vaccines", and International Application No. PCT/US2004/034693, filed October 15, 2004 entitled "EphA2 Vaccines" each of which is incorporated by reference herein in its entirety.

In a specific embodiment, an Eph/Ephrin Modulator is an Ephrin Vaccine. As used herein, the term "Ephrin vaccine" refers to any reagent that elicits or mediates an immune response against Ephrin on Eprhin-expressing cells. In certain embodiments, an Ephrin vaccine is an Ephrin antigenic peptide of the invention, an expression vehicle (*e.g*., a naked nucleic acid or a viral or bacterial vector or a cell) for an Ephrin antigenic peptide (*e.g*., which delivers the Ephrin antigenic peptide), or T cells or antigen presenting cells (*e.g*., dendritic cells or macrophages) that have been primed with the Ephrin antigenic peptide of the invention. As used herein, the terms "Ephrin antigenic peptide" and "Ephrin antigenic polypeptide" refer to an Ephrin polypeptide, or a fragment, analog, or derivative thereof comprising one or more B cell epitopes or T cell epitopes of an Ephrin. The Ephrin polypeptide may be from any species. In certain embodiments, an Ephrin polypeptide refers to the mature, processed form of an Ephrin. In other embodiments, an Eph polypeptide refers to an immature form of an Ephrin.

The present invention thus provides Eph/Ephrin Modulators that are Eph receptor vaccines. In a specific embodiment, an Eph/Ephrin Modulator is an Eph receptor- and/or Ephrin antigenic peptide expression vehicle expressing an Eph receptor or an Ephrin antigenic peptide that can elicit or mediate a cellular immune response, a humoral response, or both, against cells that overexpress an Eph receptor or an Ephrin. Where the immune response is a cellular immune response, it can be a Tc, Th1 or a Th2 immune response. In a one embodiment, the immune response is a Th2 cellular immune response. In another embodiment, an Eph receptor or an Ephrin antigenic peptide expressed by an Eph receptor-/Ephrin- antigenic peptide expression vehicle is an Eph receptor or Ephrin antigenic peptide that is capable of eliciting an immune response against Eph receptor- and/or Ephrin-expressing cells involved in an infection.

In a specific embodiment, the Eph receptor- and/or Ephrin antigenic expression vehicle is a microorganism expressing an Eph receptor and/or an Ephrin antigenic peptide. In another specific embodiment, the Eph receptor- and/or Ephrin antigenic expression vehicle is an attenuated bacteria. Non-limiting examples of bacteria that can be utilized in accordance with the invention as an expression vehicle include *Listeria monocytogenes,* include but are not limited to *Borrelia burgdorferi, Brucella melitensis, Escherichia coli, enteroinvasive Escherichia coli, Legionella pneumophila, Salmonella typhi, Salmonella typhimurium, Shigella spp., Streptococcus spp., Treponema pallidum, Yersinia enterocohtica, Listeria monocytogenes, Mycobacterium avium, Mycobacterium bovis, Mycobacterium tuberculosis, BCG, Mycoplasma hominis, Rickettsiae quintana, Cryptococcus neoformans, Histoplasma capsulatum, Pneumocystis carnii, Eimeria acervulina, Neospora caninum, Plasmodium falciparum, Sarcocystis suihominis, Toxoplasma gondii, Leishmania amazonensis, Leishmania major, Leishmania mexacana, Leptomonas karyophilus, Phytomonas spp., Trypanasoma cruzi, Encephahtozoon cuniculi, Nosema helminthorum, Unikaryon legeri.* In a specific embodiment, an Eph/Ephrin Modulator vaccine is *Listeria-*based vaccine expresses an Eph receptor and/or an Ephrin antigenic peptide. In a further embodiment, the *Listeria-*based vaccine expressing an Eph receptor- and/or an Ephrin antigenic peptide is attenuated. In a specific embodiment, an Eph/Ephrin Modulator vaccine is not *Listeria*-based or is not EphA2-based or is not Eph receptor-based.

In another embodiment, the Eph receptor- and/or Ephrin antigenic peptide expression vehicle is a virus expressing an Eph receptor- and/or an Ephrin antigenic peptide. Non-limiting examples of viruses that can be utilized in accordance with the invention as an expression vehicle include RNA viruses (*e.g*., single stranded RNA viruses and double stranded RNA viruses), DNA viruses (*e.g*., double stranded DNA viruses), enveloped viruses, and non-enveloped viruses. Other non-limiting examples of viruses useful as Eph receptor-and/or Ephrin antigenic peptide expression vehicles include retroviruses (including but not limited to lentiviruses), adenoviruses, adeno-associated viruses, or herpes simplex viruses. Preferred viruses for administration to human subjects are attenuated viruses. A virus can be attenuated, for example, by exposing the virus to mutagens, such as ultraviolet irradiation or chemical mutagens, by multiple passages and/or passage in non-permissive hosts, and/or genetically altering the virus to reduce the virulence and pathogenicity of the virus.

Microorganisms can be produced by a number of techniques well known in the art. For example, antibiotic-sensitive strains of microorganisms can be selected, microorganisms can be mutated, and mutants that lack virulence factors can be selected, and new strains of microorganisms with altered cell wall lipopolysaccharides can be constructed. In certain embodiments, the microorganisms can be attenuated by the deletion or disruption of DNA sequences which encode for virulence factors which insure survival of the microorganisms in the host cell, especially macrophages and neutrophils, by, for example, homologous recombination techniques and chemical or transposon mutagenesis. Many, but not all, of these studied virulence factors are associated with survival in macrophages such that these factors are specifically expressed within macrophages due to stress, for example, acidification, or are used to induced specific host cell responses, for example, macropinocytosis, Fields et al., 1986, Proc. Natl. Acad. Sci. USA 83:5189-5193. Bacterial virulence factors include, for example: cytolysin; defensin resistance loci; DNA K; fimbriae; GroEL; inv loci; lipoprotein; LPS; lysosomal fusion inhibition; macrophage survival loci; oxidative stress response loci; pho loci (*e.g*., PhoP and PhoQ ); pho activated genes (pag; *e.g*., pagB and pagC); phoP and phoQ regulated genes (prg); porins; serum resistance peptide; virulence plasmids (such as spvB, traT and ty2).

Yet another method for the attenuation of the microorganisms is to modify substituents of the microorganism which are responsible for the toxicity of that microorganism. For example, lipopolysaccharide (LPS) or endotoxin is primarily responsible for the pathological effects of bacterial sepsis. The component of LPS which results in this response is lipid A (LA). Elimination or mitigation of the toxic effects of LA results in an attenuated bacteria since 1) the risk of septic shock in the patient would be reduced and 2) higher levels of the bacterial Eph receptor or Ephrin antigenic peptide expression vehicle could be tolerated.

*Rhodobacter* (Rhodopseudomonas) *sphaeroides* and *Rhodobacter capsulatus* each possess a monophosphoryl lipid A (MLA) which does not elicit a septic shock response in experimental animals and, further, is an endotoxin antagonist. Loppnow et al., 1990, Infect. Immun. 58:3743-3750; Takayma et al., 1989, Infect. Immun. 57:1336-1338. Gram negative bacteria other than *Rhodobacter* can be genetically altered to produce MLA, thereby reducing its potential of inducing septic shock.

Yet another example for altering the LPS of bacteria involves the introduction of mutations in the LPS biosynthetic pathway. Several enzymatic steps in LPS biosynthesis and the genetic loci controlling them in a number of bacteria have been identified, and several mutant bacterial strains have been isolated with genetic and enzymatic lesions in the LPS pathway. In certain embodiments, the LPS pathway mutant is a firA mutant. firA is the gene that encodes the enzyme UDP-3-O(R-30 hydroxymyristoyl)-glycocyamine N-acyltransferase, which regulates the third step in endotoxin biosynthesis (Kelley et al., 1993, J. Biol. Chem. 268:19866-19874).

As a method of insuring the attenuated phenotype and to avoid reversion to the non-attenuated phenotype, the bacteria may be engineered such that it is attenuated in more than one manner, *e.g*., a mutation in the pathway for lipid A production and one or more mutations to auxotrophy for one or more nutrients or metabolites, such as uracil biosynthesis, purine biosynthesis, and arginine biosynthesis.

The Eph receptor or Ephrin antigenic peptides are preferably expressed in a microorganism, such as bacteria, using a heterologous gene expression cassette. A heterologous gene expression cassette is typically comprised of the following ordered elements: (1) prokaryotic promoter; (2) Shine-Dalgarno sequence; (3) secretion signal (signal peptide); and, (4) heterologous gene. Optionally, the heterologous gene expression cassette may also contain a transcription termination sequence, in constructs for stable integration within the bacterial chromosome. While not required, inclusion of a transcription termination sequence as the final ordered element in a heterologous gene expression cassette may prevent polar effects on the regulation of expression of adjacent genes, due to read- through transcription.

The expression vectors introduced into the microorganism Eph receptor or Ephrin vaccines are preferably designed such that microorganism-produced Eph receptor or Ephrin peptides and, optionally, prodrug converting enzymes, are secreted by microorganism. A number of bacterial secretion signals are well known in the art and may be used in the compositions and methods of the present invention. In certain embodiments of the present invention, the bacterial Eph receptor or Ephrin antigenic peptide expression vehicles are engineered to be more susceptible to an antibiotic and/or to undergo cell death upon administration of a compound. In other embodiments of the present invention, the bacterial Eph receptor or Ephrin antigenic peptide expression vehicles are engineered to deliver suicide genes to the target Eph receptor- or Ephrin-expressing cells. These suicide genes include prodrug converting enzymes, such as Herpes simplex thymidine kinase (TK) and bacterial cytosine deaminase (CD). TK phosphorylates the non-toxic substrates acyclovir and ganciclovir, rendering them toxic via their incorporation into genomic DNA. CD converts the non-toxic 5-fluorocytosine (5-FC) into 5-fluorouracil (5-FU), which is toxic via its incorporation into RNA. Additional examples of pro-drug converting enzymes encompassed by the present invention include cytochrome p450 NADPH oxidoreductase which acts upon mitomycin C and porfiromycin (Murray et al., 1994, J. Pharmacol. Exp. Therapeut. 270:645-649). Other exemplary pro-drug converting enzymes that may be used include: carboxypeptidase; beta-glucuronidase; penicillin-V -amidase; penicillin-G-amidase; betalactamase; beta.-glucosidase; nitroreductase; and carboxypeptidase A.

Exemplary secretion signals that can be used with gram-positive microorganisms include SecA (Sadaie et al., 1991, Gene 98:101-105), SecY (Suh et al., 1990, Mol. Microbiol. 4:305-314), SecE (Jeong et al., 1993, Mol. Microbiol. 10:133-142), FtsY and FfH (PCT/NL 96/00278), and PrsA (International Publication No. WO 94/19471). Exemplary secretion signals that may be used with gram-negative microorganisms include those of soluble cytoplasmic proteins such as SecB and heat shock proteins; that of the peripheral membrane-associated protein SecA; and those of the integral membrane proteins SecY, SecE, SecD and SecF.

The promoters driving the expression of the Eph receptor or Ephrin antigenic peptides and, optionally, pro-drug converting enzymes, may be either constitutive, in which the peptides or enzymes are continually expressed, inducible, in which the peptides or enzymes are expressed only upon the presence of an inducer molecule(s), or cell-type specific control, in which the peptides or enzymes are expressed only in certain cell types. For example, a suitable inducible promoter can be a promoter responsible for the bacterial "SOS" response (Friedberg et al., In: DNA Repair and Mutagenesis, pp. 407-455, Am. Soc. Microbiol. Press, 1995). Such a promoter is inducible by numerous agents including chemotherapeutic alkylating agents such as mitomycin (Oda et al., 1985, Mutation Research 147:219-229; Nakamura et al., 1987, Mutation Res. 192:239-246; Shimda et al., 1994, Carcinogenesis 15:2523-2529) which is approved for use in humans. Promoter elements which belong to this group include umuC, sulA and others (Shinagawa et al., 1983, Gene 23:167-174; Schnarr et al., 1991, Biochemie 73:423-431). The sulA promoter includes the ATG of the sulA gene and the following 27 nucleotides as well as 70 nucleotides upstream of the ATG (Cole, 1983, Mol. Gen. Genet. 189:400-404). Therefore, it is useful both in expressing foreign genes and in creating gene fusions for sequences lacking initiating codons.

In certain embodiments, an Eph/Ephrin Modulator vaccine does not comprise a microorganism.

In a specific embodiment, an antigenic peptide is not EphA2₅₈ (IMNDMPIYM; SEQ ID NO:55) or is not EphA2₅₅₀ (VLAGVGFFI; SEQ ID NO:56). In another specific embodiment, an antigenic peptide is not (TLADFDPRV; SEQ ID NO:57), (VLLLVLAGV; SEQ ID NO:58), (VLAGVGFFI; SEQ ID NO:59), (IMNDMPIYM; SEQ ID NO:60), (SLLGLKDQV; SEQ ID NO:61), (WLVPIGQCL; SEQ ID NO:62), (LLWGCALAA; SEQ ID NO:63), GLTRTSVTV; SEQ ID NO:64), (NLYYAESDL; SEQ ID NO:65), or (KLNVEERSV; SEQ ID NO:66). In yet another sepcific embodiment, an antigenic peptide is not (IMGQFSHHN; SEQ ID NO:67), (YSVCNVMSG; SEQ ID NO:68), (MQNIMNDMP; SEQ ID NO:69), (EAGIMGQFSHHNIIR; SEQ ID NO:70), (PIYMYSVCNVMSG; SEQ ID NO:71) or (DLMQNIMNDMPIYMYS; SEQ ID NO:72).

### 5.2 PROPHYLACTIC/THERAPEUTIC METHODS

The present invention provides methods for treating, managing, preventing and/or ameliorating a disorder associated with aberrant (*i.e*., increased, decreased or inappropriate) Eph receptor and/or Ephrin expression and/or activity. Such disorders include, but are not limited, to non-neoplastic hyperproliferative disorders such as, for example, those disclosed in U.S. Pat. Appn. Ser. No. 10/823,254 (April 12, 2004), which is incorporated by reference herein in its entirety); hypoproliferative disorders such as, for example, those disclosed in U.S. Pat. Appn. Ser. No. 10/823,259 (April 12, 2004), which is incorporated by reference herein in its entirety); cancer (benign, malignant and metastatic forms of cancer); autoimmune disorders (*e.g*., graft versus host disease; "GVHD"); and inflammatory disorders, and pulmonary Langerhans cell histopcytosis ("PLCH"). In a specific embodiment, the Eph/Ephrin Modulator of the invention inhibits the production of VEGF. In another specific embodiment, the Eph/Ephrin Modulator inhibits the proliferation of cancer cells. In a further specific embodiment, the Eph/Ephrin Modulator inhibits the migration of cancer cells. In yet a further specific embodiment, the Eph/Ephrin Modulator inhibits the invasion of cancer cells. Other disorders to be treated, prevented, managed and/or ameliorated by the methods of the present invention include non-neoplastic hyperproliferative epithelial and/or endothelial cell disorders, including, but not limited to, disorders associated with increased deposition of extracellular matrix components (*e.g*., collagen, proteoglycans, tenascin and fibronectin) and/or aberrant (*i.e.,* increased) angiogenesis. Non-limiting examples of such disorders include cirrhosis, fibrosis (*e.g*., fibrosis of the liver, kidney, lungs, heart, retina and other viscera), asthma, ischemia, atherosclerosis, diabetic retinopathy, retinopathy of prematurity, vascular restenosis, macular degeneration, rheumatoid arthritis, osteoarthritis, infantile hemangioma, verruca vulgaris, Kaposi's sarcoma, neurofibromatosis, recessive dystrophic epidermolysis bullosa, ankylosing spondylitis, systemic lupus, Reiter's syndrome, Sjogren's syndrome, endometriosis, preeclampsia, atherosclerosis, coronary artery disease, psoriatic arthropathy and psoriasis, as disclosed in U.S. Provisional Application Serial No. 60/622,517, filed October 27, 2004, entitled "Modulators of EphA2 and EphrinA1 For The Treatment of Fibrosis-Related Disease", which is incorporated by reference herein in its entirety. Additional disorders to be treated, prevented, managed and/or ameliorated by the methods of the present invention include, but are not limited to, infections by pathogens such as viruses, bacteria, fungi and protozoa, as disclosed in U.S. Provisional Application No. 60/622,489, filed October 27, 2004, entitled, "Use of Modulators of EphA2 and EphrinA1 For The Treatment of Infections", which is incorporated by reference herein in its entirety. In a one embodiment, such pathogen infections are intracellular infections. In a specific embodiment, the intracellular pathogen is not respiratory syncytial virus (RSV). The methods of the present invention comprise the administration of an effective amount of one or more Eph/Ephrin Modulators. In another embodiment, such methods comprise the administration of an effective amount of one or more Eph/Ephrin Modulators in combination with one or more therapeutic or prophylactic agents that are not Eph/Ephrin Modulator-based.

The present invention also provides methods for treating, managing, preventing and/or ameliorating a disorder associated with aberrant (*i.e*., increased, decreased or inappropriate) Eph receptor and/or Ephrin expression, one or more Eph/Ephrin Modulators and one or more other therapies (see Section 5.2.2, *infra* for examples of such therapies). Preferably, such other therapies useful in the treatment, management, prevention and/or amelioration of a disorder associated with aberrant (*i.e*., increased, decreased or inappropriate) Eph receptor and/or Ephrin expression are used in combination with Eph/Ephrin Modulators in accordance with the invention.

The dosage amounts and frequencies of administration provided herein are encompassed by the terms "effective amount", "therapeutically effective" and "prophylactically effective". The dosage and frequency further will typically vary according to factors specific for each patient depending on the specific therapeutic or prophylactic agents administered, the severity and type of non-neoplastic hyperproliferative epithelial and/or endothelial cell disorder, the route of administration, as well as age, body weight, response, and the past medical history of the patient. Suitable regimens can be selected by one skilled in the art by considering such factors and by following, for example, dosages reported in the literature and recommended in the Physician's Desk Reference (56th ed., 2002). See Section 4.6.3 for specific dosage amounts and frequencies of administration of the prophylactic and therapeutic agents provided by the invention.

### 5.2.1 Patient Population

The present invention encompasses methods for treating, managing, or preventing a disorder associated with aberrant (*i.e*., increased, decreased or inappropriate) Eph receptor and/or Ephrin expression, or a symptom thereof, in a subject, which method comprises administering one or more Eph/Ephrin Modulators of the invention. The subject is preferably a mammal such as non-primate (*e.g*., cows, pigs, horses, cats, dogs, rats, etc.) or a primate (*e.g*., monkeys, such as cynomolgous monkeys or humans). In a preferred embodiment, the subject is a human.

The methods of the invention comprise the administration of one or more Eph/Ephrin Modulators of the invention to patients suffering from or expected to suffer from (*e.g*., patients with a genetic predisposition for or patients that have previously suffered from) a disorder associated with aberrant (*i.e*., increased, decreased or inappropriate) Eph receptor and/or Ephrin expression. Such patients may have been previously treated or are currently being treated for the disorder associated with aberrant (*i.e*., increased, decreased or inappropriate) Eph receptor and/or Ephrin expression, *e.g*., with a non-Eph/Ephrin Modulator therapy. In accordance with the invention, an Eph/Ephrin Modulator may be used as any line of therapy, including, but not limited to, a first, second, third and fourth line of therapy. Further, in accordance with the invention, an Eph/Ephrin Modulator can be used before any adverse effects or intolerance of the non-Eph/Ephrin Modulator therapies occur. The invention encompasses methods for administering one or more Eph/Ephrin Modulators of the invention to prevent the onset or recurrence of a disorder associated with aberrant (*i.e*., increased, decreased or inappropriate) Eph receptor and/or Ephrin expression.

In one embodiment, the invention also provides methods of treatment or management of a disorder associated with aberrant (*i.e*., increased, decreased or inappropriate) Eph receptor and/or Ephrin expression as alternatives to current therapies. In a specific embodiment, the current therapy has proven or may prove too toxic (*i.e*., results in unacceptable or unbearable side effects) for the patient. In another embodiment, an Eph/Ephrin Modulator decreases the side effects as compared to the current therapy. In another embodiment, the patient has proven refractory to a current therapy. In certain embodiments, one or more Eph/Ephrin Modulators of the invention can be administered to a patient in need thereof instead of another therapy to treat a disorder associated with aberrant (*i.e*., increased, decreased or inappropriate) Eph receptor and/or Ephrin expression.

The present invention also encompasses methods for administering one or more Eph/Ephrin Modulators of the invention to treat or ameliorate symptoms of a disorder associated with aberrant (*i.e*., increased, decreased or inappropriate) Eph receptor and/or Ephrin expression in patients that are or have become refractory to non-Eph/Ephrin Modulator therapies. The determination of whether the symptoms are refractory can be made either *in vivo* or *in vitro* by any method known in the art for assaying the effectiveness of a therapy on affected cells in the a disorder associated with aberrant (*i.e*., increased, decreased or inappropriate) Eph receptor and/or Ephrin expression, particularly epithelial and/or endothelial cells, or in patients that are or have become refractory to non-Eph/Ephrin Modulator therapies.

### 5.2.2 Other Prophylactic/Therapeutic Agents

The invention provides methods for treating, managing, preventing and/or ameliorating a a disorder associated with aberrant (*i.e*., increased, decreased or inappropriate) Eph receptor and/or Ephrin expression by administering one or more Eph/Ephrin Modulators of the invention in combination with one or more therapies. Preferably, those other therapies are currently being used or are useful in the treatment, management or prevention of a a disorder associated with aberrant (*i.e*., increased, decreased or inappropriate) Eph receptor and/or Ephrin expression. In a specific embodiment, the invention provides a method of treating, managing, preventing and/or ameliorating a disorder associated with aberrant (*i.e*., increased, decreased or inappropriate) Eph receptor and/or Ephrin expression, the method comprising administering to a subject in need thereof an effective amount of an Eph/Ephrin Modulator and an effective amount of a therapy other than an Eph/Ephrin Modulator. Any therapy (*e.g*., prophylactic or therapeutic agents) which is known to be useful, or which has been used or is currently being used for the prevention, management, treatment or amelioration of a disorder associated with aberrant (*i.e*., increased, decreased or inappropriate) Eph receptor and/or Ephrin expression or a symptom thereof can be used in combination with an Eph/Ephrin Modulator in accordance with the invention described herein. See, *e.g*., Gilman et al., Goodman and Gilman's: The Pharmacological Basis of Therapeutics, Tenth Ed., McGraw-Hill, New York, 2001; The Merck Manual of Diagnosis and Therapy, Berkow, M.D. et al. (eds.), 17th Ed., Merck Sharp & Dohme Research Laboratories, Rahway, NJ, 1999; and Cecil Textbook of Medicine, 20th Ed., Bennett and Plum (eds.), W.B. Saunders, Philadelphia, 1996, for information regarding therapies, in particular prophylactic or therapeutic agents, which have been or are currently being used for preventing, treating, managing, and/or ameliorating a disorder associated with aberrant (*i.e*., increased, decreased or inappropriate) Eph receptor and/or Ephrin expression or a symptom thereof. Therapeutic or prophylactic agents include, but are not limited to, small molecules, synthetic drugs, peptides, polypeptides, proteins, nucleic acids, (*e.g*., DNA and RNA nucleotides including, but not limited to, antisense nucleotide sequences, triple helices, RNAi, and nucleotide sequences encoding biologically active proteins, polypeptides or peptides) antibodies, synthetic or natural inorganic molecules, mimetic agents, and synthetic or natural organic molecules. Examples of prophylactic and therapeutic agents include, but are not limited to, immunomodulatory agents, anti-angiogenic agents, TNF-α antagonists, anti-inflammatory agents (*e.g*., adrenocorticoids, corticosteroids, (*e.g*., beclomethasone, budesonide, flunisolide, fluticasone, triamcinolone, methylprednisolone, prednisolone, prednisone, hydrocortisone), glucocorticoids, steroids, and non-steroidal anti-inflammatory drugs (*e.g*., aspirin, ibuprofen, diclofenac, and COX-2 inhibitors), anticholinergic agents (*e.g*., ipratropium bromide and oxitropium bromide), sulphasalazine, penicillamine, dapsone, antihistamines, anti-malarial agents (*e.g*., hydroxychloroquine), anti-viral agents, and antibiotics (*e.g*., dactinomycin (formerly actinomycin), bleomycin, erythromycin, penicillin, mithramycin, and anthramycin (AMC)), anti-cancer therapies, anti-viral agents, anti-bacterial agents, and anti-fungal agents.

In one embodiment, an Eph/Ephrin Modulator of the invention is administered to a subject in need thereof in combination with a therapy currently used or known to treat, manage, prevent and/or ameliorate a disorder associated with aberrant (*i.e*., increased, decreased or inappropriate) Eph receptor and/or Ephrin expression. In another embodiment, an Eph/Ephrin Modulator of the invention is administered to a subject in need thereof in combination with an immunomodulatory agent. In another embodiment, an Eph/Ephrin Modulator of the invention is administered to a subject in need thereof in combination with an anti-angiogenic agent. In another embodiment, an Eph/Ephrin Modulator of the invention is administered to a subject in need thereof in combination with a TNF-α antagonist. In yet another embodiment, an Eph/Ephrin Modulator of the invention is administered to a subject in need thereof in combination with an anti-cancer agent. The therapies can be administered to a subject in need thereof sequentially or concurrently. In particular, the therapies should be administered to a subject at exactly the same time or in a sequence within a time interval such that the therapies can act together to provide an increased benefit than if they were administered otherwise. In a specific embodiment, the combination therapies of the invention comprise an effective amount of one or more Eph/Ephrin Modulators of the invention and an effective amount of at least one other therapy which has the same mechanism of action as said Eph/Ephrin Modulators of the invention. In a specific embodiment, the combination therapies of the invention comprise an effective amount of one or more Eph/Ephrin Modulators of the invention and an effective amount of at least one other therapy (*e.g*., prophylactic or therapeutic agent) which has a different mechanism of action than said Eph/Ephrin Modulators of the invention. In certain embodiments, the combination therapies of the present invention improve the prophylactic or therapeutic effect of one or more antibodies of the invention by functioning together with the Eph/Ephrin Modulators of the invention to have an additive or synergistic effect. In certain embodiments, the combination therapies of the present invention reduce the side effects associated with the prophylactic or therapeutic agents. In various embodiments, the therapies are administered to a patient less than 1 hour apart, at about 1 hour apart, at about 1 hour to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, a about 8 hours to about 9 hours apart, at about 9 hours to about 10 hours apart, at about 10 hours to about 11 hours apart, at about 11 hours to about 12 hours apart, no more than 24 hours apart or no more than 48 hours apart. In preferred embodiments, two or more therapies are administered within the same patient visit.

The prophylactic or therapeutic agents of the combination therapies can be administered to a subject, preferably a human subject, in the same pharmaceutical composition. Alternatively, the prophylactic or therapeutic agents of the combination therapies can be administered concurrently to a subject in separate pharmaceutical compositions. The prophylactic or therapeutic agents may be administered to a subject by the same or different routes of administration.

In a specific embodiment, a pharmaceutical composition comprising one or more Eph/Ephrin Modulators of the invention described herein is administered to a subject, preferably a human, to prevent, treat, manage and/or ameliorate a disorder associated with aberrant (*i.e*., increased, decreased or inappropriate) Eph receptor and/or Ephrin expression. In accordance with the invention, pharmaceutical compositions of the invention may also comprise one or more therapies (*e.g*., prophylactic or therapeutic agents), other than the Eph/Ephrin Modulators of the invention, which are currently being used, have been used, or are known to be useful in the prevention, treatment or amelioration of one or more symptoms associated with a disorder associated with aberrant (*i.e.,* increased, decreased or inappropriate) Eph receptor and/or Ephrin expression.

### 5.2.2.1 Immunomodulatory Agents

In certain embodiments, the present invention provides compositions comprising one or more Eph/Ephrin Modulators of the invention and one or more immunomodulatory agents (*i.e*., agents which modulate the immune response in a subject), and methods for treating, managing, preventing or ameliorating a disorder associated with aberrant (*i.e*., increased, decreased or inappropriate) Eph receptor and/or Ephrin expression or a symptom thereof, in a subject comprising the administration of said compositions. The invention also provides methods for treating, managing, preventing or ameliorating a disorder associated with aberrant (*i.e*., increased, decreased or inappropriate) Eph receptor and/or Ephrin expression or a symptom thereof comprising the administration of an Eph/Ephrin Modulator in combination with one or more immunomodulatory agents. In a specific embodiment of the invention, the immunomodulatory agent inhibits or suppresses the immune response in a human subject. Immunomodulatory agents are well-known to one skilled in the art and can be used in the methods and compositions of the invention.

Any immunomodulatory agent well-known to one of skill in the art may be used in the methods and compositions of the invention. Immunomodulatory agents can affect one or more or all aspects of the immune response in a subject. Aspects of the immune response include, but are not limited to, the inflammatory response, the complement cascade, leukocyte and lymphocyte differentiation, proliferation, and/or effector function, monocyte and/or basophil counts, and the cellular communication among cells of the immune system. In certain embodiments of the invention, an immunomodulatory agent modulates one aspect of the immune response. In other embodiments, an immunomodulatory agent modulates more than one aspect of the immune response. In one embodiment of the invention, the administration of an immunomodulatory agent to a subject inhibits or reduces one or more aspects of the subject's immune response capabilities. In a specific embodiment of the invention, the immunomodulatory agent inhibits or suppresses the immune response in a subject. In accordance with the invention, an immunomodulatory agent is not antibody that specifically binds to an Eph receptor (*e.g*., EphA1, EphA2, EphA3, EphA4, EphA5, EphA6, EphA7, EphA8, EphA10, EphB1, EphB2, EphB3, EphB4, EphB5 or EphB6) or an Ephrin (*e.g*., EphrinA1, EphrinA2, EphrinA3, EphrinA4, EphrinA5, EphrinB1, EphrinB2 or EphrinB3) polypeptide. In certain embodiments, an immunomodulatory agent is not an anti-angiogenic agent. In certain embodiments, an immunomodulatory agent is not an anti-inflammatory agent. In other emobidments, an immunomodulatory agent is not a TNF-α antagonist. In certain embodiments, an immunomodulatory agent is not an anti-cancer agent.

Examples of immunomodulatory agents include, but are not limited to, proteinaceous agents such as cytokines, peptide mimetics, and antibodies (*e.g*., human, humanized, chimeric, monoclonal, polyclonal, Fvs, ScFvs, Fab or F(ab)2 fragments or epitope binding fragments), nucleic acid molecules (*e.g*., antisense nucleic acid molecules and triple helices), small molecules, organic compounds, and inorganic compounds. In particular, immunomodulatory agents include, but are not limited to, methotrexate, leflunomide, cyclophosphamide, cytoxan, Immuran, cyclosporine A, minocycline, azathioprine, antibiotics (*e.g*., FK506 (tacrolimus)), methylprednisolone (MP), corticosteroids, steroids, mycophenolate mofetil, rapamycin (sirolimus), mizoribine, deoxyspergualin, brequinar, malononitriloamindes (*e.g*., leflunamide), T cell receptor modulators, cytokine receptor modulators, and modulators mast cell modulators.

Examples of T cell receptor modulators include, but are not limited to, anti-T cell receptor antibodies (*e.g*., anti-CD4 antibodies (*e.g*., cM-T412 (Boeringer), IDEC-CE9.1® (IDEC and SKB), mAB 4162W94, Orthoclone and OKTcdr4a (Janssen-Cilag)), anti-CD3 antibodies (*e.g*., Nuvion (Product Design Labs), OKT3 (Johnson & Johnson), or Rituxan (IDEC)), anti-CD5 antibodies (*e.g*., an anti-CD5 ricin-linked immunoconjugate), anti-CD7 antibodies (*e.g*., CHH-380 (Novartis)), anti-CD8 antibodies, anti-CD40 ligand monoclonal antibodies (*e.g*., IDEC-131 (IDEC)), anti-CD52 antibodies (*e.g.,* CAMPATH 1H (Ilex)), anti-CD2 antibodies (*e.g*., siplizumab (MedImmune, Inc., International Publication Nos. WO 02/098370 and WO 02/069904)), anti-CD11a antibodies (*e.g*., Xanelim (Genentech)), and anti-B7 antibodies (*e.g*., IDEC-114) (IDEC))), CTLA4-immunoglobulin, and LFA-3TIP (Biogen, International Publication No. WO 93/08656 and U.S. Patent No. 6,162,432).

Examples of cytokine receptor modulators include, but are not limited to, soluble cytokine receptors (*e.g*., the extracellular domain of a TNF-α receptor or a fragment thereof, the extracellular domain of an IL-1β receptor or a fragment thereof, and the extracellular domain of an IL-6 receptor or a fragment thereof), cytokines or fragments thereof (*e.g*., interleukin IL-2, IL-3, IL-4, IL-5, IL-6, IL-7, IL-8, IL-9, IL-10, IL-11, IL-12, IL-13, IL-15, IL-23, TNF-α, TNF-β, interferon (IFN)-α, IFN-β, IFN-γ, and GM-CSF), anti-cytokine receptor antibodies (*e.g*., anti-IFN receptor antibodies, anti-IL-2 receptor antibodies (*e.g*., Zenapax (Protein Design Labs)), anti-IL-3 receptor antibodies, anti-IL-4 receptor antibodies, anti-IL-6 receptor antibodies, anti-IL-10 receptor antibodies, anti-IL-12 receptor antibodies, anti-IL-13 receptor antibodies, anti-IL-15 receptor antibodies, and anti-IL-23 receptor antibodies), anti-cytokine antibodies (*e.g*., anti-IFN antibodies, anti-TNF-α antibodies, anti-IL-1β antibodies, anti-IL-3 antibodies, anti-IL-6 antibodies, anti-IL-8 antibodies (*e.g*., ABX-IL-8 (Abgenix)), anti-IL-12 antibodies, anti-IL-13 antibodies, anti-IL-15 antibodies, and anti-IL-23 antibodies).

In a specific embodiment, a cytokine receptor modulator is IL-3, IL-4, IL-10, or a fragment thereof. In another embodiment, a cytokine receptor modulator is an anti-IL-1β antibody, anti-IL-6 antibody, anti-IL-12 receptor antibody, or anti-TNF-α antibody. In another embodiment, a cytokine receptor modulator is the extracellular domain of a TNF-α receptor or a fragment thereof. In certain embodiments, a cytokine receptor modulator is not a TNF-α antagonist.

In one embodiment, a cytokine receptor modulator is a mast cell modulator. In an alternative embodiment, a cytokine receptor modulator is not a mast cell modulator. Examples of mast cell modulators include, but are not limited to stem cell factor (c-kit receptor ligand) inhibitors (*e.g*., mAb 7H6, mAb 8H7a, pAb 1337, FK506, CsA, dexamthasone, and fluconcinonide), c-kit receptor inhibitors (*e.g*., STI 571 (formerly known as CGP 57148B)), mast cell protease inhibitors (*e.g*., GW-45, GW-58, wortmannin, LY 294002, calphostin C, cytochalasin D, genistein, KT5926, staurosproine, and lactoferrin), relaxin ("RLX"), IgE antagonists (*e.g*., antibodies rhuMAb-E25 omalizumab, HMK-12 and 6HD5, and mAB Hu-901), IL-3 antagonists, IL-4 antagonists, IL-10 antagonists, and TGF-beta.

An immunomodulatory agent may be selected to interfere with the interactions between the T helper subsets (TH1 or TH2) and B cells to inhibit neutralizing antibody formation. Antibodies that interfere with or block the interactions necessary for the activation of B cells by TH (T helper) cells, and thus block the production of neutralizing antibodies, are useful as immunomodulatory agents in the methods of the invention. For example, B cell activation by T cells requires certain interactions to occur (Durie et al., Immunol. Today, 15(9):406-410 (1994)), such as the binding of CD40 ligand on the T helper cell to the CD40 antigen on the B cell, and the binding of the CD28 and/or CTLA4 ligands on the T cell to the B7 antigen on the B cell. Without both interactions, the B cell cannot be activated to induce production of the neutralizing antibody.

The CD40 ligand (CD40L)-CD40 interaction is a desirable point to block the immune response because of its broad activity in both T helper cell activation and function as well as the absence of redundancy in its signaling pathway. Thus, in a specific embodiment of the invention, the interaction of CD40L with CD40 is transiently blocked at the time of administration of one or more of the immunomodulatory agents. This can be accomplished by treating with an agent which blocks the CD40 ligand on the TH cell and interferes with the normal binding of CD40 ligand on the T helper cell with the CD40 antigen on the B cell. An antibody to CD40 ligand (anti-CD40L) (available from Bristol-Myers Squibb Co; see, *e.g*., European patent application 555,880, published Aug. 18, 1993) or a soluble CD40 molecule can be selected and used as an immunomodulatory agent in accordance with the methods of the invention.

An immunomodulatory agent may be selected to inhibit the interaction between TH1 cells and cytotoxic T lymphocytes ("CTLs") to reduce the occurrence of CTL-mediated killing. An immunomodulatory agent may be selected to alter (*e.g*., inhibit or suppress) the proliferation, differentiation, activity and/or function of the CD4⁺ and/or CD8⁺ T cells. For example, antibodies specific for T cells can be used as immunomodulatory agents to deplete, or alter the proliferation, differentiation, activity and/or function of CD4⁺ and/or CD8⁺ T cells.

In one embodiment of the invention, an immunomodulatory agent that reduces or depletes T cells, preferably memory T cells, is administered to a subject at risk of or with a disorder associated with or characterized by aberrant expression and/or activity of an IL-9 polypeptide, a disorder associated with or characterized by aberrant expression of an IL-9R or one or more subunits thereof, an autoimmune disease, an inflammatory disease, a proliferative disease, or an infection (preferably, a respiratory infection) in accordance with the methods of the invention. See, *e.g*., U.S. Pat. No. 4,658,019. In another embodiment of the invention, an immunomodulatory agent that inactivates CD8⁺ T cells is administered to a subject at risk of or with non-neoplastic hyperproliferative epithelial and/or endothelial cell disorder in accordance with the methods of the invention. In a specific embodiment, anti-CD8 antibodies are used to reduce or deplete CD8⁺ T cells.

In another embodiment, an immunomodulatory agent which reduces or inhibits one or more biological activities (*e.g*., the differentiation, proliferation, and/or effector functions) of TH0, TH1, and/or TH2 subsets of CD4⁺ T helper cells is administered to a subject at risk of or with a non-neoplastic hyperproliferative epithelial and/or endothelial cell disorder in accordance with the methods of the invention. One example of such an immunomodulatory agent is IL-4. IL-4 enhances antigen-specific activity of TH2 cells at the expense of the TH1 cell function (see, *e.g*., Yokota et al, 1986 Proc. Natl. Acad. Sci., USA, 83:5894-5898; and U.S. Pat. No. 5,017,691). Other examples of immunomodulatory agents that affect the biological activity (*e.g*., proliferation, differentiation, and/or effector functions) of T-helper cells (in particular, TH1 and/or TH2 cells) include, but are not limited to, IL-2, IL-4, IL-5, IL-6, IL-10, IL-12, IL-13, IL-15, IL-23, and interferon (IFN)-γ.

In another embodiment, an immunomodulatory agent administered to a subject at risk of or with a non-neoplastic hyperproliferative epithelial and/or endothelial cell disorder in accordance with the methods of the invention is a cytokine that prevents antigen presentation. In a specific embodiment, an immunomodulatory agent used in the methods of the invention is IL-10. IL-10 also reduces or inhibits macrophage action which involves bacterial elimination.

An immunomodulatory agent may be selected to reduce or inhibit the activation, degranulation, proliferation, and/or infiltration of mast cells. In certain embodiments, the immunomodulatory agent interferes with the interactions between mast cells and mast cell activating agents, including, but not limited to stem cell factors (c-kit ligands), IgE, IL-4, environmental irritants, and infectious agents. In a specific embodiment, the immunomodulatory agent reduces or inhibits the response of mast cells to environmental irritants such as, but not limited to pollen, dust mites, tobacco smoke, and/or pet dander. In another specific embodiment, the immunomodulatory agent reduces or inhibits the response of mast cells to infectious agents, such as viruses, bacteria, and fungi. Examples of mast cell modulators that reduce or inhibit the activation, degranulation, proliferation, and/or infiltration of mast cells include, but are not limited to, stem cell factor (c-kit receptor ligand) inhibitors (*e.g*., mAb 7H6, mAb 8H7a, and pAb 1337 (see Mendiaz et al., 1996, Eur J Biochem 293(3):842-849), FK506 and CsA (Ito et al., 1999 Arch Dermatol Res 291(5):275-283), dexamthasone and fluconcinonide (see Finooto et al., 1997, J. Clin. Invest. 99(7):1721-1728)), c-kit receptor inhibitors (*e.g*., STI 571 (formerly known as CGP 57148B) (see Heinrich et al., 2000 Blood 96(3):925-932)), mast cell protease inhibitors (*e.g*., GW-45 and GW-58 (see, Temkin et al., 2002, J Immunol 169(5):2662-2669), wortmannin, LY 294002, calphostin C,and cytochalasin D (see Vosseller et al., 1997, Mol Biol Cell 1997:909-922), genistein, KT5926, and staurosproine (see Nagai et al. 1995, Biochem Biophys Res Commun 208(2):576-581), and lactoferrin (see He et al., 2003 Biochem Pharmacol 65(6):1007-1015)), relaxin ("RLX") (see Bani et al., 2002 Int Immunopharmacol 2(8):1195-1294),), IgE antagonists (*e.g*., antibodies rhuMAb-E25 omalizumab (see Finn et al., 2003 J Allergy Clin Immuno 111(2):278-284; Corren et al., 2003 J Allergy Clin Immuno 111(1):87-90; Busse and Neaville, 2001 Curr Opin Allergy Clin Immunol. 1(1):105-108; and Tang and Powell, 2001, Eur J Pediatr 160(12): 696-704), HMK-12 and 6HD5 (see Miyajima et al., 2202 Int Arch Allergy Immuno 128(1):24-32), and mAB Hu-901 (see van Neerven et al., 2001 Int Arch Allergy Immuno 124(1-3):400), IL-3 antagonist, IL-4 antagonists, IL-10 antagonists, and TGF-beta (see Metcalfe et al., 1995, Exp Dermatol 4(4 Pt 2):227-230).

In one embodiment, proteins, polypeptides or peptides (including antibodies) that are utilized as immunomodulatory agents are derived from the same species as the recipient of the proteins, polypeptides or peptides so as to reduce the likelihood of an immune response to those proteins, polypeptides or peptides. In another embodiment, when the subject is a human, the proteins, polypeptides, or peptides that are utilized as immunomodulatory agents are human or humanized.

In accordance with the invention, one or more immunomodulatory agents are administered to a subject at risk of or with a non-neoplastic hyperproliferative epithelial and/or endothelial cell disorder prior to, subsequent to, or concomitantly with an antibody that specifically binds to an Eph receptor or an Ephrin polypeptide. In one embodiment, one or more immunomodulatory agents are administered in combination with an antibody that specifically binds to an Eph receptor or an Ephrin polypeptide to a subject at risk of or with a disorder associated with aberrant (*i.e*., increased, decreased or inappropriate) Eph receptor and/or Ephrin expression to reduce or inhibit one or more aspects of the immune response as deemed necessary by one of skill in the art. Any technique well-known to one skilled in the art can be used to measure one or more aspects of the immune response in a particular subject, and thereby determine when it is necessary to administer an immunomodulatory agent to said subject. In another embodiment, a mean absolute lymphocyte count of approximately 500 cells/mm³, 600 cells/mm³, 650 cells/mm³, 700 cells/mm³, 750 cells/mm³, 800 cells/mm³, 900 cells/mm³, 1000 cells/mm³, 1100 cells/mm³, or 1200 cells/mm³ is maintained in a subject. In another embodiment, a subject at risk of or with a disorder associated with aberrant (*i.e*., increased, decreased or inappropriate) Eph receptor and/or Ephrin expression is not administered an immunomodulatory agent if their absolute lymphocyte count is 500 cells/mm³ or less, 550 cells/mm³ or less, 600 cells/mm³ or less, 650 cells/mm³ or less, 700 cells/mm³ or less, 750 cells/mm³ or less, or 800 cells/mm³ or less.

In one embodiment, one or more immunomodulatory agents are administered in combination with an antibody that specifically binds to an Eph receptor or Ephrin polypeptide to a subject at risk of or with a disorder associated with aberrant (*i.e*., increased, decreased or inappropriate) Eph receptor and/or Ephrin expression so as to transiently reduce or inhibit one or more aspects of the immune response. Such a transient inhibition or reduction of one or more aspects of the immune system can last for hours, days, weeks, or months. In one embodiment, the transient inhibition or reduction in one or more aspects of the immune response lasts for a few hours (*e.g*., 2 hours, 4 hours, 6 hours, 8 hours, 12 hours, 14 hours, 16 hours, 18 hours, 24 hours, 36 hours, or 48 hours), a few days (*e.g*., 3 days, 4 days, 5 days, 6 days, 7 days, or 14 days), or a few weeks (*e.g*., 3 weeks, 4 weeks, 5 weeks or 6 weeks). The transient reduction or inhibition of one or more aspects of the immune response enhances the prophylactic and/or therapeutic effect(s) of an antibody that specifically binds to an Eph receptor or an Ephrin polypeptide.

Nucleic acid molecules encoding proteins, polypeptides, or peptides with immunomodulatory activity or proteins, polypeptides, or peptides with immunomodulatory activity can be administered to a subject at risk of or with a non-neoplastic hyperproliferative epithelial and/or endothelial cell disorder in accordance with the methods of the invention. Further, nucleic acid molecules encoding derivatives, analogs, or fragments of proteins, polypeptides, or peptides with immunomodulatory activity, or derivatives, analogs, or fragments of proteins, polypeptides, or peptides with immunomodulatory activity can be administered to a subject at risk of or with a non-neoplastic hyperproliferative epithelial and/or endothelial cell disorder in accordance with the methods of the invention. In one embodiment, such derivatives, analogs, and fragments retain the immunomodulatory activity of the full-length, wild-type protein, polypeptide, or peptide.

Immunomodulatory agents can affect one or more or all aspects of the immune response in a subject. Aspects of the immune response include, but are not limited to, the inflammatory response, the complement cascade, leukocyte and lymphocyte proliferation, monocyte and/or basophil counts, and cellular communication among cells of the immune system. In certain embodiments of the invention, an immunomodulatory agent modulates one aspect of the immune response. In other embodiments, an immunomodulatory agent modulates more than one aspect of the immune response. In another embodiment of the invention, the administration of an immunomodulatory agent to a subject inhibits or reduces one or more aspects of the subject's immune response capabilities.

In accordance with the invention, one or more immunomodulatory agents can be administered to a subject with a disorder associated with aberrant (*i.e*., increased, decreased or inappropriate) Eph receptor and/or Ephrin expression, prior to, subsequent to, or concomitantly with an Eph/Ephrin Modulator of the invention. In one embodiment, one or more immunomodulatory agents are administered to a subject with a disorder associated with aberrant (*i.e*., increased, decreased or inappropriate) Eph receptor and/or Ephrin expression to reduce or inhibit one or more aspects of the immune response as necessary. Any technique well-known to one skilled in the art can be used to measure one or more aspects of the immune response, and thereby determine when it is necessary to administer an immunomodulatory agent. In another embodiment, one or more immunomodulatory agents are administered to a subject with a disorder associated with aberrant (*i.e*., increased, decreased or inappropriate) Eph receptor and/or Ephrin expression, so as to transiently reduce or inhibit one or more aspects of the immune response. Such a transient inhibition or reduction of one or more aspects of the immune system can last for hours, days, weeks, or months. The transient reduction or inhibition of one or more aspects of the immune response potentiates the therapeutic effect of the Eph/Ephrin Modulator of the invention.

In other embodiments, other immunomodulatory agents which can be used in the compositions and methods of the invention can be those that are commercially available and known to function as immunomodulatory agents. The immunomodulatory agents include, but are not limited to, agents such as cytokines, antibodies (*e.g*., human, humanized, chimeric, monoclonal, polyclonal, Fvs, scFvs, Fab or F(ab)₂ fragments or epitope binding fragments), inorganic compounds, or peptide mimetics. Further examples of immunomodulatory agents include, but are not limited to, anti-IL-13 monoclonal antibodies, anti-IL-4 monoclonal antibodies, anti-IL-5 monoclonal antibodies, anti-IL-2R antibodies (*e.g*., anti-Tac monoclonal antibody and BT 536), anti-CD4 monoclonal antibodies, anti-CD3 monoclonal antibodies, the anti-CD3 monoclonal human antibody OKT3, anti-CD8 monoclonal antibodies, anti-CD40 ligand monoclonal antibodies, anti-CD2 monoclonal antibodies (*e.g*., International Patent Publication No. WO 02/070007 published September 12, 2002), CTLA4-immunoglobulin, cyclophosphamide, cyclosporine A, macrolide antibiotics (*e.g*., FK506 (tacrolimus)), methylprednisolone (MP), corticosteroids, mycophenolate mofetil, rapamycin (sirolimus), mizoribine, deoxyspergualin, brequinar, malononitriloamindes.(*e.g*., leflunamide), beta 2-agonists, leukotriene antagonists, and agents that decrease IgE levels.

In one embodiment, the immunomodulatory agent decreases the amount of IL-9. In another embodiment, the immunomodulatory agent is an antibody (*e.g*. a monoclonal antibody) or fragment thereof that specifically binds to IL-9 (see, *e.g*., U.S. Patent Application No. 10/823,810, filed April 12, 2004 entitled "Methods of Preventing or Treating Respiratory Conditions" by Reed, U.S. Patent Application No. 10/823,523 filed April 12, 2004 entitled "Recombinant IL-9 Antibodies and Uses Thereof" by Reed, and U.S. Provisional Application No. 60/561,845 filed April 12, 2004 entitled "Anti-IL-9 Antibody Formulations and Uses Thereof' by Reed, all of which are incorporated by reference herein in their entireties. Although not intending to be bound by a particular mechanism of action, the use of anti-IL-9 antibodies neutralize the ability of IL-9 to have a biological effect and thereby blocks or decreases inflammatory cell recruitment.

The immunomodulator activity of an immunomodulatory agent can be determined *in vitro* and/or *in vivo* by any technique well-known to one skilled in the art, including, *e.g*., by CTL assays, proliferation assays, immunoassays (e.g. ELISAs) for the expression of particular proteins such as co-stimulatory molecules and cytokines, and FACS.

### 5.2.2.2 Anti-Inflammatory Therapies

In certain embodiments, the present invention provides compositions comprising one or more Eph/Ephrin Modulators of the invention and one or more anti-inflammatory agents, and methods for treating, managing, preventing or ameliorating a disorder associated with aberrant (*i.e*., increased, decreased or inappropriate) Eph receptor and/or Ephrin expression in a subject comprising the administration of said compositions. In a particular embodiment, such disorders are inflammatory disorders (*e.g*., rheumatoid arthritis). Any anti-inflammatory agent, including agents useful in therapies for inflammatory disorders, well-known to one of skill in the art can be used in the compositions and methods of the invention. Non-limiting examples of anti-inflammatory agents include non-steroidal anti-inflammatory drugs (NSAIDs), steroidal anti-inflammatory drugs, anticholinergics (*e.g*., atropine sulfate, atropine methylnitrate, and ipratropium bromide (ATROVENT™)), beta2-agonists (*e.g*., abuterol (VENTOLIN™ and PROVENTIL™), bitolterol (TORNALATE™), levalbuterol (XOPONEX™), metaproterenol (ALUPENT™), pirbuterol (MAXAIR™), terbutlaine (BRETHAIRE™ and BRETHINE™), albuterol (PROVENTIL™, REPETABS™, and VOLMAX™), formoterol (FORADIL AEROLIZER™), and salmeterol (SEREVENT™ and SEREVENT DISKUS™)), and methylxanthines (*e.g*., theophylline (UNIPHYL™, THEO-DUR™, SLO-BID™, AND TEHO-42™)). Examples of NSAIDs include, but are not limited to, aspirin, ibuprofen, celecoxib (CELEBREX™), diclofenac (VOLTAREN™), etodolac (LODINE™), fenoprofen (NALFON™), indomethacin (INDOCIN™), ketoralac (TORADOL™), oxaprozin (DAYPRO™), nabumentone (RELAFEN™), sulindac (CLINORIL™), tolmentin (TOLECTIN™), rofecoxib (VIOX™), naproxen (ALEVE™, NAPROSYN™), ketoprofen (ACTRON™) and nabumetone (RELAFEN™). Such NSAIDs function by inhibiting a cyclooxgenase enzyme (*e.g*., COX-1 and/or COX-2). Examples of steroidal anti-inflammatory drugs include, but are not limited to, glucocorticoids, dexamethasone (DECADRON™), corticosteroids (*e.g*., methylprednisolone (MEDROL™), cortisone, hydrocortisone, prednisone (PREDNISONE™ and DELTASONE™), prednisolone (PRELONE™ and PEDIAPRED™), triamcinolone, azulfidine, and inhibitors of eicosanoids (*e.g*., prostaglandins, thromboxanes, and leukotrienes (*see* Table 8, *infra,* for non-limiting examples of leukotriene and typical dosages of such agents)).

In certain embodiments, the anti-inflammatory agent is an agent useful in the prevention, management, treatment, and/or amelioration of asthma or one or more symptoms thereof. Non-limiting examples of such agents include adrenergic stimulants (*e.g*., catecholamines (*e.g*., epinEphrine, isoproterenol, and isoetharine), resorcinols (*e.g.,* metaproterenol, terbutaline, and fenoterol), and saligenins (*e.g*., salbutamol)), adrenocorticoids, blucocorticoids, corticosteroids (*e.g*., beclomethadonse, budesonide, flunisolide, fluticasone, triamcinolone, methylprednisolone, prednisolone, and prednisone), other steroids, beta2-agonists (*e.g*., albtuerol, bitolterol, fenoterol, isoetharine, metaproterenol, pirbuterol, salbutamol, terbutaline, formoterol, salmeterol, and albutamol terbutaline), anti-cholinergics (*e.g*., ipratropium bromide and oxitropium bromide), IL-4 antagonists (including antibodies), IL-5 antagonists (including antibodies), IL-13 antagonists (including antibodies), PDE4-inhibitor, NF-Kappa-β inhibitor, VLA-4 inhibitor, CpG, anti-CD23, selectin antagonists (TBC 1269), mast cell protease inhibitors (*e.g*., tryptase kinase inhibitors (*e.g*., GW-45, GW-58, and genisteine), phosphatidylinositide-3' (PI3)-kinase inhibitors (*e.g*., calphostin C), and other kinase inhibitors (*e.g*., staurosporine) (see Temkin et al., 2002 J Immunol 169(5):2662-2669; Vosseller et al., 1997 Mol. Biol. Cell 8(5):909-922; and Nagai et al., 1995 Biochem Biophys Res Commun 208(2):576-581)), a C3 receptor antagonists (including antibodies), immunosuppressant agents (*e.g*., methotrexate and gold salts), mast cell modulators (*e.g*., cromolyn sodium (INTAL™) and nedocromil sodium (TILADE™)), and mucolytic agents (*e.g*., acetylcysteine)). In a specific embodiment, the anti-inflammatory agent is a leukotriene inhibitor (*e.g*., montelukast (SINGULAIR™), zafirlukast (ACCOLATE™), pranlukast (ONON™), or zileuton (ZYFLO™) (*see* Table 8)).

**Table 8. Leukotriene Inhibitors for Asthma Therapy**

| Leukotriene Modifier | Usual Daily Dosage |
|---|---|
| Montelukast (SINGULAIR™) | 4 mg for 2-5 years old |
| | 5 mg for 6 to 15 years old |
| | 10mg for 15 years and older |
| Zafirlukast (ACCOLATE™) | 10 mg b.i.d. for 5 to 12 years old twice daily |
| | 20 mg b.i.d. for 12 years or older twice daily |
| Pranlukast (ONON™) | Only avialable in Asia |
| Zyleuton (ZYFLO™) | 600 mg four times a day for 12 years and older |

In certain embodiments, the anti-inflammatory agent is an agent useful in preventing, treating, managing, and/or ameliorating allergies or one or more symptoms thereof. Non-limiting examples of such agents include antimediator drugs (*e.g*., antihistamine, *see* Table 9, *infra* for non-limiting examples of antihistamine and typical dosages of such agents), corticosteroids, decongestants, sympathomimetic drugs (*e.g*., α-adrenergic and β-adrenergic drugs), TNX901 (Leung et al., 2003, N Engl J Med 348(11):986-993), IgE antagonists (*e.g*., antibodies rhuMAb-E25 omalizumab (see Finn et al., 2003 J Allergy Clin Immuno 111(2):278-284; Corren et al., 2003 J Allergy Clin Immuno 111(1):87-90; Busse and Neaville, 2001 Curr Opin Allergy Clin Immuno 1(1):105-108; and Tang and Powell, 2001, Eur J Pediatr 160(12): 696-704), HMK-12 and 6HD5 (see Miyajima et al., 2202 Int Arch Allergy Immuno 128(1):24-32), and mAB Hu-901 (see van Neerven *et al.,* 2001 Int Arch Allergy Immuno 124(1-3):400), theophylline and its derivatives, glucocorticoids, and immunotherapies (*e.g*., repeated long-term injection of allergen, short course desensitization, and venom immunotherapy).

**Table 9. H₁ Antihistamines**

| **Chemical class and representative drugs** | **Usual daily dosage** |
|---|---|
| Ethanolamine | |
| Diphehydramine | 25-50 mg every 4-6 hours |
| Clemastine | 0.34-2.68 mg every 12 hours |
| Ethylenediamine | |
| Tripelennamine | 25-50 mg every 4-6 hours |
| Alkylamine | |
| Brompheniramine | 4 mg every 4-6 hours; or 8-12 mg of SR form every 8-12 hour |
| Chlorpheniramine | 4 mg every 4-6 hours; or 8-12 mg of SR form every 8-12 hour |
| Triprolidine (1.25 mg/5ml) | 2.5 mg every 4-6 hours |
| Phenothiazine | |
| Promethazine | 25 mg at bedtime |
| Piperazine | |
| Hydroxyzine | 25 mg every 6-8 hours |
| Piperidines | |
| Astemizole (nonsedating) | 10 mg/day |
| Azatadine | 1-2 mg every 12 hours |
| Cetirzine | 10 mg/day |
| Cyproheptadine | 4 mg every 6-8 hour |
| Fexofenadine (nonsedating) | 60 mg every 12 hours |
| Loratidine (nonsedating) | 10 mg every 24 hours |

Anti-inflammatory therapies and their dosages, routes of administration, and recommended usage are known in the art and have been described in such literature as the Physician's Desk Reference (57th ed., 2003).

### 5.2.2.3 Anti-Angiogenic Therapies

In certain embodiments, the present invention provides compositions comprising one or more Eph/Ephrin Modulators of the invention and one or more anti-angiogenic agents, and methods for treating, managing, preventing or ameliorating a disorder associated with aberrant (*i.e*., increased, decreased or inappropriate) Eph receptor and/or Ephrin expression in a subject comprising the administration of said compositions. Any anti-angiogenic agent well-known to one of skill in the art can be used in the compositions and methods of the invention.

Any anti-angiogenic agent well-known to one of skill in the art can be used in the compositions and methods of the invention. Non-limiting examples of anti-angiogenic agents include proteins, polypeptides, peptides, fusion proteins, antibodies (*e.g*., human, humanized, chimeric, monoclonal, polyclonal, Fvs, ScFvs, Fab fragments, F(ab)₂ fragments, and antigen-binding fragments thereof) such as antibodies that specifically bind to TNF-α, nucleic acid molecules (*e.g*., antisense molecules or triple helices), organic molecules, inorganic molecules, and small molecules that reduce or inhibit angiogenesis. In particular, examples of anti-angiogenic agents, include, but are not limited to, endostatin, angiostatin, apomigren, anti-angiogenic antithrombin III, the 29 kDa N-terminal and a 40 kDa C-terminal proteolytic fragments of fibronectin, a uPA receptor antagonist, the 16 kDa proteolytic fragment of prolactin, the 7.8 kDa proteolytic fragment of platelet factor-4, the anti-angiogenic 24 amino acid fragment of platelet factor-4, the anti-angiogenic factor designated 13.40, the anti-angiogenic 22 amino acid peptide fragment of thrombospondin I, the anti-angiogenic 20 amino acid peptide fragment of SPARC, RGD and NGR containing peptides, the small anti-angiogenic peptides of laminin, fibronectin, procollagen and EGF, integrin αᵥβ₃ antagonists, acid fibroblast growth factor (aFGF) antagonists, basic fibroblast growth factor (bFGF) antagonists, vascular endothelial growth factor (VEGF) antagonists (*e.g*., anti-VEGF antibodies (*e.g*., AVASTIN™ (Genentech)),VEGF receptor (VEGFR) antagonists (*e.g*., anti-VEGFR antibodies) and anti-integrin antagonists (*e.g*., REOPRO® (abciximab) (Centocor) which binds to theglycoprotein IIb/IIIa receptor on the platelets for the prevention of clot formation).

Non-limiting examples of anti-angiogenic agents include proteins, polypeptides, peptides, fusion proteins, antibodies (*e.g*., human, humanized, chimeric, monoclonal, polyclonal, Fvs, ScFvs, Fab fragments, F(ab)₂ fragments, and antigen-binding fragments thereof) such as antibodies that specifically bind to TNF-α, nucleic acid molecules (*e.g*., antisense molecules or triple helices), organic molecules, inorganic molecules, and small molecules that reduce or inhibit angiogenesis. In particular, examples of anti-angiogenic agents, include, but are not limited to, endostatin, angiostatin, apomigren, anti-angiogenic antithrombin III, the 29 kDa N-terminal and a 40 kDa C-terminal proteolytic fragments of fibronectin, a uPA receptor antagonist, the 16 kDa proteolytic fragment of prolactin, the 7.8 kDa proteolytic fragment of platelet factor-4, the anti-angiogenic 24 amino acid fragment of platelet factor-4, the anti-angiogenic factor designated 13.40, the anti-angiogenic 22 amino acid peptide fragment of thrombospondin I, the anti-angiogenic 20 amino acid peptide fragment of SPARC, RGD and NGR containing peptides, the small anti-angiogenic peptides of laminin, fibronectin, procollagen and EGF, integrin αᵥβ₃ antagonists, acid fibroblast growth factor (aFGF) antagonists, basic fibroblast growth factor (bFGF) antagonists, vascular endothelial growth factor (VEGF) antagonists (*e.g*., anti-VEGF antibodies), and VEGF receptor (VEGFR) antagonists (*e.g*., anti-VEGFR antibodies).

Examples of integrin αᵥβ₃ antagonists include, but are not limited to, proteinaceous agents such as non-catalytic metalloproteinase fragments, RGD peptides, peptide mimetics, fusion proteins, disintegrins or derivatives or analogs thereof, and antibodies that specifically bind to integrin αᵥβ₃, nucleic acid molecules, organic molecules, and inorganic molecules. Non-limiting examples of antibodies that specifically bind to integrin αᵥβ₃ include 11D2 (Searle), LM609 (Scripps), and VITAXIN™ (MedImmune, Inc.). Non-limiting examples of small molecule peptidometric integrin αᵥβ₃ antagonists include S836 (Searle) and S448 (Searle). Examples of disintegrins include, but are not limited to, Accutin. The invention also encompasses the use of any of the integrin αᵥβ₃ antagonists disclosed in the following U.S. Patents and International publications in the compositions and methods of the invention: U.S. Patent Nos. 5,149,780; 5,196,511; 5,204,445; 5,262,520; 5,306,620; 5,478,725; 5,498,694; 5,523,209; 5,578,704; 5,589,570; 5,652,109; 5,652,110; 5,693,612; 5,705,481; 5,753,230; 5,767,071; 5,770,565; 5,780,426; 5,817,457; 5,830,678; 5,849,692; 5,955,572; 5,985,278; 6,048,861; 6,090,944; 6,096,707; 6,130,231; 6,153,628; 6,160,099; and 6,171,588; and International Publication Nos. WO 95/22543; WO 98/33919; WO 00/78815; and WO 02/070007, each of which is incorporated herein by reference in its entirety. In one embodiment, the anti-angiogenic agent is VITAXIN™ (MedImmune, Inc.) or an antigen-binding fragment thereof

In a specific embodiment of the invention, an anti-angiogenic agent is endostatin. Naturally occurring endostatin consists of the C-terminal∼180 amino acids of collagen XVIII (cDNAs encoding two splice forms of collagen XVIII have GenBank Accession Nos. AF18081 and AF18082). In another embodiment of the invention, an anti-angiogenic agent is a plasminogen fragment (the coding sequence for plasminogen can be found in GenBank Accession Nos. NM_000301 and A33096). Angiostatin peptides naturally include the four kringle domains of plasminogen, kringle 1 through kringle 4. It has been demonstrated that recombinant kringle 1, 2 and 3 possess the anti-angiogenic properties of the native peptide, whereas kringle 4 has no such activity (Cao et al., 1996, J. Biol. Chem. 271:29461-29467). Accordingly, the angiostatin peptides comprises at least one and preferably more than one kringle domain selected from the group consisting of kringle 1, kringle 2 and kringle 3. In a specific embodiment, the anti-angiogenic peptide is the 40 kDa isoform of the human angiostatin molecule, the 42 kDa isoform of the human angiostatin molecule, the 45 kDa isoform of the human angiostatin molecule, or a combination thereof. In another embodiment, an anti-angiogenic agent is the kringle 5 domain of plasminogen, which is a more potent inhibitor of angiogenesis than angiostatin (angiostatin comprises kringle domains 1-4). In another embodiment of the invention, an anti-angiogenic agent is antithrombin III. Antithrombin III, which is referred to hereinafter as antithrombin, comprises a heparin binding domain that tethers the protein to the vasculature walls, and an active site loop which interacts with thrombin. When antithrombin is tethered to heparin, the protein elicits a conformational change that allows the active loop to interact with thrombin, resulting in the proteolytic cleavage of said loop by thrombin. The proteolytic cleavage event results in another change of conformation of antithrombin, which (i) alters the interaction interface between thrombin and antithrombin and (ii) releases the complex from heparin (Carrell, 1999, Science 285:1861-1862, and references therein). O'Reilly et al. (1999, Science 285:1926-1928) have discovered that the cleaved antithrombin has potent anti-angiogenic activity. Accordingly, in one embodiment, an anti-angiogenic agent is the anti-angiogenic form of antithrombin. In another embodiment of the invention, an anti-angiogenic agent is the 40 kDa and/or 29 kDa proteolytic fragment of fibronectin.

In another embodiment of the invention, an anti-angiogenic agent is a urokinase plasminogen activator (uPA) receptor antagonist. In one mode of the embodiment, the antagonist is a dominant negative mutant of uPA (see, *e.g*., Crowley et al., 1993, Proc. Natl. Acad. Sci. USA 90:5021-5025). In another mode of the embodiment, the antagonist is a peptide antagonist or a fusion protein thereof (Goodson et al., 1994, Proc. Natl. Acad. Sci. USA 91:7129-7133). In yet another mode of the embodiment, the antagonist is a dominant negative soluble uPA receptor (Min et al., 1996, Cancer Res. 56:2428-2433). In another embodiment of the invention, a therapeutic molecule of the invention is the 16 kDa N-terminal fragment of prolactin, comprising approximately 120 amino acids, or a biologically active fragment thereof (the coding sequence for prolactin can be found in GenBank Accession No. NM_000948). In another embodiment of the invention, an anti-angiogenic agent is the 7.8 kDa platelet factor-4 fragment. In another embodiment of the invention, a therapeutic molecule of the invention is a small peptide corresponding to the anti-angiogenic 13 amino acid fragment of platelet factor-4, the anti-angiogenic factor designated 13.40, the anti-angiogenic 22 amino acid peptide fragment of thrombospondin I , the anti-angiogenic 20 amino acid peptide fragment of SPARC, the small anti-angiogenic peptides of laminin, fibronectin, procollagen, or EGF, or small peptide antagonists of integrin αᵥβ₃ or the VEGF receptor. In another embodiment, the small peptide comprises an RGD or NGR motif. In certain embodiments, an anti-angiogenic agent is a TNF-α antagonist. In other embodiments, an anti-angiogenic agent is not a TNF-α antagonist.

Nucleic acid molecules encoding proteins, polypeptides, or peptides with anti-angiogenic activity, or proteins, polypeptides or peptides with anti-angiogenic activity can be administered to a subject at risk of or with a non-neoplastic hyperproliferative epithelial and/or endothelial cell disorder in accordance with the methods of the invention. Further, nucleic acid molecules encoding derivatives, analogs, fragments, or variants of proteins, polypeptides, or peptides with anti-angiogenic activity, or derivatives, analogs, fragments, or variants of proteins, polypeptides, or peptides with anti-angiogenic activity can be administered to a subject at risk of or with a non-neoplastic hyperproliferative epithelial and/or endothelial cell disorder in accordance with the methods of the invention. In one embodiment, such derivatives, analogs, variants, and fragments retain the anti-angiogenic activity of the full-length, wild-type protein, polypeptide, or peptide.

Proteins, polypeptides, or peptides that can be used as anti-angiogenic agents can be produced by any technique well-known in the art or described herein. Proteins, polypeptides or peptides with anti-angiogenic activity can be engineered so as to increase the *in vivo* half-life of such proteins, polypeptides, or peptides utilizing techniques well-known in the art or described herein. In one embodiment, anti-angiogenic agents that are commercially available are used in the compositions and methods of the invention. The anti-angiogenic activity of an agent can be determined *in vitro* and/or *in vivo* by any technique well-known to one skilled in the art.

### 5.2.2.4 TNF-α Antagonists

Any TNF-α antagonist well-known to one of skill in the art can be used in the compositions and methods of the invention. Non-limiting examples of TNF-α antagonists include proteins, polypeptides, peptides, fusion proteins, antibodies (*e.g*., human, humanized, chimeric, monoclonal, polyclonal, Fvs, ScFvs, Fab fragments, F(ab)₂ fragments, and antigen-binding fragments thereof) such as antibodies that specifically bind to TNF-α, nucleic acid molecules (*e.g*., antisense molecules or triple helices), organic molecules, inorganic molecules, and small molecules that blocks, reduces, inhibits or neutralizes a function, an activity and/or expression of TNF-α. In various embodiments, a TNF-α antagonist reduces the function, activity and/or expression of TNF-α by at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95% or at least 99% relative to a control such as phosphate buffered saline (PBS).

Examples of antibodies that specifically bind to TNF-α include, but are not limited to, infliximab (REMICADE®; Centacor), D2E7 (Abbott Laboratories/Knoll Pharmaceuticals Co., Mt. Olive, N.J.), CDP571 which is also known as HUMICADE™ and CDP-870 (both of Celltech/Pharmacia, Slough, U.K.), and TN3-19.12 (Williams et al., 1994, Proc. Natl. Acad. Sci. USA 91: 2762-2766; Thorbecke et al., 1992, Proc. Natl. Acad. Sci. USA 89:7375-7379). The present invention also encompasses the use of antibodies that specifically bind to TNF-α disclosed in the following U.S. Patents in the compositions and methods of the invention: 5,136,021; 5,147,638; 5,223,395; 5,231,024; 5,334,380; 5,360,716; 5,426,181; 5,436,154; 5,610,279; 5,644,034; 5,656,272; 5,658,746; 5,698,195; 5,736,138; 5,741,488; 5,808,029; 5,919,452; 5,958,412; 5,959,087; 5,968,741; 5,994,510; 6,036,978; 6,114,517; and 6,171,787; each of which are herein incorporated by reference in their entirety. Examples of soluble TNF-α receptors include, but are not limited to, sTNF-R1 (Amgen), etanercept (ENBREL™; Immunex) and its rat homolog RENBREL™, soluble inhibitors of TNF-α derived from TNFrI, TNFrII (Kohno et al., 1990, Proc. Natl. Acad. Sci. USA 87:8331-8335), and TNF-α Inh (Seckinger et al, 1990, Proc. Natl. Acad. Sci. USA 87:5188-5192).

In one embodiment, a TNF-α antagonist used in the compositions and methods of the invention is a soluble TNF-α receptor. In a specific embodiment, a TNF-α antagonist used in the compositions and methods of the invention is etanercept (ENBREL™; Immunex) or a fragment, derivative or analog thereof. In another embodiment, a TNF-α antagonist used in the compositions and methods of the invention is an antibody that specifically binds to TNF-α. In a specific embodiment, a TNF-α antagonist used in the compositions and methods of the invention is infliximab (REMICADE®; Centacor) a derivative, analog or antigen-binding fragment thereof.

Other TNF-α antagonists encompassed by the invention include, but are not limited to, IL-10, which is known to block TNF-α production via interferon γ-activated macrophages (Oswald et al. 1992, Proc. Natl. Acad. Sci. USA 89:8676-8680), TNFR-IgG (Ashkenazi et al., 1991, Proc. Natl. Acad. Sci. USA 88:10535-10539), the murine product TBP-1 (Serono/Yeda), the vaccine CytoTAb (Protherics), antisense molecule104838 (ISIS), the peptide RDP-58 (SangStat), thalidomide (Celgene), CDC-801 (Celgene), DPC-333 (Dupont), VX-745 (Vertex), AGIX-4207 (AtheroGenics), ITF-2357 (Italfarmaco), NPI-13021-31 (Nereus), SCIO-469 (Scios), TACE targeter (Immunix/AHP), CLX-120500 (Calyx), Thiazolopyrim (Dynavax), auranofin (Ridaura) (SmithKline Beecham Pharmaceuticals), quinacrine (mepacrine dichlorohydrate), tenidap (Enablex), Melanin (Large Scale Biological), and anti-p38 MAPK agents by Uriach.

Nucleic acid molecules encoding proteins, polypeptides, or peptides with TNF-α antagonist activity, or proteins, polypeptides, or peptides with TNF-α antagonist activity can be administered to a subject at risk of or with an inflammatory or autoimmune disease in accordance with the methods of the invention. Further, nucleic acid molecules encoding derivatives, analogs, fragments or variants of proteins, polypeptides, or peptides with TNF-α antagonist activity, or derivatives, analogs, fragments or variants of proteins, polypeptides, or peptides with TNF-α antagonist activity can be administered to a subject at risk of or with an inflammatory or autoimmune disease in accordance with the methods of the invention. In one embodiment, such derivatives, analogs, variants and fragments retain the TNF-α antagonist activity of the full-length, wild-type protein, polypeptide, or peptide.

Proteins, polypeptides, or peptides that can be used as TNF-α antagonists can be produced by any technique well-known in the art or described herein. Proteins, polypeptides or peptides with TNF-α antagonist activity can be engineered so as to increase the *in vivo* half-life of such proteins, polypeptides, or peptides utilizing techniques well-known in the art or described herein. In one embodiment, agents that are commercially available and known to function as TNF-α antagonists are used in the compositions and methods of the invention. The TNF-α antagonist activity of an agent can be determined *in vitro* and/or *in vivo* by any technique well-known to one skilled in the art.

### 5.2.2.5 Anti-Cancer Agents

Any therapy (*e.g*., therapeutic or prophylactic agent) which is known to be useful, has been used, or is currently being used for the prevention, treatment, management, or amelioration of a proliferative disorder, such as cancer (benign, malignant or metastatic), or one or more symptoms thereof can be used in compositions and method of the invention. Therapies (*e.g*., therapeutic or prophylactic agents) include, but are not limited to, peptides, polypeptides, fusion proteins, nucleic acid molecules, small molecules, mimetic agents, synthetic drugs, inorganic molecules, and organic molecules. Non-limiting examples of cancer therapies include chemotherapies, radiation therapies, hormonal therapies, and/or biological therapies/immunotherapies.

In certain embodiments, the anti-cancer agent is an immunomodulatory agent, such as a chemotherapeutic agent. In certain other embodiments, the anti-cancer agent is an immunomodulatory agent other than a chemotherapeutic agent. In other embodiments, the anti-cancer agent is not an immunomodulatory agent. In specific embodiments, the anticancer agent is an anti-angiogenic agent. In other embodiments, the anti-cancer agent is not an anti-angiogenic agent. In specific embodiments, the anti-cancer agent is an anti-inflammatory agent. In other embodiments, the anti-cancer agent is not an anti-inflammatory agent.

In particular embodiments, the anti-cancer agent is, but not limited to: acivicin; aclarubicin; acodazole hydrochloride; acronine; adozelesin; aldesleukin; altretamine; ambomycin; ametantrone acetate; aminoglutethimide; amsacrine; anastrozole; anthramycin; asparaginase; asperlin; azacitidine; azetepa; azotomycin; batimastat; benzodepa; bicalutamide; bisantrene hydrochloride; bisnafide dimesylate; bisphosphonates (*e.g.*, pamidronate (Aredria), sodium clondronate (Bonefos), zoledronic acid (Zometa), alendronate (Fosamax), etidronate, ibandornate, cimadronate, risedromate, and tiludromate); bizelesin; bleomycin sulfate; brequinar sodium; bropirimine; busulfan; cactinomycin; calusterone; caracemide; carbetimer; carboplatin; carmustine; carubicin hydrochloride; carzelesin; cedefingol; chlorambucil; cirolemycin; cisplatin; cladribine; crisnatol mesylate; cyclophosphamide; cytarabine; dacarbazine; dactinomycin; daunorubicin hydrochloride; decitabine; dexormaplatin; dezaguanine; dezaguanine mesylate; diaziquone; docetaxel; doxorubicin; doxorubicin hydrochloride; droloxifene; droloxifene citrate; dromostanolone propionate; duazomycin; edatrexate; eflornithine hydrochloride; EphA2 inhibitors (*e.g*., anti-EphA2 antibodies that result in the phosphorylation of EphA2 and the degration of EphA2 (see, U.S. Patent Application No. 60/418,213 which is incorporated herein by reference in its entirety); elsamitrucin; enloplatin; enpromate; epipropidine; epirubicin hydrochloride; erbulozole; esorubicin hydrochloride; estramustine; estramustine phosphate sodium; etanidazole; etoposide; etoposide phosphate; etoprine; fadrozole hydrochloride; fazarabine; fenretinide; floxuridine; fludarabine phosphate; fluorouracil; flurocitabine; fosquidone; fostriecin sodium; gemcitabine; gemcitabine hydrochloride; hydroxyurea; idarubicin hydrochloride; ifosfamide; ilmofosine; interleukin II (including recombinant interleukin II, or rIL2), interferon alpha-2a; interferon alpha-2b; interferon alpha-nl ; interferon alpha-n3; interferon beta-I a; interferon gamma-I b; iproplatin; irinotecan hydrochloride; lanreotide acetate; letrozole; leuprolide acetate; liarozole hydrochloride; lometrexol sodium; lomustine; losoxantrone hydrochloride; masoprocol; maytansine; mechlorethamine hydrochloride; anti-CD2 antibodies (*e.g.*, siplizumab (MedImmune Inc.; International Publication No. WO 02/098370, which is incorporated herein by reference in its entirety)); megestrol acetate; melengestrol acetate; melphalan; menogaril; mercaptopurine; methotrexate; methotrexate sodium; metoprine; meturedepa; mitindomide; mitocarcin; mitocromin; mitogillin; mitomalcin; mitomycin; mitosper; mitotane; mitoxantrone hydrochloride; mycophenolic acid; nocodazole; nogalamycin; ormaplatin; oxisuran; paclitaxel; pegaspargase; peliomycin; pentamustine; peplomycin sulfate; perfosfamide; pipobroman; piposulfan; piroxantrone hydrochloride; plicamycin; plomestane; porfimer sodium; porfiromycin; prednimustine; procarbazine hydrochloride; puromycin; puromycin hydrochloride; pyrazofurin; riboprine; rogletimide; safingol; safingol hydrochloride; semustine; simtrazene; sparfosate sodium; sparsomycin; spirogermanium hydrochloride; spiromustine; spiroplatin; streptonigrin; streptozocin; sulofenur; talisomycin; tecogalan sodium; tegafur; teloxantrone hydrochloride; temoporfin; teniposide; teroxirone; testolactone; thiamiprine; thioguanine; thiotepa; tiazofurin; tirapazamine; toremifene citrate; trestolone acetate; triciribine phosphate; trimetrexate; trimetrexate glucuronate; triptorelin; tubulozole hydrochloride; uracil mustard; uredepa; vapreotide; verteporfin; vinblastine sulfate; vincristine sulfate; vindesine; vindesine sulfate; vinepidine sulfate; vinglycinate sulfate; vinleurosine sulfate; vinorelbine tartrate; vinrosidine sulfate; vinzolidine sulfate; vorozole; zeniplatin; zinostatin; zorubicin hydrochloride.

Other anti-cancer drugs include, but are not limited to: 20-epi-1,25 dihydroxyvitamin D3; 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti-dorsalizing morphogenetic protein-1; antiandrogen, prostatic carcinoma; antiestrogen; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor; bicalutamide; bisantrene; bisaziridinylspermine; bisnafide; bistratene A; bizelesin; breflate; bropirimine; budotitane; buthionine sulfoximine; calcipotriol; calphostin C; camptothecin derivatives; canarypox IL-2; capecitabine; carboxamide-amino-triazole; carboxyamidotriazole; CaRest M3; CARN 700; cartilage derived inhibitor; carzelesin; casein kinase inhibitors (ICOS); castanospermine; cecropin B; cetrorelix; chlorlns; chloroquinoxaline sulfonamide; cicaprost; cis-porphyrin; cladribine; clomifene analogues; clotrimazole; collismycin A; collismycin B; combretastatin A4; combretastatin analogue; conagenin; crambescidin 816; crisnatol; cryptophycin 8; cryptophycin A derivatives; curacin A; cyclopentanthraquinones; cycloplatam; cypemycin; cytarabine ocfosfate; cytolytic factor; cytostatin; dacliximab; decitabine; dehydrodidemnin B; deslorelin; dexamethasone; dexifosfamide; dexrazoxane; dexverapamil; diaziquone; didemnin B; didox; diethylnorspermine; dihydro-5-azacytidine; dihydrotaxol, dioxamycin; diphenyl spiromustine; docetaxel; docosanol; dolasetron; doxifluridine; droloxifene; dronabinol; duocarmycin SA; ebselen; ecomustine; edelfosine; edrecolomab; eflornithine; elemene; emitefur; epirubicin; epristeride; estramustine analogue; estrogen agonists; estrogen antagonists; etanidazole; etoposide phosphate; exemestane; fadrozole; fazarabine; fenretinide; filgrastim; finasteride; flavopiridol; flezelastine; fluasterone; fludarabine; fluorodaunorunicin hydrochloride; forfenimex; formestane; fostriecin; fotemustine; gadolinium texaphyrin; gallium nitrate; galocitabine; ganirelix; gelatinase inhibitors; gemcitabine; glutathione inhibitors; HMG CoA reductase inhibitors (*e.g*., atorvastatin, cerivastatin, fluvastatin, lescol, lupitor, lovastatin, rosuvastatin, and simvastatin); hepsulfam; heregulin; hexamethylene bisacetamide; hypericin; ibandronic acid; idarubicin; idoxifene; idramantone; ilmofosine; ilomastat; imidazoacridones; imiquimod; immunostimulant peptides; insulin-like growth factor-1 receptor inhibitor; interferon agonists; interferons; interleukins; iobenguane; iododoxorubicin; ipomeanol, 4- iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; letrozole; leukemia inhibiting factor; leukocyte alpha interferon; leuprolide+estrogen+progesterone; leuprorelin; levamisole; LFA-3TIP (Biogen, Cambridge, MA; International Publication No. WO 93/0686 and U.S. Patent No. 6,162,432); liarozole; linear polyamine analogue; lipophilic disaccharide peptide; lipophilic platinum compounds; lissoclinamide 7; lobaplatin; lombricine; lometrexol; lonidamine; losoxantrone; lovastatin; loxoribine; lurtotecan; lutetium texaphyrin; lysofylline; lytic peptides; maitansine; mannostatin A; marimastat; masoprocol; maspin; matrilysin inhibitors; matrix metalloproteinase inhibitors; menogaril; merbarone; meterelin; methioninase; metoclopramide; MIF inhibitor; mifepristone; miltefosine; mirimostim; mismatched double stranded RNA; mitoguazone; mitolactol; mitomycin analogues; mitonafide; mitotoxin fibroblast growth factor-saporin; mitoxantrone; mofarotene; molgramostim; monoclonal antibody, human chorionic gonadotrophin; monophosphoryl lipid A+myobacterium cell wall sk; mopidamol; multiple drug resistance gene inhibitor; multiple tumor suppressor 1-based therapy; mustard anticancer agent; mycaperoxide B; mycobacterial cell wall extract; myriaporone; N-acetyldinaline; N-substituted benzamides; nafarelin; nagrestip; naloxone+pentazocine; napavin; naphterpin; nartograstim; nedaplatin; nemorubicin; neridronic acid; neutral endopeptidase; nilutamide; nisamycin; nitric oxide modulators; nitroxide antioxidant; nitrullyn; O6-benzylguanine; octreotide; okicenone; oligonucleotides; onapristone; ondansetron; ondansetron; oracin; oral cytokine inducer; ormaplatin; osaterone; oxaliplatin; oxaunomycin; paclitaxel; paclitaxel analogues; paclitaxel derivatives; palauamine; palmitoylrhizoxin; pamidronic acid; panaxytriol; panomifene; parabactin; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; porfimer sodium; porfiromycin; prednisone; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylene conjugate; raf antagonists; raltitrexed; ramosetron; ras farnesyl protein transferase inhibitors; ras inhibitors; ras-GAP inhibitor; retelliptine demethylated; rhenium Re 186 etidronate; rhizoxin; ribozymes; RII retinamide; rogletimide; rohitukine; romurtide; roquinimex; rubiginone B1; ruboxyl; safingol; saintopin; SarCNU; sarcophytol A; sargramostim; Sdi 1 mimetics; semustine; senescence derived inhibitor 1; sense oligonucleotides; signal transduction inhibitors; signal transduction modulators; single chain antigen binding protein; sizofiran; sobuzoxane; sodium borocaptate; sodium phenylacetate; solverol; somatomedin binding protein; sonermin; sparfosic acid; spicamycin D; spiromustine; splenopentin; spongistatin 1; squalamine; stem cell inhibitor; stem-cell division inhibitors; stipiamide; stromelysin inhibitors; sulfinosine; superactive vasoactive intestinal peptide antagonist; suradista; suramin; swainsonine; synthetic glycosaminoglycans; tallimustine; 5-fluorouracil; leucovorin; tamoxifen methiodide; tauromustine; tazarotene; tecogalan sodium; tegafur; tellurapyrylium; telomerase inhibitors; temoporfin; temozolomide; teniposide; tetrachlorodecaoxide; tetrazomine; thaliblastine; thiocoraline; thrombopoietin; thrombopoietin mimetic; thymalfasin; thymopoietin receptor agonist; thymotrinan; thyroid stimulating hormone; tin ethyl etiopurpurin; tirapazamine; titanocene bichloride; topsentin; toremifene; totipotent stem cell factor; translation inhibitors; tretinoin; triacetyluridine; triciribine; trimetrexate; triptorelin; tropisetron; turosteride; tyrosine kinase inhibitors; tyrphostins; UBC inhibitors; ubenimex; urogenital sinus-derived growth inhibitory factor; urokinase receptor antagonists; vapreotide; variolin B; vector system, erythrocyte gene therapy; thalidomide; velaresol; veramine; verdins; verteporfin; vinorelbine; vinxaltine; VITAXIN™ (see U.S. Patent Pub. No. US 2002/0168360 A1, dated November 14, 2002, entitled "Methods of Preventing or Treating Inflammatory or Autoimmune Disorders by Administering Integrin αᵥβ3 Antagonists in Combination With Other Prophylactic or Therapeutic Agents"); vorozole; zanoterone; zeniplatin; zilascorb; and zinostatin stimalamer.

In specific embodiments, radiation therapy comprising the use of x-rays, gamma rays and other sources of radiation to destroy the cancer cells is used in combination with the antibodies of the invention. In specific embodiments, the radiation treatment is administered as external beam radiation or teletherapy, wherein the radiation is directed from a remote source. In other specific embodiments, the radiation treatment is administered as internal therapy or brachytherapy wherein a radioactive source is placed inside the body close to cancer cells or a tumor mass.

Cancer therapies and their dosages, routes of administration and recommended usage are known in the art and have been described in such literature as the Physician's Desk Reference (56th ed., 2002).

### 5.3 CHARACTERIZATION AND DEMONSTRATION OF THERAPEUTIC OR PROPHYLACTIC UTILITY

Toxicity and efficacy of the prophylactic and/or therapeutic protocols of the instant invention can be determined by standard pharmaceutical procedures in cell cultures or experimental animals, *e.g*., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Prophylactic and/or therapeutic agents that exhibit large therapeutic indices are preferred. While prophylactic and/or therapeutic agents that exhibit toxic side effects may be used, care should be taken to design a delivery system that targets such agents to the site of affected tissue in order to minimize potential damage to uninfected cells and, thereby, reduce side effects.

The data obtained from the cell culture assays and animal studies can be used in formulating a range of dosage of the prophylactic and/or therapeutic agents for use in humans. The dosage of such agents lies preferably within a range of circulating concentrations that include the ED₅₀ with little or no toxicity. The dosage may vary within this range depending upon the dosage form employed and the route of administration utilized. For any agent used in the method of the invention, the therapeutically effective dose can be estimated initially from cell culture assays. A dose may be formulated in animal models to achieve a circulating plasma concentration range that includes the IC₅₀ (*i.e.,* the concentration of the test compound that achieves a half-maximal inhibition of symptoms) as determined in cell culture. Such information can be used to more accurately determine useful doses in humans. Levels in plasma may be measured, for example, by high performance liquid chromatography.

The therapeutic or prophylactic activity of the therapies used in accordance with the present invention also can be determined by using various experimental animal models. For example, but not by way of limitation, for the study of cancer, the SCID mouse model or transgenic mice where a mouse EphA2 is replaced with the human EphA2, nude mice with human xenografts, or any animal model (including hamsters, rabbits, etc.) known in the art and described in Relevance of Tumor Models for Anticancer Drug Development (1999, eds. Fiebig and Burger); Contributions to Oncology (1999, Karger); The Nude Mouse in Oncology Research (1991, eds. Boven and Winograd); and Anticancer Drug Development Guide (1997 ed. Teicher), each of which incorporated by reference herein in its entirety, may be used.

### 5.3.1 Demonstration of Therapeutic Utility

The protocols and compositions of the invention are preferably tested *in vitro,* and then *in vivo,* for the desired therapeutic or prophylactic activity, prior to use in humans. For example, *in vitro* assays which can be used to determine whether administration of a specific therapeutic protocol is indicated, include *in vitro* cell culture assays in which a patient tissue sample is grown in culture, and exposed to or otherwise administered a protocol, and the effect of such protocol upon the tissue sample is observed, *e.g*., increased phosphorylation/degradation of an Eph receptor and/or Ephrin. A lower level of proliferation or survival of the contacted cells indicates that the therapeutic agent is effective to treat the condition in the patient. Alternatively, instead of culturing cells from a patient, therapeutic agents and methods may be screened using cells of a tumor or malignant cell line. Many assays standard in the art can be used to assess such survival and/or growth; for example, cell proliferation can be assayed by measuring ³H-thymidine incorporation, by direct cell count, by detecting changes in transcriptional activity of known genes such as proto-oncogenes (*e.g*., fos, myc) or cell cycle markers; cell viability can be assessed by trypan blue staining, differentiation can be assessed visually based on changes in morphology, increased phosphorylation/degradation of an Eph receptor and/or Ephrin, etc.

Compounds for use in therapy can be tested in suitable animal model systems prior to testing in humans, including but not limited to in rats, mice, chicken, cows, monkeys, rabbits, hamsters, etc., for example, the animal models described above. The compounds can then be used in the appropriate clinical trials.

Further, any assays known to those skilled in the art can be used to evaluate the prophylactic and/or therapeutic utility of the combinatorial therapies disclosed herein for treatment or prevention of cancer.

### 5.4 PHARMACEUTICAL COMPOSITIONS

### 5.4.1 Gene Therapy

In a specific embodiment, nucleotide sequences comprising nucleic acids encoding an antibody of the invention or another prophylactic or therapeutic agent are administered to treat, prevent, manage, and/or ameliorate a disorder associated with aberrant (*i.e*., increased, decreased or inappropriate) Eph receptor and/or Ephrin expression or one or more symptoms thereof by way of gene therapy. Gene therapy refers to therapy performed by the administration to a subject of an expressed or expressible nucleic acid. In this embodiment of the invention, the nucleic acids produce their encoded antibody of the invention or prophylactic or therapeutic agent that mediates a prophylactic or therapeutic effect.

Any of the methods for gene therapy available in the art can be used according to the present invention. For general reviews of the methods of gene therapy, see Goldspiel et al., 1993, Clinical Pharmacy 12:488-505; Wu and Wu, 1991, Biotherapy 3:87-95; Tolstoshev, 1993, Ann. Rev. Pharmacol. Toxicol. 32:573-596; Mulligan, Science 260:926-932 (1993); and Morgan and Anderson, 1993, Ann. Rev. Biochem. 62:191-217; May, 1993, TIBTECH 11(5):155-215. Methods commonly known in the art of recombinant DNA technology which can be used are described in Ausubel et al. (eds.), Current Protocols in Molecular Biology, John Wiley & Sons, NY (1993); and Kriegler, Gene Transfer and Expression, A Laboratory Manual, Stockton Press, NY (1990).

In one embodiment, the method of the invention comprises administration of a composition comprising nucleic acids encoding an antibody of the invention or another prophylactic or therapeutic agent, said nucleic acids being part of an expression vector that expresses an antibody of the invention, another prophylactic or therapeutic agent, or fragments or chimeric proteins or heavy or light chains thereof in a suitable host. In particular, such nucleic acids have promoters, preferably heterologous promoters, operably linked to the antibody coding region, said promoter being inducible or constitutive, and, optionally, tissue- specific. In another embodiment, nucleic acid molecules are used in which the coding sequences of an antibody of the invention or another prophylactic or therapeutic agent and any other desired sequences are flanked by regions that promote homologous recombination at a desired site in the genome, thus providing for intrachromosomal expression of the antibody encoding nucleic acids (Koller and Smithies, 1989, Proc. Natl. Acad. Sci. USA 86:8932-8935; Zijlstra et al., 1989, Nature 342:435-438). In specific embodiments, the expressed antibody of the invention or other prophylactic or therapeutic agent is a single chain antibody; alternatively, the nucleic acid sequences include sequences encoding both the heavy and light chains, or fragments thereof, of the antibody of the invention or another prophylactic or therapeutic agent.

Delivery of the nucleic acids into a subject may be either direct, in which case the subject is directly exposed to the nucleic acid or nucleic acid-carrying vectors, or indirect, in which case, cells are first transformed with the nucleic acids in vitro, then transplanted into the subject. These two approaches are known, respectively, as *in vivo* or *ex vivo* gene therapy.

In a specific embodiment, the nucleic acid sequences are directly administered *in vivo,* where they are expressed to produce the encoded product. This can be accomplished by any of numerous methods known in the art, *e.g*., by constructing them as part of an appropriate nucleic acid expression vector and administering it so that they become intracellular, *e.g*., by infection using defective or attenuated retrovirals or other viral vectors (see U.S. Patent No. 4,980,286), or by direct injection of naked DNA, or by use of microparticle bombardment (*e.g.,* a gene gun; Biolistic, Dupont), or coating with lipids or cell-surface receptors or transfecting agents, encapsulation in liposomes, microparticles, or microcapsules, or by administering them in linkage to a peptide which is known to enter the nucleus, by administering it in linkage to a ligand subject to receptor-mediated endocytosis (see, *e.g*., Wu and Wu, 1987, J. Biol. Chem. 262:4429-4432) (which can be used to target cell types specifically expressing the receptors). In another embodiment, nucleic acid-ligand complexes can be formed in which the ligand comprises a fusogenic viral peptide to disrupt endosomes, allowing the nucleic acid to avoid lysosomal degradation. In yet another embodiment, the nucleic acid can be targeted *in vivo* for cell specific uptake and expression, by targeting a specific receptor (see, *e.g*., International Publication Nos. WO 92/06180; WO 92/22635; W092/20316; W093/14188; and WO 93/20221). Alternatively, the nucleic acid can be introduced intracellularly and incorporated within host cell DNA for expression, by homologous recombination (Koller and Smithies, 1989, Proc. Natl. Acad. Sci. USA 86:8932-8935; and Zijlstra et al., 1989, Nature 342:435-438).

In a specific embodiment, viral vectors that contains nucleic acid sequences encoding an antibody of the invention, another prophylactic or therapeutic agent, or fragments thereof are used. For example, a retroviral vector can be used (see Miller et al., 1993, Meth. Enzymol. 217:581-599). These retroviral vectors contain the components necessary for the correct packaging of the viral genome and integration into the host cell DNA. The nucleic acid sequences encoding an antibody of the invention or another prophylactic or therapeutic agent to be used in gene therapy are cloned into one or more vectors, which facilitates delivery of the gene into a subject. More detail about retroviral vectors can be found in Boesen et al., 1994, Biotherapy 6:291-302, which describes the use of a retroviral vector to deliver the mdr 1 gene to hematopoietic stem cells in order to make the stem cells more resistant to chemotherapy. Other references illustrating the use of retroviral vectors in gene therapy are: Clowes et al., 1994, J. Clin. Invest. 93:644-651; Klein et al., 1994, Blood 83:1467-1473; Salmons and Gunzberg, 1993, Human Gene Therapy 4:129-141; and Grossman and Wilson, 1993, Curr. Opin. in Genetics and Devel. 3:110-114.

Adenoviruses are other viral vectors that can be used in gene therapy. Adenoviruses are especially attractive vehicles for delivering genes to respiratory epithelia. Adenoviruses naturally infect respiratory epithelia where they cause a mild disease. Other targets for adenovirus-based delivery systems are liver, the central nervous system, endothelial cells, and muscle. Adenoviruses have the advantage of being capable of infecting non-dividing cells. Kozarsky and Wilson, 1993, Current Opinion in Genetics and Development 3:499-503 present a review of adenovirus-based gene therapy. Bout et al., 1994, Human Gene Therapy 5:3-10 demonstrated the use of adenovirus vectors to transfer genes to the respiratory epithelia of rhesus monkeys. Other instances of the use of adenoviruses in gene therapy can be found in Rosenfeld et al., 1991, Science 252:431-434; Rosenfeld et al., 1992, Cell 68:143-155; Mastrangeli et al., 1993, J. Clin. Invest. 91:225-234; PCT Publication W094/12649; and Wang et al., 1995, Gene Therapy 2:775-783. In a specific embodiment, adenovirus vectors are used.

Adeno-associated virus (AAV) has also been proposed for use in gene therapy (Walsh et al., 1993, Proc. Soc. Exp. Biol. Med. 204:289-300; and U.S. Patent No. 5,436,146).

Another approach to gene therapy involves transferring a gene to cells in tissue culture by such methods as electroporation, lipofection, calcium phosphate mediated transfection, or viral infection. Usually, the method of transfer includes the transfer of a selectable marker to the cells. The cells are then placed under selection to isolate those cells that have taken up and are expressing the transferred gene. Those cells are then delivered to a subject.

In this embodiment, the nucleic acid is introduced into a cell prior to administration *in vivo* of the resulting recombinant cell. Such introduction can be carried out by any method known in the art, including but not limited to transfection, electroporation, microinjection, infection with a viral or bacteriophage vector containing the nucleic acid sequences, cell fusion, chromosome-mediated gene transfer, microcell-mediated gene transfer, spheroplast fusion, etc. Numerous techniques are known in the art for the introduction of foreign genes into cells (see, *e.g*., Loeffler and Behr, 1993, Meth. Enzymol. 217:599-618; Cohen et al., 1993, Meth. Enzymol. 217:618-644; Clin. Pharma. Ther. 29:69-92 (1985)) and may be used in accordance with the present invention, provided that the necessary developmental and physiological functions of the recipient cells are not disrupted. The technique should provide for the stable transfer of the nucleic acid to the cell, so that the nucleic acid is expressible by the cell and preferably heritable and expressible by its cell progeny.

The resulting recombinant cells can be delivered to a subject by various methods known in the art. Recombinant blood cells (*e.g*., hematopoietic stem or progenitor cells) are preferably administered intravenously. The amount of cells envisioned for use depends on the several factors including, but not limited to, the desired effects and the patient state, and can be determined by one skilled in the art.

Cells into which a nucleic acid can be introduced for purposes of gene therapy encompass any desired, available cell type, and include but are not limited to epithelial cells, endothelial cells, keratinocytes, fibroblasts, muscle cells, hepatocytes; blood cells such as T lymphocytes, B lymphocytes, monocytes, macrophages, neutrophils, eosinophils, mast cells, megakaryocytes, granulocytes; various stem or progenitor cells, in particular hematopoietic stem or progenitor cells (*e.g*., as obtained from bone marrow, umbilical cord blood, peripheral blood, fetal liver, etc.). In a specific embodiment, the cell used for gene therapy is autologous to the subject.

In an embodiment in which recombinant cells are used in gene therapy, nucleic acid sequences encoding an antibody or fragment thereof are introduced into the cells such that they are expressible by the cells or their progeny, and the recombinant cells are then administered *in vivo* for therapeutic effect. In a specific embodiment, stem or progenitor cells are used. Any stem and/or progenitor cells which can be isolated and maintained *in vitro* can potentially be used in accordance with this embodiment of the present invention (see *e.g.*, PCT Publication WO 94/08598; Stemple and Anderson, 1992, Cell 7 1:973-985; Rheinwald, 1980, Meth. Cell Bio. 21A:229; and Pittelkow and Scott, 1986, Mayo Clinic Proc. 61:771).

In a specific embodiment, the nucleic acid to be introduced for purposes of gene therapy comprises an inducible promoter operably linked to the coding region, such that expression of the nucleic acid is controllable by controlling the presence or absence of the appropriate inducer of transcription.

### 5.5 COMPOSITIONS AND METHODS OF ADMINISTRATION

### 5.5.1 Pharmaceutical Compositions

The invention provides methods of treatment and prophylaxis by administering to a subject an effective amount of an Eph/Ephrin Modulator of the invention that agonizes and/or antagonizes one or more Eph receptors and/or Ephrins to modulate their expression and/or activity; or a pharmaceutical composition comprising an Eph/Ephrin Modulator of the invention in combination with a pharmaceutically acceptable carrier.

In one embodiment, a pharmaceutical composition of the invention comprises a purified Eph/Ephrin Modulator of the invention (*e.g*., the Eph/Ephrin Modulator is preferably substantially free from substances that limit its effect or produce undesired side-effects). The subject is preferably an animal, including but not limited to animals such as cows, pigs, horses, chickens, cats, dogs, *etc.,* and is preferably a mammal, and most preferably human.

The compositions of the invention include bulk drug compositions useful in the manufacture of pharmaceutical compositions (*e.g*., impure or non-sterile compositions) and pharmaceutical compositions (*i.e*., compositions that are suitable for administration to a subject or patient) which can be used in the preparation of unit dosage forms. Such compositions comprise a therapeutically effective amount of a therapeutic agent disclosed herein (*e.g*., an Eph/Ephrin Modulator) and a pharmaceutically acceptable carrier. In certain embodiments, compositions of the invention comprise a therapeutically effective amount of one or more one or more proteins of the invention and a pharmaceutically acceptable carrier. In a further embodiment, the composition of the invention further comprises an additional cancer or non-cancer therapeutic. In specific embodiments, the therapeutic agent(s) in the composition are purified.

In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant (*e.g*., Freund's adjuvant (complete and incomplete) or, more preferably, MF59C.1 adjuvant available from Chiron, Emeryville, CA), excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like.

Generally, the ingredients of compositions of the invention are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The compositions of the invention can be formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

In one embodiment of the invention, the compositions of the invention are pyrogen-free formulations which are substantially free of endotoxins and/or related pyrogenic substances. Endotoxins include toxins that are confined inside a microorganism and are released only when the microorganisms are broken down or die. Pyrogenic substances also include fever-inducing, thermostable substances (glycoproteins) from the outer membrane of bacteria and other microorganisms. Both of these substances can cause fever, hypotension and shock if administered to humans. Due to the potential harmful effects, even low amounts of endotoxins must be removed from intravenously administered pharmaceutical drug solutions. The Food & Drug Administration ("FDA") has set an upper limit of 5 endotoxin units (EU) per dose per kilogram body weight in a single one hour period for intravenous drug applications (The United States Pharmacopeial Convention, Pharmacopeial Forum 26 (1):223 (2000)). When therapeutic proteins are administered in amounts of several hundred or thousand milligrams per kilogram body weight, as can be the case with monoclonal antibodies, even trace amounts of harmful and dangerous endotoxin must be removed. Preferably, endotoxin and pyrogen levels in the composition are less then 10 EU/mg, or less then 5 EU/mg, or less then 1 EU/mg, or less then 0.1 EU/mg, or less then 0.01 EU/mg, or less then 0.001 EU/mg.

Various delivery systems are known and can be used to administer a therapeutic agent useful for treating or managing a pre-cancerous condition, *e.g*., encapsulation in liposomes, microparticles, microcapsules, recombinant cells capable of expressing the polypeptide fragment, receptor-mediated endocytosis (see, *e.g.,* Wu and Wu, 1987, J. Biol. Chem. 262:4429-4432), construction of a nucleic acid as part of a retroviral or other vector, etc. Methods of administering a therapeutic agent include, but are not limited to, parenteral administration (*e.g*., intradermal, intramuscular, intraperitoneal, intravenous and subcutaneous), epidural, and mucosal (*e.g.*, intranasal, inhaled, and oral routes). In a specific embodiment, therapeutic agents of the invention are administered intramuscularly, intravenously, or subcutaneously. The therapeutic agents may be administered by any convenient route, for example by infusion or bolus injection, by absorption through epithelial or mucocutaneous linings (*e.g*., oral mucosa, rectal and intestinal mucosa, etc.) and may be administered together with other biologically active agents. Administration can be systemic or local.

In a specific embodiment, it may be desirable to administer the therapeutic agents for use in methods of the invention locally to the area in need of treatment; this may be achieved by, for example, and not by way of limitation, local infusion, by injection, or by means of an implant, said implant being of a porous, non-porous, or gelatinous material, including membranes, such as sialastic membranes, or fibers.

In yet another embodiment, the therapeutic agent can be delivered in a controlled release or sustained release system. In one embodiment, a pump may be used to achieve controlled or sustained release (see Langer, *supra;* Sefton, 1987, CRC Crit. Ref. Biomed. Eng. 14:20; Buchwald et al., 1980, Surgery 88:507; Saudek et al., 1989, N. Engl. J. Med. 321:574). In another embodiment, polymeric materials can be used to achieve controlled or sustained release of the agents of the invention (see *e.g*., Medical Applications of Controlled Release, Langer and Wise (eds.), CRC Pres., Boca Raton, Florida (1974); Controlled Drug Bioavailability, Drug Product Design and Performance, Smolen and Ball (eds.), Wiley, New York (1984); Ranger and Peppas, 1983, J. Macromol. Sci. Rev. Macromol. Chem. 23:61; see also Levy et al., 1985, Science 228:190; During et al., 1989, Ann. Neurol. 25:351; Howard et al., 1989, J. Neurosurg. 7 1:105); U.S. Patent Nos. 5,679,377; 5,916,597; 5,912,015; 5,989,463; 5,128,326; International Publication Nos. WO 99/15154 and WO 99/20253. Examples of polymers used in sustained release formulations include, but are not limited to, poly(2-hydroxy ethyl methacrylate), poly(methyl methacrylate), poly(acrylic acid), poly(ethylene-co-vinyl acetate), poly(methacrylic acid), polyglycolides (PLG), polyanhydrides, poly(N-vinyl pyrrolidone), poly(vinyl alcohol), polyacrylamide, poly(ethylene glycol), polylactides (PLA), poly(lactide-co-glycolides) (PLGA), and polyorthoesters. In one embodiment, the polymer used in a sustained release formulation is inert, free of leachable impurities, stable on storage, sterile, and biodegradable. In yet another embodiment, a controlled or sustained release system can be placed in proximity of the therapeutic target, thus requiring only a fraction of the systemic dose (see, *e.g*., Goodson, in Medical Applications of Controlled Release, supra, vol. 2, pp. 115-138 (1984)).

Controlled release systems are discussed in the review by Langer (1990, Science 249:1527-1533). Any technique known to one of skill in the art can be used to produce sustained release formulations comprising one or more therapeutic agents of the invention. See, *e.g.,* U.S. Patent No. 4,526,938; International Publication Nos. WO 91/05548 and WO 96/20698; Ning et al., 1996, Radiotherapy & Oncology 39:179-189; Song et al., 1995, PDA Journal of Pharmaceutical Science & Technology 50:372-397; Cleek et al., 1997, Pro. Int'l. Symp. Control. Rel. Bioact. Mater. 24:853-854; and Lam et al., 1997, Proc. Int'l. Symp-Control Rel. Bioact. Mater. 24:759-760.

### 5.5.2 Formulations

Pharmaceutical compositions for use in accordance with the present invention may be formulated in conventional manner using one or more physiologically acceptable carriers or excipients.

Thus, the agents for use in the methods of the invention and their physiologically acceptable salts and solvates may be formulated for administration by inhalation or insufflation (either through the mouth or the nose) or oral, parenteral or mucosal (such as buccal, vaginal, rectal, sublingual) administration. In a further embodiment, local or systemic parenteral administration is used.

For oral administration, the pharmaceutical compositions may take the form of, for example, tablets or capsules prepared by conventional means with pharmaceutically acceptable excipients such as binding agents (*e.g*., pregelatinised maize starch, polyvinylpyrrolidone or hydroxypropyl methylcellulose); fillers (*e.g.*, lactose, microcrystalline cellulose or calcium hydrogen phosphate); lubricants (*e.g*., magnesium stearate, talc or silica); disintegrants (*e.g*., potato starch or sodium starch glycolate); or wetting agents (*e.g*., sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (*e.g*., sorbitol syrup, cellulose derivatives or hydrogenated edible fats); emulsifying agents (*e.g.*, lecithin or acacia); non-aqueous vehicles (*e.g*., almond oil, oily esters, ethyl alcohol or fractionated vegetable oils); and preservatives (*e.g*., methyl or propyl-p-hydroxybenzoates or sorbic acid). The preparations may also contain buffer salts, flavoring, coloring and sweetening agents as appropriate.

Preparations for oral administration may be suitably formulated to give controlled release of the active compound.

For buccal administration the compositions may take the form of tablets or lozenges formulated in conventional manner.

For administration by inhalation, the therapeutic agents for use according to the present invention are conveniently delivered in the form of an aerosol spray presentation from pressurized packs or a nebulizer, with the use of a suitable propellant, *e.g*., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of *e.g*., gelatin for use in an inhaler or insufflator may be formulated containing a powder mix of the compound and a suitable powder base such as lactose or starch.

The therapeutic agents may be formulated for parenteral administration by injection, *e.g*., by bolus injection or continuous infusion. Formulations for injection may be presented in unit dosage form, *e.g*., in ampoules or in multi-dose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, *e.g*., sterile pyrogen-free water, before use.

The therapeutic agents may also be formulated in rectal compositions such as suppositories or retention enemas, *e.g*., containing conventional suppository bases such as cocoa butter or other glycerides.

In addition to the formulations described previously, the therapeutic agents may also be formulated as a depot preparation. Such long acting formulations may be administered by implantation (for example subcutaneously or intramuscularly) or by intramuscular injection. Thus, for example, the therapeutic agents may be formulated with suitable polymeric or hydrophobic materials (for example as an emulsion in an acceptable oil) or ion exchange resins, or as sparingly soluble derivatives, for example, as a sparingly soluble salt.

The invention also provides that a therapeutic agent is packaged in a hermetically sealed container such as an ampoule or sachette indicating the quantity. In one embodiment, the therapeutic agent is supplied as a dry sterilized lyophilized powder or water free concentrate in a hermetically sealed container and can be reconstituted, *e.g.*, with water or saline to the appropriate concentration for administration to a subject.

In one embodiment of the invention, the formulation and administration of various therapies a disorder associated with aberrant (*i.e*., increased, decreased or aberrant) expression of one or more Eph receptors and/or Ephrins (such as chemotherapeutic, biological/immunotherapeutic and hormonal therapeutic agents) are known in the art and often described in the Physician's Desk Reference, 56th ed. (2002).

In other embodiments of the invention, radiation therapy agents such as radioactive isotopes can be given orally as liquids in capsules or as a drink. Radioactive isotopes can also be formulated for intravenous injections. The skilled oncologist can determine the preferred formulation and route of administration.

The compositions may, if desired, be presented in a pack or dispenser device that may contain one or more unit dosage forms containing the active ingredient. The pack may for example comprise metal or plastic foil, such as a blister pack. The pack or dispenser device may be accompanied by instructions for administration.

### 5.6 DOSAGE AND FREQUENCY OF ADMINISTRATION

The amount of a prophylactic or therapeutic agent or a composition of the invention which will be effective in the prevention, treatment, management, and/or amelioration of a disorder associated with aberrant (i.e., increased, decreased or inappropriate) Eph receptor and/or Ephrin expression, or one or more symptoms thereof can be determined by standard clinical methods. The frequency and dosage will vary also according to factors specific for each patient depending on the specific therapies (*e.g*., the specific therapeutic or prophylactic agent or agents) administered, the severity of the disorder, disease, or condition, the route of administration, as well as age, body, weight, response, and the past medical history of the patient. For example, the dosage of a prophylactic or therapeutic agent or a composition of the invention which will be effective in the treatment, prevention, management, and/or amelioration of a disorder associated with aberrant (*i.e*., increased, decreased or inappropriate) Eph receptor and/or Ephrin expression, or one or more symptoms thereof can be determined by administering the composition to an animal model such as, *e.g*., the animal models disclosed herein or known in to those skilled in the art. In addition, *in vitro* assays may optionally be employed to help identify optimal dosage ranges. Suitable regimens can be selected by one skilled in the art by considering such factors and by following, for example, dosages are reported in literature and recommended in the Physician's Desk Reference (57th ed., 2003).

Exemplary doses of a small molecule include milligram or microgram amounts of the small molecule per kilogram of subject or sample weight (*e.g*., about 1 microgram per kilogram to about 500 milligrams per kilogram, about 100 micrograms per kilogram to about 5 milligrams per kilogram, or about 1 microgram per kilogram to about 50 micrograms per kilogram). For antibodies, proteins, polypeptides, peptides and fusion proteins encompassed by the invention, the dosage administered to a patient is typically 0.0001 mg/kg to 100 mg/kg of the patient's body weight. In one embodiment, the dosage administered to a patient is between 0.0001 mg/kg and 20 mg/kg, 0.0001 mg/kg and 10 mg/kg, 0.0001 mg/kg and 5 mg/kg, 0.0001 and 2 mg/kg, 0.0001 and 1 mg/kg, 0.0001 mg/kg and 0.75 mg/kg, 0.0001 mg/kg and 0.5 mg/kg, 0.0001 mg/kg to 0.25 mg/kg, 0.0001 to 0.15 mg/kg, 0.0001 to 0.10 mg/kg, 0.001 to 0.5 mg/kg, 0.01 to 0.25 mg/kg or 0.01 to 0.10 mg/kg of the patient's body weight. Generally, human antibodies have a longer half-life within the human body than antibodies from other species due to the immune response to the foreign polypeptides. Thus, lower dosages of human antibodies and less frequent administration is often possible. Further, the dosage and frequency of administration of antibodies of the invention or fragments thereof may be reduced by enhancing uptake and tissue penetration of the antibodies by modifications such as, for example, lipidation.

In a specific embodiment, the dosage of antibodies, polypeptides, peptides, compositions, or combination therapies of the invention administered to prevent, treat, manage, and/or ameliorate a disorder associated with aberrant (*i.e*., increased, decreased or aberrant) expression of one or more Eph receptors and/or Ephrins, or one or more symptoms thereof in a patient is 150 µg/kg or less, 125 µg/kg or less, 100 µg/kg or less, 95 µg/kg or less, 90 µg/kg or less, 85 µg/kg or less, 80 µg/kg or less, 75 µg/kg or less, 70 µg/kg or less, 65 µg/kg or less, 60 µg/kg or less, 55 µg/kg or less, 50 µg/kg or less, 45 µg/kg or less, 40 µg/kg or less, 35 µg/kg or less, 30 µg/kg or less, 25 µg/kg or less, 20 µg/kg or less, 15 µg/kg or less, 10 µg/kg or less, 5 µg/kg or less, 2.5 µg/kg or less, 2 µg/kg or less, 1.5 µg/kg or less, 1 µg/kg or less, 0.5 µg/kg or less, or 0.5 µg/kg or less of a patient's body weight. In another embodiment, the dosage of the antibodies, compositions, or combination therapies of the invention administered to prevent, treat, manage, and/or ameliorate a disorder associated with aberrant (i.e., increased, decreased or aberrant) expression of one or more Eph receptors and/or Ephrins, or one or more symptoms thereof in a patient is a unit dose of 0.1 mg to 20 mg, 0.1 mg to 15 mg, 0.1 mg to 12 mg, 0.1 mg to 10 mg, 0.1 mg to 8 mg, 0.1 mg to 7 mg, 0.1 mg to 5 mg, 0.1 to 2.5 mg, 0.25 mg to 20 mg, 0.25 to 15 mg, 0.25 to 12 mg, 0.25 to 10 mg, 0.25 to 8 mg, 0.25 mg to 7m g, 0.25 mg to 5 mg, 0.5 mg to 2.5 mg, 1 mg to 20 mg, 1 mg to 15 mg, 1 mg to 12 mg, 1 mg to 10 mg, 1 mg to 8 mg, 1 mg to 7 mg, 1 mg to 5 mg, or 1 mg to 2.5 mg.

In certain embodiments, a subject is administered one or more doses of an effective amount of one or more antibodies, compositions, or combination therapies of the invention, wherein the an effective amount of said antibodies, compositions, or combination therapies prevents at least 20% to 25%, at least 25% to 30%, at least 30% to 35%, at least 35% to 40%, at least 40% to 45%, at least 45% to 50%, at least 50% to 55%, at least 55% to 60%, at least 60% to 65%, at least 65% to 70%, at least 70% to 75%, at least 75% to 80%, or up to at least 85% of endogenous ligand (*e.g*., an Ephrin) from binding to its receptor. In certain embodiments, a subject is administered one or more doses of an effective amount of one or more or more antibodies, compositions, or combination therapies of the invention, wherein the dose of an effective amount of said antibodies, compositions, or combination therapies reduces and/or inhibits mast cell degranulation at least 20% to 25%, at least 25% to 30%, at least 30% to 35%, at least 35% to 40%, at least 40% to 45%, at least 45% to 50%, at least 50% to 55%, at least 55% to 60%, at least 60% to 65%, at least 65% to 70%, at least 70% to 75%, at least 75% to 80%, at least 80 to 85%, at least 85% to 90%, at least 90% to 95%, or at least 95% to 98% relative to a control such as PBS in an *in vitro* and/or *in vivo* assay well-known in the art. In certain embodiments, a subject is administered one or more doses of an effective amount of one or more or more antibodies, compositions, or combination therapies of the invention, wherein the dose of an effective amount of said antibodies, compositions, or combination therapies reduces and/or inhibits mast cell activation at least 20% to 25%, at least 25% to 30%, at least 30% to 35%, at least 35% to 40%, at least 40% to 45%, at least 45% to 50%, at least 50% to 55%, at least 55% to 60%, at least 60% to 65%, at least 65% to 70%, at least 70% to 75%, at least 75% to 80%, at least 80 to 85%, at least 85% to 90%, at least 90% to 95%, or at least 95% to 98% relative to a control such as PBS in an *in vitro* and/or *in vivo* assay well-known in the art. In certain embodiments, a subject is administered one or more doses of an effective amount of one or more or more antibodies, compositions, or combination therapies of the invention, wherein the dose of an effective amount of said antibodies, compositions, or combination therapies reduces and/or inhibits mast cell proliferation at least 20% to 25%, at least 25% to 30%, at least 30% to 35%, at least 35% to 40%, at least 40% to 45%, at least 45% to 50%, at least 50% to 55%, at least 55% to 60%, at least 60% to 65%, at least 65% to 70%, at least 70% to 75%, at least 75% to 80%, at least 80 to 85%, at least 85% to 90%, at least 90% to 95%, or at least 95% to 98% relative to a control such as PBS in an *in vitro* and/or *in vivo* assay well-known in the art. In certain embodiments, a subject is administered one or more doses of an effective amount of one or more or more antibodies, compositions, or combination therapies of the invention, wherein the dose of an effective amount of said antibodies, compositions, or combination therapies reduces and/or inhibits mast cell infiltration at least 20% to 25%, at least 25% to 30%, at least 30% to 35%, at least 35% to 40%, at least 40% to 45%, at least 45% to 50%, at least 50% to 55%, at least 55% to 60%, at least 60% to 65%, at least 65% to 70%, at least 70% to 75%, at least 75% to 80%, at least 80 to 85%, at least 85% to 90%, at least 90% to 95%, or at least 95% to 98% relative to a control such as PBS in an *in vitro* and/or *in vivo* assay well-known in the art.

In other embodiments, a subject is administered one or more doses of an effective amount of one or more Eph/Ephrin Modulators of the invention (*e.g*., a polypeptide or antibody), wherein the dose of an effective amount achieves a serum titer of at least 0.1 µg/ml, at least 0.5 µg/ml, at least 1 µg/ml, at least 2 µg/ml, at least 5 µg/ml, at least 6 µg/ml, at least 10 µg/ml, at least 15 µg/ml, at least 20 µg/ml, at least 25 µg/ml, at least 50 µg/ml, at least 100 µg/ml, at least 125 µg/ml, at least 150 µg/ml, at least 175 µg/ml, at least 200 µg/ml, at least 225 µg/ml, at least 250 µg/ml, at least 275 µg/ml, at least 300 µg/ml, at least 325 µg/ml, at least 350 µg/ml, at least 375 µg/ml, or at least 400 µg/ml of the antibodies of the invention. In yet other embodiments, a subject is administered a dose of an effective amount of one or more antibodies of the invention to achieve a serum titer of at least 0.1 µg/ml, at least 0.5 µg/ml, at least 1 µg/ml, at least, 2 µg/ml, at least 5 µg/ml, at least 6 µg/ml, at least 10 µg/ml, at least 15 µg/ml, at least 20 µg/ml, at least 25 µg/ml, at least 50 µg/ml, at least 100 µg/ml, at least 125 µg/ml, at least 150 µg/ml, at least 175 µg/ml, at least 200 µg/ml, at least 225 µg/ml, at least 250 µg/ml, at least 275 µg/ml, at least 300 µg/ml, at least 325 µg/ml, at least 350 µg/ml, at least 375 µg/ml, or at least 400 µg/ml of the antibodies and a subsequent dose of an effective amount of one or more antibodies of the invention is administered to maintain a serum titer of at least 0.1 µg/ml, 0.5 µg/ml, 1 µg/ml, at least, 2 µg/ml, at least 5 µg/ml, at least 6 µg/ml, at least 10 µg/ml, at least 15 µg/ml, at least 20 µg/ml, at least 25 µg/ml, at least 50 µg/ml, at least 100 µg/ml, at least 125 µg/ml, at least 150 µg/ml, at least 175 µg/ml, at least 200 µg/ml, at least 225 µpg/ml, at least 250 µg/ml, at least 275 µg/ml, at least 300 µg/ml, at least 325 µg/ml, at least 350 µg/ml, at least 375 µg/ml, or at least 400 µg/ml. In accordance with these embodiments, a subject may be administered 1, 2, 3, 4, 5, 6, 7, 8, 9, 10, 11, 12 or more subsequent doses.

In a specific embodiment, the invention provides methods of preventing, treating, managing, or treating a disorder associated with aberrant (*i.e*., increased, decreased or inappropriate) Eph receptor and/or Ephrin expression or one or more symptoms thereof, said method comprising administering to a subject in need thereof a dose of at least 10 µg, at least 15 µg, at least 20 µg, at least 25 µg, at least 30 µg, at least 35 µg, at least 40 µg, at least 45 µg, at least 50 µg, at least 55 µg, at least 60 µg, at least 65 µg, at least 70 µg, at least 75 µg, at least 80 µg, at least 85 µg, at least 90 µg, at least 95 µg, at least 100 µg, at least 105 µg, at least 110 µg, at least 115 µg, or at least 120 µg of one or more antibodies, combination therapies, or compositions of the invention. In another embodiment, the invention provides a method of preventing, treating, managing, and/or ameliorating a disorder associated with aberrant (*i.e*., increased, decreased or aberrant) expression of one or more Eph receptors and/or Ephrins or one or more symptoms thereof, said methods comprising administering to a subject in need thereof a dose of at least 10 µg, at least 15 µg, at least 20 µg, at least 25 µg, at least 30 µg, at least 35 µg, at least 40 µg, at least 45 µg, at least 50 µg, at least 55 µg, at least 60 µg, at least 65 µg, at least 70 µg, at least 75 µg, at least 80 µg, at least 85 µg, at least 90 µg, at least 95 µg, at least 100 µg, at least 105 µg, at least 110 µg, at least 115 µg, or at least 120 µg of one or more antibodies, combination therapies, or compositions of the invention once every 3 days, once every 4 days, once every 5 days, once every 6 days, once every 7 days, once every 8 days, once every 10 days, once every two weeks, once every three weeks, or once a month.

The present invention provides methods of preventing, treating, managing, or preventing a disorder associated with aberrant (*i.e*., increased, decreased or inappropriate) Eph receptor and/or Ephrin expression or one or more symptoms thereof, said method comprising: (a) administering to a subject in need thereof one or more doses of a prophylactically or therapeutically effective amount of one or more antibodies, combination therapies, or compositions of the invention; and (b) monitoring the plasma level/concentration of the said administered antibody or antibodies in said subject after administration of a certain number of doses of the said antibody or antibodies. Moreover, for example, said certain number of doses is 1, 2, 3, 4, 5, 6, 7, 8, 9, 10, 11, or 12 doses of a prophylactically or therapeutically effective amount one or more antibodies, compositions, or combination therapies of the invention.

In a specific embodiment, the invention provides a method of preventing, treating, managing, and/or ameliorating a disorder associated with aberrant (*i.e*., increased, decreased or inappropriate) Eph receptor and/or Ephrin expression or one or more symptoms thereof, said method comprising: (a) administering to a subject in need thereof a dose of at least 10 µg (*e.g*., at least 15 µg, at least 20 µg, at least 25 µg, at least 30 µg, at least 35 µg, at least 40 µg, at least 45 µg, at least 50 µg, at least 55 µg, at least 60 µg, at least 65 µg, at least 70 µg, at least 75 µg, at least 80 µg, at least 85 µg, at least 90 µg, at least 95 µg, or at least 100 µg) of one or more antibodies of the invention; and (b) administering one or more subsequent doses to said subject when the plasma level of the antibody or antibodies administered in said subject is less than 0.1 µg/ml, less than 0.25 µg/ml, less than 0.5 µg/ml, less than 0.75 µg/ml, or less than 1 µg/ml. In another embodiment, the invention provides a method of preventing, treating, managing, and/or ameliorating a disorder associated with aberrant (*i.e*., increased, decreased or inappropriate) Eph receptor and/or Ephrin expression or one or more symptoms thereof, said method comprising: (a) administering to a subject in need thereof one or more doses of at least 10 µg (*e.g*. at least 15 µg, at least 20 µg, at least 25 µg, at least 30 µg, at least 35 µg, at least 40 µg, at least 45 µg, at least 50 µg, at least 55 µg, at least 60 µg, at least 65 µg, at least 70 µg, at least 75 µg, at least 80 µg, at least 85 µg, at least 90 µg, at least 95 µg, or at least 100 µg) of one or more antibodies of the invention; (b) monitoring the plasma level of the administered antibody or antibodies of the invention in said subject after the administration of a certain number of doses; and (c) administering a subsequent dose of the antibody or antibodies of the invention when the plasma level of the administered antibody or antibodies in said subject is less than 0.1 µg/ml, less than 0.25 µg/ml, less than 0.5 µg/ml, less than 0.75 µg/ml, or less than 1 µg/ml. In one embodiment, said certain number of doses is 1, 2, 3, 4, 5, 6, 7, 8, 9, 10, 11, or 12 doses of an effective amount of one or more antibodies of the invention.

In certain embodiments, the Eph receptor- or Ephrin antigenic peptides and anti-idiotypic antibodies of the invention are formulated at 1 mg/ml, 5 mg/ml, 10 mg/ml, and 25 mg/ml for intravenous injections and at 5 mg/ml, 10 mg/ml, and 80 mg/ml for repeated subcutaneous administration and intramuscular injection.

Where the Eph receptor- or Ephrin vaccine is a bacterial vaccine, the vaccine can be formulated at amounts ranging between approximately 1x10² CFU/ml to approximately 1x10¹² CFU/ml, for example at 1x10² CFU/ml, 5x10² CFU/ml, 1x10³ CFU/ml, 5x10³ CFU/ml, 1x10⁴ CFU/ml, 5x10⁴ CFU/ml, 1x10⁵ CFU/ml, 5x10⁵ CFU/ml, 1x10⁶ CFU/ml, 5x10⁶ CFU/ml, 1x10⁷ CFU/ml, 5x10⁷ CFU/ml, 1x10⁸ CFU/ml, 5x10⁸ CFU/ml, 1x10⁹ CFU/ml, 5x10⁹ CFU/ml, 1x10¹⁰ CFU/ml, 5x10¹⁰ CFU/ml, 1x10¹¹ CFU/ml, 5x10¹¹ CFU/ml, or 1x10¹² CFU/ml.

For Eph receptor- and Ephrin antigenic peptides or anti-idiotypic antibodies, the dosage administered to a patient is typically 0.1 mg/kg to 100 mg/kg of the patient's body weight. In one embodiment, the dosage administered to a patient is between 0.1 mg/kg and 20 mg/kg of the patient's body weight, or 1 mg/kg to 10 mg/kg of the patient's body weight.

With respect to the dosage of bacterial Eph receptor- and Ephrin vaccines of the invention, the dosage is based on the amount colony forming units (c.f.u.). Generally, in various embodiments, the dosage ranges are from about 1.0 c.f.u./kg to about 1x10¹⁰ c.f.u./kg; from about 1.0 c.f.u./kg to about 1x10⁸ c.f.u./kg; from about 1x10² c.f.u./kg to about 1x10⁸ c.fu./kg; and from about 1x10⁴ c.f.u./kg to about 1x 10⁸ c.f.u./kg. Effective doses may be extrapolated from dose-response curves derived animal model test systems. In certain exemplary embodiments, the dosage ranges are 0.001-fold to 10,000-fold of the murine LD₅₀, 0.01-fold to 1,000-fold of the murine LD₅₀, 0.1-fold to 500-fold of the murine LD₅₀, 0.5-fold to 250-fold of the murine LD₅₀, 1-fold to 100-fold of the murine LD₅₀, and 5-fold to 50-fold of the murine LD₅₀. In certain specific embodiments, the dosage ranges are 0.00.1-fold, 0.01-fold, 0.1-fold, 0.5-fold, 1-fold, 5-fold, 10-fold, 50-fold, 100-fold, 200-fold, 500-fold, 1,000-fold, 5,000-fold or 10,000-fold of the murine LD₅₀.

Therapies (*e.g*., prophylactic or therapeutic agents), other than antibodies of the invention, which have been or are currently being used to prevent, treat, manage, and/or ameliorate a disorder associated with aberrant (*i.e*., increased, decreased or inappropriate) Eph receptor and/or Ephrin expression or one or more symptoms thereof can be administered in combination with one or more antibodies of the invention according to the methods of the invention to treat, manage, prevent, and/or ameliorate a disorder associated with aberrant (*i.e*., increased, decreased or inappropriate) Eph receptor and/or Ephrin expression or one or more symptoms thereof. In one embodiment, the dosages of prophylactic or therapeutic agents used in combination therapies of the invention are lower than those which have been or are currently being used to prevent, treat, manage, and/or ameliorate a disorder associated with aberrant (*i.e*., increased, decreased or inappropriate) Eph receptor and/or Ephrin expression or one or more symptoms thereof. The recommended dosages of agents currently used for the prevention, treatment, management, or amelioration of a disorder associated with aberrant (*i.e*., increased, decreased or inappropriate) Eph receptor and/or Ephrin expression or one or more symptoms thereof can be obtained from any reference in the art including, but not limited to, Hardman et al., eds., 2001, Goodman & Gilman's The Pharmacological Basis Of Basis Of Therapeutics, 10th ed., Mc-Graw-Hill, New York; Physician's Desk Reference (PDR) 57th ed., 2003, Medical Economics Co., Inc., Montvale, NJ, which are incorporated herein by reference in its entirety.

In various embodiments, the therapies (*e.g*., prophylactic or therapeutic agents) are administered less than 5 minutes apart, less than 30 minutes apart, 1 hour apart, at about 1 hour apart, at about 1 to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, at about 12 hours to 18 hours apart, 18 hours to 24 hours apart, 24 hours to 36 hours apart, 36 hours to 48 hours apart, 48 hours to 52 hours apart, 52 hours to 60 hours apart, 60 hours to 72 hours apart, 72 hours to 84 hours apart, 84 hours to 96 hours apart, or 96 hours to 120 hours part. In specific embodiments, two or more therapies are administered within the same patent visit.

In certain embodiments, one or more Eph/Ephrin Modulators of the invention and one or more other therapies (*e.g*., prophylactic or therapeutic agents) are cyclically administered. Cycling therapy involves the administration of a first therapy (*e.g*., a first prophylactic or therapeutic agent) for a period of time, followed by the administration of a second therapy (*e.g*., a second prophylactic or therapeutic agent) for a period of time, optionally, followed by the administration of a third therapy (*e.g*., prophylactic or therapeutic agent) for a period of time and so forth, and repeating this sequential administration, *i.e*., the cycle in order to reduce the development of resistance to one of the therapies, to avoid or reduce the side effects of one of the therapies, and/or to improve the efficacy of the therapies.

In certain embodiments, the administration of the same Eph/Ephrin Modulators of the invention may be repeated and the administrations may be separated by at least 1 day, 2 days, 3 days, 5 days, 10 days, 15 days, 30 days, 45 days, 2 months, 75 days, 3 months, or at least 6 months. In other embodiments, the administration of the same therapy (*e.g*., prophylactic or therapeutic agent) other than an antibody of the invention may be repeated and the administration may be separated by at least at least 1 day, 2 days, 3 days, 5 days, 10 days, 15 days, 30 days, 45 days, 2 months, 75 days, 3 months, or at least 6 months.

### 5.7 KITS

The invention provides a pharmaceutical pack or kit comprising one or more containers filled with an Eph/Ephrin Modulator of the invention. Additionally, one or more other prophylactic or therapeutic agents useful for the treatment of a disorder associated with aberrant (*i.e*., increased, decreased or aberrant) expression of one or more Eph receptors and/or Ephrins or other relevant agents can also be included in the pharmaceutical pack or kit. In certain embodiments, the other prophylactic or therapeutic agent is an immunomodulatory agent (*e.g*., anti-Eph receptor antibody or anti-Ephrin antibody). The invention also provides a pharmaceutical pack or kit comprising one or more containers filled with one or more of the ingredients of the pharmaceutical compositions of the invention. Optionally associated with such container(s) can be a notice in the form prescribed by a governmental agency regulating the manufacture, use or sale of pharmaceuticals or biological products, which notice reflects approval by the agency of manufacture, use or sale for human administration.

### 5.7.1 Diagnostic/Prognostic Tests

The invention further provides diagnostic methods using the Eph/Ephrin Modulators of the invention to evaluate the efficacy of cancer treatment, either Eph/Ephrin Modulator-based or not Eph/Ephrin Modulator-based. In general, increased Eph receptor and/or Ephrin expression is associated with increasingly invasive and metastatic cancers. Accordingly, depending on the disorder to be treated, a reduction in Eph receptor and/or Ephrin expression with a particular treatment indicates that the treatment is reducing the invasiveness and/or metastatic potential of cancer. In particular embodiments, the diagnostic methods of the invention provide methods of imaging and localizing metastases and methods of diagnosis and prognosis using tissues and fluids distal to the primary tumor site (as well as methods using tissues and fluids of the primary tumor), for example, whole blood, sputum, urine, serum, fine needle aspirates (*i.e*., biopsies). In other embodiments, the diagnostic methods of the invention provide methods of imaging and localizing metastases and methods of diagnosis and prognosis *in vivo.* In such embodiments, primary metastatic tumors are detected using an antibody of the invention, for example, an exposed Eph receptor epitope antibody. The antibodies of the invention may also be used for immunohistochemical analyses of frozen or fixed cells or tissue assays.

In another embodiment, kits comprising the pharmaceutical compositions or diagnostic reagents of the invention are provided.

### 5.8 ARTICLES OF MANUFACTURE

The present invention also encompasses a finished packaged and labeled pharmaceutical product. This article of manufacture includes the appropriate unit dosage form in an appropriate vessel or container such as a glass vial or other container that is hermetically sealed. The pharmaceuctical product may be formulated in single dose vials as a sterile liquid that contains 10 mM histidine buffer at pH 6.0 and 150 mM sodium chloride. Each 1.0 mL of solution may contain 100 mg of protein, 1.6 mg of histidine and 8.9 mg of sodium chloride in water for injection. During the manufacturing process the pH of the formulation buffer is adjusted to 6.0 using hydrochloric acid. In the case of dosage forms suitable for parenteral administration the active ingredient, e.g., an antibody of the invention that specifically binds to an Eph receptor and/or Ephrin, is sterile and suitable for administration as a particulate free solution. In other words, the invention encompasses both parenteral solutions and lyophilized powders, each being sterile, and the latter being suitable for reconstitution prior to injection. Alternatively, the unit dosage form may be a solid suitable for oral, transdermal, intransal, or topical delivery.

In one embodiment, the unit dosage form is suitable for intravenous, intramuscular, intranasal, oral, topical or subcutaneous delivery. Thus, the invention encompasses solutions, preferably sterile, suitable for each delivery route.

As with any pharmaceutical product, the packaging material and container are designed to protect the stability of the product during storage and shipment. Further, the products of the invention include instructions for use or other informational material that advise the physician, technician or patient on how to appropriately prevent or treat the disorder in question. In other words, the article of manufacture includes instruction means indicating or suggesting a dosing regimen including, but not limited to, actual doses, monitoring procedures, total lymphocyte, mast cell counts, T cell counts, IgE production, and other monitoring information.

Specifically, the invention provides an article of manufacture comprising packaging material, such as a box, bottle, tube, vial, container, sprayer, insufflator, intravenous (i.v.) bag, envelope and the like; and at least one unit dosage form of a pharmaceutical agent contained within said packaging material, wherein said pharmaceutical agent comprises an antibody that specifically binds to an Eph receptor and/or Ephrin and wherein said packaging material includes instruction means which indicate that said antibody can be used to prevent, manage, treat, and/or ameliorate one or more symptoms associated with a disorder associated with aberrant expression and/or activity of an Eph receptor and/or Ephrin polypeptide, a disorder associated with aberrant expression and/or activity of an Eph receptor and/or Ephrin, or one or more symptoms thereof by administering specific doses and using specific dosing regimens as described herein.

The invention also provides an article of manufacture comprising packaging material, such as a box, bottle, tube, vial, container, sprayer, insufflator, intravenous (i.v.) bag, envelope and the like; and at least one unit dosage form of each pharmaceutical agent contained within said packaging material, wherein one pharmaceutical agent comprises an antibody that specifically binds to an Eph recpetor and/or Ephrin polypeptide and the other pharmaceutical agent comprises a second, different antibody that specifically binds to an Eph receptor and/or Ephrin polypeptide, and wherein said packaging material includes instruction means which indicate that said agents can be used to treat, prevent and/or ameliorate a disorder associated with aberrant (*i.e.*, increased, decreased or aberrant) expression of one or more Eph receptors and/or Ephrins, or one or more symptoms thereof by administering specific doses and using specific dosing regimens as described herein.

The invention also provides an article of manufacture comprising packaging material, such as a box, bottle, tube, vial, container, sprayer, insufflator, intravenous (i.v.) bag, envelope and the like; and at least one unit dosage form of each pharmaceutical agent contained within said packaging material, wherein one pharmaceutical agent comprises an antibody that specifically binds to an Eph receptor and/or Ephrin polypeptide and the other pharmaceutical agent comprises a prophylactic or therapeutic agent other than an antibody that specifically binds to an Eph receptor and/or Ephrin polypeptide, and wherein said packaging material includes instruction means which indicate that said agents can be used to treat, prevent and/or ameliorate one or more symptoms associated with a disorder associated with aberrant (*i.e*., increased, decreased or aberrant) expression of one or more Eph receptors and/or Ephrins, or one or more symptoms thereof by administering specific doses and using specific dosing regimens as described herein.

The present invention provides that the adverse effects that may be reduced or avoided by the methods of the invention are indicated in informational material enclosed in an article of manufacture for use in preventing, treating and/or ameliorating one or more symptoms associated with a disorder associated with aberrant (*i.e*., increased, decreased or aberrant) expression of one or more Eph receptors and/or Ephrins. Adverse effects that may be reduced or avoided by the methods of the invention include, but are not limited to, vital sign abnormalities (fever, tachycardia, bardycardia, hypertension, hypotension), hematological events (anemia, lymphopenia, leukopenia, thrombocytopenia), headache, chills, dizziness, nausea, asthenia, back pain, chest pain (chest pressure), diarrhea, myalgia, pain, pruritus, psoriasis, rhinitis, sweating, injection site reaction, and vasodilatation. Since antibodies of the invention that specifically bind to an Eph receptor and/or Ephrin polypeptide may be immunosuppressive, prolonged immunosuppression may increase the risk of infection, including opportunistic infections. Prolonged and sustained immunosuppression may also result in an increased risk of developing certain types of cancer.

Further, the information material enclosed in an article of manufacture for use in preventing, treating, managing, and/or ameliorating a disorder associated with aberrant (*i.e*., increased, decreased or aberrant) expression of one or more Eph receptors and/or Ephrins or one or more symptoms thereof can indicate that foreign proteins may also result in allergic reactions, including anaphylaxis, or cytosine release syndrome. The information material should indicate that allergic reactions may exhibit only as mild pruritic rashes or they may be severe such as erythroderma, Stevens-Johnson syndrome, vasculitis, or anaphylaxis. The information material should also indicate that anaphylactic reactions (anaphylaxis) are serious and occasionally fatal hypersensitivity reactions. Allergic reactions including anaphylaxis may occur when any foreign protein is injected into the body. They may range from mild manifestations such as urticaria or rash to lethal systemic reactions. Anaphylactic reactions occur soon after exposure, usually within 10 minutes. Patients may experience paresthesia, hypotension, laryngeal edema, mental status changes, facial or pharyngeal angioedema, airway obstruction, bronchospasm, urticaria and pruritus, serum sickness, arthritis, allergic nephritis, glomerulonephritis, temporal arthritis, or eosinophilia.

### 6. EXAMPLES

**EXAMPLE 1**

**Affinity optimization of a human Anti-Human Anti-Eph Monoclonal Antibody GEA44, and "dialing in" unique binding characteristics of the optimized variants to multiple EphA receptors.**

**Reagents**

All chemicals were of analytical grade. Restriction enzymes and DNA-modifying enzymes, and T4 ligase and T7 DNA polymerase were purchased from New England Biolabs, Inc. (Beverly, MA). Custom oligonucleotides were synthesized from Invitrogen (Carlsbad, CA). Human EphA4-Fc fusion protein (consisting of the human EphA4 ectodomain fused with the Fc portion of a human IgG1 or EphA4-His fusion protein consisting of the human EphA4 ectodomain fused to a Histidine tag were expressed in human embryonic kidney (HEK) 293 cells and purified by protein G affinity chromatography using standard protocols. Streptavidin magnetic beads were purchased from Dynal (Lake Success, NY). Human EphA4-Fc or EphA4-His biotinylation was carried out using an EZ-Link Sulfo-NHS-LC-Biotinylation Kit according to the manufacturer's instructions (Pierce, Rockford, IL).

**Construction of the variable genes into a phage expression vector**

A Fab version of human mAb GEA44 was cloned into an M13-based phage expression vector. This vector allows the expression of Fab fragments that contain the first constant domain of a human γ1 heavy chain and the constant domain of a human kappa (κ) light chain under the control of the *lacZ* promoter (Dall'Acqua et al., 2005, Methods, 36:43-60). The cloning was carried out by hybridization mutagenesis (Kunkel et al., 1987, Methods Enzymol., 154:367-382) as described (Wu et al., 2003, Methods Mol Biol., 207:197-212).

**PCR of GEA44 variable heavy and light chains**: Briefly the variable heavy chain and light chain from GEA44 were amplified using approximately 0.5 pmol of each of the following gene-specific oligonucleotide primers:

**GEA44 VL for biotin loop:**

*5'- ggtcgttccattttactcccactccGAAATTGTGCTGACTCAGTCTCC-3' (5' biotinylated);

**GEA44VL back loop:**

5'gatgaagacagatggtgcagccacagtacgTTTGATCTCCACCTTGGTCCCTTGG-3';

**GEA44VH for biotin loop:**
*5'gctggtggtgccgttctatagccatagcCAGGTGCAGCTGTTGCAGTCTGGAGC-3'(5' biotinylated),

**GEA44VH back loop:**

5' ggaagaccgatgggcccttggtggaggcTGAGGAGACGGTGACCAGGGTGCC 3'.

Oligonucleotides **GEA44 VH** and **VL for biotin loop** contain a M13 gene 3 leader overlapping sequence (lower case). Oligonucleotides **GEA44 VH** and **VL back loop** contain a CH1 and Cκ overlapping sequence respectively (lower case). VH-GEA44 and VL-GEA44 heavy and light chain genes, used in the context of a parental Fab positive control and template for mutagenesis reactions, were synthesized by PCR from the corresponding GEA44 IgG constructs for mammalian expression (produced at MedImmune, Inc.) using the **GEA44 VH for biotin loop/ GEA44 VH back loop and GEA44VL for biotin loop /GEA44 VL back loop** oligonucleotide combinations, respectively (see below).

**Fab template construction:** Minus single-stranded DNA corresponding to the GEA44 V regions was purified from PCR products by ethanol precipitation after dissociation of the double-stranded PCR product using sodium hydroxide and elimination of the biotinylated strand by streptavidin-coated magnetic beads as described (Wu et al., 2003, Methods Mol Biol., 207:197-212; Wu et al., 2003, Methods Mol Biol, 207:213-233). Approximate equimolar amounts of the minus strands of V_{H}-GEA44 and V_{L} GEA44 were mixed with the single stranded M13 loop vector, These variable genes anneal specifically to two regions of the vector containing each one palindromic loop. Those loops contained a unique Xba I site which, when restricted by Xba I, allows for the selection of the vectors that contain both V_{L} and V_{H} chains fused in frame with the human kappa κ constant and first human γ1 constant regions, respectively (Wu et al., 2003, Methods Mol Biol., 207:197-212; Wu et al., 2003, Methods Mol Biol, 207:213-233), at the expense of the digested parental template. Synthesized DNA was then electroporated into XL1-Blue for plaque formation on XL1-Blue bacterial lawn or production of Fab fragments as described (Wu et al., 2003, Methods Mol Biol., 207:197-212). Fab clones expressing both the GEA44 variable light and variable heavy chains were sequenced (see Figure 6) and screened by ELISA for binding to the EphA4 receptor, confirming the binding properties of the Fab (See assay described below).

**Affinity optimization of Fab GEA44 by parsimonious randomization of each CDR region:** Each amino acid of all 6 Complementary-Determining Regions (CDRs) was individually, randomly mutated using two separate libraries per amino acid (Wu et al., 2003, Methods Mol Biol., 207:197-212). Encoding either 8 amino acids (NSS) or 12 amino acids (NWS) at every CDR amino acid position, each individual degenerate primer was used in a single hybridization mutagenesis reaction (Wu et al., 2003, Methods Mol Biol., 207:197-212; Dall'Acqua et al., 2005, Methods, 36:43-60), and then combined for generation of the corresponding CDR libraries. Briefly, each degenerate primer was phosphorylated, then used in a 10:1 ratio with uridinylated GEA44 Fab (called Fab20 thereafter) single-stranded DNA template (prepared as described in (Wu et al., 2003, Methods Mol Biol., 207:213-233) in an annealing reaction where the temperature was lowered from 95°C to 55°C over 1 hour. T4 ligase and T7 DNA polymerase was added to the annealed reaction and the reaction was incubated for 1.5 hours at 37°C. Synthesis products for every amino acid of each CDR were pooled, however NSS and NWS libraries were kept segregated and screened independently. Typically, one µl of the pooled CDR library synthesized DNA was then electroporated into XL1-Blue for plaque formation on XL1-Blue bacterial lawn or production of Fab fragments as described (Wu et al., 2003, Methods Mol Biol., 207:213-233).

**Screening of the libraries**

**Primary screen:** The primary screen consisted of a single point ELISA (SPE) which was carried out using supernatants containing soluble, secreted Fab protein prepared from 1 ml-bacterial culture grown in 96 deep-well plates and infected with individual recombinant M13 clones essentially as described (Wu et al., 2003, Methods Mol Biol, 207:213-233; Dall'Acqua et. al., 2005, Methods, 36:43-60). Briefly, this capture ELISA involves coating individual wells of a 96-well Maxisorp Immunoplate with approximately 20ng of a goat anti-human Fd antibody (BioDesign Saco, ME), blocking with 3% BSA/PBS for 2 h at 37°C and incubating with samples (soluble, secreted Fabs) for 2 h at room temperature. 200 ng/well of biotinylated human EphA4-Fc or EphA4-His was then added for 2 h at room temperature. This was followed by incubation with neutravidin-horseradish peroxydase (HRP) conjugate (Pierce, IL) for 40 min at room temperature. HRP activity was detected with tetra methyl benzidine (TMB) substrate and the reaction quenched with 0.2 M H₂SO₄. Plates were read at 450 nm.

**Result of the primary screen**: Typically, clones exhibiting an OD 450nm signal at least two times greater than the parental Fab 20 were re-grown at a 15 ml scale, and re-assayed by the same ELISA in duplicate wells to confirm the positive result. Clones, which repeated, were then sequenced and assayed by using an Activity ELISA (see below) to estimate the fold increase of binding to the antigen of interest.

**Secondary screen:** In order to further characterize the previously identified single-change affinity optimized variants (see above), a secondary screen using Fab fragments expressed in periplasmic extracts prepared from 15 ml-bacterial culture (Wu et al., 2003, Methods Mol Biol, 207:213-233) was carried out. More precisely, two ELISAs were used:
(i) an Activity ELISA in which individual wells of a 96-well Maxisorp Immunoplate were coated with ~ 1µg of human EphA4-His and blocked with 3%BSA/PBS for 2 h at 37°C. 2-fold serially diluted samples were then added and incubated for 1 h at room temperature. Incubation with a goat anti-human kappa horseradish peroxydase (HRP) conjugate then followed. HRP activity was detected with TMB substrate and the reaction quenched with 0.2 M H₂SO₄. Plates were read at 450 nm; (ii) an anti-human Fab quantification ELISA which was carried out essentially as described (Wu et al., 2003, Methods Mol Biol, 207:213-233). Briefly, individual wells of a 96-well BIOcoat plate (BD Biosciences, CA) were incubated with 2-fold serially diluted samples or standard (human IgG Fab, 100-1.56 ng/ml). Incubation with a goat anti-human kappa horseradish peroxydase (HRP) conjugate then followed. HRP activity was detected with TMB substrate and the reaction quenched with 0.2 M H₂SO4. Plates were read at 450 nm.

**Results of the secondary screen**: The two-part secondary ELISA screen described in section 3.2.1 allowed us to compare Fab clone 20 and the affinity optimized variants to each other in terms of binding to human EphA4 by normalizing their Fab concentrations. All single -change, affinity optimized variant Fabs exhibited better binding to human EphA4 when compared with the parental Fab of mAb GEA44 (see Figure 7).

**Construction and Characterization of combinatorial variants from CDR affinity optimized clones:** To engineer a combinatorial variant with further improvement in binding, all single amino acid changes which improved binding when compared to parental Fab20 by Activity ELISA were combined to create a small, focused combinatorial library. Briefly, degenerate primers encoding all identified amino acid changes as well as the parental amino acid at the same position were designed (see Figure 11). In an annealing reaction where all primers were included and synthesis followed (see above), a combinatorial library was constructed and screened as previously described (see above).

**Results of primary screening on EphA4**: Typically, clones exhibiting an OD 450nm signal at least two times greater than the parental Fab 20 were re-grown at a 15 ml scale, and re-assayed by ELISA (described above) in duplicate wells (above) to confirm the positive result. Clones which repeated were then sequenced and assayed using an Activity ELISA (see above) to estimate the fold increase of binding to the antigen of interest. From the 12 combinatorial variants selected and sequenced, 9 had unique combinations of CDR amino acid changes, making each variant different from one another by one to three amino acids at the primary sequence level. The nine affinity optimized combinatorial variants carried forward for further evaluation were: 1A4, 1B10, 1D11, 1G11, 2C9, 3A12, 3C6, 6B7, and 8B4 (see Figures 5A and 9).

**Results of secondary screening on EphA4**: Combinatorial variants were analyzed by a secondary screen as described above to estimate the improved binding affinities of the combinatorial variants. All variants had significantly improved affinities for human EphA4 when compared to Fab20 (see Figures 8 and 10).

**Secondary screening with additional EphA receptors:** Since the parental mAb GEA44 not only binds to human EphA4 but also to multiple EphA receptors, additional secondary screening assays were performed using the Activity ELISA (see above) and various recombinant EphA receptor proteins. More precisely, Nunc Maxisorp plates were coated with the following rEphA proteins: rhuEphA1-Fc, rmuEphA2-Fc, rmuEphA3-Fc, rmuEphA4-Fc, rratEphA5-Fc, rmuEphA6-Fc, rmuEphA7-Fc, rEphA8; all at 0.5µg/well (all R&D Systems) rhuEphA2-Fc; 0.5µg/well, and rhuEphA4-His; 0.1µg/well (both produced at MedImmune, Inc.). Plates were blocked with 3% BSA + 1% Tween20 for 2h. All nine affinity optimized combinatorial variants (see above) along with a negative control and Fab20 parental were tested on a single assay plate coated with one of the aforementioned antigens, then starting from undiluted periplasmic Fab samples, titrated in 3 serial 1:2 dilutions in duplicate and incubated 1 h at room temperature. Incubation with a goat anti-human kappa horseradish peroxydase (HRP) conjugate then followed. HRP activity was detected with TMB substrate and the reaction quenched with 0.2 M H₂SO₄. Plates were read at 450 nm. Data was analyzed by normalizing Fab concentrations to the corresponding activity data (see Figures 10B-10H).

**BINDING ANALYSIS**

**Cloning, expression and purification of the of mAb GEA44 affinity optimized combinatorial variants in a human IgG1 format:** The variable regions of the 9 combinatorial variants were PCR-amplified from the corresponding V region-encoding M13 phage vectors using pfu DNA polymerase (Dall' Acqua et. al., 2005, Methods, 36:43-60). They were then individually cloned into mammalian expression vectors encoding a human cytomegalovirus major immediate early (hCMVie) enhancer, promoter and 5'-untranslated region (M. Boshart, et al., 1985, Cell, 41:521-530). In this system, a human yl chain is secreted along with a human κ chain (Johnson, et al., 1997, Infect. Dis., 176:1215-1224). The different constructs were expressed transiently in HEK 293 cells and harvested 72 and 144 hours post-transfection. The secreted, soluble human IgG1s were purified from the conditioned media directly on 1 ml HiTrap protein A columns according to the manufacturer's instructions (APBiotech, Inc., Piscataway, NJ). Purified human IgG1s (typically > 95% homogeneity, as judged by SDS-PAGE) were dialyzed against phosphate buffered saline (PBS), flash frozen and stored at -70°C.

**Secondary screening of IgG constructs of affinity optimized combinatorial variants with additional EphA receptors**: With purified IgG constructs of the nine combinatorial variants, additional secondary screening assays were performed using the Activity ELISA (see above) and recombinant EphA receptor proteins. More precisely, Nunc Maxisorp plates were coated with the following rEphA proteins: rhuEphA1-Fc; 0.5µg/well, rmuEphA2-Fc; 0.5µg/well, rmuEphA3-Fc: 0.1µg/well, rmuEphA4-Fc; 0.1µg/well, rratEphA5-Fc; 0.5µg/well, rmuEphA6-Fc; 0.5 µg/well, rmuEphA7-Fc; 0.µg/well, rEphA8; 0.2µg/well (all R&D Systems), rhuEphA2-Fc; 0.5µg/well, and rhuEphA4-His; 0.1µg/well (both produced at MedImmune, Inc.). Plates were blocked with 3% BSA + 0.1% Tween20 for 2h. All nine IgG affinity optimized combinatorial variants (see above) along with a negative control and parental mAb GEA44 were tested on a single assay plate coated with one of the aforementioned antigens, then starting from 1µg/ml of purified IgG, titrated in 3 serial 1:2 dilutions in duplicate and incubated 1 h at room temperature. Incubation with a goat anti-human kappa horseradish peroxydase (HRP) conjugate then followed. HRP activity was detected with TMB substrate and the reaction quenched with 0.2 M H₂SO₄. Plates were read at 450 nm. Data was analyzed by normalizing Fab concentrations to the corresponding activity data (see Figures 12A-12J and 14). These procedures resulted in antibodies with tailored binding specificities to the Eph family of receptors.

**EXAMPLE 2** Using methods provided herein or otherwise known to one skilled in the art, an antibody is generated against a single member within the RTK family/class of receptors, for example, a class I, II, III, IV, V, VI, VII, VIII, IX, X, XI, XII, XIII, XIV, XV, XVI, XVII, XVIII, or XIX RTK. As provided herein or otherwise known to one skilled in the art, the CDRs of the antibody are randomized to create a library (e.g. a combinatorial library). The library is then subtracted against the original RTK member used to generate the parental antibody. The subtracted library is then selected for binding to RTK(s) of interest to members within a class of the RTK family of receptors. This subtracted library is then selected for the binding specificities to the RTK(s) of interest, with the result being antibodies with tailored binding specificities to members within a class of the RTK family of receptors.

**EXAMPLE 3**
Using methods provided herein or otherwise known to one skilled in the art, a pan-specific antibody to members of, for example, a class I, II, III, IV, V, VI, VII, VIII, IX, X, XI, XII, XIII, XIV, XV, XVI, XVII, XVIII, or XIX RTK is generated. As provided herein or otherwise known to one skilled in the art, the CDRs of the pan-specific antibody are randomized to create a library (e.g. a combinatorial library). The library is then subtracted against a given (or several) RTK members within the class that one does not want the antibody to bind to. This subtracted library is then selected for the binding specificities to the RTK(s) of interest, with the result being antibodies with tailored binding specificities to members within a class of the RTK family of receptors.

**EXAMPLE 4**
Using methods provided herein or otherwise known to one skilled in the art, a pan-specific antibody to members of, for example, a class I, II, III, IV, V, VI, VII, VIII, IX, X, XI, XII, XIII, XIV, XV, XVI, XVII, XVIII, or XIX RTK is generated. As provided herein or otherwise known to one skilled in the art, the CDRs of the pan-specific antibody are randomized to create a library (e.g. a combinatorial library). The combinatorial library is then directly selected against one or several RTK(s) that are desired targets for binding, with the result being antibodies with tailored binding specificities to members within a class of the RTK family of receptors.

**EXAMPLE 5**
Using methods provided herein or otherwise known to one skilled in the art, a pan-specific antibody to members of, for example, a class I, II, III, IV, V, VI, VII, VIII, IX, X, XI, XII, XIII, XIV, XV, XVI, XVII, XVIII, or XIX RTK is generated. Following the steps set forth in any of the previous Examples, the step involving a combinatorial library can be substituted with the use of each individual CDR library, with the end result being antibodies with tailored binding specificities to members within a class of the RTK family of receptors.

### 7. EQUIVALENTS

Those skilled in the art will recognize or be able to ascertain using no more than routine experimentation, many equivalents to the specific embodiments of the invention described herein. Such equivalents are intended to be encompassed by the following claims.

All references cited herein are incorporated herein by reference in their entirety and for all purposes to the same extent as if each individual publication or patent or patent application was specifically and individually indicated to be incorporated by reference in its entirety for all purposes.

The present invention is not to be limited in scope by the specific embodiments described herein. Indeed, various modifications of the invention in addition to those described herein will become apparent to those skilled in the art from the foregoing description and accompanying figures. Such modifications are intended to fall within the scope of the appended claims.

## Claims

1. An antibody that specifically binds the Eph receptor epitope to which monoclonal antibody GEA44 (having V_{H} defined by SEQ ID No. 114 and V_{L}. defined by SEQ ID No. 115) binds.

2. An antibody that has been tallored to specifically bind desired combinations of members of the Eph family of receptors, said binding being represented by the following formulas: EphA(x) + [EphB(y)](n₂); EphA(x)+ [EphA(x)](n₁); EphB(y) + [EphB(y)](n₂); EphBE(y) + [EphA(x)](n₁) or [EphA(x)(n₁)+ [EphB(y)](n₂), wherein (x) is 1, 2, 3, 4, 5, 6, 7, 8, or 10, (y) is 1, 3, 4, 5 or 6, (n₁) is a multiple of 0-8, and (n₂) is a multiple of 0-5.

3. The antibody of claim 2, with the proviso that said antibody is not D7, EA2, EA5, B210, B233, or B208.

4. The antibody of claim 2, wherein said antibody is an agonistic antibody.

5. The antibody of claim 2, wherein said antibody is selected from the group consisting of 1A4 (having V_{H} defined by SEQ ID No. 116 and V_{L} defined by SEQ ID No. 117), 1B10 (having V_{H} defined by SEQ ID No, 118 and V_{L} defined by SEQ ID No. 119), 1D11 (having V_{H} defined by SEQ ID No. 120 and V_{L} defined by SEQ ID No. 121), 1G11 (having V_{H} defined by SEQ ID No. 122 and V_{L}. defined by SEQ ID No. 123), 2C9 (having V_{H} defined by SEQ ID No. 124 and V_{L} defined by SEQ ID No. 125), 11H1 (having V_{H} defined by No, 124 and V_{L} defined by SEQ ID No.125), 11H1 (having V_{H} defined by SEQ ID No. 134 and V_{L} defined by SEQ ID No. 135), (having V_{H} defined by SEQ ID No, 126 and V_{L} defined by SEQ ID No. 127), 3C6 (having V_{H} defined by SEQ ID No. 128 and V_{L} defined by SEQ ID No. 129), 6B7 (having V_{H} defined by SEQ ID No. 130 and V_{L} defined by SEQ ID No. 131), and 8B4 (having V_{H} CDRS defined by SEQ ID Nos. 190 to 192 and V_{L} CDRs defined by SEQ ID NOs. 193 to 195).

6. The antibody of claim 2, wherein said antibody specifically binds at least two, three, four, five, six, seven, eight, nine, ten, eleven, twelve, thirteen, fourteen, fifteen or sixteen members of the Eph family of receptors.

7. The antibody of claim 2, wherein said antibody specifically binds at least two, three, four, five, six, seven, eight or nine members of the EphA family of receptors.

8. The antibody of claim 2, wherein said antibody specifically binds all members of the EphA family of receptors.

9. The antibody of claim 2, wherein said antibody specifically binds at least two, three, four, five or six members of the EphB family of receptors,

10. The antibody of claim 2, wherein said antibody specifically binds all members of the EphB family of receptors.

11. The antibody of claim 2, wherein said antibody is human or humanized.

12. The antibody of claim 2, wherein said antibody specifically binds to a first Eph receptor with a first affinity, and specifically binds to at least a second Eph receptor with an affinity that is equal to, increased, and/or decreased as compared to said first affinity.

13. The antibody of claim 12, wherein said affinity of said antibody for at least a second Eph receptor is decreased.

14. The antibody of claim 12, wherein said affinity of said antibody that is increased and/or decreased is increased and/or decreased by at least 1.1 fold, at least 1,2 fold, at least 1,3 fold, at least 1,4 fold, at least 1,5 fold, at least 1,6 fold, at least 1,7 fold, at least 1,8 fold, at least 1.9 fold, at least 2 fold, at least 2.5 fold, at least 3 fold, at least 3.5 fold, at least 4 fold, at least 4,5 fold , at least 5 fold, at least 6 fold, at least 7 fold, at least 8 fold, at least 9 fold, at least 10 fold, at least 15 fold, at least 20 fold, at least 30 fold, at least 40 fold, at least 50 fold, at least 100 fold, at least 200 fold, at least 300 fold, at least 400 fold, at least 500 fold, or at least 1000 fold,

15. The antibody of claim 12, wherein said first affinity is a K_{D} of at least about 1×10⁶ M⁻¹, at least about 1×10⁷ M⁻¹, at least about 1×10⁸ M⁻¹, at least about 1×10⁹ M⁻¹, at least about 1×10¹⁰ M⁻¹, at least about 1×10¹¹ M⁻¹, at least about 1×10¹² M⁻¹, at least about 1/10¹³M⁻¹, at least about 1×10¹⁴ M⁻¹, or at least about 1×10¹⁵ M⁻¹.

16. The antibody of claim 12, wherein said first affinity is a K_{D} of between about 1×10⁶ M⁻¹ and about 1×10⁷ M⁻¹, between about 1×10⁷ M⁻¹ and about 1×10⁸ M⁻¹, between about 1×10⁸ M⁻¹ and about 1×10⁹ M⁻¹ between about 1×10⁹ M⁻¹ and about 1×10¹⁰ M⁻¹, between about 1×10¹⁰ M⁻¹ and about 1×10¹¹M⁻¹ between about 1×10¹¹ M⁻¹ and about 1×10¹² M⁻¹ between about 1×10¹² M⁻¹ and about 1×10¹³ M⁻¹, between about 1×10¹³ M⁻¹ and about 1×10¹⁴ M⁻¹, or between about 1×10¹⁴ M⁻¹ and about 1×10¹⁶ M⁻¹.

17. A cell line which produces the antibody of claim 2.
